(19) Europäisches Patentamt / European Patent Office / Office européen des brevets

(11) **EP 4 471 029 A1**

(12) **EUROPEAN PATENT APPLICATION**
published in accordance with Art. 153(4) EPC

(43) Date of publication:
**04.12.2024 Bulletin 2024/49**

(21) Application number: **23746237.9**

(22) Date of filing: **19.01.2023**

(51) International Patent Classification (IPC):
***C07D 471/04*** *(2006.01)* ***C07D 471/14*** *(2006.01)*
***A61K 31/519*** *(2006.01)* ***A61K 31/4745*** *(2006.01)*
***A61K 31/4375*** *(2006.01)* ***A61P 35/00*** *(2006.01)*

(52) Cooperative Patent Classification (CPC):
**A61K 31/4375; A61K 31/4745; A61K 31/519; A61P 35/00; C07D 471/04; C07D 471/14**

(86) International application number:
**PCT/CN2023/073092**

(87) International publication number:
**WO 2023/143356 (03.08.2023 Gazette 2023/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**26.01.2022 CN 202210097016**
**12.08.2022 CN 202210965087**
**24.11.2022 CN 202211486374**
**09.01.2023 CN 202310030336**

(71) Applicant: **Suzhou Genhouse Bio Co., Ltd.**
**Suzhou,Jiangsu 215123 (CN)**

(72) Inventors:
- **WANG, Kuifeng**
  **Suzhou, Jiangsu 215123 (CN)**
- **ZHANG, Guiping**
  **Suzhou, Jiangsu 215123 (CN)**
- **LI, Jiapeng**
  **Suzhou, Jiangsu 215123 (CN)**
- **Faridoon**
  **Suzhou, Jiangsu 215123 (CN)**
- **ZHENG, Jiyue**
  **Suzhou, Jiangsu 215123 (CN)**
- **TONG, Chenhua**
  **Suzhou, Jiangsu 215123 (CN)**
- **ZHANG, Wanchun**
  **Suzhou, Jiangsu 215123 (CN)**
- **ZHANG, Tao**
  **Suzhou, Jiangsu 215123 (CN)**
- **SHEN, Ying**
  **Suzhou, Jiangsu 215123 (CN)**

(74) Representative: **ZBM Patents - Zea, Barlocci & Markvardsen**
**Rambla de Catalunya, 123**
**08008 Barcelona (ES)**

(54) **METHIONINE ADENOSYLTRANSFERASE 2A INHIBITOR FOR TREATING MTAP DELETION-TYPE CANCER**

(57) A methionine adenosyltransferase (MAT) 2A inhibitor represented by formula (I), a preparation method thereof, a pharmaceutical composition comprising the same, and pharmaceutical use thereof are disclosed. The compound has excellent MAT2A inhibitory activity.

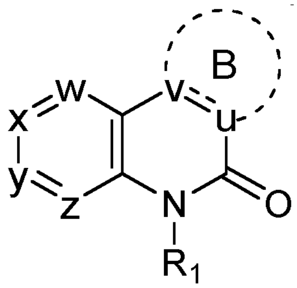

(I)



Processed by Luminess, 75001 PARIS (FR)

## Description

### TECHNICAL FIELD

The present application relates to a compound for treating cancer, in particular to a methionine adenosyltransferase 2A inhibitor, a preparation method thereof, a pharmaceutical composition comprising the same, and pharmaceutical use thereof.

### BACKGROUND

Methionine adenosyltransferase (MAT), also known as S-adenosylmethionine synthetase, has the major biological function of catalyzing the reaction of methionine with ATP to produce a biological methyl donor S-adenosylmethionine (SAM, AdoMet, or SAMe). The MAT-catalyzed reaction is considered to be an important rate-limiting step in the methionine metabolic cycle.

The MAT family includes three family members, MAT1A, MAT2A, and MAT2B. MAT1A is specifically expressed in human liver, while MAT2A and MAT2B are widely distributed in multiple human tissues. In normal liver cells, there is a dynamic equilibrium between MAT1A and MAT2A, which together maintain intracellular SAM homeostasis, whereas in hepatocellular carcinoma (HCC), MAT1A expression is abnormally down-regulated, and MAT2A expression is abnormally up-regulated. This gene expression change from MAT1A to MAT2A promotes cancer invasion and metastasis, and is closely related to increased metastasis and relatively poor recurrence-free survival (RFS) of HCC patients. See, e.g., Ruizhi Wang et al., Molecular Carcinogenesis 57 (9), (2018) 1201-1212. In addition to hepatocellular carcinoma, the abnormally increased expression level of MAT2A is also present in various tumors such as gastric cancer, colorectal cancer, and pancreatic cancer, and is closely related to the development and progression of tumors. MAT2A plays an important role in the pathogenesis of various tumors, and gene silencing of MAT2A can inhibit the proliferation of cancer cells such as liver cancer cells or gastric cancer cells and induce apoptosis. Therefore, MAT2A has become a target for drugs of antitumor therapy. See, e.g., T. Li et al., J. Cancer 7 (10) (2016) 1317-1327 and T. Zhang et al., Acta Histochemica 115 (2013) 48-55.

Further, it has been reported that MAT2A is a synthetic lethal target in MTAP-deficient cancer. MTAP, i.e., *S*-methyl-5'-thioadenosine phosphorylase, is widely distributed in normal tissues and cells, and mainly catalyzes the conversion of methylthioadenosine (MTA) to 5'-methylthioribose-1-phosphate (MTRP) and adenine. The process is an important procedure in the methionine salvage pathway in the human body. The gene encoding human MTAP is located in the chromosome 9p21 region. Studies have shown that the chromosome 9p21 region has a homozygous deletion frequency in all tumors of about 15%, and the deletion frequency varies among different tumors. The cancer with relatively high MTAP deletion frequency includes glioma, mesothelioma, esophageal cancer, bladder cancer, pancreatic cancer, melanoma, non-small cell lung cancer, head and neck cancer, cholangiocarcinoma, esophagogastric cancer, osteosarcoma, brain diffuse glioma, gastric cancer, adrenocortical carcinoma, breast cancer, thymus cancer, hepatocellular carcinoma, ovarian cancer, renal cell carcinoma, and the like. Further, K Marjon et al. found that S-methyl-5'-thioadenosine phosphorylase (MTAP)-deficient cancer was more sensitive to signaling pathways targeting MAT2A/PRMT5/RIOK1 (Cell Reports Volume 15, Issue 3, 2016, 574-587). When MTAP is deleted, the metabolic pathway of MTA is inhibited, resulting in the accumulation of MTA *in vivo* in a large amount. The accumulation of MTA in a large amount will partially inhibit the activity of PRMT5. MAT2A is a key enzyme for SAM production in cells. Inhibition of MAT2A activity results in SAM reduction, causing PRMT5 to lack the corresponding substrate. Therefore, in MTAP-deficient tumor cells, inhibition of MAT2A further inhibits the methylation function of PRMT5, which in turn causes defects in RNA cleavage, gene expression, and chromosomal integrity, thereby inhibiting tumor cell growth.

Given that MAT2A is abnormally expressed in various human tumors (such as liver cancer, pancreatic cancer, gastric cancer, and colon cancer), and inhibition of MAT2A can selectively reduce the proliferative capacity of MTAP-deficient tumor cells and has weak effect on normal cells with MTAP normally expressed, the development of MAT2A inhibitors can be used as an effective tumor treatment method.

The present application provides an effective methionine adenosyltransferase 2A inhibitor, a preparation method thereof, a pharmaceutical composition comprising the same, and pharmaceutical use thereof, in particular for treating MTAP-deficient cancer.

### SUMMARY

In one aspect, the present application provides a compound of formula (I) or a stereoisomer, a tautomer, a solvate, a hydrate, a prodrug, a stable isotopic derivative, or a pharmaceutically acceptable salt thereof,

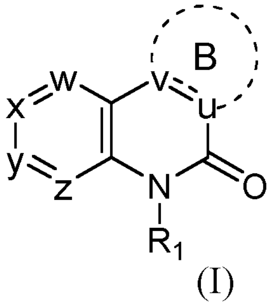

(I)

In some embodiments, the compound of formula (I) or the stereoisomer, the tautomer, the solvate, the hydrate, the prodrug, the stable isotopic derivative, or the pharmaceutically acceptable salt thereof has a structure of formula (IA):

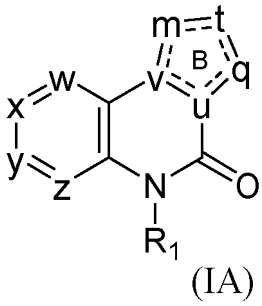

(IA)

In some embodiments, the compound of formula (I) or the stereoisomer, the tautomer, the solvate, the hydrate, the prodrug, the stable isotopic derivative, or the pharmaceutically acceptable salt thereof has a structure of formula ($IB_1$), ($IB_2$), ($IB_3$), ($IB_4$), or (IBs):

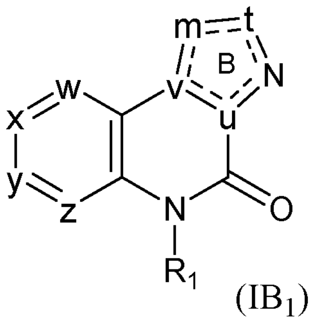

($IB_1$),

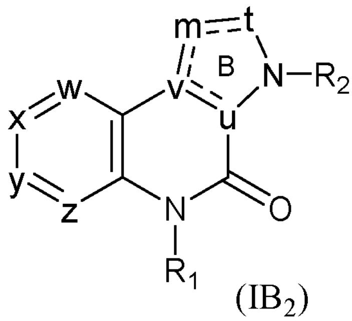

($IB_2$),

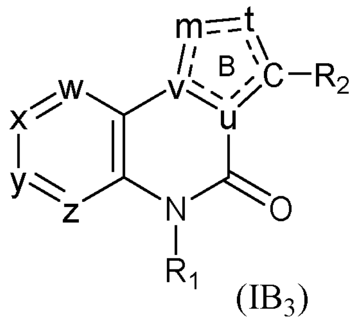

($IB_3$),

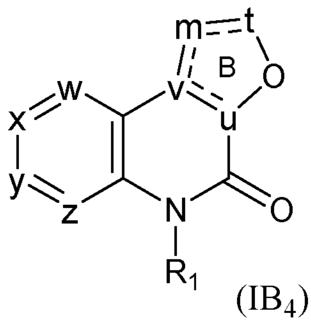

($IB_4$),

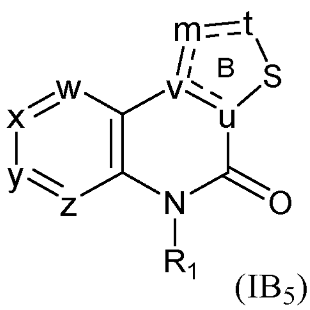

($IB_5$)

In some embodiments, the compound of formula (I) or the stereoisomer, the tautomer, the solvate, the hydrate, the prodrug, the stable isotopic derivative, or the pharmaceutically acceptable salt thereof has a structure of formula ($IC_1$), ($IC_2$), ($IC_3$), ($IC_4$), or ($IC_5$):

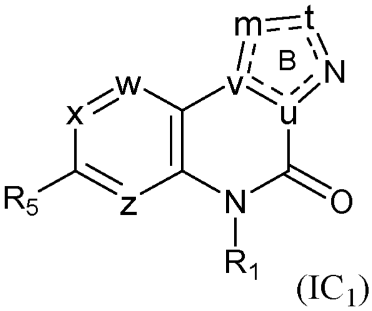
(IC1),
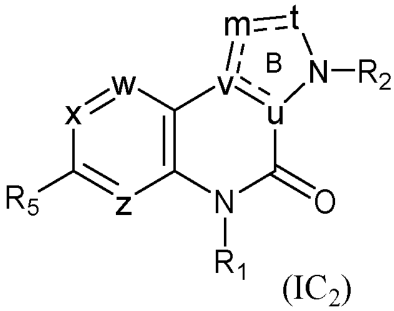
(IC2),
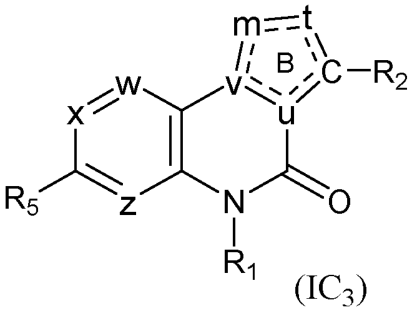
(IC3),
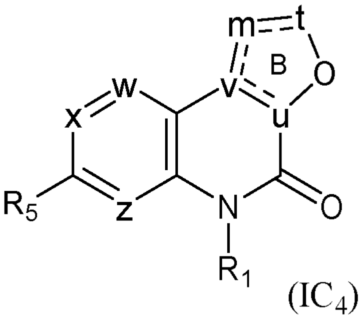
(IC4),
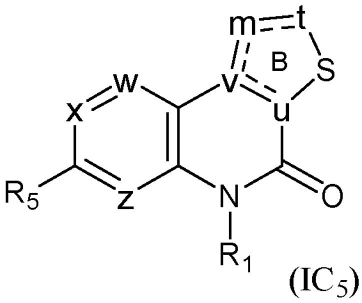
(IC5)

In some embodiments, the compound of formula (I) or the stereoisomer, the tautomer, the solvate, the hydrate, the prodrug, the stable isotopic derivative, or the pharmaceutically acceptable salt thereof has optionally the following structures:

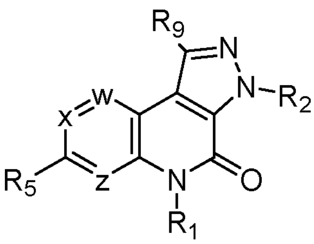
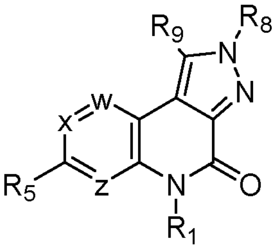
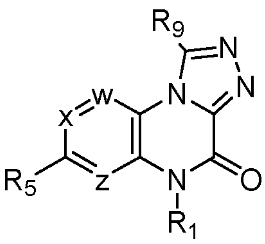
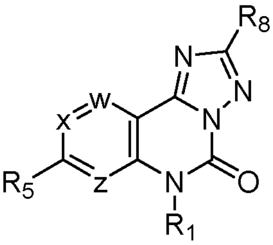
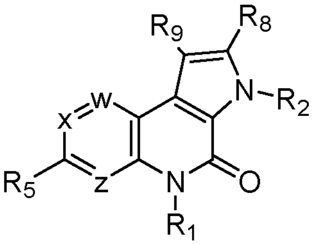
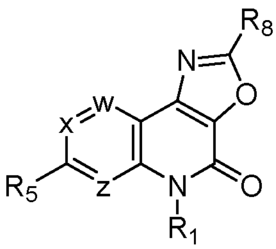

In some embodiments, the compound of formula (I) or the stereoisomer, the tautomer, the solvate, the hydrate, the prodrug, the stable isotopic derivative, or the pharmaceutically acceptable salt thereof has optionally the following structures:

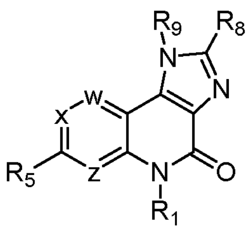

In another aspect, the present application provides a preparation method for the compound of formula (I) described above.

In another aspect, the present application provides a composition comprising a therapeutically effective amount of the compound of formula (I) or the stereoisomer, the tautomer, the solvate, the hydrate, the prodrug, the stable isotopic derivative, or the pharmaceutically acceptable salt thereof according to any one of the above, and one or more pharmaceutically acceptable carriers, diluents, or excipients.

In another aspect, the present application provides use of the compound of formula (I) or the stereoisomer, the tautomer, the solvate, the hydrate, the prodrug, the stable isotopic derivative, or the pharmaceutically acceptable salt thereof according to any one of the above, or the composition according to any one of the above in inhibiting methionine adenosyltransferase 2A (MAT2A), wherein the inhibitory effect may be *in vitro* inhibition or *in vivo* inhibition.

In another aspect, the present application provides use of the compound of formula (I) or the stereoisomer, the tautomer, the solvate, the hydrate, the prodrug, the stable isotopic derivative, or the pharmaceutically acceptable salt thereof according to any one of the above, or the composition according to any one of the above in preparing a drug for inhibiting methionine adenosyltransferase 2A (MAT2A).

In another aspect, the present application provides use of the compound of formula (I) or the stereoisomer, the tautomer, the solvate, the hydrate, the prodrug, the stable isotopic derivative, or the pharmaceutically acceptable salt thereof according to any one of the above, or the composition according to any one of the above in treating and/or preventing cancer, in particular cancer in which a gene encoding methylthioadenosine phosphorylase (MTAP) is deleted and/or is not fully functional.

In another aspect, the present application provides use of the compound of formula (I) or the stereoisomer, the tautomer, the solvate, the hydrate, the prodrug, the stable isotopic derivative, or the pharmaceutically acceptable salt thereof according to any one of the above, or the composition according to any one of the above in preparing a drug for treating and/or preventing cancer, in particular cancer in which a gene encoding methylthioadenosine phosphorylase (MTAP) is deleted and/or is not fully functional.

In another aspect, the present application provides a method for treating and/or preventing cancer, comprising administering to a patient a therapeutically effective amount of the compound of formula (I) or the stereoisomer, the tautomer, the solvate, the hydrate, the prodrug, the stable isotopic derivative, or the pharmaceutically acceptable salt thereof according to any one of the above, or the composition according to any one of the above, wherein the cancer includes those in which a gene encoding methylthioadenosine phosphorylase (MTAP) is deleted and/or is not fully functional.

The methionine adenosyltransferase 2A inhibitor with a novel structure provided by the present application has excellent inhibitory activity.

## DETAILED DESCRIPTION

In order to better understand the content of the present application, the terms in the present application are explained as follows.

"Alkyl" refers to a saturated aliphatic hydrocarbon group that is a linear or branched group containing 1 to 20 carbon atoms, preferably alkyl containing 1 to 12 carbon atoms, and more preferably alkyl containing 1 to 6 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched-chain isomers thereof, and the like. The alkyl may be optionally independently substituted with one or more of the substituents described herein.

"Alkylene" refers to a saturated linear or branched aliphatic hydrocarbon group having 2 residues derived from the parent alkane by removal of two hydrogen atoms from the same carbon atom or two different carbon atoms. It is a linear or branched group containing 1 to 20 carbon atoms, preferably containing 1 to 12 carbon atoms, and more preferably alkylene containing 1 to 6 carbon atoms. Non-limiting examples of alkylene include, but are not limited to, methylene ($-CH_2-$), 1,1-ethylene ($-CH(CH_3)-$), 1,2-ethylene ($-CH_2CH_2-$), 1,1-propylene ($-CH(CH_2CH_3)-$), 1,2-propylene ($-CH_2CH(CH_3)-$), 1,3-propylene ($-CH_2CH_2CH_2-$), 1,4-butylene ($-CH_2CH_2CH_2CH_2-$), and the like. The alkylene may be optionally independently substituted with one or more of the substituents described herein.

"Alkenyl" refers to a linear or branched monovalent hydrocarbon group containing 2 to 8 carbon atoms and having at least one site of unsaturation (i.e., a carbon-carbon $sp^2$ double bond), wherein the alkenyl may be optionally

independently substituted with one or more substituents described herein, and includes groups having *"cis"* and *"trans"* orientations (or "*E*" and "*Z*" orientations). Examples include, but are not limited to, vinyl ($-CH=CH_2$), allyl ($-CH_2CH=CH_2$), and the like.

"Alkynyl" refers to a linear or branched monovalent hydrocarbon group containing 2 to 8 carbon atoms and having at least one site of unsaturation (i.e., a carbon-carbon sp triple bond), wherein the alkynyl may be optionally independently substituted with one or more substituents described herein. Examples include, but are not limited to, ethynyl (-C=CH), propynyl (propargyl and $-CH_2C{\equiv}CH$), and the like.

"Carbocyclic ring" or "cycloalkyl" that may be used interchangeably herein refers to a saturated or partially unsaturated monocyclic or polycyclic hydrocarbon substituent. The cycloalkyl ring contains 3 to 20 carbon atoms, preferably 3 to 12 carbon atoms, more preferably 3 to 8 carbon atoms, and most preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spirocycloalkyl, fused cycloalkyl, and bridged cycloalkyl. The cycloalkyl may be optionally independently substituted with one or more of the substituents described herein.

"Spirocycloalkyl" refers to a 5- to 20-membered polycyclic group in which monocyclic rings share one carbon atom (referred to as a spiro atom). It may contain one or more double bonds, but none of the rings has a fully conjugated $\pi$-electron system. It is preferably 6- to 14-membered, and is more preferably 7- to 10-membered. According to the number of spiro atoms shared among rings, spirocycloalkyl may be monospirocycloalkyl, bispirocycloalkyl or polyspirocycloalkyl, preferably monospirocycloalkyl and bispirocycloalkyl. Non-limiting examples of spirocycloalkyl include:

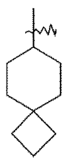 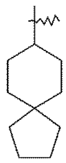 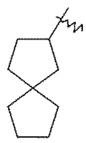 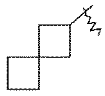 and 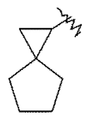

"Fused cycloalkyl" refers to a 5- to 20-membered all-carbon polycyclic group in which each ring shares a pair of adjacent carbon atoms with the other rings in the system, wherein one or more of the rings may contain one or more double bonds. It is preferably 6- to 14-membered, and is more preferably 7- to 10-membered. According to the number of the formed rings, fused cycloalkyl may be bicyclic, tricyclic, tetracyclic, or polycyclic fused cycloalkyl, and is preferably bicyclic or tricyclic. Non-limiting examples of fused cycloalkyl include:

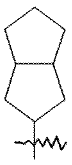 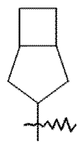 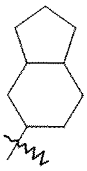 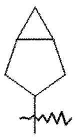 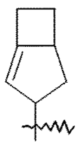 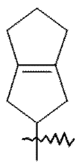 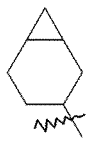 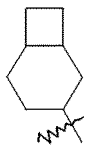 and 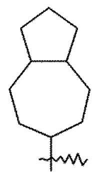

"Bridged cycloalkyl" refers to a 5- to 20-membered all-carbon polycyclic group in which any two rings share two carbon atoms that are not directly connected, and it may contain one or more double bonds. It is preferably 6- to 14-membered, and is more preferably 7- to 10-membered. According to the number of the formed rings, bridged cycloalkyl may be bicyclic, tricyclic, tetracyclic or polycyclic bridged cycloalkyl, preferably bicyclic, tricyclic or tetracyclic, and more preferably bicyclic or tricyclic. Non-limiting examples of bridged cycloalkyl include:

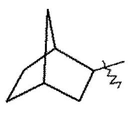 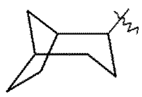 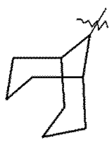 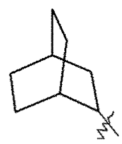 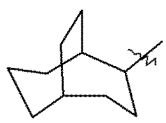

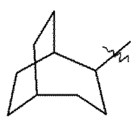 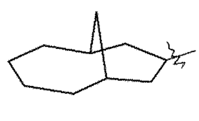 and 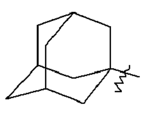

The cycloalkyl ring includes those in which the cycloalkyl described above (including monocyclic, spiro, fused

and bridged rings) is fused to an aryl, heteroaryl or heterocycloalkyl ring, wherein the ring connected to the parent structure is cycloalkyl. Non-limiting examples include indanyl, tetrahydronaphthyl, benzocycloheptanyl, and the like.

"Alkoxy" refers to (alkyl)-O-, wherein the alkyl is as defined above. Non-limiting examples of alkoxy include methoxy, ethoxy, propoxy, and butoxy. The alkoxy may be optionally independently substituted with one or more of the substituents described herein.

"Heterocyclic ring" or "heterocyclyl" that may be used interchangeably herein refers to a saturated or partially unsaturated monocyclic or polycyclic substituent containing 3 to 20 ring atoms, wherein one or more of the ring atoms are heteroatoms selected from nitrogen, oxygen and sulfur, the sulfur optionally being substituted with oxo (i.e., to form sulfoxide or sulfone), but excluding a cyclic portion of -O-O-, -O-S- or -S-S-; the remaining ring atoms are carbon. Preferably, it contains 3 to 12 (e.g., 3, 4, 5, 6, 7, 8, 9, 10, 11, and 12) ring atoms, wherein 1 to 4 (e.g., 1, 2, 3, and 4) are heteroatoms; more preferably, it contains 3 to 8 ring atoms (e.g., 3, 4, 5, 6, 7, and 8), wherein 1-3 (e.g., 1, 2, and 3) are heteroatoms. Non-limiting examples of monocyclic heterocyclyl include pyrrolidinyl, tetrahydropyranyl, 1,2,3,6-tetrahydropyridinyl, piperidinyl, piperazinyl, morpholinyl, thiomorpholinyl, homopiperazinyl, and the like. Polycyclic heterocyclyl includes spiroheterocyclyl, fused heterocyclyl, and bridged heterocyclyl. The heterocyclyl may be optionally independently substituted with one or more of the substituents described herein.

"Spiroheterocyclyl" refers to a 5- to 20-membered polycyclic heterocyclyl group in which monocyclic rings share one atom (referred to as a spiro atom), wherein one or more of the ring atoms are heteroatoms selected from nitrogen, oxygen and sulfur, the sulfur optionally being substituted with oxo (i.e., to form sulfoxide or sulfone); the remaining ring atoms are carbon. It may contain one or more double bonds. It is preferably 6- to 14-membered, and more preferably 7- to 10-membered (e.g., 7-membered, 8-membered, 9-membered or 10-membered). According to the number of spiro atoms shared among rings, spiroheterocyclyl may be monospiroheterocyclyl, bispiroheterocyclyl or polyspiroheterocyclyl, preferably, monospiroheterocyclyl and bispiroheterocyclyl. Non-limiting examples of spiroheterocyclyl include:

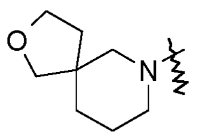 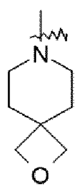 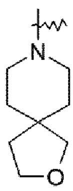 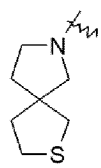 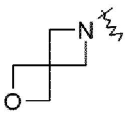 and 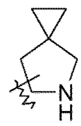

"Fused heterocyclyl" refers to a 5- to 20-membered polycyclic heterocyclyl group in which each ring shares a pair of adjacent atoms with the other rings in the system, wherein one or more of the rings may contain one or more double bonds, wherein one or more of the ring atoms are heteroatoms selected from nitrogen, oxygen and sulfur, the sulfur optionally being substituted with oxo (i.e., to form sulfoxide or sulfone); the remaining ring atoms are carbon. It is preferably 6- to 14-membered, and more preferably 7- to 10-membered (e.g., 7-membered, 8-membered, 9-membered or 10-membered). According to the number of the formed rings, fused heterocyclyl may be bicyclic, tricyclic, tetracyclic, or polycyclic fused heterocyclyl, and is preferably bicyclic or tricyclic. Non-limiting examples of fused heterocyclyl include:

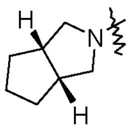 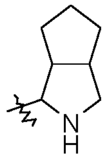 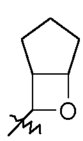 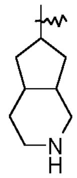 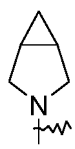 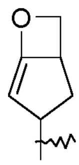 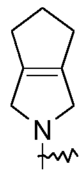 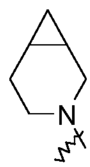

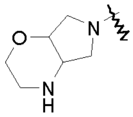 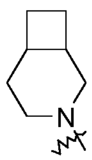 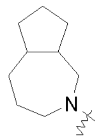 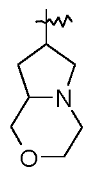 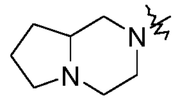 and 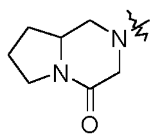

"Bridged heterocyclyl" refers to a 5- to 14-membered polycyclic heterocyclyl group in which any two rings share two atoms that are not directly connected, and it may contain one or more double bonds, wherein one or more ring atoms are heteroatoms selected from nitrogen, oxygen, and sulfur, the sulfur optionally being substituted with oxo (i.e., to form sulfoxide or sulfone); the remaining ring atoms are carbon. It is preferably 6- to 14-membered, and more preferably 7- to 10-membered (e.g., 7-membered, 8-membered, 9-membered or 10-membered). According to the number of the formed rings, bridged heterocyclyl may be bicyclic, tricyclic, tetracyclic, or polycyclic bridged heterocyclyl, and is preferably

bicyclic, tricyclic, or tetracyclic. Non-limiting examples of bridged heterocyclyl include:

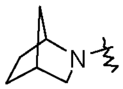 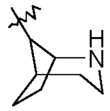 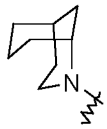 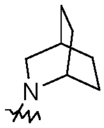

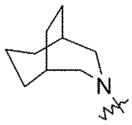 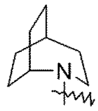 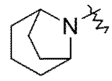 and 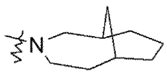

The heterocyclyl ring includes those in which the heterocyclyl described above (including monocyclic, spiro heterocyclic, fused heterocyclic and bridged heterocyclic rings) is fused to an aryl, heteroaryl or cycloalkyl ring, wherein the ring connected to the parent structure is heterocyclyl; its non-limiting examples include:

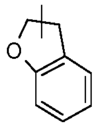 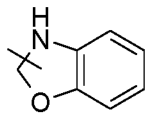 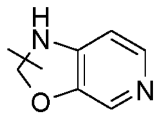 and 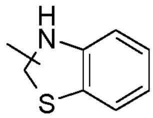

"Aromatic ring" or "aryl" that may be used interchangeably herein refers to a 6- to 14-membered all-carbon monocyclic or fused polycyclic (fused polycyclic rings are rings that share a pair of adjacent carbon atoms) group with a conjugated π-electron system, which is preferably 6- to 10-membered, e.g., phenyl and naphthyl. The aryl ring includes those in which the aryl ring described above is fused to a heteroaryl, heterocyclyl or cycloalkyl ring, wherein the ring connected to the parent structure is an aryl ring; its non-limiting examples include:

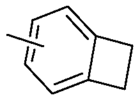 , 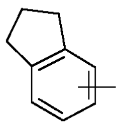 , 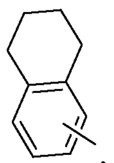 , 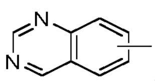 , 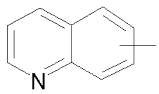 , 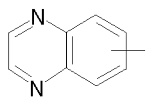 , 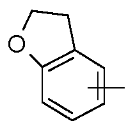 ,

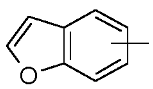 , 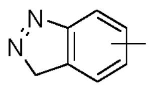 , 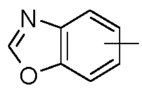 , 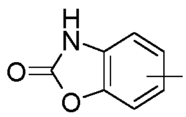 , 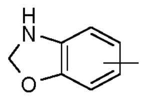 , 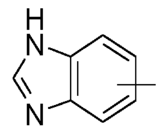 ,

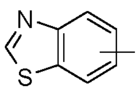 , 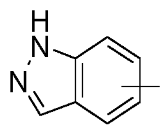 , 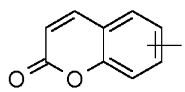 , and 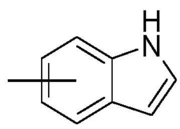

The aryl may be optionally independently substituted with one or more of the substituents described herein.

"Heteroaromatic ring" and "heteroaryl" that may be used interchangeably herein refers to a heteroaromatic system containing 1 to 4 (e.g., 1, 2, 3 and 4) heteroatoms and 5 to 14 ring atoms, wherein the heteroatoms are selected from oxygen, sulfur and nitrogen. The heteroaryl is preferably 5- to 10-membered (e.g., 5-, 6-, 7-, 8-, 9- or 10-membered), and is more preferably 5- or 6-membered, e.g., furanyl, thienyl, pyridinyl, pyrrolyl, *N*-alkylpyrrolyl, pyrimidinyl, pyrazinyl, pyridazinyl, imidazolyl, pyrazolyl, triazolyl, tetrazolyl, etc. The heteroaryl ring includes those in which the heteroaryl described above is fused to an aryl, heterocyclyl or cycloalkyl ring, wherein the ring connected to the parent structure is a heteroaryl ring; its non-limiting examples include:

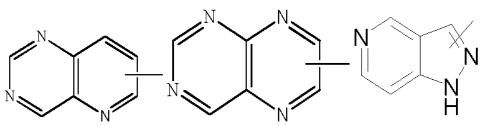 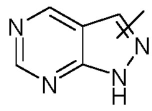 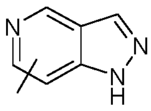 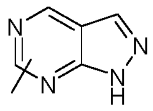 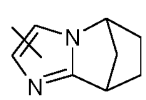

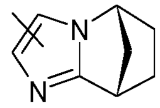 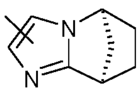 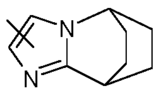 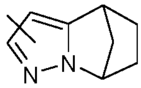 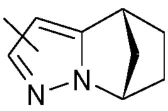 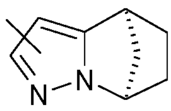

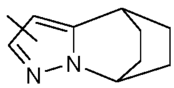 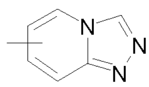 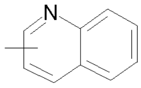 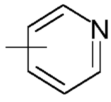 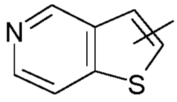 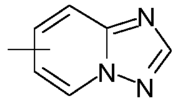 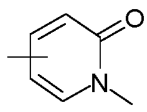

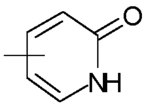 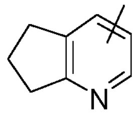 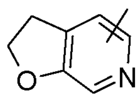 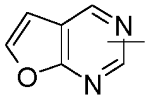 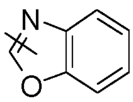 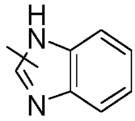 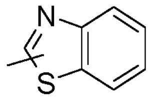

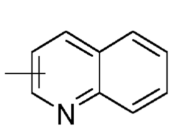 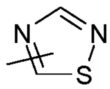 and 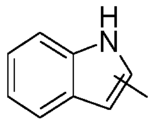

The heteroaryl may be optionally independently substituted with one or more of the substituents described herein, which when substituted, may be substituted at any available linking site.

The cycloalkyl, heterocyclyl, aryl and heteroaryl described above include residues derived from the parent ring by removal of one hydrogen atom from a ring atom, or residues derived from the parent ring by removal of two hydrogen atoms from the same ring atom or two different ring atoms, i.e., "divalent cycloalkyl", "divalent heterocyclyl", "arylene" and "heteroarylene".

"Cycloalkyloxy" refers to cycloalkyl-O-, wherein the cycloalkyl is as defined above. The cycloalkyloxy may be optionally independently substituted with one or more of the substituents described herein.

"Heterocyclyloxy" refers to heterocyclyl-O-, wherein the heterocyclyl is as defined above. The heterocyclyloxy may be optionally independently substituted with one or more of the substituents described herein.

"Aryloxy" refers to aryl-O-, wherein the aryl is as defined above. The aryloxy may be optionally independently substituted with one or more of the substituents described herein.

"Heteroaryloxy" refers to heteroaryl-O-, wherein the heteroaryl is as defined above. The heteroaryloxy may be optionally independently substituted with one or more of the substituents described herein.

"Sulfonyl" refers to $-SO_2-$. The sulfonyl may be optionally substituted with the substituent described herein, such as alkylsulfonyl, cycloalkylsulfonyl, heterocyclylsulfonyl, arylsulfonyl, wherein the alkyl, cycloalkyl, heterocyclyl, and aryl are as defined above.

"Alkylthio" refers to alkyl-S-, wherein the alkyl is as defined above.

"Halogen" refers to fluorine, chlorine, bromine, or iodine.

"Cyano" refers to -CN.

"Hydroxyl" refers to -OH.

"Oxo" refers to "=O".

"Carboxyl" refers to -C(=O)OH.

"Amino" refers to $-NH_2$, which is optionally substituted with one or more of the substituents described herein, to form, for example, alkylamino, dialkylamino, cycloalkylamino, heterocyclylamino, and arylamino, wherein the alkyl, cycloalkyl, heterocyclyl, and aryl are as defined above.

"Alkylamino" refers to the RNH- group, wherein R is the alkyl as defined above. For example, $C_1$-$C_6$ alkylamino may be methylamino, ethylamino, and the like.

"Dialkylamino" refers to the RR'N- group, wherein R and R' are each the alkyl as defined above. For example, di($C_1$-$C_6$)alkylamino may be dimethylamino, methylethylamino, and the like.

"Ureido" means the -NH-C(=O)-NH2 group, which is optionally substituted with one or more of the substituents described herein.

"Acyl" refers to the "-C(=O)-" group, which is optionally substituted with any substituent to form, for example, alkylacyl, alkoxyacyl, cycloalkylacyl, heterocyclylacyl, arylacyl, aminoacyl, etc., e.g., $C_1$-$C_6$ alkylacyl, $C_1$-$C_6$ alkoxyacyl, cycloalkylacyl having 3-10 ring atoms, heterocyclylacyl having 4-10 ring atoms, aryl acyl having 6-10 ring atoms, etc., wherein the alkyl, alkoxy, cycloalkyl, heterocyclyl, and aryl are as defined above. For example, non-limiting examples of $C_1$-$C_6$ alkylacyl include formyl, acetyl, 3-methylpentanoyl, and the like.

The compounds disclosed herein include isotopic derivatives thereof. The term "isotopic derivative" refers to compounds that differ in structure only by having one or more enriched isotopic atoms. For example, compounds with the structure disclosed herein having "deuterium" or "tritium" in place of hydrogen, or $^{18}$F-fluorine labeling ($^{18}$F isotope) in place of fluorine, or $^{11}$C-, $^{13}$C- or $^{14}$C-enriched carbon ($^{11}$C-, $^{13}$C- or $^{14}$C-carbon labeling; $^{11}$C-, $^{13}$C- or $^{14}$C-isotope) in place of a carbon atom are within the scope of the present disclosure. Such a compound can be used as an analytical tool or a probe in, for example, a biological assay, or may be used as a tracer for *in vivo* diagnostic imaging of disease, or as a tracer in a pharmacodynamic, pharmacokinetic, or receptor study. The various deuterated forms of the compound of the present disclosure mean that each available hydrogen atom connected to a carbon atom may be independently replaced with a deuterium atom. Those skilled in the art can synthesize the compounds in deuterated form by reference to the relevant literature. Commercially available deuterated starting materials can be used in preparing the deuterated compounds, or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like. Deuterides can generally retain comparable activity to non-deuterated compounds and can achieve better metabolic stability when deuterated at certain specific sites, thereby achieving certain therapeutic advantages.

"Optional" or "optionally" means that the event or circumstance subsequently described may, but does not necessarily, occur, and that the description includes instances where the event or circumstance occurs or does not occur. For example, "heterocyclyl group optionally substituted with alkyl" means that alkyl may be, but not necessarily, present, and that the description includes instances where the heterocyclyl group is or is not substituted with alkyl.

"Each independently" means that at least two groups (or ring systems) with the same or similar value ranges in the structure may have the same or different meanings under a certain circumstance. For example, X and Y are each independently hydrogen, halogen, hydroxyl, cyano, alkyl or aryl, meaning that when X is hydrogen, Y may be hydrogen or halogen, hydroxyl, cyano, alkyl or aryl; similarly, when Y is hydrogen, X may be hydrogen or halogen, hydroxyl, cyano, alkyl or aryl.

"Substituted" means that one or more, preferably 1-5, more preferably 1-3 hydrogen atoms in the group are independently substituted with a corresponding number of substituents. Those skilled in the art are able to determine (experimentally or theoretically) possible or impossible substitution without undue effort. For example, it may be unstable when amino or hydroxyl having a free hydrogen is bound to a carbon atom having an unsaturated (such as olefin) bond.

For drugs or pharmacologically active agents, the term "therapeutically effective amount" refers to an amount of a medicament or an agent that is sufficient to provide the desired effect but is non-toxic. The determination of the effective amount varies from person to person. It depends on the age and general condition of a subject, as well as the particular active substance used. The appropriate effective amount in a case may be determined by those skilled in the art in the light of routine tests.

"Pharmaceutical composition" refers to a mixture containing one or more of the compounds described herein or a physiologically/pharmaceutically acceptable salt or pro-drug thereof, and other chemical components, for example, physiologically/pharmaceutically acceptable carriers and excipients. The pharmaceutical composition is intended to promote administration to an organism and facilitate the absorption of the active ingredient so that it can exert its biological activity.

"Pharmaceutically acceptable salt" refers to the salts of the compound of the present disclosure, which are safe and effective for use in the body of a mammal and possess the requisite biological activities. The salts may be prepared separately during the final separation and purification of the compound, or by reacting an appropriate group with an appropriate base or acid. Bases commonly used to form pharmaceutically acceptable salts include inorganic bases such as sodium hydroxide and potassium hydroxide, and organic bases such as ammonia. Acids commonly used to form pharmaceutically acceptable salts include inorganic acids and organic acids.

"Solvate" used herein refers to a substance formed by the physical association of the compound of the present disclosure to one or more, preferably 1 to 3, solvent molecules, whether organic or inorganic. The physical association includes hydrogen bonding. In certain cases, e.g., when one or more, preferably 1 to 3, solvent molecules are incorporated in the crystal lattice of the crystalline solid, the solvate will be isolated. Exemplary solvates include, but are not limited to, hydrates, ethanolates, methanolates, and isopropanolates. Solvation methods are well known in the art.

"Hydrate" refers to a substance formed by the binding of the compounds of the present invention or the pharmaceutically acceptable salt thereof to water through non-covalent intermolecular forces. Common hydrates include, but are not limited to, hemihydrate, monohydrate, dihydrate, trihydrate, etc.

"Prodrug" refers to a substance that can be converted *in vivo* under physiological conditions, e.g., by hydrolysis in

blood, to generate the active parent drug compound.

"Pharmaceutically acceptable" means that those compounds, materials, compositions, and/or dosage forms that are, within the scope of reasonable medical judgment, suitable for use in contact with the tissues of patients without excessive toxicity, irritation, allergic reaction, or other problems or complications, and are commensurate with a reasonable benefit/risk ratio and effective for the intended use.

The "stereoisomer" refers to compounds having the same chemical structure but differing in the arrangement of atoms or groups in space. Stereoisomers include enantiomers, diastereoisomers, conformers (rotamers), geometric isomers (*cis*/*trans* isomers), atropisomers, and the like.

"Tautomer" refers to isomers of a compound that differ from each other in proton position and/or electron distribution. Examples of tautomers include, but are not limited to, enol-keto tautomers, imine-enamine tautomers, amide-imidic acid tautomers, amine-imine tautomers, and tautomeric forms of heteroaryl, wherein the heteroaryl comprises a ring atom connected to an -NH-moiety of the ring and an =N- moiety of the ring, such as pyrazole, imidazole, benzimidazole, triazole, pyridotriazole, piperidinotriazole, and tetrazole.

*"In vitro"* refers to a biological entity, a biological process, or a biological reaction under artificial conditions outside the body. By way of example, cells grown *in vitro* are understood to be cells grown in an environment outside the body, for example in test tubes, culture plates or microtiter plates.

In one aspect, the present application provides a compound of formula (I) or a stereoisomer, a tautomer, a solvate, a hydrate, a prodrug, a stable isotopic derivative, or a pharmaceutically acceptable salt thereof,

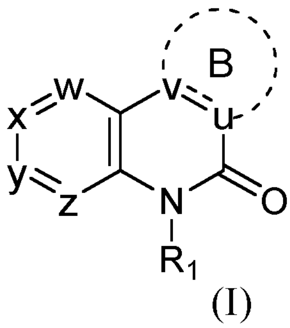

(I) ,

wherein:

w is $CR_3$ or N;
x is $CR_4$ or N;
y is $CR_5$ or N;
z is $CR_6$ or N;
v and u are each independently selected from C or N;

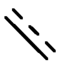 indicates that it may be a single bond or double bond;

$R_3$ and $R_5$ are each independently selected from hydrogen, alkyl, alkenyl, alkynyl, alkoxy, sulfonyl, halogen, cycloalkyl, cyano, amino, acyl, hydroxyl, heteroaryl, heteroaryloxy, heterocyclyl, heterocyclyloxy, and cycloalkyloxy; $R_3$ is optionally further substituted with one or more $R_{3a}$, and $R_5$ is optionally further substituted with one or more $R_{5a}$, wherein each $R_{3a}$ and each $R_{5a}$ are independently selected from alkyl, alkenyl, alkynyl, alkoxy, alkylamino, dialkylamino, halogen, sulfonyl, cycloalkyl, cyano, amino, acyl, hydroxyl, heteroaryl, heteroaryloxy, cycloalkyloxy, heterocyclyl, and heterocyclyloxy; optionally, $R_{3a}$ is further substituted with one or more $R_{3b}$, and $R_{5a}$ is further substituted with one or more $R_{5b}$, wherein each $R_{3b}$ and each $R_{5b}$ are independently selected from alkyl, halogen, hydroxyl, alkoxy, amino, and heterocyclyloxy;

$R_4$ and $R_6$ are each independently selected from hydrogen, alkyl, alkenyl, alkynyl, alkoxy, alkylthio, sulfonyl, halogen, cycloalkyl, cyano, amino, and acyl; optionally, $R_4$ is further substituted with one or more $R_{4a}$, and $R_6$ is further substituted with one or more $R_{6a}$, wherein each $R_{4a}$ and each $R_{6a}$ are independently selected from alkyl, halogen, amino, alkylamino, and dialkylamino;

$R_1$ is $-X_1-R_7$, wherein $X_1$ is a bond or alkylene; optionally, $X_1$ is substituted with one or more $X_{1a}$, and each $X_{1a}$ is independently selected from halogen, alkyl, and cycloalkyl; $R_7$ is selected from cycloalkyl, aryl, heteroaryl, and heterocyclyl, wherein $R_7$ is optionally substituted with one or more $R_{7a}$, and each $R_{7a}$ is independently selected from alkyl, alkoxy, hydroxyl, sulfonyl, halogen, cyano, alkenyl, alkynyl, carboxyl, acyl, amino, cycloalkyl, aryl, heterocyclyl, heteroaryl, oxo, and ureido; optionally, $R_{7a}$ is further substituted with one or more $R_{7b}$, wherein $R_{7b}$ is independently selected from halogen, alkyl, alkoxy, hydroxyl, amino, alkenyl, alkynyl, alkylamino, dialkylamino, sulfonyl, heterocyclyl, and heteroaryl;

B is selected from 5-, 6-, or 7-membered aryl or heteroaryl, wherein B is optionally substituted with one or more $R_2$; each $R_2$ is independently selected from hydrogen, halogen, alkyl, alkoxy, alkenyl, alkynyl, cycloalkyl, cycloalkyloxy, heterocyclyl, heterocyclyloxy, aryl, heteroaryl, heteroaryloxy, cyano, amino, acyl, sulfonyl, and oxo, or two adjacent $R_2$ form a 3- to 7-membered carbocyclic ring, heterocyclic ring, aromatic ring, or heteroaromatic ring; optionally, $R_2$ or the 3- to 7-membered carbocyclic ring, heterocyclic ring, aromatic ring, or heteroaromatic ring formed by two adjacent $R_2$ is further substituted with one or more $R_{2a}$, wherein each $R_{2a}$ is independently selected from halogen, alkyl, heterocyclyl, heterocyclyloxy, amino, heteroaryl, cycloalkyl, aryl, alkylamino, dialkylamino, hydroxyl, sulfonyl, alkoxy, cyano, alkenyl, acyl, alkynyl, and oxo; optionally, $R_{2a}$ is further substituted with one or more $R_{2b}$, wherein each $R_{2b}$ is independently selected from alkyl substituted with 0-3 halogens, amino, alkylamino, dialkylamino, alkoxy substituted with 0-3 halogens, halogen, hydroxyl, unsubstituted heterocyclyl or heterocyclyl substituted with alkyl, cyano, and oxo, preferably selected from alkyl, amino, alkylamino, dialkylamino, alkoxy, halogen, hydroxyl, and oxo.

In some embodiments, B is 6-membered heteroaryl.

In some embodiments, B is selected from:

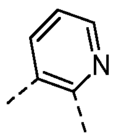 , 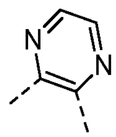 , 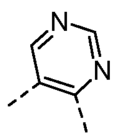 , 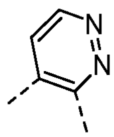 , 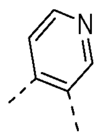 , 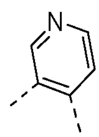 , 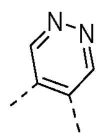 , 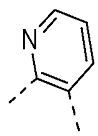 , and 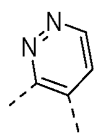 ,

wherein the dashed line 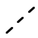 indicates where B is connected to the parent structure.

In some embodiments, B is 5-membered aryl or heteroaryl.

In some embodiments, B is 5-membered heteroaryl comprising at least two heteroatoms selected from N, O, and S.

In some embodiments, B is optionally substituted with one or more $R_2$, wherein each $R_2$ is independently selected from hydrogen, halogen, $C_1$-$C_6$ alkyl, $C_1$-$C_6$ alkoxy, $C_2$-$C_6$ alkenyl, $C_2$-$C_6$ alkynyl, cycloalkyl having 3-10 ring atoms, cycloalkyloxy having 3-10 ring atoms, heterocyclyl having 4-10 ring atoms, heterocyclyloxy having 4-10 ring atoms, aryl having 6-10 ring atoms, heteroaryl having 5-10 ring atoms, heteroaryloxy having 5-10 ring atoms, cyano, amino, acyl, sulfonyl, and oxo; preferably, $R_2$ is selected from hydrogen, halogen, $C_1$-$C_4$ alkyl, $C_1$-$C_4$ alkoxy, $C_2$-$C_4$ alkenyl, $C_2$-$C_4$ alkynyl, cycloalkyl having 3-6 ring atoms, cycloalkyloxy having 3-6 ring atoms, heterocyclyl having 5-6 ring atoms, heterocyclyloxy having 5-6 ring atoms, aryl having 6-10 ring atoms, heteroaryl having 5-6 ring atoms, heteroaryloxy having 5-6 ring atoms, cyano, amino, acyl, sulfonyl, and oxo; more preferably, $R_2$ is selected from hydrogen, methyl, ethyl, n-propyl, isopropyl, cyano, methoxy, and oxo.

In some embodiments, two adjacent $R_2$ form a 5-6 membered carbocyclic ring, heterocyclic ring, or heteroaromatic ring, preferably 5-6 membered heterocyclic ring or heteroaromatic ring, and more preferably

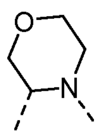 or 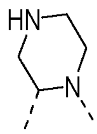 ,

wherein the dashed line 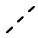 indicates where the ring is connected to the parent structure.

In some embodiments, $R_2$ or the ring structure formed by two adjacent $R_2$ is further substituted with one or more $R_{2a}$, wherein each $R_{2a}$ is independently selected from halogen, $C_1$-$C_6$ alkyl, heterocyclyl having 4-10 ring atoms, heterocyclyloxy having 4-10 ring atoms, amino, heteroaryl having 5-10 ring atoms, cycloalkyl having 3-10 ring atoms, aryl having 6-10 ring atoms, $C_1$-$C_6$ alkylamino, di($C_1$-$C_6$)alkylamino, hydroxyl, sulfonyl, $C_1$-$C_6$ alkoxy, cyano, $C_2$-$C_6$ alkenyl, $C_2$-$C_6$ alkynyl, and oxo; preferably, each $R_{2a}$ is independently selected from halogen, $C_1$-$C_4$ alkyl, heterocyclyl having 5-6 ring atoms, heterocyclyloxy having 5-6 ring atoms, amino, heteroaryl having 5-6 ring atoms, cycloalkyl having 3-6 ring atoms, aryl having 6-10 ring atoms, $C_1$-$C_3$ alkylamino, di($C_1$-$C_3$)alkylamino, hydroxyl, sulfonyl, $C_1$-$C_4$ alkoxy, cyano, $C_2$-$C_4$ alkenyl, $C_2$-$C_4$ alkynyl, and oxo; more preferably, each $R_{2a}$ is independently selected from methyl, chlorine, fluorine, phenyl, cyclopropyl, oxo, amino, methylamino, dimethylamino, methoxy, hydroxyl, pyridinyl, pyrazolyl, and pyrimidinyl; most preferably, each $R_{2a}$ is independently selected from methyl, chlorine, fluorine, phenyl, cyclopropyl, oxo, amino, methylamino, dimethylamino, methoxy, hydroxyl, pyridinyl, and pyrazolyl.

In some embodiments, $R_{2a}$ is further substituted with one or more $R_{2b}$, wherein each $R_{2b}$ is independently selected from $C_1$-$C_3$ alkyl substituted with 0-3 halogens, $C_1$-$C_3$ alkoxy substituted with 0-3 halogens, halogen, hydroxyl,

unsubstituted 5-6 membered heterocyclyl or 5-6 membered heterocyclyl substituted with $C_1$-$C_3$ alkyl, cyano, and oxo, preferably $C_1$-$C_3$ alkyl, $C_1$-$C_3$ alkoxy, halogen, hydroxyl, and oxo; more preferably, each $R_{2b}$ is independently selected from methoxy and methyl.

In some embodiments, each $R_{2b}$ is selected from piperazinyl substituted with alkyl or unsubstituted piperazinyl, preferably piperazinyl substituted with $C_1$-$C_3$ alkyl.

In some embodiments, each $R_{2b}$ is independently selected from methoxy, methyl, trifluoromethyl, difluoromethyl, difluoromethoxy, N-methylpiperazinyl, and cyano.

In some embodiments, $R_3$ is selected from hydrogen, $C_1$-$C_6$ alkyl, $C_2$-$C_6$ alkenyl, $C_2$-$C_6$ alkynyl, $C_1$-$C_6$ alkoxy, sulfonyl, halogen, cycloalkyl having 3-10 ring atoms, cyano, amino, acyl, hydroxyl, heteroaryl having 5-10 ring atoms, heteroaryloxy having 5-10 ring atoms, heterocyclyl having 5-10 ring atoms, heterocyclyloxy having 5-10 ring atoms, and cycloalkyloxy having 3-10 ring atoms; preferably, $R_3$ is selected from hydrogen, $C_1$-$C_4$ alkyl, $C_2$-$C_4$ alkenyl, $C_2$-$C_4$ alkynyl, $C_1$-$C_4$ alkoxy, halogen, cycloalkyl having 3-6 ring atoms, cyano, amino, acyl, hydroxyl, heteroaryl having 5-6 ring atoms, heteroaryloxy having 5-6 ring atoms, heterocyclyl having 5-6 ring atoms, heterocyclyloxy having 5-6 ring atoms, and cycloalkyloxy having 3-6 ring atoms; more preferably, $R_3$ is selected from hydrogen, methyl, ethyl, n-propyl, isopropyl, methoxy, ethoxy, propoxy, and piperidinyloxy.

In some embodiments, $R_3$ is further substituted with one or more $R_{3a}$, wherein each $R_{3a}$ is independently selected from $C_1$-$C_6$ alkyl, $C_2$-$C_6$ alkenyl, $C_2$-$C_6$ alkynyl, $C_1$-$C_6$ alkoxy, $C_1$-$C_6$ alkylamino, di($C_1$-$C_6$ alkyl)amino, halogen, sulfonyl, cycloalkyl having 3-10 ring atoms, cyano, amino, acyl, hydroxyl, heteroaryl having 5-10 ring atoms, heteroaryloxy having 5-10 ring atoms, cycloalkyloxy having 3-10 ring atoms, heterocyclyl having 5-10 ring atoms, and heterocyclyloxy having 5-10 ring atoms; preferably, $R_{3a}$ is each independently selected from $C_1$-$C_3$ alkyl, $C_2$-$C_4$ alkenyl, $C_2$-$C_4$ alkynyl, $C_1$-$C_3$ alkoxy, $C_1$-$C_3$ alkylamino, di($C_1$-$C_3$ alkyl)amino, halogen, sulfonyl, cycloalkyl having 3-6 ring atoms, cyano, amino, acyl, hydroxyl, heteroaryl having 5-6 ring atoms, heteroaryloxy having 5-6 ring atoms, cycloalkyloxy having 3-6 ring atoms, heterocyclyl having 5-6 ring atoms, and heterocyclyloxy having 5-6 ring atoms; more preferably, $R_{3a}$ is each independently selected from methyl and dimethylamino.

In some embodiments, $R_{3a}$ is further substituted with one or more $R_{3b}$, wherein each $R_{3b}$ is independently selected from $C_1$-$C_3$ alkyl, halogen, hydroxyl, $C_1$-$C_3$ alkoxy, and amino.

In some embodiments, $R_4$ is selected from hydrogen, $C_1$-$C_6$ alkyl, $C_2$-$C_6$ alkenyl, $C_2$-$C_6$ alkynyl, $C_1$-$C_6$ alkoxy, $C_1$-$C_6$ alkylthio, sulfonyl, halogen, cycloalkyl having 3-10 ring atoms, cyano, amino, and acyl; preferably, $R_4$ is selected from hydrogen, $C_1$-$C_4$ alkyl, $C_2$-$C_4$ alkenyl, $C_2$-$C_4$ alkynyl, $C_1$-$C_4$ alkoxy, $C_1$-$C_4$ alkylthio, sulfonyl, halogen, cycloalkyl having 3-6 ring atoms, cyano, amino, and acyl; more preferably, $R_4$ is selected from hydrogen, methyl, ethyl, *n*-propyl, isopropyl, fluorine, chlorine, bromine, and cyano.

In some embodiments, $R_4$ is further substituted with one or more $R_{4a}$, wherein each $R_{4a}$ is independently selected from $C_1$-$C_6$ alkyl, halogen, amino, $C_1$-$C_6$ alkylamino, and di($C_1$-$C_6$)alkylamino; preferably, $R_{4a}$ is each independently selected from $C_1$-$C_3$ alkyl, halogen, amino, $C_1$-$C_3$ alkylamino, and di($C_1$-$C_3$)alkylamino.

In some embodiments, $R_5$ is selected from halogen, cyano, amino, hydroxyl, $C_1$-$C_6$ alkyl, $C_2$-$C_6$ alkenyl, $C_2$-$C_6$ alkynyl, $C_1$-$C_6$ alkoxy, cycloalkyl having 3-10 ring atoms, cycloalkyloxy having 3-10 ring atoms, heterocyclyl having 4-10 ring atoms, and heteroaryl having 5-10 ring atoms; preferably, $R_5$ is selected from halogen, $C_1$-$C_6$ alkyl, $C_1$-$C_6$ alkoxy, cycloalkyl having 3-6 ring atoms, cycloalkyloxy having 3-6 ring atoms, heterocyclyl having 4-6 ring atoms, and heteroaryl having 5-6 ring atoms; more preferably, $R_5$ is selected from chlorine, fluorine, bromine, iodine, cyano, ethyl, *n*-propyl, isopropyl, methyl, cyclopropyl, methoxy, and cyclopropyloxy.

In some embodiments, $R_5$ is further substituted with one or more $R_{5a}$, wherein each $R_{5a}$ is independently selected from $C_1$-$C_6$ alkyl, $C_1$-$C_6$ alkoxy, halogen, cycloalkyl having 3-10 ring atoms, cyano, amino, and hydroxyl; preferably, $R_{5a}$ is each independently selected from $C_1$-$C_6$ alkyl, halogen, cyano, and cycloalkyl having 3-6 ring atoms; more preferably, $R_{5a}$ is each independently selected from fluorine and cyano.

In some embodiments, $R_6$ is selected from hydrogen, $C_1$-$C_6$ alkyl, $C_2$-$C_6$ alkenyl, $C_2$-$C_6$ alkynyl, $C_1$-$C_6$ alkoxy, $C_1$-$C_6$ alkylthio, sulfonyl, halogen, cycloalkyl having 3-10 ring atoms, cyano, amino, and acyl; preferably, $R_6$ is selected from hydrogen, $C_1$-$C_4$ alkyl, $C_2$-$C_4$ alkenyl, $C_2$-$C_4$ alkynyl, $C_1$-$C_4$ alkoxy, $C_1$-$C_4$ alkylthio, sulfonyl, halogen, cycloalkyl having 3-6 ring atoms, cyano, amino, and acyl; more preferably, $R_6$ is selected from hydrogen, fluorine, chlorine, methyl, ethyl, n-propyl, isopropyl, cyclopropyl, methoxy, cyclopropyloxy, and cyano.

In some embodiments, $R_6$ is further substituted with one or more $R_{6a}$, wherein each $R_{6a}$ is independently selected from $C_1$-$C_6$ alkyl, halogen, amino, $C_1$-$C_6$ alkylamino, and di($C_1$-$C_6$)alkylamino; preferably, $R_{6a}$ is each independently selected from $C_1$-$C_3$ alkyl, halogen, amino, $C_1$-$C_3$ alkylamino, and di($C_1$-$C_3$)alkylamino.

In some embodiments, $R_1$ is -$X_1$-$R_7$, wherein $X_1$ is a bond or methylene.

In some embodiments, $X_1$ is substituted with one or more $X_{1a}$, wherein each $X_{1a}$ is independently selected from halogen and $C_1$-$C_3$ alkyl, preferably methyl.

In some embodiments, $R_7$ is selected from cycloalkyl having 4-10 ring atoms, aryl having 6-12 ring atoms, heteroaryl having 5-12 ring atoms, and heterocyclyl having 3-12 ring atoms; preferably, $R_7$ is selected from the following groups:

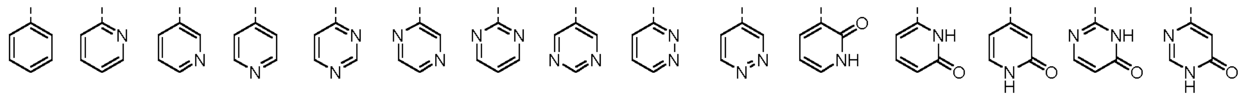

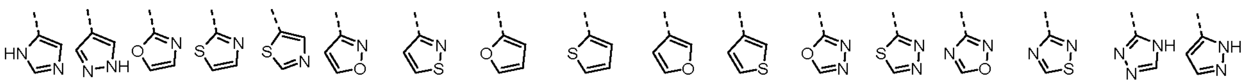

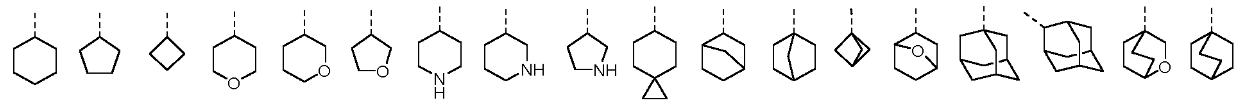

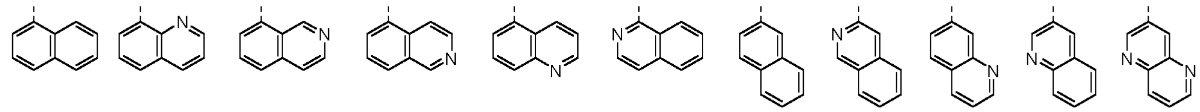

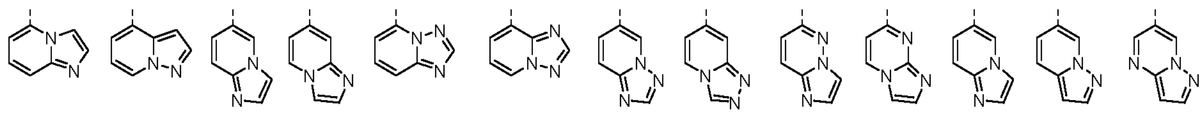

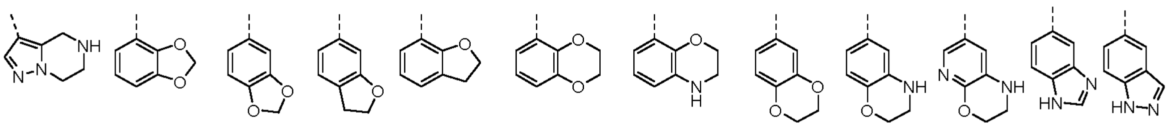

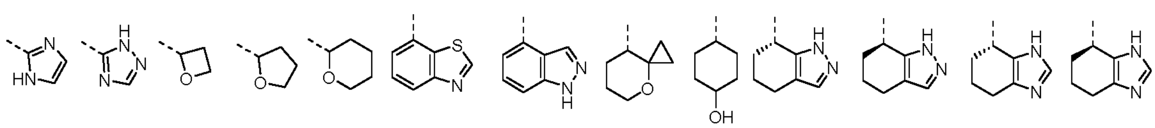

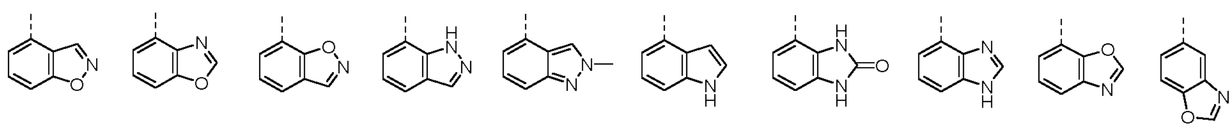

more preferably, $R_7$ is selected from the following groups:

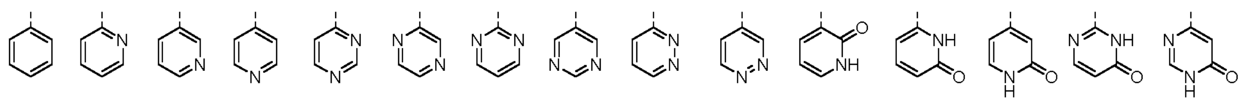

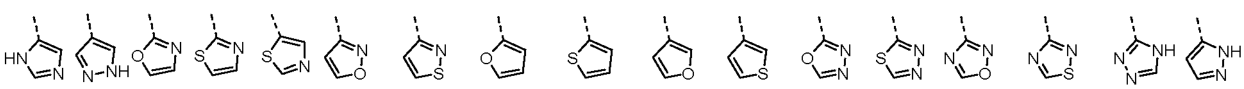

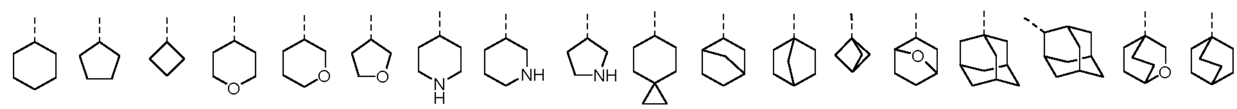

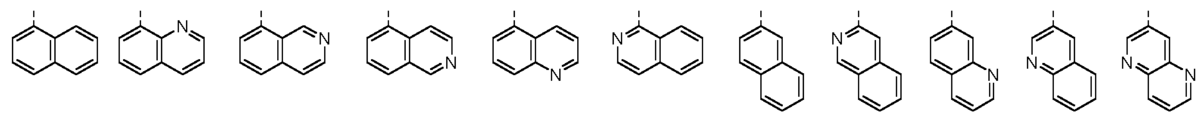

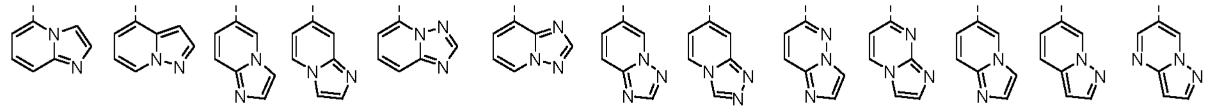

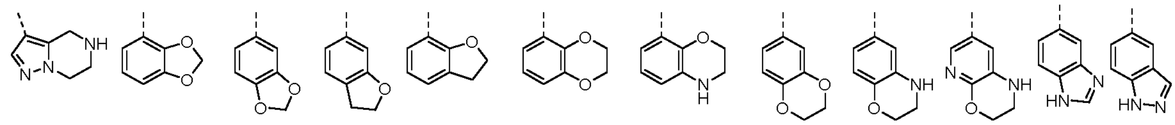

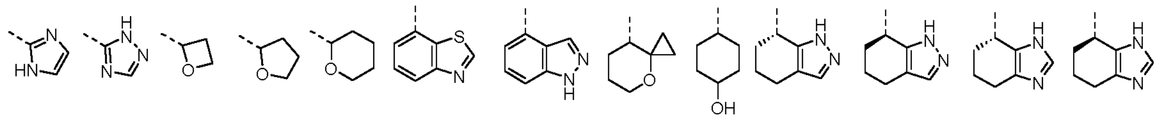

wherein the dashed line indicates where $R_7$ is connected to $X_1$.

In some embodiments, $R_7$ is optionally substituted with one or more $R_{7a}$, wherein each $R_{7a}$ is independently selected from oxo, halogen, $C_1$-$C_6$ alkyl, cycloalkyl having 3-10 ring atoms, $C_1$-$C_6$ alkoxy, cycloalkyloxy having 3-6 ring atoms, cyano, amino, hydroxyl, $C_2$-$C_6$ alkenyl, $C_2$-$C_6$ alkynyl, sulfonyl, acyl, carboxyl, heterocyclyl having 3-12 ring atoms, heteroaryl having 5-10 ring atoms, and ureido; preferably, $R_{7a}$ is each independently selected from oxo, halogen, $C_1$-$C_4$ alkyl, cycloalkyl having 3-6 ring atoms, $C_1$-$C_4$ alkoxy, cycloalkyloxy having 3-4 ring atoms, cyano, amino, hydroxyl, $C_2$-$C_4$ alkenyl, $C_2$-$C_4$ alkynyl, sulfonyl, acyl, carboxyl, heterocyclyl having 3-6 ring atoms, heteroaryl having 5-6 ring atoms, and ureido; more preferably, $R_{7a}$ is each independently selected from fluorine, chlorine, bromine, hydroxyl, cyano, methoxy, methyl, cyclopropyloxy, and cyclopropyl; most preferably, $R_{7a}$ is each independently selected from fluorine, chlorine, bromine, hydroxyl, cyano, methoxy, methyl, and cyclopropyloxy.

In some embodiments, $R_{7a}$ is further substituted with one or more $R_{7b}$, wherein $R_{7b}$ is independently selected from halogen, $C_1$-$C_6$ alkyl, $C_1$-$C_6$ alkoxy, hydroxyl, amino, $C_2$-$C_6$ alkenyl, $C_2$-$C_6$ alkynyl, $C_1$-$C_6$ alkylamino, di($C_1$-$C_6$)alkylamino, sulfonyl, heterocyclyl having 3-6 ring atoms, and heteroaryl having 5-6 ring atoms; preferably, each $R_{7b}$ is independently selected from halogen, $C_1$-$C_3$ alkyl, $C_1$-$C_3$ alkoxy, hydroxyl, amino, $C_2$-$C_4$ alkenyl, $C_2$-$C_4$ alkynyl, $C_1$-$C_3$ alkylamino, di($C_1$-$C_3$)alkylamino, sulfonyl, heterocyclyl having 3-6 ring atoms, and heteroaryl having 5-6 ring atoms; more preferably, each $R_{7b}$ is independently selected from fluorine, chlorine, and bromine.

In some embodiments, when $X_1$ is methylene, $R_7$ is not substituted or unsubstituted aryl.

In some embodiments, $R_1$ is selected from the following structures, wherein the dashed line indicates where $R_1$ is connected to the parent structure:

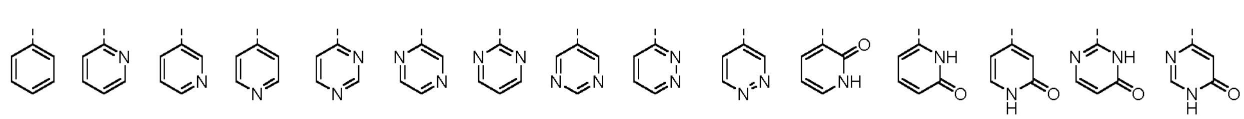

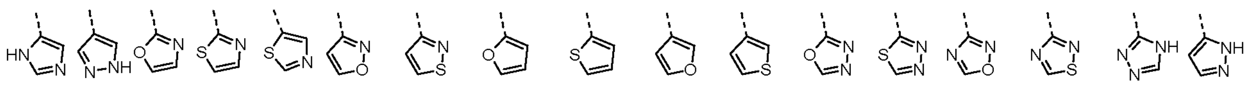

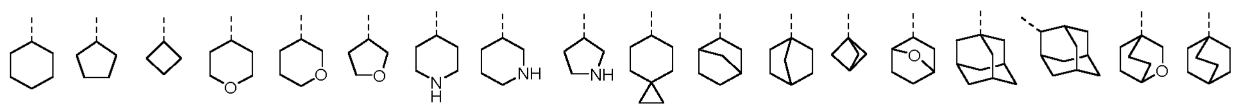

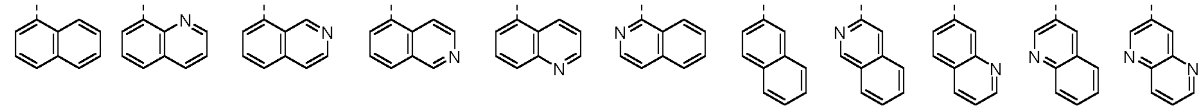

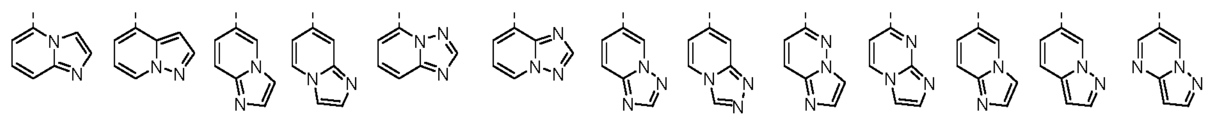

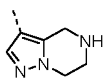 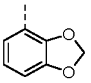 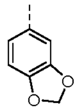 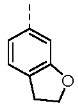 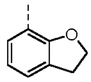 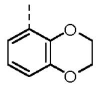 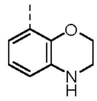 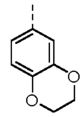 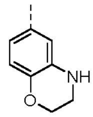 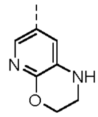 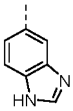 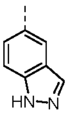

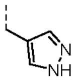 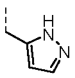 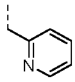 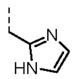 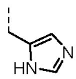 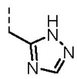 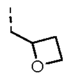 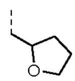 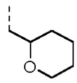 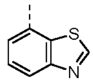 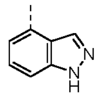 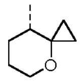 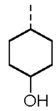

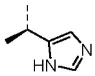 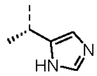 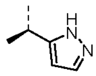 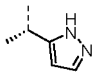 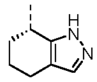 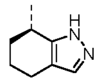 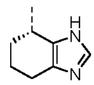 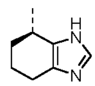

In some embodiments, w is $CR_3$, x is $CR_4$, and y is $CR_5$, wherein $R_3$, $R_4$, and $R_5$ are as defined in any one of the above embodiments.

In some embodiments, when y is $CR_5$, $R_5$ is not hydrogen.

In some embodiments, the compound of formula (I) or the stereoisomer, the tautomer, the solvate, the hydrate, the prodrug, the stable isotopic derivative, or the pharmaceutically acceptable salt thereof according to any one of the above, wherein the compound has a structure of formula (IA):

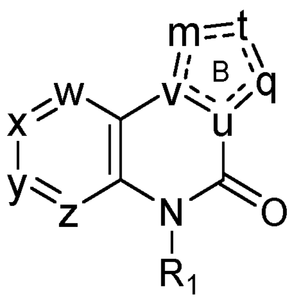

(IA) ,

wherein:

m is selected from N, O, S, $NR_9$, and $CR_9$;
t is selected from N, O, S, $NR_8$, and $CR_8$,
q is selected from N, O, S, $NR_2$, and $CR_2$;
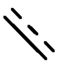 indicates that it may be a single bond or double bond;
wherein w, x, y, z, v, u, $R_1$, or $R_2$ is as defined in any one of the above embodiments.

In some embodiments, at least two of m, t, q, v, and u are substituted or unsubstituted heteroatoms; preferably, the heteroatom is N, O, or S.

In some embodiments, when $X_1$ is methylene, $R_7$ is not substituted or unsubstituted aryl.

In some embodiments, w is $CR_3$, x is $CR_4$, and y is $CR_5$, wherein $R_3$, $R_4$, and $R_5$ are as defined in any one of the above embodiments.

In some embodiments, when y is $CR_5$, $R_5$ is not hydrogen.

In some embodiments, the compound of formula (I) or the stereoisomer, the tautomer, the solvate, the hydrate, the prodrug, the stable isotopic derivative, or the pharmaceutically acceptable salt thereof according to any one of the above, wherein the compound has a structure of formula ($IB_1$), ($IB_2$), ($IB_3$), ($IB_4$), or (IBs):

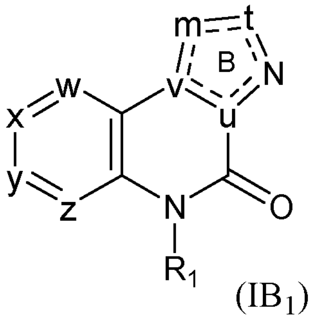
(IB$_1$),
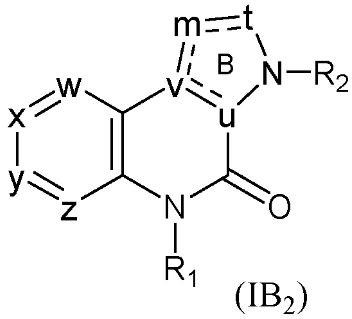
(IB$_2$),
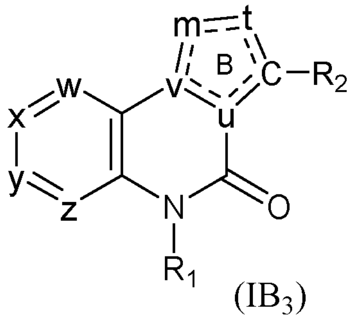
(IB$_3$),

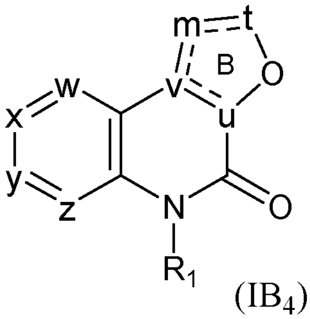
(IB$_4$),
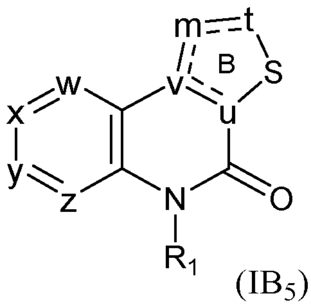
(IB$_5$);

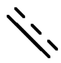

indicates that it may be a single bond or double bond;

wherein w, x, y, z, v, u, m, t, $R_1$, or $R_2$ is as defined in any one of the above embodiments.

In some embodiments, in formula (IB$_1$), (IB$_2$), (IB$_4$), or (IB$_5$), at least one of v, u, m, and t is a substituted or unsubstituted heteroatom; preferably, the heteroatom is N, O, or S.

In some embodiments, in formula (IB$_3$), at least two of v, u, m, and t are substituted or unsubstituted heteroatoms; preferably, the heteroatom is N, O, or S.

In some embodiments, when $X_1$ is methylene, $R_7$ is not substituted or unsubstituted aryl.

In some embodiments, w is $CR_3$, x is $CR_4$, and y is $CR_5$, wherein $R_3$, $R_4$, and $R_5$ are as defined in any one of the above embodiments.

In some embodiments, when y is $CR_5$, $R_5$ is not hydrogen.

In some embodiments, the compound of formula (I) or the stereoisomer, the tautomer, the solvate, the hydrate, the prodrug, the stable isotopic derivative, or the pharmaceutically acceptable salt thereof according to any one of the above, wherein the compound has a structure of formula (IC$_1$), (IC$_2$), (IC$_3$), (IC$_4$), or (ICs):

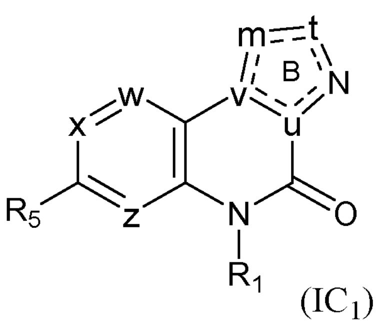
(IC$_1$),
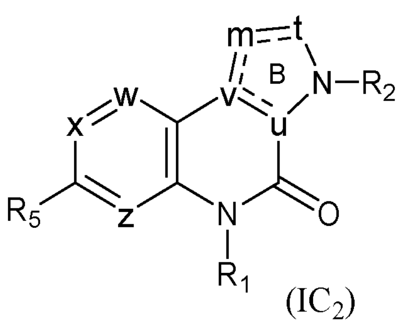
(IC$_2$),
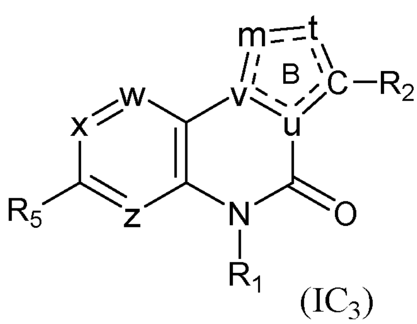
(IC$_3$),

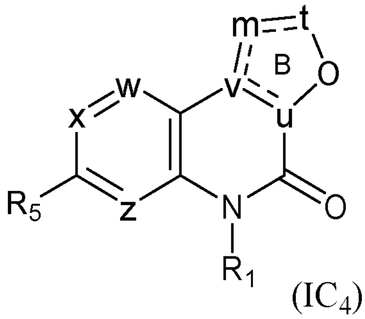
($IC_4$),
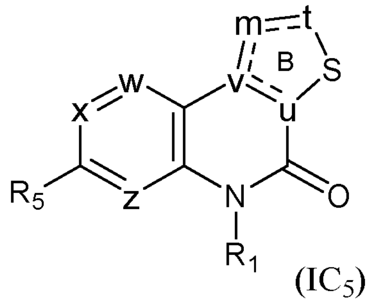
($IC_5$);

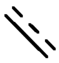 indicates that it may be a single bond or double bond;

wherein w, x, z, v, u, m, t, $R_1$, $R_2$, or $R_5$ is as defined in any one of the above embodiments.

In some embodiments, in formula ($IC_1$), ($IC_2$), ($IC_4$), or ($IC_5$), at least one of v, u, m, and t is a substituted or unsubstituted heteroatom; preferably, the heteroatom is N, O, or S.

In some embodiments, in formula ($IC_3$), at least two of v, u, m, and t are substituted or unsubstituted heteroatoms; preferably, the heteroatom is N, O, or S.

In some embodiments, when $X_1$ is methylene, $R_7$ is not substituted or unsubstituted aryl.

In some embodiments, w is $CR_3$, and x is $CR_4$, wherein $R_3$ and $R_4$ are as defined in any one of the above embodiments.

In some embodiments, the compound of formula (I) or the stereoisomer, the tautomer, the solvate, the hydrate, the prodrug, the stable isotopic derivative, or the pharmaceutically acceptable salt thereof according to any one of the above, wherein ring B of the compound is selected from the following structures:

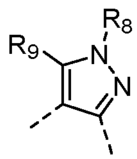 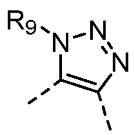 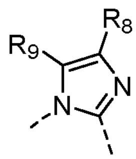 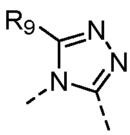 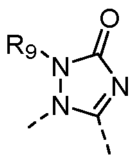 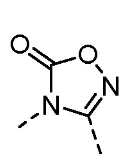 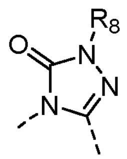 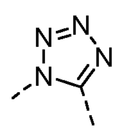

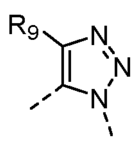 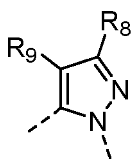 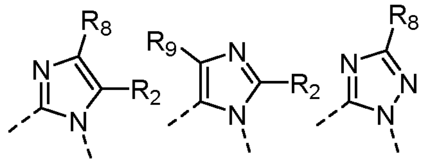 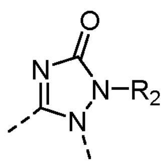 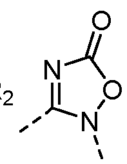 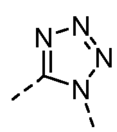

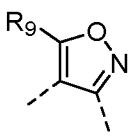 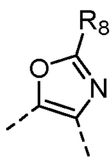 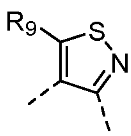 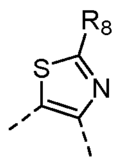 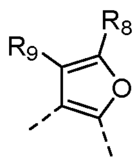 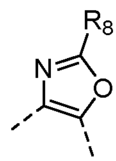 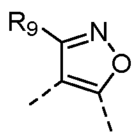 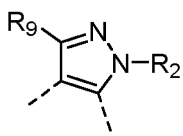

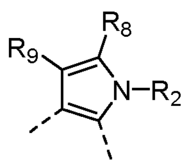 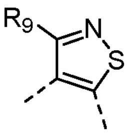 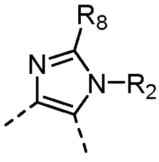 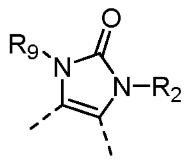 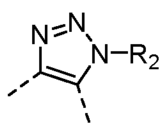 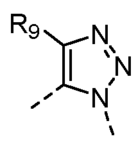 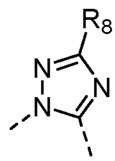 ;

wherein the dashed line 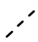 indicates where B is connected to the parent structure.

In some embodiments, the compound of formula (I) or the stereoisomer, the tautomer, the solvate, the hydrate, the prodrug, the stable isotopic derivative, or the pharmaceutically acceptable salt thereof according to any one of the above, wherein the compound is selected from the following structures:

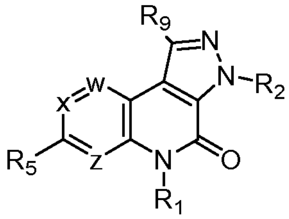
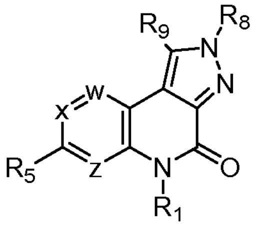
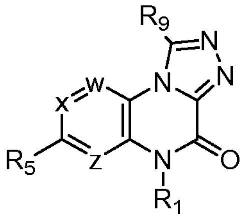

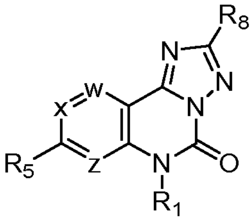
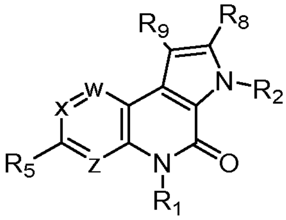
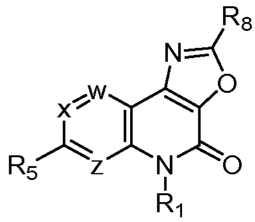
;

preferably, the compound is selected from the following structures:

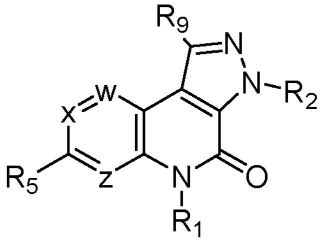
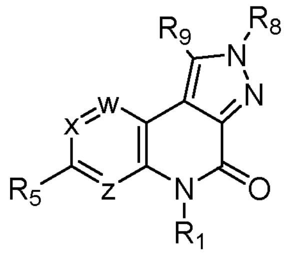
;

wherein w, x, z, $R_1$, $R_2$, or $R_5$ is as defined in any one of the above embodiments.

In some embodiments, when $X_1$ is methylene, $R_7$ is not substituted or unsubstituted aryl.

In some embodiments, w is $CR_3$, and x is $CR_4$, wherein $R_3$ and $R_4$ are as defined in any one of the above embodiments.

In some embodiments, $R_2$, $R_8$, and $R_9$ according to any one of the above are each independently selected from hydrogen, halogen, alkyl, alkoxy, alkenyl, alkynyl, cycloalkyl, cycloalkyloxy, heterocyclyl, heterocyclyloxy, aryl, heteroaryl, heteroaryloxy, cyano, amino, acyl, sulfonyl, and oxo.

In some embodiments, $R_2$ according to any one of the above is selected from hydrogen, halogen, $C_1$-$C_6$ alkyl, $C_1$-$C_6$ alkoxy, $C_2$-$C_6$ alkenyl, $C_2$-$C_6$ alkynyl, cycloalkyl having 3-10 ring atoms, cycloalkyloxy having 3-10 ring atoms, heterocyclyl having 4-10 ring atoms, heterocyclyloxy having 4-10 ring atoms, aryl having 6-10 ring atoms, heteroaryl having 5-10 ring atoms, heteroaryloxy having 5-10 ring atoms, cyano, amino, acyl, sulfonyl, and oxo; preferably, $R_2$ is selected from hydrogen, halogen, $C_1$-$C_4$ alkyl, $C_1$-$C_4$ alkoxy, $C_2$-$C_4$ alkenyl, $C_2$-$C_4$ alkynyl, cycloalkyl having 3-6 ring atoms, cycloalkyloxy having 3-6 ring atoms, heterocyclyl having 5-6 ring atoms, heterocyclyloxy having 5-6 ring atoms, aryl having 6-10 ring atoms, heteroaryl having 5-6 ring atoms, heteroaryloxy having 5-6 ring atoms, cyano, amino, acyl, sulfonyl, and oxo; more preferably, $R_2$ is selected from hydrogen, methyl, ethyl, n-propyl, isopropyl, cyano, methoxy, and oxo.

In some embodiments, $R_8$ and $R_9$ according to any one of the above are each independently selected from hydrogen, halogen, $C_1$-$C_6$ alkyl, $C_1$-$C_6$ alkoxy, $C_2$-$C_6$ alkenyl, $C_2$-$C_6$ alkynyl, cycloalkyl having 3-10 ring atoms, cycloalkyloxy having 3-10 ring atoms, heterocyclyl having 4-10 ring atoms, heterocyclyloxy having 4-10 ring atoms, aryl having 6-10 ring atoms, heteroaryl having 5-10 ring atoms, heteroaryloxy having 5-10 ring atoms, cyano, amino, acyl, sulfonyl, and oxo; preferably, $R_8$ and $R_9$ are each independently selected from hydrogen, halogen, $C_1$-$C_4$ alkyl, $C_1$-$C_4$ alkoxy, $C_2$-$C_4$ alkenyl, $C_2$-$C_4$ alkynyl, cycloalkyl having 3-6 ring atoms, cycloalkyloxy having 3-6 ring atoms, heterocyclyl having 5-6 ring atoms, heterocyclyloxy having 5-6 ring atoms, aryl having 6 ring atoms, heteroaryl having 5-6 ring atoms, heteroaryloxy having 5-6 ring atoms, cyano, amino, acyl, sulfonyl, and oxo; more preferably, $R_8$ and $R_9$ are each independently selected from hydrogen, methyl, ethyl, n-propyl, isopropyl, cyano, methoxy, oxo, cyclopropyl, tetrahydropyrrolyl, tetrahydrofuranyl, phenyl, and propenyl.

In some embodiments, adjacent $R_8$ and $R_2$ form a 3-7 membered carbocyclic ring, heterocyclic ring, aromatic ring, or heteroaromatic ring, preferably 5-6 membered carbocyclic ring, heterocyclic ring, or heteroaromatic ring, and more preferably 5-6 membered heterocyclic ring or heteroaromatic ring.

In some embodiments, adjacent $R_8$ and $R_9$ form a 3-7 membered carbocyclic ring, heterocyclic ring, aromatic ring, or heteroaromatic ring, preferably 5-6 membered carbocyclic ring, heterocyclic ring, or heteroaromatic ring, and more preferably 5-6 membered heterocyclic ring or heteroaromatic ring, for example

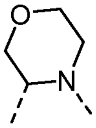 or 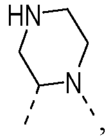 ,

wherein the dashed line 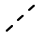 indicates where the ring is connected to the parent structure.

In some embodiments, $R_2$ is further substituted with one or more $R_{2a}$.

In some embodiments, $R_8$ is further substituted with one or more $R_{8a}$.

In some embodiments, $R_9$ is further substituted with one or more $R_{9a}$.

In some embodiments, the ring formed by adjacent $R_8$ and $R_2$ is further substituted with one or more $R_{2a}$ or $R_{8a}$.

In some embodiments, the ring formed by adjacent $R_8$ and $R_9$ is further substituted with one or more $R_{9a}$ or $R_{8a}$.

In some embodiments, $R_{2a}$, $R_{9a}$, or $R_{8a}$ according to any one of the above is each independently selected from halogen, alkyl, heterocyclyl, heterocyclyloxy, amino, heteroaryl, acyl, cycloalkyl, aryl, alkylamino, dialkylamino, hydroxyl, sulfonyl, alkoxy, cyano, alkenyl, alkynyl, and oxo.

In some embodiments, each $R_{2a}$ is independently selected from halogen, $C_1$-$C_6$ alkyl, heterocyclyl having 4-10 ring atoms, heterocyclyloxy having 4-10 ring atoms, amino, heteroaryl having 5-10 ring atoms, cycloalkyl having 3-10 ring atoms, aryl having 6-10 ring atoms, $C_1$-$C_6$ alkylamino, di($C_1$-$C_6$)alkylamino, hydroxyl, sulfonyl, $C_1$-$C_6$ alkoxy, cyano, $C_2$-$C_6$ alkenyl, $C_2$-$C_6$ alkynyl, and oxo; preferably, each $R_{2a}$ is independently selected from halogen, $C_1$-$C_4$ alkyl, heterocyclyl having 5-6 ring atoms, heterocyclyloxy having 5-6 ring atoms, amino, heteroaryl having 5-6 ring atoms, cycloalkyl having 3-6 ring atoms, aryl having 6-10 ring atoms, $C_1$-$C_3$ alkylamino, di($C_1$-$C_3$)alkylamino, hydroxyl, sulfonyl, $C_1$-$C_4$ alkoxy, cyano, $C_2$-$C_4$ alkenyl, $C_2$-$C_4$ alkynyl, and oxo; more preferably, each $R_{2a}$ is independently selected from methyl, chlorine, fluorine, phenyl, cyclopropyl, oxo, amino, methylamino, dimethylamino, methoxy, hydroxyl, pyridinyl, pyrazolyl, and pyrimidinyl; most preferably, each $R_{2a}$ is independently selected from methyl, chlorine, fluorine, phenyl, cyclopropyl, oxo, amino, methylamino, dimethylamino, methoxy, hydroxyl, pyridinyl, and pyrazolyl.

In some embodiments, $R_{8a}$ or $R_{9a}$ is each independently selected from halogen, $C_1$-$C_6$ alkyl, heterocyclyl having 4-10 ring atoms, heterocyclyloxy having 4-10 ring atoms, amino, heteroaryl having 5-10 ring atoms, cycloalkyl having 3-10 ring atoms, aryl having 6-10 ring atoms, $C_1$-$C_6$ alkylamino, di($C_1$-$C_6$)alkylamino, hydroxyl, sulfonyl, $C_1$-$C_6$ alkoxy, cyano, $C_2$-$C_6$ alkenyl, $C_2$-$C_6$ alkynyl, and oxo; preferably, each $R_{8a}$ or each $R_{9a}$ is independently selected from halogen, $C_1$-$C_4$ alkyl, heterocyclyl having 5-6 ring atoms, heterocyclyloxy having 5-6 ring atoms, amino, heteroaryl having 5-6 ring atoms, cycloalkyl having 3-6 ring atoms, aryl having 6 ring atoms, $C_1$-$C_3$ alkylamino, di($C_1$-$C_3$)alkylamino, hydroxyl, sulfonyl, $C_1$-$C_4$ alkoxy, cyano, $C_2$-$C_4$ alkenyl, $C_2$-$C_4$ alkynyl, and oxo; more preferably, each $R_{8a}$ or each $R_{9a}$ is independently selected from methyl, chlorine, fluorine, bromine, phenyl, cyclopropyl, oxo, amino, methylamino, dimethylamino, methoxy, hydroxyl, pyridinyl, pyrazolyl, sulfonyl, morpholinyl, tetrahydrofuranyl, isoxazolyl, and pyrimidinyl; most preferably, each $R_{8a}$ or each $R_{9a}$ is independently selected from methyl, chlorine, fluorine, bromine, phenyl, cyclopropyl, oxo, amino, methylamino, dimethylamino, methoxy, hydroxyl, pyridinyl, pyrazolyl, sulfonyl, morpholinyl, tetrahydrofuranyl, and isoxazolyl.

In some embodiments, $R_{2a}$ is further substituted with one or more $R_{2b}$.

In some embodiments, $R_{8a}$ is further substituted with one or more $R_{8b}$.

In some embodiments, $R_{9a}$ is further substituted with one or more $R_{9b}$.

In some embodiments, each $R_{2b}$, each $R_{8b}$, or each $R_{9b}$ is independently selected from alkyl substituted with 0-3 halogens, alkoxy substituted with 0-3 halogens, halogen, hydroxyl, unsubstituted heterocyclyl or heterocyclyl substituted with alkyl, cyano, and oxo, preferably selected from alkyl, alkoxy, halogen, hydroxyl, and oxo.

In some embodiments, each $R_{2b}$ is independently selected from $C_1$-$C_3$ alkyl substituted with 0-3 halogens, $C_1$-$C_3$ alkoxy substituted with 0-3 halogens, halogen, hydroxyl, unsubstituted 5-6 membered heterocyclyl or 5-6 membered heterocyclyl substituted with $C_1$-$C_3$ alkyl, cyano, and oxo, preferably $C_1$-$C_3$ alkyl, $C_1$-$C_3$ alkoxy, halogen, hydroxyl, and oxo; preferably, each $R_{2b}$ is independently selected from methoxy and methyl.

In some embodiments, $R_{8b}$ or $R_{9b}$ is each independently selected from $C_1$-$C_3$ alkyl substituted with 0-3 halogens, $C_1$-$C_3$ alkoxy substituted with 0-3 halogens, halogen, hydroxyl, unsubstituted 5-6 membered heterocyclyl or 5-6 membered heterocyclyl substituted with $C_1$-$C_3$ alkyl, cyano, and oxo, preferably $C_1$-$C_3$ alkyl, $C_1$-$C_3$ alkoxy, halogen, hydroxyl, and oxo; preferably, $R_{8b}$ or $R_{9b}$ is each independently selected from methoxy and methyl.

In some embodiments, $R_{8b}$ or $R_{9b}$ is each independently selected from methoxy, methyl, trifluoromethyl, difluoromethyl, difluoromethoxy, N-methylpiperazinyl, and cyano.

It should be understood that the compound of formula (I) or the stereoisomer, the tautomer, the solvate, the

hydrate, the prodrug, the stable isotopic derivative, or the pharmaceutically acceptable salt thereof described herein includes, for example, formula (IA), ($IB_1$), ($IB_2$), ($IB_3$), ($IB_4$), ($IB_5$), ($IC_1$), ($IC_2$), ($IC_3$), ($IC_4$), or ($IC_5$), or includes the structure of the following formula:

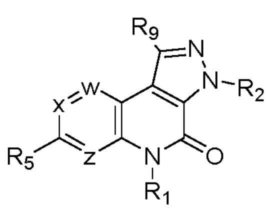
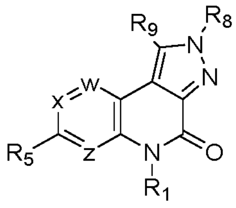
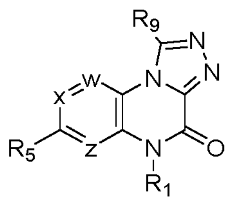
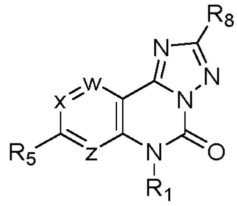
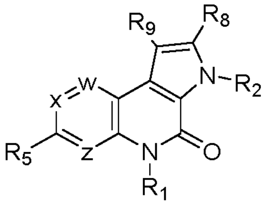
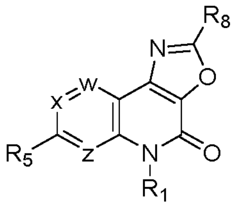
,

wherein each substituent definition thereof may optionally be derived from any one of the above embodiments or a combination of the embodiments.

For example, in one embodiment, the compound or the stereoisomer, the tautomer, the solvate, the hydrate, the prodrug, the stable isotopic derivative, or the pharmaceutically acceptable salt thereof described herein, wherein:

$R_3$ is selected from hydrogen, $C_1$-$C_6$ alkyl, $C_2$-$C_6$ alkenyl, $C_2$-$C_6$ alkynyl, $C_1$-$C_6$ alkoxy, sulfonyl, halogen, cycloalkyl having 3-10 ring atoms, cyano, amino, acyl, hydroxyl, heteroaryl having 5-10 ring atoms, heteroaryloxy having 5-10 ring atoms, heterocyclyl having 5-10 ring atoms, heterocyclyloxy having 5-10 ring atoms, and cycloalkyloxy having 3-10 ring atoms;
optionally, $R_3$ is further substituted with one or more $R_{3a}$, wherein each $R_{3a}$ is independently selected from $C_1$-$C_6$ alkyl, $C_2$-$C_6$ alkenyl, $C_2$-$C_6$ alkynyl, $C_1$-$C_6$ alkoxy, $C_1$-$C_6$ alkylamino, di($C_1$-$C_6$ alkyl)amino, halogen, sulfonyl, cycloalkyl having 3-10 ring atoms, cyano, amino, acyl, hydroxyl, heteroaryl having 5-10 ring atoms, heteroaryloxy having 5-10 ring atoms, cycloalkyloxy having 3-10 ring atoms, heterocyclyl having 5-10 ring atoms, and heterocyclyloxy having 5-10 ring atoms;
optionally, $R_{3a}$ is further substituted with one or more $R_{3b}$, wherein each $R_{3b}$ is independently selected from $C_1$-$C_3$ alkyl, halogen, hydroxyl, $C_1$-$C_3$ alkoxy, and amino;
$R_4$ is selected from hydrogen, $C_1$-$C_6$ alkyl, $C_2$-$C_6$ alkenyl, $C_2$-$C_6$ alkynyl, $C_1$-$C_6$ alkoxy, $C_1$-$C_6$ alkylthio, sulfonyl, halogen, cycloalkyl having 3-10 ring atoms, cyano, amino, and acyl;
optionally, $R_4$ is further substituted with one or more $R_{4a}$, wherein each $R_{4a}$ is independently selected from $C_1$-$C_6$ alkyl, halogen, amino, $C_1$-$C_6$ alkylamino, and di($C_1$-$C_6$)alkylamino;
$R_5$ is selected from halogen, cyano, amino, hydroxyl, $C_1$-$C_6$ alkyl, $C_2$-$C_6$ alkenyl, $C_2$-$C_6$ alkynyl, $C_1$-$C_6$ alkoxy, cycloalkyl having 3-10 ring atoms, cycloalkyloxy having 3-10 ring atoms, heterocyclyl having 4-10 ring atoms, and heteroaryl having 5-10 ring atoms;
optionally, $R_5$ is further substituted with one or more $R_{5a}$, wherein each $R_{5a}$ is independently selected from $C_1$-$C_6$ alkyl, $C_1$-$C_6$ alkoxy, halogen, cycloalkyl having 3-10 ring atoms, cyano, amino, and hydroxyl;
$R_6$ is selected from hydrogen, $C_1$-$C_6$ alkyl, $C_2$-$C_6$ alkenyl, $C_2$-$C_6$ alkynyl, $C_1$-$C_6$ alkoxy, $C_1$-$C_6$ alkylthio, sulfonyl, halogen, cycloalkyl having 3-10 ring atoms, cyano, amino, and acyl;
optionally, $R_6$ is further substituted with one or more $R_{6a}$, wherein each $R_{6a}$ is independently selected from $C_1$-$C_6$ alkyl, halogen, amino, $C_1$-$C_6$ alkylamino, and di($C_1$-$C_6$)alkylamino;
$R_1$ is -$X_1$-$R_7$, wherein $X_1$ is a bond or methylene;
optionally, $X_1$ is substituted with one or more $X_{1a}$, wherein each $X_{1a}$ is independently selected from halogen and $C_1$-$C_3$ alkyl;
$R_7$ is selected from cycloalkyl having 4-10 ring atoms, aryl having 6-12 ring atoms, heteroaryl having 5-12 ring atoms, and heterocyclyl having 3-12 ring atoms;
$R_7$ is optionally substituted with one or more $R_{7a}$, wherein each $R_{7a}$ is independently selected from oxo, halogen, $C_1$-$C_6$ alkyl, cycloalkyl having 3-10 ring atoms, $C_1$-$C_6$ alkoxy, cycloalkyloxy having 3-6 ring atoms, cyano, amino, hydroxyl, $C_2$-$C_6$ alkenyl, $C_2$-$C_6$ alkynyl, sulfonyl, acyl, carboxyl, heterocyclyl having 3-12 ring atoms, heteroaryl having 5-10 ring atoms, and ureido;

optionally, $R_{7a}$ is further substituted with one or more $R_{7b}$, wherein $R_{7b}$ is independently selected from halogen, $C_1$-$C_6$ alkyl, $C_1$-$C_6$ alkoxy, hydroxyl, amino, $C_2$-$C_6$ alkenyl, $C_2$-$C_6$ alkynyl, $C_1$-$C_6$ alkylamino, di($C_1$-$C_6$)alkylamino, sulfonyl, heterocyclyl having 3-6 ring atoms, and heteroaryl having 5-6 ring atoms;

when $X_1$ is methylene, $R_7$ is not substituted or unsubstituted aryl;

$R_2$ is selected from hydrogen, halogen, $C_1$-$C_6$ alkyl, $C_1$-$C_6$ alkoxy, $C_2$-$C_6$ alkenyl, $C_2$-$C_6$ alkynyl, cycloalkyl having 3-10 ring atoms, cycloalkyloxy having 3-10 ring atoms, heterocyclyl having 4-10 ring atoms, heterocyclyloxy having 4-10 ring atoms, aryl having 6-10 ring atoms, heteroaryl having 5-10 ring atoms, heteroaryloxy having 5-10 ring atoms, cyano, amino, acyl, sulfonyl, and oxo;

optionally, $R_2$ is further substituted with one or more $R_{2a}$, wherein each $R_{2a}$ is independently selected from halogen, $C_1$-$C_6$ alkyl, heterocyclyl having 4-10 ring atoms, heterocyclyloxy having 4-10 ring atoms, amino, heteroaryl having 5-10 ring atoms, cycloalkyl having 3-10 ring atoms, aryl having 6-10 ring atoms, $C_1$-$C_6$ alkylamino, di($C_1$-$C_6$) alkylamino, hydroxyl, sulfonyl, $C_1$-$C_6$ alkoxy, cyano, $C_2$-$C_6$ alkenyl, $C_2$-$C_6$ alkynyl, and oxo;

optionally, $R_{2a}$ is further substituted with one or more $R_{2b}$, wherein each $R_{2b}$ is independently selected from $C_1$-$C_3$ alkyl substituted with 0-3 halogens, $C_1$-$C_3$ alkoxy substituted with 0-3 halogens, halogen, hydroxyl, unsubstituted 5-6 membered heterocyclyl or 5-6 membered heterocyclyl substituted with $C_1$-$C_3$ alkyl, cyano, and oxo, preferably $C_1$-$C_3$ alkyl, $C_1$-$C_3$ alkoxy, halogen, hydroxyl, and oxo;

$R_8$ and $R_9$ are each independently selected from hydrogen, halogen, $C_1$-$C_6$ alkyl, $C_1$-$C_6$ alkoxy, $C_2$-$C_6$ alkenyl, $C_2$-$C_6$ alkynyl, cycloalkyl having 3-10 ring atoms, cycloalkyloxy having 3-10 ring atoms, heterocyclyl having 4-10 ring atoms, heterocyclyloxy having 4-10 ring atoms, aryl having 6-10 ring atoms, heteroaryl having 5-10 ring atoms, heteroaryloxy having 5-10 ring atoms, cyano, amino, acyl, sulfonyl, and oxo, or adjacent $R_8$ and $R_9$ form a 5-6 membered carbocyclic ring, heterocyclic ring, or heteroaromatic ring;

optionally, $R_8$ is further substituted with one or more $R_{8a}$; optionally, $R_9$ is further substituted with one or more $R_{9a}$; optionally, the carbocyclic ring, heterocyclic ring, or heteroaromatic ring formed by adjacent $R_8$ and $R_9$ is further substituted with one or more $R_{8a}$ or $R_{9a}$;

$R_{8a}$ or $R_{9a}$ is each independently selected from halogen, $C_1$-$C_6$ alkyl, heterocyclyl having 4-10 ring atoms, heterocyclyloxy having 4-10 ring atoms, amino, heteroaryl having 5-10 ring atoms, cycloalkyl having 3-10 ring atoms, aryl having 6-10 ring atoms, $C_1$-$C_6$ alkylamino, di($C_1$-$C_6$)alkylamino, hydroxyl, sulfonyl, $C_1$-$C_6$ alkoxy, cyano, $C_2$-$C_6$ alkenyl, $C_2$-$C_6$ alkynyl, and oxo;

optionally, $R_{8a}$ is further substituted with one or more $R_{8b}$; optionally, $R_{9a}$ is further substituted with one or more $R_{9b}$;

$R_{8b}$ or $R_{9b}$ is each independently selected from $C_1$-$C_3$ alkyl substituted with 0-3 halogens, $C_1$-$C_3$ alkoxy substituted with 0-3 halogens, halogen, hydroxyl, unsubstituted 5-6 membered heterocyclyl or 5-6 membered heterocyclyl substituted with $C_1$-$C_3$ alkyl, cyano, and oxo, preferably $C_1$-$C_3$ alkyl, $C_1$-$C_3$ alkoxy, halogen, hydroxyl, and oxo.

In one embodiment, the compound or the stereoisomer, the tautomer, the solvate, the hydrate, the prodrug, the stable isotopic derivative, or the pharmaceutically acceptable salt thereof described herein, wherein:

$R_3$ is selected from hydrogen, $C_1$-$C_4$ alkyl, $C_2$-$C_4$ alkenyl, $C_2$-$C_4$ alkynyl, $C_1$-$C_4$ alkoxy, halogen, cycloalkyl having 3-6 ring atoms, cyano, amino, acyl, hydroxyl, heteroaryl having 5-6 ring atoms, heteroaryloxy having 5-6 ring atoms, heterocyclyl having 5-6 ring atoms, heterocyclyloxy having 5-6 ring atoms, and cycloalkyloxy having 3-6 ring atoms;

optionally, $R_3$ is further substituted with one or more $R_{3a}$, wherein each $R_{3a}$ is independently selected from $C_1$-$C_3$ alkyl, $C_2$-$C_4$ alkenyl, $C_2$-$C_4$ alkynyl, $C_1$-$C_3$ alkoxy, $C_1$-$C_3$ alkylamino, di($C_1$-$C_3$ alkyl)amino, halogen, sulfonyl, cycloalkyl having 3-6 ring atoms, cyano, amino, acyl, hydroxyl, heteroaryl having 5-6 ring atoms, heteroaryloxy having 5-6 ring atoms, cycloalkyloxy having 3-6 ring atoms, heterocyclyl having 5-6 ring atoms, and heterocyclyloxy having 5-6 ring atoms;

optionally, $R_{3a}$ is further substituted with one or more $R_{3b}$, wherein each $R_{3b}$ is independently selected from $C_1$-$C_3$ alkyl, halogen, hydroxyl, $C_1$-$C_3$ alkoxy, and amino;

$R_4$ is selected from hydrogen, $C_1$-$C_4$ alkyl, $C_2$-$C_4$ alkenyl, $C_2$-$C_4$ alkynyl, $C_1$-$C_4$ alkoxy, $C_1$-$C_4$ alkylthio, sulfonyl, halogen, cycloalkyl having 3-6 ring atoms, cyano, amino, and acyl;

optionally, $R_4$ is further substituted with one or more $R_{4a}$, wherein each $R_{4a}$ is independently selected from $C_1$-$C_3$ alkyl, halogen, amino, $C_1$-$C_3$ alkylamino, and di($C_1$-$C_3$)alkylamino;

$R_5$ is selected from halogen, $C_1$-$C_6$ alkyl, $C_1$-$C_6$ alkoxy, cycloalkyl having 3-6 ring atoms, cycloalkyloxy having 3-6 ring atoms, heterocyclyl having 4-6 ring atoms, and heteroaryl having 5-6 ring atoms;

optionally, $R_5$ is further substituted with one or more $R_{5a}$, wherein each $R_{5a}$ is independently selected from $C_1$-$C_6$ alkyl, halogen, cyano, and cycloalkyl having 3-6 ring atoms;

$R_6$ is selected from hydrogen, $C_1$-$C_4$ alkyl, $C_2$-$C_4$ alkenyl, $C_2$-$C_4$ alkynyl, $C_1$-$C_4$ alkoxy, $C_1$-$C_4$ alkylthio, sulfonyl, halogen, cycloalkyl having 3-6 ring atoms, cyano, amino, and acyl;

optionally, $R_6$ is further substituted with one or more $R_{6a}$, wherein each $R_{6a}$ is independently selected from $C_1$-$C_3$ alkyl, halogen, amino, $C_1$-$C_3$ alkylamino, and di($C_1$-$C_3$)alkylamino;

$R_1$ is $-X_1-R_7$, wherein $X_1$ is a bond or methylene;
optionally, $X_1$ is substituted with one or more $X_{1a}$, wherein $X_{1a}$ is methyl;
$R_7$ is selected from the following groups:

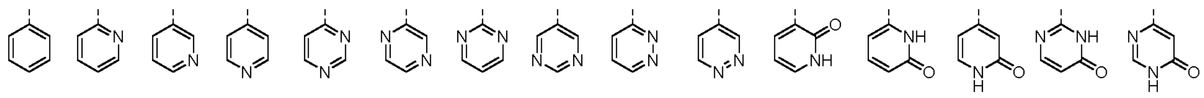

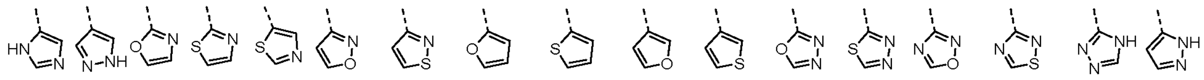

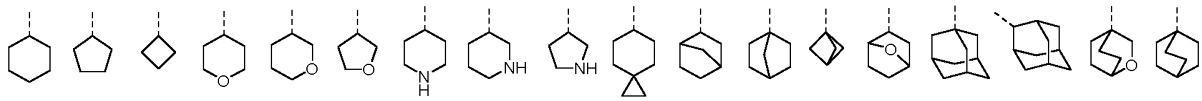

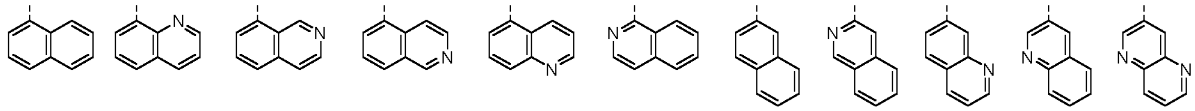

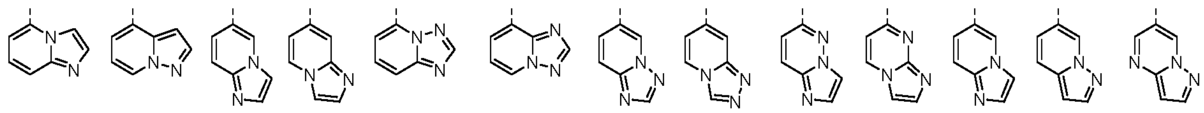

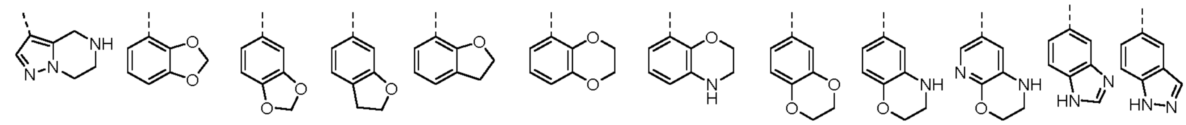

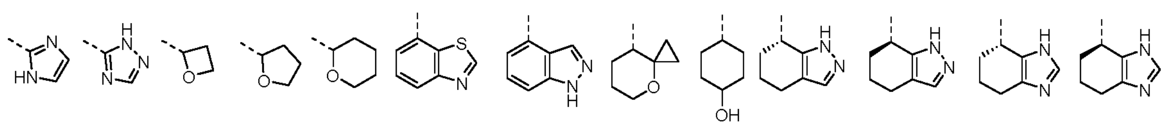

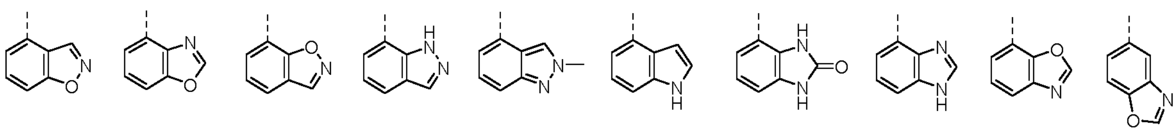

preferably, $R_7$ is selected from the following groups:

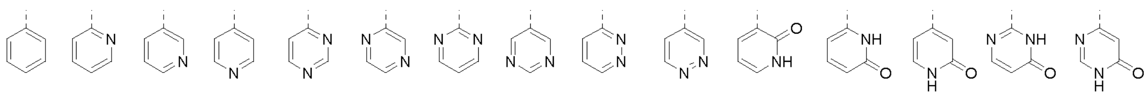

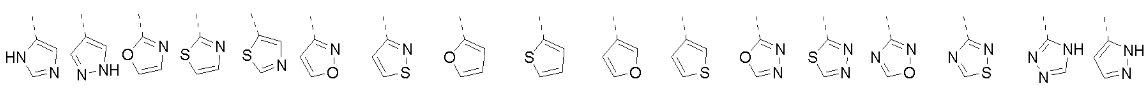

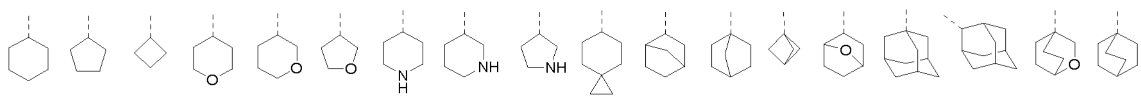

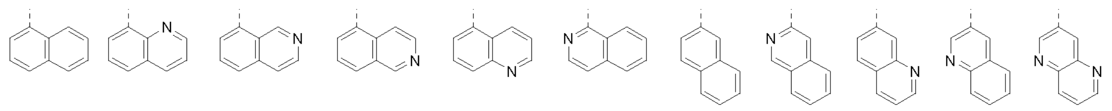

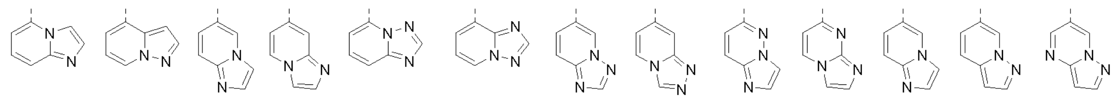

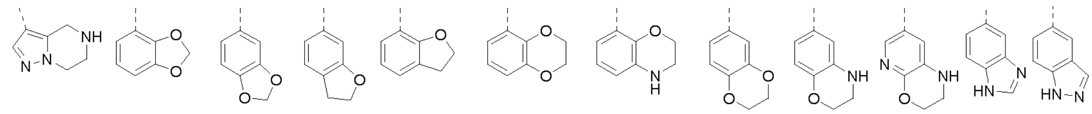

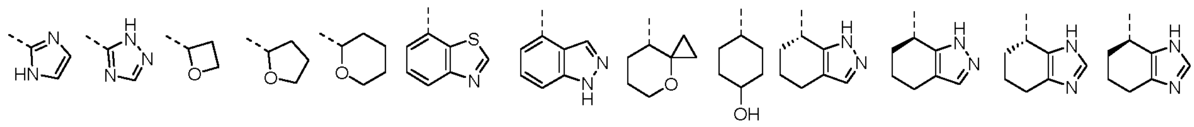
,

wherein the dashed line 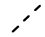 indicates where $R_7$ is connected to $X_1$;

$R_7$ is optionally substituted with one or more $R_{7a}$, wherein each $R_{7a}$ is independently selected from oxo, halogen, $C_1$-$C_4$ alkyl, cycloalkyl having 3-6 ring atoms, $C_1$-$C_4$ alkoxy, cycloalkyloxy having 3-4 ring atoms, cyano, amino, hydroxyl, $C_2$-$C_4$ alkenyl, $C_2$-$C_4$ alkynyl, sulfonyl, acyl, carboxyl, heterocyclyl having 3-6 ring atoms, heteroaryl having 5-6 ring atoms, and ureido;

optionally, $R_{7a}$ is further substituted with one or more $R_{7b}$, wherein $R_{7b}$ is each independently selected from halogen, $C_1$-$C_3$ alkyl, $C_1$-$C_3$ alkoxy, hydroxyl, amino, $C_2$-$C_4$ alkenyl, $C_2$-$C_4$ alkynyl, $C_1$-$C_3$ alkylamino, di($C_1$-$C_3$)alkylamino, sulfonyl, heterocyclyl having 3-6 ring atoms, and heteroaryl having 5-6 ring atoms;

when $X_1$ is methylene, $R_7$ is not substituted or unsubstituted aryl;

$R_2$ is selected from hydrogen, halogen, $C_1$-$C_4$ alkyl, $C_1$-$C_4$ alkoxy, $C_2$-$C_4$ alkenyl, $C_2$-$C_4$ alkynyl, cycloalkyl having 3-6 ring atoms, cycloalkyloxy having 3-6 ring atoms, heterocyclyl having 5-6 ring atoms, heterocyclyloxy having 5-6 ring atoms, aryl having 6-10 ring atoms, heteroaryl having 5-6 ring atoms, heteroaryloxy having 5-6 ring atoms, cyano, amino, acyl, sulfonyl, and oxo;

optionally, $R_2$ is further substituted with one or more $R_{2a}$, wherein each $R_{2a}$ is independently selected from halogen, $C_1$-$C_4$ alkyl, heterocyclyl having 5-6 ring atoms, heterocyclyloxy having 5-6 ring atoms, amino, heteroaryl having 5-6 ring atoms, cycloalkyl having 3-6 ring atoms, aryl having 6-10 ring atoms, $C_1$-$C_3$ alkylamino, di($C_1$-$C_3$)alkylamino, hydroxyl, sulfonyl, $C_1$-$C_4$ alkoxy, cyano, $C_2$-$C_4$ alkenyl, $C_2$-$C_4$ alkynyl, and oxo;

optionally, $R_{2a}$ is further substituted with one or more $R_{2b}$, wherein each $R_{2b}$ is independently selected from methoxy, methyl, trifluoromethyl, difluoromethyl, difluoromethoxy, N-methylpiperazinyl, and cyano, preferably methoxy and methyl;

$R_8$ and $R_9$ are each independently selected from hydrogen, halogen, $C_1$-$C_4$ alkyl, $C_1$-$C_4$ alkoxy, $C_2$-$C_4$ alkenyl, $C_2$-$C_4$ alkynyl, cycloalkyl having 3-6 ring atoms, cycloalkyloxy having 3-6 ring atoms, heterocyclyl having 5-6 ring atoms, heterocyclyloxy having 5-6 ring atoms, aryl having 6 ring atoms, heteroaryl having 5-6 ring atoms, heteroaryloxy having 5-6 ring atoms, cyano, amino, acyl, sulfonyl, and oxo, or adjacent $R_8$ and $R_9$ form a 5-6 membered heterocyclic ring or heteroaromatic ring;

optionally, $R_8$ is further substituted with one or more $R_{8a}$; optionally, $R_9$ is further substituted with one or more $R_{9a}$; optionally, the heterocyclic ring or heteroaromatic ring formed by adjacent $R_8$ and $R_9$ is further substituted with one or more $R_{8a}$ or $R_{9a}$;

$R_{8a}$ or $R_{9a}$ is each independently selected from halogen, $C_1$-$C_4$ alkyl, heterocyclyl having 5-6 ring atoms, heterocyclyloxy having 5-6 ring atoms, amino, heteroaryl having 5-6 ring atoms, cycloalkyl having 3-6 ring atoms, aryl having 6 ring atoms, $C_1$-$C_3$ alkylamino, di($C_1$-$C_3$)alkylamino, hydroxyl, sulfonyl, $C_1$-$C_4$ alkoxy, cyano, $C_2$-$C_4$ alkenyl, $C_2$-$C_4$ alkynyl, and oxo;

optionally, $R_{8a}$ is further substituted with one or more $R_{8b}$; optionally, $R_{9a}$ is further substituted with one or more $R_{9b}$;

$R_{8b}$ or $R_{9b}$ is each independently selected from methoxy, methyl, trifluoromethyl, difluoromethyl, difluoromethoxy, N-methylpiperazinyl, and cyano, preferably methoxy and methyl.

In one embodiment, the compound or the stereoisomer, the tautomer, the solvate, the hydrate, the prodrug, the stable isotopic derivative, or the pharmaceutically acceptable salt thereof described herein, wherein:

$R_3$ is selected from hydrogen, methyl, ethyl, n-propyl, isopropyl, methoxy, ethoxy, propoxy, and piperidinyloxy;
optionally, $R_3$ is further substituted with one or more $R_{3a}$, wherein each $R_{3a}$ is independently selected from methyl and dimethylamino;
optionally, $R_{3a}$ is further substituted with one or more $R_{3b}$, wherein each $R_{3b}$ is independently selected from $C_1$-$C_3$ alkyl, halogen, hydroxyl, $C_1$-$C_3$ alkoxy, and amino;
$R_4$ is selected from hydrogen, methyl, ethyl, n-propyl, isopropyl, fluorine, chlorine, bromine, and cyano;
optionally, $R_4$ is further substituted with one or more $R_{4a}$, wherein each $R_{4a}$ is independently selected from $C_1$-$C_3$ alkyl, halogen, amino, $C_1$-$C_3$ alkylamino, and di($C_1$-$C_3$)alkylamino;
$R_5$ is selected from chlorine, fluorine, bromine, iodine, cyano, ethyl, n-propyl, isopropyl, methyl, cyclopropyl, methoxy, and cyclopropyloxy;
optionally, $R_5$ is further substituted with one or more $R_{5a}$, wherein each $R_{5a}$ is independently selected from fluorine and cyano;
$R_6$ is selected from hydrogen, fluorine, chlorine, methyl, ethyl, n-propyl, isopropyl, cyclopropyl, methoxy, cyclopropyloxy, and cyano;
optionally, $R_6$ is further substituted with one or more $R_{6a}$, wherein each $R_{6a}$ is independently selected from $C_1$-$C_3$ alkyl, halogen, amino, $C_1$-$C_3$ alkylamino, and di($C_1$-$C_3$)alkylamino;
$R_1$ is $-X_1-R_7$, wherein $X_1$ is a bond or methylene;
optionally, $X_1$ is substituted with one or more $X_{1a}$, wherein $X_{1a}$ is methyl;
$R_7$ is selected from the following groups:

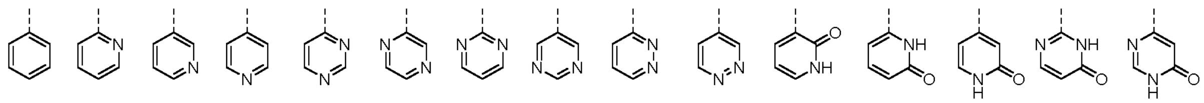

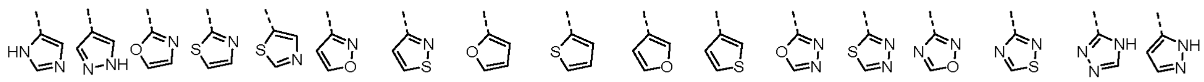

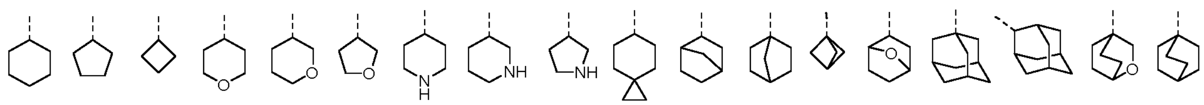

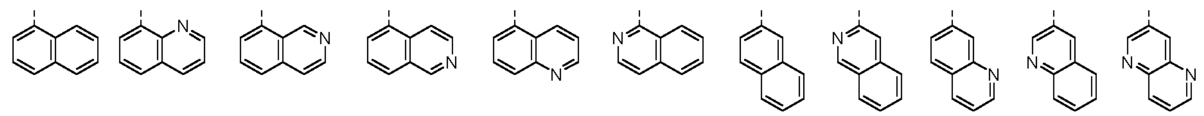

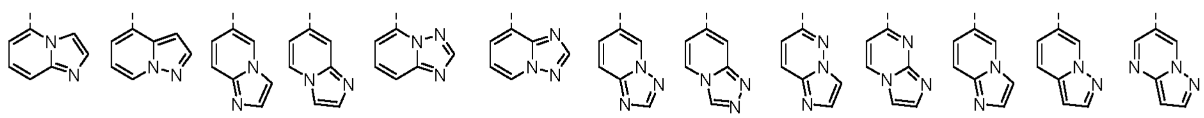

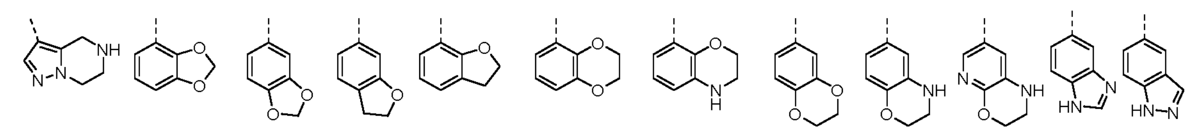

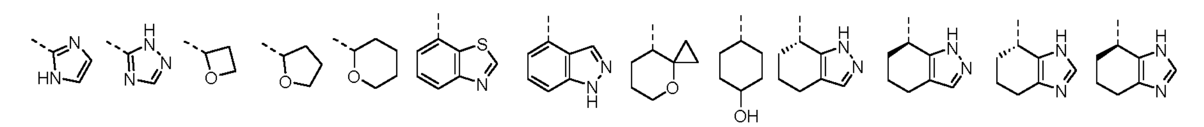

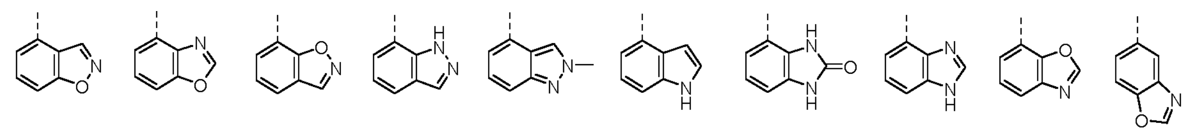,

preferably, $R_7$ is selected from the following groups:

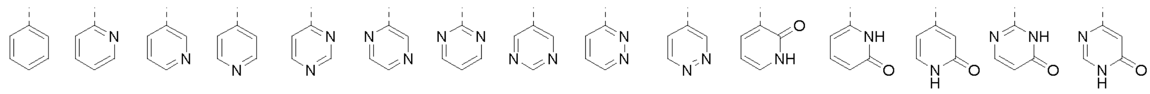

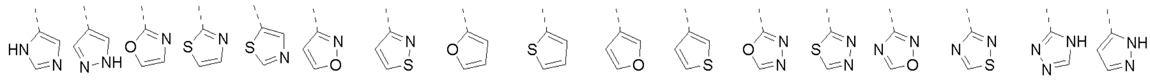

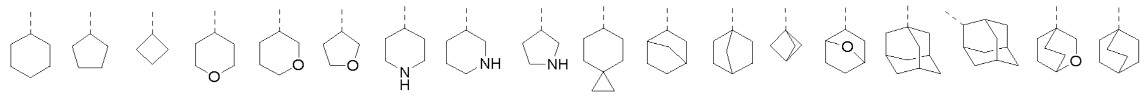

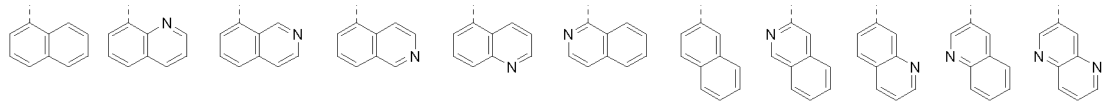

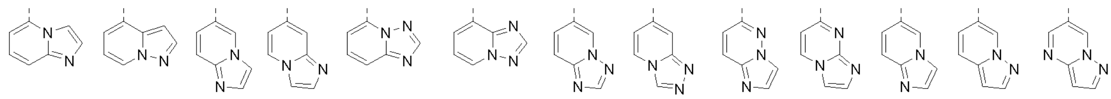

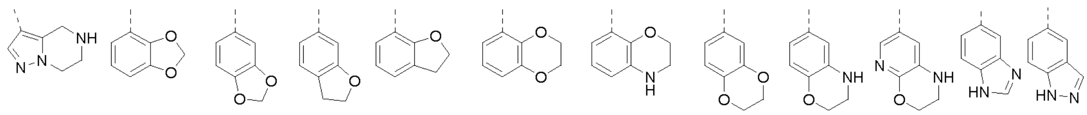

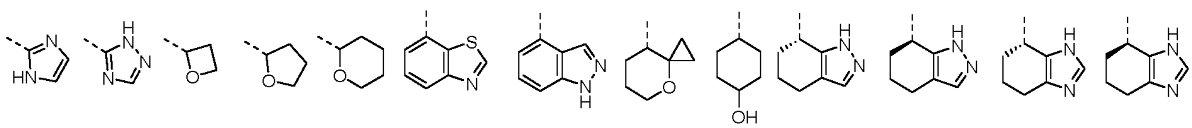
,

wherein the dashed line 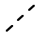 indicates where $R_7$ is connected to $X_1$;
$R_7$ is optionally substituted with one or more $R_{7a}$, wherein each $R_{7a}$ is independently selected from fluorine, chlorine, bromine, hydroxyl, cyano, methoxy, methyl, cyclopropyloxy, and cyclopropyl, preferably selected from fluorine, chlorine, bromine, hydroxyl, cyano, methoxy, methyl, and cyclopropyloxy;
optionally, $R_{7a}$ is further substituted with one or more $R_{7b}$, wherein $R_{7b}$ is independently selected from fluorine, chlorine, and bromine;
when $X_1$ is methylene, $R_7$ is not substituted or unsubstituted aryl;
$R_2$ is selected from hydrogen, methyl, ethyl, *n*-propyl, isopropyl, cyano, methoxy, and oxo;
optionally, $R_2$ is further substituted with one or more $R_{2a}$, wherein each $R_{2a}$ is independently selected from methyl, chlorine, fluorine, phenyl, cyclopropyl, oxo, amino, methylamino, dimethylamino, methoxy, hydroxyl, pyridinyl, pyrazolyl, and pyrimidinyl, preferably selected from methyl, chlorine, fluorine, phenyl, cyclopropyl, oxo, amino, methylamino, dimethylamino, methoxy, hydroxyl, pyridinyl, and pyrazolyl;
optionally, $R_{2a}$ is further substituted with one or more $R_{2b}$, wherein each $R_{2b}$ is independently selected from methoxy, methyl, trifluoromethyl, difluoromethyl, difluoromethoxy, *N*-methylpiperazinyl, and cyano, preferably methoxy and methyl;
$R_8$ and $R_9$ are each independently selected from hydrogen, methyl, ethyl, *n*-propyl, isopropyl, cyano, methoxy, oxo, cyclopropyl, tetrahydropyrrolyl, tetrahydrofuranyl, phenyl, and propenyl, or adjacent $R_8$ and $R_9$ form

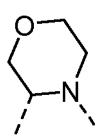 or 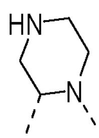;

optionally, $R_8$ is further substituted with one or more $R_{8a}$; optionally, $R_9$ is further substituted with one or more $R_{9a}$; optionally, the ring formed by adjacent $R_8$ and $R_9$ is further substituted with one or more $R_{8a}$ or $R_{9a}$;
$R_{8a}$ or $R_{9a}$ is each independently selected from methyl, chlorine, fluorine, bromine, phenyl, cyclopropyl, oxo, amino, methylamino, dimethylamino, methoxy, hydroxyl, pyridinyl, pyrazolyl, sulfonyl, morpholinyl, tetrahydrofuranyl, isoxazolyl, and pyrimidinyl; preferably, each $R_{8a}$ or each $R_{9a}$ is independently selected from methyl, chlorine, fluorine, bromine, phenyl, cyclopropyl, oxo, amino, methylamino, dimethylamino, methoxy, hydroxyl, pyridinyl, pyrazolyl, sulfonyl, morpholinyl, tetrahydrofuranyl, and isoxazolyl;
optionally, $R_{8a}$ is further substituted with one or more $R_{8b}$; optionally, $R_{9a}$ is further substituted with one or more $R_{9b}$;
$R_{8b}$ or $R_{9b}$ is each independently selected from methoxy, methyl, trifluoromethyl, difluoromethyl, difluoromethoxy, *N*-methylpiperazinyl, and cyano, preferably methoxy and methyl.

The present application provides a compound of formula (I) or a stereoisomer, a tautomer, a solvate, a hydrate, a prodrug, a stable isotopic derivative, or a pharmaceutically acceptable salt thereof, wherein the compound has optionally the following structures:

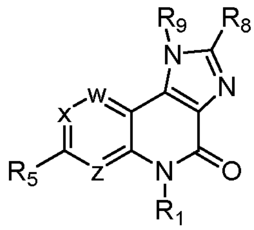

,

In some embodiments, w is $CR_3$, and $R_3$ is selected from hydrogen, $C_1$-$C_6$ alkyl, $C_2$-$C_6$ alkenyl, $C_2$-$C_6$ alkynyl, $C_1$-$C_6$ alkoxy, sulfonyl, halogen, cycloalkyl having 3-10 ring atoms, cyano, amino, acyl, hydroxyl, heteroaryl having 5-10 ring atoms, heteroaryloxy having 5-10 ring atoms, heterocyclyl having 5-10 ring atoms, heterocyclyloxy having 5-10 ring atoms, and cycloalkyloxy having 3-10 ring atoms; preferably, $R_3$ is selected from hydrogen, $C_1$-$C_4$ alkyl, $C_2$-$C_4$ alkenyl, $C_2$-$C_4$ alkynyl, $C_1$-$C_4$ alkoxy, halogen, cycloalkyl having 3-6 ring atoms, cyano, amino, acyl, hydroxyl, heteroaryl having 5-6 ring atoms, heteroaryloxy having 5-6 ring atoms, heterocyclyl having 5-6 ring atoms, heterocyclyloxy having 5-6 ring atoms, and cycloalkyloxy having 3-6 ring atoms; more preferably, $R_3$ is selected from hydrogen, methyl, ethyl, *n*-propyl, isopropyl, methoxy, ethoxy, propoxy, and piperidinyloxy;
optionally, $R_3$ is further substituted with one or more $R_{3a}$, wherein each $R_{8a}$ is independently selected from $C_1$-$C_6$ alkyl, $C_2$-$C_6$ alkenyl, $C_2$-$C_6$ alkynyl, $C_1$-$C_6$ alkoxy, $C_1$-$C_6$ alkylamino, di($C_1$-$C_6$ alkyl)amino, halogen, sulfonyl, cycloalkyl having 3-10 ring atoms, cyano, amino, acyl, hydroxyl, heteroaryl having 5-10 ring atoms, heteroaryloxy having 5-10 ring atoms, cycloalkyloxy having 3-10 ring atoms, heterocyclyl having 5-10 ring atoms, and heterocyclyloxy having 5-10 ring atoms; preferably, $R_{3a}$ is each independently selected from $C_1$-$C_3$ alkyl, $C_2$-$C_4$ alkenyl, $C_2$-$C_4$ alkynyl, $C_1$-$C_3$ alkoxy, $C_1$-$C_3$ alkylamino, di($C_1$-$C_3$ alkyl)amino, halogen, sulfonyl, cycloalkyl having 3-6 ring atoms, cyano, amino, acyl, hydroxyl, heteroaryl having 5-6 ring atoms, heteroaryloxy having 5-6 ring atoms, cycloalkyloxy having 3-6 ring atoms, heterocyclyl having 5-6 ring atoms, and heterocyclyloxy having 5-6 ring atoms; more preferably, $R_{3a}$ is each independently selected from methyl and dimethylamino; optionally, $R_{3a}$ is further substituted with one or more $R_{3b}$, wherein each $R_{3b}$ is independently selected from $C_1$-$C_3$ alkyl, halogen, hydroxyl, $C_1$-$C_3$ alkoxy, and amino.

In some embodiments, x is $CR_4$, and $R_4$ is selected from $C_1$-$C_6$ alkyl, $C_2$-$C_6$ alkenyl, $C_2$-$C_6$ alkynyl, $C_1$-$C_6$ alkoxy, $C_1$-$C_6$ alkylthio, sulfonyl, halogen, cycloalkyl having 3-10 ring atoms, cyano, amino, and acyl; preferably, $R_4$ is selected from $C_1$-$C_4$ alkyl, $C_2$-$C_4$ alkenyl, $C_2$-$C_4$ alkynyl, $C_1$-$C_4$ alkoxy, $C_1$-$C_4$ alkylthio, sulfonyl, halogen, cycloalkyl having 3-6 ring atoms, cyano, amino, and acyl; more preferably, $R_4$ is selected from methyl, ethyl, *n*-propyl, isopropyl, fluorine, chlorine, bromine, and cyano;
optionally, $R_4$ is further substituted with one or more $R_{4a}$, wherein each $R_{4a}$ is independently selected from $C_1$-$C_6$ alkyl, halogen, amino, $C_1$-$C_6$ alkylamino, and di($C_1$-$C_6$)alkylamino; preferably, $R_{4a}$ is each independently selected from $C_1$-$C_3$ alkyl, halogen, amino, $C_1$-$C_3$ alkylamino, and di($C_1$-$C_3$)alkylamino.

In some embodiments, $R_5$ is selected from halogen, cyano, amino, hydroxyl, $C_1$-$C_6$ alkyl, $C_2$-$C_6$ alkenyl, $C_2$-$C_6$ alkynyl, $C_1$-$C_6$ alkoxy, cycloalkyl having 3-10 ring atoms, cycloalkyloxy having 3-10 ring atoms, heterocyclyl having 4-10 ring atoms, and heteroaryl having 5-10 ring atoms; preferably, $R_5$ is selected from halogen, $C_1$-$C_6$ alkyl, $C_1$-$C_6$ alkoxy, cycloalkyl having 3-6 ring atoms, cycloalkyloxy having 3-6 ring atoms, heterocyclyl having 4-6 ring atoms, and heteroaryl having 5-6 ring atoms; more preferably, $R_5$ is selected from chlorine, fluorine, bromine, iodine, cyano, ethyl, *n*-propyl, isopropyl, methyl, cyclopropyl, methoxy, and cyclopropyloxy;
optionally, $R_5$ is further substituted with one or more $R_{5a}$, wherein each $R_{5a}$ is independently selected from $C_1$-$C_6$ alkyl, $C_1$-$C_6$ alkoxy, halogen, cycloalkyl having 3-10 ring atoms, cyano, amino, and hydroxyl; preferably, $R_{5a}$ is each indepen-

dently selected from $C_1$-$C_6$ alkyl, halogen, cyano, and cycloalkyl having 3-6 ring atoms; more preferably, $R_{5a}$ is each independently selected from fluorine and cyano.

In some embodiments, z is $CR_6$ or N;

$R_6$ is selected from hydrogen, $C_1$-$C_6$ alkyl, $C_2$-$C_6$ alkenyl, $C_2$-$C_6$ alkynyl, $C_1$-$C_6$ alkoxy, $C_1$-$C_6$ alkylthio, sulfonyl, halogen, cycloalkyl having 3-10 ring atoms, cyano, amino, and acyl; preferably, $R_6$ is selected from hydrogen, $C_1$-$C_4$ alkyl, $C_2$-$C_4$ alkenyl, $C_2$-$C_4$ alkynyl, $C_1$-$C_4$ alkoxy, $C_1$-$C_4$ alkylthio, sulfonyl, halogen, cycloalkyl having 3-6 ring atoms, cyano, amino, and acyl; more preferably, $R_6$ is selected from hydrogen, fluorine, chlorine, methyl, ethyl, n-propyl, isopropyl, cyclopropyl, methoxy, cyclopropyloxy, and cyano;

optionally, $R_6$ is further substituted with one or more $R_{6a}$, wherein each $R_{6a}$ is independently selected from $C_1$-$C_6$ alkyl, halogen, amino, $C_1$-$C_6$ alkylamino, and di($C_1$-$C_6$)alkylamino; preferably, $R_{6a}$ is each independently selected from $C_1$-$C_3$ alkyl, halogen, amino, $C_1$-$C_3$ alkylamino, and di($C_1$-$C_3$)alkylamino.

In some embodiments, $R_1$ is -$X_1$-$R_7$, wherein $X_1$ is a bond or methylene;

optionally, $X_1$ is substituted with one or more $X_{1a}$, wherein each $X_{1a}$ is independently selected from halogen and $C_1$-$C_3$ alkyl, preferably methyl;

$R_7$ is selected from cycloalkyl having 4-10 ring atoms, aryl having 6-12 ring atoms, heteroaryl having 5-12 ring atoms, and heterocyclyl having 3-12 ring atoms;

preferably, $R_7$ is selected from the following groups:

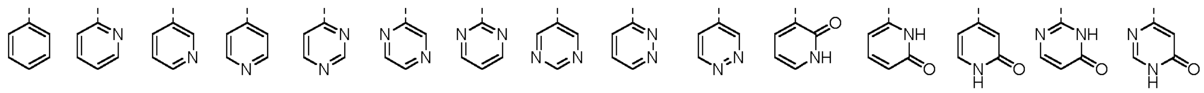

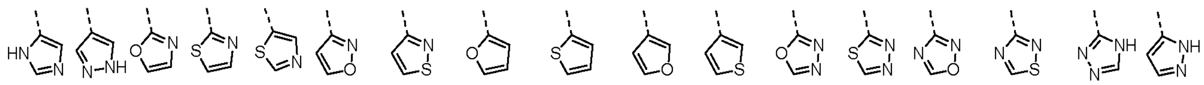

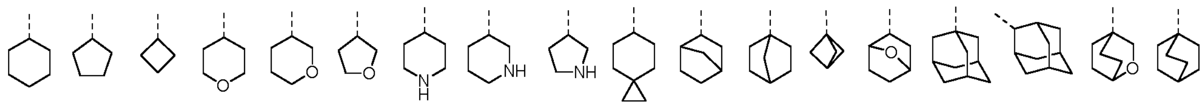

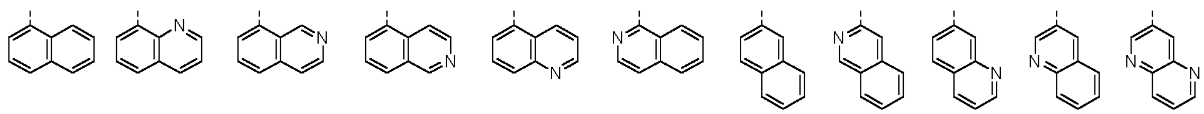

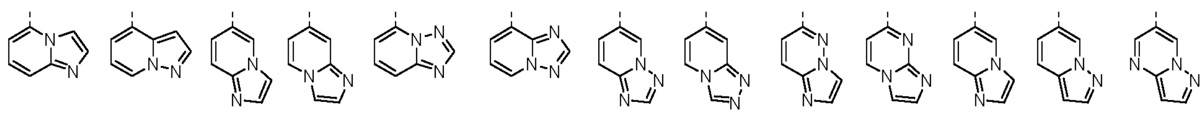

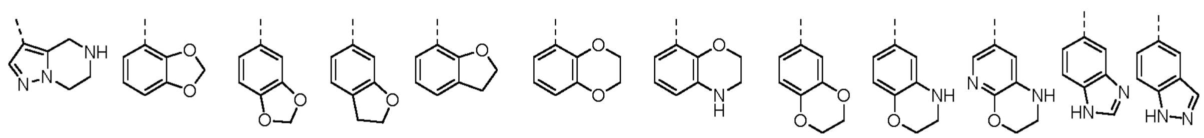

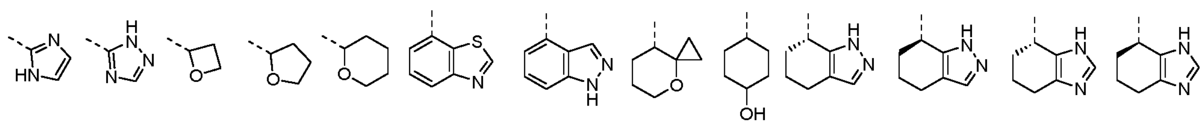

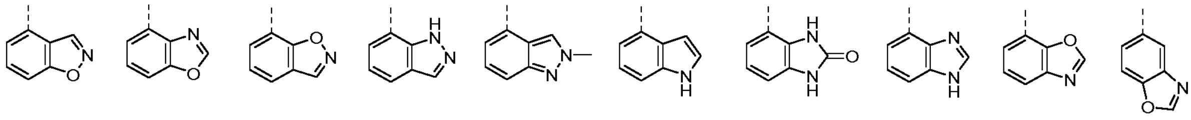,

more preferably, $R_7$ is selected from the following groups:

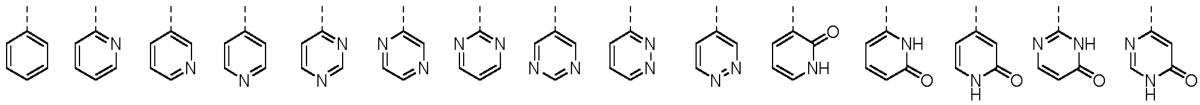

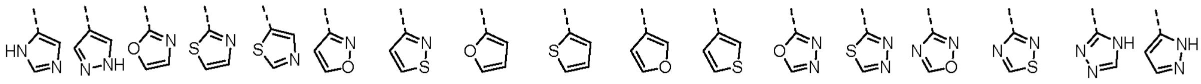

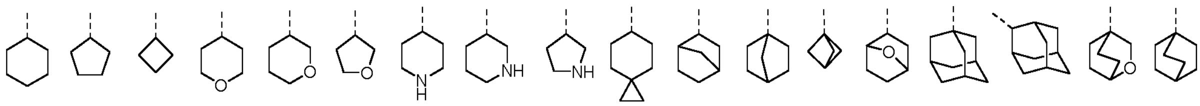

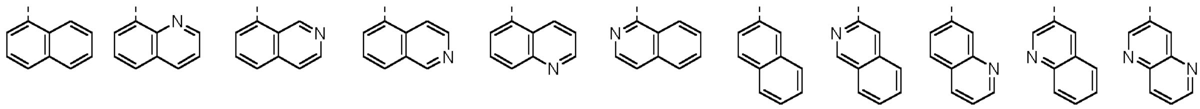

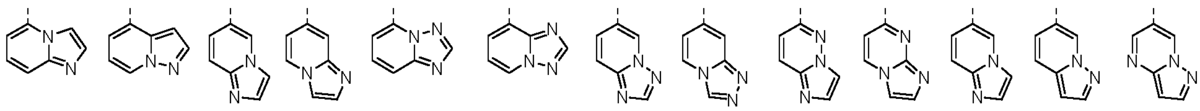

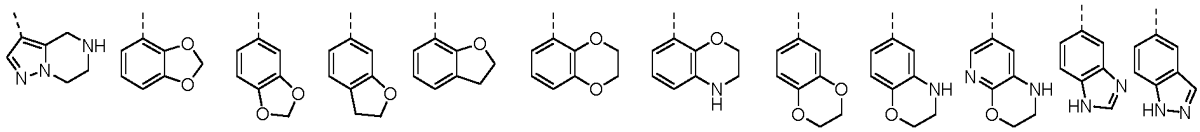

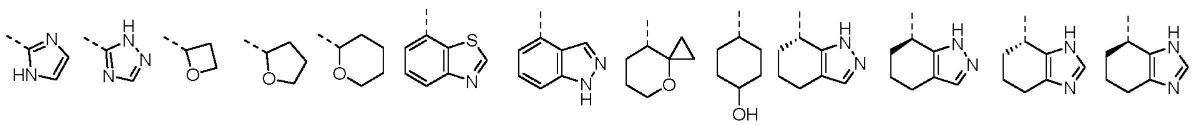,

$R_7$ is optionally substituted with one or more $R_{7a}$, wherein each $R_{7a}$ is independently selected from oxo, halogen, $C_1$-$C_6$ alkyl, cycloalkyl having 3-10 ring atoms, $C_1$-$C_6$ alkoxy, cycloalkyloxy having 3-6 ring atoms, cyano, amino, hydroxyl, $C_2$-$C_6$ alkenyl, $C_2$-$C_6$ alkynyl, sulfonyl, acyl, carboxyl, heterocyclyl having 3-12 ring atoms, heteroaryl having 5-10 ring atoms, and ureido; preferably, $R_{7a}$ is each independently selected from oxo, halogen, $C_1$-$C_4$ alkyl, cycloalkyl having 3-6 ring atoms, $C_1$-$C_4$ alkoxy, cycloalkyloxy having 3-4 ring atoms, cyano, amino, hydroxyl, $C_2$-$C_4$ alkenyl, $C_2$-$C_4$ alkynyl, sulfonyl, acyl, carboxyl, heterocyclyl having 3-6 ring atoms, heteroaryl having 5-6 ring atoms, and ureido; more preferably, $R_{7a}$ is each independently selected from fluorine, chlorine, bromine, hydroxyl, cyano, methoxy, methyl, cyclopropyloxy, and cyclopropyl;

optionally, $R_{7a}$ is further substituted with one or more $R_{7b}$, wherein $R_{7b}$ is independently selected from halogen, $C_1$-$C_6$ alkyl, $C_1$-$C_6$ alkoxy, hydroxyl, amino, $C_2$-$C_6$ alkenyl, $C_2$-$C_6$ alkynyl, $C_1$-$C_6$ alkylamino, di($C_1$-$C_6$)alkylamino, sulfonyl, heterocyclyl having 3-6 ring atoms, and heteroaryl having 5-6 ring atoms; preferably, each $R_{7b}$ is independently selected from halogen, $C_1$-$C_3$ alkyl, $C_1$-$C_3$ alkoxy, hydroxyl, amino, $C_2$-$C_4$ alkenyl, $C_2$-$C_4$ alkynyl, $C_1$-$C_3$ alkylamino, di($C_1$-$C_3$)alkylamino, sulfonyl, heterocyclyl having 3-6 ring atoms, and heteroaryl having 5-6 ring atoms; more preferably, each $R_{7b}$ is independently selected from fluorine, chlorine, and bromine;

when $X_1$ is methylene, $R_7$ is not substituted or unsubstituted aryl.

In some embodiments, $R_8$ and $R_9$ are each independently selected from hydrogen, halogen, $C_1$-$C_6$ alkyl, $C_1$-$C_6$

alkoxy, $C_2$-$C_6$ alkenyl, $C_2$-$C_6$ alkynyl, cycloalkyl having 3-10 ring atoms, cycloalkyloxy having 3-10 ring atoms, heterocyclyl having 4-10 ring atoms, heterocyclyloxy having 4-10 ring atoms, aryl having 6-10 ring atoms, heteroaryl having 5-10 ring atoms, heteroaryloxy having 5-10 ring atoms, cyano, amino, acyl, sulfonyl, and oxo, or adjacent $R_8$ and $R_9$ form a 5-6 membered carbocyclic ring, heterocyclic ring, or heteroaromatic ring; preferably, $R_8$ and $R_9$ are each independently selected from hydrogen, halogen, $C_1$-$C_4$ alkyl, $C_1$-$C_4$ alkoxy, $C_2$-$C_4$ alkenyl, $C_2$-$C_4$ alkynyl, cycloalkyl having 3-6 ring atoms, cycloalkyloxy having 3-6 ring atoms, heterocyclyl having 5-6 ring atoms, heterocyclyloxy having 5-6 ring atoms, aryl having 6 ring atoms, heteroaryl having 5-6 ring atoms, heteroaryloxy having 5-6 ring atoms, cyano, amino, acyl, sulfonyl, and oxo, or adjacent $R_8$ and $R_9$ form a 5-6 membered heterocyclic ring or heteroaromatic ring; more preferably, $R_8$ and $R_9$ are each independently selected from hydrogen, methyl, ethyl, *n*-propyl, isopropyl, cyano, methoxy, oxo, cyclopropyl, tetrahydropyrrolyl, tetrahydrofuranyl, phenyl, and propenyl, or adjacent $R_8$ and $R_9$ form

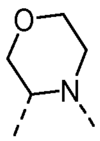 or 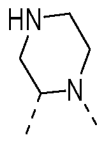;

optionally, $R_8$ is further substituted with one or more $R_{8a}$; optionally, $R_9$ is further substituted with one or more $R_{9a}$; optionally, the carbocyclic ring, heterocyclic ring, or heteroaromatic ring formed by adjacent $R_8$ and $R_9$ is further substituted with one or more $R_{8a}$ or $R_{9a}$;

$R_{8a}$ or $R_{9a}$ is each independently selected from halogen, $C_1$-$C_6$ alkyl, heterocyclyl having 4-10 ring atoms, heterocyclyloxy having 4-10 ring atoms, amino, heteroaryl having 5-10 ring atoms, cycloalkyl having 3-10 ring atoms, aryl having 6-10 ring atoms, $C_1$-$C_6$ alkylamino, di($C_1$-$C_6$)alkylamino, hydroxyl, sulfonyl, $C_1$-$C_6$ alkoxy, cyano, $C_2$-$C_6$ alkenyl, $C_2$-$C_6$ alkynyl, and oxo; preferably, each $R_{8a}$ or each $R_{9a}$ is independently selected from halogen, $C_1$-$C_4$ alkyl, heterocyclyl having 5-6 ring atoms, heterocyclyloxy having 5-6 ring atoms, amino, heteroaryl having 5-6 ring atoms, cycloalkyl having 3-6 ring atoms, aryl having 6 ring atoms, $C_1$-$C_3$ alkylamino, di($C_1$-$C_3$)alkylamino, hydroxyl, sulfonyl, $C_1$-$C_4$ alkoxy, cyano, $C_2$-$C_4$ alkenyl, $C_2$-$C_4$ alkynyl, and oxo; more preferably, each $R_{8a}$ or each $R_{9a}$ is independently selected from methyl, chlorine, fluorine, bromine, phenyl, cyclopropyl, oxo, amino, methylamino, dimethylamino, methoxy, hydroxyl, pyridinyl, pyrazolyl, sulfonyl, morpholinyl, tetrahydrofuranyl, isoxazolyl, and pyrimidinyl;

optionally, $R_{8a}$ is further substituted with one or more $R_{8b}$; optionally, $R_{9a}$ is further substituted with one or more $R_{9b}$;

$R_{8b}$ or $R_{9b}$ is each independently selected from $C_1$-$C_3$ alkyl substituted with 0-3 halogens, $C_1$-$C_3$ alkoxy substituted with 0-3 halogens, halogen, hydroxyl, unsubstituted 5-6 membered heterocyclyl or 5-6 membered heterocyclyl substituted with $C_1$-$C_3$ alkyl, cyano, and oxo, preferably $C_1$-$C_3$ alkyl, $C_1$-$C_3$ alkoxy, halogen, hydroxyl, and oxo; preferably, $R_{8b}$ or $R_{9b}$ is each independently selected from methoxy and methyl.

In another aspect, the present application provides a compound of formula (I) or a stereoisomer, a tautomer, a solvate, a hydrate, a prodrug, a stable isotopic derivative, or a pharmaceutically acceptable salt thereof, wherein the compound has the following structures:

| | | | | | |
|---|---|---|---|---|---|
| | | B2 | 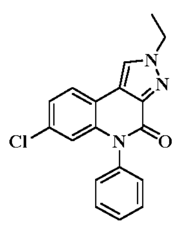 | B3 | 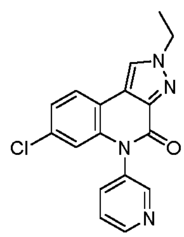 |
| B4 | 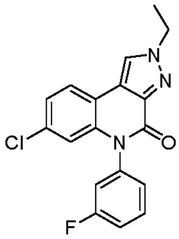 | B5 | 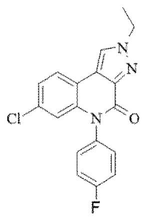 | B6 | 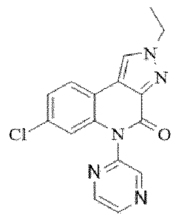 |

(continued)

| | | | | | |
|---|---|---|---|---|---|
| B7 | 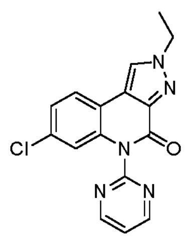 | B8 | 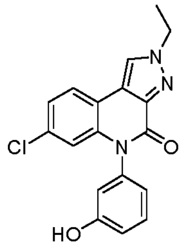 | B9 | 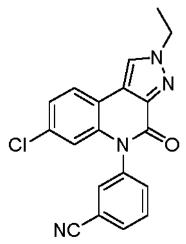 |
| B10 | 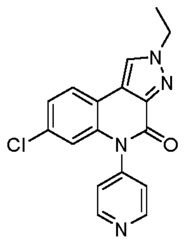 | B11 | 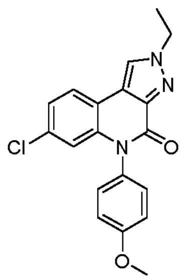 | B12 | 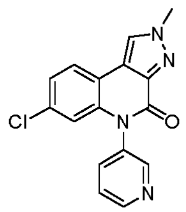 |
| B13 | 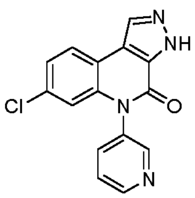 | B14 | 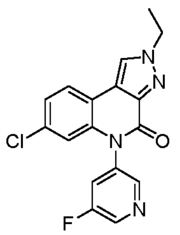 | B15 | 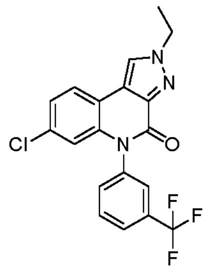 |
| B16 | 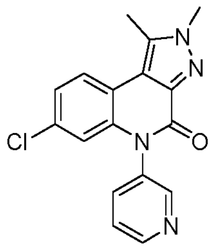 | B17 | 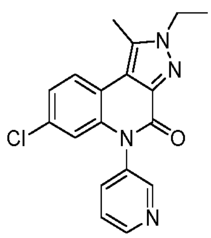 | B18 | 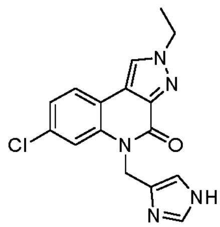 |
| B19 | 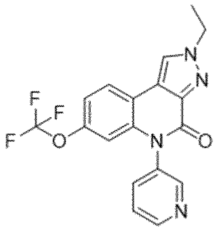 | B20 | 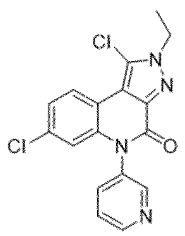 | B21 | 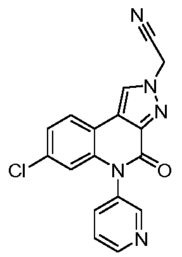 |
| B22 | 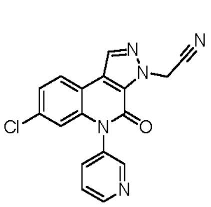 | B23 | 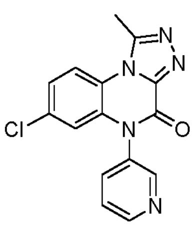 | B24 | 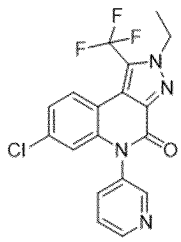 |

(continued)

| | | | | | |
|---|---|---|---|---|---|
| B25 | 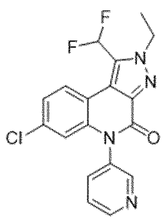 | B26 | 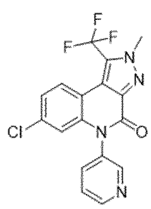 | B27 | 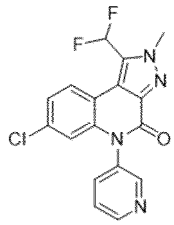 |
| B28 | 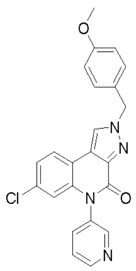 | B29 | 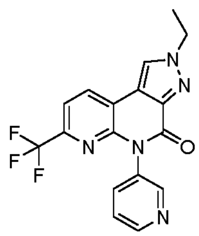 | B30 | 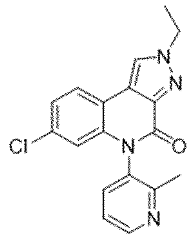 |
| B31 | 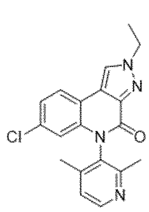 | B32 | 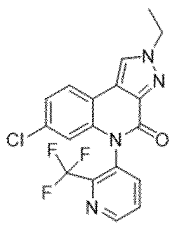 | B33 | 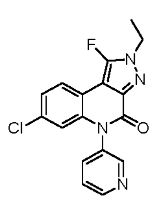 |
| B34 | 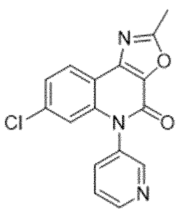 | B35 | 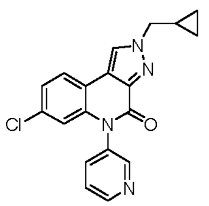 | B36 | 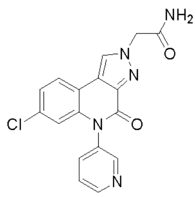 |
| B37 | 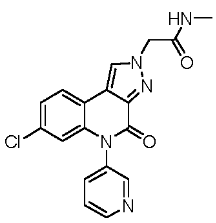 | B38 | 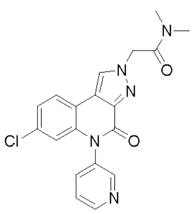 | B39 | 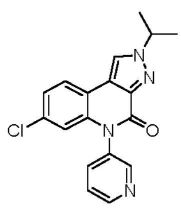 |
| B40 | 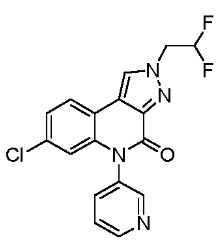 | B41 | 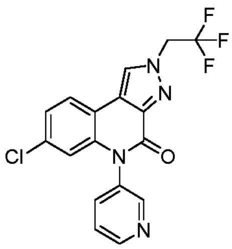 | B42 | 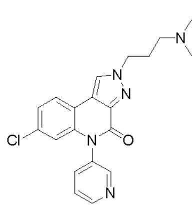 |
| B43 | 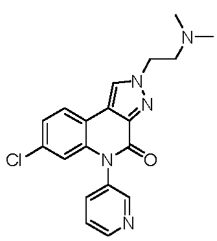 | B44 | 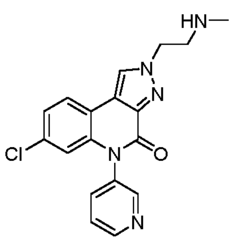 | B45 | 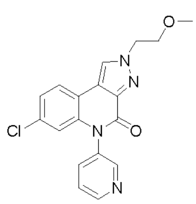 |

(continued)

| | | | | | |
|---|---|---|---|---|---|
| B46 | 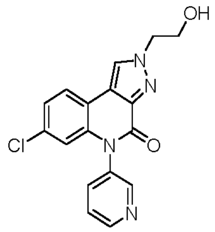 | B47 | 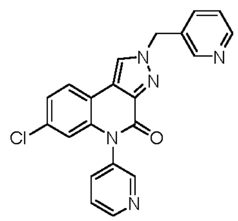 | B48 | 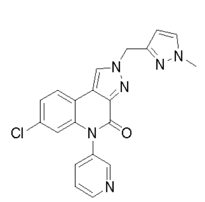 |
| B49 | 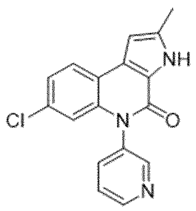 | B50 | 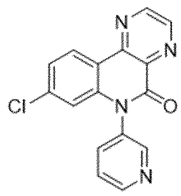 | B51 | 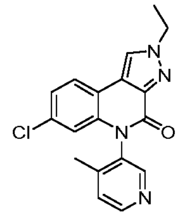 |
| B52 | 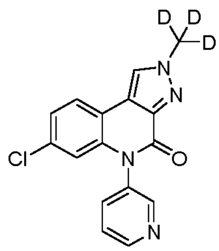 | B53 | 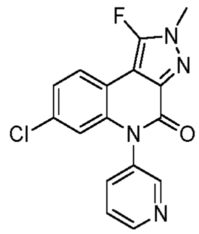 | B54 | 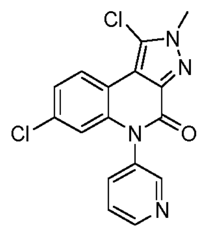 |
| B55 | 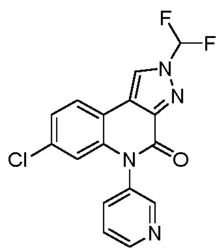 | B56 | 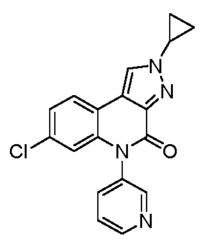 | B57 | 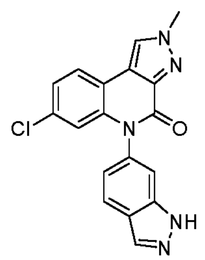 |
| B58 | 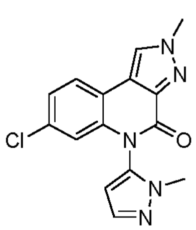 | B59 | 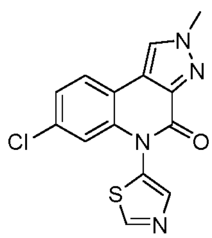 | B60 | 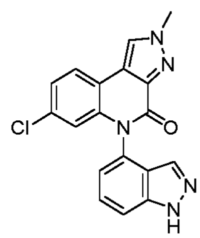 |
| B61 | 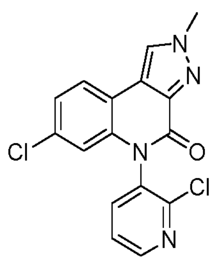 | B62 | 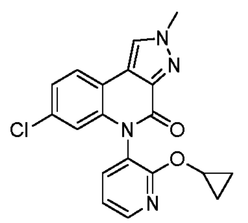 | B63 | 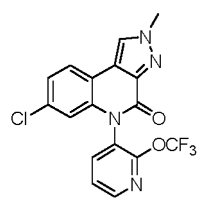 |

(continued)

| | | | | | |
|---|---|---|---|---|---|
| B64 | 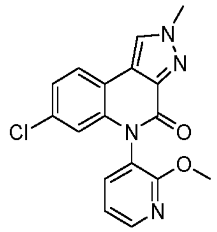 | B65 | 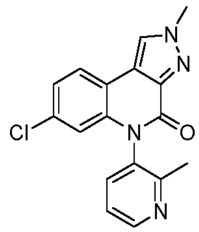 | B66 | 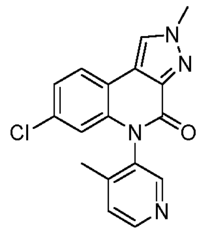 |
| B67 | 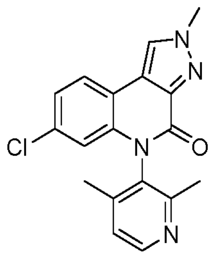 | B68 | 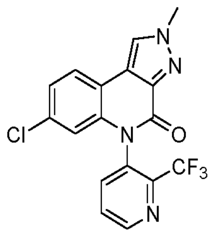 | B69 | 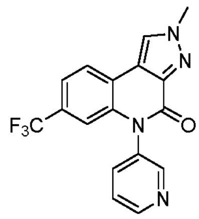 |
| B70 | 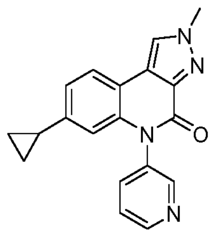 | B71 | 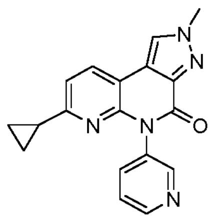 | B72 | 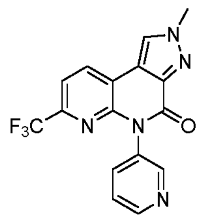 |
| B73 | 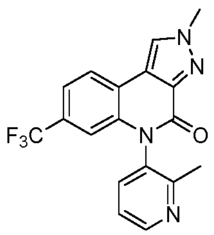 | B74 | 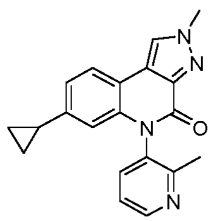 | B75 | 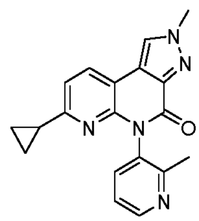 |
| B76 | 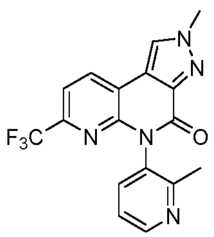 | B77 | 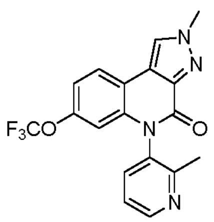 | B78 | 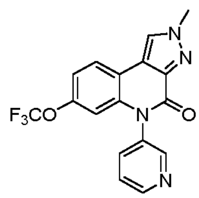 |
| B79 | 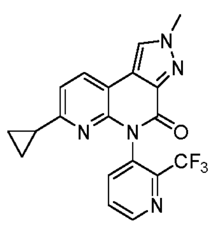 | B80 | 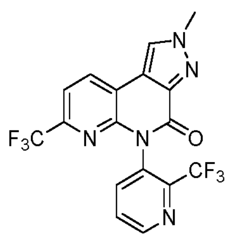 | B81 | 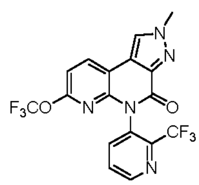 |

(continued)

| | | | | | |
|---|---|---|---|---|---|
| B82 | 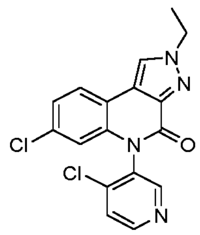 | B83 | 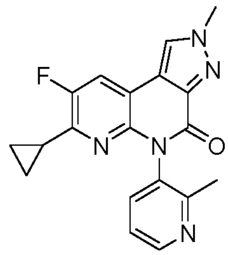 | B84 | 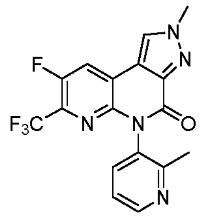 |
| B85 | 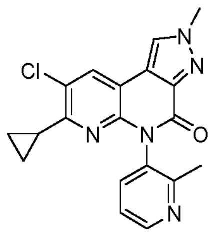 | B86 | 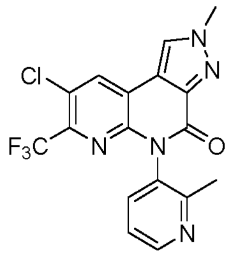 | B87 | 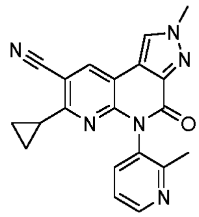 |
| B88 | 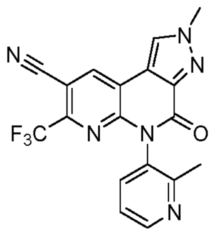 | B89 | 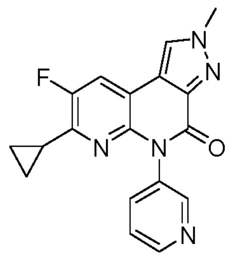 | B90 | 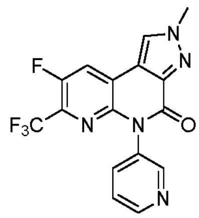 |
| B91 | 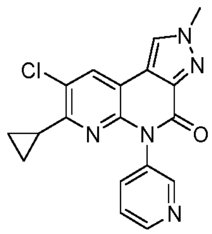 | B92 | 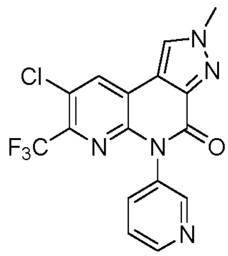 | B93 | 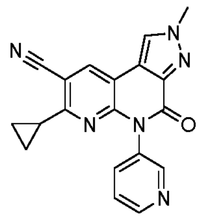 |
| B94 | 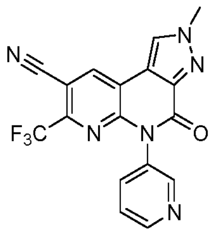 | B95 | 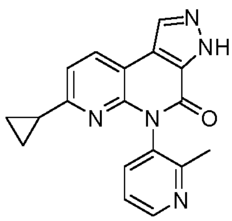 | B96 | 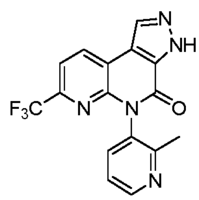 |
| B97 | 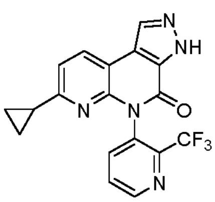 | B98 | 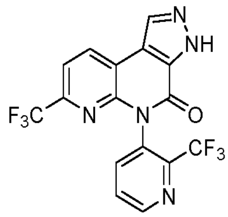 | B99 | 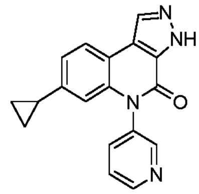 |

(continued)

| | | | | | |
|---|---|---|---|---|---|
| B100 | 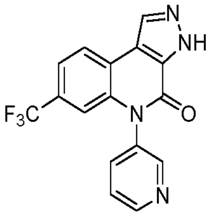 | B101 | 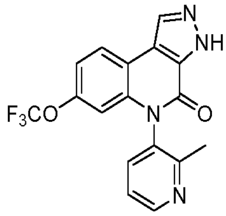 | B102 | 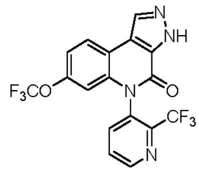 |
| B103 | 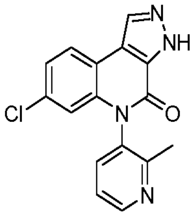 | B104 | 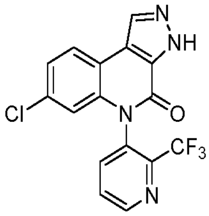 | B105 | 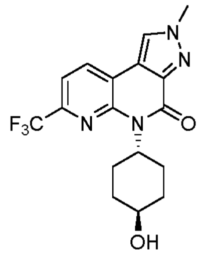 |
| B106 | 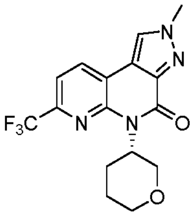 | B107 | 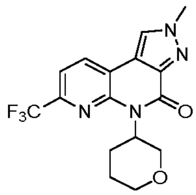 | B108 | 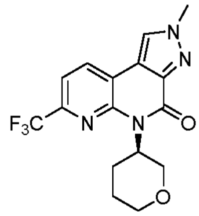 |
| B109 | 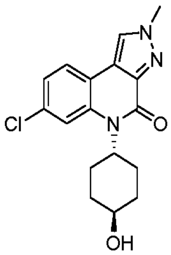 | B110 | 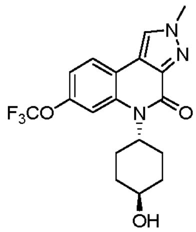 | B111 | 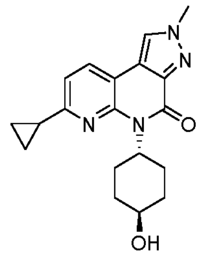 |
| B112 | 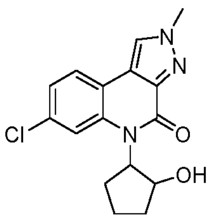 | B113 | 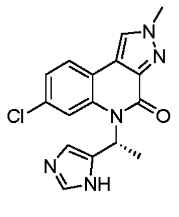 | B114 | 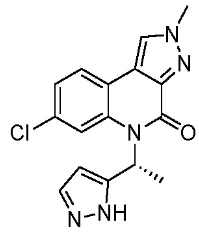 |
| B115 | 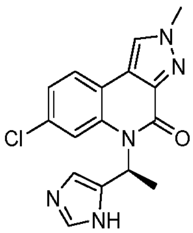 | B116 | 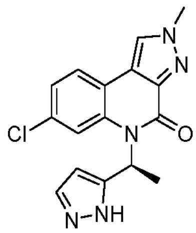 | B117 | 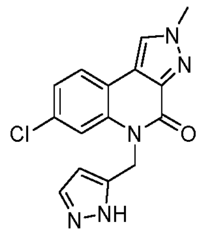 |
| B118 | 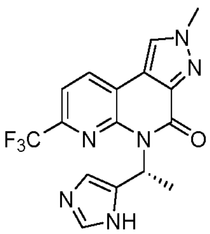 | B119 | 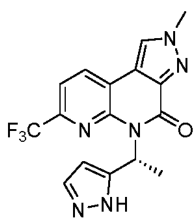 | B120 | 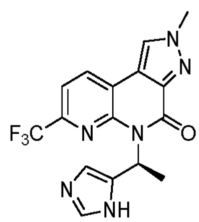 |

(continued)

| | | | | | |
|---|---|---|---|---|---|
| B121 | 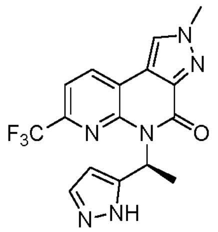 | B122 | 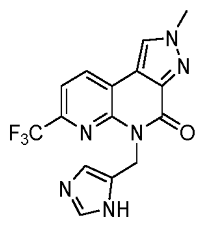 | B123 | 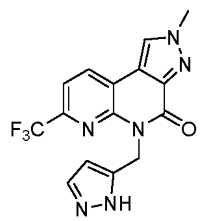 |
| B124 | 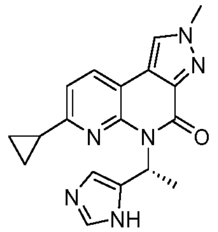 | B125 | 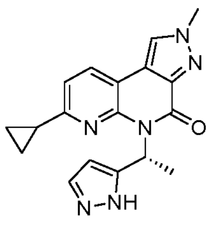 | B126 | 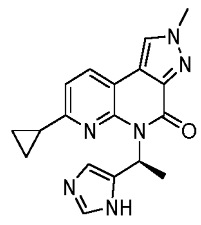 |
| B127 | 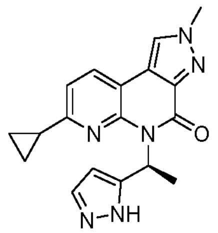 | B128 | 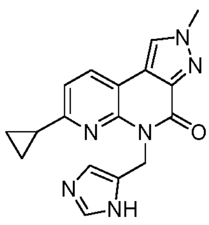 | B129 | 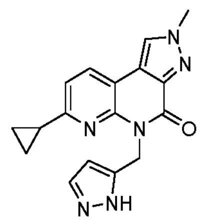 |
| B130 | 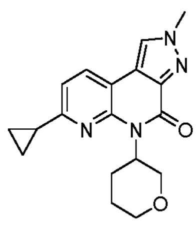 | B131 | 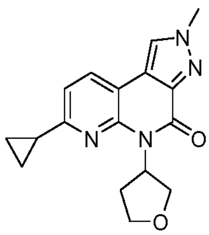 | B132 | 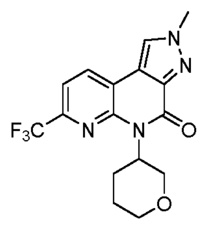 |
| B133 | 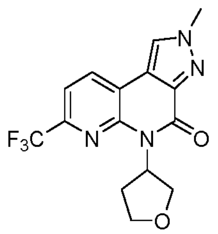 | B134 | 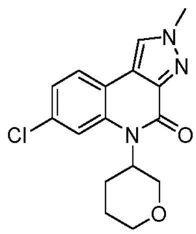 | B135 | 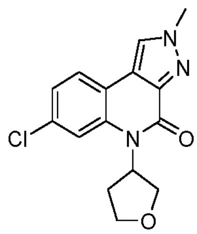 |
| B136 | 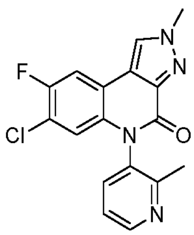 | B137 | 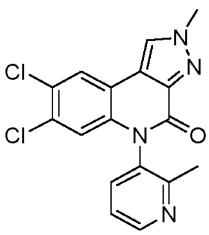 | B138 | 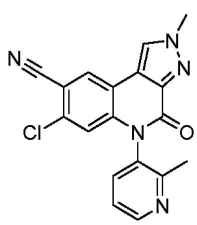 |
| B139 | 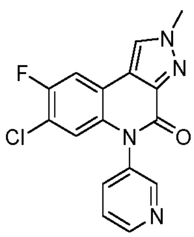 | B140 | 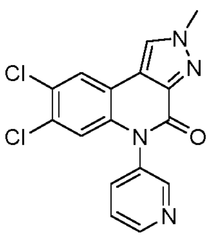 | B141 | 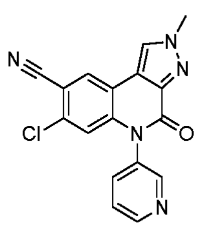 |

(continued)

| | | | | | |
|---|---|---|---|---|---|
| B142 | 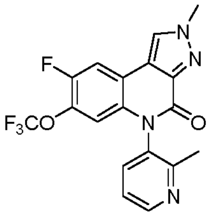 | B143 | 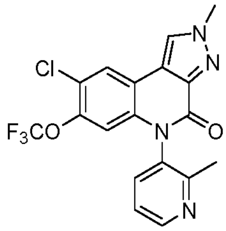 | B144 | 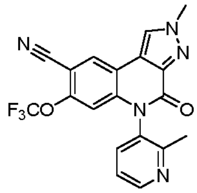 |
| B145 | 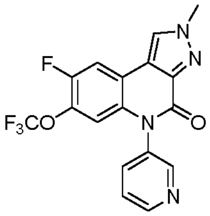 | B146 | 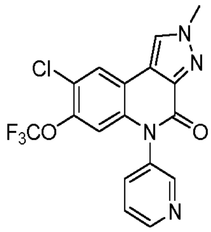 | B147 | 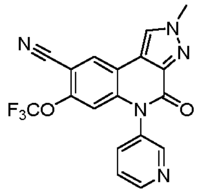 |
| B148 | 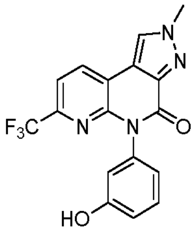 | B149 | 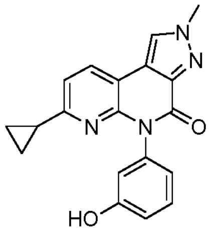 | B150 | 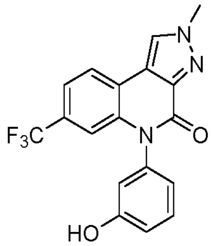 |
| B151 | 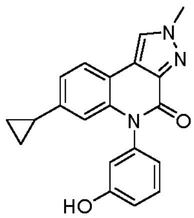 | B152 | 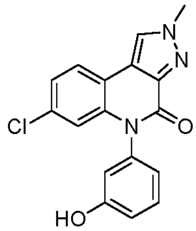 | B153 | 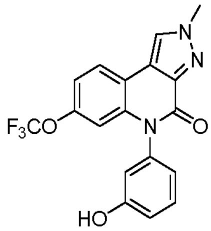 |
| B154 | 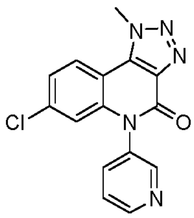 | B155 | 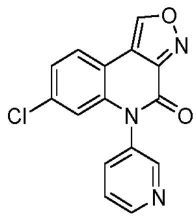 | B156 | 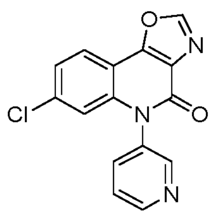 |
| B157 | 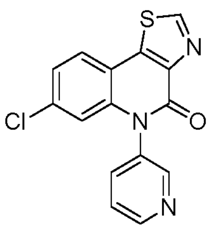 | B158 | 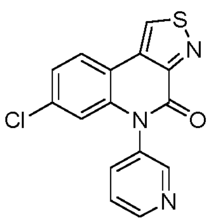 | B159 | 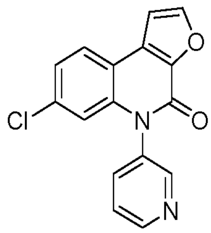 |
| B160 | 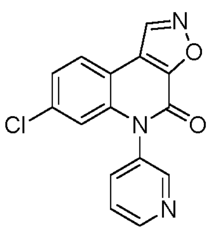 | B161 | 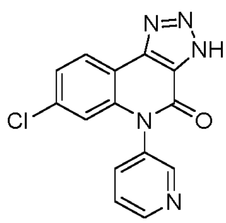 | B162 | 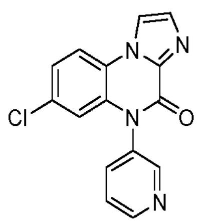 |

(continued)

| | | | | | |
|---|---|---|---|---|---|
| B163 | 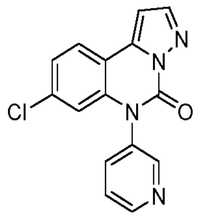 | B164 | 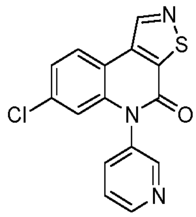 | B165 | 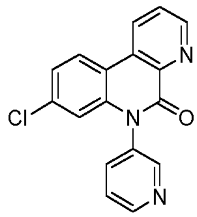 |
| B166 | 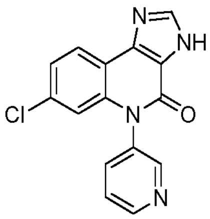 | B167 | 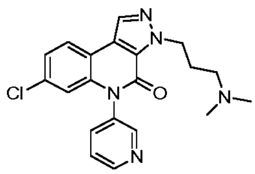 | B168 | 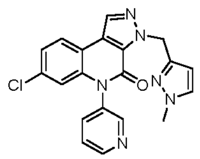 |
| B169 | 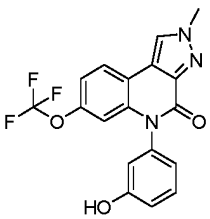 | B170 | 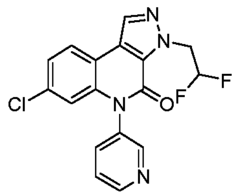 | B171 | 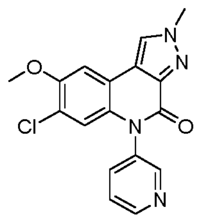 |
| B172 | 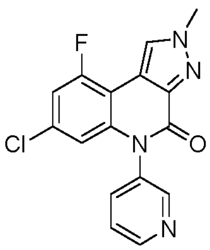 | B173 | 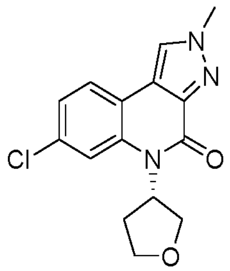 | B174 | 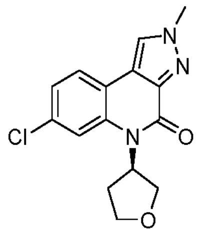 |
| B175 | 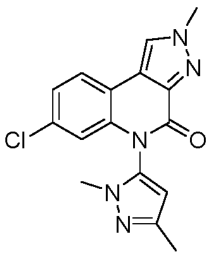 | B176 | 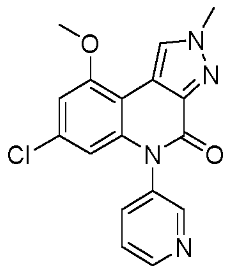 | B177 | 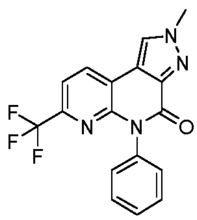 |
| B178 | 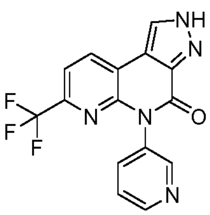 | B179 | 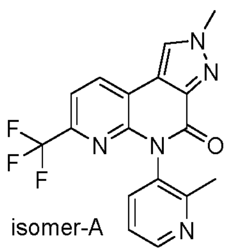 isomer-A | B180 | 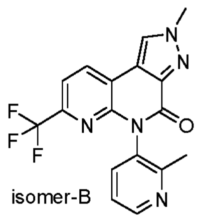 isomer-B |

(continued)

| | | | | | |
|---|---|---|---|---|---|
| B181 | 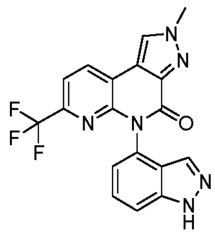 | B182 | 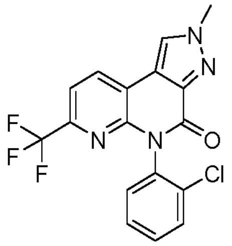 | B183 | 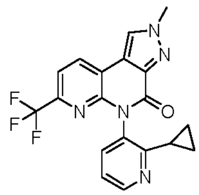 |
| B184 | 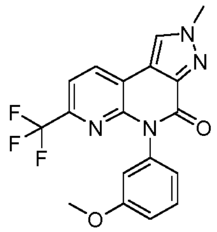 | B185 | 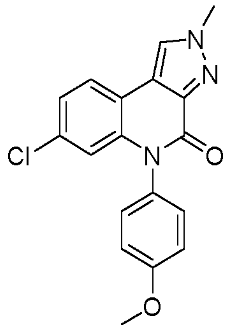 | B186 | 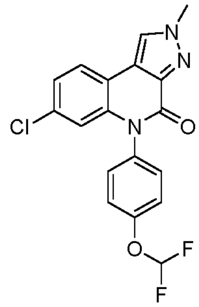 |
| B187 | 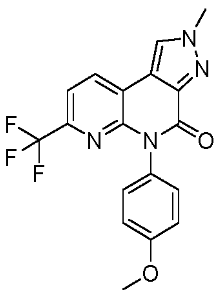 | B188 | 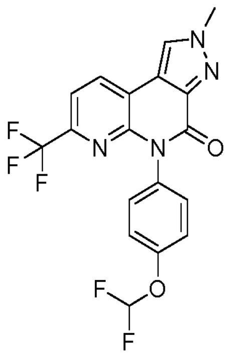 | B189 | 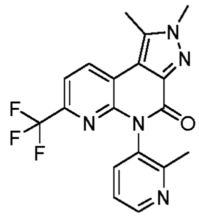 |
| B190 | 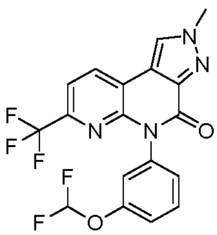 | B191 | 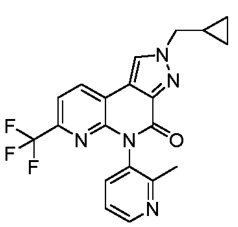 | B192 | 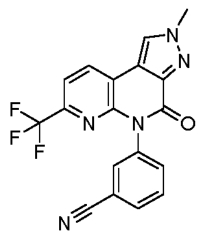 |
| B193 | 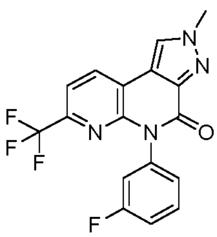 | B194 | 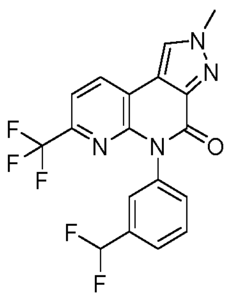 | B195 | 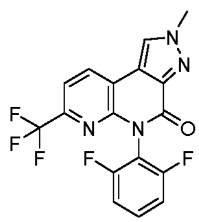 |

(continued)

| | | | | | |
|---|---|---|---|---|---|
| B196 | 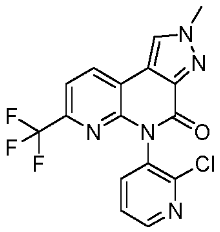 | B197 | 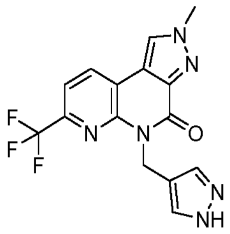 | B198 | 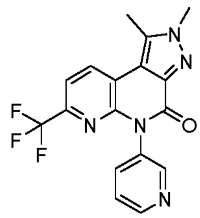 |
| B199 | 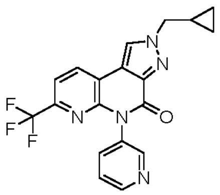 | B200 | 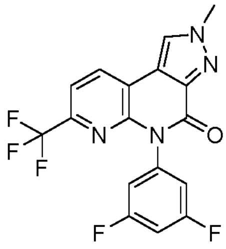 | B201 | 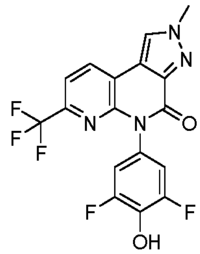 |
| B202 | 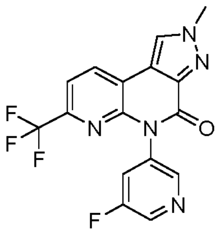 | B203 | 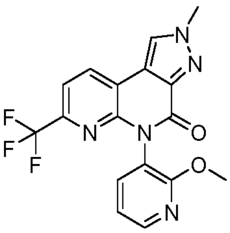 | B204 | 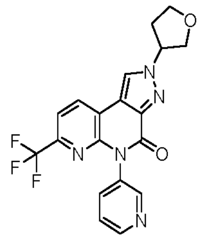 |
| B205 | 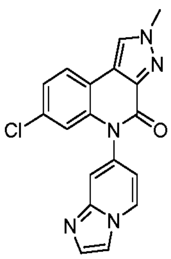 | B206 | 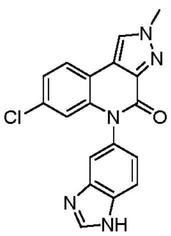 | B207 | 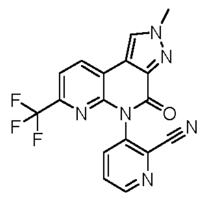 |
| B208 | 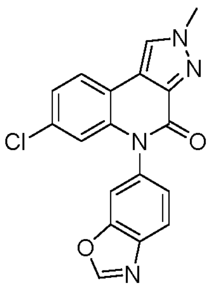 | B209 | 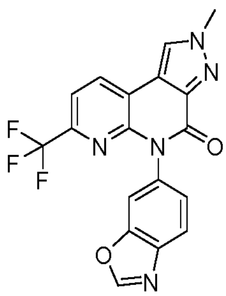 | B210 | 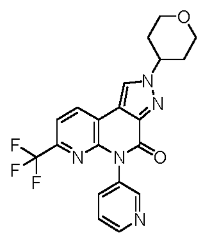 |
| B211 | 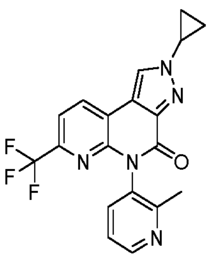 | B212 | 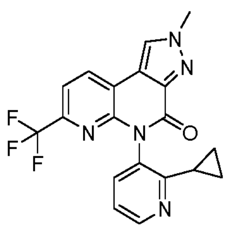 Isomer A | B213 | 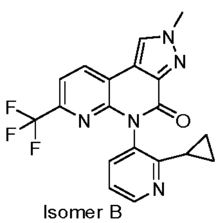 Isomer B |

(continued)

| | | | | | |
|---|---|---|---|---|---|
| B214 | 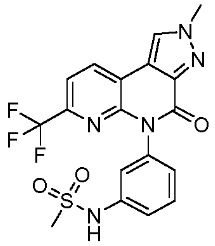 | B215 | 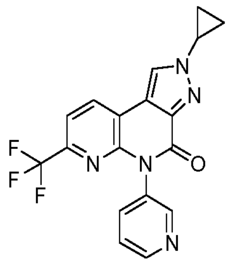 | B216 | 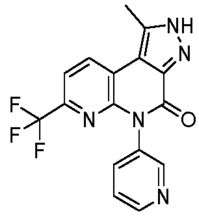 |
| B217 | 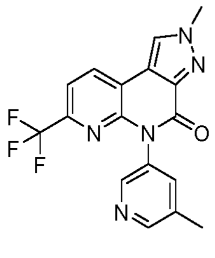 | B218 | 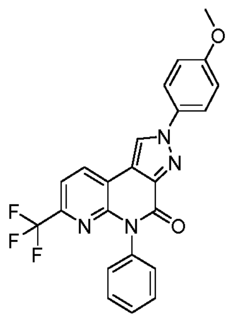 | B219 | 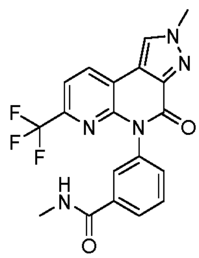 |
| B220 | 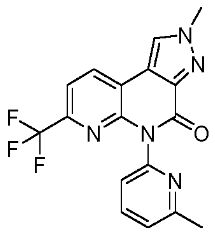 | B221 | 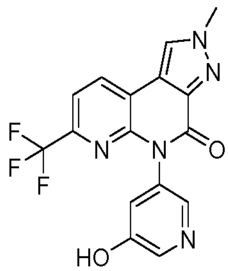 | B222 | 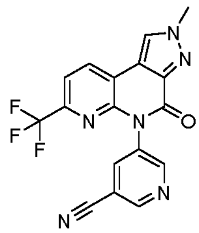 |
| B223 | 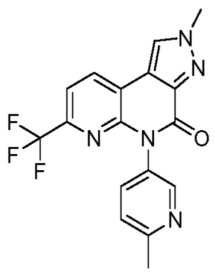 | B224 | 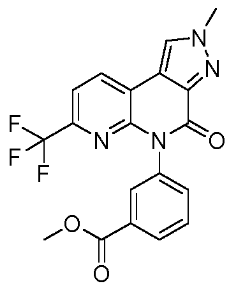 | B225 | 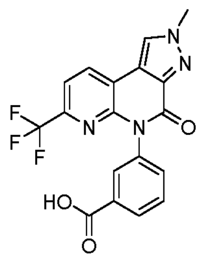 |
| B226 | 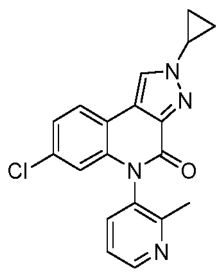 | B227 | 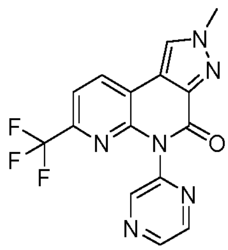 | B228 | 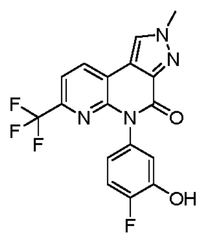 |

(continued)

| | | | | | |
|---|---|---|---|---|---|
| B229 | 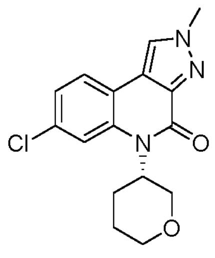 | B230 | 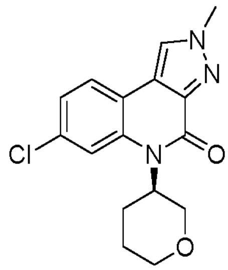 | B231 | 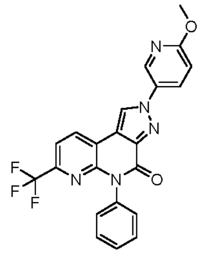 |
| B232 | 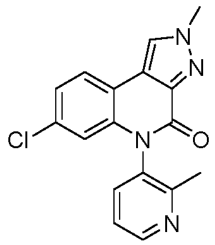 IsomerA | B233 | 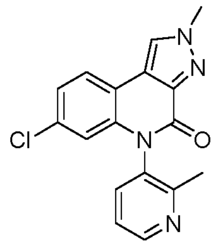 IsomerB | B234 | 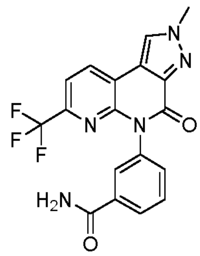 |
| B235 | 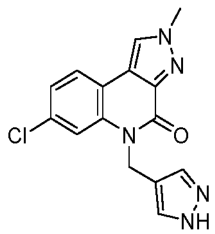 | B236 | 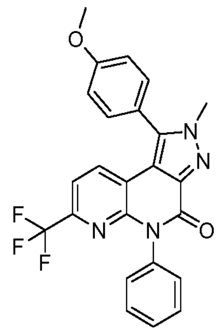 | B237 | 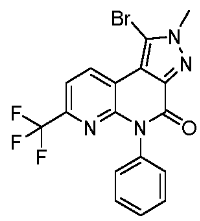 |
| B238 | 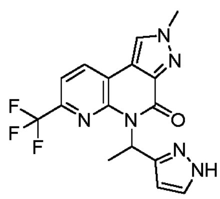 | B239 | 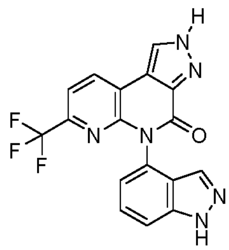 | B240 | 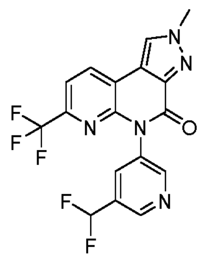 |
| B241 | 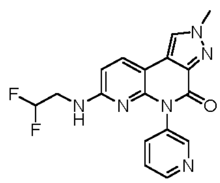 | B242 | 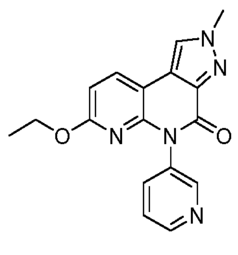 | B243 | 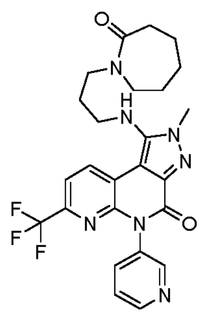 |

(continued)

| | | | | | |
|---|---|---|---|---|---|
| B244 | 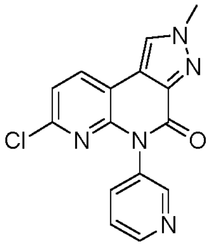 | B245 | 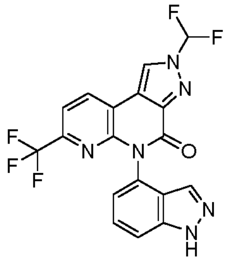 | B246 | 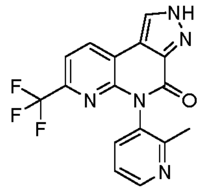 Isomer A |
| B247 | 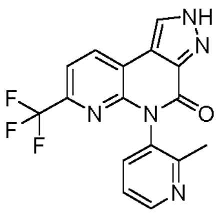 Isomer B | B248 | 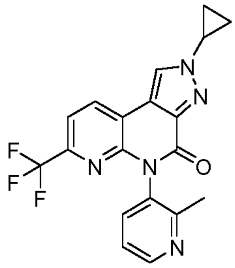 Isomer A | B249 | 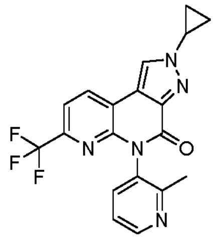 Isomer B |
| B250 | 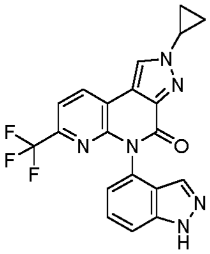 | B251 | 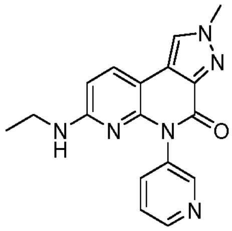 | B252 | 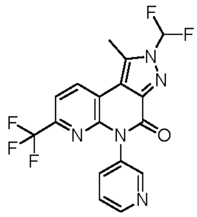 |
| B253 | 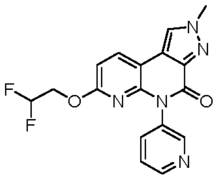 | B254 | 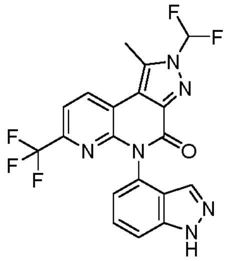 | B255 | 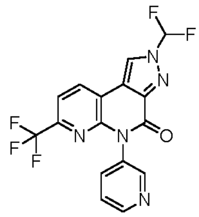 |
| B256 | 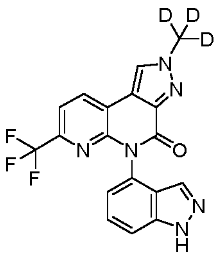 | B257 | 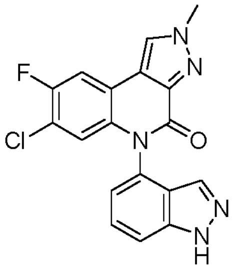 | B258 | 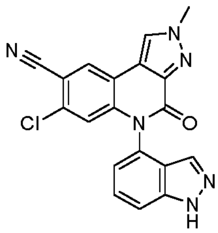 |
| B259 | 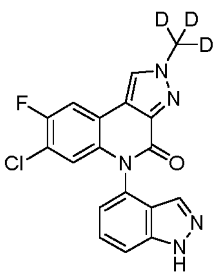 | B260 | 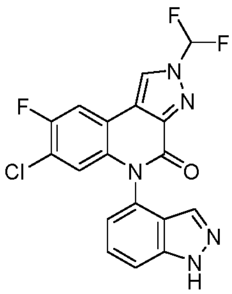 | B261 | 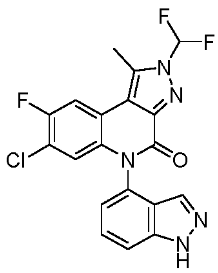 |

(continued)

| | | | | | |
|---|---|---|---|---|---|
| B262 | 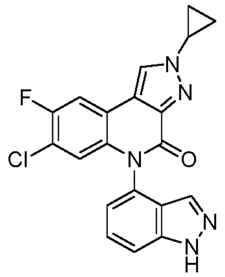 | B263 | 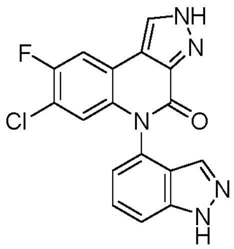 | B264 | 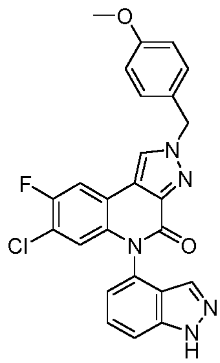 |
| B265 | 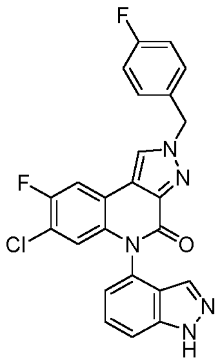 | B266 | 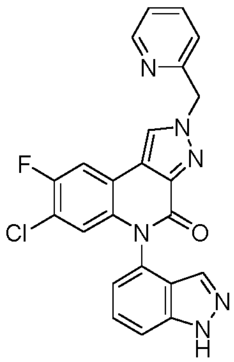 | B267 | 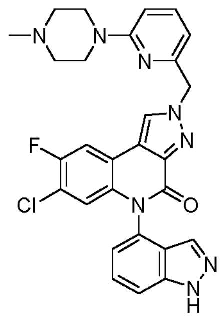 |
| B268 | 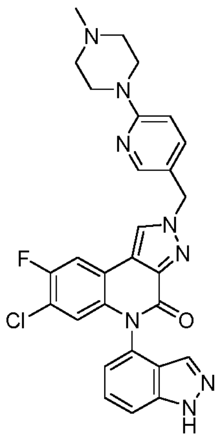 | B269 | 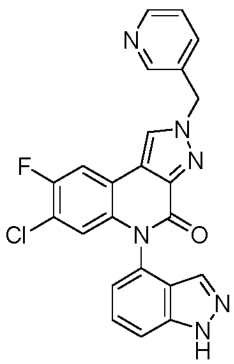 | B270 | 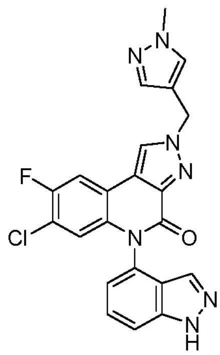 |
| B271 | 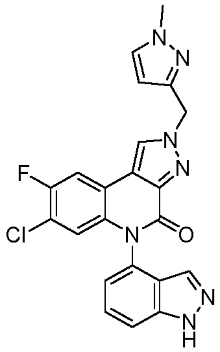 | B272 | 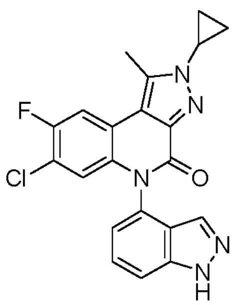 | B273 | 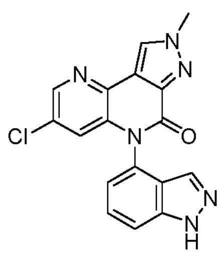 |

(continued)

| | | | | | |
|---|---|---|---|---|---|
| B274 | 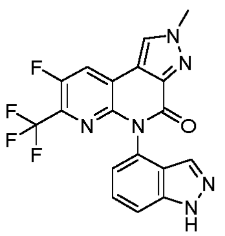 | B275 | 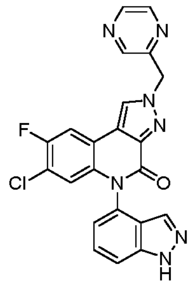 | B276 | 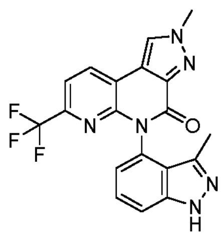 |
| B277 | 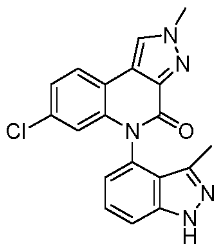 | B278 | 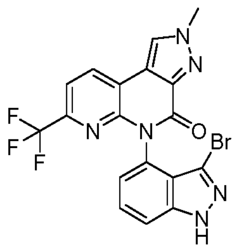 | B279 | 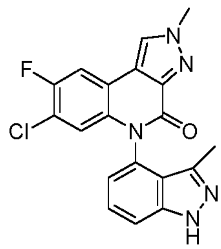 |
| B280 | 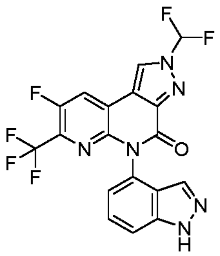 | B281 | 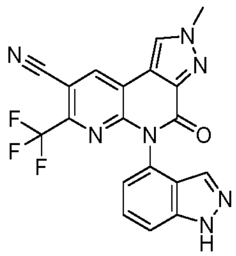 | B282 | 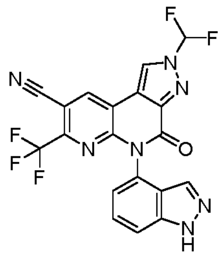 |
| B283 | 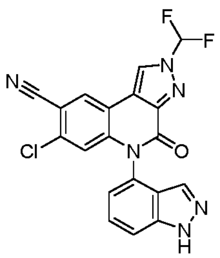 | B284 | 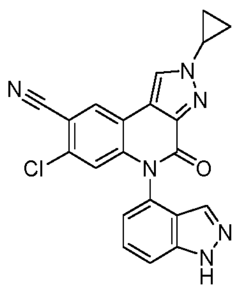 | B285 | 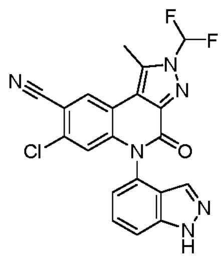 |
| B286 | 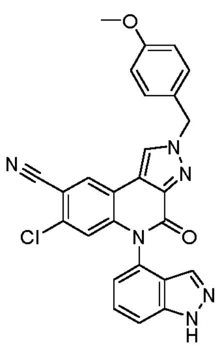 | B287 | 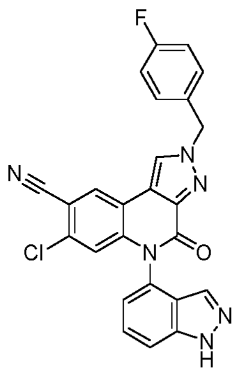 | B288 | 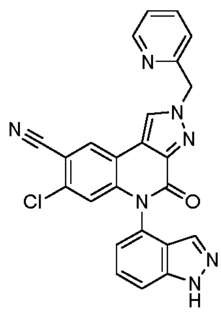 |

(continued)

| | | | | | |
|---|---|---|---|---|---|
| B289 | 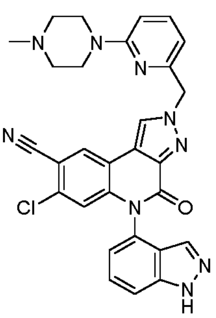 | B290 | 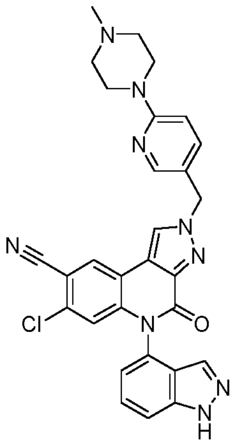 | B291 | 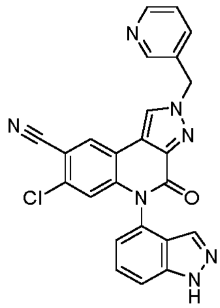 |
| B292 | 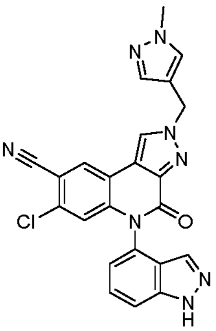 | B293 | 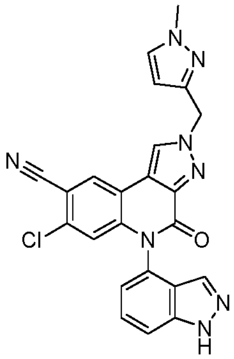 | B294 | 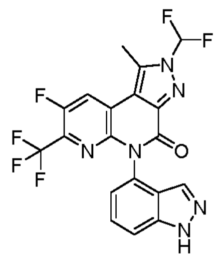 |
| B295 | 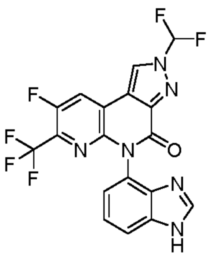 | B296 | 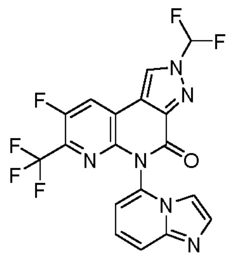 | B297 | 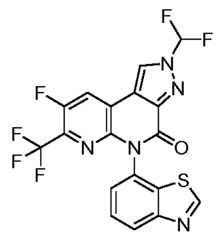 |
| B298 | 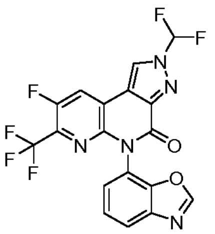 | B299 | 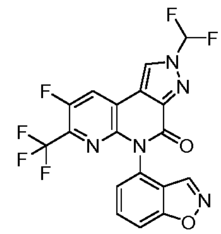 | B300 | 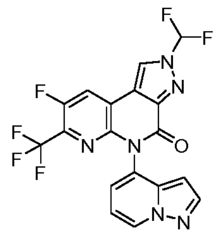 |
| B301 | 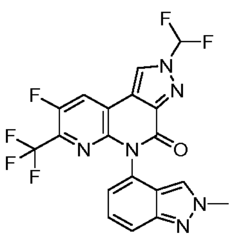 | B302 | 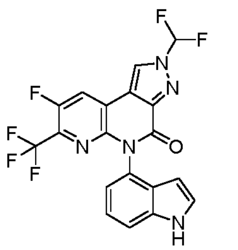 | B303 | 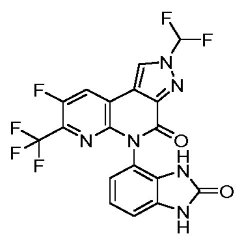 |

(continued)

| | | | | | |
|---|---|---|---|---|---|
| B304 | 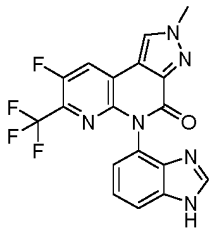 | B305 | 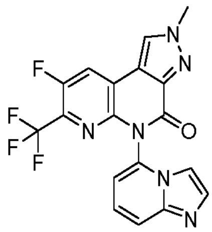 | B306 | 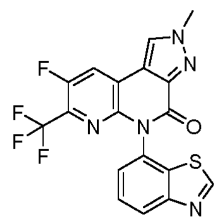 |
| B307 | 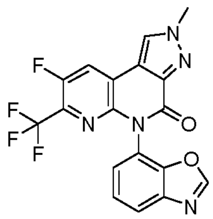 | B308 | 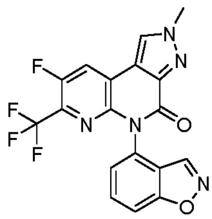 | B309 | 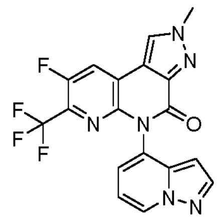 |
| B310 | 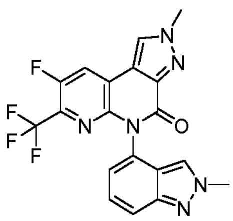 | B311 | 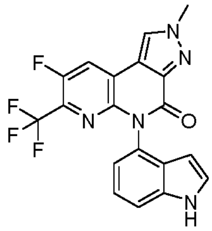 | B312 | 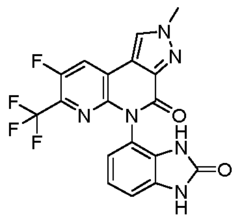 |
| B313 | 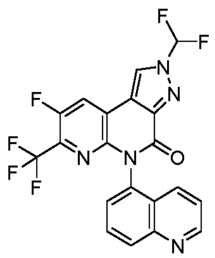 | B314 | 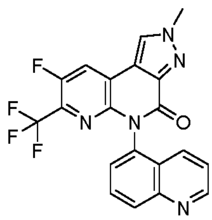 | B315 | 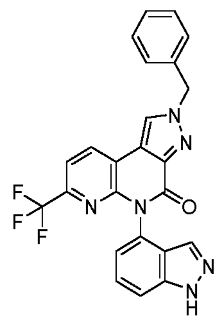 |
| B316 | 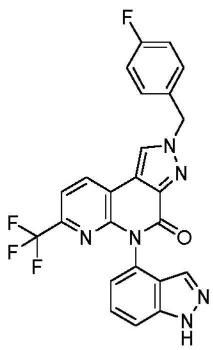 | B317 | 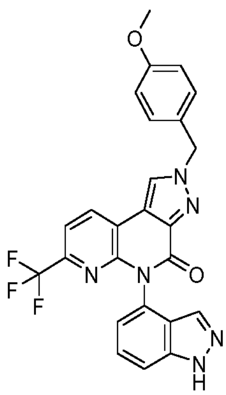 | B318 | 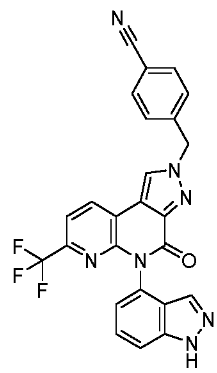 |

(continued)

| | | | | | |
|---|---|---|---|---|---|
| B319 | 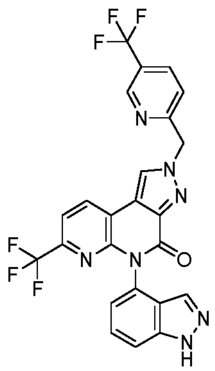 | B320 | 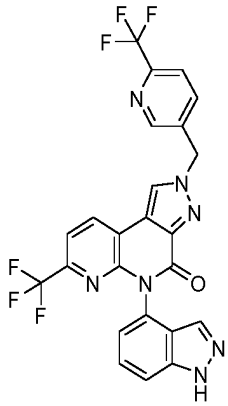 | B321 | 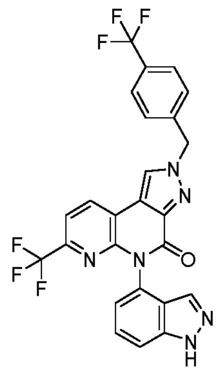 |
| B322 | 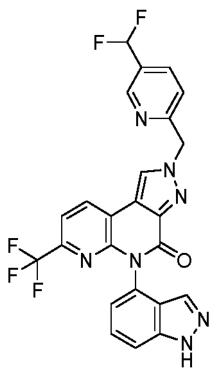 | B323 | 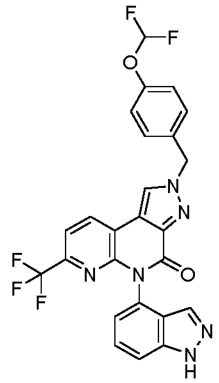 | B324 | 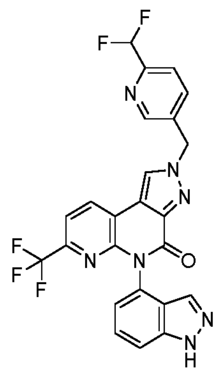 |
| B325 | 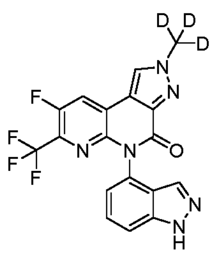 | B326 | 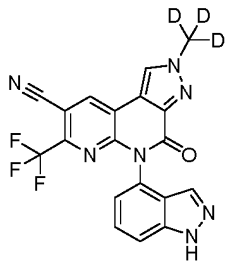 | B327 | 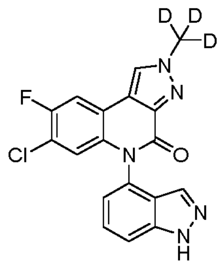 |
| B328 | 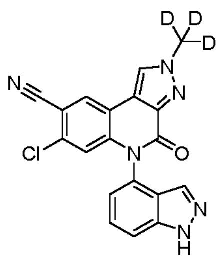 | B329 | 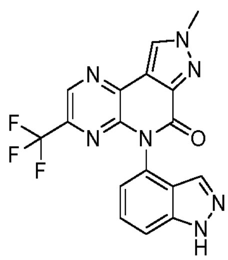 | B330 | 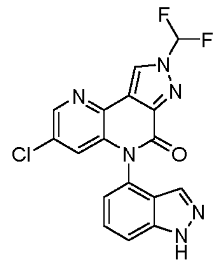 |
| B331 | 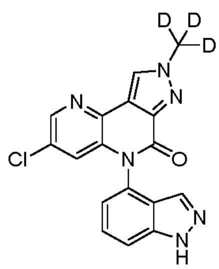 | B332 | 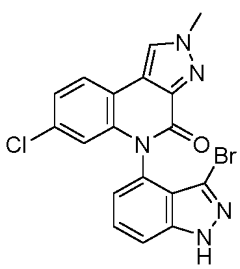 | B333 | 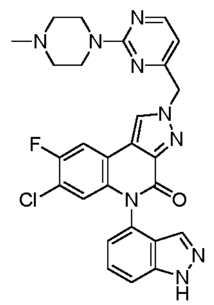 |

(continued)

| | | | | | |
|---|---|---|---|---|---|
| B334 | 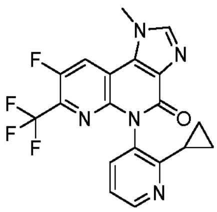 | B335 | 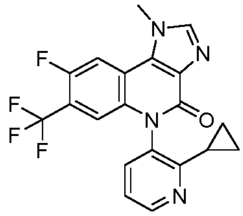 | B336 | 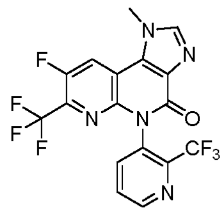 |
| B337 | 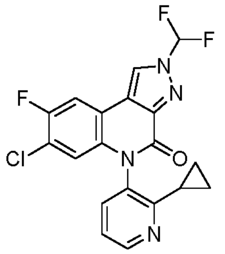 | B338 | 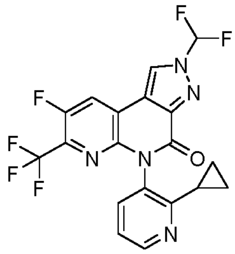 | B339 | 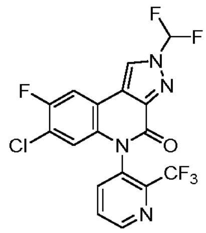 |
| B340 | 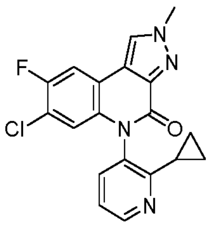 | B341 | 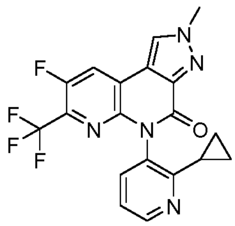 | B342 | 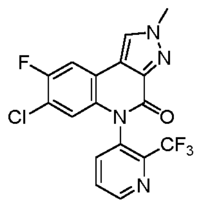 |
| B343 | 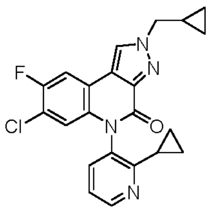 | B344 | 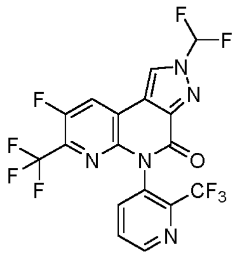 | B345 | 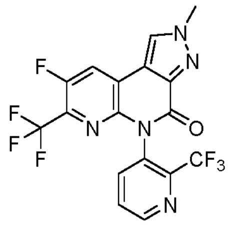 |
| B346 | 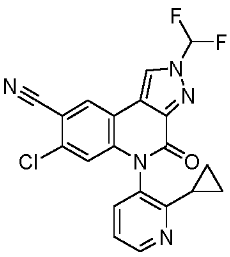 | B347 | 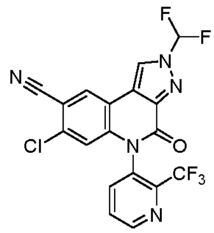 | B348 | 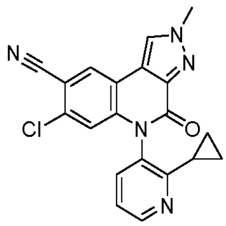 |
| B349 | 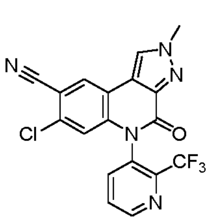 | B350 | 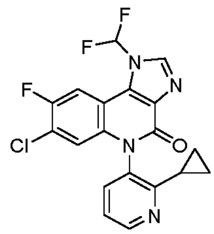 | B351 | 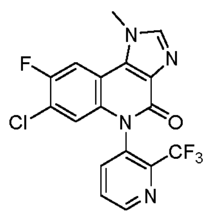 |

(continued)

| | | | | | |
|---|---|---|---|---|---|
| B352 | 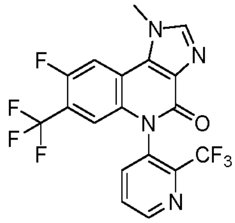 | B353 | 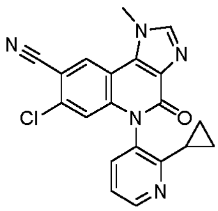 | B354 | 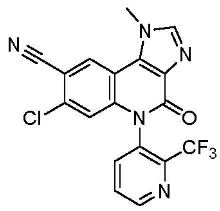 |
| B355 | 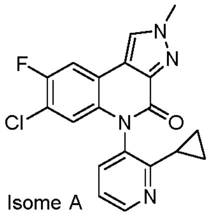 Isome A | B356 | 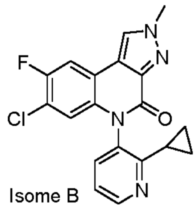 Isome B | B357 | 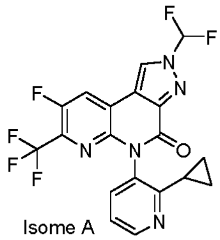 Isome A |
| B358 | 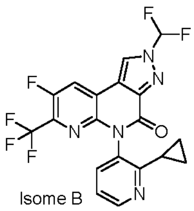 Isome B | B359 | 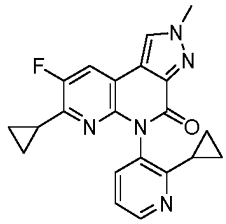 | B360 | 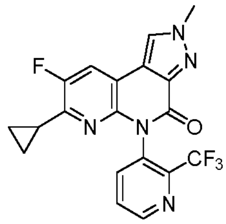 |
| B361 | 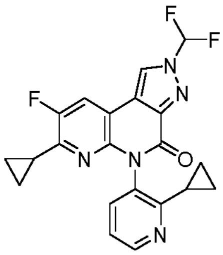 | B362 | 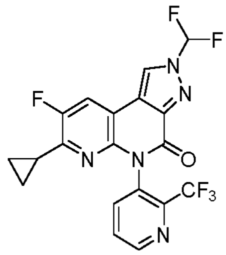 | B363 | 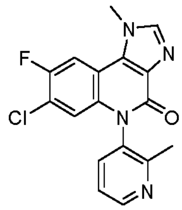 |
| B364 | 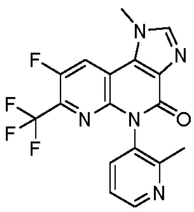 | B365 | 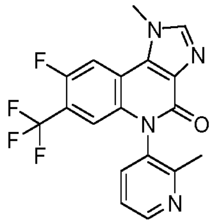 | B366 | 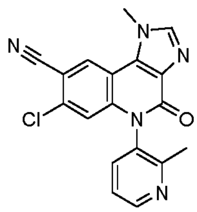 |
| B367 | 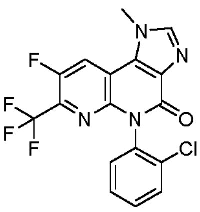 | B368 | 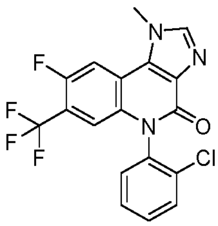 | B369 | 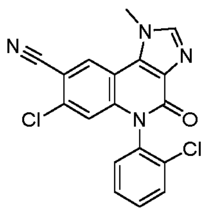 |
| B370 | 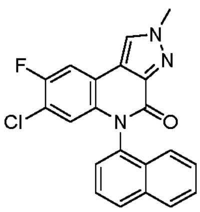 | B371 | 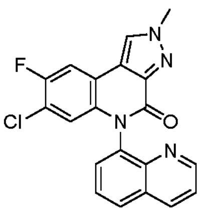 | B372 | 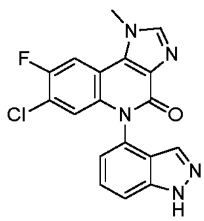 |

(continued)

| | | | | | |
|---|---|---|---|---|---|
| B373 | 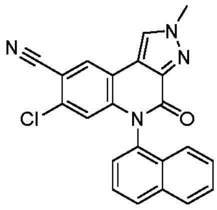 | B374 | 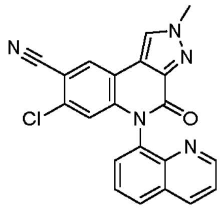 | B375 | 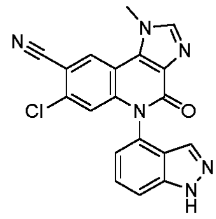 |
| B376 | 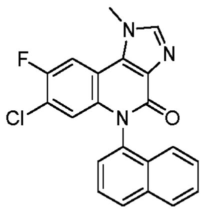 | B377 | 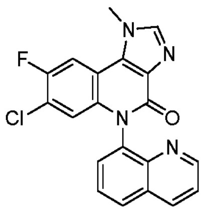 | B378 | 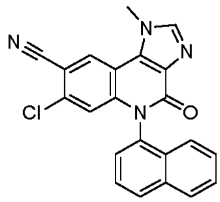 |
| B379 | 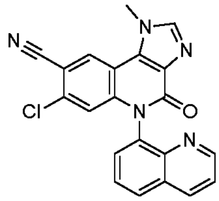 | B380 | 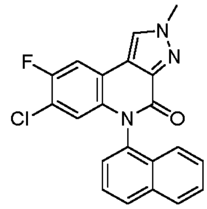 | B381 | 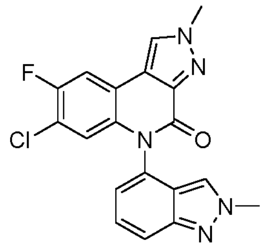 |
| B382 | 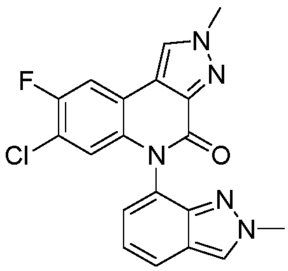 | B383 | 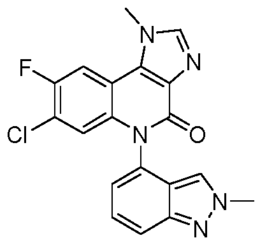 | B384 | 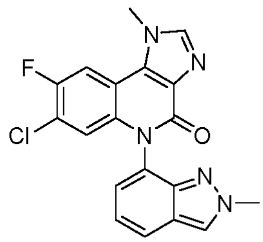 |
| B385 | 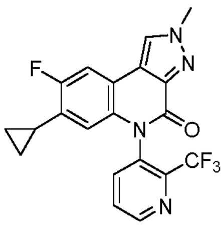 | B386 | 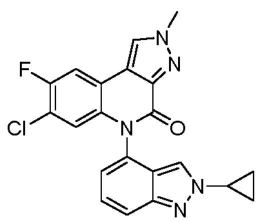 | B387 | 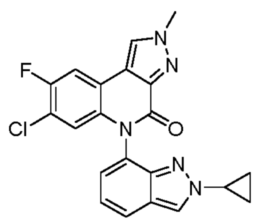 |
| B388 | 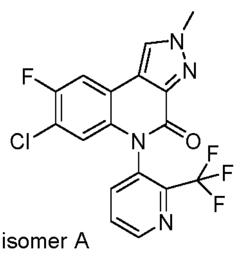 isomer A | B389 | 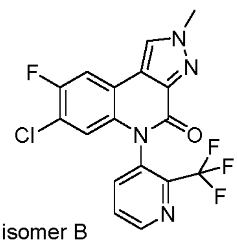 isomer B | B390 | 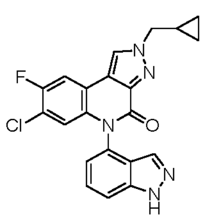 |
| B391 | 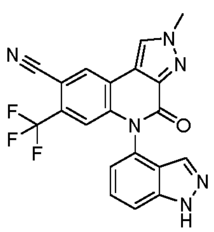 | B392 | 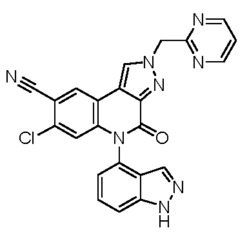 | B393 | 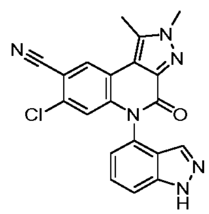 |

(continued)

| | | | | | |
|---|---|---|---|---|---|
| B394 | 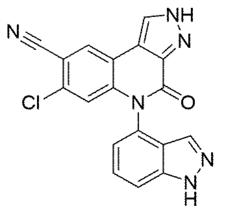 | B395 | 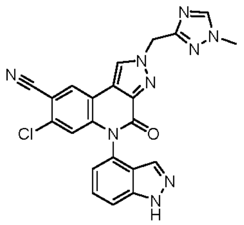 | B396 | 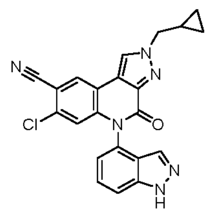 |
| B397 | 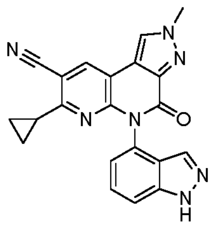 | B398 | 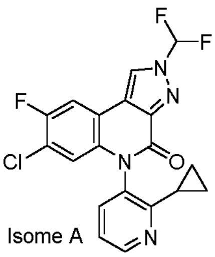 Isome A | B399 | 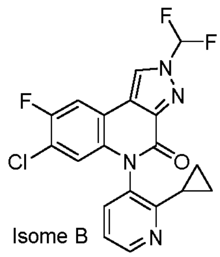 Isome B |
| B400 | 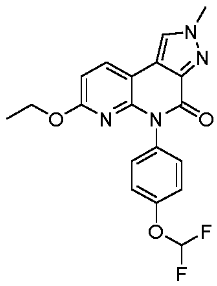 | B401 | 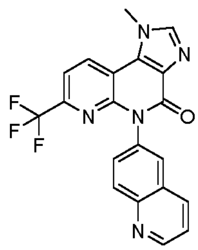 | B402 | 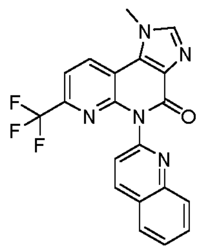 |
| B403 | 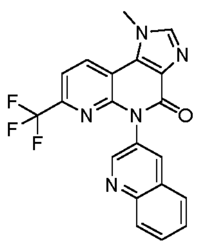 | B404 | 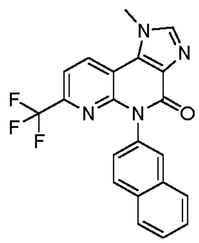 | B405 | 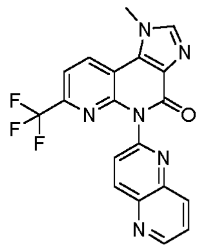 |
| B406 | 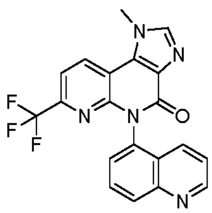 | B407 | 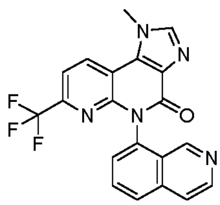 | B408 | 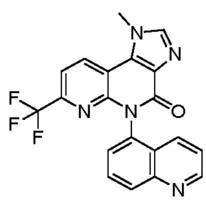 |
| B409 | 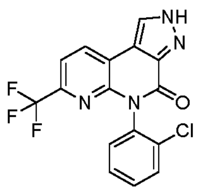 | B410 | 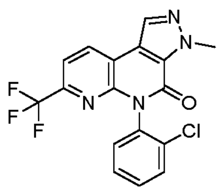 | B411 | 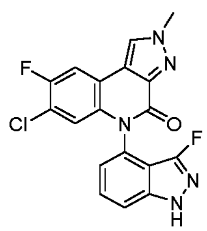 |

(continued)

| | | | | | |
|---|---|---|---|---|---|
| B412 | 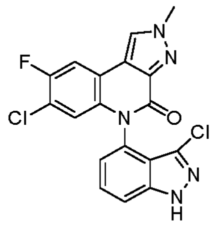 | B413 | 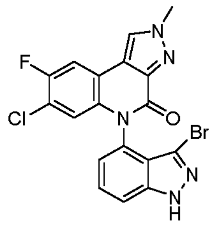 | B414 | 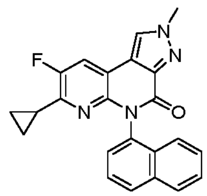 |
| B415 | 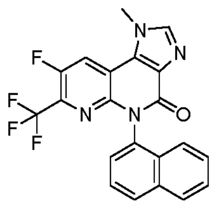 | B416 | 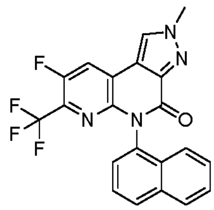 | B417 | 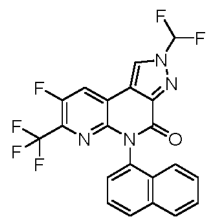 |
| B418 | 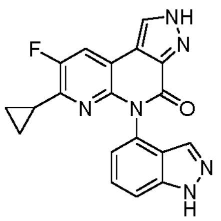 | B419 | 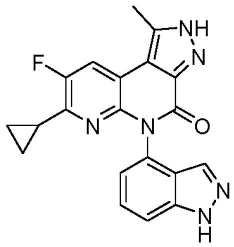 | B420 | 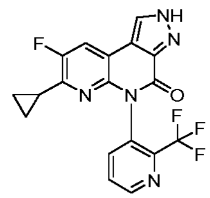 |
| B421 | 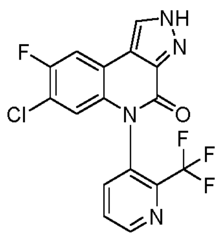 | B422 | 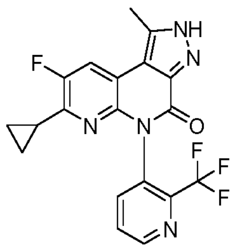 | B423 | 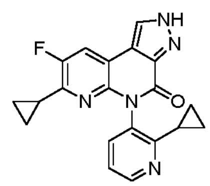 |
| B424 | 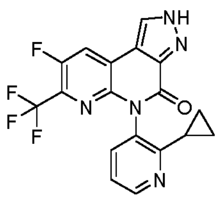 | B425 | 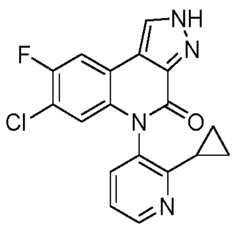 | B427 | 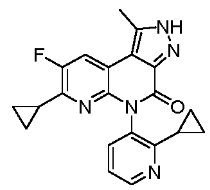 |
| B428 | 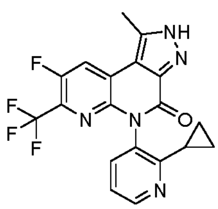 | B429 | 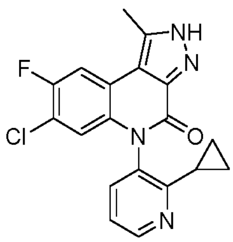 | B430 | 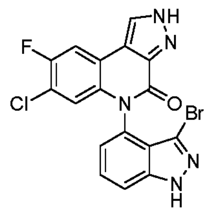 |

In another aspect, the present application provides a preparation method for an exemplary compound, comprising the following routes or steps:

Route 1

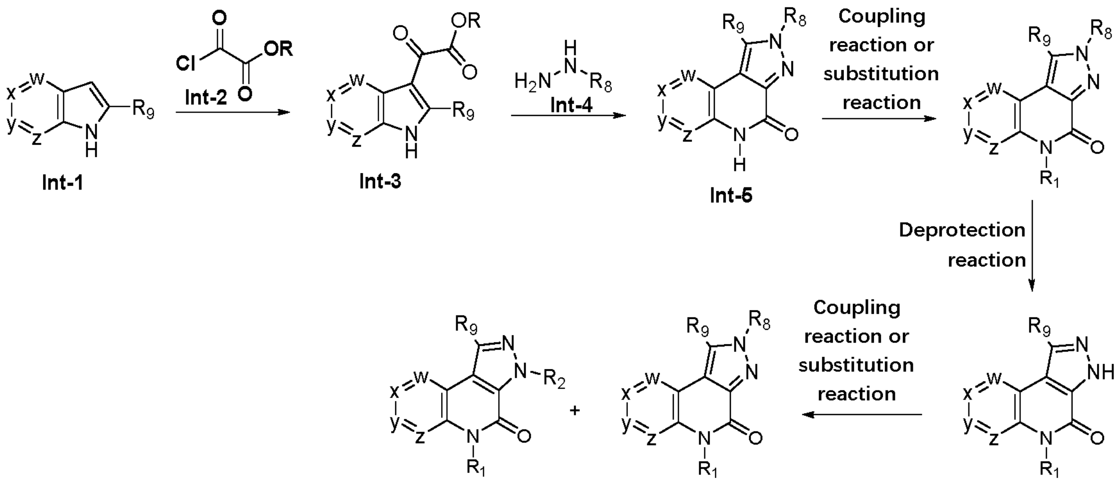


wherein R is alkyl.

Route 2:

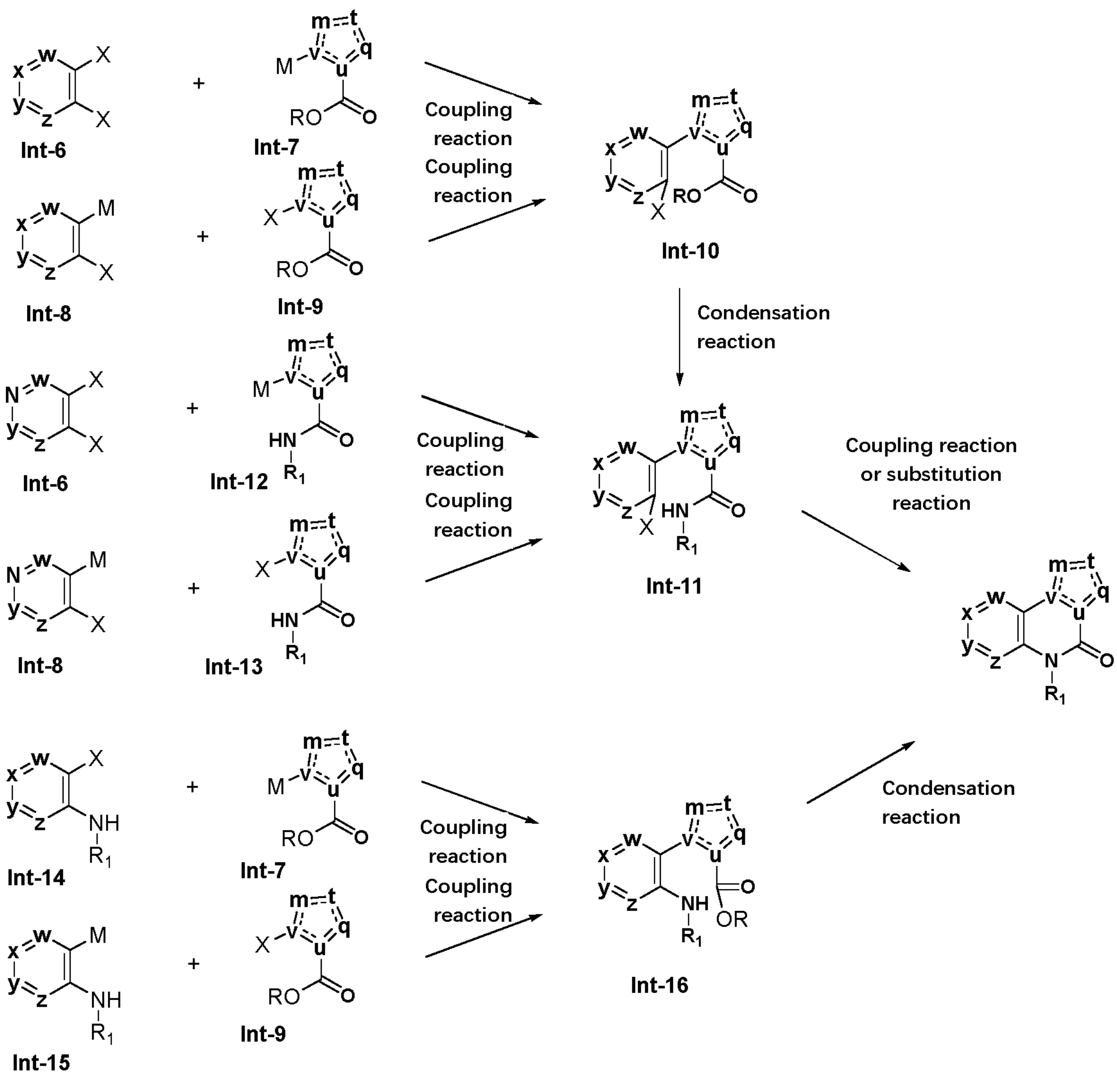


wherein X is halogen, R is hydrogen or alkyl, and M is boric acid, borate, or a metal.

In another aspect, the present application provides a composition comprising a therapeutically effective amount of the compound of formula (I) or the stereoisomer, the tautomer, the solvate, the hydrate, the prodrug, the stable isotopic

derivative, or the pharmaceutically acceptable salt thereof according to any one of the above, and one or more pharmaceutically acceptable carriers, diluents, or excipients.

In another aspect, the present application provides use of the compound of formula (I) or the stereoisomer, the tautomer, the solvate, the hydrate, the prodrug, the stable isotopic derivative, or the pharmaceutically acceptable salt thereof according to any one of the above, or the composition according to any one of the above in inhibiting methionine adenosyltransferase 2A (MAT2A), wherein the inhibitory effect may be *in vitro* inhibition or *in vivo* inhibition.

In another aspect, the present application provides use of the compound of formula (I) or the stereoisomer, the tautomer, the solvate, the hydrate, the prodrug, the stable isotopic derivative, or the pharmaceutically acceptable salt thereof according to any one of the above, or the composition according to any one of the above in preparing a drug for inhibiting methionine adenosyltransferase 2A (MAT2A).

In another aspect, the present application provides use of the compound of formula (I) or the stereoisomer, the tautomer, the solvate, the hydrate, the prodrug, the stable isotopic derivative, or the pharmaceutically acceptable salt thereof according to any one of the above, or the composition according to any one of the above in treating and/or preventing cancer, in particular cancer in which a gene encoding methylthioadenosine phosphorylase (MTAP) is deleted and/or is not fully functional, including but not limited to colon cancer.

In another aspect, the present application provides use of the compound of formula (I) or the stereoisomer, the tautomer, the solvate, the hydrate, the prodrug, the stable isotopic derivative, or the pharmaceutically acceptable salt thereof according to any one of the above, or the composition according to any one of the above in preparing a drug for treating and/or preventing cancer, in particular cancer in which a gene encoding methylthioadenosine phosphorylase (MTAP) is deleted and/or is not fully functional.

In another aspect, the present application provides a method for treating and/or preventing cancer, comprising administering to a patient a therapeutically effective amount of the compound of formula (I) or the stereoisomer, the tautomer, the solvate, the hydrate, the prodrug, the stable isotopic derivative, or the pharmaceutically acceptable salt thereof according to any one of the above, or the composition according to any one of the above, wherein the cancer includes those in which a gene encoding methylthioadenosine phosphorylase (MTAP) is deleted and/or is not fully functional.

Example 1: Synthesis of Compound B2

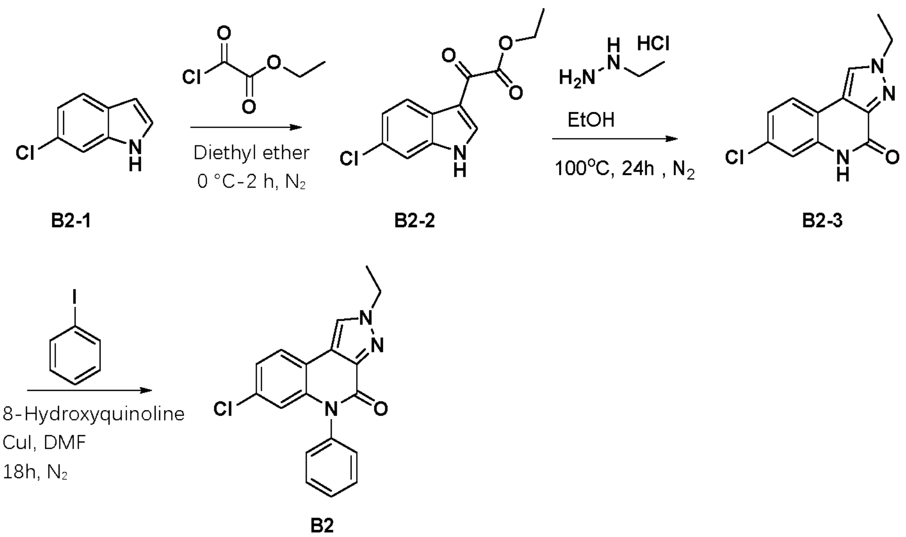


B2-1 B2-2 B2-3

B2

1) Synthesis of intermediate **B2-2**:

**B2-1** (2 g, 13.19 mmol) and pyridine (1.36 g, 17.15 mmol) were dissolved in diethyl ether (40 mL) under nitrogen atmosphere, ethyl chloroglyoxylate (2.16 g, 15.83 mmol) was added at 0 °C, and the mixture was stirred for 2 h. The formed yellow precipitate was filtered off, washed with water, and dried to give a yellow solid (1.5 g, 45%).

2) Synthesis of intermediate **B2-3**:

**B2-2** (1.0 g, 3.97 mmol), ethylhydrazine hydrochloride (0.50 g, 5.16 mmol), and acetic acid (2.38 g, 39.7 mmol)

were dispersed in ethanol (20 mL) under nitrogen atmosphere, and the mixture was heated to 100 °C and stirred for reaction for 24 h. The mixture was cooled to room temperature, concentrated under reduced pressure, and separated by column chromatography to give **B2-3** (0.3 g, 30.5%) as a brown solid.

3) Synthesis of product B2:

**B2-3** (0.2 g, 0.81 mmol), iodobenzene (0.20 g, 0.97 mmol), cuprous iodide (0.031 g, 0.16 mmol), 8-hydroxyquinoline (0.047 g, 0.32 mmol), and potassium phosphate (0.34 g, 1.62 mmol) were dispersed in DMF (10 mL) under nitrogen atmosphere, and the mixture was heated to 110 °C and reacted for 18 h. The mixture was cooled to room temperature, diluted with water, and extracted with ethyl acetate. The organic phase was washed twice with saturated ammonium chloride, dried over anhydrous sodium sulfate, and filtered. The filtrate was concentrated to dryness by rotary evaporation, and purified by column chromatography and preparative thin-layer chromatography to give **B2** (0.07 g, 27%) as a pale pink solid.

Example 2: Synthesis of Compound **B3**

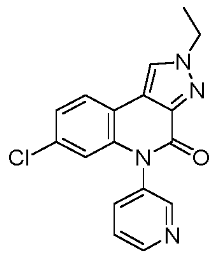

Compound **B3** was synthesized according to a synthetic route similar to that of Example 1 with iodobenzene replaced by 3-iodopyridine and potassium phosphate replaced by potassium *tert*-butoxide in step 3.

Example 3: Synthesis of Compound **B4**

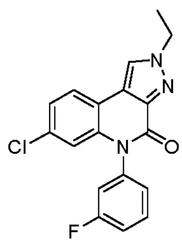

Compound **B4** was synthesized according to a synthetic route similar to that of Example 1 with iodobenzene replaced by 3-fluoroiodobenzene and potassium phosphate replaced by potassium *tert*-butoxide in step 3.

Example 4: Synthesis of Compound **B5**

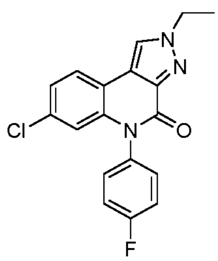

Compound **B5** was synthesized according to a synthetic route similar to that of Example 1 with iodobenzene replaced by 4-fluoroiodobenzene and potassium phosphate replaced by potassium *tert*-butoxide in step 3.

Example 5: Synthesis of Compound **B6**

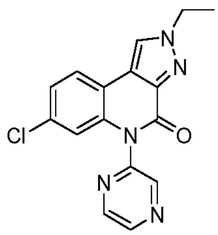

Compound **B6** was synthesized according to a synthetic route similar to that of Example 1 with iodobenzene replaced by 2-iodopyrazine and potassium phosphate replaced by potassium *tert*-butoxide in step 3.

Example 6: Synthesis of Compound **B7**

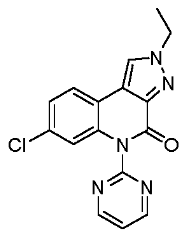

Compound **B7** was synthesized according to a synthetic route similar to that of Example 1 with iodobenzene replaced by 2-iodopyrimidine and potassium phosphate replaced by potassium *tert*-butoxide in step 3.

Example 7: Synthesis of Compound **B8**

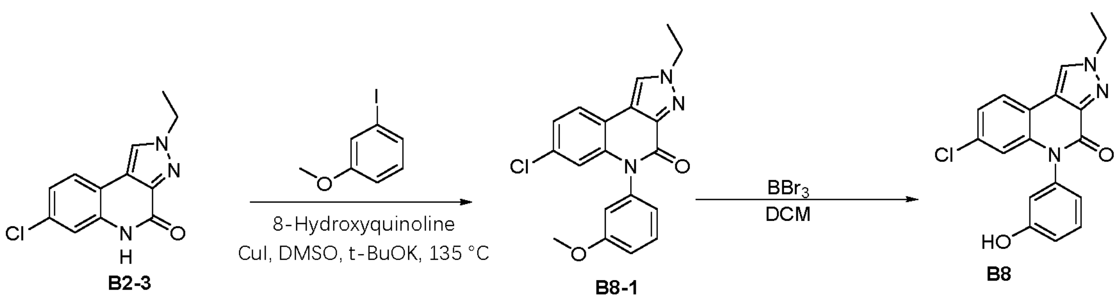

**B2-3** **B8-1** **B8**

1) Synthesis of intermediate **B8-1**

**B2-3** (100 mg, 0.40 mmol), 3-methoxyiodobenzene (377.9 mg, 1.61 mmol), cuprous iodide (76.71 mg, 0.40 mmol), 8-hydroxyquinoline (29.03 mg, 0.20 mmol), and potassium *tert*-butoxide (112.21 mg, 1.01 mmol) were dispersed in DMSO (15 mL) under nitrogen atmosphere, and the mixture was heated to 135 °C and reacted overnight. The mixture was cooled to room temperature, diluted with water, and extracted with ethyl acetate. The organic phase was washed with saturated brine, dried over anhydrous sodium sulfate, and filtered. The filtrate was concentrated to dryness by rotary evaporation, and purified by column chromatography and preparative thin-layer chromatography to give **B8-1** (96.5 mg, 68%) as a brown solid.

2) Synthesis of product B8:

**B8-1** (60 mg, 0.17 mmol) was dissolved in dichloromethane (10 mL), a 1.0 M solution of boron tribromide in dichloromethane (0.5 mL, 0.5 mmol) was added dropwise under nitrogen atmosphere at -78 °C, and the mixture was warmed to room temperature and reacted for 3 h. The mixture was quenched with 1 mL of methanol, extracted 3 times with ethyl acetate, washed with saturated brine, dried over anhydrous sodium sulfate, and filtered. The filtrate was concentrated to dryness by rotary evaporation, purified by preparative HPLC, and separated to give **B8** (32.4 mg, 56%) as a white solid.

Example 8: Synthesis of Compound **B9**

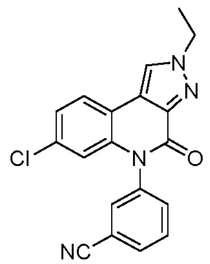

Compound **B9** was synthesized according to a synthetic route similar to that of Example 1 with iodobenzene replaced by 3-iodobenzonitrile and potassium phosphate replaced by potassium *tert*-butoxide in step 3.

Example 9: Synthesis of Compound **B10**

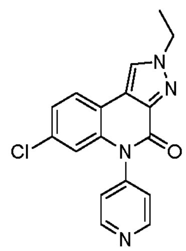

Compound **B10** was synthesized according to a synthetic route similar to that of Example 1 with iodobenzene replaced by 4-iodopyridine and potassium phosphate replaced by potassium *tert*-butoxide in step 3.

Example 10: Synthesis of Compound **B11**

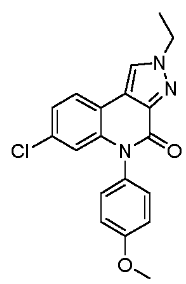

Compound **B11** was synthesized according to a synthetic route similar to that of Example 1 with iodobenzene replaced by 4-iodoanisole and potassium phosphate replaced by potassium *tert*-butoxide in step 3.

Example 11: Synthesis of Compound **B12**

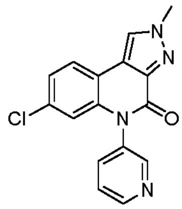

Compound **B12** was synthesized according to a synthetic route similar to that of Example 1 with ethylhydrazine hydrochloride replaced by methylhydrazine sulfate in step 2 and iodobenzene replaced by 3-iodopyridine in step 3.

Example 12: Synthesis of Compound **B28**

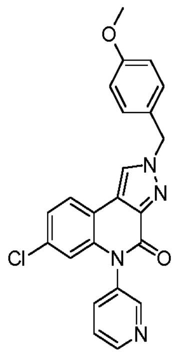

Compound **B28** was synthesized according to a synthetic route similar to that of Example 1 with ethylhydrazine hydrochloride replaced by *p*-methoxybenzylhydrazine in step 2 and iodobenzene replaced by 3-iodopyridine in step 3.

Example 13: Synthesis of Compound **B13**

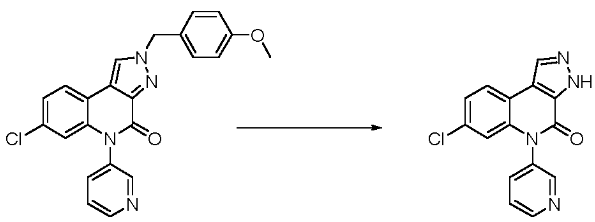

Compound **B28** (1.0 g, 2.4 mmol) was mixed with trifluoroacetic acid (20 mL), and the mixture was stirred for reaction at 40 °C for 16 h. After the reaction was completed, the reaction solution was concentrated under reduced pressure, adjusted to pH = 9 with a saturated sodium bicarbonate solution, and extracted with ethyl acetate (50 mL × 3). The combined organic phases were washed with saturated brine, dried over anhydrous sodium sulfate, filtered, and concentrated under reduced pressure. The residue was purified by silica gel column chromatography (dichloromethane:methanol = 20: 1) to give compound **B13** (600 mg, 65%) as a yellow solid.

Example 14: Synthesis of Compound **B14**

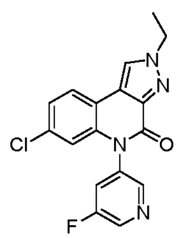

Compound **B14** was synthesized according to a synthetic route similar to that of Example 1 with iodobenzene replaced by 5-fluoro-3-iodopyridine and potassium phosphate replaced by potassium *tert*-butoxide in step 3.

Example 15: Synthesis of Compound **B15**

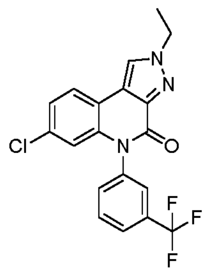

Compound **B15** was synthesized according to a synthetic route similar to that of Example 1 with iodobenzene replaced by 3-iodotrifluorotoluene and potassium phosphate replaced by potassium *tert*-butoxide in step 3.

Example 16: Synthesis of Compound **B16**

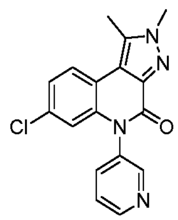

Compound **B16** was synthesized according to a synthetic route similar to that of Example 1 with **B2-1** replaced by

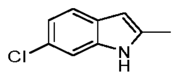

in step 1, ethylhydrazine hydrochloride replaced by methylhydrazine sulfate in step 2, and iodobenzene replaced by 3-iodopyridine in step 3.

Example 17: Synthesis of Compound **B17**

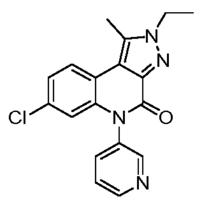

Compound **B17** was synthesized according to a synthetic route similar to that of Example 1 with **B2-1** replaced by

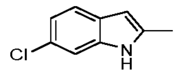

in step 1 and iodobenzene replaced by 3-iodopyridine in step 3.

Example 18: Synthesis of Compound **B18**

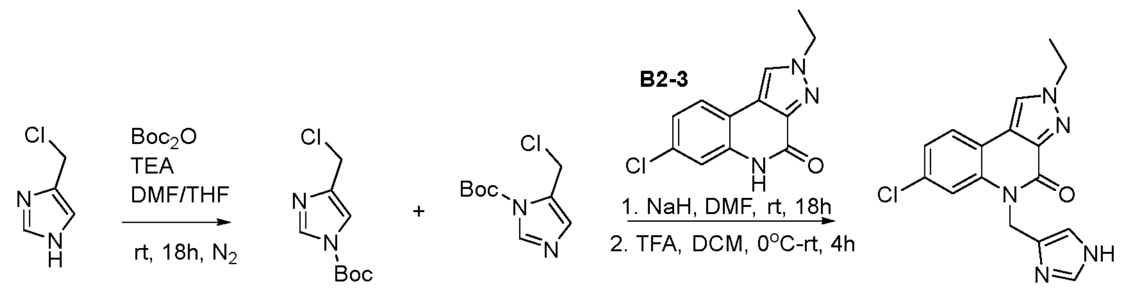


**B18-1** **B18-2A** and **B18-2B** **B18**

Synthesis of intermediate B18-2

B18-1 (0.2 g, 1.31 mmol) was dissolved in DMF (2 mL) under an ice-water bath under nitrogen atmosphere, a solution of triethylamine (0.15 g, 1.44 mmol) in DMF (1.0 mL) and a solution of di-*tert*-butyl dicarbonate (0.31 g,1.44 mmol) in THF (1.5 mL) were slowly added, and the mixture was warmed to room temperature and reacted overnight. The mixture was diluted with water, and extracted twice with ethyl acetate. The organic phases were combined, washed with water and saturated brine, dried over anhydrous sodium sulfate, and filtered. The filtrate was concentrated to dryness by rotary evaporation, and purified by column chromatography to separately give compounds **B18-2A** (110 mg, 39%) and **B18-2B** (90 mg, 32%). **B18-2A**: $^1H$ NMR (400 MHz, Chloroform-*d*) δ 8.11 (s, 1H), 7.45 (s, 1H), 4.61 (s, 2H), 1.68 (s, 9H). **B18-2B** $^1H$ NMR (400 MHz, Chloroform-*d*) δ 8.11 (s, 1H), 7.45 (s, 1H), 4.61 (s, 2H), 1.68 (s, 9H).

Synthesis of product B18

B2-3 (100 mg, 0.40 mmol) was dissolved in DMF (3 mL) under an ice-water bath under nitrogen atmosphere, NaH (20.8 mg, 0.52 mmol, 60% purity) was added, and the mixture was stirred at room temperature for 30 min. B18-2A(87 mg, 0.40 mmol) was dissolved in DMF (2 mL), and the mixture was stirred at room temperature overnight. The mixture was diluted with water, and extracted with ethyl acetate. The organic phase was washed with saturated ammonium chloride, dried over anhydrous sodium sulfate, and filtered. The filtrate was concentrated to dryness by rotary evaporation, and purified by column chromatography to give a pale yellow solid (125 mg, 73%). The resulting solid (100 mg, 0.23 mmol) was dissolved in dichloromethane (2 mL), trifluoroacetic acid (1.0 mL, 6.71 mmol) was added under an ice-water bath, and the mixture was stirred at room temperature for 4 h. The mixture was concentrated under reduced pressure and purified by preparative HPLC to give a white solid (43 mg, 57%).

Example 19: Synthesis of Compound B19

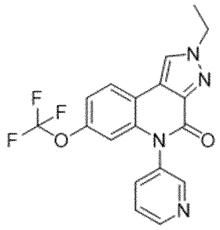

Compound **B19** was synthesized according to a synthetic route similar to that of Example 1 with **B2-1** replaced by

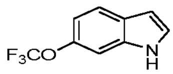

in step 1 and iodobenzene replaced by 3-iodopyridine in step 3.

Example 20: Synthesis of Compound B20

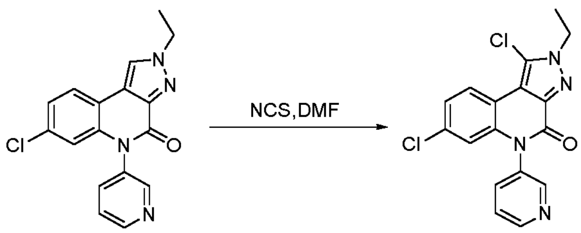


**B3** **B20**

Compound **B3** (100 mg, 0.31 mmol) and *N*-chlorosuccinimide (41.04 mg, 0.31 mmol) were separately added to *N-N* dimethylformamide (6 mL), and the mixture was heated to 60 °C and stirred for reaction overnight. The reaction solution was cooled to room temperature, poured into water, extracted with ethyl acetate, and washed with saturated brine. The separated organic phase was dried over anhydrous sodium sulfate, filtered, and concentrated. The concentration residue was purified by preparative high performance liquid chromatography to give compound **B20** (57.6 mg, 52%) as an off-white solid.

Example 21: Synthesis of Compounds **B21** and **B22**

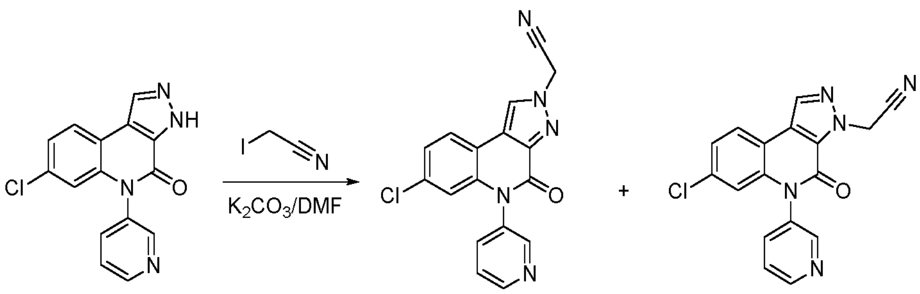


**B13** **B21** **B22**

Potassium carbonate (465 mg, 3.37 mmol) was added to compound **B13** (400 mg, 1.35 mmol) and iodoacetonitrile (270 mg, 1.62 mmol) in *N*,*N*-dimethylformamide (10 mL) at room temperature, and the mixture was stirred at room temperature for reaction overnight. The reaction solution was quenched with water (20 mL) and extracted with ethyl acetate (30 mL × 3). The combined organic phases were washed with saturated brine, dried over anhydrous sodium sulfate, filtered, and concentrated under reduced pressure. The residue was purified by preparative high performance liquid chromatography to separately give compound **B21** (4.1 mg, 0.8%) as a white solid and compound **B22** (84.5 mg,

17%) as a white solid.

Example 22: Synthesis of Compound **B23**

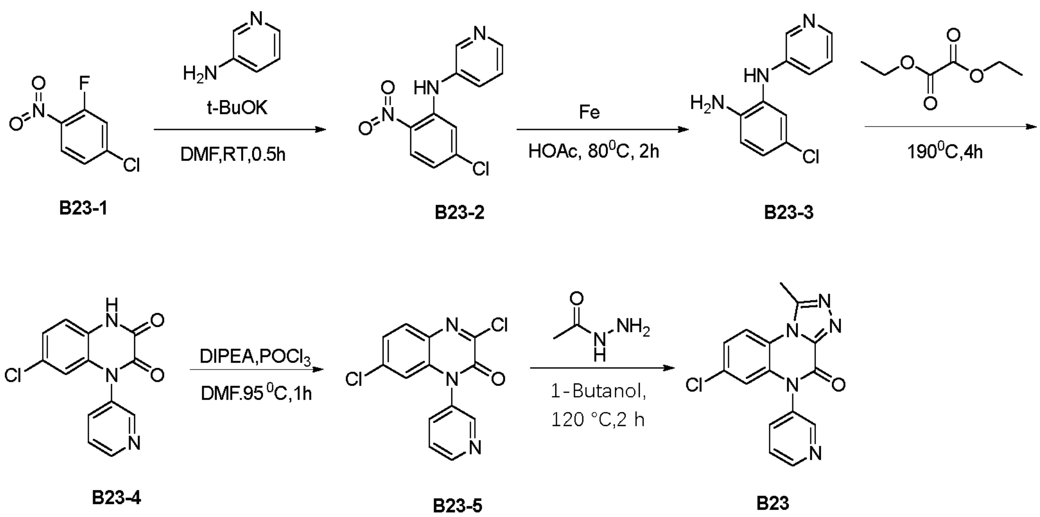


B23-1 B23-2 B23-3

B23-4 B23-5 B23

1) Synthesis of intermediate **B23-2**:

**B23-1** (5 g, 32.15 mmol) and 3-aminopyridine (4.13 g, 32.15 mmol) were dissolved in *N,N*-dimethylformamide (50 mL), potassium *tert*-butoxide (7.55 g, 67.5 mmol) was added, and the mixture was reacted at room temperature for 30 min. Water was added, and a solid was precipitated. The mixture was filtered under vacuum. The filter cake was sequentially washed with water, isopropanol, and isopropyl ether, and dried to give a black solid (4.8 g, 60%).

2) Synthesis of intermediate **B23-3**:

Reduced iron powder (5.4 g, 96.4 mmol) was added to a solution of **B23-2** (4.8 g, 19.3 mmol) in acetic acid (50 mL). The mixture was warmed to 80 °C and stirred for 2 h. The mixture was cooled to room temperature, adjusted to pH = 8 with saturated $NaHCO_3$, and extracted 2 times with ethyl acetate. The organic phases were combined, dried, and concentrated under reduced pressure to give **B23-3** (4 g, 96%).

3) Synthesis of intermediate **B23-4**:

**B23-3** (4.0 g, 18.2 mmol) and diethyl oxalate (20 mL) were mixed, heated, and refluxed overnight. The reaction was completed as monitored by thin-layer chromatography. The mixture was cooled to room temperature and filtered. The filter cake was washed with ethanol and dried to give **B23-4** (3 g, 60%).

4) Synthesis of intermediate **B23-5**:

**B23-4** (1 g, 3.65 mmol) was dissolved in DMF (50 mL), DIPEA (1 g, 7.3 mmol) and $POCl_3$ (1.8 g, 11.68 mmol) were added, and the mixture was warmed to 95 °C and reacted for 1 h. The mixture was cooled to room temperature, poured slowly into ice water, and extracted twice with dichloromethane. The organic phases were combined, dried over anhydrous sodium sulfate, and filtered. The filtrate was concentrated to dryness by rotary evaporation to give **B23-5** (800 mg, 74.7%) as a yellow solid.

5) Synthesis of product **B23**:

**B23-5** (0.2 g, 0.68 mmol) was dissolved in *n*-butanol (10 mL), acethydrazide (0.06 g,0.81 mmol) was added, and the mixture was warmed to 120 °C and reacted for 2 h. The mixture was cooled to room temperature, diluted with water, and extracted with ethyl acetate. The organic phase was washed with saturated brine, dried over anhydrous sodium sulfate, and filtered. The filtrate was concentrated to dryness by rotary evaporation, and purified by preparative thin-layer

chromatography to give **B23** (31 mg, 15%) as a white solid.

Example 23: Synthesis of Compound **B29**

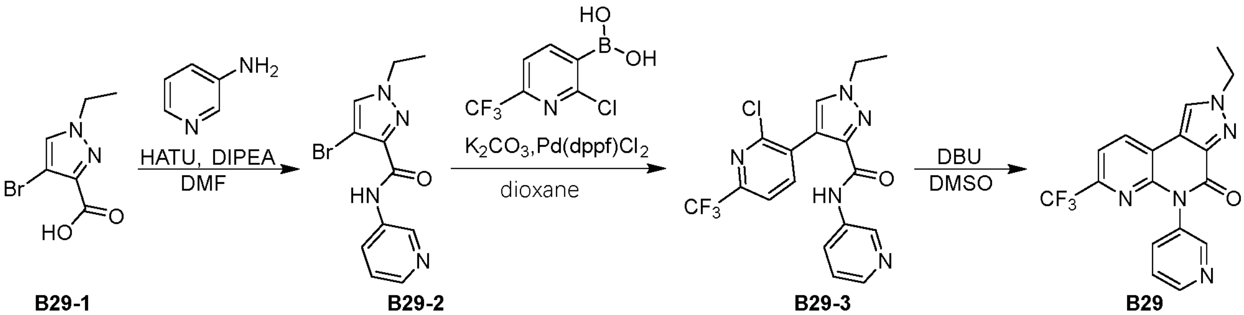


B29-1 B29-2 B29-3 B29

1) Synthesis of intermediate **B29-2**:

**B29-1** (500 mg, 2.2 mmol) and 3-aminopyridine (244 mg, 2.6 mmol) were dissolved in *N,N*-dimethylformamide (10 mL), diisopropylethylamine (568 mg, 4.4 mmol) and HATU (1.2 g, 3.3 mmol) were added, and the mixture was reacted at room temperature for 30 min. The mixture was directly concentrated to dryness by rotary evaporation, and purified by column chromatography to give **B29-2** (450 mg, 70%).

2) Synthesis of intermediate **B29-3**:

**B29-2** (450 mg, 1.5 mmol) and 2-chloro-6-trifluoromethylpyridine-3-boronic acid (360 mg, 1.6 mmol) were dissolved in 1,4-dioxane (10 mL), and potassium carbonate (621 mg, 4.5 mmol) and Pd(dppf)$Cl_2$ (110 mg, 0.15 mmol) were added. The mixture was reacted at 100 °C for 2 h under nitrogen atmosphere. The mixture was directly concentrated to dryness by rotary evaporation, and purified by column chromatography to give **B29-3** (150 mg, 25%) as a yellow solid.

3) Synthesis of product **B29**:

**B29-3** (100 mg, 0.25 mmol) was dissolved in dimethyl sulfoxide (5 mL), DBU (76 mg, 0.05 mmol) was added, and the mixture was warmed to 140 °C and reacted overnight. The reaction solution was separated by reversed-phase preparative chromatography to give **B29** (23 mg, 25%) as a brown solid.

Example 24: Synthesis of Compound **B30**

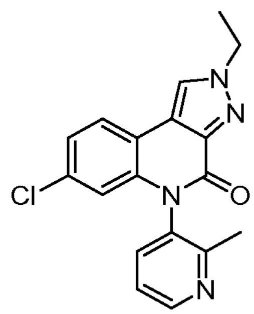

Compound **B30** was obtained according to a synthetic route similar to that of Example 23 with 3-aminopyridine replaced by 2-methyl-3-aminopyridine in step 1 and 2-chloro-6-trifluoromethylpyridine-3-boronic acid replaced by 2-fluoro-4-chlorophenylboronic acid in step 2.

Example 25: Synthesis of Compound **B47**

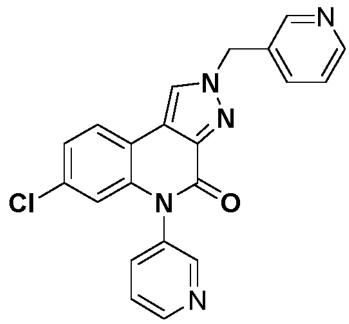

Compound **B47** was obtained according to a synthetic route similar to that of Example 1 with ethylhydrazine replaced by 3-pyridinylmethylhydrazine in step 2 and iodobenzene replaced by 3-iodopyridine in step 3.

Example 26: Synthesis of Compound **B60**

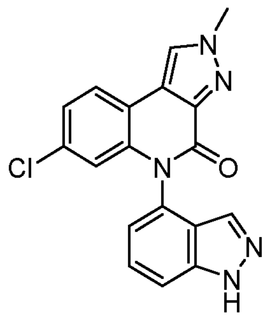

Compound **B60** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by

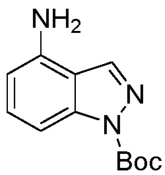

in step 1 and 2-chloro-6-trifluoromethylpyridine-3-phenylboronic acid replaced by 2-fluoro-4-chlorophenylboronic acid in step 2 and Boc simultaneously left during the last ring-closing step.

Example 27: Synthesis of Compound **B72**

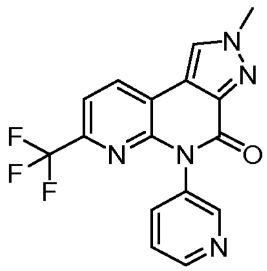

Compound **B72** was obtained according to a synthetic route similar to that of Example 23 with 4-bromo-1-ethylpyrazol-3-carboxylic acid replaced by 4-bromo-1-methylpyrazole-3-carboxylic acid in step 1.

Example 28: Synthesis of Compound **B139**

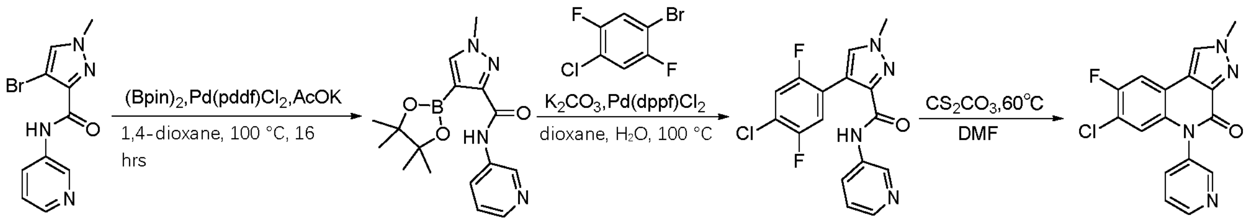


**B139-1** **B139-2** **B139-3** **B139**

1) Synthesis of intermediate **B139-1:**

Compound **B139-1** was obtained according to the preparation method for **B29-2** in Example 23 with 4-bromo-1-ethylpyrazole-3-carboxylic acid replaced by 4-bromo-1-methylpyrazole-3-carboxylic acid.

2) Synthesis of intermediate **B139-2**:

**B139-1** (200 mg, 0.7 mmol) and bis(pinacolato)diboron (220 mg, 0.9 mmol) were dissolved in 1,4-dioxane (2 mL), and potassium acetate (210 mg, 2.1 mmol) and Pd(dppf)$Cl_2$ (52 mg, 0.1 mmol) were added. The mixture was stirred at 100 °C overnight under nitrogen atmosphere. The mixture was directly concentrated to dryness by rotary evaporation, and purified by column chromatography to give brown oily **B139-2** (230 mg, 86%).

3) Synthesis of intermediate **B139-3**:

**B139-2** (270 mg, 0.82 mmol) and 1-bromo-4-chloro-2,5-difluorobenzene (220 mg, 0.88 mmol) were dissolved in 1,4-dioxane (5 mL) and water (0.5 mL), and potassium carbonate (350 mg, 2.53 mmol) and Pd(dppf)$Cl_2$ (90 mg, 0.12 mmol) were added. The mixture was stirred at 100 °C overnight under nitrogen atmosphere. The mixture was directly concentrated to dryness by rotary evaporation, and purified by column chromatography to give orange oily **B139-3** (140 mg, 98%).

4) Synthesis of product **B139**:

**B139-3** (140 mg, 0.40 mmol) was dissolved in *N,N*-dimethylformamide (5 mL), cesium carbonate (390 mg, 1.20 mmol) was added, and the mixture was warmed to 60 °C and stirred for two days. The reaction solution was separated by reversed-phase preparative chromatography to give **B139** (46 mg, 34%) as a white solid.

Example 29: Synthesis of Compound **B148**

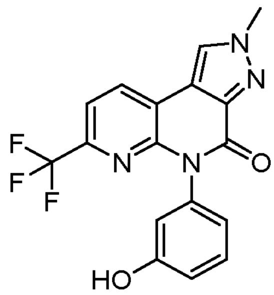

Compound **B148** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 3-aminophenol in step 1.

Example 30: Synthesis of Compound **B171**

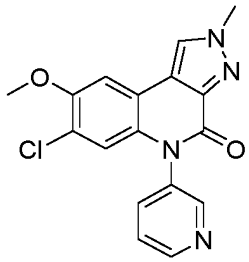

Compound **B171** was obtained according to a synthetic route similar to that of Example 28 with 1-bromo-4-chloro-2,5-difluorobenzene replaced by 1-bromo-4-chloro-2-fluoro-5-methoxybenzene in step 2.

Example 31: Synthesis of Compound **B172**

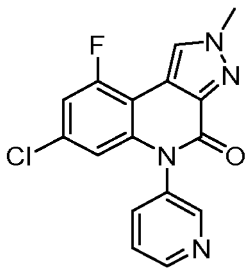

Compound **B172** was obtained according to a synthetic route similar to that of Example 28 with 1-bromo-4-chloro-2,5-difluorobenzene replaced by 2-bromo-5-chloro-1,3-difluorobenzene in step 2.

Example 32: Synthesis of Compound **B176**

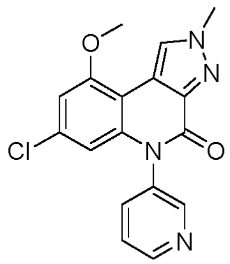

Compound **B176** was obtained according to a synthetic route similar to that of Example 28 with 1-bromo-4-chloro-2,5-difluorobenzene replaced by 2-bromo-5-chloro-1-fluoro-3-methoxybenzene in step 2.

Example 33: Synthesis of Compound **B178**

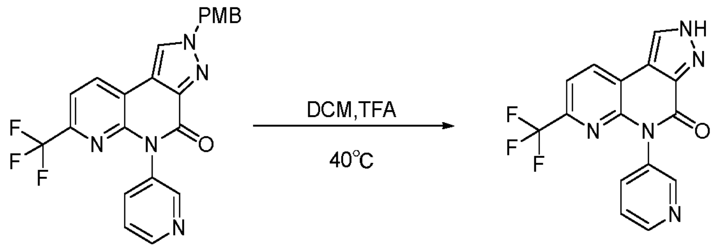


**B178-1** **B178**

1) Synthesis of intermediate **B178-1:**

Intermediate **B178-1** was obtained according to the preparation method for **B29** in Example 23 with 4-bromo-1-ethylpyrazole-3-carboxylic acid replaced by 4-bromo-1-*p*-methoxybenzylpyrazole-3-carboxylic acid in step 1.

2) Synthesis of product **B178:**

Trifluoroacetic acid (1.5 mL) was slowly added dropwise to a solution of **B178-1** (90 mg, 0.18 mmol) in dichloromethane (5 mL), and the mixture was stirred at room temperature overnight. The reaction solution was separated by reversed-phase preparative chromatography to give **B178** (14 mg, 20%) as a white solid.

Example 34: Synthesis of Compound **B76**

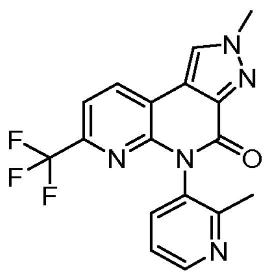

Compound **B76** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 3-amino-2-methylpyridine in step 1.

Example 35: Synthesis of Compounds **B179** and **B180**

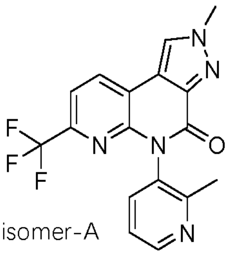
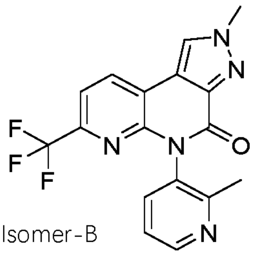

isomer-A Isomer-B

B76 was subjected to chiral resolution (IC-3.0 cm, n-hexane:ethanol = 30:70, 25 mL/min, 230 nm) to give B179 (retention time: 8.11 min) and B180 (retention time: 14.98 min).

Example 36: Synthesis of Compound **B181**

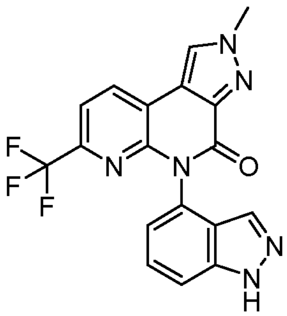

Compound **B181** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by

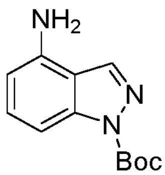

in step 1 and Boc simultaneously left during the last ring-closing step.

Example 37: Synthesis of Compound **B239**

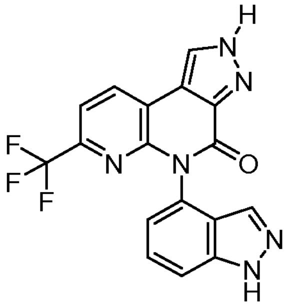

Compound **B239** was obtained according to a synthetic route similar to that of Example 33 with 3-aminopyridine replaced by

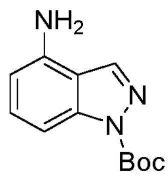

in step 1.

Example 38: Synthesis of Compound **B245**

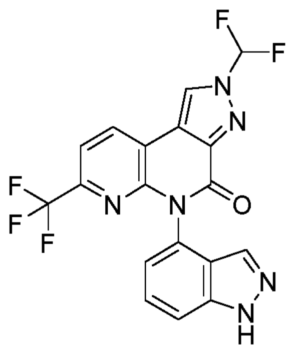

Compound **B245** was obtained according to a synthetic route similar to that of Example 23 with 4-bromo-1-ethylpyrazole-3-carboxylic acid replaced by 4-bromo-1-difluoromethylpyrazole-3-carboxylic acid and 3-aminopyridine replaced by

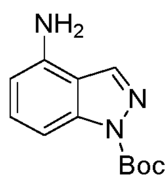

in step 1 and Boc simultaneously left during the last ring-closing step.

Example 39: Synthesis of Compound **B250**

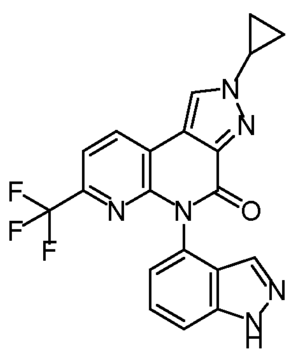

Compound **B250** was obtained according to a synthetic route similar to that of Example 23 with 4-bromo-1-ethylpyrazole-3-carboxylic acid replaced by 4-bromo-1-cyclopropylpyrazole-3-carboxylic acid and 3-aminopyridine

replaced by

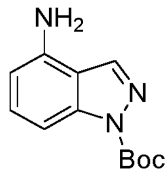

in step 1 and Boc simultaneously left during the last ring-closing step.

Example 40: Synthesis of Compound **B254**

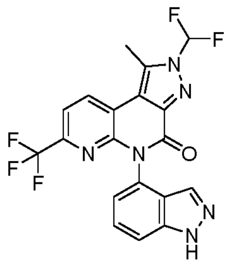

Compound **B254** was obtained according to a synthetic route similar to that of Example 23 with 4-bromo-1-ethylpyrazole-3-carboxylic acid replaced by 5-methyl-4-bromo-1-difluoromethylpyrazole-3-carboxylic acid and 3-aminopyridine replaced by

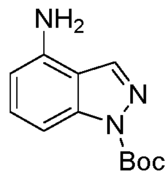

in step 1 and Boc simultaneously left during the last ring-closing step.

Example 41: Synthesis of Compound **B256**

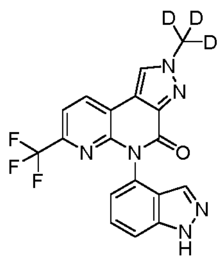

Compound **B256** was obtained according to a synthetic route similar to that of Example 23 with 4-bromo-1-ethylpyrazole-3-carboxylic acid replaced by 4-bromo-1-trideuteromethylpyrazole-3-carboxylic acid and 3-aminopyridine replaced by

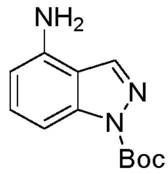

in step 1 and Boc simultaneously left during the last ring-closing step.

Example 42: Synthesis of Compound **B257**

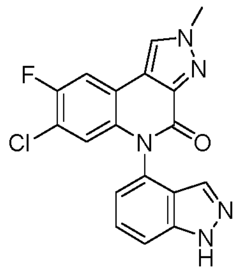

Compound **B257** was obtained according to a synthetic route similar to that of Example 28 with 3-aminopyridine replaced by

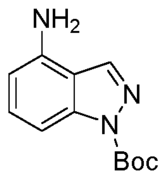

in step 1 and Boc simultaneously left during the last ring-closing step.

Example 43: Synthesis of Compound **B182**

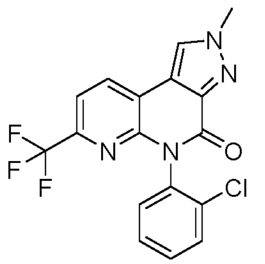

Compound **B182** was obtained according to a synthetic route similar to that of Example 23 with 3-amino-1-methylpyridine replaced by o-chloroaniline and 4-bromo-1-ethyl-3-carboxypyrazole replaced by 4-bromo-1-methyl-3-carboxypyrazole in step 1.

Example 44: Synthesis of Compounds **B183, B212,** and **B213**

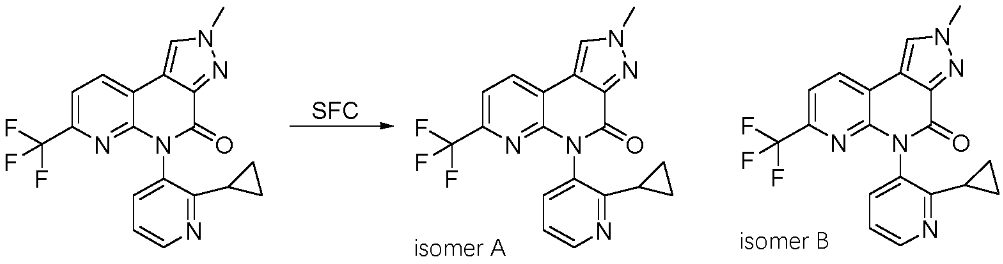


B183 B212 B213

Compound **B183** was obtained according to a synthetic route similar to that of Example 23 with 3-amino-1-methylpyridine replaced by 2-cyclopropyl-3-aminopyridine and 4-bromo-1-ethyl-3-carboxypyrazole replaced by 4-bromo-1-methyl-3-carboxypyrazole in step 1.

**B183** was subjected to chiral resolution (REGIS(S,S)WHELK-O1 (250 mm × 25 mm, 10 μm), methanol = 45%, 70 g/min, 4 min) to give **B212** (retention time: 1.97 min) and **B213** (retention time: 2.35 min).

Example 45: Synthesis of Compound **B258**

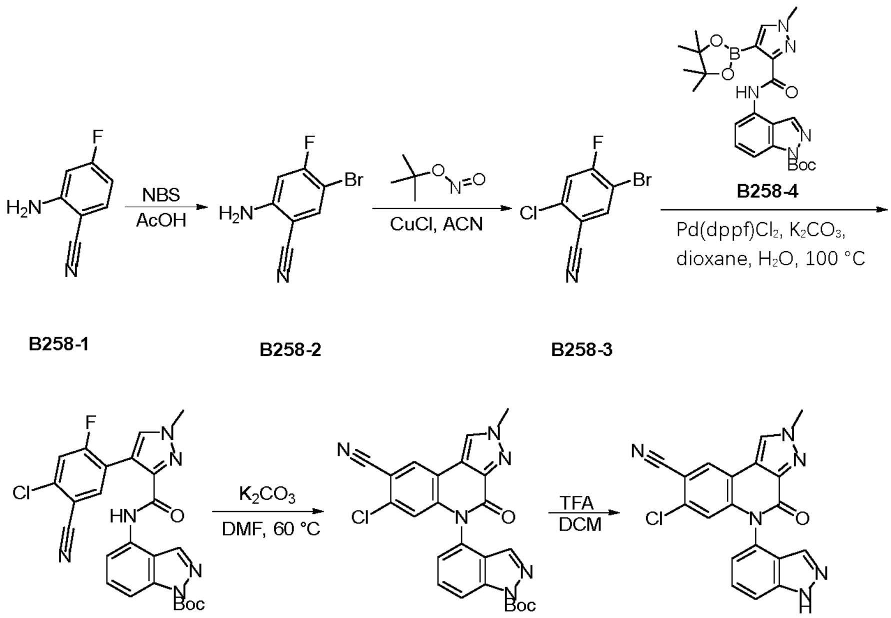


B258-1 B258-2 B258-3

B258-5 B258-6 B258

1) Synthesis of intermediate **B258-2:**

N-Bromosuccinimide (3.92 g, 22.04 mmol) was added to a solution of **B258-1** (3.0 g, 22.04 mmol) in acetic acid (20 mL). The mixture was warmed to 75 °C and stirred for 5 h. The reaction solution was cooled to room temperature and concentrated, and the crude product was separated by column chromatography to give **B258-2** (3.5 g, 74%) as a white solid.

2) Synthesis of intermediate **B258-3:**

**B258-2** (500 mg, 2.33 mmol), cuprous bromide (280 mg, 2.80 mmol), and acetonitrile (5 mL) were added to a reaction flask. *tert*-Butyl nitrite (720 mg, 6.99 mmol) was added at 0 °C, and the mixture was stirred for 4 h. The reaction solution was diluted with ethyl acetate (50 mL) and washed with water (50 mL). The organic phase was concentrated, and the crude product was purified by column chromatography to give **B258-3** (150 mg, 28%) as a white solid product.

3) Synthesis of intermediate **B258-4:**

Compound **B258-4** was obtained according to a synthetic route similar to that for **B139-2** in Example 28 with 3-aminopyridine replaced by 1-Boc-4-aminoindazole.

4) Synthesis of intermediate **B258-5**:

**B258-3** (100 mg, 0.43 mmol), **B258-4** (150 mg, 0.52 mmol), 1,1'-bis(diphenylphosphino)ferrocenepalladium dichloride (31 mg, 0.04 mmol), potassium carbonate (180 mg, 3.90 mmol), 1,4-dioxane (2 mL), and water (0.2 mL) were added to a reaction flask. The mixture was stirred at 100 °C for 16 h. The reaction solution was diluted with ethyl acetate (50 mL) and washed with water (50 mL). The organic phase was concentrated, and the crude product was purified by column chromatography to give **B258-5** (100 mg, 59%) as a white solid product.

5) Synthesis of intermediate **B258-6:**

**B258-5** (100 mg, 0.25 mmol), potassium carbonate (96 mg, 0.63 mmol), and*N*,*N*-dimethylformamide (1 mL) were added to a reaction flask. The mixture was stirred at 60 °C for 16 h. The reaction solution was diluted with dichloromethane (20 mL) and washed with water (10 mL). The organic phase was concentrated to give **B258-6** (70 mg, 74%) as a brown oily substance.

6) Synthesis of product **B258:**

**B258-6** (70 mg, 0.064 mmol), trifluoroacetic acid (0.5 mL), and dichloromethane (1 mL) were added to a reaction flask. The mixture was stirred at room temperature for 2 h. The reaction solution was diluted with dichloromethane (20 mL) and washed with water (10 mL). The organic phase was concentrated, and the crude product was purified by reversed-phase column chromatography to give **B258** (12 mg, 20%) as a white solid.

Example 46: Synthesis of Compound **B260**

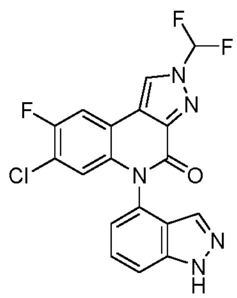

Compound **B260** was obtained according to a synthetic route similar to that of Example 28 with 3-amino-1-methylpyridine replaced by *N*-Boc-4-aminoindazole and 4-bromo-1-ethyl-3-carboxypyrazole replaced by 4-bromo-1-difluoromethyl-3-carboxypyrazole in step 1.

Example 47: Synthesis of Compound **B273**

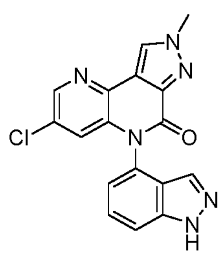

Compound **B273** was obtained according to a synthetic route similar to that of Example 45 with **B258-3** replaced by 2-bromo-5-chloro-3-fluoropyridine.

Example 48: Synthesis of Compound **B274**

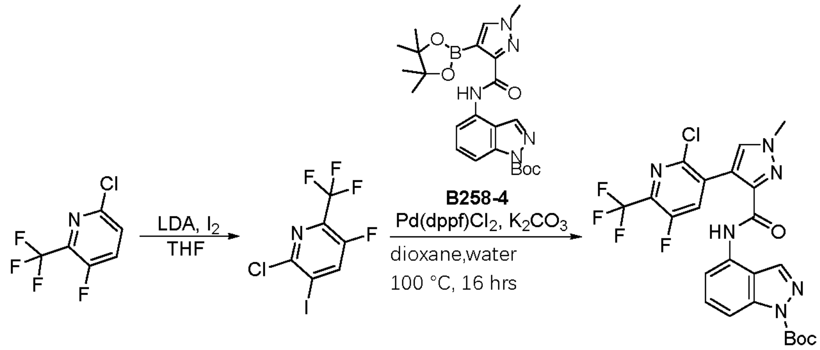


**B274-1** **B274-2** **B274-3**

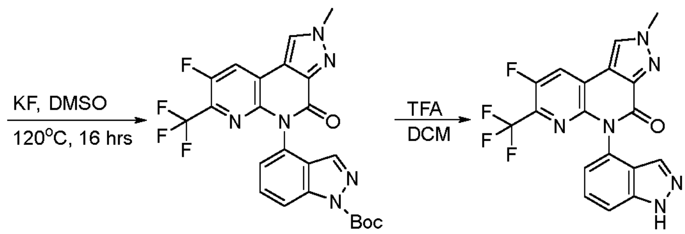


**B274-4** **B274**

1) Synthesis of intermediate **B274-2**:

Lithium diisopropylamide/tetrahydrofuran (0.5 mL, 1 mmol, 2 mol/L) was added to a solution of **B274-1** (100 mg, 0.5 mmol) in tetrahydrofuran (3 mL) at -78 °C, meanwhile the solution being stirred. After the mixture was reacted at -78 °C for 10 min, iodine (150 mg, 0.6 mmol) was added, and the mixture was warmed to room temperature and reacted for 2 h. Water (10 mL) was added, and the mixture was extracted twice with dichloromethane (10 mL). The organic phases were combined and concentrated to dryness by rotary evaporation to remove the solvent, thus giving crude **B274-2** (150 mg, yield: 100%), which was directly used in the next step.

2) Synthesis of product **B274:**

Compound **B274** was obtained according to a synthetic route similar to that of Example 45 with **B258-3** replaced by **B274-2**.

Example 49: Synthesis of Compounds **B332** and **B227**

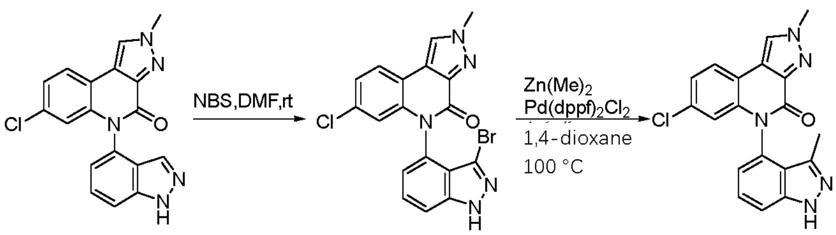


**B60** **B332** **B227**

1) Synthesis of product **B332:**

NBS (30 mg, 1.68 mmol) was added to a solution of **B60** (50 mg, 0.14 mmol) in DMF (1 mL), meanwhile the solution being stirred. The reaction system was reacted at room temperature for 1 h. The reaction solution was directly purified by reversed-phase chromatographic column to give **B332** (13 mg, 22%) as a white solid.

2) Synthesis of product **B227:**

[1,1'-bis(Diphenylphosphino)ferrocene]palladium dichloride (8 mg, 0.01 mmol) and a dimethylzinc/toluene solution (0.2 mL, 0.2 mmol, 1 mol/L) were added to a solution of **B332** (50 mg, 0.117 mmol) in 1,4-dioxane (1 mL), meanwhile the solution being stirred. The mixture was warmed to 100 °C and reacted at room temperature for 3 h. The reaction was quenched with water. The mixture was concentrated to dryness by rotary evaporation to remove the solvent and purified by thin layer chromatography to give **B227** as a white solid (6.2 mg, 11%).

Example 50: Synthesis of Compound **B24**

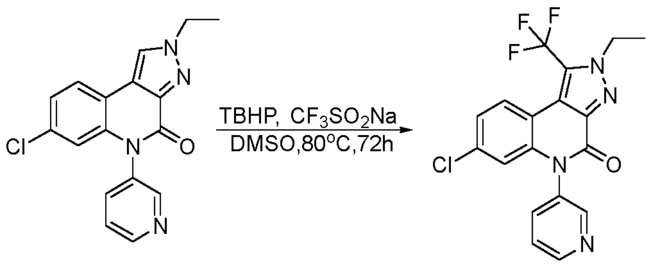


**B3** **B24**

Sodium trifluoromethanesulfonate (274 mg, 2 mmol) was added to a solution of **B3** (310 mg, 1 mmol) and TBHP (180 mg, 2 mmol) in DMSO (5 mL). The mixture was heated to 80 °C and stirred for 72 h under nitrogen atmosphere. The reaction solution was cooled to room temperature and directly separated and purified by reversed-phase preparative chromatography to give **B24** (35 mg, 9%) as a white solid.

Example 51: Synthesis of Compound **B25**

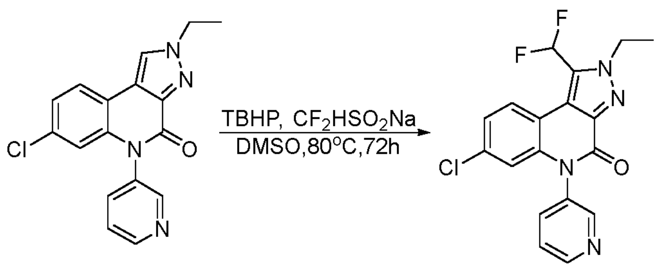


**B3** **B25**

Sodium difluoromethanesulfonate (274 mg, 2 mmol) was added to a solution of **B3** (310 mg, 1 mmol) and TBHP (180 mg, 2 mmol) in DMSO (5 mL). The mixture was heated to 80 °C and stirred for 72 h under nitrogen atmosphere. The reaction solution was cooled to room temperature and directly separated and purified by reversed-phase preparative chromatography to give B25 (15 mg, 4%) as a white solid.

Example 52: Synthesis of Compound **B26**

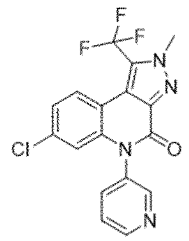

Compound **B26** was obtained according to a synthetic route similar to that of Example 50 with B3 replaced by **B12** in step 1.

Example 53: Synthesis of Compound **B27**

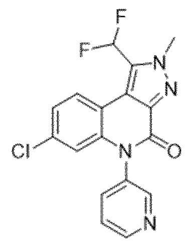

Compound **B27** was obtained according to a synthetic route similar to that of Example 51 with **B3** replaced by **B12** in step 1.

Example 54: Synthesis of Compound **B339**

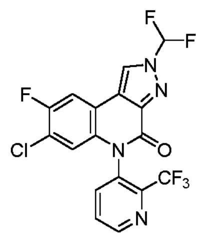

Compound **B339** was obtained according to a synthetic route similar to that of Example 28 with 3-aminopyridine replaced by 2-trifluoromethyl-3-aminopyridine and 4-bromo-1-methyl-3-carboxypyrazole replaced by 4-bromo-1-difluoromethyl-3-carboxypyrazole in step 1.

Example 55: Synthesis of Compounds **B337, B398,** and **B399**

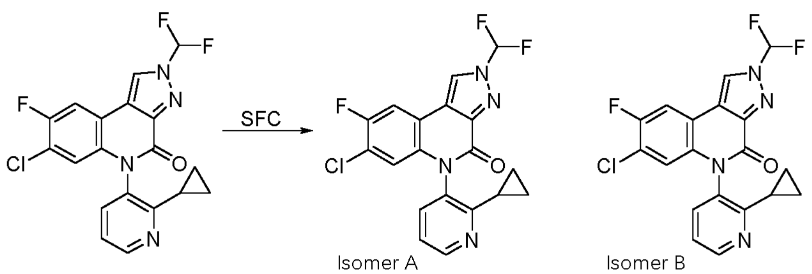


**B337** **B398** **B399**

1) Synthesis of compound **B337:**

Compound **B337** was obtained according to a synthetic route similar to that of Example 28 with 3-aminopyridine replaced by 2-cyclopropyl-3-aminopyridine and 4-bromo-1-methyl-3-carboxypyrazole replaced by 4-bromo-1-difluoromethyl-3-carboxypyrazole in step 1.

2) Resolution of products **B398** and **B399:**

B337 was subjected to chiral resolution (IB-3.0 cm, *n*-hexane:ethanol = 40:60, 25 mL/min, 214 nm) to give B398 (retention time: 10.2 min) and B399 (retention time: 13.9 min).

Example 56: Synthesis of Compound **B31**

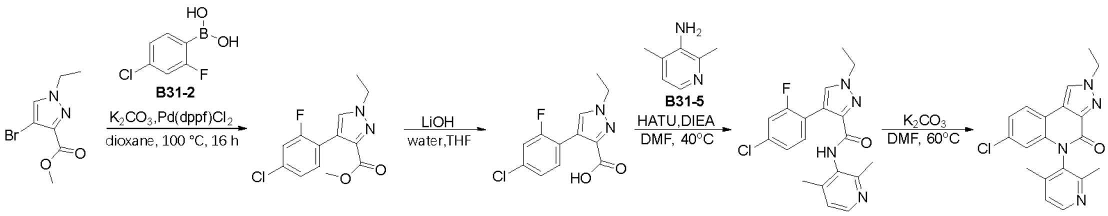


B31-1 B31-3 B31-4 B31-6 B31

1) Synthesis of intermediate **B31-3:**

A solution **of B31-1** (2.32 g, 10 mmol), **B31-2** (1.74 g, 10 mmol), potassium carbonate (4.14 g, 30 mmol), and Pd(dppf)$Cl_2$ (0.73 g, 1 mmol) in dioxane (50 mL) was heated and stirred at 100 °C for 16 h under nitrogen atmosphere. The reaction solution was cooled to room temperature and directly purified by column chromatography to give **B31-3** (1.2 g, 42%) as a brown solid.

2) Synthesis of intermediate **B31-4**

Lithium hydroxide (115 mg, 48 mmol) was added to a mixture of **B31-3** (1.13 g, 40 mmol) in tetrahydrofuran (15 mL)/water (20 mL). The mixture was stirred at room temperature for 0.5 h. The reaction solution was concentrated to dryness, thus giving a crude product **B31-4,** which was directly used in the next step.

3) Synthesis of intermediate **B31-6**

Diisopropylethylamine (387 mg, 3 mmol) and HATU (456 mg, 1.2 mmol) were added to a solution of **B31-4** (268 mg, 1 mmol) and **B31-5** (122 mg, 1 mmol) in DMF (5 mL). The mixture was stirred at 40 °C for 3 h. The reaction solution was directly purified by column chromatography to give B31-6 (130 mg, 36%) as a brown oily substance.

4) Synthesis of product **B31**:

A mixture of **B31-6** (71 mg, 0.2 mmol) and $K_2CO_3$ (27 mg, 0.6 mmol) in DMF (3 mL) was stirred at 60 °C for 16 h. The reaction solution was cooled to room temperature and directly separated by column chromatography to give **B31** (50 mg, 73%) as a white solid.

Example 57: Synthesis of Compound **B32**

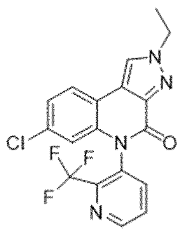

Compound **B32** was obtained according to a synthetic route similar to that of Example 56 with 2,4-dimethyl-3-aminopyridine replaced by 2-trifluoromethyl-3-aminopyridine in step 3.

Example 58: Synthesis of Compound **B33**

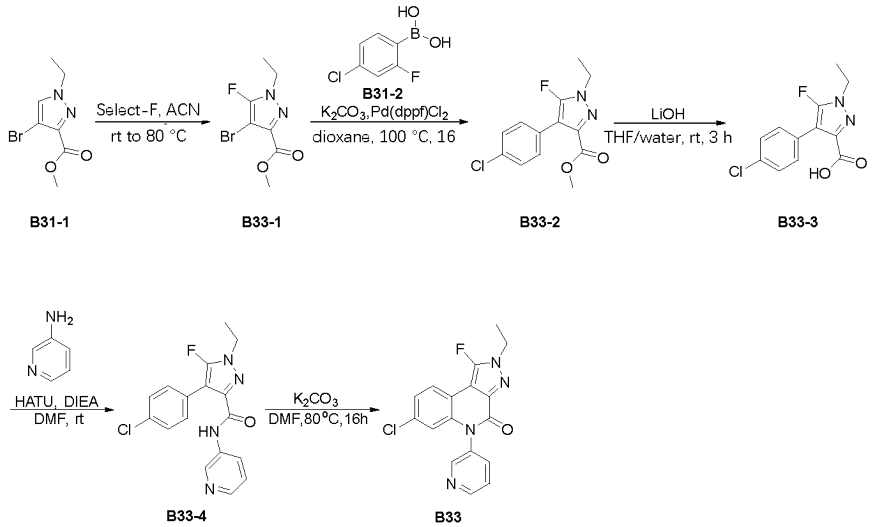


1) Synthesis of intermediate **B33-1:**

A solution of **B31-1** (5.0 g, 21.4 mmol) and Select-F (30.3 g, 85.8 mmol) in acetonitrile (100 mL) was heated to 80 °C and stirred for reaction for 4 days under nitrogen atmosphere. The reaction solution was washed with a saturated aqueous sodium bicarbonate solution and extracted with ethyl acetate. The organic phase was concentrated, and separated and purified by column chromatography to give **B33-1** (1.73 g, 32%) as a white solid.

2) Synthesis of product **B33:**

Compound **B33** was obtained according to a synthetic route similar to that of Example 56 with **B31-1** replaced by **B33-1** and 2,4-dimethyl-3-aminopyridine replaced by 3-aminopyridine.

Example 59: Synthesis of Compound **B34**

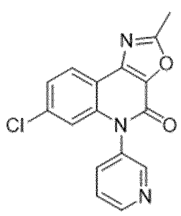

Compound **B34** was obtained according to a synthetic route similar to that of Example 56 with **B31-1** replaced by

methyl 4-bromo-2-methyloxazole-5-carboxylate in step 1 and 2,4-dimethyl-3-aminopyridine replaced by 3-aminopyridine in step 3.

Example 60: Synthesis of Compound **B35**

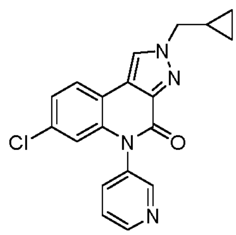

Compound **B35** was obtained according to a synthetic route similar to that of Example 56 with **B31-1** replaced by methyl 4-bromo-2-cyclopropylmethyl-5-carboxylate in step 1 and 2,4-dimethyl-3-aminopyridine replaced by 3-aminopyridine in step 3.

Example 61: Synthesis of Compounds **B36, B37,** and **B38**

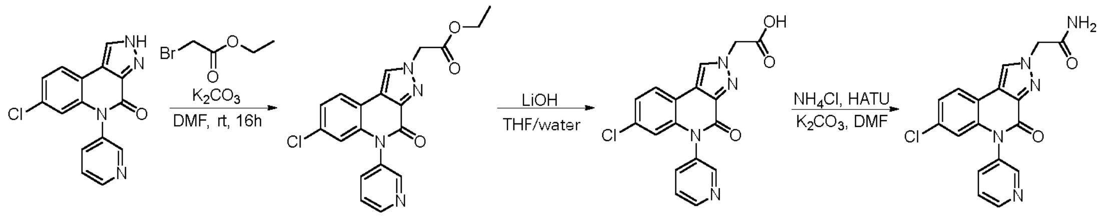


B13 B36-1 B36-2 B36

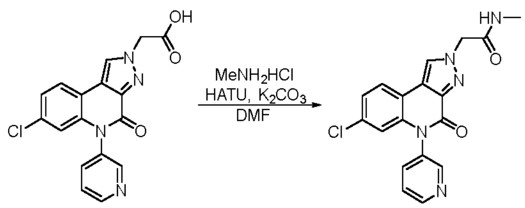


B36-2 B37

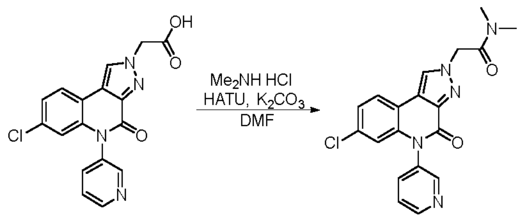


B36-2 B38

1) Synthesis of intermediate **B36-1**:

Potassium carbonate (836 mg, 6.06 mmol) was added to a solution of **B13** (600 mg, 2.02 mmol) and ethyl bromoacetate (405 mg, 2.42 mmol) in DMF (15 mL), and the mixture was stirred at room temperature for 16 h. The mixture was washed with water, extracted with ethyl acetate, concentrated, and purified by reversed-phase preparative chromatography to give **B36-1** (200 mg, 19%) as a yellow solid.

**2) Synthesis of intermediate B36-2**:

Lithium hydroxide monohydrate (69 mg, 1.65 mmol) was added to a mixture of **B36-1** (210 mg, 0.55 mmol) in tetrahydrofuran (15 mL)/water (2 mL), and the mixture was stirred at room temperature for 16 h. The mixture was neutralized to weak acidity with diluted hydrochloric acid (1 M) and extracted with dichloromethane. The organic phase was concentrated to dryness, thus giving **B36-2** (200 mg, 70%) as a yellow solid.

3) Synthesis of products **B36**, **B37**, and **B38**:

Potassium carbonate (58 mg, 0.42 mmol) and HATU (80 mg, 0.21 mmol) were added to a solution of **B36-2** (50 mg, 0.14 mmol) and ammonium chloride (11 mg, 0.21 mmol) in DMF (5 mL). The mixture was stirred at room temperature for 16 h. The mixture was treated with water and extracted with ethyl acetate. The organic phase was concentrated, and purified by reversed-phase preparative chromatography to give **B36** (4.0 mg, 6%) as a white solid. Compounds **B37** and **B38** were separately obtained according to a preparation method similar to that for **B36** with ammonium chloride replaced by methylamine hydrochloride or dimethylamine hydrochloride.

Example 62: Synthesis of Compound **B39**

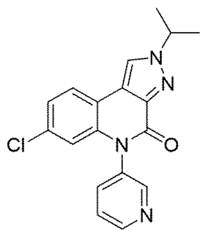

Compound **B39** was obtained according to a synthetic route similar to that of Example 1 with ethylhydrazine replaced by isopropylhydrazine in step 2 and iodobenzene replaced by 3-iodopyridine in step 3.

Example 63: Synthesis of Compounds **B40** and **B170**

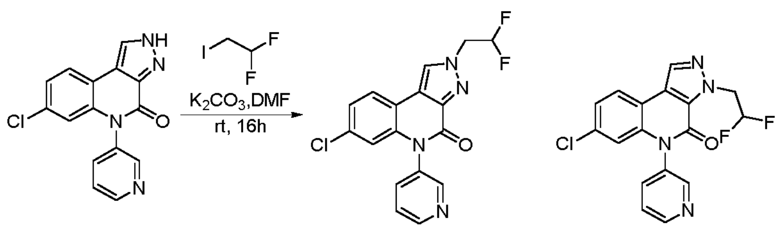


**B13** **B40** **B170**

Potassium carbonate (279 mg, 2.02 mmol) was added to a solution of **B13** (200 mg, 0.67 mmol) and 1,1-difluoro-2-iodoethane in DMF (8 mL). The reaction solution was stirred at room temperature for 16 h, treated with water, and extracted with ethyl acetate. The organic phase was concentrated, and separated by reversed-phase preparative chromatography to give **B40** (18 mg, 7%) and B170 (69 mg, 27%) as white solids.

Example 64: Synthesis of Compound **B41**

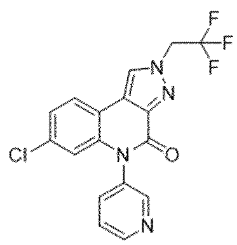

Compound **B41** was obtained according to a synthetic route similar to that of Example 1 with ethylhydrazine replaced by trifluoroethylhydrazine in step 2 and iodobenzene replaced by 3-iodopyridine in step 3.

Example 65: Synthesis of Compounds **B42** and **B167**

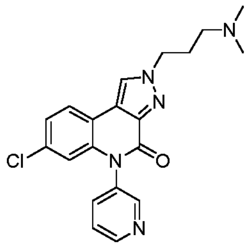
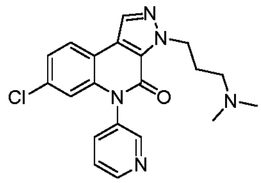

**B42** **B167**

Compounds **B42** and **B167** were obtained according to a synthetic route similar to that of Example 63 with 1,1-difluoro-2-iodoethane replaced by *N*,*N*-dimethyl-3-bromopropylamine in step 2.

Example 66: Synthesis of Compound **B43**

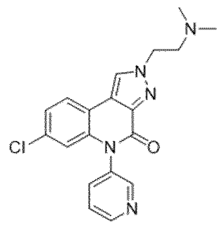

Compound B43 was obtained according to a synthetic route similar to that of Example 63 with 1,1-difluoro-2-iodoethane replaced by *N,N*-dimethyl-2-bromoethylamine in step 2.

Example 67: Synthesis of Compound **B46**

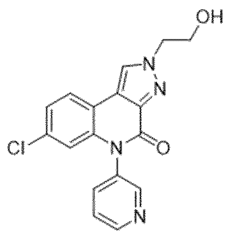

Compound **B46** was obtained according to a synthetic route similar to that of Example 1 with ethylhydrazine replaced by hydroxyethylhydrazine in step 2 and iodobenzene replaced by 3-iodopyridine in step 3.

Example 68: Synthesis of Compound **B45**

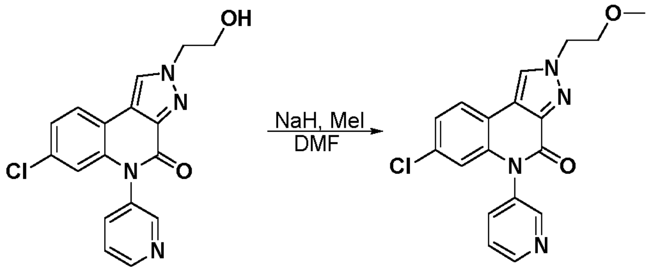


B46 B45

NaH (12 mg, 60%, 0.3 mmol) was added to a solution of **B46** (100 mg, 0.3 mmol) in DMF (15 mL) at 0 °C, and the mixture was stirred at room temperature for 20 min. Iodomethane (43 mg, 0.3 mmol) was added, and the mixture was stirred at room temperature for 2 h. The mixture was treated with saturated brine and extracted with ethyl acetate. The organic phase was concentrated and separated by column chromatography to give **B45** (38 mg, 30%).

Example 69: Synthesis of Compounds **B48** and **B168**

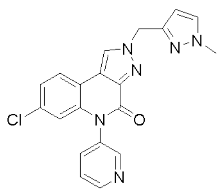

B48

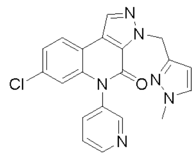

B168

Compound **B48** and isomer **B168** were obtained according to a synthetic route similar to that of Example 63 with 1,1-difluoro-2-iodoethane replaced by (1-methyl-1H-pyrazol-3-yl)methyl methanesulfonate in step 2.

Example 70: Synthesis of Compound **B50**

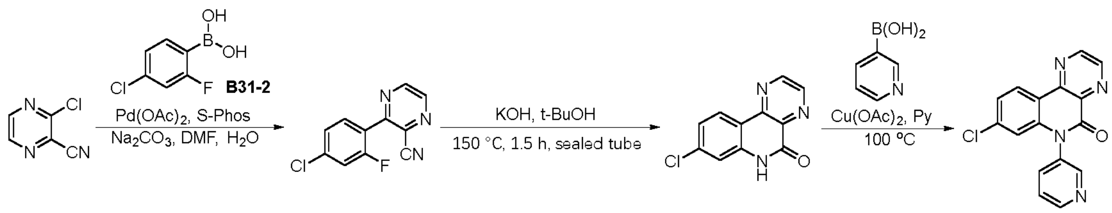


B50-1 B50-2 B50-3 B50

1) Synthesis of intermediate **B50-2:**

Palladium acetate (80 mg, 0.36 mmol) and 2-dicyclohexylphosphino-2',6'-dimethoxybiphenyl (29 mg, 0.72 mmol) were added to **B50-1** (1 g, 7.17 mmol), **B31-2** (1.38 g, 7.89 mmol), and sodium carbonate (3 g, 28.68 mmol) in a mixed solvent of ethylene glycol dimethyl ether (8 mL) and water (4 mL). The reaction solution was stirred at 80 °C for 1 h under nitrogen atmosphere. Water was added, and the mixture was extracted with ethyl acetate. The organic phase was concentrated and separated by column chromatography to give **B50-2** (1.2 g, 72%) as a yellow solid.

**2) Synthesis of intermediate B50-3:**

Potassium hydroxide (0.6 g, 10.7 mmol) was added to a suspension of **B50-2** (500 mg, 2.14 mmol) in *tert*-butanol (20 mL). The mixture was stirred at 120 °C for 1.5 h. After the reaction solution was cooled to room temperature, 20 mL of water was added, and the mixture was acidified with 6 N hydrochloric acid to give a suspension, which was filtered and dried under vacuum to give **B50-3** (420 mg, 85%) as a pale yellow solid.

3) Synthesis of product **B50:**

Copper acetate (0.39 g, 2.16 mmol) was added to a solution of **B50-3** (250 mg, 1.08 mmol) and pyridine-3-boronic acid (660 mg, 5.4 mmol) in pyridine (15 mL). The reaction solution was stirred at 100 °C for 16 h open-to-air. Water was added, and the mixture was extracted with ethyl acetate. The organic phase was concentrated and separated by column chromatography to give **B50** (7 mg, 2%) as a white solid.

Example 71: Synthesis of Compound **B51**

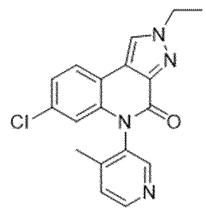

Compound **B51** was obtained according to a synthetic route similar to that of Example 56 with 2,4-dimethyl-3-aminopyridine replaced by 4-methyl-3-aminopyridine in step 3.

Example 72: Synthesis of Compound **B55**

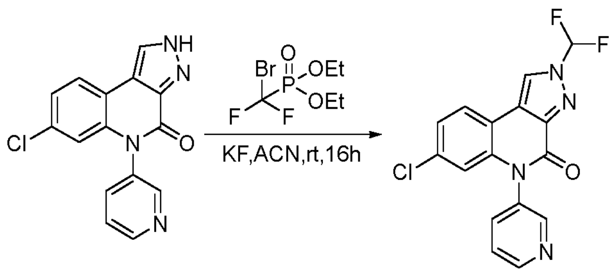


B13 B55

Potassium fluoride (78 mg, 1.35 mmol) was added to a mixture of **B13** (200 mg, 0.67 mmol) and diethyl monobromodifluoromethylphosphate (180 mg, 0.67 mmol) in acetonitrile (10 mL), and the mixture was stirred at room temperature for 16 h. The reaction solution was treated with water and extracted with ethyl acetate. The organic phase was concentrated and separated by reversed-phase preparative chromatography to give **B55** (46 mg, 18%) as a yellow solid.

Example 73: Synthesis of Compound **B57**

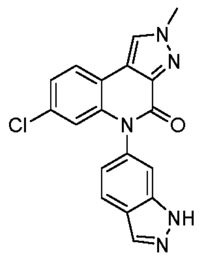

Compound **B57** was obtained according to a synthetic route similar to that of Example 56 with methyl 4-bromo-1-ethylpyrazole-3-carboxylate replaced by methyl 4-bromo-1-methylpyrazole-3-carboxylate in step 1 and 2,4-dimethyl-3-aminopyridine replaced by 5-aminoindazole in step 3.

Example 74: Synthesis of Compound **B58**

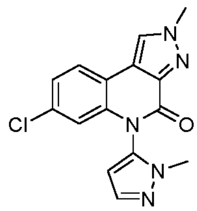

Compound **B58** was obtained according to a synthetic route similar to that of Example 56 with methyl 4-bromo-1-ethylpyrazole-3-carboxylate replaced by methyl 4-bromo-1-methylpyrazole-3-carboxylate in step 1 and 2,4-dimethyl-3-aminopyridine replaced by 1-methyl-5-aminopyrazole in step 3.

Example 75: Synthesis of Compound **B65**

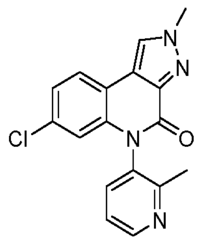

Compound **B65** was obtained according to a synthetic route similar to that of Example 56 with methyl 4-bromo-1-ethylpyrazole-3-carboxylate replaced by methyl 4-bromo-1-methylpyrazole-3-carboxylate in step 1 and 2,4-dimethyl-3-aminopyridine replaced by 2-methyl-3-aminopyridine in step 3.

Example 76: Synthesis of Compound **B68**

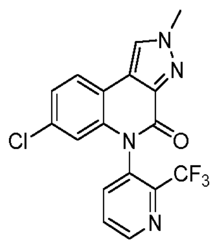

Compound **B68** was obtained according to a synthetic route similar to that of Example 56 with methyl 4-bromo-1-ethylpyrazole-3-carboxylate replaced by methyl 4-bromo-1-methylpyrazole-3-carboxylate in step 1 and 2,4-dimethyl-3-aminopyridine replaced by 2-trifluoromethyl-3-aminopyridine in step 3.

Example 77: Synthesis of Compound **B69**

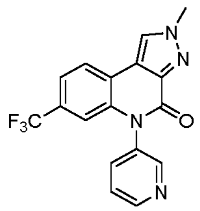

Compound **B69** was obtained according to a synthetic route similar to that of Example 23 with 4-bromo-1-ethylpyrazole-3-carboxylic acid replaced by 4-bromo-1-methylpyrazole-3-carboxylic acid in step 1 and 2-chloro-6-trifluoromethylpyridine-3-boronic acid replaced by 2-fluoro-4-trifluoromethylphenylboronic acid in step 2.

Example 78: Synthesis of Compound **B70**

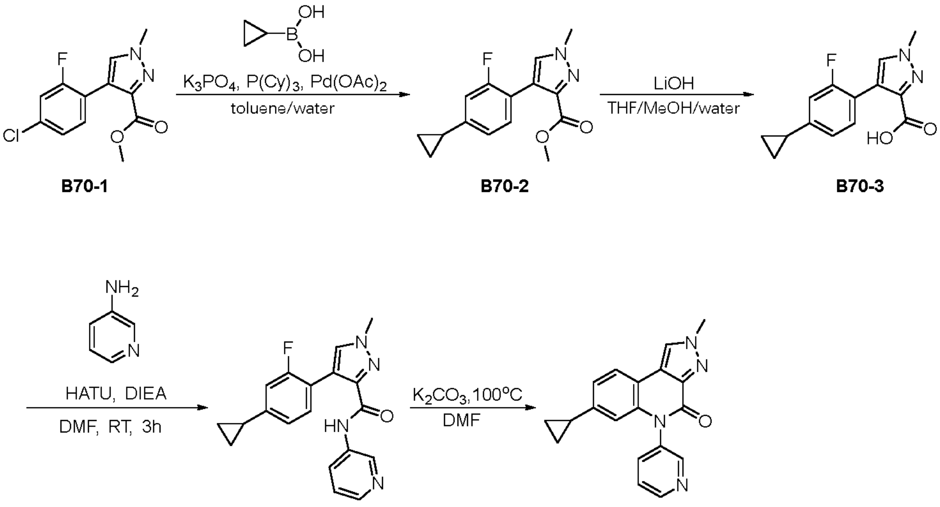


B70-1 B70-2 B70-3 B70-4 B70

1) Synthesis of intermediate **B70-1:**

Compound **B70-1** was obtained according to the preparation method for compound **B31-3** in Example 56 with methyl 4-bromo-1-ethylpyrazole-3-carboxylate replaced by methyl 4-bromo-1-methylpyrazole-3-carboxylate.

2) Synthesis of intermediate **B70-2:**

Water (0.3 mL), potassium phosphate (1.05 g, 4.97 mmol), tricyclohexylphosphorus (0.48 M toluene solution, 0.295 mL, 0.142 mmol), and palladium acetate (16 mg, 0.071 mmol) were sequentially added to a solution of **B70-1** (300 mg, 1.42 mmol) in toluene (6 mL). The mixture was stirred at 100 °C for 4 h under nitrogen atmosphere. The mixture was cooled. Water was added. The mixture was extracted with ethyl acetate. The organic phase was concentrated and separated by column chromatography to give **B70-2** (211 mg, 86%) as a yellow solid.

3) Synthesis of product **B70:**

Compound **B70** was obtained according to a synthetic route similar to that of Example 56 with **B31-3** replaced by **B70-2** and **B31-5** replaced by 3-aminopyridine.

Example 79: Synthesis of Compound **B71**

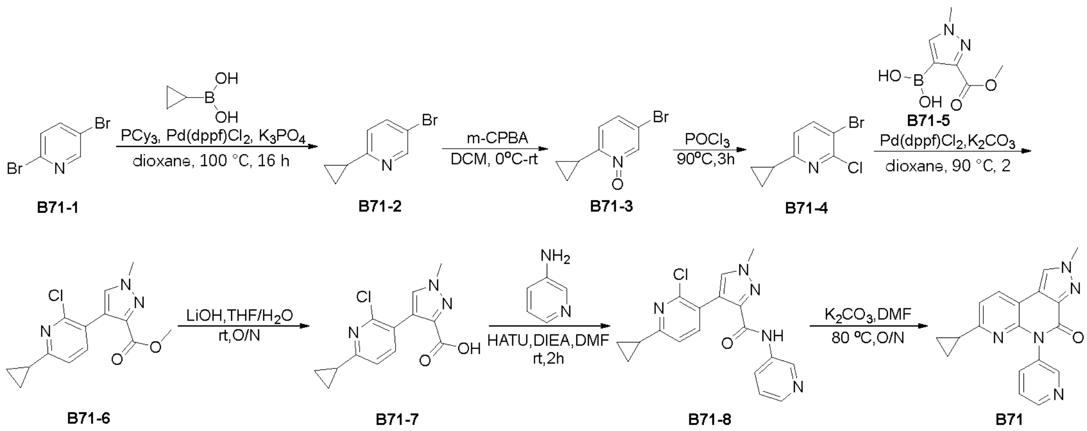


B71-1 B71-2 B71-3 B71-4 B71-6 B71-7 B71-8 B71

1) Synthesis of intermediate **B71-2:**

A mixture of **B71-1** (5.0 g, 21 mmol), cyclopropylboronic acid (1.97 g, 23 mmol), Pd(dppf)$Cl_2$ (1.54 g, 2.1 mmol), potassium phosphate (11.1 g, 53 mmol), and tricyclohexylphosphorus (1.18 g, 4.2 mmol) in dioxane (100 mL) was heated to 100 °C and stirred for 17 h under nitrogen atmosphere. The mixture was treated with water and extracted with ethyl acetate. The organic phase was concentrated and separated by column chromatography to give **B71-2** (2.52 g, 60%) as a yellow solid.

2) Synthesis of intermediate **B71-3:**

*m*-Chloroperoxybenzoic acid (5.16 g, 30 mmol) was added to a solution of **B71-2** (2.97 g, 15 mmol) in dichloromethane (60 mL), and the mixture was stirred for 17 h. The reaction solution was washed with a saturated aqueous sodium sulfite solution, concentrated, and separated and purified by column chromatography to give **B71-3** (1.65 g, 51%) as a yellow solid.

3) Synthesis of intermediate **B71-4:**

**B71-3** (1.65 g, 7.7 mmol) was heated to 90 °C in phosphorus oxychloride (20 mL), and the mixture was stirred for 3 h. The phosphorus oxychloride was removed by distillation under reduced pressure. The residue was poured into ice water, treated with a saturated aqueous sodium bicarbonate solution to pH ≈ 8, and extracted with dichloromethane. The organic phase was dried, concentrated, and separated and purified by column chromatography to give **B71-4** (1.2 g, 67%) as a yellow solid.

4) Synthesis of intermediate **B71-6:**

A mixture of **B71-4** (276 mg, 1.2 mmol), **B71-5** (480 mg, 1.8 mmol), Pd(dppf)$Cl_2$ (90 mg, 0.12 mmol), and potassium carbonate (498 mg, 3.6 mmol) in dioxane (10 mL)/water (1 mL) was heated to 90 °C and stirred for 2 h under nitrogen atmosphere. The reaction solution was treated with water, extracted with ethyl acetate, concentrated, and separated by column chromatography to give **B71-6** (260 mg, 74%) as a yellow solid.

5) Synthesis of product **B71:**

Compound **B71** was obtained according to a synthetic route similar to that of Example 56 with **B31-3** replaced by **B71-6** and **B31-5** replaced by 3-aminopyridine.

Example 80: Synthesis of Compound **B74**

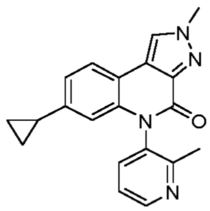

Compound **B74** was obtained according to a synthetic route similar to that of Example 78 with 3-aminopyridine replaced by 2-methyl-3-aminopyridine in step 3.

Example 81: Synthesis of Compound **B75**

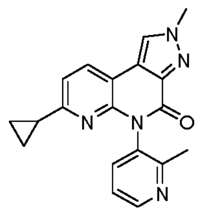

Compound **B75** was obtained according to a synthetic route similar to that of Example 79 with 3-aminopyridine replaced by 2-methyl-3-aminopyridine in step 6.

Example 82: Synthesis of Compound B77

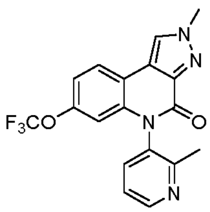

Compound **B77** was synthesized according to a synthetic route similar to that of Example 79 with **B71-4** replaced by 1-bromo-2-fluoro-4-trifluoromethoxybenzene in step 4 and 3-aminopyridine replaced by 2-methyl-3-aminopyridine in step 6.

Example 83: Synthesis of Compound **B78**

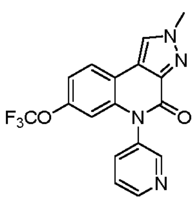

Compound **B78** was synthesized according to a synthetic route similar to that of Example 79 with **B71-4** replaced by 1-bromo-2-fluoro-4-trifluoromethoxybenzene in step 4.

Example 84: Synthesis of Compound **B80**

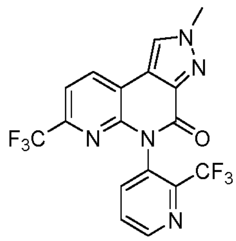

Compound **B80** was synthesized according to a synthetic route similar to that of Example 23 with **B29-1** replaced by 4-bromo-1-methyl-pyrazole-3-carboxylic acid and 3-aminopyridine replaced by 2-trifluoromethyl-3-aminopyridine in step 1.

Example 85: Synthesis of Compound **B95**

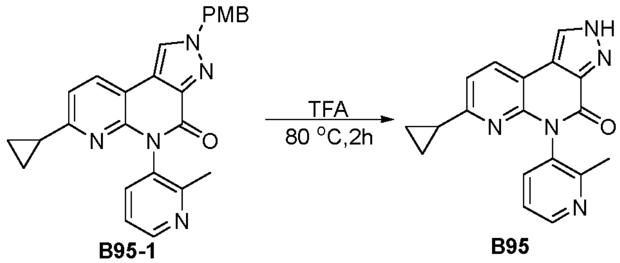


**B95-1** **B95**

1) Synthesis of intermediate **B95-1:**

Intermediate **B95-1** was obtained according to the synthetic route of Example 79 with methyl 4-boronic acid-1-methylpyrazole-3-carboxylate replaced by methyl 4-boronic acid-1-*p*-methoxybenzylpyrazole-3-carboxylate in step 4 and 3-aminopyridine replaced by 2-methyl-3-aminopyridine in step 6.

2) Synthesis of product **B95:**

B95 was obtained according to the synthetic route of Example 33 with **B178-1** replaced by **B95-1.**

Example 86: Synthesis of Compound **B96**

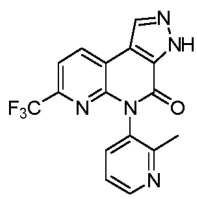

Compound **B96** was synthesized according to a synthetic route similar to that of Example 33 with 3-aminopyridine replaced by 2-methyl-3-aminopyridine.

Example 87: Synthesis of Compound **B99**

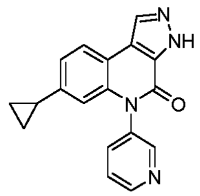

Compound **B99** was synthesized according to a synthetic route similar to that of Example 85 with 2-methyl-3-aminopyridine replaced by 3-aminopyridine.

Example 88: Synthesis of Compound **B101**

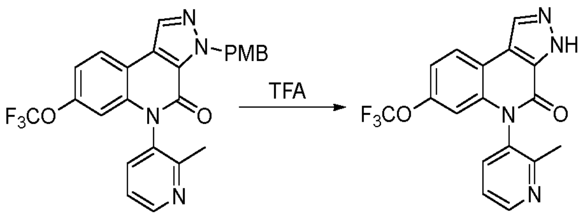


**B101-1** **B101**

1) Synthesis of intermediate **B101-1:**

Compound **B101-1** was synthesized according to a synthetic route similar to that of Example 79 with **B71-4** replaced by 1-bromo-2-fluoro-4-trifluoromethoxybenzene and **B71-5** replaced by methyl 4-boronic acid-1-*p*-methoxybenzylpyrazole-3-carboxylate in step 4.

2) Synthesis of product **B101:**

**B101** was obtained according to the synthetic route of Example 33 with **B178-1** replaced by **B101-1.**

Example 89: Synthesis of Compound **B103**

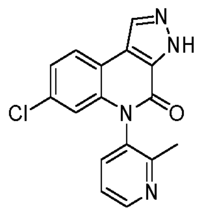

Compound **B103** was synthesized according to a synthetic route similar to that of Example 13 with 3-aminopyridine replaced by 2-methyl-3-aminopyridine.

Example 90: Synthesis of Compound **B109**

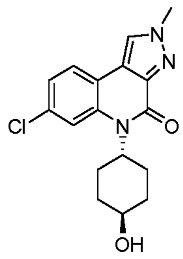

Compound **B109** was synthesized according to a synthetic route similar to that of Example 56 with methyl 4-bromo-1-ethylpyrazole-3-carboxylate replaced by methyl 4-bromo-1-methylpyrazole-3-carboxylate in step 1 and 2,4-dimethyl-3-aminopyridine replaced by *trans*-4-amino-cyclohexanol in step 3.

Example 91: Synthesis of Compound **B117**

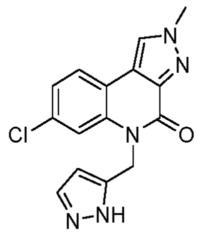

Compound **B117** was synthesized according to a synthetic route similar to that of Example 56 with methyl 4-bromo-1-ethylpyrazole-3-carboxylate replaced by methyl 4-bromo-1-methylpyrazole-3-carboxylate in step 1 and 2,4-dimethyl-3-aminopyridine replaced by 3-aminomethylpyrazole in step 3.

Example 92: Synthesis of Compound **B122**

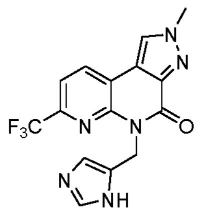

Compound **B122** was synthesized according to a synthetic route similar to that of Example 23 with methyl 4-bromo-1-ethylpyrazole-3-carboxylate replaced by methyl 4-bromo-1-methylpyrazole-3-carboxylate and 3-aminopyridine replaced by 3-aminomethylimidazole in step 1.

Example 93: Synthesis of Compound **B123**

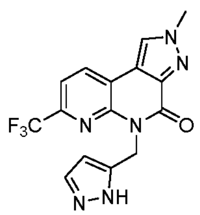

Compound **B123** was synthesized according to a synthetic route similar to that of Example 23 with methyl 4-bromo-1-ethylpyrazole-3-carboxylate replaced by methyl 4-bromo-1-methylpyrazole-3-carboxylate and 3-aminopyridine replaced by 3-aminomethylpyrazole in step 1.

Example 94: Synthesis of Compound **B150**

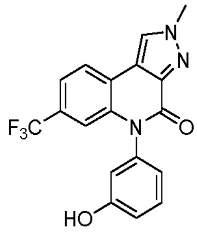

Compound **B150** was synthesized according to a synthetic route similar to that of Example 23 with methyl 4-bromo-1-ethylpyrazole-3-carboxylate replaced by methyl 4-bromo-1-methylpyrazole-3-carboxylate and 3-aminopyridine replaced by 3-hydroxyaniline in step 1.

Example 95: Synthesis of Compound **B152**

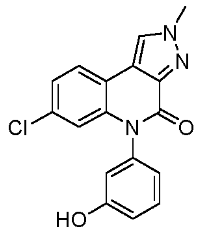

Compound **B152** was synthesized according to a synthetic route similar to that of Example 56 with methyl 4-bromo-1-ethylpyrazole-3-carboxylate replaced by methyl 4-bromo-1-methylpyrazole-3-carboxylate in step 1 and 2,4-dimethyl-3-aminopyridine replaced by 3-hydroxyaniline in step 3.

Example 96: Synthesis of Compound **B169**

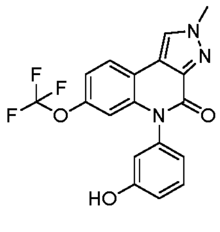

Compound **B169** was synthesized according to a synthetic route similar to that of Example 79 with **B71-4** replaced by 1-bromo-2-fluoro-4-trifluoromethoxybenzene in step 4 and 3-aminopyridine replaced by 3-hydroxyaniline in step 6.

Example 97: Synthesis of Compound **B173**

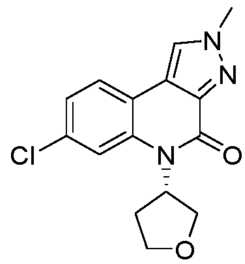

Compound **B173** was synthesized according to a synthetic route similar to that of Example 56 with methyl 4-bromo-1-ethylpyrazole-3-carboxylate replaced by methyl 4-bromo-1-methylpyrazole-3-carboxylate in step 1 and 2,4-dimethyl-3-aminopyridine replaced by (*S*)-3-aminotetrahydrofuran in step 3.

Example 98: Synthesis of Compound **B174**

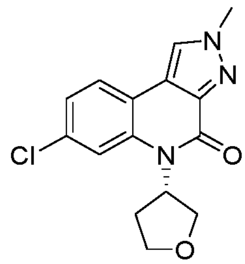

Compound **B174** was synthesized according to a synthetic route similar to that of Example 56 with methyl 4-bromo-1-ethylpyrazole-3-carboxylate replaced by methyl 4-bromo-1-methylpyrazole-3-carboxylate in step 1 and 2,4-dimethyl-3-aminopyridine replaced by (*R*)-3-aminotetrahydrofuran in step 3.

Example 99: Synthesis of Compound **B175**

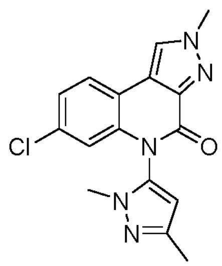

Compound **B175** was synthesized according to a synthetic route similar to that of Example 56 with methyl 4-bromo-1-ethylpyrazole-3-carboxylate replaced by methyl 4-bromo-1-methylpyrazole-3-carboxylate in step 1 and 2,4-dimethyl-3-aminopyridine replaced by 1,3-dimethyl-5-aminopyrazole in step 3.

Example 100: Synthesis of Compound **B177**

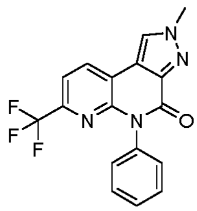

Compound **B177** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by aniline in step 1.

Example 101: Synthesis of Compound **B184**

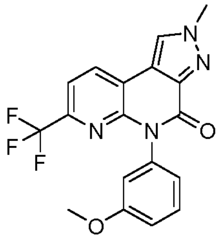

Compound **B184** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 3-methoxyaniline in step 1.

Example 102: Synthesis of Compound **B185**

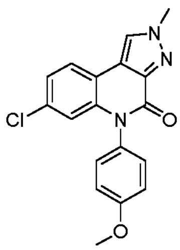

Compound **B185** was obtained according to a synthetic route similar to that of Example 56 with 4-bromo-1-ethylpyrazole-3-carboxylic acid replaced by 4-bromo-1-methylpyrazole-3-carboxylic acid in step 1 and 2,4-dimethyl-3-aminopyridine replaced by 4-methoxyaniline in step 3.

Example 103: Synthesis of Compound **B186**

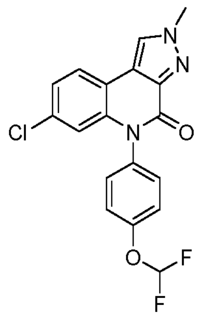

Compound **B186** was obtained according to a synthetic route similar to that of Example 56 with 4-bromo-1-ethylpyrazole-3-carboxylic acid replaced by 4-bromo-1-methylpyrazole-3-carboxylic acid in step 1 and 2,4-dimethyl-3-aminopyridine replaced by 4-difluoromethoxyaniline in step 3.

Example 104: Synthesis of Compound **B187**

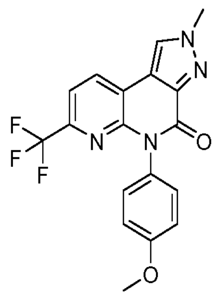

Compound **B187** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 4-methoxyaniline in step 1.

Example 105: Synthesis of Compound **B188**

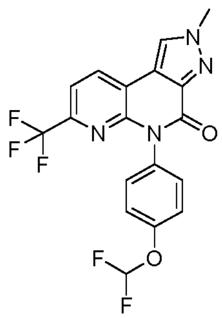

Compound **B188** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 4-difluoromethoxyaniline in step 1.

Example 106: Synthesis of Compound **B189**

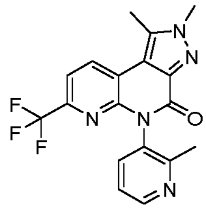

Compound **B189** was obtained according to a synthetic route similar to that of Example 23 with 4-bromo-1-ethylpyrazole-3-carboxylic acid replaced by 4-bromo-1,5-dimethylpyrazole-3-carboxylic acid and 3-aminopyridine replaced by 2-methyl-3-aminopyridine in step 1.

Example 107: Synthesis of Compound **B190**

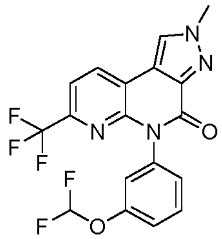

Compound **B190** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 3-difluoromethoxyaniline in step 1.

Example 108: Synthesis of Compound **B191**

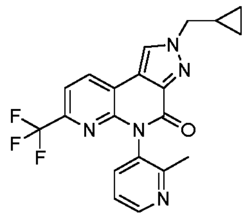

Compound **B191** was obtained according to a synthetic route similar to that of Example 23 with 4-bromo-1-ethylpyrazole-3-carboxylic acid replaced by 4-bromo-1-cyclopropylmethylpyrazole-3-carboxylic acid and 3-aminopyridine replaced by 2-methyl-3-aminopyridine in step 1.

Example 109: Synthesis of Compound **B192**

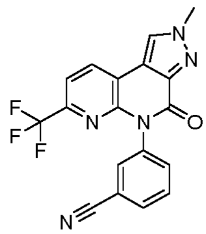

Compound **B192** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 3-cyanoaniline in step 1.

Example 110: Synthesis of Compound **B193**

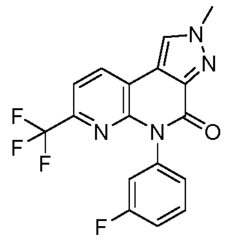

Compound **B193** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 3-fluoroaniline in step 1.

Example 111: Synthesis of Compound **B194**

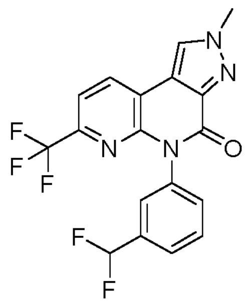

Compound **B194** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 3-difluoromethylaniline in step 1.

Example 112: Synthesis of Compound **B195**

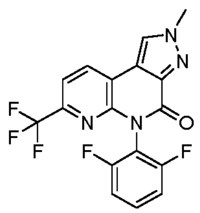

Compound **B195** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 2,6-difluoroaniline in step 1.

Example 113: Synthesis of Compound **B196**

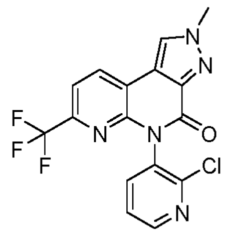

Compound **B196** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 2-chloro-3-aminopyridine in step 1.

Example 114: Synthesis of Compound **B197**

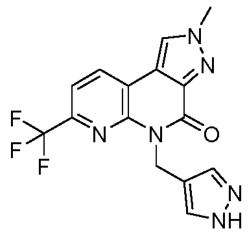

Compound **B197** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 4-aminomethylpyrazole in step 1.

Example 115: Synthesis of Compound **B198**

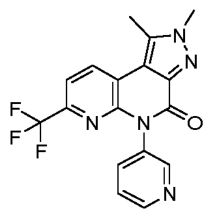

Compound **B198** was obtained according to a synthetic route similar to that of Example 23 with 4-bromo-1-ethylpyrazole-3-carboxylic acid replaced by 4-bromo-1,5-dimethylpyrazole-3-carboxylic acid in step 1.

Example 116: Synthesis of Compound **B199**

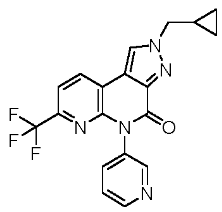

Compound **B199** was obtained according to a synthetic route similar to that of Example 23 with 4-bromo-1-ethylpyrazole-3-carboxylic acid replaced by 4-bromo-1-cyclopropylmethylpyrazole-3-carboxylic acid in step 1.

Example 117: Synthesis of Compound **B200**

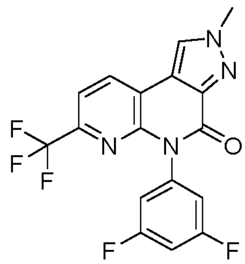

Compound **B200** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 3,5-difluoroaniline in step 1.

Example 118: Synthesis of Compound **B201**

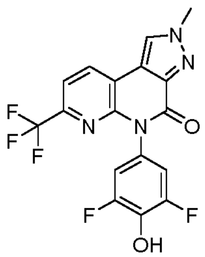

Compound B201 was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 3,5-difluoro-4-hydroxyaniline in step 1.

Example 119: Synthesis of Compound **B202**

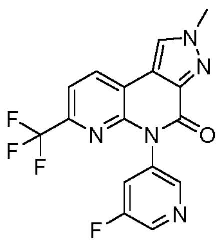

Compound **B202** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 3-amino-5-fluoropyridine in step 1.

Example 120: Synthesis of Compound **B203**

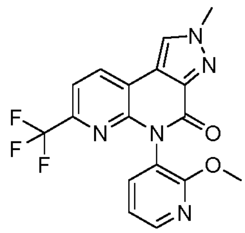

Compound **B203** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 2-methoxy-3-aminopyridine in step 1.

Example 121: Synthesis of Compound **B204**

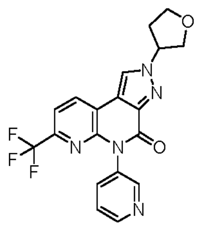

Compound **B204** was obtained according to a synthetic route similar to that of Example 23 with 4-bromo-1-ethylpyrazole-3-carboxylic acid replaced by 4-bromo-1-(3-tetrahydrofuran)pyrazole-3-carboxylic acid in step 1.

Example 122: Synthesis of Compound **B205**

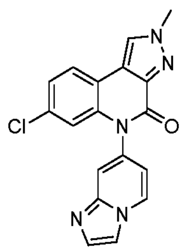

Compound **B205** was obtained according to a synthetic route similar to that of Example 56 with 4-bromo-1-ethylpyrazole-3-carboxylic acid replaced by 4-bromo-1-methylpyrazole-3-carboxylic acid in step 1 and 2,4-dimethyl-3-aminopyridine replaced by

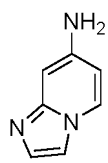

in step 3.

Example 123: Synthesis of Compound **B206**

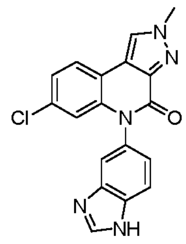

Compound **B206** was obtained according to a synthetic route similar to that of Example 56 with 4-bromo-1-ethylpyrazole-3-carboxylic acid replaced by 4-bromo-1-methylpyrazole-3-carboxylic acid in step 1 and 2,4-dimethyl-3-aminopyridine replaced by

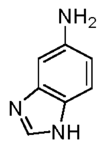

in step 3.

Example 124: Synthesis of Compound **B207**

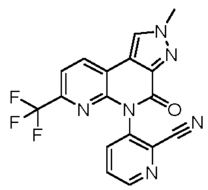

Compound **B207** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 2-cyano-3-aminopyridine in step 1.

Example 125: Synthesis of Compound **B208**

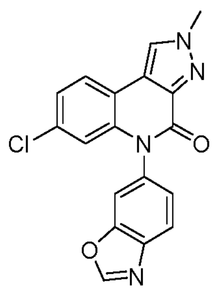

Compound **B208** was obtained according to a synthetic route similar to that of Example 56 with 4-bromo-1-ethylpyrazole-3-carboxylic acid replaced by 4-bromo-1-methylpyrazole-3-carboxylic acid in step 1 and 2,4-dimethyl-3-aminopyridine replaced by

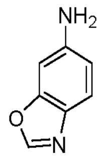

in step 3.

Example 126: Synthesis of Compound **B209**

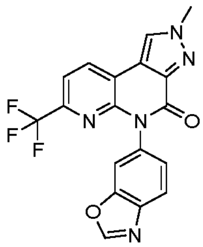

Compound **B209** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by

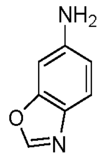

in step 1.

Example 127: Synthesis of Compound **B210**

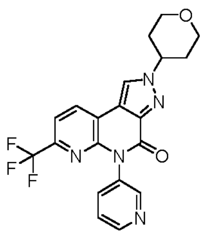

Compound **B210** was obtained according to a synthetic route similar to that of Example 23 with 4-bromo-1-ethylpyrazole-3-carboxylic acid replaced by 4-bromo-1-(4-tetrahydropyran)pyrazole-3-carboxylic acid in step 1.

Example 128: Synthesis of Compound **B211**

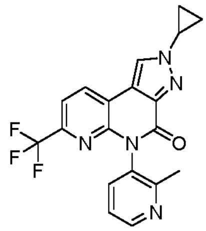

Compound **B211** was obtained according to a synthetic route similar to that of Example 23 with 4-bromo-1-ethylpyrazole-3-carboxylic acid replaced by 4-bromo-1-cyclopropylpyrazole-3-carboxylic acid and 3-aminopyridine replaced by 2-methyl-3-aminopyridine in step 1.

Example 129: Synthesis of Compound **B214**

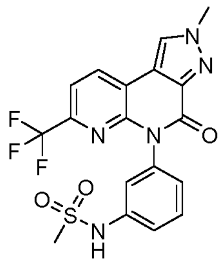

Compound **B214** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by

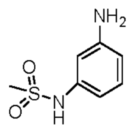

in step 1.

Example 130: Synthesis of Compound **B215**

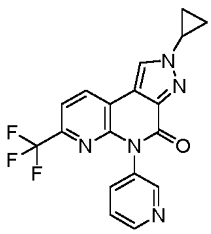

Compound **B215** was obtained according to a synthetic route similar to that of Example 23 with 4-bromo-1-ethylpyrazole-3-carboxylic acid replaced by 4-bromo-1-cyclopropylpyrazole-3-carboxylic acid in step 1.

Example 131: Synthesis of Compound **B216**

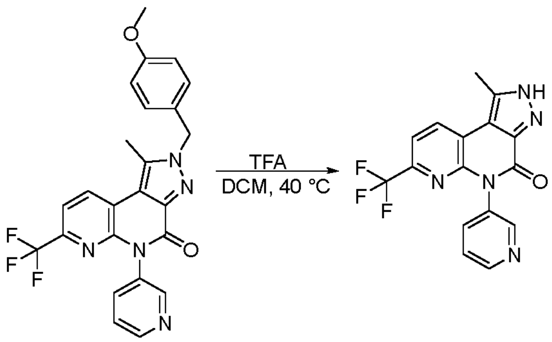


B216-1 B216

1) Synthesis of intermediate **B216-1:**

Compound **B216-1** was obtained according to a synthetic route similar to that of Example 23 with 4-bromo-1-ethylpyrazole-3-carboxylic acid replaced by 4-bromo-1-*p*-methoxybenzyl-5-methylpyrazole-3-carboxylic acid in step 1.

2) Synthesis of product **B216:**

**B216** was obtained according to the synthetic route of Example 33 with **B178-1** replaced by **B216-1.**

Example 132: Synthesis of Compound **B333**

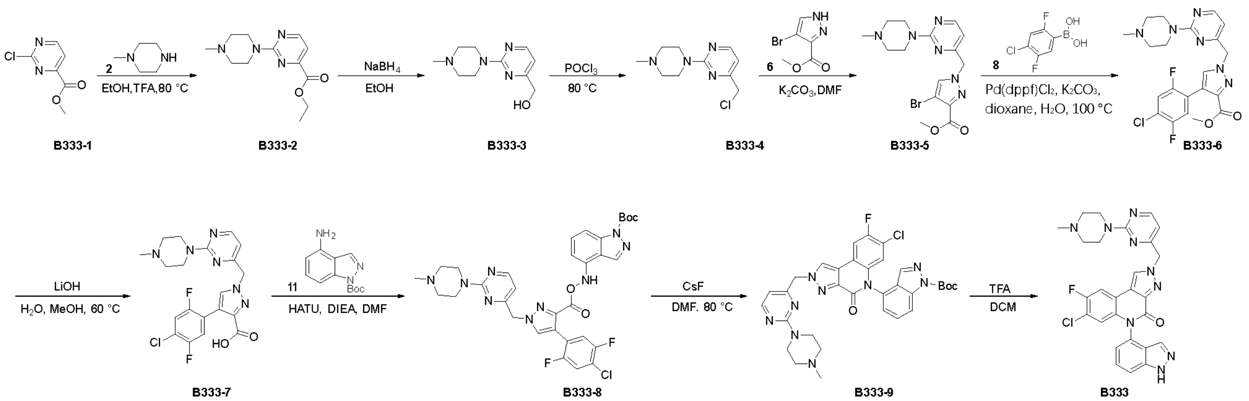


B333-1 B333-2 B333-3 B333-4 B333-5 B333-6

B333-7 B333-8 B333-9 B333

1) Synthesis of intermediate **B333-2:**

Compound **B333-1** (2 g, 11.59 mmol) and triethylamine (2.35 g, 23.18 mmol) were added to a solution of ethanol (10 mL), *N*-methylpiperazine (1.2 g, 11.94 mmol) was added with stirring, and the reaction solution was warmed to 80 °C and stirred overnight. Water was added to the reaction solution, and the mixture was extracted with dichloromethane. The organic phase was washed with saturated brine, dried over sodium sulfate, filtered, and concentrated to give **B333-2** (2 g, 73%) as a yellow oily product.

2) Synthesis of intermediate **B333-3:**

Compound **B333-2** (2 g, 7.99 mmol) was added to a solution of ethanol (20 mL), sodium borohydride (1 g, 26.43 mmol) was added with stirring, and the reaction was stirred at room temperature overnight. The reaction solution was filtered and concentrated to give **B333-3** (1.5 g, 90%) as an off-white solid product.

3) Synthesis of intermediate **B333-4:**

Compound **B333-3** (1.5 g, 7.20 mmol) was added to a solution of phosphorus oxychloride (6 mL), and the mixture was warmed to 80 °C and stirred for 4 h. Water and ethyl acetate were added to the reaction solution, and the pH was

adjusted to 7-9 with a saturated aqueous sodium carbonate solution. The phases were separated and extracted with ethyl acetate. The organic phase was washed with water, washed with saturated brine, concentrated, and separated by column chromatography to give **B333-4** (0.7 g, 42%) as a pale yellow oily product.

4) Synthesis of intermediate **B333-5:**

Compounds methyl 4-bromopyrazole-3-carboxylate (450 mg, 2.05 mmol) and potassium carbonate (850 mg, 6.15 mmol) were added to a solution of *N*,*N*-dimethylformamide (5 mL). Compound **B333-4** (700 mg, 2.16 mmol) was added with stirring, and the mixture was stirred at room temperature overnight. Water was added to the reaction solution, and the mixture was extracted with ethyl acetate. The organic phase was washed with water, washed with saturated brine, concentrated, and separated by column chromatography to give **B333-5** (0.7 g, 86%) as a pale yellow oily product.

5) Synthesis of intermediate **B333-6:**

Compound **B333-5** (700 mg, 1.77 mmol), 2,5-difluoro-3-chlorophenylboronic acid (700 mg, 2.55 mmol), potassium carbonate (730 mg, 5.28 mmol), and 1,1'-bis(diphenylphosphino)ferrocene dichloropalladium(II) (120 mg, 0.16 mmol) were added to a mixed solution of dioxane (10 mL) and water (1 mL), and the mixture was purged three times with nitrogen. The mixture was warmed to 100 °C and stirred overnight. The reaction solution was concentrated and separated by column chromatography to give **B333-6** (600 mg, 73%) as a pale yellow oily product.

6) Synthesis of product **B333**

Compound **B333** was obtained according to a synthetic route similar to that of Example 56 with **B31-**3 replaced by **B333-6,** 2,4-dimethyl-3-aminopyridine replaced by

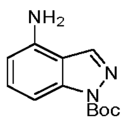,

and a deprotection reaction finally performed.

Example 133: Synthesis of Compound **B268**

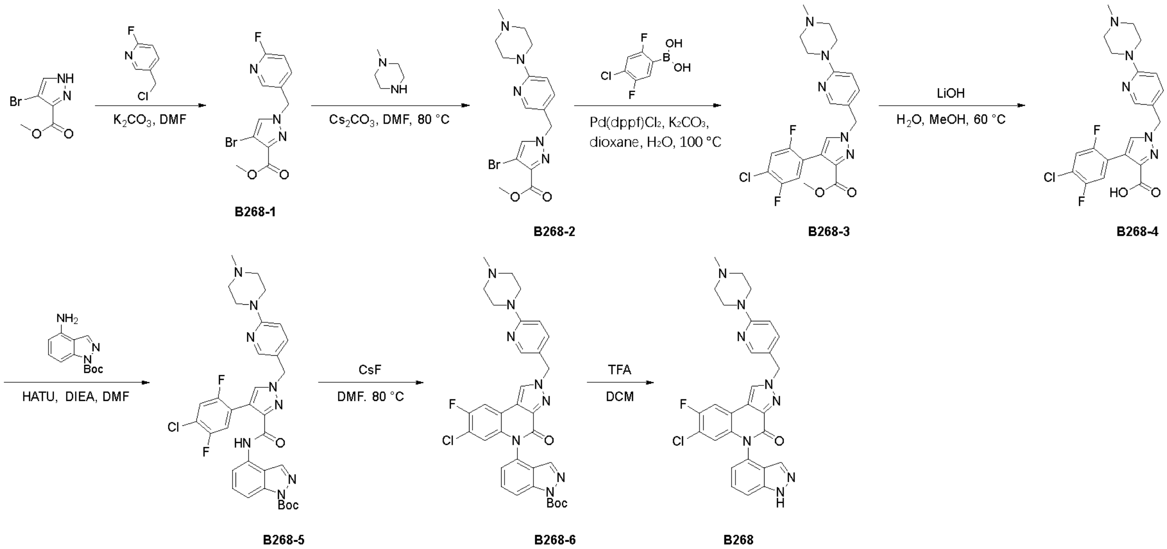


1) Synthesis of intermediate **B268-1:**

Methyl 4-bromopyrazole-3-carboxylate (1 g, 4.88 mmol) and potassium carbonate (2.02 g, 5.37 mmol) were added to a solution of *N*,*N*-dimethylformamide (10 mL). 2-Fluoro-5-pyridinylmethyl chloride (780 mg, 2.16 mmol) was added with stirring, and the mixture was stirred at room temperature overnight. Water was added to the reaction solution,

and the mixture was extracted with dichloromethane. The organic phase was concentrated, and separated by column chromatography to give **B268-1** (0.5 g, 65%) as a pale yellow oily product.

2) Synthesis of intermediate **B268-2:**

**B268-1** (0.5 g, 1.59 mmol) was added to a solution of *N*,*N*-dimethylformamide (5 mL). Cesium carbonate (1.55 g, 4.77 mmol) and methylpiperazine (290 mg, 2.86 mmol) were added with stirring, and the mixture was stirred at 80 °C for reaction overnight. The reaction solution was directly separated by column chromatography to give **B268-2** (0.5 g, 79%) as a colorless oily substance.

3) Synthesis of product **B268**

Compound **B268** was obtained according to a synthetic route similar to that of Example 132 with **B333-5** replaced by **B268-2.**

Example 134: Synthesis of Compound **B336**

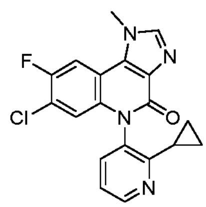

Compound **B336** was obtained according to a synthetic route similar to that of Example 28 with 4-bromo-1-methylpyrazole-3-carboxylic acid replaced by 5-bromo-1-methylimidazole-4-carboxylic acid and 3-aminopyridine replaced by 2-cyclopropyl-3-aminopyridine in step 1.

Example 135: Synthesis of Compound **B376**

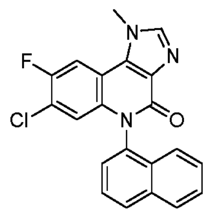

Compound **B376** was obtained according to a synthetic route similar to that of Example 28 with 4-bromo-1-methylpyrazole-3-carboxylic acid replaced by 5-bromo-1-methylimidazole-4-carboxylic acid and 3-aminopyridine replaced by 1-aminonaphthalene in step 1.

Example 136: Synthesis of Compound **B340**

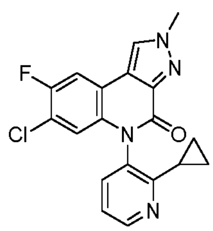

Compound **B340** was obtained according to a synthetic route similar to that of Example 28 with 3-aminopyridine replaced by 2-cyclopropyl-3-aminopyridine in step 1.

Example 137: Synthesis of Compound **B370**

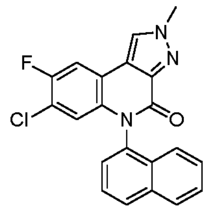

Compound **B370** was obtained according to a synthetic route similar to that of Example 28 with 3-aminopyridine replaced by 1-aminonaphthalene in step 1.

Example 138: Synthesis of Compound **B371**

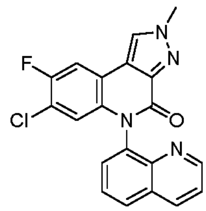

Compound **B371** was obtained according to a synthetic route similar to that of Example 28 with 3-aminopyridine replaced by 8-aminoquinoline in step 1.

Example 139: Synthesis of Compound **B373**

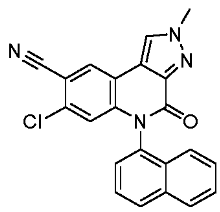

Compound **B373** was obtained according to a synthetic route similar to that of Example 45 with 1-Boc-4-aminoindazole replaced by 1-aminonaphthalene.

Example 140: Synthesis of Compound **B217**

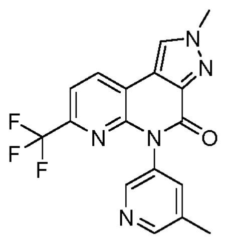

Compound **B217** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 5-methyl-3-aminopyridine in step 1.

Example 141: Synthesis of Compound **B218**

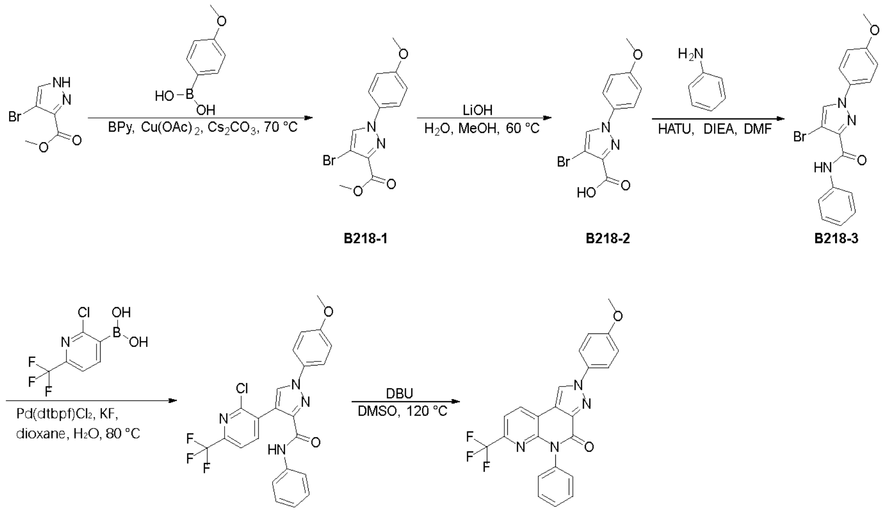


B218-1 B218-2 B218-3

B218-4 B218

1) Synthesis of intermediate **B218-1:**

Methyl 4-bromopyrazole-3-carboxylate (2 g, 9.76 mmol), 6-methoxy-3-boronic acid (2.5 g, 16.45 mmol), copper acetate (2 g, 10 mmol), 2,2-bipyridine (1.5 g, 9.6 mmol), sodium carbonate (2.5 g, 23.59 mmol), and dichloroethane (10 mL) were added to a reaction flask. The mixture was stirred at 70 °C for 16 h. The reaction solution was filtered, and the organic phase was concentrated. The crude product was separated and purified by column chromatography to give **B218-1** (0.5 g, 16%) as a white solid.

2) Synthesis of intermediate **B218-2:**

**B218-1** (0.5 g, 1.61 mmol), lithium hydroxide (0.2 g, 8.35 mmol), methanol (5 mL), and water (5 mL) were added to a reaction flask. The mixture was stirred at 60 °C for 16 h. The reaction solution was concentrated, adjusted to pH = 1, extracted with ethyl acetate, and washed with saturated brine. The organic phase was concentrated to give **B218-2** (0.5 g, 79%) as a brown oily product.

3) Synthesis of product **B218:**

Compound **B218** was obtained according to a synthetic route similar to that of Example 23 with 4-bromo-1-ethylpyrazole-3-carboxylic acid replaced by **B218-2** and 3-aminopyridine replaced by aniline in step 1.

Example 142: Synthesis of Compound **B219**

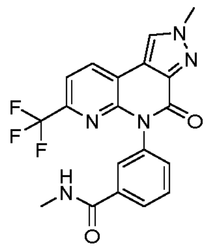

Compound **B219** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 3-aminobenzamide in step 1.

Example 143: Synthesis of Compound **B220**

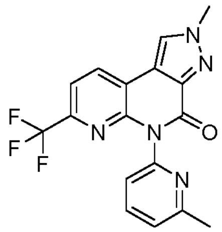

Compound **B220** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 2-amino-6-methylpyridine in step 1.

Example 144: Synthesis of Compound **B221**

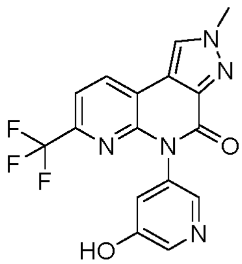

Compound **B221** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 3-amino-5-hydroxypyridine in step 1.

Example 145: Synthesis of Compound **B222**

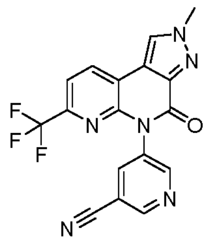

Compound **B222** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 3-amino-5-cyanopyridine in step 1.

Example 146: Synthesis of Compound **B223**

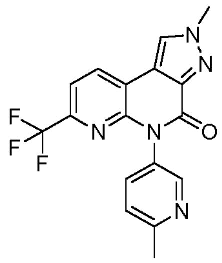

Compound **B223** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 3-amino-6-methylpyridine in step 1.

Example 147: Synthesis of Compounds **B224** and **B225**

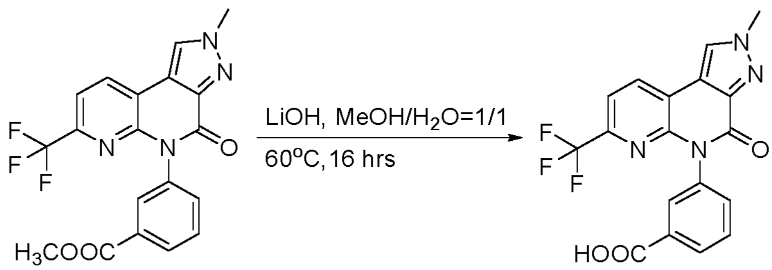


**B224** **B225**

1) Synthesis of product **B224:**

Compound **B224** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by methyl 3-aminobenzoate in step 1.

2) Synthesis of product **B225:**

**B224** (100 mg, 0.3 mmol), lithium hydroxide (18 mg, 0.8 mmol), methanol (2 mL), and water (2 mL) were added to a reaction flask, and the mixture was stirred at 60 °C for 16 h. The reaction solution was acidified with a solution (4 M) of hydrogen chloride in dioxane, diluted with dichloromethane (50 mL), and washed with water. The organic phase was concentrated and purified by reversed-phase column chromatography to give **B225** (8 mg, 8%) as a white solid.

Example 148: Synthesis of Compound **B227**

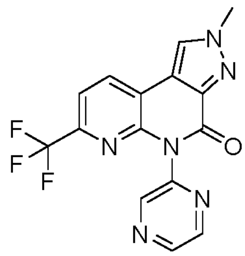

Compound **B227** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 2-aminopyrazine in step 1.

Example 149: Synthesis of Compound **B226**

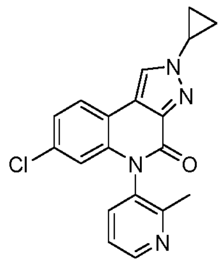

Compound **B226** was obtained according to a synthetic route similar to that of Example 56 with 4-bromo-1-ethylpyrazole-3-carboxylic acid replaced by 4-bromo-1-cyclopropylpyrazole-3-carboxylic acid in step 1 and 2,4-dimethyl-3-aminopyridine replaced by 2-methyl-3-aminopyridine in step 3.

Example 150: Synthesis of Compound **B228**

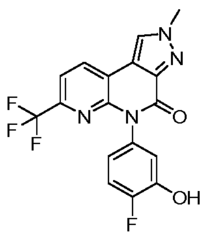

Compound **B228** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 3-hydroxyl-4-fluoro-aniline in step 1.

Example 151: Synthesis of Compound **B229**

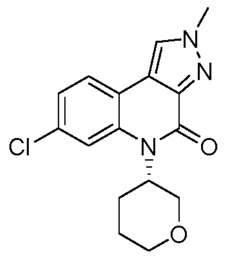

Compound **B229** was obtained according to a synthetic route similar to that of Example 56 with 4-bromo-1-ethylpyrazole-3-carboxylic acid replaced by 4-bromo-1-methylpyrazole-3-carboxylic acid in step 1 and 2,4-dimethyl-3-aminopyridine replaced by (*S*)-3-aminotetrahydropyran in step 3.

Example 152: Synthesis of Compound **B230**

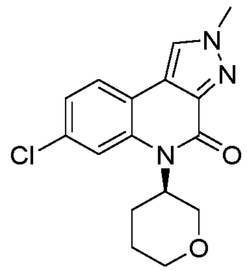

Compound **B230** was obtained according to a synthetic route similar to that of Example 56 with 4-bromo-1-ethylpyrazole-3-carboxylic acid replaced by 4-bromo-1-methylpyrazole-3-carboxylic acid in step 1 and 2,4-dimethyl-3-aminopyridine replaced by (*R*)-3-aminotetrahydropyran in step 3.

Example 153: Synthesis of Compound **B231**

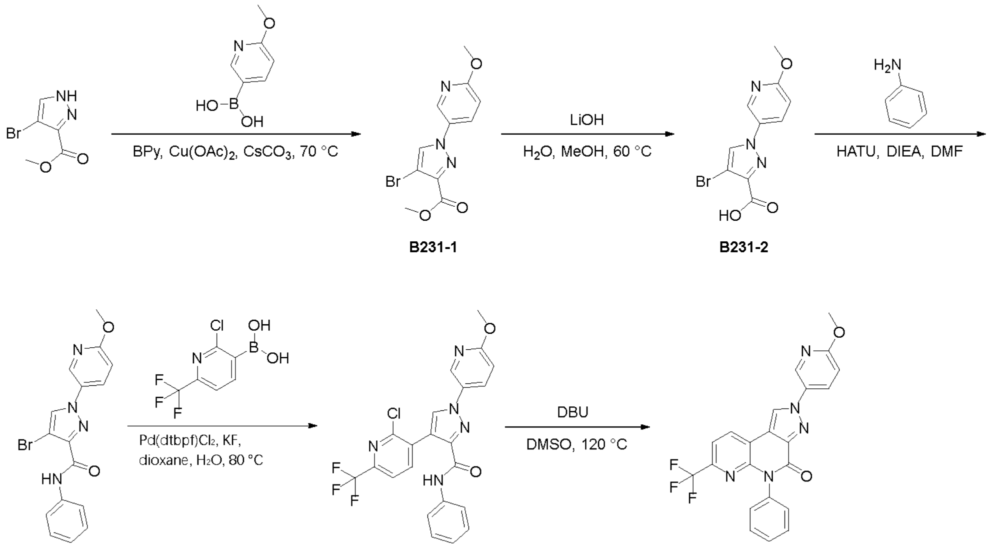


B231-1 B231-2 B231-3 B231-4 B231

1) Synthesis of intermediate **B231-1:**

Methyl 4-bromopyrazole-3-carboxylate (2 g, 9.76 mmol), 6-methoxy-3-pyridinylboronic acid (1.49 g, 2.44 mmol), copper acetate (1.21 g, 3.17 mmol), 2,2-bipyridine (1.68 g, 10.74 mmol), sodium carbonate (2.07 g, 19.52 mmol), and

dichloroethane (10 mL) were added to a reaction flask. The mixture was stirred at 70 °C for 16 h. The reaction solution was filtered, and the organic phase was concentrated. The crude product was purified by column chromatography to give **B231-1** (1.2 g, 78%) as a white solid product.

2) Synthesis of product **B231:**

Compound **B231** was obtained according to a synthetic route similar to that of Example 23 with **B29-**1 replaced by **B231-1** and 3-aminopyridine replaced by aniline in step 1.

Example 154: Synthesis of Compound **B234**

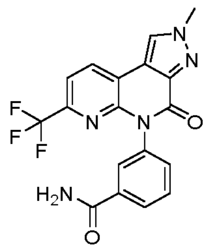

Compound **B234** was obtained according to a synthetic route similar to that of Example 23 with 4-bromo-1-ethylpyrazole-3-carboxylix acid replaced by 4-bromo-1-methylpyrazole-3-carboxylic acid and 3-amonipyridine replaced by 3-aminobenzamide in step 1.

Example 155: Synthesis of Compound **B235**

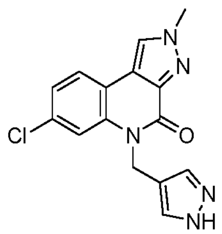

Compound **B235** was obtained according to a synthetic route similar to that of Example 56 with methyl 4-bromo-1-ethylpyrazole-3-carboxylate replaced by methyl 4-bromo-1-methylpyrazole-3-carboxylate in step 1 and 2,4-dimethyl-3-aminopyridine replaced by 4-aminomethylpyrazole in step 3.

Example 156: Synthesis of Compounds **B236** and **B237**

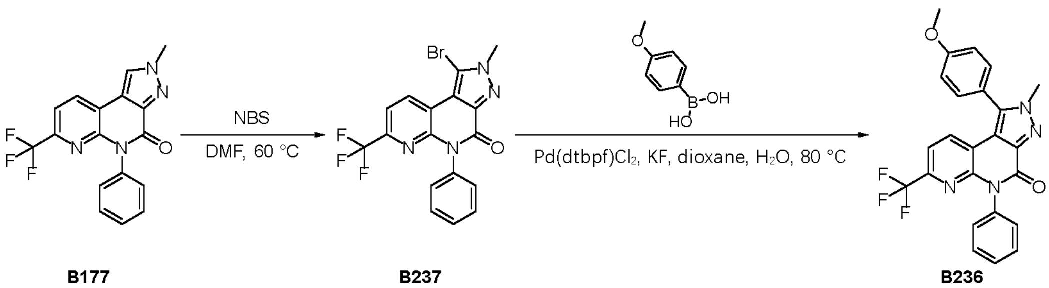


**B177** **B237** **B236**

1) Synthesis of compound **B237:**

B177 (400 mg, 1.16 mmol), *N*-bromosuccinimide (310 mg, 1.74 mmol), and*N*,*N*-dimethylformamide (5 mL) were added to a reaction flask. The mixture was stirred at 60 °C for 16 h. The reaction solution was poured into water and extracted with ethyl acetate. The organic phases were combined, washed with saturated brine, dried, and purified by column chromatography to give **B237** (300 mg, 61%) as a white solid product.

2) Synthesis of compound **B236:**

**B237** (150 mg, 0.35 mmol), *p*-methoxyphenylboronic acid (80 mg, 0.52 mmol), [1,1'-bis(di-*tert*-butylphosphino) ferrocene]palladium dichloride (23 mg, 0.035 mmol), potassium fluoride (61 mg, 1.05 mmol), 1,4-dioxane (3 mL), and water (0.6 mL) were added to a reaction flask. The mixture was stirred at 80 °C for 16 h. The reaction solution was diluted with ethyl acetate and washed with water. The organic phase was concentrated, and the crude product was purified by column chromatography to give **B236** (50 mg, 31%) as a white solid product.

Example 157: Synthesis of Compound **B238**

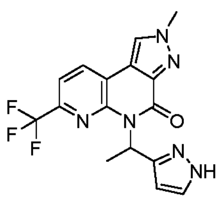

Compound **B238** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 1-(3-pyrazolyl)-ethylamine in step 1.

Example 158: Synthesis of Compound **B240**

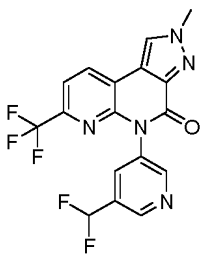

Compound **B240** was obtained according to a synthetic route similar to that of Example 23 with 4-bromo-1-ethylpyrazole-3-carboxylic acid replaced by 4-bromo-1-methylpyrazole-3-carboxylic acid and 3-aminopyridine replaced by 3-amino-5-difluoromethylpyridine in step 1.

Example 159: Synthesis of Compounds **B241** and **B244**

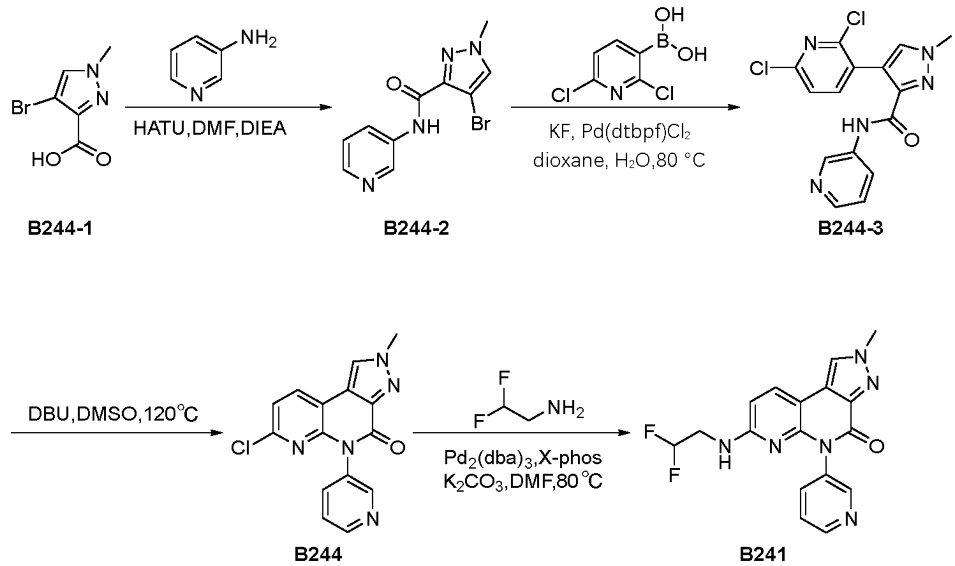


B244-1 B244-2 B244-3

B244 B241

1) Synthesis of intermediate **B244-2:**

2-(7-Azabenzotriazol)-*N*,*N*,*N*',*N*'-tetramethyluronium hexafluorophosphate (4.15 g, 10.93 mmol) and *N*,*N*-diisopropylethylamine (1.83 g, 14.14 mmol) were added to a solution of compound **B244-1** (2 g, 9.76 mmol) in DMF (10 mL), meanwhile the solution being stirred, and the mixture was stirred at room temperature for 0.5 h. 3-Aminopyridine (1.5 g, 15.9 mmol) was then added to the mixed solution, and the mixture was stirred at room temperature overnight. Water was added to the reaction solution, and the mixture was extracted with ethyl acetate. The organic phases were combined, washed with water, washed with saturated brine, dried over sodium sulfate, filtered, and concentrated to give **B244-2** (2.2 g, 80%) as an orange solid product.

2) Synthesis of compound **B244-3:**

Compound **B244-2** (2 g, 7.11 mmol), 2,6-dichloro-3-pyridineboronic acid (1.7 g, 8.86 mmol), potassium fluoride (1.25 g, 21.51 mmol), and dichloro[1,1'-bis(di-tert-butylphosphino)ferrocene]palladium(II) (400 mg, 0.086 mmol) were added to a mixed solution of dioxane (10 mL) and water (1 mL), and the mixture was purged three times with nitrogen. The mixture was heated to 80 °C and stirred overnight. The reaction solution was directly concentrated and separated by column chromatography to give **B244-3** (2 g, 80%) as a black oily product.

3) Synthesis of compound **B244:**

1,8-Diazabicyclo[5.4.0]undec-7-ene (2.1 g, 13.79 mmol) was added to a solution of compound **B244-3** (1.6 g, 4.6 mmol) in dimethyl sulfoxide (8 mL), meanwhile the solution being stirred. The mixture was purged three times with nitrogen. The mixture was warmed to 120 °C and stirred overnight. Water was added to the reaction solution, and the mixture was extracted with dichloromethane. The organic phases were combined, washed with water, washed with saturated brine, dried over sodium sulfate, concentrated, and separated by column chromatography to give **B244** (500 mg, 35%) as a white solid.

4) Synthesis of compound **B241:**

Compound **B244** (40 mg, 0.13 mmol), 2,2-difluoroethylamine (11 mg, 0.13 mmol), potassium carbonate (50 mg, 0.36 mmol), tris(dibenzylideneacetone)dipalladium (10 mg, 0.09 mmol), and 2-dicyclohexylphosphino-2',4',6'-triisopropylbiphenyl (40 mg, 0.08 mmol) were added to DMF (2 mL), and the mixture was purged three times with nitrogen. The mixture was heated to 80 °C and stirred overnight. The reaction solution was directly separated by column chromatography to give **B241** (24 mg, 54%) as an off-white solid.

Example 160: Synthesis of Compound **B242**

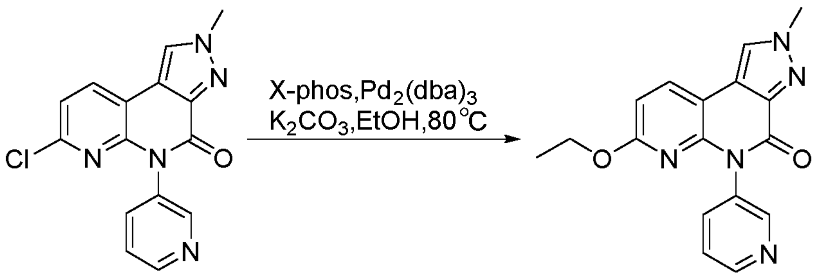


**B244** **B242**

Compound **B244** (80 mg, 0.26 mmol), potassium carbonate (120 mg, 0.86 mmol), tris(dibenzylideneacetone) dipalladium (24 mg, 0.026 mmol), and 2-dicyclohexylphosphino-2',4',6'-triisopropylbiphenyl (82 mg, 0.17 mmol) were added to a mixed solution of ethanol (1 mL), and the mixture was purged three times with nitrogen. The mixture was heated to 80 °C and stirred overnight. The reaction solution was separated by reversed-phase column chromatography to give **B242** (28 mg, 33%) as a white solid.

Example 161: Synthesis of Compound **B243**

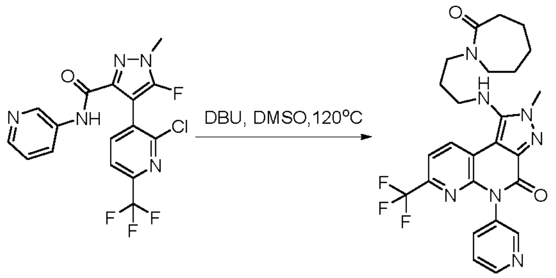


**B243-1** **B243**

1) Synthesis of intermediate **B243-1:**

Compound **B243-1** was obtained according to a synthetic route similar to that for intermediate B33-4 in Example 58 with B31-1 replaced by methyl 4-bromo-1-methylpyrazole-3-carboxylate in step 1 and B31-2 replaced by 2-chloro-6-trifluoromethylpyridine-3-boronic acid in step 2.

2) Synthesis of compound **B243:**

1,8-Diazabicyclo[5.4.0]undec-7-ene (170 mg, 1.12 mmol) was added to a solution of **B243-1** (150 mg, 0.38 mmol) in DMSO (3 mL), meanwhile the solution being stirred. The mixture was warmed to 120 °C and stirred overnight. Water was added to the reaction solution, and the mixture was extracted with dichloromethane. The organic phase was concentrated and separated by column chromatography to give **B243** (23 mg, 12%) as a white solid product.

Example 162: Synthesis of Compound **B251**

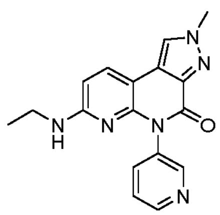

Compound **B251** was obtained according to a synthetic route similar to that of Example 159 with 2,2-difluoroethylamine replaced by ethylamine in step 4.

Example 163: Synthesis of Compound **B252**

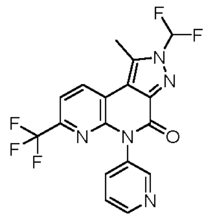

Compound **B252** was obtained according to a synthetic route similar to that of Example 23 with B29-1 replaced by 4-bromo-1-difluoromethyl-5-methylpyrazole-3-carboxylic acid in step 1.

Example 164: Synthesis of Compound **B253**

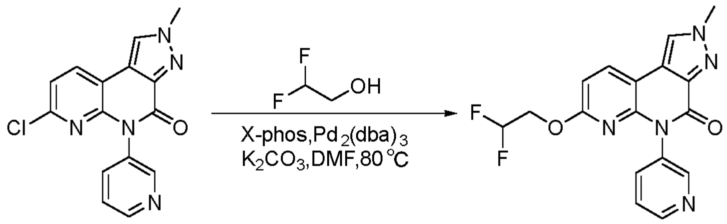


**B244** **B253**

**B244** (40 mg, 0.13 mmol), 2,2-difluoroethanol (30 mg, 0.37 mmol), potassium carbonate (60 mg, 0.43 mmol), tris(dibenzylideneacetone)dipalladium (20 mg, 0.022 mmol), and 2-dicyclohexylphosphino-2',4',6'-triisopropylbiphenyl (60 mg, 0.13 mmol) were added to a mixed solution of DMF (1 mL). The mixture was purged three times with nitrogen, heated to 80 °C, and stirred overnight. The reaction solution was directly separated by column chromatography to give **B253** (35 mg, 76%) as an off-white solid product.

Example 165: Synthesis of Compound **B255**

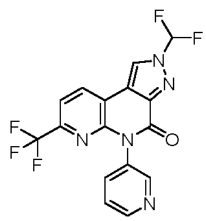

Compound **B255** was obtained according to a synthetic route similar to that of Example 23 with B29-1 replaced by 4-bromo-1-difluoromethylpyrazole-3-carboxylic acid in step 1.

Example 166: Synthesis of Compound **B263**

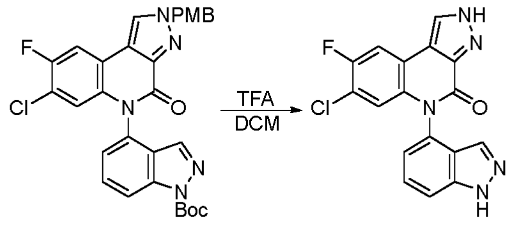


B263-1 B263

1) Synthesis of intermediate **B263-1:**

Intermediate **B263-1** was synthesized according to a synthetic route similar to that of Example 28 with 4-bromo-1-methylpyrazole-3-carboxylic acid replaced by 4-bromo-1-*p*-methoxybenzylpyrazole-3-carboxylic acid and 3-aminopyridine replaced by 1-Boc-4-aminoindazole.

2) Synthesis of compound **B263:**

**B263** was synthesized according to a preparation method similar to that for **B178** in Example 33.

Example 167: Synthesis of Compound **B267**

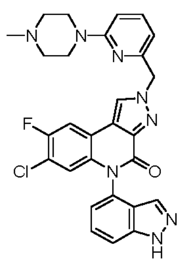

Compound **B267** was obtained according to a synthetic route similar to that of Example 133 with 3-chloromethyl-6-fluoropyridine replaced by 2-chloromethyl-6-fluoropyridine in step 1.

Example 168: Synthesis of Compounds **B342, B388,** and **B389**

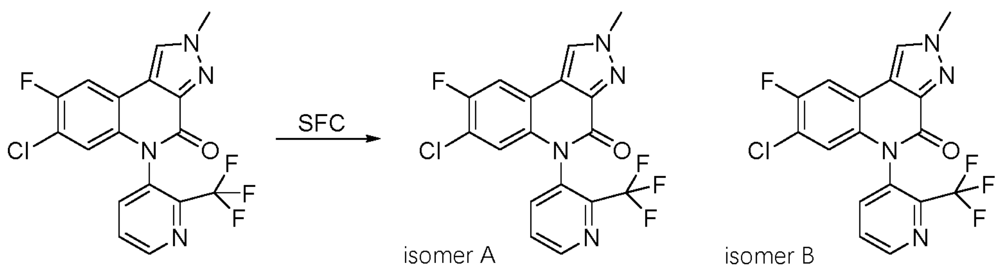


B342 B388 B389

1) Synthesis of product B342:

Compound **B342** was obtained according to a synthetic route similar to that of Example 28 with 3-aminopyridine replaced by 2-trifluoromethyl-3-aminopyridine.

2) Resolution of products B388 and B389:

**B342** was subjected to chiral resolution (IA-3.0 cm, n-hexane/ethanol = 50:50, 25 mL/min, 230 nm) to give **B388** (retention time: 9.79 min) and **B389** (retention time: 14.33 min).

Example 169: Synthesis of Compound **B348**

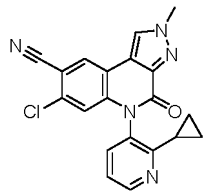

Compound **B348** was obtained according to a synthetic route similar to that of Example 45 with 1-Boc-3-aminoindazole replaced by 2-cyclopropyl-3-aminopyridine.

Example 170: Synthesis of Compound **B372**

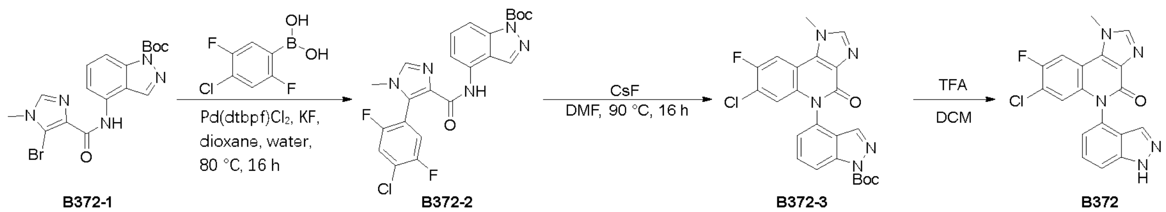


B372-1 B372-2 B372-3 B372

1) Synthesis of intermediate **B372-1:**

Compound **B372-1** was obtained according to a preparation method similar to that for **B29-2** in Example 23 with **B29-1** replaced by 5-bromo-1-methylimidazole-4-carboxylic acid and 3-aminopyridine replaced by 1-Boc-3-aminoindazole.

2) Synthesis of intermediate **B372-2:**

**B372-1** (0.5 g, 1.19 mmol), (4-chloro-2,5-difluorophenyl)boronic acid (0.34 g, 1.78 mmol), [1,1'-bis(di-tert-butylphosphino)ferrocene]palladium dichloride (87 mg, 0.12 mmol), potassium fluoride (490 mg, 3.57 mmol), 1,4-dioxane (2.5 mL), and water (0.5 mL) were added to a reaction flask. The mixture was stirred at 80 °C for 16 h under nitrogen atmosphere. The reaction solution was diluted with ethyl acetate and washed with water. The organic phase was concentrated, and the crude product was purified by column chromatography to give **B372-2** (70 mg, 8%) as a white solid product.

3) Synthesis of intermediate **B372-3:**

**B372-2** (70 mg, 0.14 mmol), cesium fluoride (110 mg, 0.70 mmol), and *N*,*N*-dimethylformamide (1 mL) were added to a reaction flask. The mixture was stirred at 80 °C for 16 h. The reaction solution was diluted with dichloromethane and washed with water. The organic phase was concentrated, and the crude product was purified by column chromatography to give **B372-3** (60 mg, 89%) as a yellow solid.

4) Synthesis of compound **B372:**

**B372-3** (60 mg, 0.13 mmol), trifluoroacetic acid (0.5 mL), and dichloromethane (0.5 mL) were added to a reaction flask. The mixture was stirred at 80 °C for 16 h. The reaction solution was concentrated and purified by reversed-phase column chromatography to give **B372** (22 mg, 43%) as a white solid.

Example 171: Synthesis of Compound **B351**

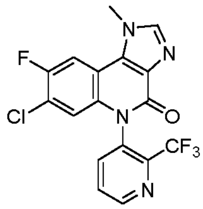

Compound **B351** was obtained according to a synthetic route similar to that of Example 170 with 1-Boc-3-aminoindazole replaced by 2-trifluoromethyl-3-aminopyridine.

Example 172: Synthesis of Compound **B374**

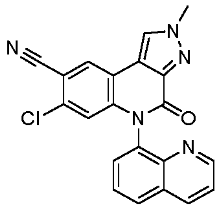

Compound **B374** was obtained according to a synthetic route similar to that of Example 45 with 1-Boc-3-aminoindazole replaced by 8-aminoquinoline.

Example 173: Synthesis of Compound **B375**

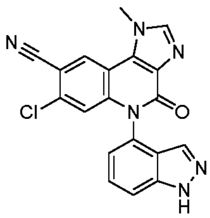

Compound **B375** was obtained according to a synthetic route similar to that of Example 170 with 4-chloro-2,5-difluorophenylboronic acid replaced by 2-fluoro-4-chloro-5-cyanophenylboronic acid pinacol ester in step 1.

Example 174: Synthesis of Compound **B377**

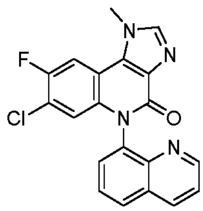

Compound **B377** was obtained according to a synthetic route similar to that of Example 170 with 1-Boc-3-aminoindazole replaced by 8-aminoquinoline.

Example 175: Synthesis of Compound **B378**

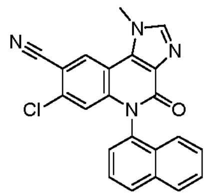

Compound **B378** was obtained according to a synthetic route similar to that of Example 170 with 4-chloro-2,5-difluorophenylboronic acid replaced by 2-fluoro-4-chloro-5-cyanophenylboronic acid pinacol ester and 1-Boc-3-aminoindazole replaced by 1-aminonaphthalene.

Example 176: Synthesis of Compound **B379**

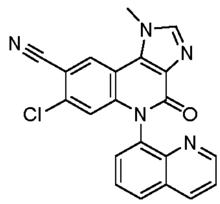

Compound **B379** was obtained according to a synthetic route similar to that of Example 170 with 4-chloro-2,5-difluorophenylboronic acid replaced by 2-fluoro-4-chloro-5-cyanophenylboronic acid pinacol ester and 1-Boc-3-aminoindazole replaced by 8-aminoquinoline.

Example 177: Synthesis of Compound **B381**

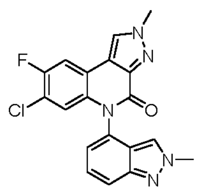

Compound **B381** was obtained according to a synthetic route similar to that of Example 28 with 2-methyl-3-aminopyridine replaced by 2-methyl-4-aminoindazole.

Example 178: Synthesis of Compound **B382**

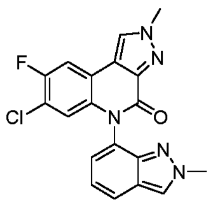

Compound **B382** was obtained according to a synthetic route similar to that of Example 28 with 2-methyl-3-aminopyridine replaced by 2-methyl-7-aminoindazole.

Example 179: Synthesis of Compound **B394**

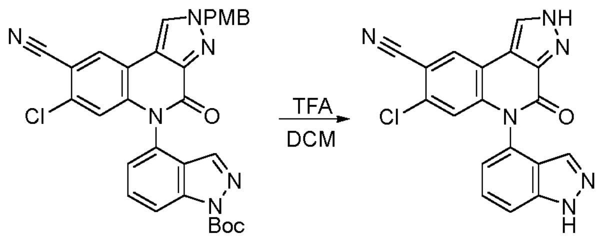


**B394-1** **B394**

1) Synthesis of intermediate **B394-1:**

Intermediate **B394-1** was obtained according to a synthetic route similar to that of Example 45 with 4-bromo-1-methylpyrazole-3-carboxylic acid replaced by 4-bromo-1-benzylpyrazole-3-carboxylic acid.

2) Synthesis of compound **B394:**

Compound **B394** was obtained according to the conditions similar to those for the preparation of **B178** in Example 33.

Example 180: Synthesis of Compound **B400**

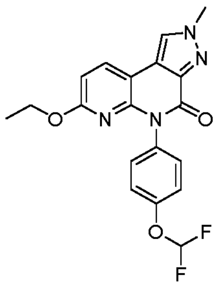

Compound **B400** was obtained according to a synthetic method similar to that for **B253** in Example 164 with B244 replaced by B186.

Example 181: Synthesis of Compound **B401**

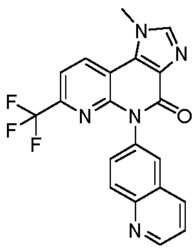

Compound **B401** was obtained according to a synthetic method similar to that of Example 23 with 4-bromo-1-ethylpyrazole-3-carboxylic acid replaced by 5-bromo-1-methylimidazole-4-carboxylic acid and 3-aminopyridine replaced by 6-aminoquinoline in step 1.

Example 182: Synthesis of Compound **B402**

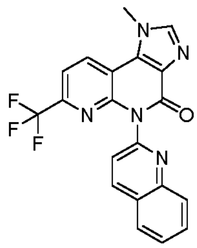

Compound **B402** was obtained according to a synthetic method similar to that of Example 23 with 4-bromo-1-ethylpyrazole-3-carboxylic acid replaced by 5-bromo-1-methylimidazole-4-carboxylic acid and 3-aminopyridine replaced by 2-aminoquinoline in step 1.

Example 183: Synthesis of Compound **B403**

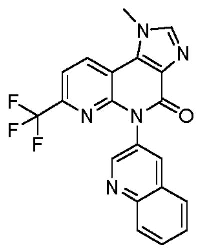

Compound **B403** was obtained according to a synthetic method similar to that of Example 23 with 4-bromo-1-ethylpyrazole-3-carboxylic acid replaced by 5-bromo-1-methylimidazole-4-carboxylic acid and 3-aminopyridine replaced by 3-aminoquinoline in step 1.

Example 184: Synthesis of Compound **B404**

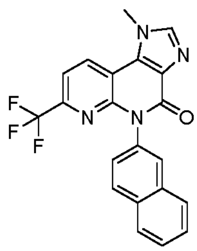

Compound **B404** was obtained according to a synthetic method similar to that of Example 23 with 4-bromo-1-ethylpyrazole-3-carboxylic acid replaced by 5-bromo-1-methylimidazole-4-carboxylic acid and 3-aminopyridine replaced by 2-aminonaphthalene in step 1.

Example 185: Synthesis of Compound **B405**

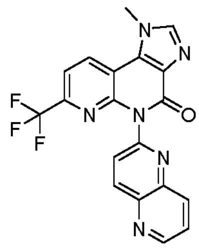

Compound **B405** was obtained according to a synthetic method similar to that of Example 23 with 4-bromo-1-ethylpyrazole-3-carboxylic acid replaced by 5-bromo-1-methylimidazole-4-carboxylic acid and 3-aminopyridine replaced by 2-amino-1,5-dinaphthyridine in step 1.

Example 186: Synthesis of Compound **B408**

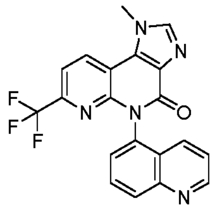

Compound **B408** was obtained according to a synthetic method similar to that of Example 23 with 4-bromo-1-ethylpyrazole-3-carboxylic acid replaced by 5-bromo-1-methylimidazole-4-carboxylic acid and 3-aminopyridine replaced by 5-aminoquinoline in step 1.

Example 187: Synthesis of Compound **B407**

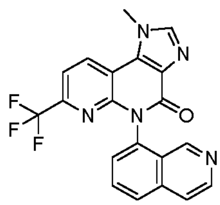

Compound **B407** was obtained according to a synthetic method similar to that of Example 23 with 4-bromo-1-ethylpyrazole-3-carboxylic acid replaced by 5-bromo-1-methylimidazole-4-carboxylic acid and 3-aminopyridine replaced by 8-aminoisoquinoline in step 1.

Example 188: Synthesis of Compound **B409**

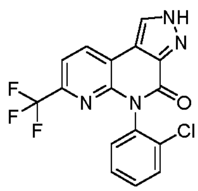

Compound **B409** was obtained according to a synthetic method similar to that of Example 33 with 3-aminopyridine replaced by o-chloroaniline.

Example 189: Synthesis of Compound **B410**

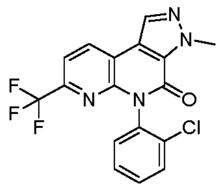

Compound **B410** was obtained according to a synthetic method similar to that of Example 23 with 4-bromo-1-ethylpyrazole-3-carboxylic acid replaced by 4-bromo-2-methylpyrazole-3-carboxylic acid and 3-aminopyridine replaced by o-chloroaniline in step 1.

Example 190: Synthesis of Compound **B406**

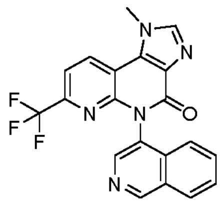

Compound **B406** was obtained according to a synthetic method similar to that of Example 23 with 4-bromo-1-ethylpyrazole-3-carboxylic acid replaced by 5-bromo-1-methylimidazole-4-carboxylic acid and 3-aminopyridine replaced by 4-aminoisoquinoline in step 1.

Example 191: Synthesis of Compound **B286**

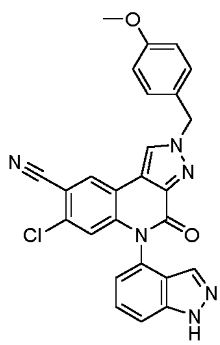

Compound **B286** was obtained according to a synthetic method similar to that of Example 45 with 4-bromo-1-methylpyrazole-3-carboxylic acid replaced by 4-bromo-1-*p*-methoxybenzylpyrazole-3-carboxylic acid.

Example 192: Synthesis of Compound **B293**

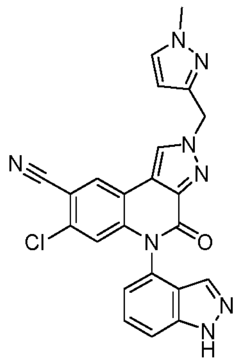

Compound **B293** was obtained according to a synthetic method similar to that of Example 45 with 4-bromo-1-methylpyrazole-3-carboxylic acid replaced by 4-bromo-1-(1-methylpyrazole-3-methyl)pyrazole-3-carboxylic acid.

Example 193: Synthesis of Compound **B393**

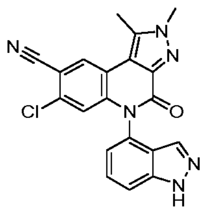

Compound **B393** was obtained according to a synthetic method similar to that of Example 45 with 4-bromo-1-methylpyrazole-3-carboxylic acid replaced by 4-bromo-1,5-dimethylpyrazole-3-carboxylic acid.

Example 194: Synthesis of Compound **B396**

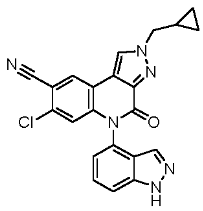

Compound **B396** was obtained according to a synthetic method similar to that of Example 45 with 4-bromo-1-methylpyrazole-3-carboxylic acid replaced by 4-bromo-1-cyclopropylmethylpyrazole-3-carboxylic acid.

Example 195: Synthesis of Compound **B412**

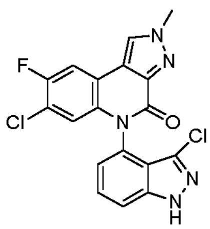

**B257** (0.15 g, 0.41 mmol) was dissolved in a solution of *N*,*N*-dimethylformamide (2 mL), *N*-chlorosuccinimide

(0.11 g, 0.82 mmol) was added with stirring, and the mixture was stirred at 25 °C for reaction for 16 h. When LCMS indicated the completion of the reaction, the reaction solution was filtered to give a filtrate, which was purified by reversed-phase column chromatography (formic acid 0.05% aqueous solution/acetonitrile) to give **B412** (7 mg, 4.22%) as a white product.

Example 196: Synthesis of Compound **B413**

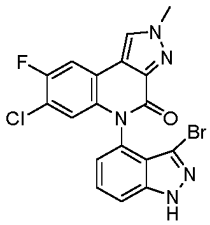

**B257** (0.15 g, 0.41 mmol) was dissolved in a solution of *N*,*N*-dimethylformamide (2 mL), *N*-bromosuccinimide (0.15 g, 0.82 mmol) was added with stirring, and the mixture was stirred at 25 °C for reaction for 16 h. When LCMS indicated the completion of the reaction, the reaction solution was filtered to give a filtrate, which was purified by reversed-phase column chromatography (formic acid 0.05% aqueous solution/acetonitrile) to give **B413** (33 mg, 18%) as a white product.

Example 197: Synthesis of Compound **B414**

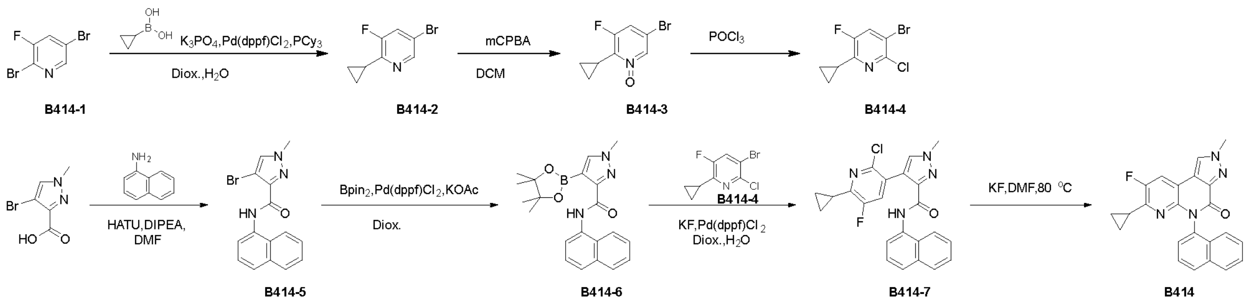


1) Synthesis of intermediate **B414-2:**

**B414-1** (5.00 g, 19.62 mmol), cyclopropylboronic acid (1.85 g, 21.58 mmol), and potassium phosphate (10.41 g, 49.05 mmol) were added to a solution of 1,4-dioxane (50 mL). After the mixture was purged three times with nitrogen, [1,1'-bis(diphenylphosphino)ferrocene]palladium dichloride (1.44 g, 1.96 mmol) and tricyclohexylphosphine (1.11 g, 3.92 mmol) were added, and the mixture was stirred at 90 °C and reacted for 6 h. When LCMS indicated the completion of the reaction, the reaction solution was poured into water, and the mixture was extracted with dichloromethane (50 mL × 2), concentrated, and purified by column chromatography (petroleum ether/dichloromethane) to give **B414-2** (2.3 g, 39%) as a white oily substance. m/z $[M+H]^+$ =215.9.

2) Synthesis of intermediate **B414-3:**

**B414-2** (2 g, 9.26 mmol) was added to a solution of dichloromethane (40 mL), *m*-chloroperoxybenzoic acid (3.2 g, 18.52 mmol) was added in portions with stirring at 0 °C, and then the mixture was reacted at 25 °C for 16 h. LCMS indicated the completion of the reaction. The reaction solution was poured into water, extracted with ethyl acetate (25 mL × 2), and concentrated to give **B414-3** (0.53 g, 22%) as a white oily substance. m/z $[M+H]^+$ = 231.9.

3) Synthesis of intermediate **B414-4:**

**B414-3** (0.53 g, 2.28 mmol) was added to a solution of phosphorus oxychloride (15 mL), and the mixture was stirred at 80 °C and reacted for 12 h. When LCMS indicated the completion of the reaction, the reaction solution was poured into ice water, extracted with ethyl acetate (10 mL × 2), and concentrated to give **B414-4** (0.11 g, yield: 16.53%) as a

colorless oily substance. m/z $[M+H]^+$ = 249.9.

4) Synthesis of intermediate **B414-5:**

4-Bromo-1-methylpyrazole-3-carboxylic acid (0.3 g, 1.46 mmol) was added to a solution of *N,N*-dimethylformamide (5 mL). 1-Naphthylamine (0.23 g, 1.61 mmol), 2-(7-azabenzotriazole)-*N*,*N*,*N*',*N*'-tetramethyluronium hexafluorophosphate (0.61 g, 1.61 mmol) and *N,N*-diisopropylethylamine (0.57 g, 4.38 mmol) were sequentially added with stirring, and the mixture was reacted at 25 °C overnight. When LCMS indicated the completion of the reaction, the reaction solution was poured into water, and extracted with ethyl acetate (20 mL × 2). The organic phases were combined and concentrated to give **B414-5** (0.21 g, 43%) as a brown solid. m/z $[M+H]^+$ = 330.1.

5) Synthesis of intermediate **B414-6:**

**B414-5** (0.21 g, 0.64 mmol) was dissolved in a solution of 1,4-dioxane (4 mL). bis(Pinacolato)diboron (0.24 g, 0.96 mmol) and potassium acetate (0.19 g, 1.92 mmol) were added with stirring. After the mixture was purged three times with nitrogen, [1,1'-bis(diphenylphosphino)ferrocene]palladium dichloride (0.05 g, 0.06 mmol) was added, and the mixture was stirred at 100 °C and reacted for 6 h. When LCMS indicated the completion of the reaction, the mixture was purified by column chromatography (ethyl acetate/petroleum ether) to give **B414-6** (0.15 g, 63%) as a white solid.

6) Synthesis of intermediate **B414-7:**

**B414-4** (0.1 g, 0.40 mmol), **B414-6** (0.15 g, 0.40 mmol), and potassium fluoride (0.07 g, 1.2 mmol) were added to a mixed solution of 1,4-dioxane (20 mL) and water (0.5 mL). After the mixture was purged three times with nitrogen, [1,1'-bis(diphenylphosphino)ferrocene]palladium dichloride (0.026 g, 0.04 mmol) was added, and the mixture was stirred at 90 °C and reacted for 12 h. When LCMS indicated the completion of the reaction, the reaction solution was poured into water, extracted with dichloromethane (50 mL × 2), concentrated, and purified by column chromatography (petroleum ether/dichloromethane) to give **B414-7** (40 mg, 24%) as a white oily substance. m/z $[M+H]^+$ =421.1.

7) Synthesis of product **B414:**

**B414-7** (40 mg, 0.09 mmol) was dissolved in a solution of *N,N*-dimethylformamide (2 mL), potassium fluoride (17 mg, 0.29 mmol) was added with stirring, and the mixture was stirred at 80 °C for reaction for 16 h. When LCMS indicated the completion of the reaction, the reaction solution was filtered to give a filtrate, which was purified by reversed-phase column chromatography (formic acid 0.05% aqueous solution/acetonitrile) to give **B414** (3 mg, 8%) as a white product.

Example 198: Synthesis of Compound **B391**

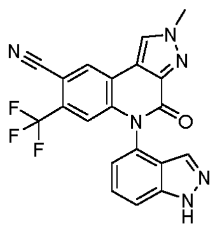

**B391** was obtained according to a synthetic method similar to that for compound B258 in Example 45 with B258-1 replaced by 4-amino-2-trifluoromethylbenzonitrile in step 1.

Example 199: Synthesis of Compound **B283**

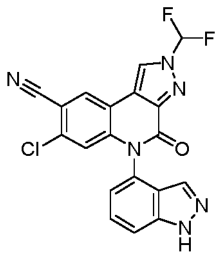

Compound **B283** was obtained according to a synthetic method similar to that for compound B258 in Example 45 with 4-bromo-1-methylpyrazole-3-carboxylic acid replaced by 4-bromo-1-difluoromethylpyrazole-3-carboxylic acid.

Example 200: Synthesis of Compound **B281**

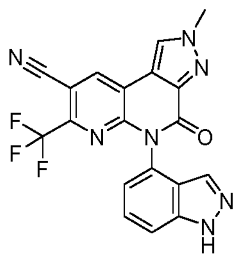

Compound **B281** was obtained according to a synthetic method similar to that of Example 48 with B274-1 replaced by 6-chloro-2-trifluoromethylnicotinonitrile in step 1.

Example 201: Synthesis of Compound **B284**

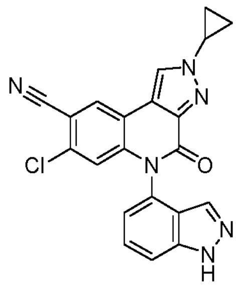

Compound **B284** was obtained according to a synthetic method similar to that of Example 45 with 4-bromo-1-methylpyrazole-3-carboxylic acid replaced by 4-bromo-1-cyclopropylpyrazole-3-carboxylic acid.

Example 202: Synthesis of Compound **B411**

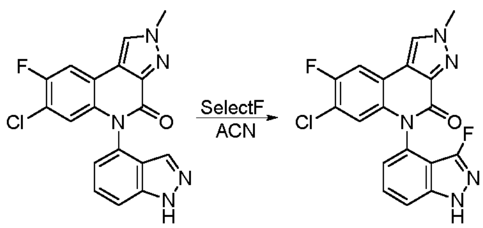


**B257** **B411**

B257 (0.15 g, 0.41 mmol) was dissolved in acetonitrile (2 mL) solution, 1-chloromethyl-4-fluoro-1,4-diazobicyclo [2.2.2]octane bis(tetrafluoroborate) (0.23 g, 0.66 mmol) was added with stirring, and the mixture was stirred at 25 °C for reaction for 36 h. The reaction solution was filtered, and the filtrate was separated by reversed-phase column chromatography to give **B411** (10 mg, 6%) as a white product.

Nuclear magnetic resonance and mass spectrometry analysis data of the exemplary compounds of the present application are shown below:

| No. | Structure | Nuclear magnetic resonance and mass spectrometry data |
|---|---|---|
| B2 | 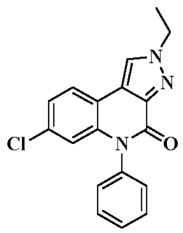 | $^{1}$H NMR (400 MHz, DMSO-$d_6$) δ 8.86 (s, 1H), 8.05 (d, J = 8.3 Hz, 1H), 7.69 (dd, J = 8.3, 6.7 Hz, 2H), 7.66 - 7.58 (m, 1H), 7.45 - 7.38 (m, 2H), 7.34 (dd, J = 8.4, 2.0 Hz, 1H), 6.39 (d, J = 2.0 Hz, 1H), 4.48 (q, J = 7.3 Hz, 2H), 1.55 (t, J = 7.3 Hz, 3H). LC-MS. $[M+H]^+$=324.1 |
| B3 | 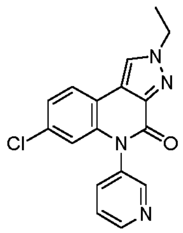 | $^{1}$H NMR (400 MHz, DMSO-$d_6$) δ 8.85 (s, 1H), 8.79 (dd, J = 4.8, 1.5 Hz, 1H), 8.63 (d, J = 2.4 Hz, 1H), 8.05 (d, J = 8.4 Hz, 1H), 7.94 (ddd, J = 8.1, 2.5, 1.6 Hz, 1H), 7.72 (dd, J = 8.1, 4.8 Hz, 1H), 7.35 (dd, J = 8.3, 2.0 Hz, 1H), 6.38 (d, J = 2.0 Hz, 1H), 4.46 (q, J = 7.3 Hz, 2H), 1.53 (t, J = 7.3 Hz, 3H). LC-MS. $[M+H]^+$=325.1 |
| B4 | 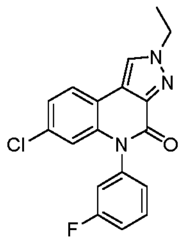 | $^{1}$H NMR (400 MHz, DMSO-$d_6$): δ 8.84 (s, 1H), 8.04-8.02 (m, 1 H), 7.73-7.67 (m, 1 H), 7.48-7.40 (m, 2H), 7.32-7.32 (m, 1H), 7.27-7.26 (m, 1H), 6.39 (s, 1H), 4.48-4.42 (m, 2H), 1.53-1.50 (m, 3 H). LC-MS. $[M+H]^+$ = 342.1 |
| B5 | 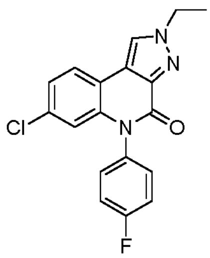 | $^{1}$H NMR (400 MHz, DMSO-$d_6$): δ 8.83 (s, 1H), 8.02 (d, J=8Hz, 1H), 7.53-7.43(m, 4H), 7.34-7.30(m, 1H), 6.40(d, *J*=1.6Hz, 1H), 4.49-4.40(m, 2H),1.56-1.48 (m, 3H). LC-MS $[M+H]^+$ =342.1 |
| B6 | 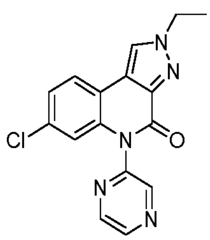 | $^{1}$H NMR (400 MHz, DMSO-$d_6$): δ 8.94-8.87 (m, 4H), 8.06(d, *J*=8.4Hz, 1H), 7.39-7.36 (m, 1H), 6.46(s, 1H),4.49-4.44(m, 2H), 1.53 (t, 3H). LC-MS $[M+H]^+$ =326.1 |
| B7 | 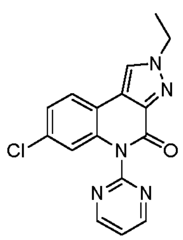 | $^{1}$H NMR (400 MHz, DMSO-$d_6$): δ 9.15 (d, J=4.4Hz, 2H), 8.88 (s, 1H), 8.07(d, *J*=8.8Hz, 1H), 7.85-7.77(m, 1H), 7.37(d, *J*=8.6Hz, 1H), 6.28(s, 1H),4.56-4.41 (m, 2H),1.58-1.48(m,3H). LC-MS $[M+H]^+$ =326.1 |

(continued)

| No. | Structure | Nuclear magnetic resonance and mass spectrometry data |
|---|---|---|
| B8 | 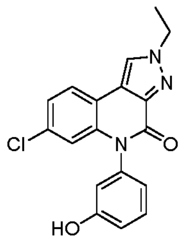 | $^1$H NMR (400 MHz, DMSO-d6): δ9.87 (brs, 1H), 8.88 (s, 1H), 8.02 (d,*J*=8.4 Hz, 1H), 7.46-7.42 (m, 1H), 7.30 (dd, $J_1$=2.0 Hz, $J_2$=8.4 Hz 1H), 6.99-6.96 (m, 1H), 6.77-6.71 (m, 2H), 6.45 (d, *J*=2.0 Hz, 1H), 4.44 (q, *J*=7.2 Hz, 2H), 1.52 (t, *J*=7.2 Hz, 3H). LC-MS $[M+H]^+$ =340.0 |
| B9 | 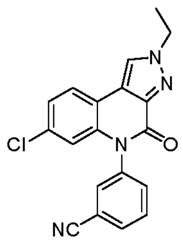 | $^1$H NMR (400 MHz, DMSO-d6): δ 8.85 (s, 1H), 8.08-8.04 (m, 3H), 7.89-7.79 (m, 2H), 7.34 (dd, $J_1$=2.0 Hz, $J_2$=8.4 Hz 1H), 6.38 (d, *J*=2.0 Hz, 1H), 4.45 (q, *J*=7.2 Hz, 2H), 1.52 (t, *J*=7.2 Hz, 3H). LC-MS $[M+H]^+$ =349.0 |
| B10 | 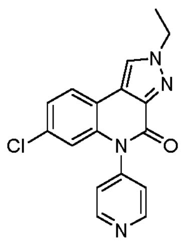 | $^1$H NMR (400 MHz, DMSO-$d_6$): δ 8.89 (d, *J*=5.2Hz, 2H), 8.85(s, 1H), 8.05 (d, *J*=7.6Hz, 1H), 7.53 (d, *J*=2.4Hz, 2H), 7.37-7.34(m, 1H), 6.40(s, 1H),4.48-4.43(m, 2H), 1.52 (t, 3H). LC-MS $[M+H]^+$ =325.1 |
| B11 | 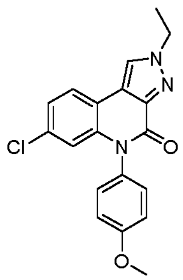 | $^1$H NMR (400 MHz, DMSO-$d_6$): δ 8.83 (s, 1H), 8.01 (d, J=8.4Hz, 1H), 7.32-7.29(m, 3H), 7.18-7.16(m, 2H), 7.44(d, *J*=2.0Hz, 1H), 4.47-4.41(m, 2H), 3.87 (s, 3H),1.53-1.50 (m, 3H). LC-MS $[M+H]^+$ =354.1 |
| B12 | 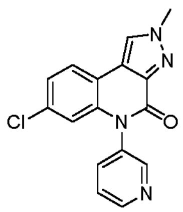 | $^1$H NMR (400 MHz, DMSO-$d_6$): δ 8.78 (d, J=4.0Hz, 2H), 8.62 (d, J=0.8Hz, 1H), 8.05 (d, J=8.0Hz, 1H), 7.96-7.93(m, 1H), 7.72-7.69(m, 1H), 7.36-7.33(m, 1H), 6.38(d, J=2 Hz, 1H), 4.16 (s, 3H). LC-MS $[M+H]^+$ =311.1 |
| B13 | 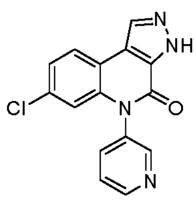 | $^1$H NMR (400 MHz, DMSO_$d_6$): δ 10.08-9.26 (m, 1H), 8.80 (dd, $J_1$=4.8 Hz, $J_2$=1.2 Hz, 1H), 8.69-8.64 (m, 2H), 8.18 (d, *J*=8.4 Hz, 1H), 8.00-7.95 (m, 1 H), 7.72 (dd, $J_1$=8.0 Hz,$J_2$=4.8 Hz,1H), 7.39 (dd, $J_1$=8.4 Hz,$J_2$=2.0 Hz, 1H), 6.43 (d, *J*=1.6 Hz, 1H). LC-MS $[M+H]^+$ = 297.0 |

(continued)

| No. | Structure | Nuclear magnetic resonance and mass spectrometry data |
|---|---|---|
| B14 | 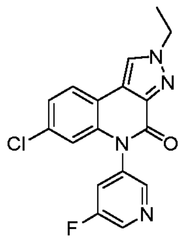 | $^1$H NMR (400 MHz, DMSO-$d_6$): δ 8.86 (s, 1H), 8.03 (d, J=2.4Hz, 1H), 8.54 (s, 1H),8.10-8.04(m, 2H), 7.38-7.35(m, 1H), 6.51(d, *J*=2.0Hz, 1H), 4.49-4.43(m, 2H),1.54-1.50 (m, 3H). LC-MS $[M+H]^+$ =343.0 |
| B15 | 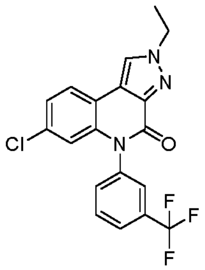 | 1H NMR (400 MHz, Chloroform-d) δ 8.14 (s, 1H), 7.90 - 7.78 (m, 2H), 7.76 (d, J = 8.3 Hz, 1H), 7.64 (d, J = 2.1 Hz, 1H), 7.61 - 7.55 (m, 1H), 7.24 (dd, J = 8.3, 1.9 Hz, 1H), 6.55 (d, J = 1.9 Hz, 1H), 4.54 (q, J = 7.3 Hz, 2H), 1.71 (t, J = 7.3 Hz, 3H). LC-MS. $[M+H]^+$=392.1 |
| B16 | 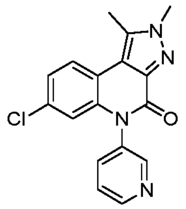 | $^1$H NMR (400 MHz, DMSO-$d_6$): δ 8.78 (d, J=3.6Hz, 1H), 8.60 (d, J=2.0Hz, 1H), 8.07 (d, J=8.4Hz, 1H), 7.94-7.91(m, 1H), 7.72-7.69(m, 1H), 7.34-7.31(m, 1H), 6.38(d, J=2 Hz, 1H), 4.07 (s, 3H),2.78(s,3H). LC-MS $[M+H]^+$ =325.0 |
| B17 | 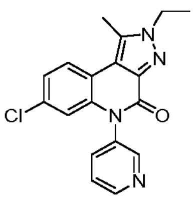 | $^1$H NMR (400 MHz, DMSO-$d_6$): δ 8.78-8.79 (m, 1H), 8.60 (d, *J* = 2.4Hz, 1H), 8.08 (d, J=8.8Hz, 1H), 7.91-7.94 (m, 1H), 7.70-7.73 (m, 1H), 7.32-7.35 (m, 1H), 6.38(d, *J*=2.0Hz, 1H), 4.44 (q, *J*=8.0Hz, 2H), 2.82 (s, 3H), 1.42 (t, *J* = 6.0Hz, 3H). LC-MS $[M+H]^+$ =339.0 |
| B18 | 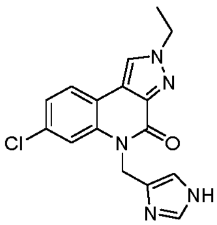 | $^1$H NMR (400 MHz, DMSO-$d_6$) δ 12.00 (s, 1H), 8.78 (s, 1H), 7.97 (d, *J* = 8.3 Hz, 1H), 7.87 (s, 1H), 7.59 (s, 1H), 7.32 (dd, *J* = 8.3, 1.8 Hz, 1H), 6.97 (s, 1H), 5.41 (s, 2H), 4.45 (q, *J* = 7.3 Hz, 2H), 1.54 (t, *J* = 7.3 Hz, 3H). LC-MS. $[M+H]^+$=328.1 |
| B19 | 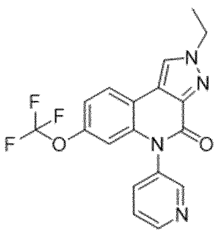 | $^1$H NMR (400 MHz, DMSO-d6): δ 8.88 (s, 1H), 8.78-8.80 (m, 1H), 8.64 (d, *J* = 2.2Hz, 1H), 8.15 (d, *J* = 8.6Hz, 1H), 7.94-7.97 (m, 1H), 7.70-7.73 (m, 1H), 7.31 (d, *J*= 9.6Hz, 1H), 6.29 (s, 1H), 4.47 (q, *J* = 7.3Hz, 2H), 1.53 (t,*J* = 7.3Hz, 3H). LC-MS $[M+H]^+$ =375.0 |

(continued)

| No. | Structure | Nuclear magnetic resonance and mass spectrometry data |
|---|---|---|
| B20 | 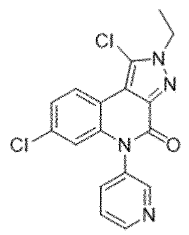 | 1HNMR (400 MHz, DMSO-d6): δ 8.80 (dd, $J_1$=1.6 Hz, $J_2$=4.8 Hz 1H), 8.64 (d, $J$=2.0 Hz, 1H), 8.30 (d, $J$=8.4 Hz, 1H), 7.97-7.94 (m, 1H), 7.74-7.71 (m, 1H), 7.43 (dd, $J_1$=1.6 Hz, $J_2$=8.0 Hz 1H), 6.42 (d, $J$=2.0 Hz, 1H), 4.49 (q, $J$=7.2 Hz, 2H), 1.48 (t, $J$=7.2 Hz, 3H). LC-MS $[M+H]^+$ =358.9 |
| B21 | 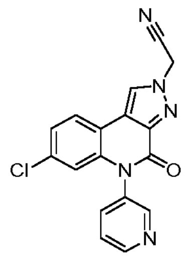 | $^1$H NMR (400 MHz, $CDCl_3$): δ 8.83 (d, $J$=3.6 Hz, 1H), 8.63-8.58 (m, 1H), 8.32 (s, 1H), 7.77-7.68 (m, 2H), 7.64-7.58 (m, 1H), 7.25-7.22 (m, 1H), 6.58 (d, $J$=1.6 Hz, 1H), 5.42 (s, 2H). LC-MS $[M+H]^+$= 336.0 |
| B22 | 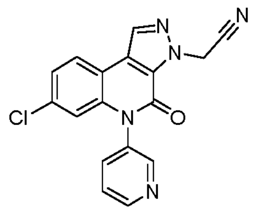 | $^1$H NMR (400 MHz, $CDCl_3$): δ 8.87 (dd,$J_1$=4.4 Hz, $J_2$=1.2 Hz, 1H), 8.61 (d, $J$=2.4 Hz, 1H), 8.30 (s, 1 H), 7.90 (d, $J$=8.4 Hz, 1H), 7.75-7.70 (m, 1 H), 7.67-7.61 (m, 1 H), 7.34 (dd,$J_1$=8.0 Hz, $J_2$= 1.6 Hz, 1H), 6.66 (d, $J$=1.6 Hz, 1H), 5.83-5.69 (m, 2 H). LC-MS $[M+H]^+$= 336.0 |
| B23 | 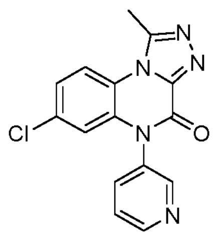 | $^1$H NMR (400 MHz, $d_6$-DMSO) δ 8.88 - 8.75 (m, 1H), 8.67 (t, $J$ = 11.2 Hz, 1H), 8.22 (d, $J$ = 8.9 Hz, 1H), 7.99 (d, $J$ = 8.1 Hz, 1H), 7.76 (dd, $J$= 8.0, 4.8 Hz, 1H), 7.47 (dd, $J$ = 8.8, 2.2 Hz, 1H), 6.54 (d, $J$ = 2.2 Hz, 1H), 3.06 (s, 3H). LCMS $[M+H]^+$ = 311.9 |
| B24 | 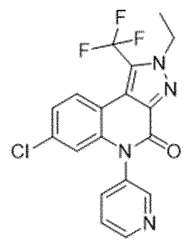 | 1H NMR (400 MHz, DMSO) δ 8.81 (dd, J = 4.8, 1.4 Hz, 1H), 8.66 (d, J = 2.2 Hz, 1H), 8.04 - 7.93 (m, 2H), 7.74 (dd, J = 8.0, 4.8 Hz, 1H), 7.46 (dd, J = 8.7, 2.1 Hz, 1H), 6.48 (d, J = 2.1 Hz, 1H), 4.70 (q, J = 7.1 Hz, 2H), 1.52 (t, J = 7.2 Hz, 3H).LC-MS $[M+H]^+$ =392.9 |
| B25 | 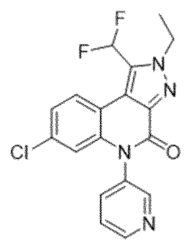 | 1H NMR (400 MHz, DMSO) δ 8.80 (dd, J = 4.8, 1.4 Hz, 1H), 8.66 (d, J = 2.2 Hz, 1H), 8.15 (d, J = 8.6 Hz, 1H), 8.10 - 7.79 (m, 2H), 7.73 (dd, J = 8.0, 4.9 Hz, 1H), 7.43 (dd, J = 8.6, 2.0 Hz, 1H), 6.44 (d, J = 2.0 Hz, 1H), 4.68 (q, J = 7.2 Hz, 2H), 1.48 (t, J = 7.2 Hz, 3H).LC-MS $[M+H]^+$ =375.0 |

(continued)

| No. | Structure | Nuclear magnetic resonance and mass spectrometry data |
|---|---|---|
| B26 | 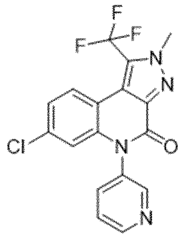 | $^1$H NMR (400 MHz, DMSO) δ 8.81 (d, *J* = 4.8 Hz, 1H), 8.66 (d, *J* = 2.2 Hz, 1H), 7.98 (dd, *J* = 8.3, 4.3 Hz, 2H), 7.73 (dd, *J* = 8.0, 4.8 Hz, 1H), 7.47 (dd, *J*= 8.7, 1.9 Hz, 1H), 6.49 (d, *J* = 1.9 Hz, 1H), 4.37 (d, *J* = 1.9 Hz, 3H). LC-MS $[M+H]^+$ =378.9 |
| B27 | 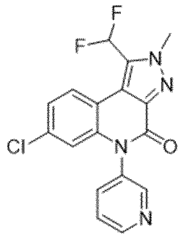 | 1H NMR (400 MHz, DMSO) δ 8.80 (d, J = 4.3 Hz, 1H), 8.76 (t, J = 27.5 Hz, 1H), 8.66 (s, 1H), 8.14 (d, J = 8.6 Hz, 1H), 8.02 - 7.66 (m, 2H), 7.43 (dd, J = 8.6, 1.9 Hz, 1H), 6.45 (d, J = 1.9 Hz, 1H), 4.33 (s, 3H). LC-MS $[M+H]^+$ =360.9 |
| B28 | 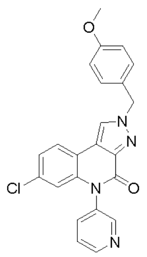 | 1H NMR (400 MHz, DMSO-d6) δ 8.88 (s, 1H), 8.78 (d, J = 4.7 Hz, 1H), 8.62 (s, 1H), 8.07 (d, J = 8.3 Hz, 1H), 7.93 (ddd, J = 8.1, 2.5, 1.5 Hz, 1H), 7.71 (dd, J = 8.0, 4.8 Hz, 1H), 7.43 - 7.30 (m, 3H), 7.03 - 6.89 (m, 2H), 6.36 (d, J = 2.0 Hz, 1H), 5.58 (s, 2H), 3.74 (s, 3H). LC-MS [M+H] + = 417.0 |
| B29 | 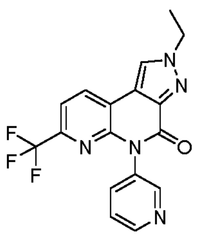 | $^1$H NMR (400 MHz, DMSO-d6): δ 9.00 (s, 1H), 8.66 (d, *J*=7.6Hz, 2H), 8.55 (d, *J*=2.0Hz 1H), 7.86-7.83 (m, 1H), 7.80-7.78 (m, 1H), 7.62-7.59 (m, 1H), 4.52-4.47 (m, 2H), 1.55 (t, 3H). LC-MS $[M+H]^+$ =360.0 |
| B30 | 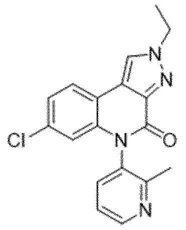 | $^1$H NMR (400 MHz, DMSO-d6): δ 8.87 (s, 1H), 8.68-8.67 (m, 1H), 8.06 (d, *J*=8.0Hz, 1H), 7.83-7.80 (m, 1H), 7.54-7.51 (m, 1H), 7.38-7.35 (m, 1H), 6.30(d, *J*=2.0Hz,1 H), 4.52-4.47 (m, 2H), 2.16 (s, 3H) , 1.56-1.52 (m, 3H). LC-MS [M+H] + =339.0 |
| B31 | 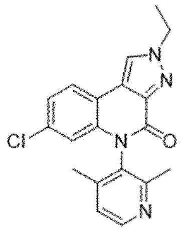 | 1H NMR (400 MHz, DMSO) δ 8.90 (s, 1H), 8.53 (d, J = 4.8 Hz, 1H), 8.11 (d, J = 8.3 Hz, 1H), 7.41 (dd, J = 13.9, 6.7 Hz, 2H), 6.29 (s, 1H), 4.47 (q, J = 7.2 Hz, 2H), 2.09 (s, 3H), 1.94 (s, 3H), 1.55 (t, J = 7.2 Hz, 3H). LC-MS $[M+H]^+$ = 353.0 |

(continued)

| No. | Structure | Nuclear magnetic resonance and mass spectrometry data |
|---|---|---|
| B32 | 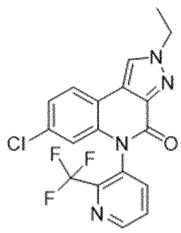 | $^1$H NMR (400 MHz, DMSO-d6): δ 8.99 (d, *J*=4.0Hz, 1H), 8.87 (s, 1H), 8.25(d, *J*=8.0Hz, 1H), 8.08-8.05 (m, 2H), 7.38-7.36 (m, 1H), 6.38 (d, *J*=1.6Hz, 1H), 4.49-4.36 (m, 2H), 1.56-1.52 (t, 3H).<br>LC-MS $[M+H]^+$ =393.0 |
| B33 | 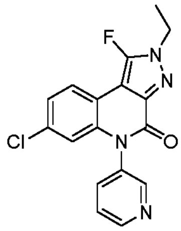 | $^1$H NMR (400 MHz, DMSO_$d_6$): δ 8.79 (d, *J*=4.8 Hz, 1 H), 8.63 (s, 1H), 7.96-7.93 (m, 1H), 7.86 (d, *J*=8.0 Hz, 1 H), 7.73-7.70 (m, 1H), 7.38 (d, *J*=8.0 Hz, 1 H), 6.39 (s, 1H), 4.42-4.37 (m, 2 H), 1.48 (t, *J*=7.6 Hz, 3 H). LC-MS [M+H] $^+$= 342.9 |
| B34 | 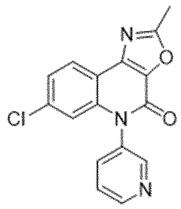 | $^1$H NMR (400 MHz, DMSO_$d_6$): δ 8.81 (d, *J*=4.0 Hz, 1 H), 8.66 (d, *J*=1.6 Hz, 1H), 8.14 (d, *J*=8.4 Hz, 1 H), 7.97 (d, *J*=8.0 Hz, 1 H), 7.75-7.72 (m, 1H), 7.50-7.48 (m, 1 H), 6.60 (S, 1 H), 2.75 (S, 3H).<br>LC-MS $[M+H]^+$ = 312.0 |
| B35 | 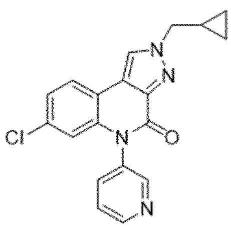 | 1H NMR (400 MHz, DMSO-d6) δ 8.88 (s, 1H), 8.78-8.79 (m, 1H), 8.63 (d, *J* = 2.4 Hz, 1H), 8.08 (d, *J* = 8.4 Hz, 1H), 8.01 - 7.90 (m, 1H), 7.69-7.73 (m, 1H), 7.34-7.36 (m, 1H), 6.38 (d, *J* = 2.0 Hz, 1H), 4.30 (d, *J* = 7.3 Hz, 2H), 1.38 (d, *J* = 12.3 Hz, 1H), 0.66 - 0.49 (m, 4H). LC-MS $[M+H]^+$ =351.0 |
| B36 | 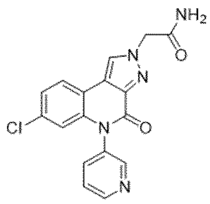 | $^1$H NMR (400 MHz, DMSO_$d_6$): δ 8.81-8.76 (m, 2H), 8.63 (d, *J*=2.4 Hz, 1H), 8.11 (d, *J*=8.0 Hz,1H), 7.97-7.92 (m, 1 H), 7.76-7.69 (m, 2 H) , 7.44-7.40 (m, 1H), 7.35 (dd, $J_1$=8.0 Hz, $J_2$=2.0 Hz,1H), 6.38 (d, *J*=2.0 Hz,1H), 5.12 (s, 2H).<br>LC-MS $[M+H]^+$ =353.9 |
| B37 | 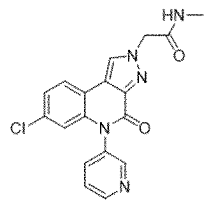 | $^1$H NMR (400 MHz, DMSO_$d_6$): δ 8.82-8.77 (m, 2H), 8.63 (d, *J*=2.0 Hz, 1H), 8.29-8.23 (m, 1H), 8.11 (d,*J*=8.4 Hz, 1H), 7.97-7.92 (m, 1H), 7.72 (dd, $J_1$=7.6 Hz, $J_2$=4.8 HZ,1H) , 7.35 (dd, $J_1$=8.0 Hz, $J_2$=2.0 Hz,1H), 6.38 (d, *J*=2.0 Hz, 1H), 5.12 (s, 2H), 2.70-2.63 (m, 3H). LC-MS $[M+H]^+$ =368.0 |
| B38 | 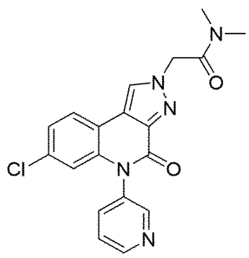 | $^1$H NMR (400 MHz, DMSO_$d_6$): δ 8.85-8.81 (m, 1H), 8.74-8.69 (m, 2 H), 8.11 (d, *J*=8.4 Hz, 1H), 8.07-8.03 (m, 1H), 7.78 (dd, $J_1$=8.0 Hz, $J_2$=4.8 Hz,1H) , 7.35 (dd, $J_1$=8.4 Hz, $J_2$=2.0 Hz,1H), 6.43 (d, *J*=1.6 Hz,1H), 5.51 (s, 2H), 3.10 (s, 3H), 2.89 (s, 3H). LC-MS $[M+H]^+$ =382.0 |

(continued)

| No. | Structure | Nuclear magnetic resonance and mass spectrometry data |
|---|---|---|
| B39 | 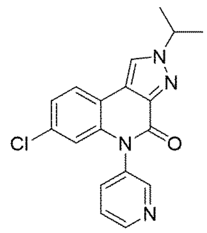 | 1H NMR (400 MHz, DMSO-d6) δ 8.95 (s, 1H), 8.81-8.82(m, 1H), 8.66 (d, *J* = 2.4 Hz, 1H), 8.09 (d, *J* = 8.3 Hz, 1H), 7.96-7.98(m, 1H), 7.72-7.76 (m, 1H), 7.36-7.40 (m, 1H), 6.39 (d, *J* = 2.0 Hz, 1H), 4.84-4.88 (m, 1H), 1.60 (d, *J* = 6.7 Hz, 6H). LC-MS $[M+H]^+$ =339.0 |
| B40 | 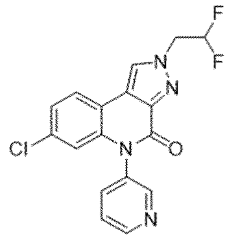 | $^1$H NMR (400 MHz, CDCl3): δ 8.82 (dd, $J_1$ =4.8 Hz, $J_2$ =1.2 Hz,1H), 8.60 (d, *J*=2.0 Hz, 1H), 8.18 (s, 1H), 7.77-7.69 (m, 2 H), 7.60 (dd, $J_1$=8.0 Hz,$J_2$=4.8 Hz,1H) , 7.23 (dd,$J_1$=8.0 Hz, $J_2$=1.6 Hz,1H), 6.56 (d, *J*=2.0 Hz, 1H), 6.42-6.11 (m, 1H), 4.81-4.72 (m,2H) . LC-MS $[M+H]^+$ =361.0 |
| B41 | 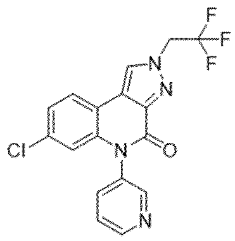 | 1H NMR (400 MHz, DMSO-d6) δ 8.97 (s, 1H), 8.78-8.80(m, 1H), 8.66 (d, *J* = 2.4 Hz, 1H), 8.18 (d, *J* = 8.4 Hz, 1H), 7.96-7.99(m, 1H), 7.70-7.73(m, 1H), 7.35-7.38(m, 1H), 6.38 (d, *J* = 2.0 Hz, 1H), 5.59 (d, *J* = 9.1 Hz, 2H). LC-MS $[M+H]^+$ =379.0 |
| B42 | 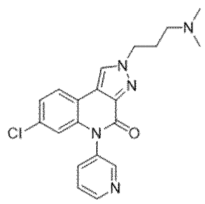 | $^1$H NMR (400 MHz, CDCl3): δ 8.81 (dd,$J_1$=4.8 Hz, $J_2$=1.6 Hz, 1H), 8.60 (d, *J*=2.4 Hz, 1H), 8.13 (s, 1H), 7.73-7.69 (m, 2 H), 7.62-7.57 (m, 1 H) , 7.20 (dd, $J_1$=8.4 Hz, $J_2$=1.6 Hz, 1H), 6.55 (d, *J*=2.0 Hz,1H), 4.50 (t, *J*=6.8 Hz, 2H), 2.31-2.25 (m, 2H) ,2.24 (s, 6H) ,2.22-2.14 (m, 2H) . LC-MS $[M+H]^+$ =382.0 |
| B43 | 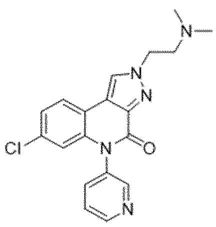 | $^1$H NMR (400 MHz, DMSO_$d_6$): δ 8.82 (s, 1H), 8.78 (dd, $J_1$ =4.8 Hz, $J_2$=1.6 Hz, 1H), 8.63 (d, *J*=2.4 Hz,1H), 8.06 (d, *J*=8.8 Hz,1H), 7.97-7.93 (m, 1 H) , 7.71 (dd,$J_1$=8.0 Hz, $J_2$=4.8 Hz, 1H), 7.34 (dd,$J_1$=8.4 Hz,$J_2$=2.0 Hz, 1H), 6.37 (d, *J*=2.0 Hz, 1H), 4.51 (t, *J*=6.4 Hz,2H), 2.78 (t, *J*=6.4 Hz,2H) , 2.20 (s, 6H) . LC-MS $[M+H]^+$=368.0. |
| B44 | 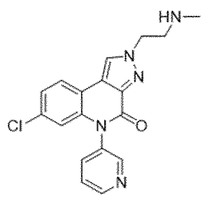 | |
| B45 | 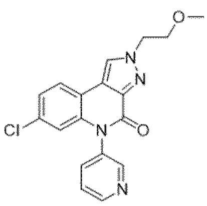 | 1HNMR (400 MHz, DMSO-d6) δ 8.82 (s, 1H), 8.78-8.79(m, 1H), 8.63 (d, *J* = 2.4 Hz, 1H), 8.09 (d, *J* = 8.4 Hz, 1H), 8.00 - 7.88 (m, 1H), 7.69-7.73(m, 1H), 7.33-7.75 (m, 1H), 6.37 (d, *J* = 2.0 Hz, 1H), 4.57-4.60(m, 2H), 3.81-3.83(m, 2H), 3.27 (s, 3H). LC-MS $[M+H]^+$ =355.0 |

(continued)

| No. | Structure | Nuclear magnetic resonance and mass spectrometry data |
|---|---|---|
| B46 | 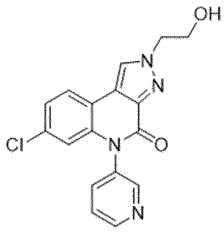 | 1H NMR (400 MHz, DMSO-d6) δ 8.81 (s, 1H), 8.78-8.79 (m, 1H), 8.62 (d, *J* = 2.4 Hz, 1H), 8.10 (d, *J* = 8.3 Hz, 1H), 7.92-7.95 (m, 1H), 7.70-7.73 (m, 1H), 7.33-7.36 (m, 1H), 6.38 (d, *J* = 2.0 Hz, 1H), 5.03-5.06 (m, 1H), 4.44-4.47 (m, 2H), 3.87-3.89 (m, 2H). LC-MS $[M+H]^+$ =341.2 |
| B47 | 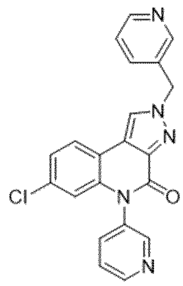 | 1H NMR (400 MHz, DMSO-d6) δ 9.01 (s, 1H), 8.92 - 8.77 (m, 1H), 8.75 - 8.62 (m, 2H), 8.63 - 8.54 (m, 1H), 8.10 (d, *J* = 8.3 Hz, 1H), 7.97 (d, *J* = 8.2 Hz, 1H), 7.82 (d, *J* = 8.0 Hz, 1H), 7.71-7.75 (m, 1H), 7.43-7.48(m, 1H), 7.36-7.39 (m, 1H), 6.39 (d, *J* = 2.0 Hz, 1H), 5.77 (s, 2H). LC-MS $[M+H]^+$ =388.0 |
| B48 | 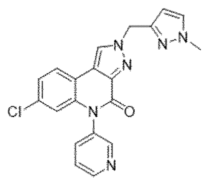 | $^1$H NMR (400 MHz, CDCl3): δ 8.80 (d, *J*=4.4 Hz, 1H), 8.58 (d, *J*=2.0 Hz, 1H), 8.13 (s, 1H), 7.73-7.65 (m, 2H), 7.61-7.57 (m, 1H) , 7.35 (d, *J*=2.4 Hz, 1H), 7.16 (dd, $J_1$=8.8 Hz, $J_2$=2.0 Hz,1H), 6.52 (d, *J*=2.0 Hz, 1H), 6.32 <d, *J*=2.4 Hz, 1H) ,5.60 (s, 2H) ,3.93 (s, 3H) . LC-MS $[M+H]^+$ =391.0 |
| B49 | 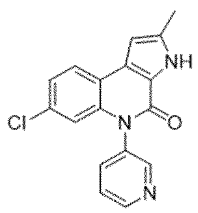 | |
| B50 | 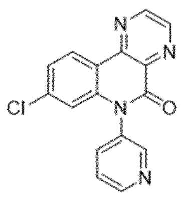 | $^1$H NMR (400 MHz, DMSO) δ 9.22 (d, J = 2.1 Hz, 1H), 9.09 (d, J = 2.1 Hz, 1H), 8.86 (dd, J = 4.8, 1.5 Hz, 1H), 8.79 (d, J = 8.6 Hz, 1H), 8.74 (d, J = 1.9 Hz, 1H), 8.04 (ddd, J = 8.1, 2.4, 1.6 Hz, 1H), 7.79 (ddd, J = 8.1, 4.8, 0.7 Hz, 1H), 7.54 (dd, J = 8.5, 1.9 Hz, 1H), 6.56 (d, J = 1.9 Hz, 1H). LC-MS $[M+H]^+$ = 309.1 |
| B51 | 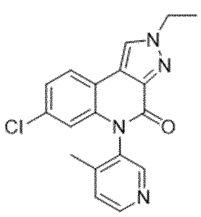 | 1H NMR (400 MHz, DMSO) δ 8.87 (s, 1H), 8.65 (d, J = 5.0 Hz, 1H), 8.48 (s, 1H), 8.10 (t, J = 13.7 Hz, 1H), 7.60 (d, J = 5.0 Hz, 1H), 7.37 (dd, J = 8.3, 1.9 Hz, 1H), 6.30 (d, J = 1.9 Hz, 1H), 4.47 (q, J = 7.3 Hz, 2H), 2.00 (s, 3H), 1.54 (t, J = 7.3 Hz, 3H). LC-MS $[M+H]^+$ = 339.0 |
| B52 | 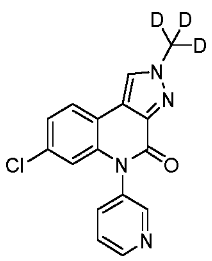 | |

(continued)

| No. | Structure | Nuclear magnetic resonance and mass spectrometry data |
|---|---|---|
| B53 | 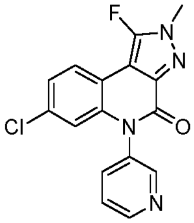 | |
| B54 | 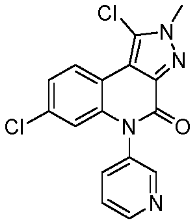 | |
| B55 | 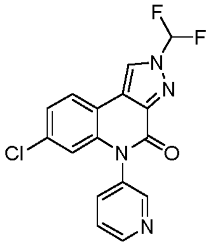 | $^1$H NMR (400 MHz, DMSO_$d_6$): δ 10.00 (s, 1H), 9.73 (d, *J*=6.0 Hz, 1H), 9.21 (d, *J*=8.4 Hz, 1H), 8.76-8.68 (m, 2H), 8.50-8.20 (m, 2 H) , 7.46-7.44 (m, 1 H), 7.06 (s, 1H). LC-MS [M+H] $^+$ = 346.9 |
| B56 | 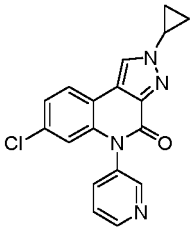 | |
| B57 | 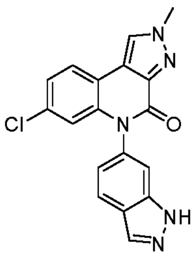 | $^1$H NMR (400 MHz, DMSO-$d_6$) δ 13.42 (s, 1H), 8.79 (s, 1H), 8.22 (s, 1H), 8.05 (d, *J* = 8.3 Hz, 1H), 7.87 - 7.78 (m, 2H), 7.31 (ddd, *J* = 14.2, 8.5, 2.0 Hz, 2H), 6.42 (d, *J* = 2.0 Hz, 1H), 4.19 (s, 3H). LC-MS [M+H] $^+$ = 350.1 |
| B58 | 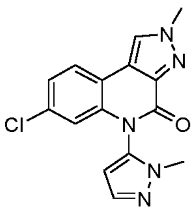 | $^1$H NMR (400 MHz, DMSO-d6) δ 8.82 (s, 1H), 8.09 (d, *J* = 8.3 Hz, 1H), 7.74 (d, *J* = 2.0 Hz, 1H), 7.43 (dd, *J* = 8.4, 2.0 Hz, 1H), 6.51 (dd, *J* = 8.8, 2.0 Hz, 2H), 4.20 (s, 3H), 3.56 (s, 3H). LC-MS [M+H] $^+$ = 314.1 |

(continued)

| No. | Structure | Nuclear magnetic resonance and mass spectrometry data |
|---|---|---|
| B59 | 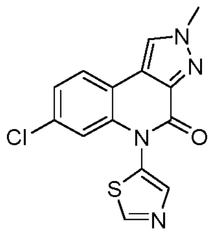 | |
| B60 | 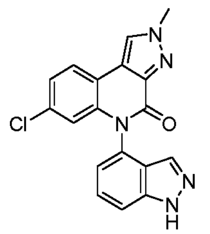 | 1H NMR (400 MHz, DMSO-d6) δ 13.49 (s, 1H), 8.84 (s, 1H), 8.10 (d, J = 8.3 Hz, 1H), 7.86 - 7.73 (m, 2H), 7.62 (dd, J = 8.4, 7.2 Hz, 1H), 7.35 (dd, J = 8.4, 2.0 Hz, 1H), 7.20 (d, J = 7.1 Hz, 1H), 6.32 (d, J =2.0 Hz, 1H), 4.21 (s, 3H). LC-MS [M+H] + = 350.1 |
| B61 | 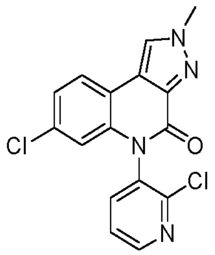 | |
| B62 | 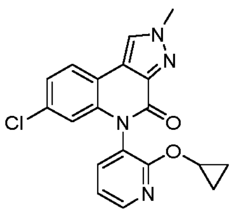 | |
| B63 | 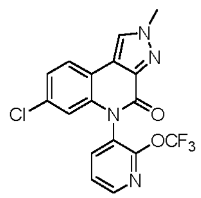 | |
| B64 | 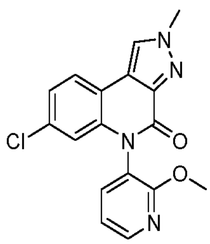 | |

(continued)

| No. | Structure | Nuclear magnetic resonance and mass spectrometry data |
|---|---|---|
| B65 | 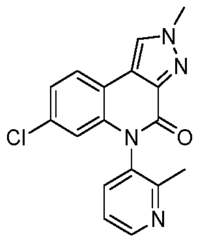 | $^1$H NMR (400 MHz, DMSO-$d_6$) δ 8.83 (s, 1H), 8.70 (dd, *J* = 4.8, 1.6 Hz, 1H), 8.10 (d, *J* = 8.4 Hz, 1H), 7.85 (dd, *J* = 7.9, 1.6 Hz, 1H), 7.56 (dd, *J* = 7.9, 4.8 Hz, 1H), 7.39 (dd, *J* = 8.3, 2.0 Hz, 1H), 6.33 (d, *J* = 1.9 Hz, 1H), 4.20 (s, 3H), 2.16 (s, 3H). LC-MS [M+H] $^+$ = 325.1 |
| B66 | 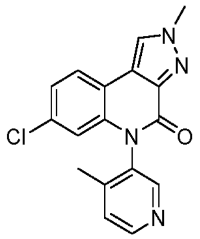 | |
| B67 | 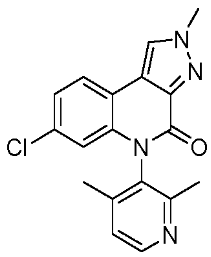 | |
| B68 | 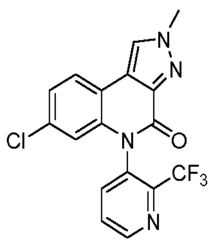 | $^1$H NMR (400 MHz, DMSO_$d_6$): δ 8.99 (d, *J*=4.8 Hz, 1 H), 8.80 (s, 1 H), 8.25 (d, *J*=7.6 Hz, 1 H), 8.08-8.04 (m, 2H), 7.38-7.35 (m, 1H), 6.38 (d, *J*=2.0 Hz, 1 H), 4.17 (s, 3H). LC-MS [M+H] $^+$ = 378.9 |
| B69 | 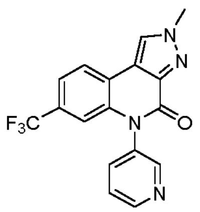 | 1H NMR (400 MHz, DMSO-d6) δ 8.95 (s, 1H), 8.84 (dd, J = 4.8, 1.5 Hz, 1H), 8.70 (d, J = 2.4 Hz, 1H), 8.29 (d, J = 8.1 Hz, 1H), 8.02 (dt, J = 8.1, 1.9 Hz, 1H), 7.76 (dd, J = 8.1, 4.8 Hz, 1H), 7.67 (dd, J = 8.2, 1.6 Hz, 1H), 6.64 (d, J = 1.6 Hz, 1H), 4.23 (s, 3H). LC-MS [M+H] $^+$ = 345.1 |
| B70 | 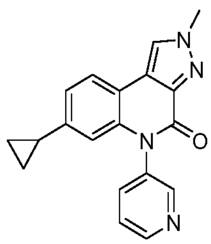 | 1H NMR (400 MHz, DMSO-d6) δ 8.76-8.77 (m, 1H), 8.69 (s, 1H), 8.57 (d, *J* = 2.4 Hz, 1H), 7.94 - 7.82 (m, 2H), 7.74 - 7.65 (m, 1H), 6.90-6.92 (m, 1H), 6.18 (d, *J* = 1.6 Hz, 1H), 4.15 (s, 3H), 1.96 - 1.65 (m, 1H), 0.96 - 0.75 (m, 2H), 0.58 - 0.33 (m, 2H). LC-MS [M+H] $^+$ = 317.1 |

(continued)

| No. | Structure | Nuclear magnetic resonance and mass spectrometry data |
|---|---|---|
| B71 | 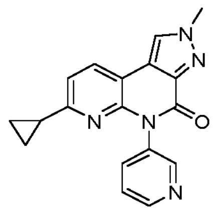 | 1H NMR (400 MHz, DMSO-d6) δ 8.76 (d, *J* = 4.8, 1H), 8.72 (s, 1H), 8.68 (s, 1H), 8.26 (t, *J* = 4.4 Hz, 1H), 8.06 (d, *J* = 8.0 Hz, 1H), 7.81-7.78 (m, 1H), 7.27 (d, *J* = 8.0 Hz, 1H), 4.16 (s, 3H), 2.02-1.96 (m, 1H), 0.80-0.75 (m, 2H), 0.45 - 0.41 (m, 2H). LC-MS $[M+H]^+$ =318.0 |
| B72 | 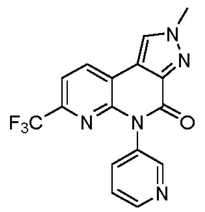 | 1H NMR (400 MHz, Methanol-d4) δ 8.76 (s, 2H), 8.61 (d, J = 8.0 Hz, 2H), 7.94 (ddd, J = 8.1, 2.4, 1.4 Hz, 1H), 7.73 (d, J = 7.9 Hz, 2H), 4.29 (s, 3H). LC-MS $[M+H]^+$ = 346.1 |
| B73 | 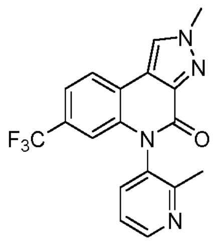 | |
| B74 | 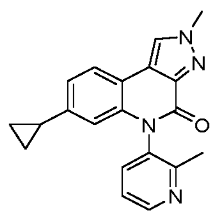 | 1HNMR (400 MHz, DMSO-d6) δ 8.70 (s, 1H), 8.64-8.66 (m, 1H), 7.89 (d, *J* = 8.0 Hz, 1H), 7.74-7.77 (m, 1H), 7.49-7.53 (m, 1H), 6.91-6.93 (m, 1H), 6.10 (d, *J* = 1.6 Hz, 1H), 4.15 (s, 3H), 2.12 (s, 3H), 1.92 - 1.51 (m, 1H), 0.86-0.88 (m, 2H), 0.48-0.51 (m, 2H). LC-MS $[M+H]^+$ =331.1 |
| B75 | 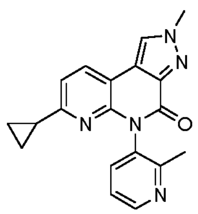 | 1H NMR (400 MHz, DMSO) δ 8.74 (s, 2H), 8.28 (d, *J* = 8.0 Hz, 1H), 8.08 (d, *J* = 8.0 Hz, 1H), 7.73 (t, *J* = 8.0 Hz, 1H), 7.28 (d, *J* = 8.0 Hz, 1H), 4.17 (s, 3H), 2.21 (s, 3H), 2.03-1.97 (m, 1H), 0.79 - 0.73 (m, 2H), 0.40 - 0.36 (m, 2H). LC-MS $[M+H]^+$ =332.1 |
| B76 | 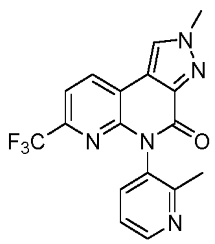 | $^1$H NMR (400 MHz, DMSO-$d_6$) δ 8.97 (s, 1H), 8.72 (d, *J* = 7.9 Hz, 1H), 8.59 (dd, *J* = 4.9, 1.6 Hz, 1H), 7.83 (d, *J* = 7.9 Hz, 1H), 7.76 (dd, *J* = 7.9, 1.6 Hz, 1H), 7.46 (dd, *J* = 7.9, 4.8 Hz, 1H), 4.24 (s, 3H), 2.13 (s, 3H). LC-MS $[M+H]^+$ = 360.1 |
| B77 | 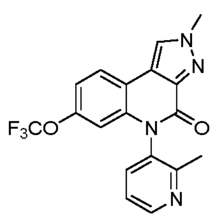 | $^1$H NMR (400 MHz, DMSO_$d_6$): δ 8.82 (s, 1H), 8.69-8.67 (m, 1H), 8.19 (d, J=8.8Hz, 1H), 7.85-7.82 (m, 1H), 7.55-7.52 (m, 1H), 7.31-7.29 (m, 1H), 6.26 (s, 1H), 4.18 (s, 3H), 2.13 (s, 3H). LC-MS $[M+H]^+$ =375.0 |

(continued)

| No. | Structure | Nuclear magnetic resonance and mass spectrometry data |
|---|---|---|
| B78 | 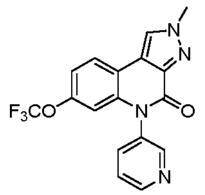 | $^1$H NMR (400 MHz, DMSO-d6): δ 8.80-8.78 (m, 2H), 8.63 (s, 1H), 8.15 (d,J=8.8Hz, 1H), 7.97-7.94 (m, 1H), 7.72-7.69 (m, 1H), 7.31-7.29 (m, 1H), 6.29 (d, *J*=1.2Hz, 1H), 4.17 (s, 3H). LC-MS $[M+H]^+$ = 361.0 |
| B79 | 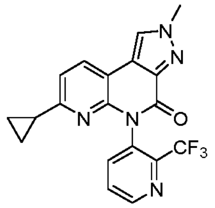 | |
| B80 | 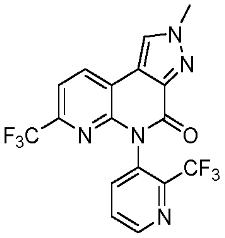 | $^1$H NMR (400 MHz, DMSO-$d_6$) δ 8.99 (s, 1H), 8.94 (dd, *J* = 4.8, 1.4 Hz, 1H), 8.75 (d, *J* = 7.7 Hz, 1H), 8.23 (dd, *J* = 8.2, 1.4 Hz, 1H), 8.02 (dd, *J* = 8.1, 4.7 Hz, 1H), 7.86 (d, *J* = 7.9 Hz, 1H), 4.24 (s, 3H). LC-MS $[M+H]^+$ = 414.1 |
| B81 | 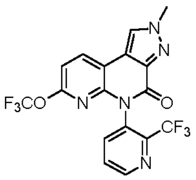 | |
| B82 | 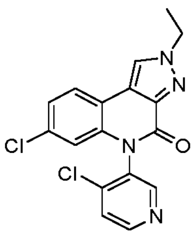 | |
| B83 | 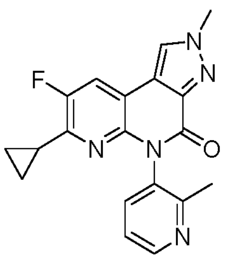 | |
| B84 | 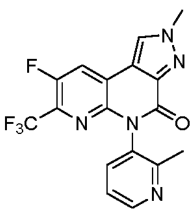 | |

(continued)

| No. | Structure | Nuclear magnetic resonance and mass spectrometry data |
|---|---|---|
| B85 | 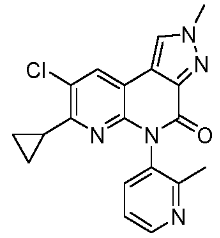 | |
| B86 | 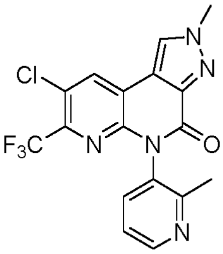 | |
| B87 | 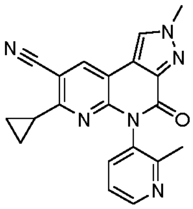 | |
| B88 | 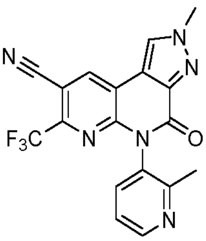 | |
| B89 | 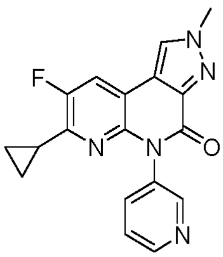 | |
| B90 | 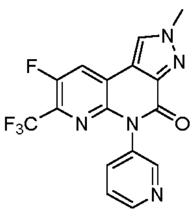 | |

(continued)

| No. | Structure | Nuclear magnetic resonance and mass spectrometry data |
|---|---|---|
| B91 | 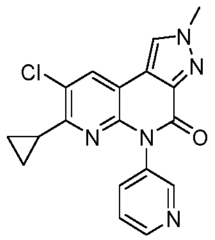 | |
| B92 | 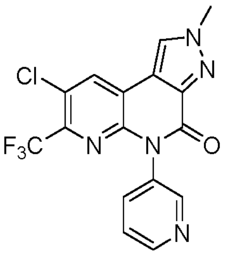 | |
| B93 | 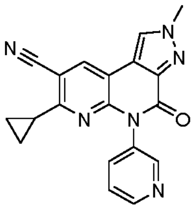 | |
| B94 | 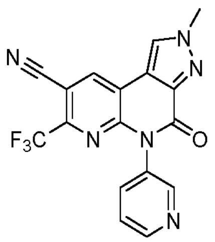 | |
| B95 | 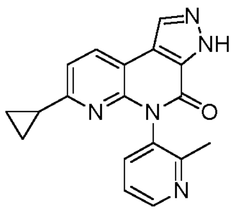 | 1H NMR (400 MHz, $CD_3OD$) δ 8.60-8.58 (m, 1H), 8.53 (s, 1H), 8.33 (d, *J* = 8.0 Hz, 1H), 7.72-7.70 (m, 1H), 7.54-7.50 (m, 1H), 7.30 (d, *J* = 8.0 Hz, 1H), 2.21 (s, 3H), 2.06-1.99 (m, 1H), 0.83-0.80 (m, 2H), 0.52-0.50 (m, 2H). LC-MS $[M+H]^+$ =318.0 |
| B96 | 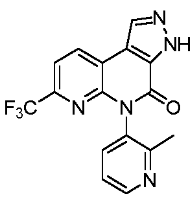 | $^1H$ NMR (400 MHz, DMSO-$d_6$) δ 14.79 (s, 1H), 8.85 (s, 2H), 8.61 (d, *J* = 4.8 Hz, 1H), 7.84 (dd, *J* = 38.5, 8.0 Hz, 2H), 7.47 (dd, *J* = 7.9, 4.8 Hz, 1H), 2.14 (s, 3H). LC-MS $[M+H]^+$ = 346.1 |
| B97 | 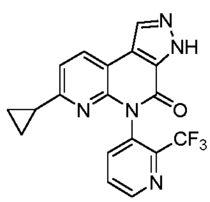 | |

(continued)

| No. | Structure | Nuclear magnetic resonance and mass spectrometry data |
|---|---|---|
| B98 | 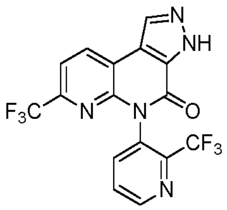 | |
| B99 | 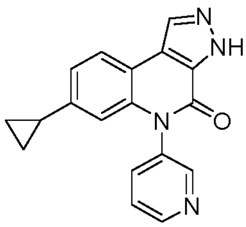 | |
| B100 | 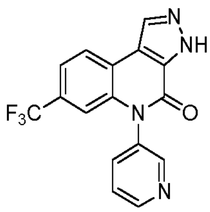 | |
| B101 | 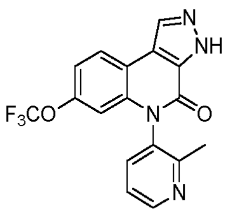 | 1H NMR (400 MHz, DMSO) δ 14.54 (s, 1H), 8.70-8.69 (m, 2H), 8.31 (d, *J* = 8.4 Hz, 1H), 7.86 (t, *J* = 6.8 Hz, 1H), 7.56-7.53 (m, 1H), 7.37 (d, *J* = 8.0 Hz, 1H), 6.28 (s, 1H), 2.15 (s, 3H). LC-MS [M+H] $^+$ =360.9 |
| B102 | 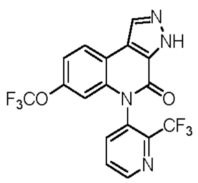 | |
| B103 | 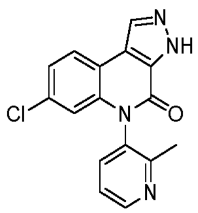 | $^1$H NMR (400 MHz, Chloroform-*d*) δ 8.82 (dd, *J* = 4.9, 1.6 Hz, 1H), 8.40 (s, 1H), 7.98 (d, *J* = 8.4 Hz, 1H), 7.64 (dd, *J* = 7.9, 1.6 Hz, 1H), 7.50 (dd, *J* = 7.9, 4.8 Hz, 1H), 7.37 (dd, *J* = 8.4, 1.9 Hz, 1H), 6.60 (d, *J* = 1.9 Hz, 1H), 2.36 (s, 3H). LC-MS [M+H] $^+$ = 311.1 |
| B104 | 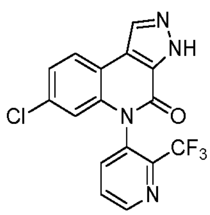 | |

(continued)

| No. | Structure | Nuclear magnetic resonance and mass spectrometry data |
|---|---|---|
| B105 | 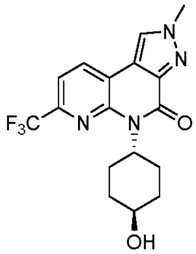 | |
| B106 | 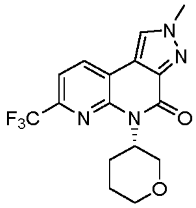 | |
| B107 | 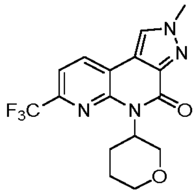 | |
| B108 | 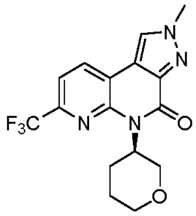 | |
| B109 | 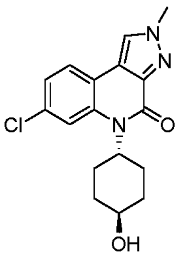 | 1H NMR (400 MHz, DMSO-d6) δ 8.65 (s, 1H), 7.96 (d, J = 8.3 Hz, 1H), 7.75 (d, J = 2.0 Hz, 1H), 7.33 (dd, J = 8.3, 1.7 Hz, 1H), 4.68 (s, 1H), 4.12 (s, 3H), 3.58 (d, J = 11.5 Hz, 1H), 2.69 (d, J = 12.5 Hz,2H), 2.04 - 1.89 (m, 2H), 1.76 - 1.62 (m, 2H), 1.58 - 1.44 (m, 2H). LC-MS $[M+H]^+$ = 332.1 |
| B110 | 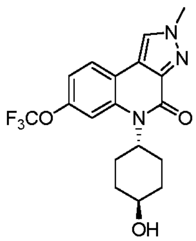 | |

(continued)

| No. | Structure | Nuclear magnetic resonance and mass spectrometry data |
|---|---|---|
| B111 | 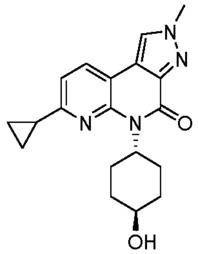 | |
| B112 | 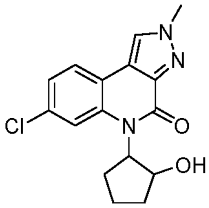 | |
| B113 | 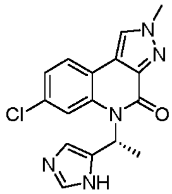 | |
| B114 | 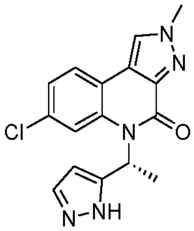 | |
| B115 | 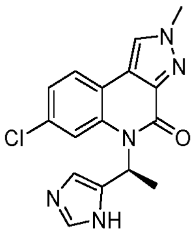 | |
| B116 | 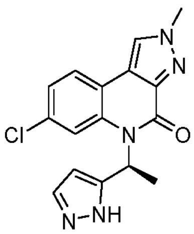 | |
| B117 | 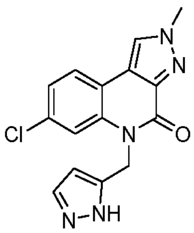 | $^1$H NMR (400 MHz, DMSO-d6) δ 12.76 (s, 1H), 8.72 (s, 1H), 7.98 (d, *J* = 8.4 Hz, 1H), 7.70 (d, *J* = 30.5 Hz, 2H), 7.32 (d, *J* = 8.4 Hz, 1H), 6.11 (s, 1H), 5.52 (s, 2H), 4.17 (s, 3H). LC-MS [M+H] $^+$ = 314.1 |

(continued)

| No. | Structure | Nuclear magnetic resonance and mass spectrometry data |
|---|---|---|
| B118 | 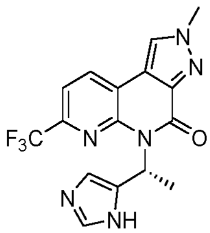 | |
| B119 | 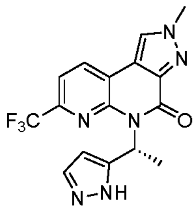 | |
| B120 | 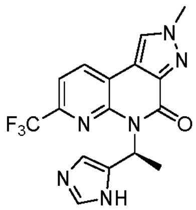 | |
| B121 | 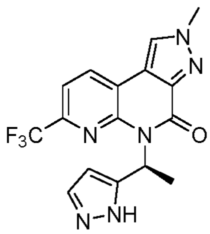 | |
| B122 | 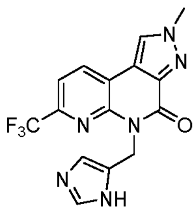 | $^1$H NMR (400 MHz, DMSO-$d_6$) δ 8.86 (s, 1H), 8.61 (d, *J* = 7.9 Hz, 1H), 8.18 (s, 1H), 7.80 (d, *J* = 7.9 Hz, 1H), 7.47 (s, 1H), 6.85 (s, 1H), 5.59 (s, 2H), 4.19 (s, 3H). LC-MS [M+H] $^+$ = 349.1 |
| B123 | 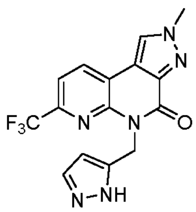 | $^1$H NMR (400 MHz, DMSO-$d_6$) δ 12.47 (s, 1H), 8.86 (s, 1H), 8.62 (d, *J* = 7.9 Hz, 1H), 7.79 (d, *J* = 7.9 Hz, 1H), 7.53 (s, 1H), 6.04 (d, *J* = 2.2 Hz, 1H), 5.64 (s, 2H), 4.17 (s, 3H). LC-MS [M+H]$^+$ =349.1 |
| B124 | 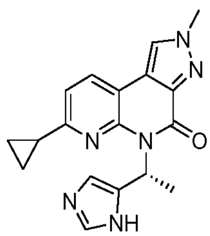 | |

(continued)

| No. | Structure | Nuclear magnetic resonance and mass spectrometry data |
|---|---|---|
| B125 | 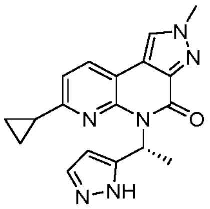 | |
| B126 | 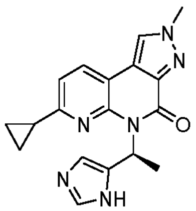 | |
| B127 | 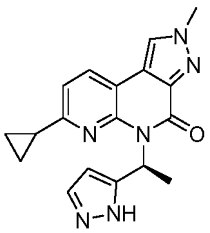 | |
| B128 | 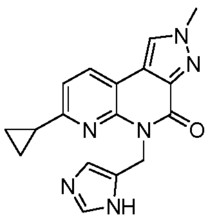 | |
| B129 | 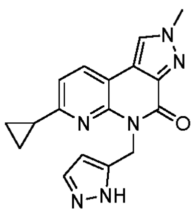 | |
| B130 | 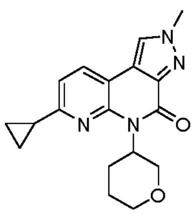 | |
| B131 | 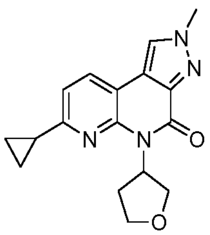 | |

(continued)

| No. | Structure | Nuclear magnetic resonance and mass spectrometry data |
|---|---|---|
| B132 | 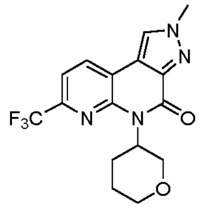 | |
| B133 | 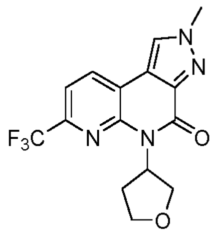 | |
| B134 | 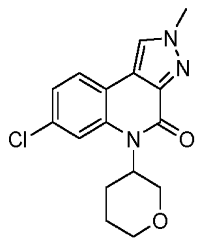 | |
| B135 | 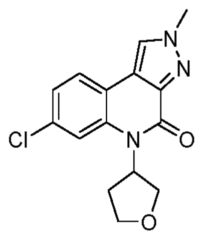 | |
| B136 | 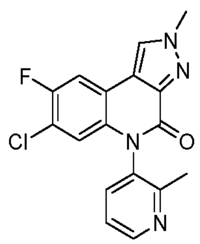 | |
| B137 | 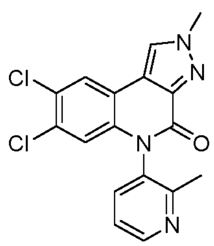 | |

(continued)

| No. | Structure | Nuclear magnetic resonance and mass spectrometry data |
|---|---|---|
| B138 | 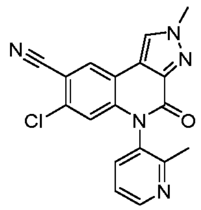 | |
| B139 | 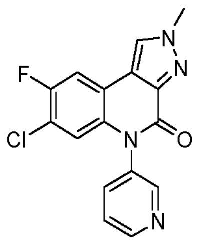 | 1H NMR (400 MHz, DMSO-d6) δ 8.87 - 8.77 (m, 2H), 8.66 (d, J = 2.5 Hz, 1H), 8.23 (d, J = 9.5 Hz, 1H), 7.97 (ddd, J = 8.1, 2.5, 1.5 Hz, 1H), 7.74 (dd, J = 8.1, 4.8 Hz, 1H), 6.52 (d, J = 6.5 Hz, 1H), 4.20 (s, 3H). LC-MS $[M+H]^+$ =329.0 |
| B140 | 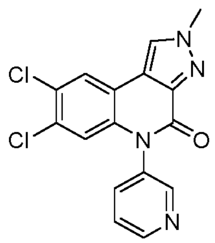 | |
| B141 | 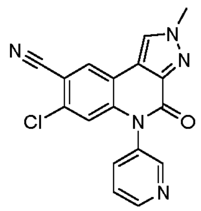 | |
| B142 | 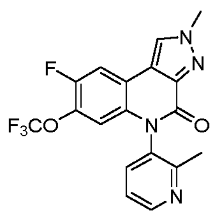 | |
| B143 | 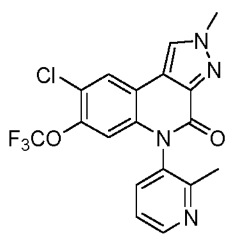 | |
| B144 | 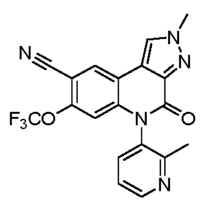 | |

(continued)

| No. | Structure | Nuclear magnetic resonance and mass spectrometry data |
|---|---|---|
| B145 | 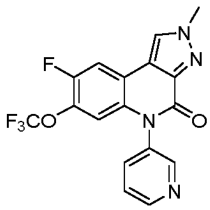 | |
| B146 | 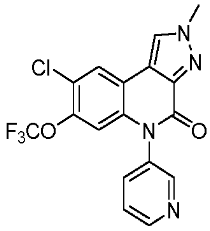 | |
| B147 | 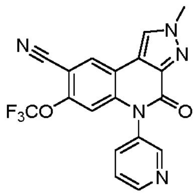 | |
| B148 | 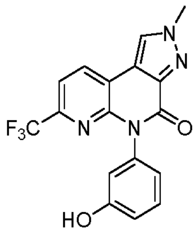 | 1H NMR (400 MHz, DMSO-d6) δ 9.63 (s, 1H), 8.93 (s, 1H), 8.69 - 8.55 (m, 1H), 7.78 (d, J = 7.9 Hz, 1H), 7.51 - 7.23 (m, 1H), 6.89 (ddd, J = 8.2, 2.4, 1.2 Hz, 1H), 6.79 - 6.61 (m, 2H), 4.22 (s, 3H). LC-MS $[M+H]^+$ = 361.1 |
| B149 | 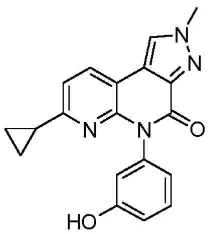 | |
| B150 | 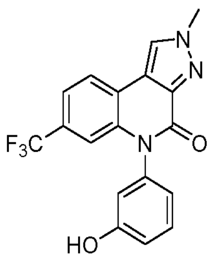 | $^1$H NMR (400 MHz, DMSO-$d_6$) δ 8.95 (s, 1H), 8.84 (dd, *J* = 4.8, 1.5 Hz, 1H), 8.70 (d, *J* = 2.4 Hz, 1H), 8.29 (d, *J* = 8.1 Hz, 1H), 8.02 (dt, *J* = 8.1, 2.0 Hz, 1H), 7.80 - 7.74 (m, 1H), 7.67 (dd, *J* = 8.2, 1.6 Hz, 1H), 6.64 (d, *J* = 1.6 Hz, 1H), 4.23 (s, 3H). LC-MS $[M+H]^+$ = 360.1 |
| B151 | 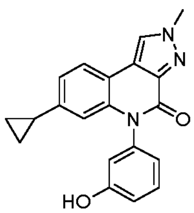 | |

(continued)

| No. | Structure | Nuclear magnetic resonance and mass spectrometry data |
|---|---|---|
| B152 | 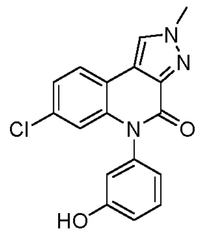 | $^1$H NMR (400 MHz, DMSO-$d_6$) δ 8.78 (s, 1H), 8.02 (d, *J* = 8.3 Hz, 1H), 7.37 - 7.29 (m, 2H), 6.88 (d, *J* = 8.3 Hz, 1H), 6.62 - 6.51 (m, 3H), 4.18 (s, 3H). LC-MS [M+H] + = 326.1 |
| B153 | 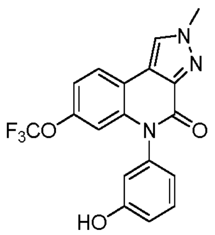 | |
| B154 | 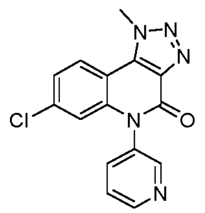 | |
| B155 | 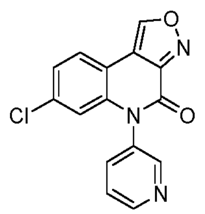 | |
| B156 | 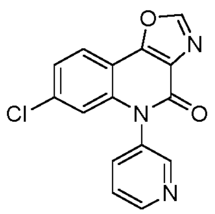 | |
| B157 | 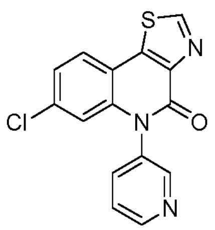 | |
| B158 | 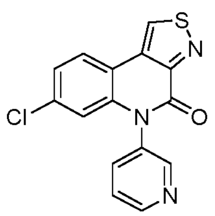 | |

(continued)

| No. | Structure | Nuclear magnetic resonance and mass spectrometry data |
|---|---|---|
| B159 | 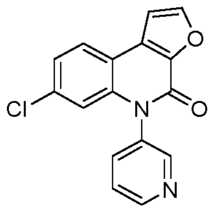 | |
| B160 | 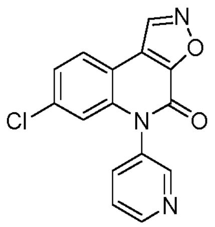 | |
| B161 | 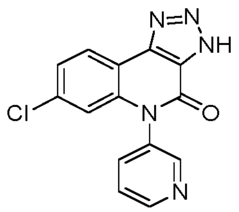 | |
| B162 | 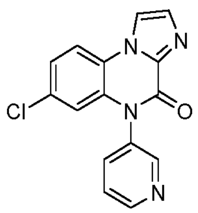 | |
| B163 | 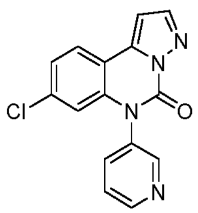 | |
| B164 | 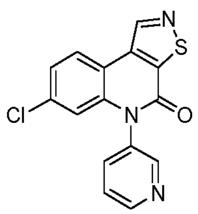 | |
| B165 | 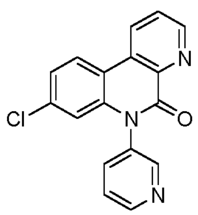 | |

(continued)

| No. | Structure | Nuclear magnetic resonance and mass spectrometry data |
| --- | --- | --- |
| B166 | 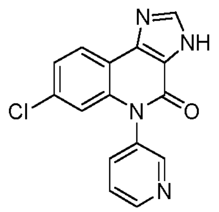 | |
| B167 | 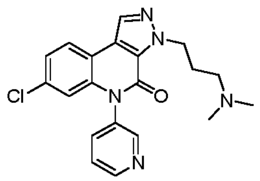 | $^1$H NMR (400 MHz, CDCl3): δ 8.84 (dd, $J_1$=4.8 Hz, $J_2$=1.6 Hz, 1H), 8.60 (d, $J$=2.4 Hz, 1H), 8.17 (s, 1H), 7.87 (d, J=8.0 Hz, 1H), 7.74-7.69 (m, 1 H) , 764-7.59 (m, 1 H), 7.30-7.26 (m, 1 H), 6.60 (d, $J$=2.0 Hz, 1H), 4.79 (t, $J$=7.2 Hz, 2H), 2.37-2.20 (m, 2H) ,2.20 (s, 6H) ,2.14-2.05 (m, 2H) . LC-MS $[M+H]^+$ =382.0 |
| B168 | 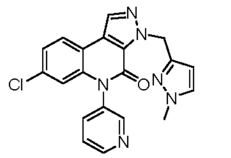 | $^1$H NMR (400 MHz, CDCl3): δ 8.84 (d, $J$=4.0 Hz, 1H), 8.61 (d, $J$=2.4 Hz, 1H), 8.21 (s, 1H), 7.86 <d,$J$=8.4 Hz, 1H), 7.74-7.70 (m, 1 H) , 7.64-7.59 (m, 1 H), 7.29-7.23 (m, 2 H), 6.60 (d, $J$=1.6 Hz, 1H), 6.22 (d, $J$=2.0 Hz, 1H), 6.02-5.93 (m, 2H) , 3.85 (s, 3 H) .LC-MS $[M+H]^+$ =391.0 |
| B169 | 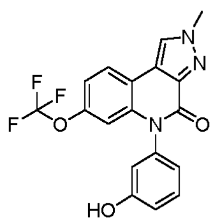 | $^1$H NMR (400 MHz, DMSO_$d_6$): δ 9.82 (s, 1H), 8.77 (s, 1H), 8.10 (d, $J$=8.4 Hz, 1H), 7.43 (t, $J$=8.0 Hz, 1H), 7.25 (d, $J$=8.4 Hz, 1 H), 6.97 (d, $J$=8.0 Hz, 1H), 6.76 (d, $J$=8.0 Hz, 1 H), 6.72-6.71 (m, 1 H), 6.36 (s, 1H), 4.16 (s, 3H). LC-MS $[M+H]^+$ = 376.0 |
| B170 | 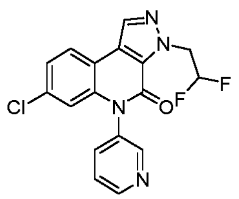 | $^1$H NMR (400 MHz, CDCl3): δ 8.86 (dd, $J_1$=4.8 Hz, $J_2$=1.6 Hz,1H), 8.61 (d, $J$=2.0 Hz, 1H), 8.26 (s, 1H), 7.89 (d,$J$=8.4 Hz, 1H), 7.74-7.69 (m, 1 H) , 7.62 (dd, $J_1$=8.0 Hz, $J_2$=4.8 Hz,1H), 7.31 (dd, $J_1$=8.4 Hz, $J_2$=2.0 Hz,1H), 6.65 (d, $J$=2.0 Hz, 1H), 6.41-6.09 (m, 1H), 5.20-5.07(m, 2H). LC-MS $[M+H]^+$ = 360.9 |
| B171 | 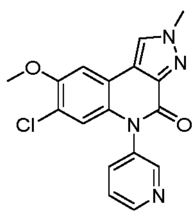 | $^1$H NMR (400 MHz, DMSO-$d_6$) δ 8.86 (s, 1H), 8.80 (dd, $J$ = 4.8, 1.5 Hz, 1H), 8.63 (d, $J$ = 2.4 Hz, 1H), 7.95 (dt, $J$ = 8.1, 1.8 Hz, 1H), 7.84 (s, 1H), 7.73 (dd, $J$ = 8.0, 4.8 Hz, 1H), 6.43 (s, 1H), 4.20 (s, 3H), 3.98 (s, 3H). LC-MS $[M+H]^+$ = 341.1 |
| B172 | 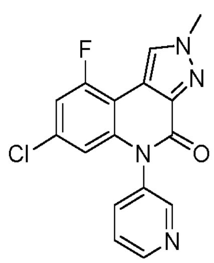 | $^1$H NMR (400 MHz, DMSO-$d_6$) δ 8.82 (d, $J$ = 4.7 Hz, 1H), 8.69 (dd, $J$ = 18.1, 2.9 Hz, 2H), 7.99 (d, $J$ = 7.6 Hz, 1H), 7.75 (dd, $J$ = 8.2, 4.7 Hz, 1H), 7.56 - 7.47 (m, 1H), 6.28 (d, $J$ = 1.7 Hz, 1H), 4.22 (d, $J$ = 3.4 Hz, 3H). LC-MS $[M+H]^+$ = 329.0 |

(continued)

| No. | Structure | Nuclear magnetic resonance and mass spectrometry data |
|---|---|---|
| B173 | 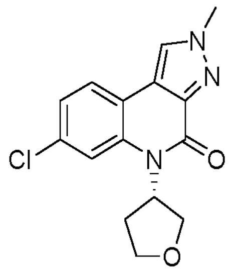 | $^1$H NMR (400 MHz, DMSO-$d_6$) δ 8.69 (s, 1H), 8.00 (d, *J* = 8.3 Hz, 1H), 7.95 (d, *J* = 1.9 Hz, 1H), 7.37 (dd, *J* = 8.3, 1.8 Hz, 1H), 6.23 (dt, *J* = 13.5, 6.8 Hz, 1H), 4.37 (dt, *J* = 8.3, 5.3 Hz, 1H), 4.15 (s, 3H), 4.05 (dd, *J* = 9.6, 4.8 Hz, 1H), 3.96 (t, *J* = 9.5 Hz, 1H), 3.78 (q, *J* = 8.4 Hz, 1H), 2.26 (m, 2H).LCMS $[M+H]^+$: 304.1. |
| B174 | 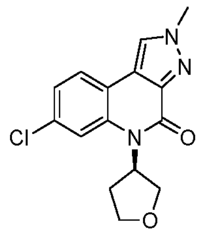 | $^1$H NMR (400 MHz, Methanol-*d*4) δ 8.47 (s, 1H), 8.07 (d, *J* = 1.8 Hz, 1H), 7.95 (d, *J* = 8.3 Hz, 1H), 7.33 (dd, *J* = 8.4, 1.9 Hz, 1H), 6.47 - 6.35 (m, 1H), 4.52 (td, *J* = 8.5, 3.2 Hz, 1H), 4.21 (s, 4H), 4.07 (t, J= 9.7 Hz, 1H), 3.90 (td, *J* = 9.3, 6.9 Hz, 1H), 2.50 - 2.31 (m, 2H). LC-MS $[M+H]^+$ = 304.1 |
| B175 | 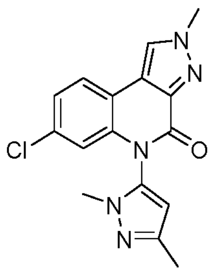 | $^1$H NMR (400 MHz, DMSO-*d*6) δ 8.82 (s, 1H), 8.08 (d, *J* = 8.3 Hz, 1H), 7.42 (dd, *J* = 8.4, 2.0 Hz, 1H), 6.56 (d, *J* = 2.0 Hz, 1H), 6.29 (s, 1H), 4.19 (s, 3H), 3.46 (s, 3H), 2.30 (s, 3H). LC-MS $[M+H]^+$ = 328.1 |
| B176 | 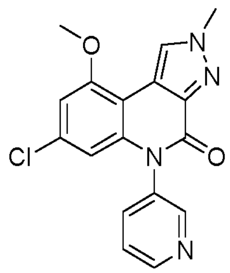 | $^1$H NMR (400 MHz, DMSO-$d_6$) δ 8.81 (d, *J* = 4.8 Hz, 1H), 8.67 (s, 1H), 8.63 (d, *J* = 2.5 Hz, 1H), 7.96 (d, *J* = 8.3 Hz, 1H), 7.73 (dd, *J* = 8.1, 4.7 Hz, 1H), 7.10 (d, J= 1.8 Hz, 1H), 6.02 (d, *J*= 1.7 Hz, 1H), 4.20 (s, 3H), 4.10 (s, 3H). LC-MS $[M+H]^+$ = 341.1. |
| B177 | 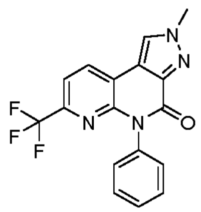 | 1H NMR (400 MHz, DMSO-d6) δ 8.94 (s, 1H), 8.68 (d, J = 7.9 Hz, 1H), 7.79 (d, J = 7.9 Hz, 1H), 7.62 - 7.46 (m, 3H), 7.38 - 7.25 (m, 2H), 4.23 (s, 3H). LC-MS $[M+H]^+$ = 345.1. |
| B178 | 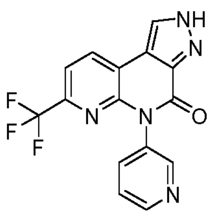 | 1H NMR (400 MHz, DMSO-d6) δ 8.70 (d, J = 4.8 Hz, 3H), 8.61 (d, J = 2.4 Hz, 1H), 7.86 (dd, J = 23.9, 8.0 Hz, 2H), 7.64 (dd, J = 8.1, 4.8 Hz, 1H). LC-MS $[M+H]^+$ = 332.1. |

(continued)

| No. | Structure | Nuclear magnetic resonance and mass spectrometry data |
|---|---|---|
| B179 | 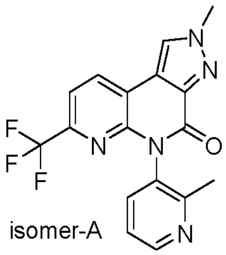 isomer-A | $^1$H NMR (400 MHz, DMSO-$d_6$) δ 8.97 (s, 1H), 8.72 (d, *J* = 7.9 Hz, 1H), 8.59 (dd, *J* = 4.9, 1.6 Hz, 1H), 7.83 (d, *J* = 7.9 Hz, 1H), 7.76 (dd, *J* = 7.9, 1.6 Hz, 1H), 7.46 (dd, *J* = 7.9, 4.8 Hz, 1H), 4.24 (s, 3H). LCMS m/z[M+H]$^+$: 360.1. |
| B180 | 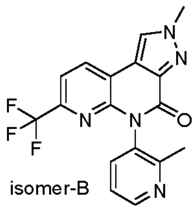 isomer-B | $^1$H NMR (400 MHz, DMSO-$d_6$) δ 8.97 (s, 1H), 8.72 (d, *J* = 7.9 Hz, 1H), 8.59 (dd, *J* = 4.9, 1.6 Hz, 1H), 7.83 (d, *J* = 7.9 Hz, 1H), 7.76 (dd, *J* = 7.9, 1.6 Hz, 1H), 7.46 (dd, *J* = 7.9, 4.8 Hz, 1H), 4.24 (s, 3H). LCMS m/z[M+H]$^+$: 360.1. |
| B181 | 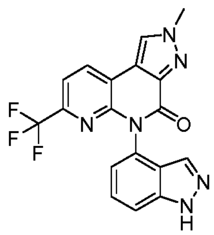 | 1H NMR (400 MHz, DMSO-d6) δ 13.23 (s, 1H), 8.98 (s, 1H), 8.71 (d, J = 7.9 Hz, 1H), 7.79 (d, J = 7.9 Hz, 1H), 7.73 (d, J = 1.5 Hz, 1H), 7.68 (d, J = 8.4 Hz, 1H), 7.52 (d, J = 1.2 Hz, 1H), 7.11 (d, J = 7.2Hz, 1H), 4.24 (s, 3H). LC-MS [M+H] $^+$ = 385.1. |
| B182 | 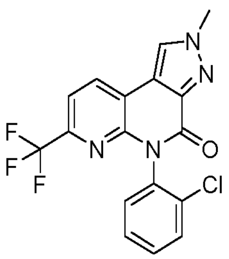 | $^1$H NMR (400 MHz, DMSO-$d_6$) δ 8.97 (s, 1H), 8.72 (dd, *J* = 7.9, 0.8 Hz, 1H), 7.83 (d, *J* = 7.9 Hz, 1H), 7.74 - 7.69 (m, 1H), 7.56 (q, *J* = 2.2 Hz, 3H), 4.23 (s, 3H). LC-MS [M+H] $^+$ = 379.1. |
| B183 | 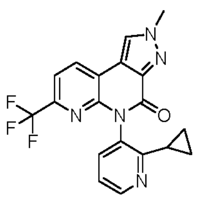 | $^1$H NMR (400 MHz, DMSO-$d_6$) δ 8.97 (s, 1H), 8.71 (dd, *J* = 8.0, 0.8 Hz, 1H), 8.54 (dd, *J* = 4.7, 1.6 Hz, 1H), 7.83 (d, *J* = 7.9 Hz, 1H), 7.72 (dd, *J* = 7.9, 1.6 Hz, 1H), 7.35 (dd, *J* = 7.9, 4.7 Hz, 1H), 4.23 (s, 3H), 1.57 (m, 1H), 1.08 - 0.98 (m, 1H), 0.82 - 0.65 (m, 2H), 0.63 - 0.53 (m, 1H). LC-MS [M+H] $^+$ = 386.1. |
| B184 | 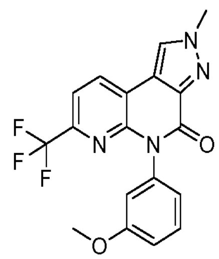 | $^1$H NMR (400 MHz, DMSO-$d_6$) δ 8.93 (s, 1H), 8.67 (d, *J* = 7.9 Hz, 1H), 7.78 (d, *J* = 7.9 Hz, 1H), 7.46 (t, *J* = 8.0 Hz, 1H), 7.07 (dd, *J* = 8.3, 2.5 Hz, 1H), 6.99 - 6.86 (m, 2H), 4.22 (s, 3H), 3.79 (s, 3H). LC-MS [M+H] $^+$ = 375.1 |

(continued)

| No. | Structure | Nuclear magnetic resonance and mass spectrometry data |
|---|---|---|
| B185 | 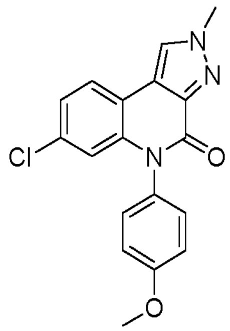 | 1H NMR (400 MHz, DMSO-$d_6$) δ 8.77 (d, *J* = 3.0 Hz, 1H), 8.04 (dd, *J* = 8.4, 3.0 Hz, 1H), 7.32 (ddd, *J* = 10.7, 6.2, 2.5 Hz, 3H), 7.21 (dd, *J* = 9.0, 2.9 Hz, 2H), 6.51 - 6.47 (m, 1H), 4.18 (d, *J* = 2.9 Hz, 3H), 3.90 (d, *J* = 3.1 Hz, 3H). LC-MS $[M+H]^+$ = 340.1 |
| B186 | 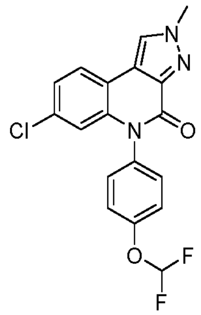 | 1H NMR (400 MHz, DMSO-d6) δ 8.79 (s, 1H), 8.06 (d, *J* = 8.3 Hz, 1H), 7.52 - 7.44 (m, 5H), 7.35 (dd, *J* = 8.4, 2.0 Hz, 1H), 6.44 (d, *J* = 2.0 Hz, 1H), 4.19 (s, 3H). LC-MS $[M+H]^+$ = 376.1 |
| B187 | 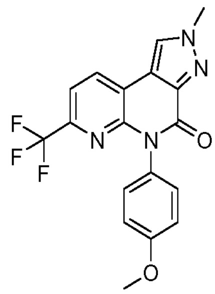 | 1H NMR (400 MHz, DMSO-d6) δ 8.92 (s, 1H), 8.65 (d, J = 7.9 Hz, 1H), 7.77 (d, J = 7.9 Hz, 1H), 7.24 (d, J = 8.8 Hz, 2H), 7.09 (d, J = 8.8 Hz, 2H), 4.21 (s, 3H), 3.87 (s, 3H). LC-MS $[M+H]^+$ = 375.1. |
| B188 | 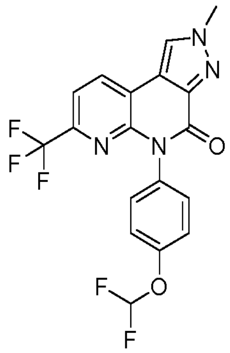 | 1H NMR (400 MHz, DMSO-d6) δ 8.91 (s, 1H), 8.65 (d, J = 7.9 Hz, 1H), 7.77 (d, J = 7.9 Hz, 1H), 7.48 - 7.28 (m, 5H), 4.21 (s, 3H). LC-MS $[M+H]^+$ = 411.1. |
| B189 | 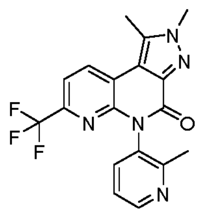 | 1H NMR (400 MHz, DMSO-d6) δ 8.71 (d, J = 8.0 Hz, 1H), 8.59 (dd, J = 4.8, 1.6 Hz, 1H), 7.78 (d, J = 8.0 Hz, 1H), 7.76 - 7.71 (m, 1H), 7.46 (dd, J = 7.9, 4.8 Hz, 1H), 4.14 (s, 3H), 2.88 (s, 3H), 2.11 (s,3H). LC-MS $[M+H]^+$ = 374.1 |

(continued)

| No. | Structure | Nuclear magnetic resonance and mass spectrometry data |
|---|---|---|
| B190 | 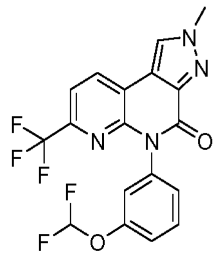 | $^1$H NMR (400 MHz, DMSO-$d_6$) δ 8.94 (s, 1H), 8.68 (d, $J$ = 7.9 Hz, 1H), 7.80 (d, $J$ = 7.9 Hz, 1H), 7.62 (t, $J$ = 8.4 Hz, 1H), 7.36 - 7.30 (m, 2H), 7.29 - 7.24 (m, 2H), 4.23 (s, 3H). LC-MS $[M+H]^+$ = 411.1 |
| B191 | 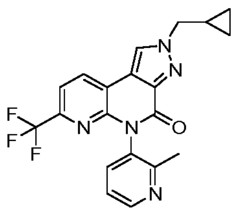 | $^1$H NMR (400 MHz, Methanol-d4) δ 8.90 (s, 1H), 8.67 (d, J = 7.9 Hz, 1H), 8.62 (dd, J = 5.0, 1.6 Hz, 1H), 7.80 (dd, J = 8.0, 1.6 Hz, 1H), 7.74 (d, J = 7.9 Hz, 1H), 7.54 (dd, J = 7.9, 4.9 Hz, 1H), 4.40 (d, J = 7.3 Hz,2H), 2.26 (s, 3H), 1.53 (tt, J = 7.7, 4.8 Hz, 1H), 0.82 - 0.71 (m, 2H), 0.60 (dt, J = 6.5, 4.7 Hz, 2H). LC-MS $[M+H]^+$ = 400.2. |
| B192 | 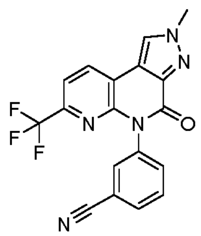 | 1H NMR (400 MHz, DMSO-d6) δ 8.96 (s, 1H), 8.70 (d, J = 7.9 Hz, 1H), 7.98 (d, J = 1.9 Hz, 2H), 7.79 (s, 3H), 4.23 (s, 3H). LC-MS $[M+H]^+$ = 370.1 |
| B193 | 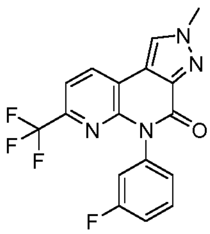 | 1H NMR (400 MHz, DMSO-d6) δ 8.94 (s, 1H), 8.68 (d, J = 7.9 Hz, 1H), 7.80 (d, J = 7.9 Hz, 1H), 7.61 (td, J = 8.1, 6.5 Hz, 1H), 7.46 - 7.28 (m, 2H), 7.22 (dd, J = 7.7, 1.7 Hz, 1H), 4.23 (s, 3H). LC-MS $[M+H]^+$ = 363.1 |
| B194 | 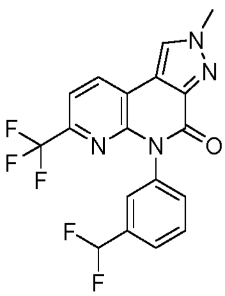 | $^1$H NMR (400 MHz, DMSO-$d_6$) δ 8.94 (s, 1H), 8.68 (d, $J$ = 7.9 Hz, 1H), 7.86 - 7.69 (m, 3H), 7.58 (d, $J$ = 19.4 Hz, 2H), 7.21 (d, $J$ = 55.6 Hz, 1H), 4.23 (s, 3H).<br><br>LC-MS $[M+H]^+$ = 395.2. |
| B195 | 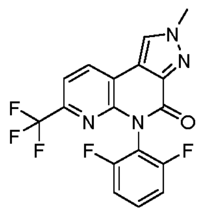 | $^1$H NMR (400 MHz, DMSO-d6) δ 8.77 (s, 1H), 8.64 (d, J = 8.0 Hz, 1H), 7.77 (d, J = 8.0 Hz, 1H), 7.66 (tt, J = 8.5, 6.3 Hz, 1H), 7.28 (t, J = 8.3 Hz, 2H), 4.32 (s, 3H). LC-MS $[M+H]^+$ = 381.2 |

(continued)

| No. | Structure | Nuclear magnetic resonance and mass spectrometry data |
|---|---|---|
| B196 | 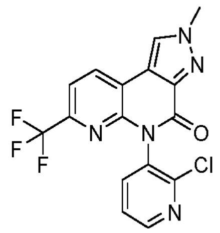 | $^1$H NMR (400 MHz, DMSO-$d_6$) δ 8.99 (s, 1H), 8.75 (d, *J* = 7.9 Hz, 1H), 8.60 (dd, *J* = 4.8, 1.8 Hz, 1H), 8.14 (dd, *J* = 7.7, 1.8 Hz, 1H), 7.87 (d, *J* = 7.9 Hz, 1H), 7.72 (dd, J= 7.8, 4.8 Hz, 1H), 4.25 (s, 3H). LC-MS $[M+H]^+$ = 380.1. |
| B197 | 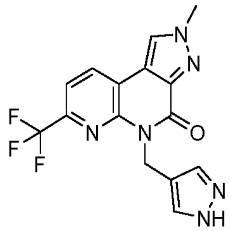 | 1H NMR (400 MHz, DMSO-d6) δ 12.65 (s, 1H), 8.87 (s, 1H), 8.64 (d, J = 7.9 Hz, 1H), 7.85 (d, J = 7.9 Hz, 1H), 7.60 (s, 2H), 5.50 (s, 2H), 4.19 (s, 3H). LC-MS $[M+H]^+$ = 349.1. |
| B198 | 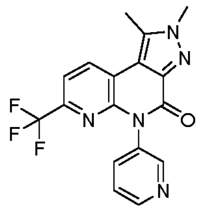 | 1H NMR (400 MHz, DMSO-d6) δ 8.79 - 8.67 (m, 2H), 8.57 (d, J = 2.4 Hz, 1H), 7.87 (dt, J = 8.1, 2.0 Hz, 1H), 7.77 (s, 1H), 7.63 (dd, J = 8.1, 4.8 Hz, 1H), 4.14 (s, 3H), 2.87 (s, 3H). LC-MS $[M+H]^+$ = 360.1. |
| B199 | 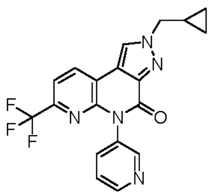 | $^1$H NMR (400 MHz, DMSO-$d_6$) δ 9.07 (s, 1H), 8.73 (d, *J* = 7.9 Hz, 1H), 8.69 (dd, *J* = 4.9, 1.5 Hz, 1H), 8.60 (d, *J* = 2.4 Hz, 1H), 7.89 (dt, *J* = 8.2, 1.8 Hz, 1H), 7.83 (d, *J* = 7.9 Hz, 1H), 7.64 (dd, *J* = 8.1, 4.8 Hz, 1H), 4.37 (d, *J* = 7.3 Hz, 2H), 1.44 (ddt, *J* = 10.6, 7.6, 3.8 Hz, 1H), 0.66 (dt, *J* = 7.8, 3.0 Hz, 2H), 0.59 - 0.49 (m, 2H).<br>LC-MS $[M+H]^+$= 386.2. |
| B200 | 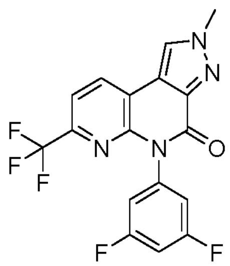 | $^1$H NMRδ 8.95 (s, 1H), 8.69 (d, *J* = 7.9 Hz, 1H), 7.82 (d, *J* = 8.0 Hz, 1H), 7.48 - 7.39 (m, 1H), 7.27 (dd, *J* = 7.7, 2.4 Hz, 2H), 4.23 (s, 3H). LC-MS $[M+H]^+$= 381.1. |
| B201 | 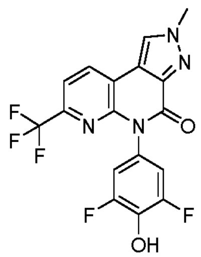 | $^1$H NMR (400 MHz, DMSO-$d_6$) δ 8.88 (d, *J* = 4.9 Hz, 1H), 8.63 (d, *J* = 7.9 Hz, 1H), 7.77 (d, *J*= 7.8 Hz, 1H), 7.13 (d, *J* = 7.9 Hz, 2H), 4.19 (s, 3H). LC-MS $[M+H]^+$= 397.1. |

(continued)

| No. | Structure | Nuclear magnetic resonance and mass spectrometry data |
|---|---|---|
| B202 | 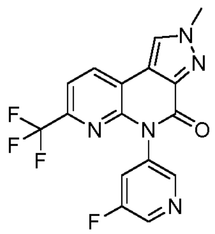 | 1H NMR (400 MHz, DMSO-d6) δ 8.96 (d, J = 1.8 Hz, 1H), 8.85 - 8.66 (m, 2H), 8.53 (d, J = 1.7 Hz, 1H), 8.09 - 7.99 (m, 1H), 7.85 (dd, J = 8.1, 1.8 Hz, 1H), 4.24 (d, J = 1.8 Hz, 3H). LC-MS $[M+H]^+$= 364.1. |
| B203 | 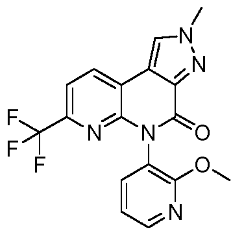 | $^{1}$H NMR (400 MHz, DMSO-*d6*) δ 8.95 (s, 1H), 8.69 (d, *J* = 7.9 Hz, 1H), 8.33 (dd, *J* = 5.0, 1.8 Hz, 1H), 7.89 - 7.75 (m, 2H), 7.22 (dd, *J* = 7.5, 5.0 Hz, 1H), 4.23 (s, 3H), 3.75 (s, 3H). LC-MS $[M+H]^+$= 376.1 |
| B204 | 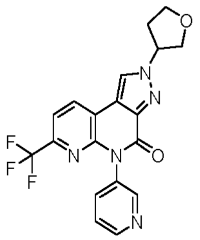 | $^{1}$H NMR (400 MHz, DMSO-*d6*) *δ* 9.10 (s, 1H), 8.74 (d, J = 7.9 Hz, 1H), 8.69 (dd, J = 4.8, 1.5 Hz, 1H), 8.58 (d, J = 2.4 Hz, 1H), 7.95 - 7.80 (m, 2H), 7.70 - 7.58 (m, 1H), 5.51 - 5.37 (m, 1H), 4.23 - 4.06(m, 3H), 3.93 (td, J = 8.4, 5.4 Hz, 1H), 2.68 - 2.56 (m, 1H), 2.49 - 2.39 (m, 1H). LC-MS $[M+H]^+$= 402.2. |
| B205 | 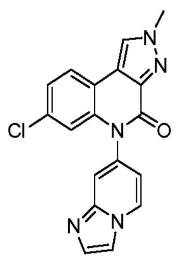 | $^{1}$H NMR (400 MHz, DMSO-*d6*) *δ* 8.80 (d, J = 1.8 Hz, 2H), 8.15 (t, J = 1.0 Hz, 1H), 8.07 (d, J = 8.3 Hz, 1H), 7.81 - 7.72 (m, 2H), 7.37 (dd, J = 8.4, 2.0 Hz, 1H), 6.94 (dd, J = 7.1, 2.0 Hz, 1H), 6.73 (d, J =2.0 Hz, 1H), 4.19 (s, 3H). LC-MS $[M+H]^+$= 350.1 |
| B206 | 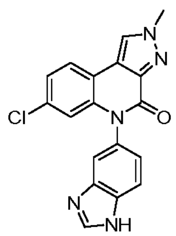 | 1H NMR (400 MHz, DMSO-$d_6$) δ 12.81 (s, 1H), 8.80 (d, *J* = 3.6 Hz, 1H), 8.42 (d, *J* = 3.6 Hz, 1H), 8.06 (dd, *J* = 8.4, 3.4 Hz, 1H), 7.82 (s, 1H), 7.66 (s, 1H), 7.32 (dd, J= 7.2, 4.7 Hz, 1H), 7.15 (s, 1H), 6.42 (t, *J* = 2.9 Hz, 1H), 4.20 (d, *J*= 3.7 Hz, 3H). LC-MS $[M+H]^+$= 350.1 |
| B207 | 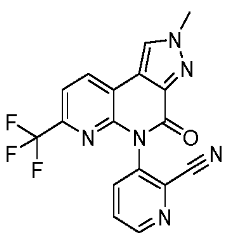 | 1HNMR (400 MHz, DMSO-$d_6$) δ 9.02 (s, 1H), 8.94 (dd, *J* = 4.8, 1.5 Hz, 1H), 8.80 (d, *J* = 7.9 Hz, 1H), 8.31 (dd, *J* = 8.3, 1.5 Hz, 1H), 8.06 (dd, *J* = 8.3, 4.8 Hz, 1H), 7.92 (d, *J* = 7.9 Hz, 1H), 4.26 (s, 3H). LC-MS $[M+H]^+$= 371.1 |

(continued)

| No. | Structure | Nuclear magnetic resonance and mass spectrometry data |
|---|---|---|
| B208 | 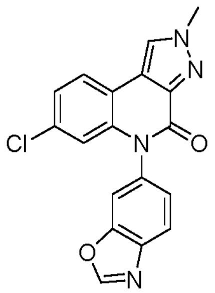 | $^1$H NMR (400 MHz, DMSO-$d_6$) δ 8.95 (s, 1H), 8.81 (s, 1H), 8.08 (dd, *J* = 8.3, 3.2 Hz, 2H), 8.00 (d, *J* = 1.8 Hz, 1H), 7.44 (dd, *J* = 8.4, 1.9 Hz, 1H), 7.35 (dd, *J* = 8.4, 2.0 Hz, 1H), 6.44 (d, *J* = 2.0 Hz, 1H), 4.20 (s, 3H). LC-MS $[M+H]^+$= 351.0 |
| B209 | 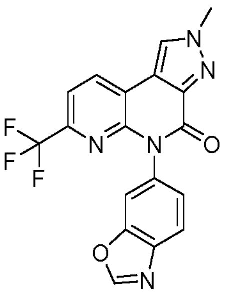 | $^1$H NMR (400 MHz, DMSO-d6) δ 8.97 (s, 1H), 8.89 (s, 1H), 8.71 (d, *J* = 7.9 Hz, 1H), 7.98 (d, *J* = 8.4 Hz, 1H), 7.91 (d, *J* = 1.8 Hz, 1H), 7.81 (d, *J* = 7.9 Hz, 1H), 7.41 (dd, *J* = 8.4, 1.9 Hz, 1H), 4.25 (s, 3H). LC-MS $[M+H]^+$= 386.1 |
| B210 | 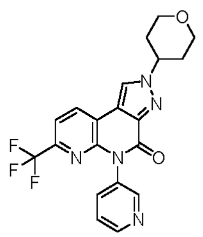 | $^1$H NMR (400 MHz, DMSO-d6) *δ*9.14 (s, 1H), 8.73 - 8.66 (m, 2H), 8.58 (dd, *J* = 2.4, 0.8 Hz, 1H), 7.88 (ddd, *J* = 8.1, 2.5, 1.6 Hz, 1H), 7.84 (d, *J* = 7.9 Hz, 1H), 7.64 (ddd, *J* = 8.0, 4.8, 0.8 Hz, 1H), 4.92 - 4.74 (m, 1H), 4.13 - 3.98 (m, 2H), 3.59 (td, *J* = 11.6, 2.4 Hz, 2H), 2.22 - 2.05 (m, 4H). LC-MS $[M+H]^+$= 416.2 |
| B211 | 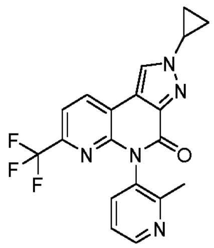 | $^1$H NMR (400 MHz, DMSO-$d_6$) *δ* 9.13 (d, *J* = 1.6 Hz, 1H), 8.73 - 8.67 (m, 1H), 8.62 - 8.57 (m, 1H), 7.84 (dd, *J* = 7.9, 1.7 Hz, 1H), 7.74 (dd, *J* = 7.9, 1.7 Hz, 1H), 7.45 (ddd, *J* = 7.6, 4.8, 1.5 Hz, 1H), 4.27 - 4.15 (m, 1H), 2.13 (d, *J* = 1.6 Hz, 3H), 1.38 - 1.31 (m, 2H), 1.26 - 1.18 (m, 2H). LC-MS $[M+H]^+$= 386.2. |
| B212 | 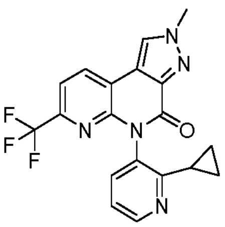 Isomer A | 1H NMR (400 MHz, DMSO-d6) δ 8.96 (s, 1H), 8.74 - 8.68 (m, 1H), 8.54 (dd, J = 4.7, 1.6 Hz, 1H), 7.82 (d, J = 7.9 Hz, 1H), 7.71 (dd, J = 7.9, 1.6 Hz, 1H), 7.35 (dd, J = 7.9, 4.7 Hz, 1H), 4.23 (s, 3H), 1.57 (tt, J = 8.4, 4.7 Hz, 1H), 1.10 - 0.97 (m, 1H), 0.84 - 0.64 (m, 2H), 0.65 - 0.48 (m, 1H). LCMS $[M+H]^+$= 386.2 |
| B213 | 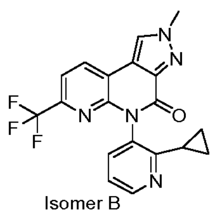 Isomer B | $^1$H NMR (400 MHz, DMSO-$d_6$) δ 8.97 (s, 1H), 8.71 (d, *J* = 7.9 Hz, 1H), 8.54 (dd, *J* = 4.8, 1.6 Hz, 1H), 7.82 (d, J= 7.9 Hz, 1H), 7.71 (dd, *J* = 7.9, 1.7 Hz, 1H), 7.35 (dd, *J* = 7.9, 4.8 Hz, 1H), 4.24 (s, 3H), 1.56 (td, *J* = 8.1, 4.1 Hz, 1H), 1.10 - 0.95 (m, 1H), 0.84 - 0.65 (m, 2H), 0.66 - 0.51 (m, 1H). LCMS $[M+H]^+$= 386.2 |

(continued)

| No. | Structure | Nuclear magnetic resonance and mass spectrometry data |
|---|---|---|
| B214 | 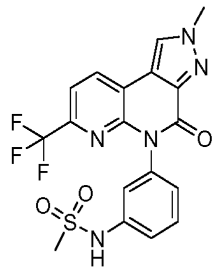 | $^1$H NMR (400 MHz, DMSO-*d6*) δ 9.92 (s, 1H), 8.94 (s, 1H), 8.68 (d, *J* = 7.9 Hz, 1H), 7.80 (d, *J* = 7.9 Hz, 1H), 7.53 (t, *J* = 8.0 Hz, 1H), 7.37 - 7.28 (m, 1H), 7.18 (t, *J* = 2.0 Hz, 1H), 7.10 (d, *J* = 8.0 Hz, 1H), 4.23 (s, 3H), 3.00 (s, 3H). LC-MS [M+H]$^+$= 438.1. |
| B215 | 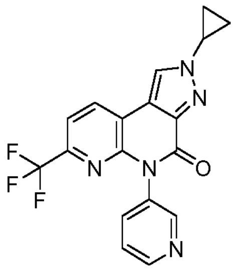 | $^1$H NMR (400 MHz, DMSO-$d_6$) δ 9.12 (d, *J* = 3.7 Hz, 1H), 8.69 (dt, *J* = 6.1, 3.2 Hz, 2H), 8.57 (d, *J* = 2.6 Hz, 1H), 7.90 - 7.78 (m, 2H), 7.64 (dd, *J* = 8.1, 4.7 Hz, 1H), 4.21 (dq, *J* = 7.6, 3.9 Hz, 1H), 1.31 (q, *J* = 4.1, 3.2 Hz, 2H), 1.22 (s, 2H).<br><br>LC-MS [M+H]$^+$= 372.1. |
| B216 | 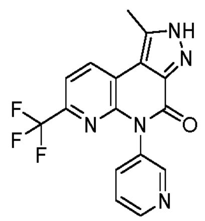 | $^1$H NMR (400 MHz, DMSO-d6) δ 14.42 (s, 1H), 8.70 (dd, *J* = 4.9, 1.5 Hz, 1H), 8.66 (d, *J* = 8.0 Hz, 1H), 8.60 (d, *J* = 2.4 Hz, 1H), 7.90 (dt, *J* = 8.0, 2.0 Hz, 1H), 7.82 (d, *J* = 8.0 Hz, 1H), 7.65 (dd, *J* = 8.0, 4.8 Hz, 1H), 2.82 (s, 3H). LC-MS [M+H]$^+$= 346.1. |
| B217 | 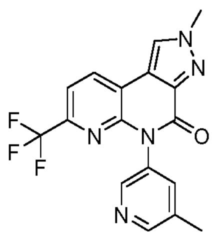 | $^1$H NMR (400 MHz, DMSO-d6) δ 8.93 (s, 1H), 8.68 (d, *J* = 7.9 Hz, 1H), 8.53 (s, 1H), 8.36 (d, *J* = 2.2 Hz, 1H), 7.80 (d, *J* = 7.9 Hz, 1H), 7.69 (d, *J* = 2.2 Hz, 1H), 4.22 (s, 3H), 2.41 (s, 3H). LC-MS [M+H]$^+$= 360.1 |
| B218 | 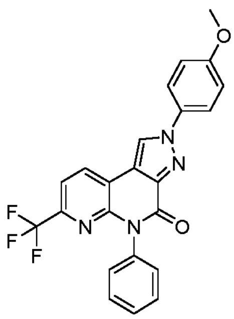 | $^1$HNMR (400 MHz, DMSO-d6) δ 9.67 (s, 1H), 8.71 (d, *J* = 7.9 Hz, 1H), 8.07 - 7.92 (m, 2H), 7.86 (d, *J* = 7.9 Hz, 1H), 7.59 (dd, *J* = 8.2, 6.7 Hz, 2H), 7.52 (d, *J* = 7.3 Hz, 1H), 7.41 - 7.35 (m, 2H), 7.28 - 7.19 (m, 2H), 3.89 (s, 3H). LC-MS [M+H]$^+$= 437.2. |
| B219 | 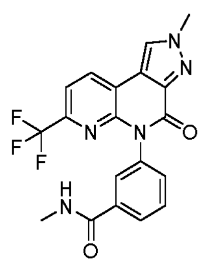 | $^1$H NMR (400 MHz, DMSO-d6) δ 8.96 (s, 1H), 8.70 (d, J = 7.8 Hz, 1H), 8.54 (d, J = 4.7 Hz, 1H), 8.06 - 7.94 (m, 1H), 7.87 - 7.76 (m, 2H), 7.66 (t, J = 7.8 Hz, 1H), 7.57 - 7.48 (m, 1H), 4.23 (s, 3H), 2.82 (d, J = 4.5 Hz, 3H). LC-MS [M+H]$^+$= 402.1. |

(continued)

| No. | Structure | Nuclear magnetic resonance and mass spectrometry data |
|---|---|---|
| B220 | 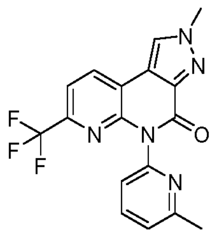 | $^1$H NMR (400 MHz, DMSO-*d6*) $\delta$ 8.96 (s, 1H), 8.69 (dd, J = 8.0, 0.9 Hz, 1H), 7.95 (d, J = 7.7 Hz, 1H), 7.80 (d, J = 7.9 Hz, 1H), 7.43 (d, J = 7.6 Hz, 1H), 7.34 (d, J = 7.7 Hz, 1H), 4.23 (s, 3H), 2.54 (d, J =2.0 Hz, 3H). LC-MS $[M+H]^+$= 360.1. |
| B221 | 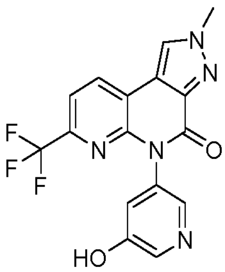 | $^1$H NMR (400 MHz, DMSO-d6) δ 10.36 (s, 1H), 8.94 (d, *J* = 3.4 Hz, 1H), 8.73 - 8.63 (m, 1H), 8.25 (d, *J* = 2.7 Hz, 1H), 8.01 (d, *J* = 2.1 Hz, 1H), 7.81 (dd, *J* = 8.0, 3.3 Hz, 1H), 7.24 (q, *J* = 2.6 Hz, 1H), 4.23 (d, *J* = 3.3Hz, 3H). LC-MS $[M+H]^+$= 362.0. |
| B222 | 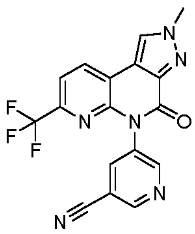 | $^1$H NMR (400 MHz, DMSO-d6) δ 9.19 (s, 1H), 8.98 (s, 2H), 8.73 (d, *J* = 7.9 Hz, 1H), 8.58 (s, 1H), 7.87 (d, *J* = 7.9 Hz, 1H), 4.24 (s, 3H). LC-MS $[M+H]^+$= 371.1. |
| B223 | 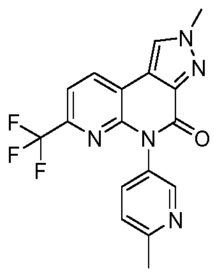 | $^1$H NMR (400 MHz, DMSO-$d_6$) $\delta$ 8.95 (s, 1H), 8.72 - 8.67 (m, 1H), 8.44 - 8.40 (m, 1H), 7.82 (d, *J* = 7.9 Hz, 1H), 7.73 (dd, *J* = 8.2, 2.5 Hz, 1H), 7.47 (d, *J* = 8.2 Hz, 1H), 4.23 (s, 3H), 2.61 (s, 3H). LC-MS $[M+H]^+$= 360.1. |
| B224 | 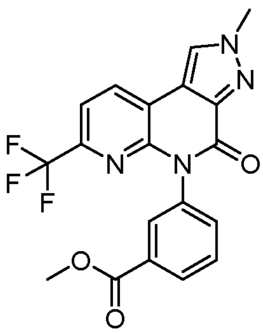 | $^1$H NMR (400 MHz, DMSO-$d_6$) δ 8.95 (d, *J* = 2.4 Hz, 1H), 8.69 (dt, *J* = 7.7, 3.4 Hz, 1H), 8.14 - 8.07 (m, 1H), 7.98 (m, 1H), 7.80 (dd, *J* = 7.9, 2.5 Hz, 1H), 7.74 (td, *J* = 7.7, 2.6 Hz, 1H), 7.70 - 7.65 (m, 1H), 4.24 (t, *J* = 3.3 Hz, 3H) , 3.90 (s, 3H). LC-MS $[M+H]^+$ = 403.1. |

(continued)

| No. | Structure | Nuclear magnetic resonance and mass spectrometry data |
|---|---|---|
| B225 | 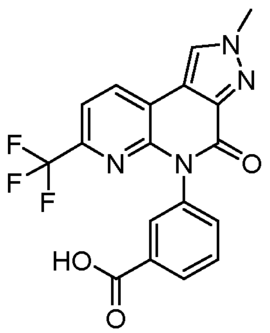 | $^1$H NMR (400 MHz, DMSO-$d_6$) δ 8.95 (s, 1H), 8.68 (d, *J* = 7.9 Hz, 1H), 8.02 (d, *J* = 7.8 Hz, 1H), 7.78 (d, *J* = 8.0 Hz, 2H), 7.53 (t, *J* = 7.8 Hz, 1H), 7.37 (d, *J* = 7.6 Hz, 1H), 4.23 (s, 3H). LC-MS $[M+H]^+$ = 389.1. |
| B226 | 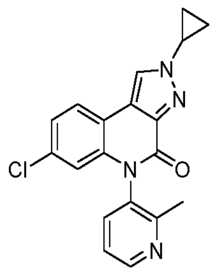 | $^1$H NMR (400 MHz, DMSO_$d_6$): δ 8.95 (s, 1H), 8.67 (d, *J*=4.8 Hz, 1 H), 8.07 (d, *J*=8.0 Hz, 1 H), 7.80 (d, *J*=8.0 Hz, 1H), 7.56-7.51 (m, 1H), 7.37 (d, *J*=8.0 Hz, 1 H), 6.29 (s, 1H), 4.16-4.11 (m, 1H), 2.14 (s, 3H), 1.30-1.26 (m, 2H), 1.19-1.14 (m, 2H). LC-MS $[M+H]^+$ = 351.0 |
| B227 | 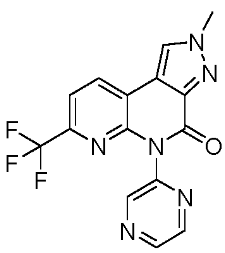 | $^1$H NMR (400 MHz, DMSO-d6) *δ* 9.00 (s, 1H), 8.91 (d, J = 1.1 Hz, 1H), 8.85 (d, J = 1.4 Hz, 2H), 8.74 (d, J = 7.9 Hz, 1H), 7.86 (d, J = 8.0 Hz, 1H), 4.25 (s, 3H).<br><br>LC-MS $[M+H]^+$ = 347.1. |
| B228 | 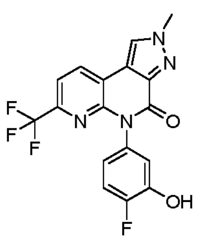 | $^1$H NMR (400 MHz, DMSO-*d6*) *δ* 10.07 (s, 1H), 8.93 (s, 1H), 8.76 - 8.60 (m, 1H), 7.79 (d, J = 7.9 Hz, 1H), 7.30 (dd, J = 11.2, 8.6 Hz, 1H), 6.92 (dd, J = 8.0, 2.5 Hz, 1H), 6.76 (ddd, J = 8.5, 4.0, 2.5 Hz,1H), 4.22 (s, 3H). LC-MS $[M+H]^+$ = 379.1. |
| B229 | 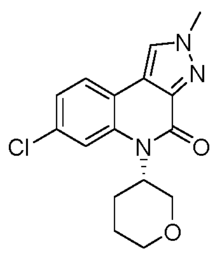 | 1H NMR (400 MHz, DMSO) δ 8.63 (s, 1H), 7.95 (d, *J* = 8.4 Hz, 1H), 7.74 (s, 1H), 7.33 (dd, *J* = 8.4 Hz, 1.2 Hz, 1H), 4.64 (s, 1H), 4.48-4.42 (m, 1H), 4.11 (s, 3H), 3.91-3.82 (m, 2H), 3.44-3.39 (m, 1H), 2.82-2.73 (m, 1H), 1.98-1.74 (m, 3H). LC-MS $[M+H]^+$ = 318.0 |
| B230 | 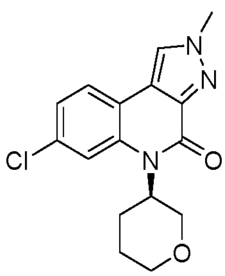 | 1H NMR (400 MHz, DMSO) δ 8.63 (s, 1H), 7.95 (d, *J* = 8.4 Hz, 1H), 7.74 (s, 1H), 7.33 (dd, *J* = 8.4 Hz, 1.2 Hz, 1H), 4.64 (s, 1H), 4.48-4.42 (m, 1H), 4.11 (s, 3H), 3.91-3.82 (m, 2H), 3.44-3.39 (m, 1H), 2.82-2.73 (m, 1H), 1.98-1.74 (m, 3H). LC-MS $[M+H]^+$ =318.0 |

(continued)

| No. | Structure | Nuclear magnetic resonance and mass spectrometry data |
|---|---|---|
| B231 | 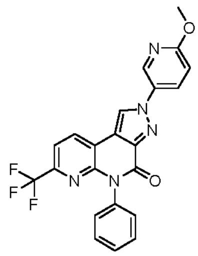 | $^1$HNMR (400 MHz, DMSO-d6) δ 9.67 (d, *J* = 0.9 Hz, 1H), 8.86 (d, *J* = 2.8 Hz, 1H), 8.69 (d, *J* = 7.9 Hz, 1H), 8.38 (dd, *J* = 9.0, 2.9 Hz, 1H), 7.86 (d, *J* = 7.9 Hz, 1H), 7.59 (dd, *J* = 8.3, 6.7 Hz, 2H), 7.56 - 7.49 (m, 1H), 7.42 - 7.36 (m, 2H), 7.18 - 7.12 (m, 1H), 3.99 (d, *J* = 0.8 Hz, 3H). LC-MS [M+H] + = 438.4. |
| B232 | 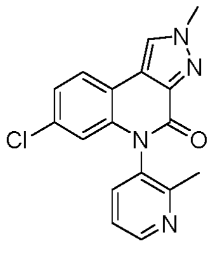 IsomerA | |
| B233 | 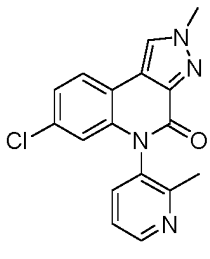 IsomerB | |
| B234 | 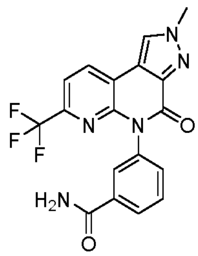 | $^1$H NMR (400 MHz, DMSO-$d_6$) δ 8.95 (s, 1H), 8.69 (d, *J* = 8.0 Hz, 1H), 8.07 - 7.99 (m, 2H), 7.85 (t, *J* = 1.9 Hz, 1H), 7.80 (d, *J* = 7.9 Hz, 1H), 7.65 (t, *J* = 7.8 Hz, 1H), 7.53 (ddd, *J* = 7.8, 2.0, 1.1 Hz, 1H), 7.46 (s, 1H), 4.23 (s, 3H). LC-MS [M+H] + = 388.3 |
| B235 | 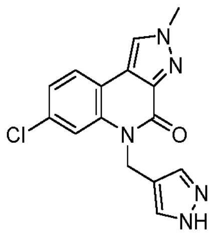 | $^1$H NMR (400 MHz, DMSO-$d_6$) δ 12.75 (s, 1H), 8.71 (s, 1H), 7.98 (d, *J* = 8.3 Hz, 1H), 7.75 - 7.64 (m, 2H), 7.44 (s, 1H), 7.33 (dd, *J* = 8.3, 1.8 Hz, 1H), 5.42 (s, 2H), 4.16 (s, 3H). LC-MS [M+H]+= 314.1. |

(continued)

| No. | Structure | Nuclear magnetic resonance and mass spectrometry data |
|---|---|---|
| B236 | 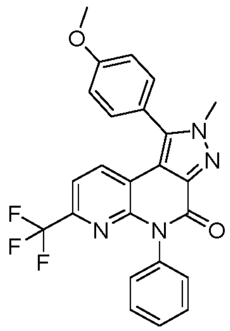 | $^1$H NMR (400 MHz, DMSO-d6) δ 7.87 (d, *J* = 8.0 Hz, 1H), 7.70 - 7.54 (m, 5H), 7.54 - 7.47 (m, 1H), 7.38 - 7.32 (m, 2H), 7.32 - 7.25 (m, 2H), 3.97 (s, 3H), 3.93 (s, 3H). LC-MS $[M+H]^+$= 451.3. |
| B237 | 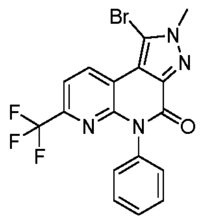 | $^1$H NMR (400 MHz, DMSO-$d_6$) δ 8.94 (d, *J* = 8.0 Hz, 1H), 7.87 (d, *J* = 8.1 Hz, 1H), 7.57 (t, *J* = 7.5 Hz, 2H), 7.50 (t, *J* = 7.3 Hz, 1H), 7.35 (d, *J* = 7.5 Hz, 2H), 4.21 (s, 3H). LC-MS $[M+H]^+$= 423. 2 |
| B238 | 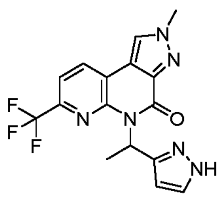 | $^1$H NMR (400 MHz, DMSO-$d_6$) δ 8.85 (s, 1H), 8.60 (d, *J* = 7.9 Hz, 1H), 7.63 (d, *J* = 96.0 Hz, 2H), 6.88 (s, 1H), 6.16 (d, *J* = 2.0 Hz, 1H), 4.19 (s, 3H), 3.98 (s, 1H), 1.97 (d, *J* = 7.1 Hz, 3H). LC-MS $[M+H]^+$= 363.2. |
| B239 | 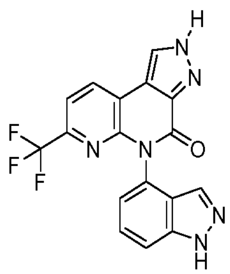 | $^1$H NMR (400 MHz, DMSO-$d_6$) δ 13.22 (s, 1H), 8.73 (d, *J* = 12.8 Hz, 2H), 7.76 (d, *J* = 7.9 Hz, 1H), 7.74 - 7.66 (m, 2H), 7.56 - 7.49 (m, 1H), 7.11 (d, *J* = 7.2 Hz, 1H). LC-MS m/z$[M+H]^+$ = 371.2. |
| B240 | 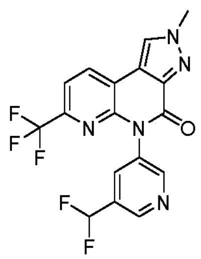 | $^1$H NMR (400 MHz, DMSO-d6) δ 8.93 (d, *J* = 9.8 Hz, 2H), 8.80 (s, 1H), 8.69 (d, *J* = 7.9 Hz, 1H), 8.19 (s, 1H), 7.82 (d, *J* = 7.9 Hz, 1H), 7.30 (t, *J* = 55.1 Hz, 1H), 4.23 (s, 3H). LC-MS $[M+H]^+$= 396.3. |
| B241 | 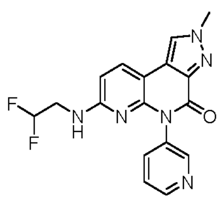 | $^1$H NMR (400 MHz, DMSO-d6) δ 8.64 (d, J = 3.3 Hz, 1H), 8.56 - 8.42 (m, 2H), 8.05 (d, J = 8.4 Hz, 1H), 7.88 - 7.73 (m, 1H), 7.59 (ddd, J = 8.0, 4.8, 0.8 Hz, 1H), 7.32 (t, J = 5.8 Hz, 1H), 6.53 (d, J = 8.5 Hz, 1H), 5.58 (tt, J = 57.1, 4.4 Hz, 1H), 4.16 (s, 3H), 3.27 - 3.05 (m, 2H). LC-MS $[M+H]^+$= 357.3. |

(continued)

| No. | Structure | Nuclear magnetic resonance and mass spectrometry data |
|---|---|---|
| B242 | 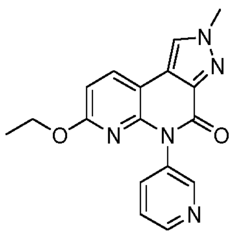 | $^1$H NMR (400 MHz, DMSO-*d6*) δ 8.73 - 8.62 (m, 2H), 8.57 (dd, *J* = 2.4, 0.8 Hz, 1H), 8.33 (d, *J* = 8.4 Hz, 1H), 7.87 (ddd, *J* = 8.0, 2.5, 1.6 Hz, 1H), 7.62 (ddd, *J* = 8.0, 4.8, 0.8 Hz, 1H), 6.74 (d, *J* = 8.3 Hz, 1H), 4.19 (s, 3H), 3.85 (q, *J* = 7.0 Hz, 2H), 1.06 (t, *J* = 7.0 Hz, 3H). LC-MS [M+H]$^+$= 322.2. |
| B243 | 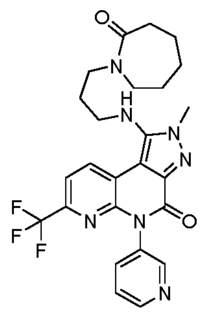 | $^1$H NMR (400 MHz, DMSO-*d6*) *δ* 8.68 (dd, J = 4.8, 1.5 Hz, 1H), 8.60 (d, J = 8.0 Hz, 1H), 8.57 - 8.52 (m, 1H), 7.85 (ddd, J = 8.1, 2.5, 1.6 Hz, 1H), 7.79 (d, J = 8.0 Hz, 1H), 7.63 (ddd, J = 8.1, 4.8, 0.8 Hz,1H), 5.73 (t, J = 6.7 Hz, 1H), 4.04 (s, 3H), 3.42 (t, J = 6.9 Hz, 2H), 3.38 (s, 2H), 3.16 (q, J = 6.9 Hz, 2H), 2.49 - 2.41 (m, 2H), 1.79 (p, J = 7.0 Hz, 2H), 1.70 - 1.59 (m, 2H), 1.52 (d, J = 5.4 Hz, 4H). LC-MS [M+H]$^+$= 514.4. |
| B244 | 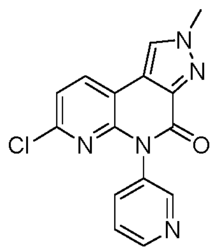 | $^1$H NMR (400 MHz, DMSO-$d_6$) δ 8.84 (s, 1H), 8.69 (dd, *J* = 4.8, 1.5 Hz, 1H), 8.57 (d, *J* = 2.5 Hz, 1H), 8.50 (d, *J* = 8.1 Hz, 1H), 7.87 (ddd, *J* = 8.1, 2.5, 1.6 Hz, 1H), 7.69 - 7.57 (m, 1H), 7.44 (d, *J* = 8.1 Hz, 1H), 4.21 (s, 3H). LC-MS [M+H]$^+$= 312. 1 |
| B245 | 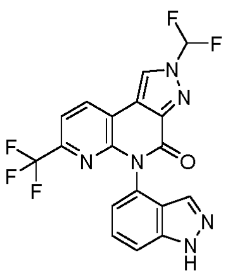 | $^1$H NMR (400 MHz, DMSO-d6) δ 13.24 (s, 1H), 9.61 (s, 1H), 8.86 (d, *J* = 7.9 Hz, 1H), 8.28 (t, *J* = 58.8 Hz, 1H), 7.91 - 7.81 (m, 2H), 7.69 (d, *J* = 8.4 Hz, 1H), 7.52 (dd, *J* = 8.4, 7.2 Hz, 1H), 7.14 (d, *J* = 7.2 Hz, 1H). LC-MS [M+H]$^+$= 421.2. |
| B246 | 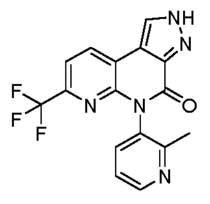<br>Isomer A | |
| B247 | 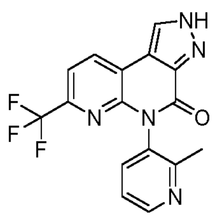<br>Isomer B | |

(continued)

| No. | Structure | Nuclear magnetic resonance and mass spectrometry data |
|---|---|---|
| B248 | 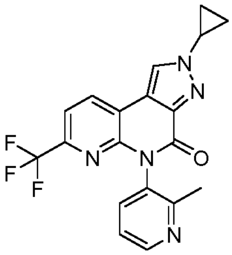 Isomer A | |
| B249 | 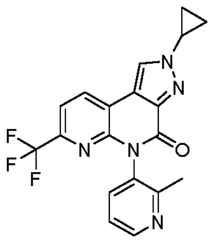 Isomer B | |
| B250 | 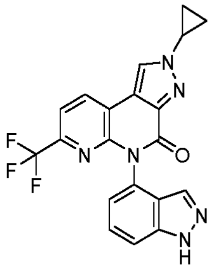 | $^1$H NMR (400 MHz, DMSO-d6) δ 13.23 (s, 1H), 9.14 (s, 1H), 8.69 (d, *J* = 7.9 Hz, 1H), 7.80 (d, *J* = 7.9 Hz, 1H), 7.74 (s, 1H), 7.68 (d, *J* = 8.4 Hz, 1H), 7.52 (t, *J* = 7.8 Hz, 1H), 7.09 (d, *J* = 7.2 Hz, 1H), 4.22 (tt, *J* =7.5, 3.9 Hz, 1H), 1.33 (q, *J* = 4.2 Hz, 2H), 1.23 (dd, *J* = 7.5, 5.0 Hz, 2H). LC-MS [M+H]$^+$= 411.3 |
| B251 | 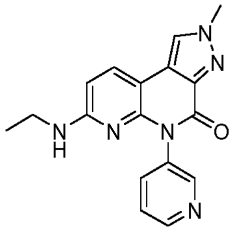 | $^1$H NMR (400 MHz, DMSO-*d6*) δ 8.63 (d, *J* = 4.9 Hz, 1H), 8.50 - 8.45 (m, 2H), 7.97 - 7.92 (m, 1H), 7.78 (dt, *J* = 8.0, 2.0 Hz, 1H), 6.84 (t, *J* = 5.5 Hz, 1H), 6.39 (d, *J* = 8.7 Hz, 1H), 4.14 (s, 3H), 2.88 - 2.78 (m, 2H), 0.84 (t, *J* = 7.1 Hz, 3H). LC-MS [M+H]$^+$= 321.2 |
| B252 | 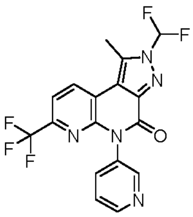 | $^1$H NMR (400 MHz, DMSO-*d6*) *δ* 8.76 (d, J = 8.0 Hz, 1H), 8.70 (dd, J = 4.8, 1.5 Hz, 1H), 8.60 (d, J = 2.5 Hz, 1H), 8.32 (t, J = 57.0 Hz, 1H), 7.89 (dt, J = 8.2, 1.8 Hz, 1H), 7.82 (d, J = 8.0 Hz, 1H), 7.65 (dd,J = 8.1, 4.8 Hz, 1H), 3.06 (s, 3H). LC-MS [M+H]$^+$= 396.2. |
| B253 | 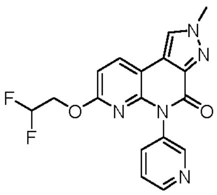 | $^1$H NMR (400 MHz, DMSO-*d6*) *δ* 8.72 (s, 1H), 8.69 (dd, J = 4.8, 1.5 Hz, 1H), 8.59 (dd, J = 2.5, 0.8 Hz, 1H), 8.43 (d, J = 8.4 Hz, 1H), 7.89 (ddd, J = 8.0, 2.5, 1.5 Hz, 1H), 7.64 (ddd, J = 8.1, 4.8, 0.8 Hz,1H), 6.88 (d, J = 8.4 Hz, 1H), 6.06 (tt, J = 55.2, 3.9 Hz, 1H), 4.20 (s, 3H), 4.06 (td, J = 14.5, 3.8 Hz, 2H). LC-MS [M+H]$^+$= 358.3. |

(continued)

| No. | Structure | Nuclear magnetic resonance and mass spectrometry data |
|---|---|---|
| B254 | 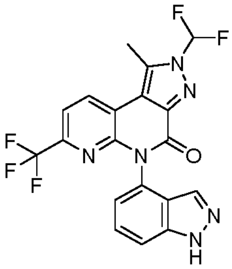 | $^1$H NMR (400 MHz, DMSO-*d6*) $\delta$ 13.28 (s, 1H), 8.82 (d, J = 8.0 Hz, 1H), 8.37 (t, J = 56.9 Hz, 1H), 7.88 - 7.80 (m, 2H), 7.73 (d, J = 8.5 Hz, 1H), 7.57 (t, J = 7.9 Hz, 1H), 7.17 (d, J = 7.3 Hz, 1H), 3.13 (s,3H). LC-MS $[M+H]^+$= 435.3. |
| B255 | 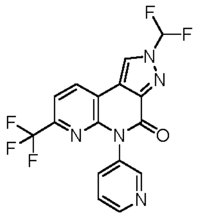 | $^1$H NMR (400 MHz, DMSO-$d_6$) $\delta$ 9.59 (s, 1H), 8.85 (d, *J* = 7.9 Hz, 1H), 8.71 (dd, *J* = 4.8, 1.5 Hz, 1H), 8.61 (dd, *J* = 2.4, 0.8 Hz, 1H), 8.28 (t, *J* = 58.8 Hz, 1H), 7.92 - 7.88 (m, 2H), 7.65 (ddd, *J* = 8.1, 4.8, 0.8 Hz, 1H). LC-MS $[M+H]^+$ = 382.3. |
| B256 | 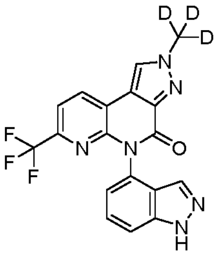 | $^1$H NMR (400 MHz, DMSO-*d6*) $\delta$ 13.28 - 13.13 (m, 1H), 8.97 (s, 1H), 8.71 (d, J = 7.9 Hz, 1H), 7.78 (d, J = 7.9 Hz, 1H), 7.72 (t, J = 1.2 Hz, 1H), 7.68 (d, J = 8.4 Hz, 1H), 7.52 (dd, J = 8.4, 7.2 Hz, 1H), 7.11 (d, J = 7.2 Hz, 1H). LC-MS m/z $[M+H]^+$ = 388.3. |
| B257 | 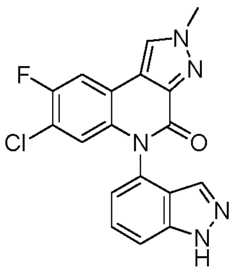 | $^1$H NMR (400 MHz, DMSO-$d_6$) $\delta$ 13.49 (s, 1H), 8.82 (s, 1H), 8.22 (d, *J* = 9.6 Hz, 1H), 7.80 (d, *J* = 9.2 Hz, 2H), 7.62 (t, *J* = 7.8 Hz, 1H), 7.20 (d, *J* = 7.2 Hz, 1H), 6.40 (d, *J* = 6.5 Hz, 1H), 4.21 (s, 3H).<br>LC-MS m/z$[M+H]^+$:368.2. |
| B258 | 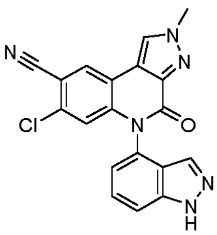 | $^1$H NMR (400 MHz, DMSO-d6) $\delta$ 13.50 (s, 1H), 8.83 (d, *J* = 20.9 Hz, 2H), 7.86 - 7.75 (m, 2H), 7.62 (t, *J* = 7.8 Hz, 1H), 7.22 (d, *J* = 7.2 Hz, 1H), 6.49 (s, 1H), 4.23 (s, 3H). LC-MS $[M+H]^+$: 375.3. |
| B259 | 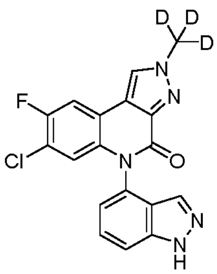 | |

(continued)

| No. | Structure | Nuclear magnetic resonance and mass spectrometry data |
|---|---|---|
| B260 | 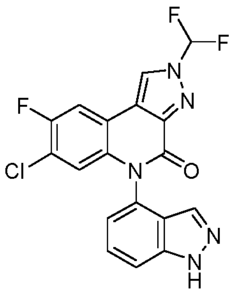 | $^1$H NMR (400 MHz, DMSO-$d_6$) $\delta$ 13.49 (s, 1H), 9.45 (s, 1H), 8.44 - 8.11 (m, 2H), 7.92 (s, 1H), 7.82 (d, J = 8.4 Hz, 1H), 7.63 (dd, J = 8.4, 7.2 Hz, 1H), 7.23 (d, J = 7.2 Hz, 1H), 6.42 (d, J = 6.4 Hz, 1H). LC-MS $[M+H]^+$: 404.1. |
| B261 | 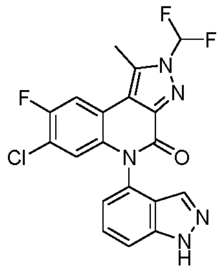 | |
| B262 | 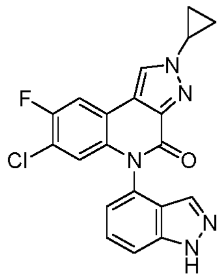 | |
| B263 | 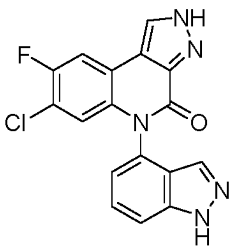 | |
| B264 | 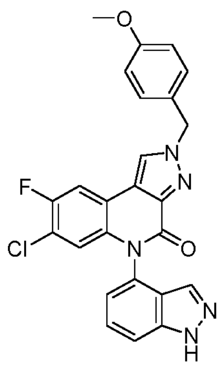 | |

(continued)

| No. | Structure | Nuclear magnetic resonance and mass spectrometry data |
|---|---|---|
| B265 | 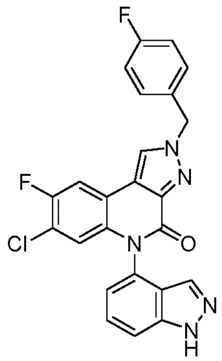 | |
| B266 | 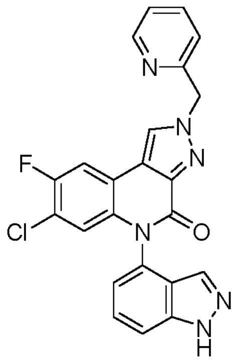 | |
| B267 | 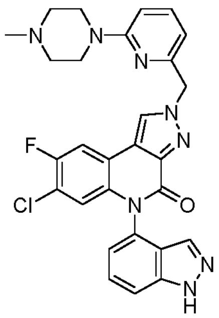 | |
| B268 | 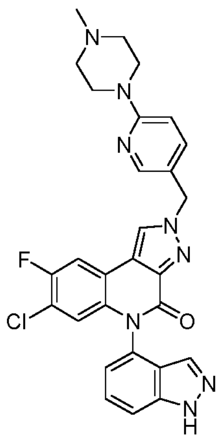 | $^1$H NMR (400 MHz, DMSO-d6) δ 13.47 (s, 1H), 8.90 (s, 1H), 8.30 (d, *J* = 2.4 Hz, 1H), 8.23 (d, *J* = 9.6 Hz, 1H), 7.85 - 7.73 (m, 2H), 7.67 (dd, *J* = 8.8, 2.5 Hz, 1H), 7.63 - 7.58 (m, 1H), 7.17 (d, *J* = 7.2 Hz, 1H), 6.92 (d, *J* = 8.8 Hz, 1H), 6.36 (d, *J* = 6.5 Hz, 1H), 5.57 (s, 2H), 3.56 (s, 4H), 2.55 (s, 3H), 2.35 (d, *J* = 5.8 Hz, 4H).<br>LC-MS $[M+H]^+$: 543.2 |

(continued)

| No. | Structure | Nuclear magnetic resonance and mass spectrometry data |
|---|---|---|
| B269 | 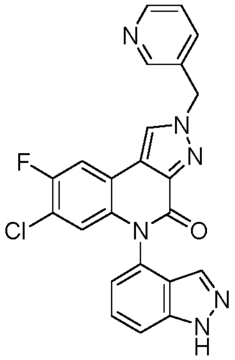 | |
| B270 | 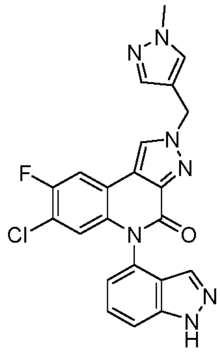 | |
| B271 | 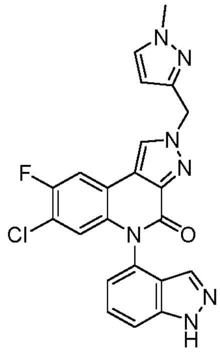 | |
| B272 | 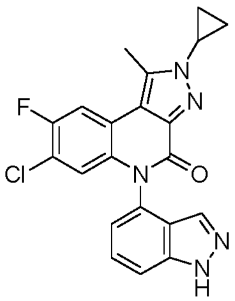 | |
| B273 | 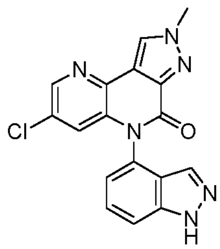 | $^1$H NMR (400 MHz, DMSO-d6) δ 13.52 (s, 1H), 8.85 (s, 1H), 8.51 (d, *J* = 2.1 Hz, 1H), 7.86 (s, 1H), 7.82 (d, *J* = 8.4 Hz, 1H), 7.62 (dd, *J* = 8.4, 7.2 Hz, 1H), 7.23 (d, *J* = 7.2 Hz, 1H), 6.69 (d, *J* = 2.1 Hz, 1H), 4.24 (s, 3H). LC-MS $[M+H]^+$ = 351.1. |

(continued)

| No. | Structure | Nuclear magnetic resonance and mass spectrometry data |
|---|---|---|
| B274 | 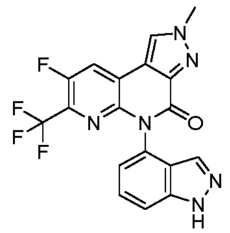 | $^1$H NMR (400 MHz, DMSO-d6) δ 13.24 (s, 1H), 8.93 (s, 1H), 8.83 (d, J = 10.3 Hz, 1H), 7.76 (s, 1H), 7.68 (d, J = 8.3 Hz, 1H), 7.57-7.47 (m, 1H), 7.12 (d, J = 7.2 Hz, 1H), 4.26 (s, 3H). LC-MS $[M+H]^+$ = 403.1. |
| B275 | 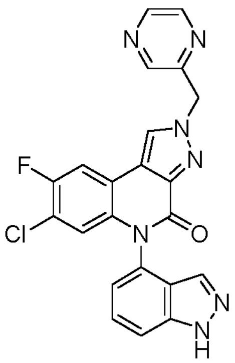 | |
| B276 | 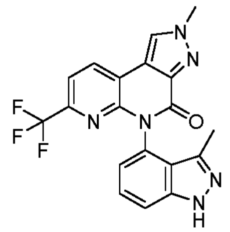 | |
| B277 | 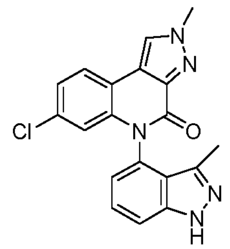 | $^1$H NMR (400 MHz, DMSO-d6) δ 13.06 (s, 1H), 8.83 (s, 1H), 8.10 (d, J = 8.4 Hz, 1H), 7.72 (d, J = 8.4 Hz, 1H), 7.58 (dd, J = 8.4, 7.2 Hz, 1H), 7.36 (dd, J = 8.4, 2.0 Hz, 1H), 7.12 (d, J = 7.1 Hz, 1H), 6.29 (d, J = 2.0 Hz, 1H), 4.21 (s, 3H), 1.80 (s, 3H). LC-MS $[M+H]^+$ = 364.2. |
| B278 | 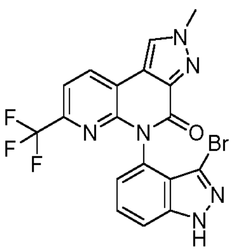 | |
| B279 | 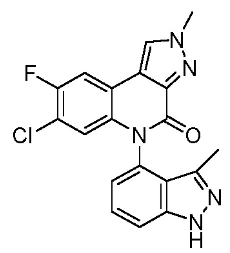 | |

(continued)

| No. | Structure | Nuclear magnetic resonance and mass spectrometry data |
|---|---|---|
| B280 | 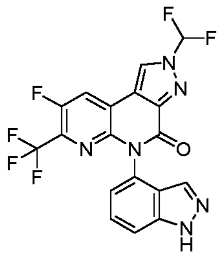 | |
| B281 | 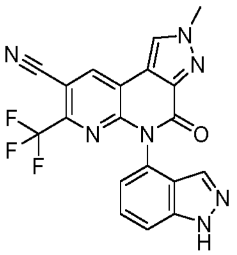 | $^1$H NMR (400 MHz, DMSO-$d_6$) δ 9.06 (s, 1H), 8.82 (s, 1H), 7.78 - 7.73 (m, 2H), 7.64 - 7.55 (m, 2H), 7.17 (d, J = 7.2 Hz, 1H), 4.31 (s, 3H). LCMS m/z $[M+H]^+$: 410.1. |
| B282 | 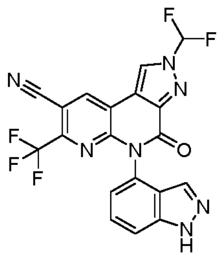 | |
| B283 | 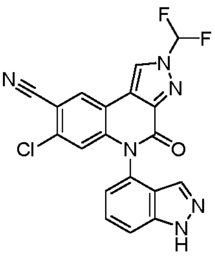 | 1H NMR (400 MHz, DMSO-*d*6) δ 13.53 (s, 1H), 9.47 (s, 1H), 8.94 (s, 1H), 8.29 (t, *J* = 58.8 Hz, 1H), 7.98 (s, 1H), 7.83 (d, *J* = 8.4 Hz, 1H), 7.63 (dd, *J* = 8.5, 7.2 Hz, 1H), 7.26 (d, *J* = 7.2 Hz, 1H), 6.50 (s, 1H). |
| B284 | 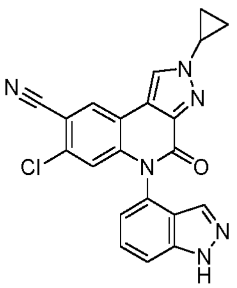 | $^1$H NMR (400 MHz, DMSO-$d_6$) δ 13.53 (s, 1H), 9.05 (s, 1H), 8.78 (s, 1H), 7.87 (s, 1H), 7.82 (d, *J* = 8.4 Hz, 1H), 7.65 - 7.59 (m, 1H), 7.21 (d, *J* = 7.2 Hz, 1H), 6.47 (s, 1H), 4.22 (dq, *J* = 7.6, 3.8 Hz, 1H), 1.31 - 1.28 (m, 2H), 1.24 - 1.20 (m, 2H). LCMS m/z $[M+H]^+$ = 401.1. |
| B285 | 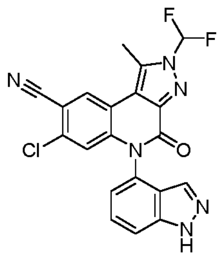 | |

(continued)

| No. | Structure | Nuclear magnetic resonance and mass spectrometry data |
|---|---|---|
| B286 | 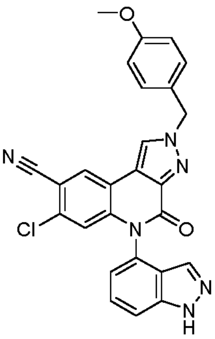 | $^1$H NMR (400 MHz, DMSO-$d_6$) $\delta$ 13.52 (s, 1H), 8.95 (s, 1H), 8.83 (s, 1H), 7.89 (s, 1H), 7.81 (d, J = 8.4 Hz, 1H), 7.61 (t, J = 7.8 Hz, 1H), 7.46 - 7.40 (m, 2H), 7.21 (d, J = 7.1 Hz, 1H), 7.04 - 6.99 (m, 2H), 6.45 (s, 1H), 5.64 (s, 2H), 3.78 (s, 3H). LCMS $[M+H]^+$ = 481.1. |
| B287 | 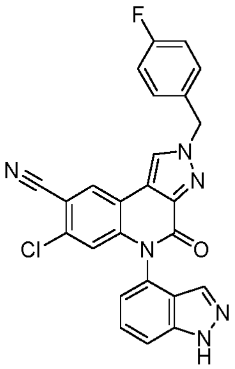 | |
| B288 | 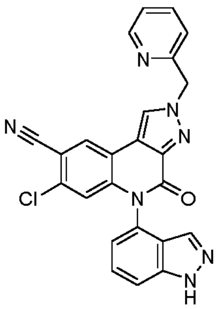 | |
| B289 | 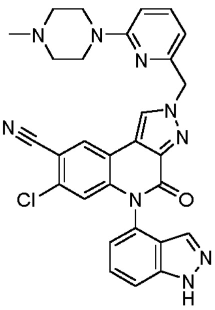 | |

(continued)

| No. | Structure | Nuclear magnetic resonance and mass spectrometry data |
|---|---|---|
| B290 | 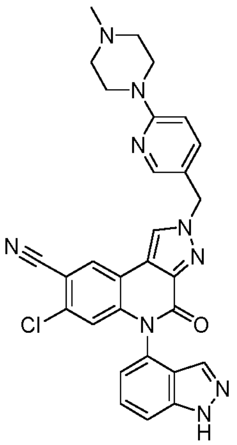 | |
| B291 | 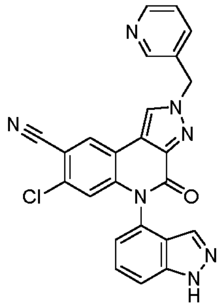 | |
| B292 | 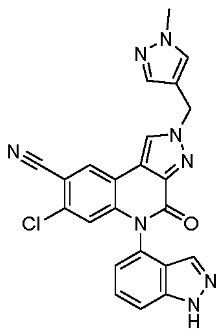 | |
| B293 | 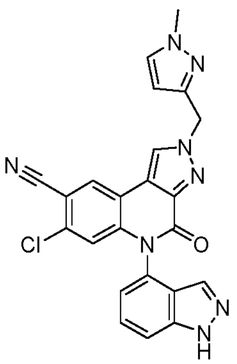 | $^1$H NMR (400 MHz, DMSO-$d_6$) δ 13.52 (s, 1H), 8.93 (s, 1H), 8.87 (s, 1H), 7.88 (s, 1H), 7.82 (d, $J$ = 8.4 Hz, 1H), 7.73 (d, $J$ = 2.2 Hz, 1H), 7.62 (t, $J$ = 7.8 Hz, 1H), 7.21 (d, $J$ = 7.2 Hz, 1H), 6.46 (s, 1H), 6.35 (d, $J$= 2.2 Hz, 1H), 5.64 (s, 2H), 3.87 (s, 3H). LCMS [M+H]$^+$ = 455.1 |

(continued)

| No. | Structure | Nuclear magnetic resonance and mass spectrometry data |
|---|---|---|
| B294 | 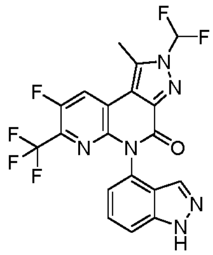 | |
| B295 | 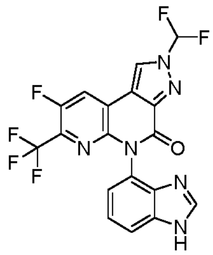 | |
| B296 | 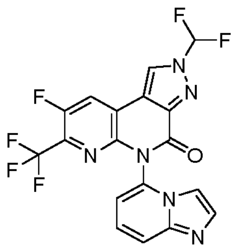 | |
| B297 | 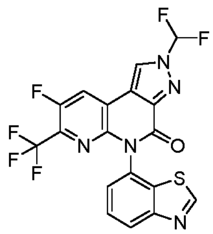 | |
| B298 | 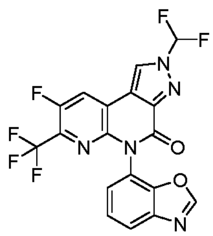 | |
| B299 | 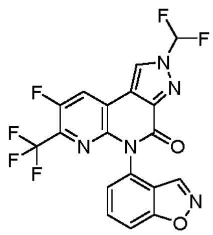 | |

(continued)

| No. | Structure | Nuclear magnetic resonance and mass spectrometry data |
|---|---|---|
| B300 | 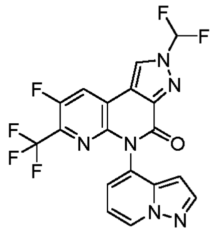 | |
| B301 | 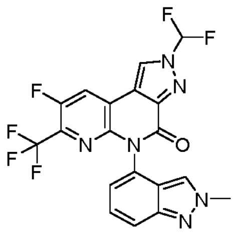 | |
| B302 | 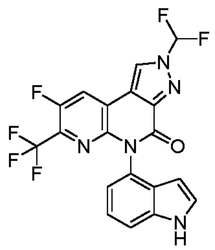 | |
| B303 | 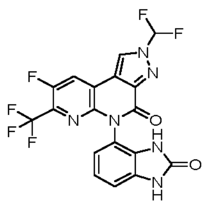 | |
| B304 | 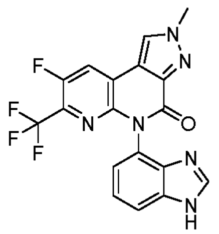 | |
| B305 | 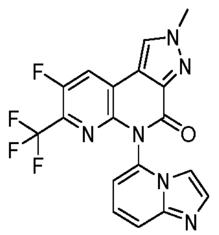 | |
| B306 | 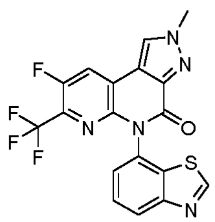 | |

(continued)

| No. | Structure | Nuclear magnetic resonance and mass spectrometry data |
|---|---|---|
| B307 | 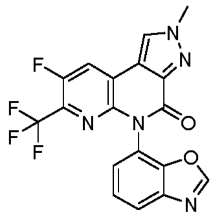 | |
| B308 | 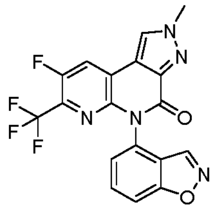 | |
| B309 | 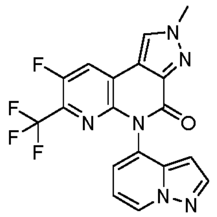 | |
| B310 | 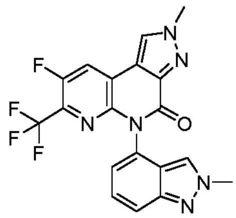 | |
| B311 | 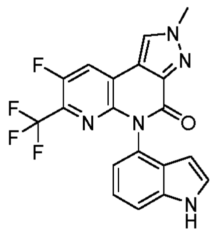 | |
| B312 | 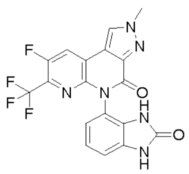 | |
| B313 | 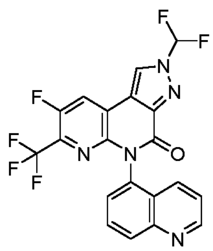 | |

(continued)

| No. | Structure | Nuclear magnetic resonance and mass spectrometry data |
|---|---|---|
| B314 | 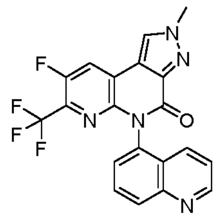 | |
| B315 | 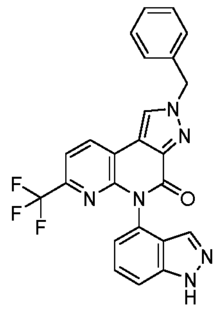 | |
| B316 | 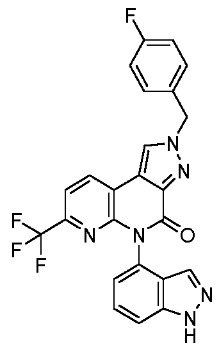 | |
| B317 | 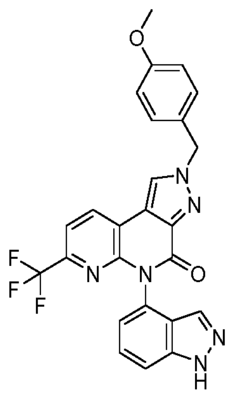 | |
| B318 | 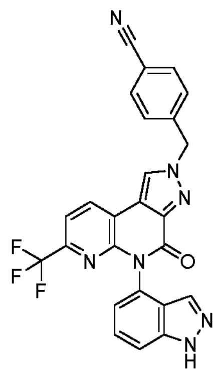 | |

(continued)

| No. | Structure | Nuclear magnetic resonance and mass spectrometry data |
|---|---|---|
| B319 | 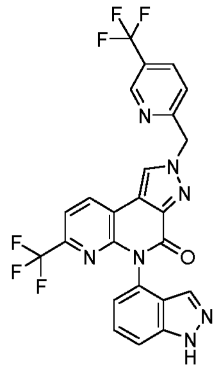 | |
| B320 | 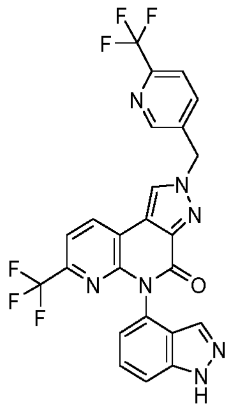 | |
| B321 | 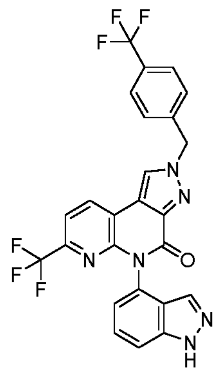 | |
| B322 | 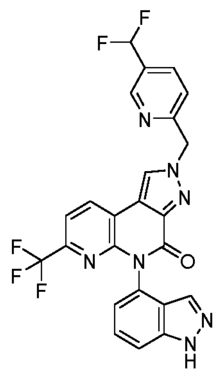 | |

(continued)

| No. | Structure | Nuclear magnetic resonance and mass spectrometry data |
|---|---|---|
| B323 | 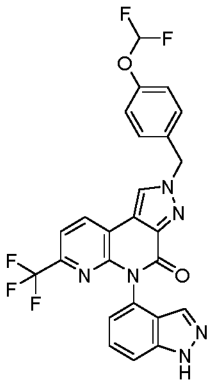 | |
| B324 | 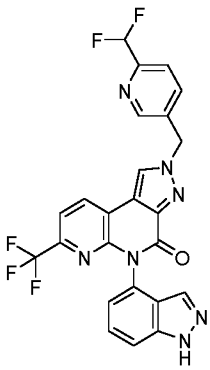 | |
| B325 | 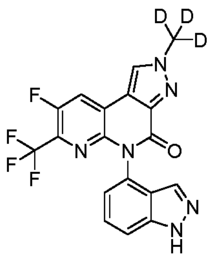 | |
| B326 | 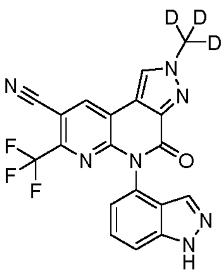 | |
| B327 | 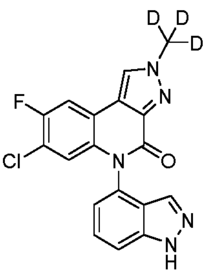 | |

(continued)

| No. | Structure | Nuclear magnetic resonance and mass spectrometry data |
|---|---|---|
| B328 | 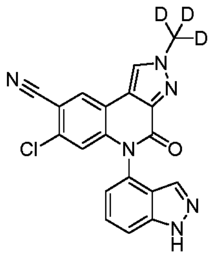 | |
| B329 | 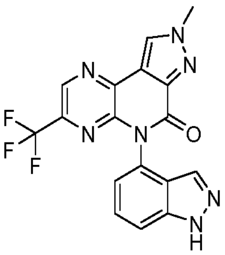 | |
| B330 | 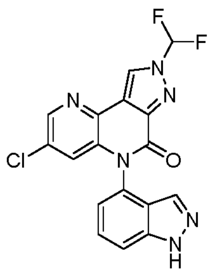 | |
| B331 | 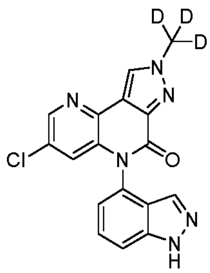 | |
| B332 | 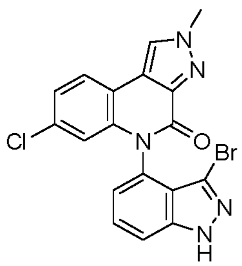 | $^1$H NMR (400 MHz, DMSO-*d6*) δ 13.88 (s, 1H), 8.82 (s, 1H), 8.09 (d, J = 8.3 Hz, 1H), 7.86 (d, J = 8.5 Hz, 1H), 7.70 (t, J = 7.9 Hz, 1H), 7.34 (dd, J = 8.3, 2.1 Hz, 1H), 7.27 (d, J = 7.2 Hz, 1H), 6.26 (d, J = 2.0 Hz, 1H), 4.20 (s, 3H). LCMS $[M+H]^+$ = 428.2. |
| B333 | 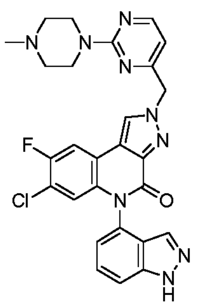 | $^1$H NMR (400 MHz, DMSO-*d6*) δ 13.49 (s, 1H), 9.00 (s, 1H), 8.39 (d, J = 4.9 Hz, 1H), 8.30 (d, J = 9.5 Hz, 1H), 7.89 - 7.76 (m, 2H), 7.62 (t, J = 7.9 Hz, 1H), 7.20 (d, J = 7.2 Hz, 1H), 6.41 (dd, J = 10.2, 5.7Hz, 2H), 5.65 (s, 2H), 3.75 (t, J = 4.9 Hz, 4H), 2.37 (t, J = 4.9 Hz, 4H), 2.23 (s, 3H). LCMS $[M+H]^+$ = 544.1 |

(continued)

| No. | Structure | Nuclear magnetic resonance and mass spectrometry data |
|---|---|---|
| B334 | 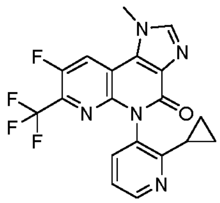 | |
| B335 | 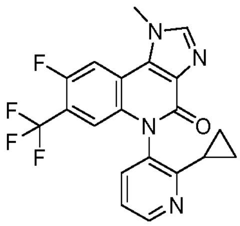 | |
| B336 | 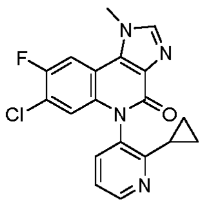 | $^1$H NMR (400 MHz, DMSO-d6) δ 8.66 (d, J = 4.7 Hz, 1H), 8.28 (t, J = 5.0 Hz, 2H), 7.77 (d, J = 7.9 Hz, 1H), 7.46 (dd, J = 7.9, 4.8 Hz, 1H), 6.62 (d, J = 6.5 Hz, 1H), 4.27 (s, 3H), 1.51 (dt, J = 8.2, 3.7 Hz, 1H), 1.07 (d, J = 6.5 Hz, 1H), 0.88 (s, 1H), 0.80 (d, J = 8.3 Hz, 1H), 0.69 (d, J = 8.1 Hz, 1H). LCMS $[M+H]^+$ = 369.1 |
| B337 | 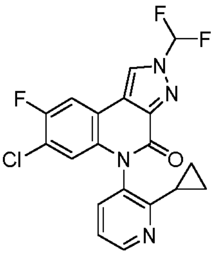 | $^1$H NMR (400 MHz, DMSO-$d_6$) δ 9.43 (s, 1H), 8.67 (dd, *J* = 4.8, 1.6 Hz, 1H), 8.44 - 8.07 (m, 2H), 7.84 (dd, *J* = 7.9, 1.6 Hz, 1H), 7.46 (dd, *J* = 7.9, 4.7 Hz, 1H), 6.51 (d, *J* = 6.4 Hz, 1H), 1.73 (dt, *J* = 8.1, 3.6 Hz, 1H), 1.11 - 0.87 (m, 2H), 0.87 - 0.66 (m, 2H).LCMS $[M+H]^+$ = 405.1 |
| B338 | 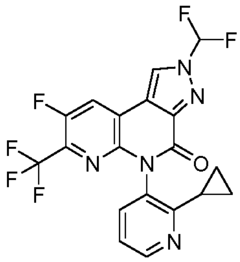 | |
| B339 | 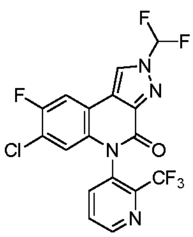 | $^1$H NMR (400 MHz, DMSO-d6) δ 9.45 (s, 1H), 9.03 (d, *J* = 4.6 Hz, 1H), 8.41 - 8.07 (m, 4H), 6.72 (d, *J* = 6.1 Hz, 1H).<br><br>LCMS $[M+H]^+$ =433.1 |
| B340 | 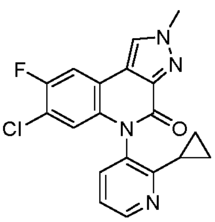 | $^1$H NMR (400 MHz, DMSO-$d_6$) δ 8.78 (s, 1H), 8.62 (dd, *J* = 4.8, 1.7 Hz, 1H), 8.21 (d, *J* = 9.6 Hz, 1H), 7.77 (dd, *J* = 7.9, 1.7 Hz, 1H), 7.42 (dd, *J* = 7.9, 4.7 Hz, 1H), 6.44 (d, *J* = 6.4 Hz, 1H), 4.18 (s, 3H), 1.56 (d, *J* = 4.8 Hz, 1H), 1.02 (d, *J* = 4.6 Hz, 1H), 0.86 (d, *J* = 3.7 Hz, 1H), 0.82 - 0.72 (m, 1H), 0.69 (d, *J* = 7.9 Hz, 1H). LCMS $[M+H]^+$ = 369.1 |

(continued)

| No. | Structure | Nuclear magnetic resonance and mass spectrometry data |
|---|---|---|
| B341 | 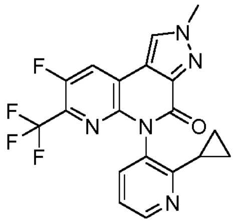 | |
| B342 | 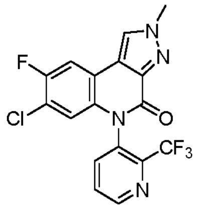 | |
| B343 | 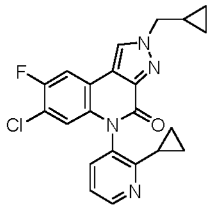 | |
| B344 | 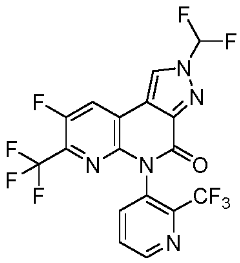 | |
| B345 | 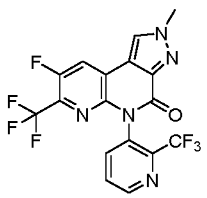 | |
| B346 | 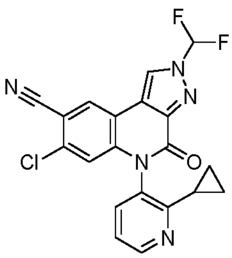 | |
| B347 | 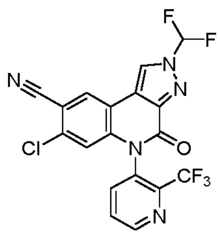 | |

(continued)

| No. | Structure | Nuclear magnetic resonance and mass spectrometry data |
|---|---|---|
| B348 | 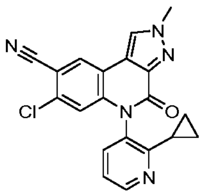 | |
| B349 | 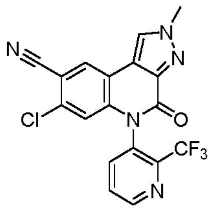 | |
| B350 | 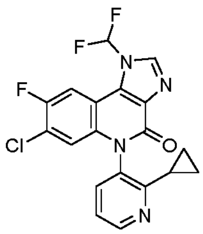 | |
| B351 | 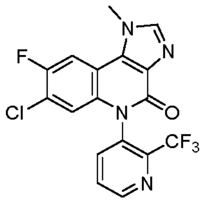 | |
| B352 | 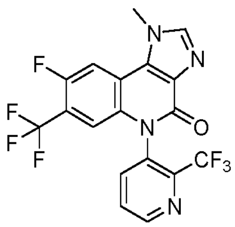 | |
| B353 | 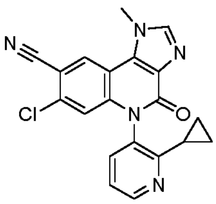 | |
| B354 | 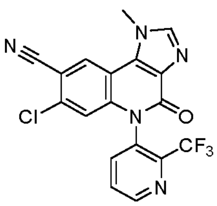 | |

(continued)

| No. | Structure | Nuclear magnetic resonance and mass spectrometry data |
|---|---|---|
| B355 | 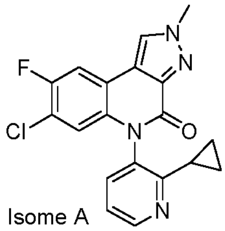 Isome A | |
| B356 | 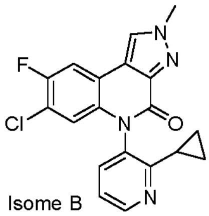 Isome B | |
| B357 | 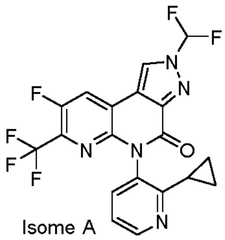 Isome A | |
| B358 | 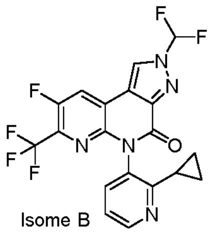 Isome B | |
| B359 | 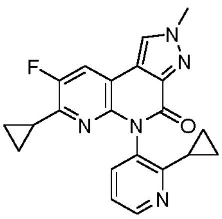 | |
| B360 | 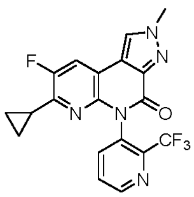 | |
| B361 | 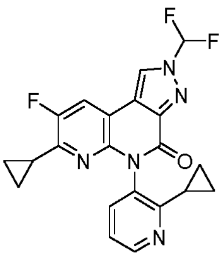 | |

(continued)

| No. | Structure | Nuclear magnetic resonance and mass spectrometry data |
|---|---|---|
| B362 | 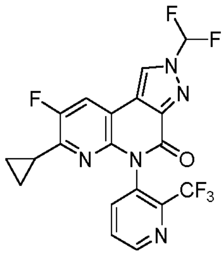 | |
| B363 | 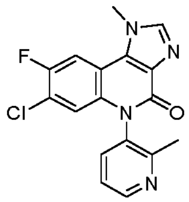 | |
| B364 | 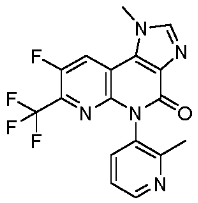 | |
| B365 | 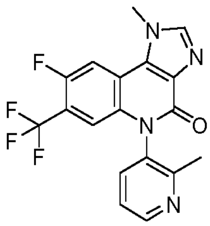 | |
| B366 | 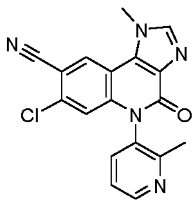 | |
| B367 | 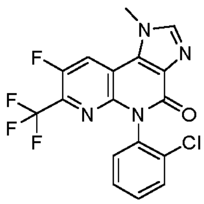 | |
| B368 | 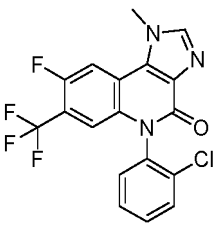 | |

(continued)

| No. | Structure | Nuclear magnetic resonance and mass spectrometry data |
| --- | --- | --- |
| B369 | 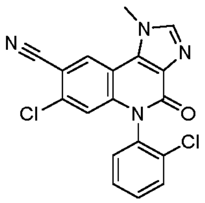 | |
| B370 | 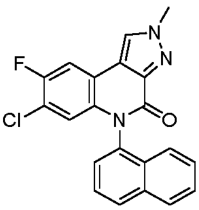 | $^1$H NMR (400 MHz, DMSO-$d_6$) δ 8.86 (s, 1H), 8.25 (dd, *J* = 14.8, 8.9 Hz, 2H), 8.18 (d, *J* = 8.2 Hz, 1H), 7.80 (s, 1H), 7.65 (dd, *J* = 14.5, 7.4 Hz, 2H), 7.49 (t, *J* = 7.6 Hz, 1H), 7.34 (d, *J* = 8.5 Hz, 1H), 6.28 (d, *J* = 6.5 Hz, 1H), 4.23 (s, 3H). LCMS $[M+H]^+$ = 378.1 |
| B371 | 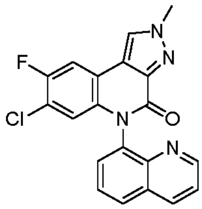 | $^1$H NMR (400 MHz, DMSO-$d_6$) δ 8.82 (s, 1H), 8.78 (dd, *J* = 4.2, 1.7 Hz, 1H), 8.60 (dd, *J* = 8.4, 1.7 Hz, 1H), 8.29 (dd, *J* = 8.2, 1.5 Hz, 1H), 8.21 (d, *J* = 9.6 Hz, 1H), 7.96 (dd, *J* = 7.3, 1.5 Hz, 1H), 7.92 - 7.86 (m, 1H), 7.65 (dd, *J* = 8.3, 4.2 Hz, 1H), 6.32 (d, *J* = 6.5 Hz, 1H), 4.22 (s, 3H). LCMS $[M+H]^+$ = 379.1 |
| B372 | 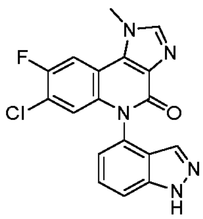 | |
| B373 | 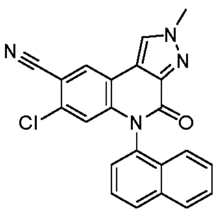 | $^1$H NMR (400 MHz, DMSO-d6) δ 8.88 (d, J = 16.8 Hz, 2H), 8.22 (dd,J = 24.4, 8.3 Hz, 2H), 7.80 (t, J = 7.8 Hz, 1H), 7.73 - 7.61 (m, 2H), 7.50 (t, J = 7.7 Hz, 1H), 7.38 (d,J = 8.4 Hz, 1H), 6.38 (s, 1H), 4.25 (s, 3H). LCMS $[M+H]^+$ = 385.1 |
| B374 | 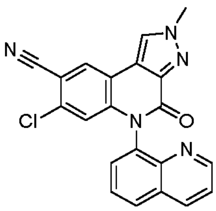 | |
| B375 | 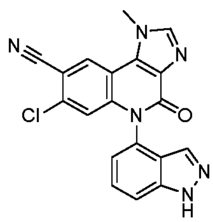 | |

(continued)

| No. | Structure | Nuclear magnetic resonance and mass spectrometry data |
|---|---|---|
| B376 | 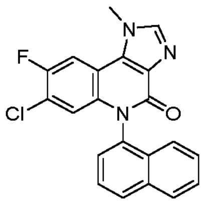 | $^1$H NMR (400 MHz, DMSO-*d*6) δ 8.35 - 8.28 (m, 2H), 8.24 (d, *J* = 8.3 Hz, 1H), 8.18 (d, *J* = 8.3 Hz, 1H), 7.81 (t, *J* = 7.8 Hz, 1H), 7.65 (t, *J* = 6.9 Hz, 2H), 7.49 (t, *J* = 7.6 Hz, 1H), 7.25 (d, *J* = 8.5 Hz, 1H), 6.44 (d, *J* =6.6 Hz, 1H), 4.31 (s, 3H). LCMS $[M+H]^+$ = 378.1 |
| B377 | 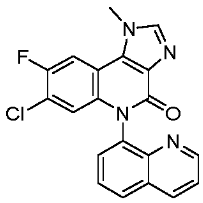 | |
| B378 | 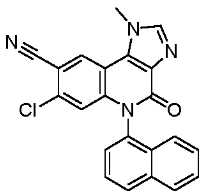 | |
| B379 | 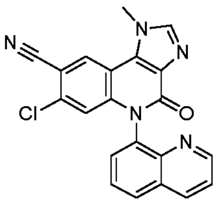 | |
| B380 | 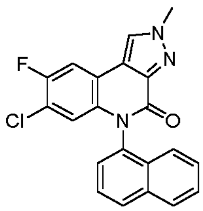 | |
| B381 | 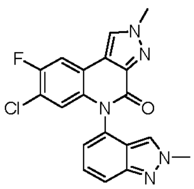 | |
| B382 | 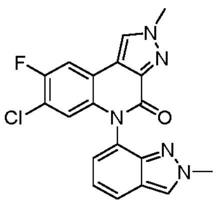 | |

(continued)

| No. | Structure | Nuclear magnetic resonance and mass spectrometry data |
|---|---|---|
| B383 | 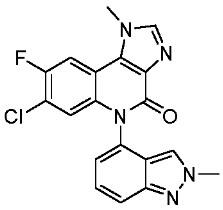 | |
| B384 | 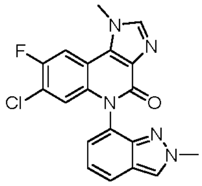 | |
| B385 | 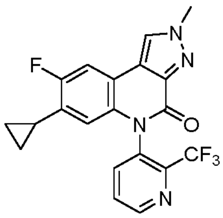 | |
| B386 | 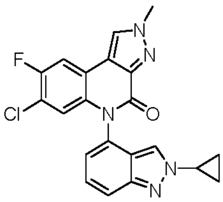 | |
| B387 | 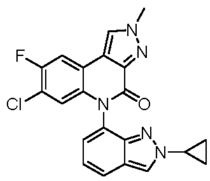 | |
| B388 | 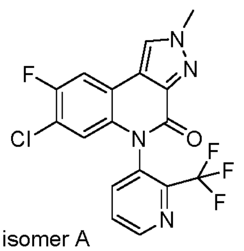 isomer A | $^1$H NMR (400 MHz, DMSO-$d_6$) δ 8.99 (dd, *J* = 4.8, 1.4 Hz, 1H), 8.79 (s, 1H), 8.24 (dd, *J* = 8.2, 1.4 Hz, 1H), 8.20 (d, *J* = 9.5 Hz, 1H), 8.05 (dd, *J* = 8.1, 4.7 Hz, 1H), 6.60 (d, *J* = 6.3 Hz, 1H), 4.18 (s, 3H). LCMS $[M+H]^+$ = 397.0 |
| B389 | 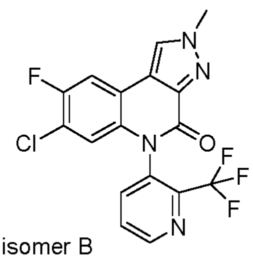 isomer B | $^1$H NMR (400 MHz, DMSO-$d_6$) δ 8.99 (dd, *J* = 4.8, 1.4 Hz, 1H), 8.79 (s, 1H), 8.24 (dd, *J* = 8.2, 1.4 Hz, 1H), 8.20 (d, *J* = 9.5 Hz, 1H), 8.05 (dd, *J* = 8.1, 4.7 Hz, 1H), 6.60 (d, *J* = 6.3 Hz, 1H), 4.18 (s, 3H). LCMS $[M+H]^+$ = 397.0 |

(continued)

| No. | Structure | Nuclear magnetic resonance and mass spectrometry data |
|---|---|---|
| B390 | 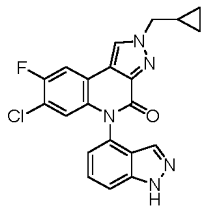 | |
| B391 | 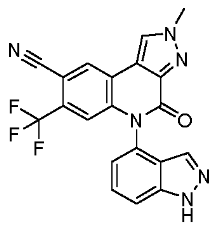 | 1H NMR (400 MHz, DMSO-*d*6) δ 13.52 (s, 1H), 9.00 (d, *J* = 14.2 Hz, 2H), 7.88 (s, 1H), 7.84 (d, *J* = 8.3 Hz, 1H), 7.64 (t, *J* = 7.8 Hz, 1H), 7.27 (d, *J* = 7.2 Hz, 1H), 6.74 (s, 1H), 4.27 (s, 3H). |
| B392 | 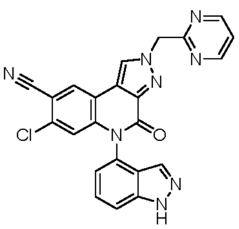 | |
| B393 | 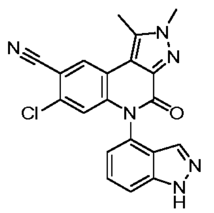 | $^1$H NMR (400 MHz, DMSO-$d_6$) δ 13.52 (s, 1H), 8.59 (s, 1H), 7.82 (d, *J* = 6.7 Hz, 2H), 7.63 (t, *J* = 7.8 Hz, 1H), 7.22 (d, *J* = 7.2 Hz, 1H), 6.51 (s, 1H), 4.14 (s, 3H), 2.91 (s, 3H). LCMS $[M+H]^+$ = 389.1 |
| B394 | 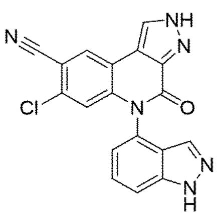 | $^1$H NMR (400 MHz, DMSO-*d*6) δ 14.69 (s, 1H), 13.52 (s, 1H), 8.94 (s, 1H), 8.80 (s, 1H), 7.90 (s, 1H), 7.83 (d, J = 8.4 Hz, 1H), 7.63 (dd, J = 8.4, 7.3 Hz, 1H), 7.25 (d, J = 7.2 Hz, 1H), 6.53 (s, 1H). LCMS $[M+H]^+$ = 361.3 |
| B395 | 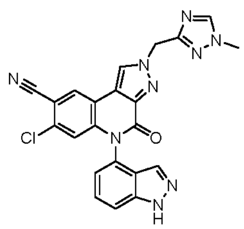 | |
| B396 | 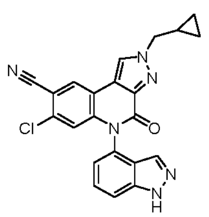 | $^1$H NMR (400 MHz, Methanol-$d_4$) δ 8.83 (s, 1H), 8.59 (s, 1H), 7.87 (d, *J* = 8.4 Hz, 1H), 7.80 (s, 1H), 7.70 (dd, *J* = 8.5, 7.2 Hz, 1H), 7.25 (d, *J* = 7.1 Hz, 1H), 6.67 (s, 1H), 4.38 (d, *J* = 7.3 Hz, 2H), 1.59 - 1.46 (m, 1H), 0.98 - 0.86 (m, 1H), 0.82 - 0.71 (m, 2H), 0.65 - 0.55 (m, 2H). LCMS $[M+H]^+$ = 415. 1 |

(continued)

| No. | Structure | Nuclear magnetic resonance and mass spectrometry data |
|---|---|---|
| B397 | 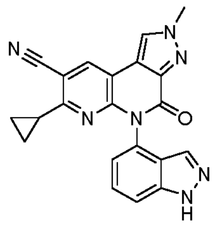 | |
| B398 | 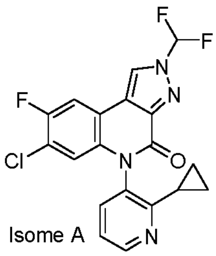 Isome A | $^1$H NMR (400 MHz, DMSO-$d_6$) $\delta$ 9.41 (s, 1H), 8.64 (dd, $J$ = 4.8, 1.6 Hz, 1H), 8.42 - 8.08 (m, 2H), 7.81 (dd, $J$ = 7.9, 1.6 Hz, 1H), 7.44 (dd, $J$ = 7.9, 4.7 Hz, 1H), 6.48 (d, $J$ = 6.4 Hz, 1H), 1.76 - 1.64 (m, 1H), 1.10 - 0.99 (m, 1H), 0.94 - 0.65 (m, 3H). LCMS $[M+H]^+$ = 405. 1 |
| B399 | 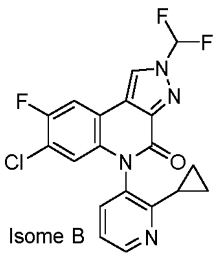 Isome B | $^1$H NMR (400 MHz, DMSO-$d_6$) $\delta$ 9.41 (s, 1H), 8.64 (dd, $J$ = 4.7, 1.6 Hz, 1H), 8.42 - 8.02 (m, 2H), 7.81 (dd, $J$ = 7.9, 1.6 Hz, 1H), 7.44 (dd, $J$ = 7.9, 4.7 Hz, 1H), 6.48 (d, $J$ = 6.4 Hz, 1H), 1.70 (tt, $J$ = 8.4, 4.6 Hz, 1H), 1.09 - 0.99 (m, 1H), 0.88 (d, $J$ = 4.8 Hz, 1H), 0.84 - 0.75 (m, 1H), 0.76 - 0.65 (m, 1H). LCMS $[M+H]^+$ = 405.1 |
| B400 | 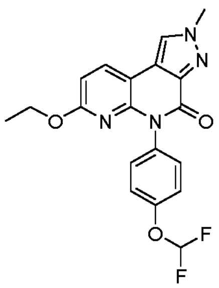 | $^1$H NMR (400 MHz, DMSO-*d6*) $\delta$ 8.66 (s, 1H), 8.31 (d, J = 8.4 Hz, 1H), 7.62 - 7.14 (m, 5H), 6.72 (d, J = 8.4 Hz, 1H), 4.18 (s, 3H), 3.88 (q, J = 7.0 Hz, 2H), 1.06 (t, J = 7.0 Hz, 3H). LCMS $[M+H]^+$ = 387.3 |
| B401 | 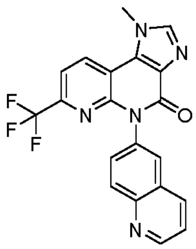 | $^1$H 1H NMR (400 MHz, DMSO-*d6*) $\delta$ 9.04 (dd, $J$ = 4.3, 1.7 Hz, 1H), 8.90 (d, $J$ = 8.2 Hz, 1H), 8.46 (dd, $J$ = 8.4, 1.7 Hz, 1H), 8.37 (s, 1H), 8.19 (d, $J$ = 8.9 Hz, 1H), 8.03 (d, $J$ = 2.3 Hz, 1H), 7.84 (d, $J$ = 8.2 Hz, 1H), 7.71 (dd, $J$ = 8.9, 2.3 Hz, 1H), 7.64 (dd, $J$ = 8.3, 4.2 Hz, 1H), 4.31 (s, 3H). LCMS $[M+H]^+$: 396.2. |
| B402 | 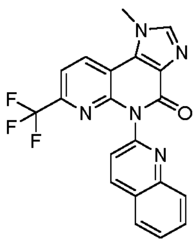 | $^1$H NMR (400 MHz, DMSO-*d*6) $\delta$ 8.92 (d, $J$ = 8.3 Hz, 1H), 8.65 (d, $J$ = 8.5 Hz, 1H), 8.40 (s, 1H), 8.24 - 8.16 (m, 1H), 8.05 (d, $J$ = 8.4 Hz, 1H), 7.92 - 7.83 (m, 2H), 7.78 (ddd, $J$ = 8.1, 6.9, 1.2 Hz,1H), 7.66 (d, $J$ = 8.5 Hz, 1H), 4.32 (s, 3H). LCMS $[M+H]^+$: 396.2. |

(continued)

| No. | Structure | Nuclear magnetic resonance and mass spectrometry data |
|---|---|---|
| B403 | 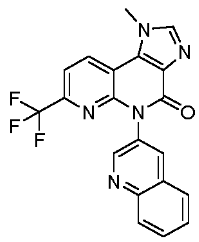 | $^1$H NMR (400 MHz, DMSO-$d_6$) $\delta$ 8.92 (d, $J$ = 8.2 Hz, 1H), 8.85 (d, $J$ = 2.4 Hz, 1H), 8.48 (d, $J$ = 2.4 Hz, 1H), 8.39 (s, 1H), 8.18 (d, $J$ = 8.4 Hz, 1H), 8.10 (d, $J$ = 8.2 Hz, 1H), 7.95 - 7.84 (m, 2H), 7.74 (t, $J$ = 7.5 Hz, 1H), 4.32 (s, 3H). LCMS $[M+H]^+$ = 396.2 |
| B404 | 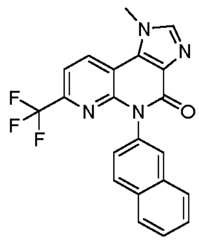 | $^1$H NMR (400 MHz, DMSO-$d6$) $\delta$8.89 (d, J = 8.2 Hz, 1H), 8.37 (s, 1H), 8.09 (t, J = 8.4 Hz, 2H), 8.02 - 7.98 (m, 1H), 7.93 (d, J = 2.0 Hz, 1H), 7.84 (d, J = 8.2 Hz, 1H), 7.63 (pd, J = 6.9, 1.5 Hz, 2H), 7.44 (dd, J = 8.6, 2.0 Hz, 1H), 4.31 (s, 3H). LCMS $[M+H]^+$: 395.2. |
| B405 | 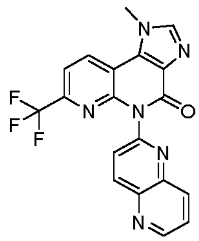 | $^1$H NMR (400 MHz, DMSO-$d6$) $\delta$ 9.17 (dd, J = 4.2, 1.6 Hz, 1H), 8.93 (d, J = 8.2 Hz, 1H), 8.72 (d, J = 8.7 Hz, 1H), 8.50 (d, J = 8.3 Hz, 1H), 8.40 (s, 1H), 7.94 (d, J = 8.7 Hz, 1H), 7.91 (dd, J = 8.5, 4.1 Hz,1H), 7.87 (d, J = 8.2 Hz, 1H), 4.33 (s, 3H). LCMS $[M+H]^+$: 397.1. |
| B406 | 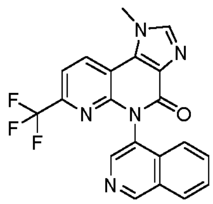 | $^1$H NMR (400 MHz, DMSO-$d_6$) $\delta$ 9.51 (d, $J$ = 0.8 Hz, 1H), 8.94 (d, $J$ = 8.2 Hz, 1H), 8.54 (s, 1H), 8.41 (s, 1H), 8.38 - 8.29 (m, 1H), 7.84 (d, $J$ = 8.2 Hz, 1H), 7.73 (dddd, $J$ = 25.2, 8.2, 6.9, 1.2 Hz, 2H), 7.44 - 7.37 (m, 1H), 4.34 (s, 3H). LCMS $[M+H]^+$: 396.1. |
| B407 | 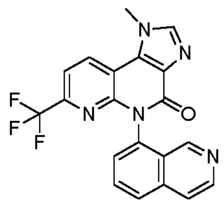 | $^1$H NMR (400 MHz, DMSO-$d6$) $\delta$ 8.94 (d, J = 8.3 Hz, 1H), 8.79 (d, J = 0.9 Hz, 1H), 8.57 (d, J = 5.7 Hz, 1H), 8.41 (s, 1H), 8.18 (d, J = 8.3 Hz, 1H), 8.04 - 7.96 (m, 2H), 7.84 (d, J = 8.2 Hz, 1H), 7.69 (dd, J = 7.3, 1.0 Hz, 1H), 4.34 (s, 3H). LCMS $[M+H]^+$: 396.2. |
| B408 | 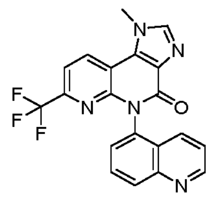 | $^1$H NMR (400 MHz, DMSO-$d_6$) $\delta$ 8.98 (dd, $J$ = 4.2, 1.6 Hz, 1H), 8.93 (d, $J$ = 8.2 Hz, 1H), 8.41 (s, 1H), 8.22 (d, $J$ = 8.5 Hz, 1H), 7.97 (dd, $J$ = 8.5, 7.4 Hz, 1H), 7.82 (dd, $J$ = 9.7, 7.8 Hz, 2H), 7.65 (dd, $J$ = 7.4, 1.1 Hz, 1H), 7.44 (dd, $J$ = 8.6, 4.1 Hz, 1H), 4.34 (s, 3H). LCMS $[M+H]^+$ = 396.2. |
| B409 | 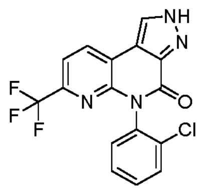 | $^1$H NMR (400 MHz, DMSO-$d_6$) $\delta$ 8.89 (s, 1H), 8.83 *(d, J*= 7.9 Hz, 1H), 7.87 (d, J= 7.9 Hz, 1H), 7.76 - 7.71 (m, 1H), 7.58 (m, 4H). LCMS $[M+H]^+$:365.1. |

(continued)

| No. | Structure | Nuclear magnetic resonance and mass spectrometry data |
|---|---|---|
| B410 | 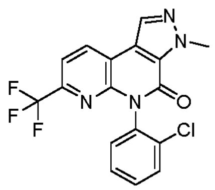 | $^1H$ NMR (400 MHz, DMSO-d6) δ 8.88 (d, *J* = 8.0 Hz, 1H), 8.72 (s, 1H), 7.89 (d, *J* = 8.0 Hz, 1H), 7.82 - 7.70 (m, 1H), 7.58 (q, *J* = 2.6, 1.8 Hz, 3H), 4.36 (s, 3H). LCMS $[M+H]^+$: 379.2. |
| B411 | 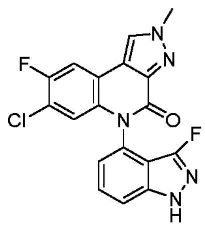 | $^1H$ NMR (400 MHz, DMSO-$d_6$) δ 13.01 (s, -1H), 8.83 (s, 1H), 8.24 (*d, J* = 9.5 Hz, 1H), 7.75 (dd, *J* = 8.2, 2.2 Hz, 1H), 7.72 - 7.69 (m, 1H), 7.35 - 7.25 (m, 1H), 6.54 (d, *J* = 6.5 Hz, 1H), 4.21 (s, 3H). LCMS m/z $[M+H]^+$ = 386.0. |
| B412 | 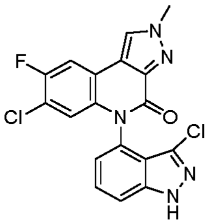 | $^1H$ NMR(400 MHz, DMSO-$d_6$) δ 13.73 (s, 1H), 8.82 (s, 1H), 8.23 (d, *J* = 9.5 Hz, 1H), 7.84 (d, *J* = 8.5 Hz, 1H), 7.70 (dd, *J* = 8.6, 7.2 Hz, 1H), 7.30 (d, *J* = 7.2 Hz, 1H), 6.41 (d, *J* = 6.4 Hz, 1H), 4.21 (s, 3H). LCMS $[M+H]^+$: 402.0. |
| B413 | 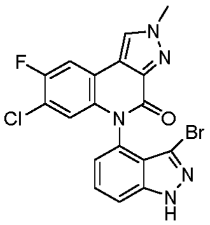 | $^1H$ NMR(400 MHz, DMSO-$d_6$) δ 13.86 (s, 1H), 8.81 (s, 1H), 8.22 (d, *J* = 9.5 Hz, 1H), 7.86 (d, *J* = 8.5 Hz, 1H), 7.69 (dd, *J* = 8.6, 7.2 Hz, 1H), 7.28 (d, *J* = 7.1 Hz, 1H), 6.36 (d, *J* = 6.5 Hz, 1H), 4.21 (s, 3H). LCMS $[M+H]^+$ = 446.0 |
| B414 | 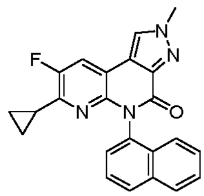 | $^1H$ NMR (400 MHz, DMSO-$d_6$) δ 8.92 (d, *J* = 33.0 Hz, 2H), 8.12 (d, *J* = 7.8 Hz, 2H), 7.70 - 7.66 (m, 2H), 7.60 (d, *J* = 7.7 Hz, 1H), 7.55 (d, *J* = 8.2 Hz, 1H), 7.50 (*d, J* = 7.2 Hz, 1H), 4.22 (s, 3H), 1.98 (s, 1H), 1.02 - 0.79 (m, 4H). LCMS $[M+H]^+$ = 385.1 |

**Experimental Example 1: Bioactivity Assay of Compound of the Present Application**

MAT2A inhibitory activity

MAT2A catalyzes the reaction of *L*-methionine and ATP to generate 5-adenosylmethionine (SAM), inorganic phosphonic acid, and inorganic diphosphonic acid. Malachite green reacts with inorganic phosphonic acid to generate a green product, and the enzyme activity of MAT2A can be indirectly reflected by detecting the content of the inorganic phosphonic acid in a sample.

The test compounds were formulated using DMSO and diluted 1:3 diluted through 10 concentration points to give a final starting concentration in the reaction system of 1000 nM. The test compounds at different concentrations described above were transferred to a 384-well plate. Experimental buffer (50 mM HEPES pH 7.5, 50 mM KCl, 10 mM $MgCl_2$, 10 mM DTT) was prepared. 2× MAT2A recombinant protein was prepared using the buffer and added to a 384-well plate. A mixed solution of 2× *L*-methionine and ATP was prepared using the buffer, and added to a 384-well plate. The enzymatic reaction was started, and the reaction was carried out at room temperature for 60 min. 20 μL of a colorimetric detection reagent was added to the above system, and incubated at room temperature for 15 min. The absorbance at 630 nm was measured. The inhibition rate of the compound on the enzyme activity was calculated, and the inhibition rate value and the logarithm value of the compound concentration were fitted using non-linear regression (dose response-variable slope) to give the $IC_{50}$

value of the test compound.

In the present application, the test compounds were tested using the above assay method, and the test compounds were scored according to the $IC_{50}$ value: (A) less than 100 nM; (B) between 100 nM and 1 μM; (C) greater than 1 μM, and (NT) $IC_{50}$ value was not tested, as shown in Table 1.

Cell symmetric dimethylarginine (SDMA) test

MAT2A catalyzes the reaction of *L*-methionine and ATP to generate SAM, inorganic phosphonic acid, and inorganic diphosphonic acid. SAM is an important methyl donor, and is a substrate of arginine methyltransferase 5 (PRMT5). PRMT5 catalyzes SDMA modification of multiple proteins within a cell. The intracellular SDMA modification level was detected by ICW (In-cell Western), and the inhibition of the test compound on the enzymatic activity of intracellular MAT2A was indirectly reflected.

In the present application, the human colon cancer cell line HCT116 and the corresponding HCT116-MTAP-knockout cell line were seeded into a 96-well cell culture plate at an appropriate cell density. After 24 h, the cells were treated using the test compounds diluted 1:4 fold at the highest concentration of 6 μM for 6 gradients, and a DMSO treatment group was set up separately. The cells were cultured in a 37 °C/5% CO2 incubator for 4 days. To test the inhibitory activity of the test compounds against tumor cell SDMA, the medium was discarded, and the plate was washed 3 times with PBST. 4% paraformaldehyde was then used at room temperature for fixation for 20 min. The fixation solution was discarded, the plate was washed 3 times with PBST, and a special blocking solution was added to each well for blocking at room temperature for 2 h. The blocking solution was discarded. After the plate was washed 3 times with PBST, the plate was incubated with SDMA antibody at 4 °C overnight. The next day, the plate was washed 3 times with PBST. The secondary antibody and DRAQ5 were diluted to an appropriate concentration with the blocking solution, and incubated at room temperature for 2 h; the plate was washed 3 times with PBST, and the fluorescence signals at 700 nm and 800 nm were detected using a Li-COR Odyssey two-color near-infrared laser imager.

The data was then processed. After the corresponding background value was subtracted from each well, statistical analysis was performed on the 800 nm/700 nm ratio for each well, a sigmoidal dose-inhibition curve was plotted using a non-linear regression model, and IC50 values were fitted and calculated. Inhibition Rate (%) = (1-SDMA dose/SDMA DMSO control) *100. SDMA dose is the value of the drug treatment group, and SDMA DMSO control is the value of the DMSO control group.

The exemplary compounds disclosed herein were tested in the above assay and determined to inhibit SDMA with $IC_{50}$ based on the following scores: (A) less than 200 nM, (B) between 200 nM and 1 μM, (C) between 1 μM and 3 μM, (D) greater than 3 μM, and (NT) not tested, as shown in Table 1 below.

Table 1. Bioactivity data for exemplary compounds of the present application

| Compound | MAT2A Inhibition (nM) | MAT2A Inhibition (nM) | Cell 96 h SDMA Inhibition (HCT116-$MTAP^{-/-}$) (nM) | Cell 96 h SDMA Inhibition (HCT116-$MTAP^{-/-}$) (nM) | Cell 96 h SDMA Inhibition (HCT116-$MTAP^{+/+}$) (nM) | Cell 96 h SDMA inhibition (HCT116-$MTAP^{+/+}$) (nM) |
|---|---|---|---|---|---|---|
| B2 | B | 104.6 | NT | NT | NT | NT |
| B3 | A | 14.2 | A | | D | 4284.5 |
| B4 | A | 38.8 | B | 781.6 | D | 9301 |
| B5 | A | 43.4 | NT | NT | NT | NT |
| B6 | A | 70.4 | D | 3570 | B | |
| B7 | B | 189.2 | NT | NT | NT | NT |
| B8 | A | 21.7 | B | 271.0 | C | 2099 |
| B9 | A | 28.6 | B | 328.0 | C | 2348 |
| B10 | C | >1000 | NT | NT | NT | NT |
| B11 | C | >1000 | NT | NT | NT | NT |
| B12 | A | 18.2 | B | 218.6 | C | 1416 |
| B13 | A | 17.2 | A | 188.2 | C | 2790.5 |
| B14 | A | 66.9 | C | | C | |

(continued)

| Compound | MAT2A Inhibition (nM) | MAT2A Inhibition (nM) | Cell 96 h SDMA Inhibition (HCT116-MTAP$^{-/-}$) (nM) | Cell 96 h SDMA Inhibition (HCT116-MTAP$^{-/-}$) (nM) | Cell 96 h SDMA Inhibition (HCT116-MTAP$^{+/+}$) (nM) | Cell 96 h SDMA inhibition (HCT116-MTAP$^{+/+}$) (nM) |
|---|---|---|---|---|---|---|
| B15 | C | >1000 | NT | NT | NT | NT |
| B16 | A | 26.8 | A | 63.6 | B | 636.3 |
| B17 | A | 24.0 | A | 51.5 | B | 635.1 |
| B18 | B | 148.6 | D | 8412 | NT | NT |
| B19 | B | 124.7 | NT | NT | NT | NT |
| B20 | A | 37.2 | B | 520.8 | D | 5149 |
| B21 | A | 89.2 | NT | NT | NT | NT |
| B22 | | | | | | |
| B23 | B | 907.9 | NT | NT | NT | NT |
| B24 | B | 115.0 | NT | NT | NT | NT |
| B25 | A | 92.8 | NT | NT | NT | NT |
| B26 | B | 349.4 | NT | NT | NT | NT |
| B27 | A | 77.0 | C | 1281.5 | D | >3000.0 |
| B28 | A | 51.6 | A | 30.5 | B | 342.3 |
| B29 | A | 18.5 | A | 90.4 | B | 598.9 |
| B30 | A | 32.7 | A | 7.6 | B | 230.0 |
| B31 | A | 65.3 | A | 168.6 | B | 970.7 |
| B32 | A | 43.5 | A | 53.9 | C | 2258 |
| B33 | A | 25.6 | C | 2646 | D | 4881 |
| B34 | NT | NT | NT | NT | NT | NT |
| B35 | A | 43.5 | NT | NT | NT | NT |
| B36 | A | 55.2 | C | 2731 | A | 24.72 |
| B37 | A | 51.7 | B | 888.5 | C | 1261.0 |
| B38 | NT | NT | NT | NT | NT | NT |
| B39 | A | 42.4 | B | 586.5 | C | 1534.5 |
| B40 | A | 22.1 | B | 375.7 | D | 4471.0 |
| B41 | A | 58.6 | NT | NT | NT | NT |
| B42 | NT | NT | NT | NT | NT | NT |
| B43 | NT | NT | NT | NT | NT | NT |
| B44 | | | | | | |
| B45 | A | 27.4 | A | 131.8 | D | 3215 |
| B46 | A | 39.7 | B | 235.9 | D | 19427 |
| B47 | A | 29.7 | A | 153.7 | D | 46997 |
| B48 | A | 29.3 | A | 63.5 | D | 5144 |
| B49 | | | | | | |
| B50 | B | 487.5 | NT | NT | NT | NT |

(continued)

| Compound | MAT2A Inhibition (nM) | MAT2A Inhibition (nM) | Cell 96 h SDMA Inhibition (HCT116-MTAP$^{-/-}$) (nM) | Cell 96 h SDMA Inhibition (HCT116-MTAP$^{-/-}$) (nM) | Cell 96 h SDMA Inhibition (HCT116-MTAP$^{+/+}$) (nM) | Cell 96 h SDMA inhibition (HCT116-MTAP$^{+/+}$) (nM) |
|---|---|---|---|---|---|---|
| B51 | A | 40.8 | D | 8518 | D | >10000 |
| B52 | | | | | | |
| B53 | | | | | | |
| B54 | | | | | | |
| B55 | A | 18.0 | A | 118 | C | 1521 |
| B56 | | | | | | |
| B57 | A | 54.3 | B | 579.2 | D | >3000 |
| B58 | A | 55.2 | B | 267.0 | C | 1186.7 |
| B59 | | | | | | |
| B60 | A | 47.5 | A | 34.9 | B | 466.9 |
| B61 | | | | | | |
| B62 | | | | | | |
| B63 | | | | | | |
| B64 | | | | | | |
| B65 | A | 34.1 | A | 11.7 | A | 191.4 |
| B66 | | | | | | |
| B67 | | | | | | |
| B68 | A | 45.1 | B | 212.6 | D | >3000 |
| B69 | A | 28.0 | A | 108.6 | C | 1474.9 |
| B70 | A | 25.2 | B | 375.4 | C | 1550.0 |
| B71 | A | 29.9 | B | 203.2 | C | 1265.6 |
| B72 | A | 18.1 | A | 76.9 | B | 479.9 |
| B73 | | | | | | |
| B74 | A | 44.3 | A | 19.3 | B | 260.9 |
| B75 | A | 43.9 | A | 14.6 | B | 298.4 |
| B76 | A | 30.0 | A | 10.0 | B | 216.0 |
| B77 | A | 43.1 | A | 32.8 | B | 395.7 |
| B78 | A | 59.8 | B | 813.0 | C | 2032 |
| B79 | | | | | | |
| B80 | A | 42.2 | B | 218.0 | D | >3000 |
| B81 | | | | | | |
| B82 | | | | | | |
| B83 | | | | | | |
| B84 | | | | | | |
| B85 | | | | | | |
| B86 | | | | | | |

(continued)

| Compound | MAT2A Inhibition (nM) | MAT2A Inhibition (nM) | Cell 96 h SDMA Inhibition (HCT116-MTAP$^{-/-}$) (nM) | Cell 96 h SDMA Inhibition (HCT116-MTAP$^{-/-}$) (nM) | Cell 96 h SDMA Inhibition (HCT116-MTAP$^{+/+}$) (nM) | Cell 96 h SDMA inhibition (HCT116-MTAP$^{+/+}$) (nM) |
|---|---|---|---|---|---|---|
| B87 | | | | | | |
| B88 | | | | | | |
| B89 | | | | | | |
| B90 | | | | | | |
| B91 | | | | | | |
| B92 | | | | | | |
| B93 | | | | | | |
| B94 | | | | | | |
| B95 | A | 48.2 | A | 4.0 | D | >3000 |
| B96 | A | 29.4 | A | 5.2 | A | 157.6 |
| B97 | | | | | | |
| B98 | | | | | | |
| B99 | | | | | | |
| B100 | | | | | | |
| B101 | A | 59.7 | A | 48.7 | D | >3000 |
| B102 | | | | | | |
| B103 | A | 54.8 | A | 27.8 | A | 53.9 |
| B104 | | | | | | |
| B105 | | | | | | |
| B106 | | | | | | |
| B107 | | | | | | |
| B108 | | | | | | |
| B109 | B | 483.0 | NT | NT | NT | NT |
| B110 | | | | | | |
| B111 | | | | | | |
| B112 | | | | | | |
| B113 | | | | | | |
| B114 | | | | | | |
| B115 | | | | | | |
| B116 | | | | | | |
| B117 | A | 41.9 | NT | NT | NT | NT |
| B118 | | | | | | |
| B119 | | | | | | |
| B120 | | | | | | |
| B121 | | | | | | |
| B122 | NT | NT | NT | NT | NT | NT |

(continued)

| Compound | MAT2A Inhibition (nM) | MAT2A Inhibition (nM) | Cell 96 h SDMA Inhibition (HCT116-MTAP$^{-/-}$) (nM) | Cell 96 h SDMA Inhibition (HCT116-MTAP$^{-/-}$) (nM) | Cell 96 h SDMA Inhibition (HCT116-MTAP$^{+/+}$) (nM) | Cell 96 h SDMA inhibition (HCT116-MTAP$^{+/+}$) (nM) |
|---|---|---|---|---|---|---|
| B123 | A | 53.7 | | | | |
| B124 | | | | | | |
| B125 | | | | | | |
| B126 | | | | | | |
| B127 | | | | | | |
| B128 | | | | | | |
| B129 | | | | | | |
| B130 | | | | | | |
| B131 | | | | | | |
| B132 | | | | | | |
| B133 | | | | | | |
| B134 | | | | | | |
| B135 | | | | | | |
| B136 | | | | | | |
| B137 | | | | | | |
| B138 | | | | | | |
| B139 | A | 37.4 | B | 774.7 | D | 6796 |
| B140 | | | | | | |
| B141 | | | | | | |
| B142 | | | | | | |
| B143 | | | | | | |
| B144 | | | | | | |
| B145 | | | | | | |
| B146 | | | | | | |
| B147 | | | | | | |
| B148 | A | 24.6 | A | 49.2 | C | 1030.0 |
| B149 | | | | | | |
| B150 | A | 27.8 | A | 54.3 | B | 861.0 |
| B151 | | | | | | |
| B152 | A | 71.8 | B | 228 | D | 17424 |
| B153 | | | | | | |
| B154 | | | | | | |
| B155 | | | | | | |
| B156 | | | | | | |
| B157 | | | | | | |
| B158 | | | | | | |

(continued)

| Compound | MAT2A Inhibition (nM) | MAT2A Inhibition (nM) | Cell 96 h SDMA Inhibition (HCT116-MTAP$^{-/-}$) (nM) | Cell 96 h SDMA Inhibition (HCT116-MTAP$^{-/-}$) (nM) | Cell 96 h SDMA Inhibition (HCT116-MTAP$^{+/+}$) (nM) | Cell 96 h SDMA inhibition (HCT116-MTAP$^{+/+}$) (nM) |
|---|---|---|---|---|---|---|
| B159 | | | | | | |
| B160 | | | | | | |
| B161 | | | | | | |
| B162 | | | | | | |
| B163 | | | | | | |
| B164 | | | | | | |
| B165 | | | | | | |
| B166 | | | | | | |
| B167 | | | | | | |
| B168 | A | 99.2 | NT | NT | NT | NT |
| B169 | A | 97.4 | NT | NT | NT | NT |
| B170 | B | 220.0 | NT | NT | NT | NT |
| B171 | A | 61.8 | B | 818.5 | D | >3000 |
| B172 | A | 68.1 | B | 771.4 | C | 2211 |
| B173 | NT | NT | NT | NT | NT | NT |
| B174 | NT | NT | NT | NT | NT | NT |
| B175 | A | 80.5 | B | 993.0 | D | 6982.5 |
| B176 | A | 24.6 | B | 263.0 | c | 1317.0 |
| B177 | A | 24.3 | B | 300.0 | B | 858.5 |
| B178 | A | 19.0 | A | 37.1 | C | 1060.0 |
| B179 | | | | | | |
| B180 | A | 14.4 | A | 10.3 | A | 99.2 |
| B181 | A | 14.7 | A | 29.0 | B | 480.2 |
| B182 | A | 56.9 | A | 176.3 | D | >3000 |
| B183 | A | 47.3 | A | 47.9 | A | 130.18 |
| B184 | B | 174.1 | NT | NT | NT | NT |
| B185 | B | 378.9 | NT | NT | NT | NT |
| B186 | B | 191.4 | NT | NT | NT | NT |
| B187 | B | 284.0 | NT | NT | NT | NT |
| B188 | A | 99.4 | NT | NT | NT | NT |
| B189 | A | 39.7 | A | 12.3 | B | 561.3 |
| B190 | B | 133.4 | NT | NT | NT | NT |
| B191 | A | 41.5 | A | 29.3 | C | 1286.1 |
| B192 | A | 26.9 | B | 255.3 | C | 2446.5 |
| B193 | A | 25.9 | B | 386.9 | C | 2113.5 |
| B194 | A | 43.7 | NT | NT | NT | NT |

(continued)

| Compound | MAT2A Inhibition (nM) | MAT2A Inhibition (nM) | Cell 96 h SDMA Inhibition (HCT116-MTAP$^{-/-}$) (nM) | Cell 96 h SDMA Inhibition (HCT116-MTAP$^{-/-}$) (nM) | Cell 96 h SDMA Inhibition (HCT116-MTAP$^{+/+}$) (nM) | Cell 96 h SDMA inhibition (HCT116-MTAP$^{+/+}$) (nM) |
|---|---|---|---|---|---|---|
| B195 | A | 20.0 | NT | NT | NT | NT |
| B196 | A | 45.1 | A | 25.3 | B | 691.0 |
| B197 | A | 90.4 | NT | NT | NT | NT |
| B198 | A | 17.1 | A | 48.5 | B | 566.7 |
| B199 | A | 53.7 | A | 184.2 | B | 899.3 |
| B200 | NT | NT | NT | NT | NT | NT |
| B201 | NT | NT | NT | NT | NT | NT |
| B202 | A | 49.9 | C | 1440.3 | D | 15321 |
| B203 | A | 51.4 | A | 66.6 | B | 633.5 |
| B204 | A | 67.4 | C | 1854.0 | C | 1189.0 |
| B205 | A | 71.1 | NT | NT | NT | NT |
| B206 | A | 93 | C | 1182.0 | C | 1286.0 |
| B207 | A | 39.6 | A | 105.7 | B | 495.4 |
| B208 | B | 178 | NT | NT | NT | NT |
| B209 | B | 122 | NT | NT | NT | NT |
| B210 | B | 603 | NT | NT | NT | NT |
| B211 | A | 35.8 | A | 39.1 | C | 1016.5 |
| B212 | | | | | | |
| B213 | A | 18.6 | A | 25.0 | B | 205.8 |
| B214 | A | 14.9 | NT | NT | NT | NT |
| B215 | A | 21.7 | B | 209.8 | C | 1182.0 |
| B216 | A | 25.3 | A | 133.6 | B | 433.3 |
| B217 | B | 118 | C | 1823.5 | D | >3000 |
| B218 | A | 77.9 | A | 39.5 | D | >3000 |
| B219 | NT | NT | NT | NT | NT | NT |
| B220 | NT | NT | NT | NT | NT | NT |
| B221 | A | 52.72 | C | 2540.0 | B | 474.6 |
| B222 | NT | NT | NT | NT | NT | NT |
| B223 | A | 77.46 | B | 691.7 | B | 582.7 |
| B224 | NT | NT | NT | NT | NT | NT |
| B225 | NT | NT | NT | NT | NT | NT |
| B226 | A | 30.9 | A | 16.5 | B | 919.1 |
| B227 | A | 51.8 | D | 4692 | D | 3031 |
| B228 | A | 37.3 | C | 2913 | D | 3219 |
| B229 | NT | NT | NT | NT | NT | NT |
| B230 | B | 236.65 | NT | NT | NT | NT |

(continued)

| Compound | MAT2A Inhibition (nM) | MAT2A Inhibition (nM) | Cell 96 h SDMA Inhibition (HCT116-MTAP$^{-/-}$) (nM) | Cell 96 h SDMA Inhibition (HCT116-MTAP$^{-/-}$) (nM) | Cell 96 h SDMA Inhibition (HCT116-MTAP$^{+/+}$) (nM) | Cell 96 h SDMA inhibition (HCT116-MTAP$^{+/+}$) (nM) |
|---|---|---|---|---|---|---|
| B231 | A | 39.85 | NT | NT | NT | NT |
| B232 | | | | | | |
| B233 | A | 13.42 | A | 6.35 | B | 249.8 |
| B234 | A | 25.48 | C | 1366 | D | >3000 |
| B235 | B | 668.40 | D | 4285 | D | >3000 |
| B236 | NT | NT | NT | NT | NT | NT |
| B237 | NT | NT | NT | NT | NT | NT |
| B238 | NT | NT | NT | NT | NT | NT |
| B239 | A | 14.7 | A | 11 | D | >10000 |
| B240 | NT | NT | NT | NT | NT | NT |
| B241 | | | | | | |
| B242 | | | | | | |
| B243 | NT | NT | NT | NT | NT | NT |
| B244 | A | 102 | NT | NT | NT | NT |
| B245 | A | 11.0 | A | 110 | D | 7200 |
| B246 | | | | | | |
| B247 | | | | | | |
| B248 | | | | | | |
| B249 | | | | | | |
| B250 | A | 28.09 | B | 110 | D | 3969 |
| B251 | A | 90.33 | NT | NT | NT | NT |
| B252 | A | 19.26 | B | 202 | NT | NT |
| B253 | B | 684.55 | NT | NT | NT | NT |
| B254 | A | 23.73 | A | 67 | NT | NT |
| B255 | A | 19.68 | B | 222 | D | 3438 |
| B256 | A | 16.9 | A | 28 | NT | NT |
| B257 | A | 39.6 | A | 70 | C | 2156 |
| B258 | A | 28 | A | 67.6 | D | >10000 |
| B259 | | | | | | |
| B260 | A | 17 | A | 57.5 | NT | NT |
| B261 | | | | | | |
| B262 | | | | | | |
| B263 | A | 9.3 | A | 3.7 | B | 687 |
| B264 | | | | | | |
| B265 | | | | | | |
| B266 | | | | | | |

(continued)

| Compound | MAT2A Inhibition (nM) | MAT2A Inhibition (nM) | Cell 96 h SDMA Inhibition (HCT116-MTAP$^{-/-}$) (nM) | Cell 96 h SDMA Inhibition (HCT116-MTAP$^{-/-}$) (nM) | Cell 96 h SDMA Inhibition (HCT116-MTAP$^{+/+}$) (nM) | Cell 96 h SDMA inhibition (HCT116-MTAP$^{+/+}$) (nM) |
|---|---|---|---|---|---|---|
| B267 | NT | NT | NT | NT | NT | NT |
| B268 | A | 81 | C | 1235 | D | >10000 |
| B269 | | | | | | |
| B270 | | | | | | |
| B271 | | | | | | |
| B272 | | | | | | |
| B273 | A | 83 | B | 517 | NT | NT |
| B274 | A | 9 | B | 147 | B | 240 |
| B275 | | | | | | |
| B276 | | | | | | |
| B277 | A | 83 | A | 61 | NT | NT |
| B278 | | | | | | |
| B279 | | | | | | |
| B280 | | | | | | |
| B281 | | | | | | |
| B282 | | | | | | |
| B283 | A | 88 | | | | |
| B284 | | | | | | |
| B285 | | | | | | |
| B286 | B | 188 | | | | |
| B287 | | | | | | |
| B288 | | | | | | |
| B289 | | | | | | |
| B290 | | | | | | |
| B291 | | | | | | |
| B292 | | | | | | |
| B293 | | | | | | |
| B294 | | | | | | |
| B295 | | | | | | |
| B296 | | | | | | |
| B297 | | | | | | |
| B298 | | | | | | |
| B299 | | | | | | |
| B300 | | | | | | |
| B301 | | | | | | |
| B302 | | | | | | |

(continued)

| Compound | MAT2A Inhibition (nM) | MAT2A Inhibition (nM) | Cell 96 h SDMA Inhibition (HCT116-MTAP$^{-/-}$) (nM) | Cell 96 h SDMA Inhibition (HCT116-MTAP$^{-/-}$) (nM) | Cell 96 h SDMA Inhibition (HCT116-MTAP$^{+/+}$) (nM) | Cell 96 h SDMA inhibition (HCT116-MTAP$^{+/+}$) (nM) |
|---|---|---|---|---|---|---|
| B303 | | | | | | |
| B304 | | | | | | |
| B305 | | | | | | |
| B306 | | | | | | |
| B307 | | | | | | |
| B308 | | | | | | |
| B309 | | | | | | |
| B310 | | | | | | |
| B311 | | | | | | |
| B312 | | | | | | |
| B313 | | | | | | |
| B314 | | | | | | |
| B315 | | | | | | |
| B316 | | | | | | |
| B317 | | | | | | |
| B318 | | | | | | |
| B319 | | | | | | |
| B320 | | | | | | |
| B321 | | | | | | |
| B322 | | | | | | |
| B323 | | | | | | |
| B324 | | | | | | |
| B325 | | | | | | |
| B326 | | | | | | |
| B327 | | | | | | |
| B328 | | | | | | |
| B329 | | | | | | |
| B330 | | | | | | |
| B331 | | | | | | |
| B332 | A | 39 | A | 9 | D | >10000 |
| B333 | A | 100 | A | 98.5 | D | >10,000 |
| B334 | | | | | | |
| B335 | | | | | | |
| B336 | A | 12 | A | 18.7 | D | >10,000 |
| B337 | A | 17 | B | 157 | D | >10,000 |
| B338 | | | | | | |

(continued)

| Compound | MAT2A Inhibition (nM) | MAT2A Inhibition (nM) | Cell 96 h SDMA Inhibition (HCT116-MTAP$^{-/-}$) (nM) | Cell 96 h SDMA Inhibition (HCT116-MTAP$^{-/-}$) (nM) | Cell 96 h SDMA Inhibition (HCT116-MTAP$^{+/+}$) (nM) | Cell 96 h SDMA inhibition (HCT116-MTAP$^{+/+}$) (nM) |
|---|---|---|---|---|---|---|
| B339 | A | 46.4 | B | 203 | D | >10,000 |
| B340 | A | 11 | NT | NT | NT | NT |
| B341 | | | | | | |
| B342 | A | 16.2 | NT | NT | NT | NT |
| B343 | | | | | | |
| B344 | | | | | | |
| B345 | | | | | | |
| B346 | | | | | | |
| B347 | | | | | | |
| B348 | B | 442 | NT | NT | NT | NT |
| B349 | | | | | | |
| B350 | | | | | | |
| B351 | A | 26 | A | 10 | D | >10,000 |
| B352 | | | | | | |
| B353 | | | | | | |
| B354 | | | | | | |
| B355 | | | | | | |
| B356 | | | | | | |
| B357 | | | | | | |
| B358 | | | | | | |
| B359 | | | | | | |
| B360 | | | | | | |
| B361 | | | | | | |
| B362 | | | | | | |
| B363 | | | | | | |
| B364 | | | | | | |
| B365 | | | | | | |
| B366 | | | | | | |
| B367 | | | | | | |
| B368 | | | | | | |
| B369 | | | | | | |
| B370 | A | 58 | B | 553.7 | D | >10,000 |
| B371 | NT | NT | NT | NT | NT | NT |
| B372 | | | | | | |
| B373 | NT | NT | NT | NT | NT | NT |
| B374 | NT | NT | NT | NT | NT | NT |

(continued)

| Compound | MAT2A Inhibition (nM) | MAT2A Inhibition (nM) | Cell 96 h SDMA Inhibition (HCT116-MTAP$^{-/-}$) (nM) | Cell 96 h SDMA Inhibition (HCT116-MTAP$^{-/-}$) (nM) | Cell 96 h SDMA Inhibition (HCT116-MTAP$^{+/+}$) (nM) | Cell 96 h SDMA inhibition (HCT116-MTAP$^{+/+}$) (nM) |
|---|---|---|---|---|---|---|
| B375 | | | | | | |
| B376 | | | | | | |
| B377 | | | | | | |
| B378 | C | >1000 | NT | NT | NT | NT |
| B379 | C | >1000 | NT | NT | NT | NT |
| B380 | | | | | | |
| B381 | NT | NT | NT | NT | NT | NT |
| B382 | NT | NT | NT | NT | NT | NT |
| B383 | | | | | | |
| B384 | | | | | | |
| B385 | | | | | | |
| B386 | | | | | | |
| B387 | | | | | | |
| B388 | C | >1000 | NT | NT | NT | NT |
| B389 | A | 13 | A | 20 | D | >10000 |
| B390 | | | | | | |
| B391 | A | 10 | | | | |
| B392 | | | | | | |
| B393 | NT | NT | NT | NT | NT | NT |
| B394 | A | 19 | A | 26 | D | >10000 |
| B395 | | | | | | |
| B396 | NT | NT | NT | NT | NT | NT |
| B397 | | | | | | |
| B398 | C | >1000 | NT | NT | NT | NT |
| B399 | A | 5.3 | A | 180 | D | >10000 |
| B400 | NT | NT | NT | NT | NT | NT |
| B401 | NT | NT | NT | NT | NT | NT |
| B402 | C | >10000 | NT | NT | NT | NT |
| B403 | C | >10000 | NT | NT | NT | NT |
| B404 | C | >10000 | NT | NT | NT | NT |
| B405 | C | >10000 | NT | NT | NT | NT |
| B406 | NT | NT | NT | NT | NT | NT |
| B407 | NT | NT | NT | NT | NT | NT |
| B408 | NT | NT | NT | NT | NT | NT |
| B409 | NT | NT | NT | NT | NT | NT |
| B410 | C | >10000 | NT | NT | NT | NT |

(continued)

| Compound | MAT2A Inhibition (nM) | MAT2A Inhibition (nM) | Cell 96 h SDMA Inhibition (HCT116-MTAP$^{-/-}$) (nM) | Cell 96 h SDMA Inhibition (HCT116-MTAP$^{-/-}$) (nM) | Cell 96 h SDMA Inhibition (HCT116-MTAP$^{+/+}$) (nM) | Cell 96 h SDMA inhibition (HCT116-MTAP$^{+/+}$) (nM) |
|---|---|---|---|---|---|---|
| B411 | | | | | | |
| B412 | A | 6.4 | | | | |
| B413 | A | 7.4 | | | | |

As can be seen from Table 1, most of the compounds had good inhibitory activity against MAT2A protein, good SDMA inhibitory activity against MATP-knockout HCT 116 cells, and a certain selectivity against MATP wild-type HCT116 cells.

Tumor cell proliferation inhibition assay

After the HCT116-MTAP-knockout (-/-) cell strain, the HCT116-MTAP wild-type (+/+) - cell strain, the DOHH-2 cell strain, or the Hut-78 cell strain was treated with the test compound for 5 days, the ATP level was measured using a CellTiter-Glo kit of Promega to evaluate the inhibition of the test compound against the growth of the tumor cell strain.

In the present application, the HCT116-MTAP-knockout (-/-) cell strain, the HCT116-MTAP wild-type (+/+) -cell strain, the DOHH-2 cell strain, or the Hut-78 cell strain was seeded into a 96-well cell culture plate at an appropriate cell density. After 24 h, the cells were treated using the test compounds diluted 1:3 fold at the highest concentration of 10 μM for 10 gradients, and a DMSO treatment group was set up separately. The cells were cultured in a 37 °C/5% CO2 incubator for 5 days. To test the proliferation inhibition of the test compound on tumor cells, the cells were equilibrated at room temperature for 10 min, followed by addition of 30 μL of cell proliferation assay reagent CellTiter-Glo(CTG) per well. The plate was shaken for 2 min, and incubated in the dark for 15 min. Chemiluminescence values were read using a Thermo Varioskan LUX-3020 multifunctional microplate reader for conversion to proliferation indices to calculate the inhibition rate of the compound on tumor cell proliferation, and the inhibition rate value and the logarithm value of the compound concentration were fitted using non-linear regression (dose response-variable slope) to give the $IC_{50}$ value of the compound.

The exemplary compounds disclosed herein were tested in the above assay and determined to inhibit cell proliferation with $IC_{50}$ based on the following scores: (A) less than 500 nM, (B) between 500 nM and 2 μM, (C) greater than or equal to 2 μM, and (NT) not tested, as shown in Table 2.

Table 2. Bioactivity data for exemplary compounds of the present application

| Compound | Tumor cell 5-day relative growth inhibition (DOHH-2) (nM) | Tumor cell 5-day relative growth inhibition (DOHH-2) (nM) | Tumor cell 5-day relative growth inhibition (HCT116-MTAP$^{-/-}$) (nM) | Tumor cell 5-day relative growth inhibition (HCT116-MTAP$^{-/-}$) (nM) | Tumor cell 5-day relative growth inhibition (HCT116-MTAP$^{+/+}$) (nM) | Tumor cell 5-day relative growth inhibition (HCT116-MTAP$^{+/+}$) (nM) | Tumor cell 5-day relative growth inhibition (Hut-78) (nM) | Tumor cell 5-day relative growth inhibition (Hut-78) (nM) |
|---|---|---|---|---|---|---|---|---|
| B2 | NT | NT | C | 2826.0 | NT | NT | NT | NT |
| B3 | B | 939.9 | A | 228.0 | NT | NT | NT | NT |
| B4 | C | | NT | NT | NT | NT | NT | NT |
| B5 | NT | NT | NT | NT | NT | NT | NT | NT |
| B6 | C | 16920 | NT | NT | NT | NT | NT | NT |
| B7 | NT | NT | NT | NT | NT | NT | NT | NT |
| B8 | B | 1002.3 | NT | NT | NT | NT | NT | NT |
| B9 | B | 1093.0 | NT | NT | NT | NT | NT | NT |

(continued)

| Compound | Tumor cell 5-day relative growth inhibition (DOHH-2) (nM) | Tumor cell 5-day relative growth inhibition (DOHH-2) (nM) | Tumor cell 5-day relative growth inhibition (HCT116-MTAP$^{-/-}$) (nM) | Tumor cell 5-day relative growth inhibition (HCT116-MTAP$^{-/-}$) (nM) | Tumor cell 5-day relative growth inhibition (HCT116-MTAP$^{+/+}$) (nM) | Tumor cell 5-day relative growth inhibition (HCT116-MTAP$^{+/+}$) (nM) | Tumor cell 5-day relative growth inhibition (Hut-78) (nM) | Tumor cell 5-day relative growth inhibition (Hut-78) (nM) |
|---|---|---|---|---|---|---|---|---|
| B10 | NT | NT | NT | NT | NT | NT | NT | NT |
| B11 | NT | NT | NT | NT | NT | NT | NT | NT |
| B12 | B | 1110.0 | A | 383.4 | B | 1654.0 | A | 144.8 |
| B13 | B | 978.8 | A | 281.5 | B | 927.8 | NT | NT |
| B14 | C | 6682.5 | NT | NT | NT | NT | NT | NT |
| B15 | NT | NT | NT | NT | NT | NT | NT | NT |
| B16 | B | 811.1 | NT | NT | NT | NT | NT | NT |
| B17 | A | 396.1 | NT | NT | NT | NT | NT | NT |
| B18 | C | >2000 | NT | NT | NT | NT | NT | NT |
| B19 | NT | NT | NT | NT | NT | NT | NT | NT |
| B20 | C | 2358.5 | NT | NT | NT | NT | NT | NT |
| B21 | NT | NT | NT | NT | NT | NT | NT | NT |
| B22 | NT | NT | NT | NT | NT | NT | NT | NT |
| B23 | NT | NT | NT | NT | NT | NT | NT | NT |
| B24 | NT | NT | NT | NT | NT | NT | NT | NT |
| B25 | NT | NT | NT | NT | NT | NT | NT | NT |
| B26 | NT | NT | NT | NT | NT | NT | NT | NT |
| B27 | C | 11730 | NT | NT | NT | NT | NT | NT |
| B28 | A | 444.2 | A | 75.0 | A | 278.7 | NT | NT |
| B29 | B | 719.4 | A | 154.2 | A | 436.6 | NT | NT |
| B30 | A | 400.3 | NT | NT | NT | NT | A | 33.1 |
| B31 | C | 8078.5 | NT | NT | NT | NT | NT | NT |
| B32 | C | 2702.5 | NT | NT | NT | NT | NT | NT |
| B33 | NT | NT | NT | NT | C | >2000 | B | 1096.0 |
| B34 | NT | NT | NT | NT | NT | NT | NT | NT |
| B35 | NT | NT | NT | NT | NT | NT | NT | NT |
| B36 | NT | NT | NT | NT | C | >2000 | C | >2000 |
| B37 | NT | NT | NT | NT | C | >2000 | C | 6432.5 |
| B38 | NT | NT | NT | NT | NT | NT | NT | NT |
| B39 | C | 3792.5 | NT | NT | NT | NT | A | 98.6 |
| B40 | C | 2402.5 | NT | NT | NT | NT | B | 1292 |
| B41 | NT | NT | NT | NT | NT | NT | NT | NT |
| B42 | NT | NT | NT | NT | NT | NT | NT | NT |
| B43 | NT | NT | NT | NT | NT | NT | NT | NT |

(continued)

| Compound | Tumor cell 5-day relative growth inhibition (DOHH-2) (nM) | Tumor cell 5-day relative growth inhibition (DOHH-2) (nM) | Tumor cell 5-day relative growth inhibition (HCT116-MTAP$^{-/-}$) (nM) | Tumor cell 5-day relative growth inhibition (HCT116-MTAP$^{-/-}$) (nM) | Tumor cell 5-day relative growth inhibition (HCT116-MTAP$^{+/+}$) (nM) | Tumor cell 5-day relative growth inhibition (HCT116-MTAP$^{+/+}$) (nM) | Tumor cell 5-day relative growth inhibition (Hut-78) (nM) | Tumor cell 5-day relative growth inhibition (Hut-78) (nM) |
|---|---|---|---|---|---|---|---|---|
| B44 | | | | | | | | |
| B45 | C | 2022.0 | NT | NT | NT | NT | NT | NT |
| B46 | C | 6030.0 | NT | NT | NT | NT | NT | NT |
| B47 | B | 1670.0 | NT | NT | NT | NT | A | 200 |
| B48 | B | 880.0 | A | 119.2 | B | 1733 | A | 81.8 |
| B49 | | | | | | | | |
| B50 | NT | NT | NT | NT | NT | NT | NT | NT |
| B51 | C | 7693 | NT | NT | NT | NT | NT | NT |
| B52 | | | | | | | | |
| B53 | | | | | | | | |
| B54 | | | | | | | | |
| B55 | NT | NT | A | 104.5 | C | 3570 | A | 83.01 |
| B56 | | | | | | | | |
| B57 | C | 2039.5 | NT | NT | NT | NT | C | 2127 |
| B58 | NT | NT | NT | NT | NT | NT | A | 205.8 |
| B59 | | | | | | | | |
| B60 | A | 39.1 | A | 12.6 | B | 1005 | A | 37.1 |
| B61 | | | | | | | | |
| B62 | | | | | | | | |
| B63 | | | | | | | | |
| B64 | | | | | | | | |
| B65 | A | 62.9 | NT | NT | NT | NT | A | 23.8 |
| B66 | | | | | | | | |
| B67 | | | | | | | | |
| B68 | NT | NT | A | 41.4 | C | >2000 | | |
| B69 | B | 1150 | | | | | | |
| B70 | B | 1930 | | | | | | |
| B71 | B | 1290 | | | | | | |
| B72 | B | 640 | A | 137.5 | B | 1684 | | |
| B73 | | | | | | | | |
| B74 | A | 340 | | | | | | |
| B75 | NT | NT | NT | NT | NT | NT | NT | NT |
| B76 | A | 82.0 | NT | NT | NT | NT | A | 10.59 |
| B77 | A | 390 | NT | NT | NT | NT | A | 87.8 |

(continued)

| Compound | Tumor cell 5-day relative growth inhibition (DOHH-2) (nM) | Tumor cell 5-day relative growth inhibition (DOHH-2) (nM) | Tumor cell 5-day relative growth inhibition (HCT116-MTAP$^{-/-}$) (nM) | Tumor cell 5-day relative growth inhibition (HCT116-MTAP$^{-/-}$) (nM) | Tumor cell 5-day relative growth inhibition (HCT116-MTAP$^{+/+}$) (nM) | Tumor cell 5-day relative growth inhibition (HCT116-MTAP$^{+/+}$) (nM) | Tumor cell 5-day relative growth inhibition (Hut-78) (nM) | Tumor cell 5-day relative growth inhibition (Hut-78) (nM) |
|---|---|---|---|---|---|---|---|---|
| B78 | | | | | | | | |
| B79 | | | | | | | | |
| B80 | NT | NT | A | 81.74 | C | 23255 | NT | NT |
| B81 | | | | | | | | |
| B82 | | | | | | | | |
| B83 | | | | | | | | |
| B84 | | | | | | | | |
| B85 | | | | | | | | |
| B86 | | | | | | | | |
| B87 | | | | | | | | |
| B88 | | | | | | | | |
| B89 | | | | | | | | |
| B90 | | | | | | | | |
| B91 | | | | | | | | |
| B92 | | | | | | | | |
| B93 | | | | | | | | |
| B94 | | | | | | | | |
| B95 | NT | NT | NT | NT | B | 621.2 | A | 18.8 |
| B96 | NT | NT | A | 5.141 | A | 292.7 | A | 27.2 |
| B97 | | | | | | | | |
| B98 | | | | | | | | |
| B99 | | | | | | | | |
| B100 | | | | | | | | |
| B101 | NT | NT | NT | NT | C | >2000 | A | 50.35 |
| B102 | | | | | | | | |
| B103 | NT | NT | NT | NT | NT | NT | A | 14.6 |
| B104 | | | | | | | | |
| B105 | | | | | | | | |
| B106 | | | | | | | | |
| B107 | | | | | | | | |
| B108 | | | | | | | | |
| B109 | NT | NT | NT | NT | NT | NT | NT | NT |
| B110 | | | | | | | | |
| B111 | | | | | | | | |

(continued)

| Compound | Tumor cell 5-day relative growth inhibition (DOHH-2) (nM) | Tumor cell 5-day relative growth inhibition (DOHH-2) (nM) | Tumor cell 5-day relative growth inhibition (HCT116-MTAP$^{-/-}$) (nM) | Tumor cell 5-day relative growth inhibition (HCT116-MTAP$^{-/-}$) (nM) | Tumor cell 5-day relative growth inhibition (HCT116-MTAP$^{+/+}$) (nM) | Tumor cell 5-day relative growth inhibition (HCT116-MTAP$^{+/+}$) (nM) | Tumor cell 5-day relative growth inhibition (Hut-78) (nM) | Tumor cell 5-day relative growth inhibition (Hut-78) (nM) |
|---|---|---|---|---|---|---|---|---|
| B112 | | | | | | | | |
| B113 | | | | | | | | |
| B114 | | | | | | | | |
| B115 | | | | | | | | |
| B116 | | | | | | | | |
| B117 | NT | NT | NT | NT | NT | NT | NT | NT |
| B118 | | | | | | | | |
| B119 | | | | | | | | |
| B120 | | | | | | | | |
| B121 | | | | | | | | |
| B122 | NT | NT | NT | NT | NT | NT | NT | NT |
| B123 | NT | NT | NT | NT | NT | NT | C | >2000 |
| B124 | | | | | | | | |
| B125 | | | | | | | | |
| B126 | | | | | | | | |
| B127 | | | | | | | | |
| B128 | | | | | | | | |
| B129 | | | | | | | | |
| B130 | | | | | | | | |
| B131 | | | | | | | | |
| B132 | | | | | | | | |
| B133 | | | | | | | | |
| B134 | | | | | | | | |
| B135 | | | | | | | | |
| B136 | | | | | | | | |
| B137 | | | | | | | | |
| B138 | | | | | | | | |
| B139 | | | | | | | | |
| B140 | | | | | | | | |
| B141 | | | | | | | | |
| B142 | | | | | | | | |
| B143 | | | | | | | | |
| B144 | | | | | | | | |
| B145 | | | | | | | | |

(continued)

| Compound | Tumor cell 5-day relative growth inhibition (DOHH-2) (nM) | Tumor cell 5-day relative growth inhibition (DOHH-2) (nM) | Tumor cell 5-day relative growth inhibition (HCT116-MTAP$^{-/-}$) (nM) | Tumor cell 5-day relative growth inhibition (HCT116-MTAP$^{-/-}$) (nM) | Tumor cell 5-day relative growth inhibition (HCT116-MTAP$^{+/+}$) (nM) | Tumor cell 5-day relative growth inhibition (HCT116-MTAP$^{+/+}$) (nM) | Tumor cell 5-day relative growth inhibition (Hut-78) (nM) | Tumor cell 5-day relative growth inhibition (Hut-78) (nM) |
|---|---|---|---|---|---|---|---|---|
| B146 | | | | | | | | |
| B147 | | | | | | | | |
| B148 | A | 443.0 | NT | NT | NT | NT | A | 135.2 |
| B149 | | | | | | | | |
| B150 | B | 653.9 | NT | NT | NT | NT | A | 139.6 |
| B151 | | | | | | | | |
| B152 | | | | | | | | |
| B153 | | | | | | | | |
| B154 | | | | | | | | |
| B155 | | | | | | | | |
| B156 | | | | | | | | |
| B157 | | | | | | | | |
| B158 | | | | | | | | |
| B159 | | | | | | | | |
| B160 | | | | | | | | |
| B161 | | | | | | | | |
| B162 | | | | | | | | |
| B163 | | | | | | | | |
| B164 | | | | | | | | |
| B165 | | | | | | | | |
| B166 | | | | | | | | |
| B167 | NT | NT | NT | NT | NT | NT | NT | NT |
| B168 | NT | NT | NT | NT | NT | NT | NT | NT |
| B169 | NT | NT | NT | NT | NT | NT | NT | NT |
| B170 | NT | NT | NT | NT | NT | NT | NT | NT |
| B171 | | | | | | | | |
| B172 | | | | | | | | |
| B173 | NT | NT | NT | NT | NT | NT | NT | NT |
| B174 | NT | NT | NT | NT | NT | NT | NT | NT |
| B175 | NT | NT | NT | NT | NT | NT | C | 2045 |
| B176 | NT | NT | NT | NT | NT | NT | B | 984.9 |
| B177 | NT | NT | NT | NT | NT | NT | B | 866.3 |
| B178 | NT | NT | A | 59.14 | C | 2000 | A | 184.1 |
| B179 | NT | NT | NT | NT | NT | NT | C | 7989 |

(continued)

| Compound | Tumor cell 5-day relative growth inhibition (DOHH-2) (nM) | Tumor cell 5-day relative growth inhibition (DOHH-2) (nM) | Tumor cell 5-day relative growth inhibition (HCT116-MTAP$^{-/-}$) (nM) | Tumor cell 5-day relative growth inhibition (HCT116-MTAP$^{-/-}$) (nM) | Tumor cell 5-day relative growth inhibition (HCT116-MTAP$^{+/+}$) (nM) | Tumor cell 5-day relative growth inhibition (HCT116-MTAP$^{+/+}$) (nM) | Tumor cell 5-day relative growth inhibition (Hut-78) (nM) | Tumor cell 5-day relative growth inhibition (Hut-78) (nM) |
|---|---|---|---|---|---|---|---|---|
| B180 | A | 134.4 | A | 9.597 | A | 191.2 | A | 17.4 |
| B181 | B | 1039 | A | 14.58 | B | 1340 | A | 36.5 |
| B182 | NT | NT | B | 1859 | C | 7337 | B | 1217.4 |
| B183 | NT | NT | NT | NT | NT | NT | A | 107.98 |
| B184 | NT | NT | NT | NT | NT | NT | NT | NT |
| B185 | NT | NT | NT | NT | NT | NT | NT | NT |
| B186 | NT | NT | NT | NT | NT | NT | NT | NT |
| B187 | NT | NT | NT | NT | NT | NT | NT | NT |
| B188 | NT | NT | C | >10000 | C | >10000 | NT | NT |
| B189 | NT | NT | A | 13.58 | A | 488.1 | A | 49.18 |
| B190 | NT | NT | NT | NT | NT | NT | NT | NT |
| B191 | NT | NT | A | 35.6 | B | 942.2 | A | 95.63 |
| B192 | NT | NT | NT | NT | NT | NT | A | 377.7 |
| B193 | NT | NT | NT | NT | NT | NT | B | 695.6 |
| B194 | NT | NT | NT | NT | NT | NT | NT | NT |
| B195 | NT | NT | NT | NT | NT | NT | NT | NT |
| B196 | NT | NT | NT | NT | NT | NT | A | 9.3 |
| B197 | NT | NT | NT | NT | NT | NT | NT | NT |
| B198 | NT | NT | A | 31.53 | B | 745 | A | 40.2 |
| B199 | NT | NT | A | 180.7 | C | 53752 | A | 323.0 |
| B200 | NT | NT | NT | NT | NT | NT | NT | NT |
| B201 | NT | NT | NT | NT | NT | NT | NT | NT |
| B202 | NT | NT | NT | NT | NT | NT | NT | NT |
| B203 | NT | NT | NT | NT | NT | NT | A | 40.4 |
| B204 | NT | NT | NT | NT | NT | NT | A | 312 |
| B205 | NT | NT | NT | NT | NT | NT | NT | NT |
| B206 | NT | NT | NT | NT | NT | NT | C | >2000 |
| B207 | NT | NT | NT | NT | NT | NT | A | 179.3 |
| B208 | NT | NT | NT | NT | NT | NT | NT | NT |
| B209 | NT | NT | NT | NT | NT | NT | NT | NT |
| B210 | NT | NT | NT | NT | NT | NT | NT | NT |
| B211 | NT | NT | NT | NT | B | 1147.3 | A | 47.7 |
| B212 | NT | NT | NT | NT | NT | NT | NT | NT |
| B213 | NT | NT | NT | NT | B | 1739.2 | A | 21.8 |

(continued)

| Compound | Tumor cell 5-day relative growth inhibition (DOHH-2) (nM) | Tumor cell 5-day relative growth inhibition (DOHH-2) (nM) | Tumor cell 5-day relative growth inhibition (HCT116-MTAP$^{-/-}$) (nM) | Tumor cell 5-day relative growth inhibition (HCT116-MTAP$^{-/-}$) (nM) | Tumor cell 5-day relative growth inhibition (HCT116-MTAP$^{+/+}$) (nM) | Tumor cell 5-day relative growth inhibition (HCT116-MTAP$^{+/+}$) (nM) | Tumor cell 5-day relative growth inhibition (Hut-78) (nM) | Tumor cell 5-day relative growth inhibition (Hut-78) (nM) |
|---|---|---|---|---|---|---|---|---|
| B214 | NT | NT | NT | NT | NT | NT | NT | NT |
| B215 | NT | NT | A | 104 | C | 4195.0 | A | 309.7 |
| B216 | NT | NT | NT | NT | C | 3587 | A | 245.0 |
| B217 | NT | NT | NT | NT | C | >2000 | C | 5434 |
| B218 | NT | NT | NT | NT | C | 3990.3 | A | 130.40 |
| B219 | NT | NT | NT | NT | NT | NT | NT | NT |
| B220 | NT | NT | NT | NT | NT | NT | NT | NT |
| B221 | NT | NT | NT | NT | C | >2000 | C | 10606 |
| B222 | NT | NT | NT | NT | NT | NT | NT | NT |
| B223 | NT | NT | NT | NT | C | >2000 | B | 1355 |
| B224 | NT | NT | NT | NT | NT | NT | NT | NT |
| B225 | NT | NT | NT | NT | NT | NT | NT | NT |
| B226 | NT | NT | NT | NT | B | 1027.3 | A | 27.7 |
| B227 | NT | NT | NT | NT | C | >2000 | C | 4709.1 |
| B228 | NT | NT | NT | NT | C | >2000 | C | 3451.2 |
| B229 | NT | NT | NT | NT | NT | NT | NT | NT |
| B230 | NT | NT | NT | NT | NT | NT | NT | NT |
| B231 | NT | NT | NT | NT | NT | NT | NT | NT |
| B232 | NT | NT | NT | NT | NT | NT | NT | NT |
| B233 | NT | NT | A | 15.52 | A | 134.81 | A | 8.05 |
| B234 | | | | | | | | |
| B235 | | | | | | | | |
| B236 | NT | NT | NT | NT | NT | NT | NT | NT |
| B237 | NT | NT | NT | NT | NT | NT | NT | NT |
| B238 | NT | NT | NT | NT | NT | NT | NT | NT |
| B239 | NT | NT | A | 50 | A | 477 | NT | NT |
| B240 | NT | NT | NT | NT | NT | NT | NT | NT |
| B241 | NT | NT | NT | NT | NT | NT | NT | NT |
| B242 | NT | NT | NT | NT | NT | NT | NT | NT |
| B243 | NT | NT | NT | NT | NT | NT | NT | NT |
| B244 | NT | NT | NT | NT | NT | NT | NT | NT |
| B245 | NT | NT | A | 242.12 | B | 1100.93 | NT | NT |
| B246 | | | | | | | | |
| B247 | | | | | | | | |

(continued)

| Compound | Tumor cell 5-day relative growth inhibition (DOHH-2) (nM) | Tumor cell 5-day relative growth inhibition (DOHH-2) (nM) | Tumor cell 5-day relative growth inhibition (HCT116-MTAP$^{-/-}$) (nM) | Tumor cell 5-day relative growth inhibition (HCT116-MTAP$^{-/-}$) (nM) | Tumor cell 5-day relative growth inhibition (HCT116-MTAP$^{+/+}$) (nM) | Tumor cell 5-day relative growth inhibition (HCT116-MTAP$^{+/+}$) (nM) | Tumor cell 5-day relative growth inhibition (Hut-78) (nM) | Tumor cell 5-day relative growth inhibition (Hut-78) (nM) |
|---|---|---|---|---|---|---|---|---|
| B248 | | | | | | | | |
| B249 | | | | | | | | |
| B250 | NT | NT | A | 299.48 | B | 938.75 | NT | NT |
| B251 | NT | NT | NT | NT | NT | NT | NT | NT |
| B252 | NT | NT | A | 378.50 | B | 1234.50 | NT | NT |
| B253 | NT | NT | NT | NT | NT | NT | NT | NT |
| B254 | NT | NT | A | 381.50 | B | 1633.50 | NT | NT |
| B255 | NT | NT | B | 1165 | C | 12823 | NT | NT |
| B256 | NT | NT | A | 29.46 | B | 577.25 | NT | NT |
| B257 | NT | NT | A | 27.46 | B | 463.95 | NT | NT |
| B258 | NT | NT | A | 235 | C | >10000 | NT | NT |
| B259 | | | | | | | | |
| B260 | NT | NT | A | 69.7 | NT | NT | NT | NT |
| B261 | | | | | | | | |
| B262 | | | | | | | | |
| B263 | | | A | 21 | A | 173 | | |
| B264 | | | | | | | | |
| B265 | | | | | | | | |
| B266 | | | | | | | | |
| B267 | NT | NT | NT | NT | NT | NT | NT | NT |
| B268 | NT | NT | C | 3646 | C | 5245 | NT | NT |
| B269 | | | | | | | | |
| B270 | | | | | | | | |
| B271 | | | | | | | | |
| B272 | | | | | | | | |
| B273 | | | B | 1400 | C | 7600 | | |
| B274 | NT | NT | A | 72.5 | C | >10,000 | NT | NT |
| B275 | | | | | | | | |
| B276 | | | | | | | | |
| B277 | NT | NT | A | 326.90 | B | 875.00 | NT | NT |
| B278 | | | | | | | | |
| B279 | | | | | | | | |
| B280 | | | | | | | | |
| B281 | | | | | | | | |

(continued)

| Compound | Tumor cell 5-day relative growth inhibition (DOHH-2) (nM) | Tumor cell 5-day relative growth inhibition (DOHH-2) (nM) | Tumor cell 5-day relative growth inhibition (HCT116-MTAP$^{-/-}$) (nM) | Tumor cell 5-day relative growth inhibition (HCT116-MTAP$^{-/-}$) (nM) | Tumor cell 5-day relative growth inhibition (HCT116-MTAP$^{+/+}$) (nM) | Tumor cell 5-day relative growth inhibition (HCT116-MTAP$^{+/+}$) (nM) | Tumor cell 5-day relative growth inhibition (Hut-78) (nM) | Tumor cell 5-day relative growth inhibition (Hut-78) (nM) |
|---|---|---|---|---|---|---|---|---|
| B282 | | | | | | | | |
| B283 | | | | | | | | |
| B284 | | | | | | | | |
| B285 | | | | | | | | |
| B286 | | | | | | | | |
| B287 | | | | | | | | |
| B288 | | | | | | | | |
| B289 | | | | | | | | |
| B290 | | | | | | | | |
| B291 | | | | | | | | |
| B292 | | | | | | | | |
| B293 | | | | | | | | |
| B294 | | | | | | | | |
| B295 | | | | | | | | |
| B296 | | | | | | | | |
| B297 | | | | | | | | |
| B298 | | | | | | | | |
| B299 | | | | | | | | |
| B300 | | | | | | | | |
| B301 | | | | | | | | |
| B302 | | | | | | | | |
| B303 | | | | | | | | |
| B304 | | | | | | | | |
| B305 | | | | | | | | |
| B306 | | | | | | | | |
| B307 | | | | | | | | |
| B308 | | | | | | | | |
| B309 | | | | | | | | |
| B310 | | | | | | | | |
| B311 | | | | | | | | |
| B312 | | | | | | | | |
| B313 | | | | | | | | |
| B314 | | | | | | | | |
| B315 | | | | | | | | |

(continued)

| Compound | Tumor cell 5-day relative growth inhibition (DOHH-2) (nM) | Tumor cell 5-day relative growth inhibition (DOHH-2) (nM) | Tumor cell 5-day relative growth inhibition (HCT116-MTAP$^{-/-}$) (nM) | Tumor cell 5-day relative growth inhibition (HCT116-MTAP$^{-/-}$) (nM) | Tumor cell 5-day relative growth inhibition (HCT116-MTAP$^{+/+}$) (nM) | Tumor cell 5-day relative growth inhibition (HCT116-MTAP$^{+/+}$) (nM) | Tumor cell 5-day relative growth inhibition (Hut-78) (nM) | Tumor cell 5-day relative growth inhibition (Hut-78) (nM) |
|---|---|---|---|---|---|---|---|---|
| B316 | | | | | | | | |
| B317 | | | | | | | | |
| B318 | | | | | | | | |
| B319 | | | | | | | | |
| B320 | | | | | | | | |
| B321 | | | | | | | | |
| B322 | | | | | | | | |
| B323 | | | | | | | | |
| B324 | | | | | | | | |
| B325 | | | | | | | | |
| B326 | | | | | | | | |
| B327 | | | | | | | | |
| B328 | | | | | | | | |
| B329 | | | | | | | | |
| B330 | | | | | | | | |
| B331 | | | | | | | | |
| B332 | | | | | | | | |
| B333 | NT | NT | A | 284 | B | 550 | NT | NT |
| B334 | | | | | | | | |
| B335 | | | | | | | | |
| B336 | NT | NT | A | 145 | C | 9700 | NT | NT |
| B337 | NT | NT | B | 579 | C | >10000 | NT | NT |
| B338 | | | | | | | | |
| B339 | | | | | | | | |
| B340 | NT | NT | NT | NT | NT | NT | NT | NT |
| B341 | | | | | | | | |
| B342 | NT | NT | A | 355 | C | >10000 | NT | NT |
| B343 | | | | | | | | |
| B344 | | | | | | | | |
| B345 | | | | | | | | |
| B346 | | | | | | | | |
| B347 | | | | | | | | |
| B348 | NT | NT | C | >10000 | C | 10000 | NT | NT |
| B349 | | | | | | | | |

(continued)

| Compound | Tumor cell 5-day relative growth inhibition (DOHH-2) (nM) | Tumor cell 5-day relative growth inhibition (DOHH-2) (nM) | Tumor cell 5-day relative growth inhibition (HCT116-MTAP$^{-/-}$) (nM) | Tumor cell 5-day relative growth inhibition (HCT116-MTAP$^{-/-}$) (nM) | Tumor cell 5-day relative growth inhibition (HCT116-MTAP$^{+/+}$) (nM) | Tumor cell 5-day relative growth inhibition (HCT116-MTAP$^{+/+}$) (nM) | Tumor cell 5-day relative growth inhibition (Hut-78) (nM) | Tumor cell 5-day relative growth inhibition (Hut-78) (nM) |
|---|---|---|---|---|---|---|---|---|
| B350 | | | | | | | | |
| B351 | NT | NT | B | 765 | C | >10000 | NT | NT |
| B352 | | | | | | | | |
| B353 | | | | | | | | |
| B354 | | | | | | | | |
| B355 | | | | | | | | |
| B356 | | | | | | | | |
| B357 | | | | | | | | |
| B358 | | | | | | | | |
| B359 | | | | | | | | |
| B360 | | | | | | | | |
| B361 | | | | | | | | |
| B362 | | | | | | | | |
| B363 | | | | | | | | |
| B364 | | | | | | | | |
| B365 | | | | | | | | |
| B366 | | | | | | | | |
| B367 | | | | | | | | |
| B368 | | | | | | | | |
| B369 | | | | | | | | |
| B370 | NT | NT | C | >10000 | C | >10000 | NT | NT |
| B371 | NT | NT | NT | NT | NT | NT | NT | NT |
| B372 | | | | | | | | |
| B373 | NT | NT | NT | NT | NT | NT | NT | NT |
| B374 | NT | NT | NT | NT | NT | NT | NT | NT |
| B375 | NT | NT | C | >10000 | C | >10000 | NT | NT |
| B376 | | | | | | | | |
| B377 | | | | | | | | |
| B378 | NT | NT | NT | NT | NT | NT | NT | NT |
| B379 | NT | NT | NT | NT | NT | NT | NT | NT |
| B380 | | | | | | | | |
| B381 | NT | NT | NT | NT | NT | NT | NT | NT |
| B382 | NT | NT | NT | NT | NT | NT | NT | NT |
| B383 | | | | | | | | |

(continued)

| Compound | Tumor cell 5-day relative growth inhibition (DOHH-2) (nM) | Tumor cell 5-day relative growth inhibition (DOHH-2) (nM) | Tumor cell 5-day relative growth inhibition (HCT116-MTAP$^{-/-}$) (nM) | Tumor cell 5-day relative growth inhibition (HCT116-MTAP$^{-/-}$) (nM) | Tumor cell 5-day relative growth inhibition (HCT116-MTAP$^{+/+}$) (nM) | Tumor cell 5-day relative growth inhibition (HCT116-MTAP$^{+/+}$) (nM) | Tumor cell 5-day relative growth inhibition (Hut-78) (nM) | Tumor cell 5-day relative growth inhibition (Hut-78) (nM) |
|---|---|---|---|---|---|---|---|---|
| B384 | | | | | | | | |
| B385 | | | | | | | | |
| B386 | | | | | | | | |
| B387 | | | | | | | | |
| B388 | | | | | | | | |
| B389 | | | A | 223 | C | >10000 | | |
| B390 | | | | | | | | |
| B391 | | | | | | | | |
| B392 | | | | | | | | |
| B393 | | | | | | | | |
| B394 | NT | NT | A | 359 | C | 6840 | NT | NT |
| B395 | | | | | | | | |
| B396 | | | | | | | | |
| B397 | | | | | | | | |
| B398 | NT | NT | NT | NT | NT | NT | NT | NT |
| B399 | NT | NT | NT | NT | NT | NT | NT | NT |
| B400 | | | C | >10000 | C | >10000 | | |
| B401 | | | C | >10000 | C | >10000 | | |
| B402 | | | C | >10000 | C | >10000 | | |
| B403 | | | C | >10000 | C | >10000 | | |
| B404 | | | C | >10000 | C | >10000 | | |
| B405 | | | C | >10000 | C | >10000 | | |
| B406 | | | C | >10000 | C | >10000 | | |
| B407 | | | C | >10000 | C | >10000 | | |
| B408 | | | C | >10000 | C | >10000 | | |
| B409 | | | B | 1529 | C | 4036 | | |
| B410 | | | C | >10000 | C | >10000 | | |

As can be seen from Table 2, most of the compounds had good proliferation inhibitory activity against MATP-knockout HCT116 cells and a certain selectivity against MATP wild-type HCT116 cells. They also had good anti-proliferative activity on MATP-deficient tumor cells DOHH2 and Hut-78.

**Experimental Example 2: Stability Test in Liver Microsomes**

Phase I metabolic stability of the test compounds in liver microsomes of CD-1 mice, Sprague-Dawley rats, beagle dogs, cynomolgus monkeys, and humans was assessed.

**Experimental system:**

The animal and human liver microsomes used in the test system were purchased from Xenotech, Corning, or other qualified suppliers and stored in a freezer at a temperature below -60 °C prior to use.

**Brief introduction of the experiment:**

The test samples and control compounds were separately incubated with animal and human liver microsomes at 37±1 °C for a period of incubation time up to 60 min. The samples were removed at the indicated time points, and the reaction was terminated with acetonitrile or other organic solvents containing an internal standard. After centrifugation, the resulting supernatant was assayed by liquid chromatography-tandem mass spectrometry (LC-MS/MS).

**Experimental method:**

1. Preparation of buffer

73.21 g of dipotassium hydrogenphosphate trihydrate and 10.78 g of potassium dihydrogenphosphate were dissolved in 4000 mL of ultrapure water. The pH value of the solution was adjusted to 7.40±0.10 using 10% phosphoric acid or 1 M potassium hydroxide to a final concentration of 100 mM.

2. Preparation of working solution

The test sample powder was prepared into a stock solution with a certain concentration using DMSO or other organic solvents, which was then further diluted using a proper organic solvent.

Control compounds testosterone, diclofenac, and propafenone were prepared into a 10 mM stock solution using DMSO, which was then further diluted using a proper organic solvent.

3. Preparation of liver microsome solution

Microsomes of each species were diluted to a 2× working solution using a 100 mM potassium phosphate buffer. The final concentration of the microsomes in the reaction system was 0.5 mg/mL.

4. Preparation of reduced nicotinamide adenine dinucleotide phosphate (NADPH) regeneration system

An appropriate amount of nicotinamide adenine dinucleotide phosphate (NADP) and isocitric acid (ISO) powder was weighed out, dissolved in a magnesium chloride solution, and shaken and mixed well. An appropriate amount of isocitrate dehydrogenase (IDH) was added, and the mixture was mixed well by gently turning upside down. The final concentrations in the reaction system were: 1 mM NADP, 1 mM magnesium chloride, 6 mM ISO, and 1 unit/mL IDH.

5. Preparation of stop solution

The stop solution was prepared using acetonitrile or other organic solvent containing an internal standard (tolbutamide or other suitable compounds). The prepared stop solution was stored in a freezer at a temperature of 2-8 °C.

6. Incubation process

Incubation was performed in a 96-well plate. 8 incubation plates were prepared and designated T0, T5, T15, T30, T45, T60, Blank60, and NCF60, respectively. The first 6 plates corresponded to reaction time points of 0, 5, 15, 30, 45, and 60 min, respectively. No test sample or control compound was added to the Blank60 plate, and samples were taken after 60 min of incubation. In the NCF60 plate, incubation was performed for 60 min using the potassium phosphate buffer instead of the NADPH regeneration system solution. Three replicates were made for samples in all conditions.

The microsomes and the test sample or control compound were mixed, and then the incubation plates Blank60, T5, T15, T30, T45, and T60 except for T0 and NCF60 were placed in a 37 °C water bath for pre-incubation for about 10 min. In the incubation plate T0, a stop solution was first added, followed by addition of the NADPH regeneration system working solution, and 98 μL of the potassium phosphate buffer was added to each sample well of the incubation plate NCF60 to initiate the reaction. After the pre-incubations of the incubation plates Blank60, T5, T15, T30, T45, and T60 were completed, 98 μL of the NADPH regeneration system working solution was added to each sample well to initiate the reaction. The reaction temperature was 37±1 °C, the final volume of the reaction was 200 μL, and the reaction system

included 0.5 mg/mL microsomes, 1.0 μM substrate, 1 mM NADP, 6 mM ISO, and 1 unit/mL IDH.

At 5, 15, 30, 45, and 60 min, a cold stop solution containing an internal standard was separately added to the reaction plate to stop the reaction.

All the reaction plates after termination were shaken up and centrifuged for 20 min at 3220× g at 4 °C. After the supernatant was diluted according to a certain proportion, LC-MS/MS analysis was performed.

**Sample analysis**

Sample analysis was carried out by the liquid chromatography-tandem mass spectrometry (LC-MS/MS) method, and the standard curve and quality control sample were not included. Semiquantitative determinations were made using the ratio of the analyte peak area to the internal standard peak area. The retention times of the analyte and internal standard, the chromatogram acquisitions and the integrals of the chromatograms were processed with software Analyst (Sciex, Framingham, Massachusetts, USA).

The CV for the internal standard peak area in each matrix in each analysis batch should be within 20%.

**Data analysis**

The *in vitro* elimination rate constant ke of the compound was obtained by converting the ratio of the peak area of the compound to the internal standard peak area in the following formula into the remaining rate:

$$\text{Remaining rate (\%)} = \frac{\text{Peak area ratio of compound to internal standard at any time point}}{\text{Peak area ratio of compound to internal standard at 0 min}} \times 100$$

$$C_t = C_0 \bullet e^{-k_e \bullet t}$$

*when* $C_t = \frac{1}{2} C_0$,

$$T_{1/2} = \frac{Ln\ 2}{k_e} = \frac{0.693}{k_e}$$

$CL_{int\ (mic)}$ = 0.693/ $T_{1/2}$ /microsome protein content (microsome concentration mg/mL during incubation)

$$CL_{int\ (liver)} = CL_{int\ (mic)} \times \text{microsome protein amount in liver (mg/g)} \times \text{liver-to-body weight ratio}$$

According to the well stirred model, the hepatic intrinsic clearance and hepatic clearance can be converted through the following formula.

$$CL_{(liver)} = (CL_{int(liver)} * Q_h)/ (CL_{int(liver)} + Q_h)$$

Parameters in the formula are shown in Table 3.

Table 3. Parameters in the data analysis formula

| Species | Liver-to-body weight ratio (g/kg body weight)[1-2] | Liver blood flow volume ($Q_h$) (mL/min/kg)[1-2] | Microsome protein amount (mg/g liver weight) |
|---|---|---|---|
| Mice | 88 | 90.0 | |
| Rat | 40 | 55.2 | |
| Dog | 32 | 30.9 | 45 |
| Monkey | 30 | 43.6 | |
| Human | 20 | 20.7 | |

The liver microsome stability data for some of the compounds are shown in Table 4:

Table 4. Liver microsome stability data for some of the compounds

| No. | Metabolic half-life in mouse liver microsome (T1/2, min) | Metabolic half-life in rat liver microsome (T1/2, min) | Metabolic half-life in human liver microsome (T1/2, min) |
|---|---|---|---|
| B12 | >145 | >145 | >145 |
| B60 | >145 | >145 | 721.5 |
| B148 | 120.0 | 142.4 | >145 |
| B181 | >145 | >145 | >145 |
| B182 | 135.9 | 20.4 | 258 |

**Experimental Example 3: Metabolic Stability Test in Hepatocytes**

This experimental example was used to test the metabolic stability of the compounds in hepatocytes.

A suspension of hepatocytes was prepared at $0.5 \times 10^6$/mL with a pre-heated medium, then 198 μL of the pre-heated cell suspension was added to a 96-well plate. To each well of the 96-well plate, 2 μL of the test compound was added so that the final concentration was 1 μM, and 2 replicate wells were set. For the T = 0 min sample, the compound was mixed well with the cells for 1 min, then 25 μL of the sample was immediately added to 125 μL of a stop solution (an acetonitrile solution containing 200 ng/mL tolbutamide and 200 ng/mL labetalol) under an ice bath and mixed well. At the same time, all the plates were placed in an incubator at 37 °C with 5% CO2, with the shaker set at 600 rpm. The sample was mixed well at 15, 30, 60, and 90 min of incubation, respectively, and 25 μL of the sample was added to 125 μL of a stop solution (an acetonitrile solution containing 200 ng/mL tolbutamide and 200 ng/mL labetalol) under an ice bath, mixed well, and shaken at 500 rpm for 10 min. Subsequently, the plate was centrifuged for 20 min at 3220× g at 4 °C. After the centrifugation was completed, 80 μL of the supernatant from each well was transferred to another 96-well plate containing 240 μL of ultrapure water. Subsequently, analysis was carried out using LC-MS/MS, and intrinsic clearance (CLint) and half-life (T1/2) were calculated.

The metabolic stability data in hepatocytes for some of the compounds are shown in Table 5:

Table 5. Metabolic stability data in hepatocytes for some of the compounds

| No. | Metabolic half-life in mouse liver microsome (T1/2, min) | Metabolic half-life in rat liver microsome (T1/2, min) | Metabolic half-life in human liver microsome (T1/2, min) |
|---|---|---|---|
| B12 | 68.8 | | |
| B148 | 14.9 | 50.9 | |
| B180 | 47.1 | 51.1 | |
| B181 | 91.1 | | >216.8 |

**Experimental Example 4: Pharmacokinetic Test in Rats**

In this example, the pharmacokinetic behavior of the compound after intravenous injection (IV) and intragastric (PO) administration in SD rats was tested.

On the day of administration, rats were weighed for actual body weight and the administration volume was

calculated. Each compound was tested in two groups with 3 rats in each group, one group administered by single intravenous injection and the other by single intragastric administration. Whole blood samples were collected via the jugular vein at prescribed time points (0.25, 0.5, 1, 2, 4, 8, and 24 h after administration). After blood sample collection, the samples were transferred to labeled commercial sample tubes containing K2-EDTA (0.85-1.15 mg), followed by centrifugation (3200× g, 4 °C, 10 min) and plasma collection. The plasma was transferred to a precooled centrifuge tube, frozen in dry ice, and stored in an ultra-low temperature freezer at -60 °C or lower until the LC-MS/MS analysis.

Plasma concentrations were determined using LC-MS/MS method. Plasma drug concentration data for the compounds were processed in a non-compartmental model using WinNonlin Version 6.3 (Pharsight, Mountain View, CA) pharmacokinetic software. The relevant pharmacokinetic parameters were calculated using linear logarithmic trapezoid method.

The pharmacokinetic results in rats for some of the compounds are shown in Table 6:

Table 6. Pharmacokinetic results in rats for some of the compounds

| No. | IV 1 mg/kg | | | PO 5 mg/kg | | F% |
|---|---|---|---|---|---|---|
| | AUC(nM*h) | T1/2 (h) | Cl ( L/h/kg) | AUC(nM*h) | T1/2 (h) | |
| B3 | 7806 | 0.08 | | 26019 | 0.97 | 67 |
| B4 | 2495 | 0.62 | 1.3 | 7949 | 2.1 | 68 |
| B12 | 18910 | 3.4 | 0.16 | 222704 | 3.7 | 217 |
| B28 | 7603 | 1.7 | 0.96 | 7603 | 3.3 | 60 |
| B60 | | | | | | |
| B181 | 119630 | 6.7 | 0.02 | 540512 | 9.5 | 90 |
| B257 | 177000 | 8.8 | 0.017 | 16120000 (PO 10 mg/kg) | 72.8 (PO 10 mg/kg) | 243 |

As can be seen from the results in Table 6, the compounds of the present invention had relatively good oral exposure and oral bioavailability in rats.

### Experimental Example 5: Pharmacokinetic Test in Mice

In this example, the pharmacokinetic behavior of the compound after intravenous injection (IV) and intragastric (PO) administration in BALB/c mice was tested.

On the day of administration, mice were weighed for actual body weight and the administration volume was calculated. Each compound was tested in two groups with 9 mice in each group, one group administered by single intravenous injection and the other group of mice by single intragastric administration. Whole blood samples were collected via the orbit at prescribed time points (0.25, 0.5, 1, 2, 4, 8, and 24 h after administration). After blood sample collection, the samples were transferred to labeled commercial sample tubes containing K2-EDTA (0.85-1.15 mg), followed by centrifugation (3200× g, 4 °C, 10 min) and plasma collection. The plasma was transferred to a precooled centrifuge tube, frozen in dry ice, and stored in an ultra-low temperature freezer at -60 °C or lower until the LC-MS/MS analysis.

Plasma concentrations were determined using LC-MS/MS method. Plasma drug concentration data for the compounds were processed in a non-compartmental model using WinNonlin Version 6.3 (Pharsight, Mountain View, CA) pharmacokinetic software. The relevant pharmacokinetic parameters were calculated using linear logarithmic trapezoid method.

The pharmacokinetic results in mice for some of the compounds are shown in Table 7:

Table 7. Pharmacokinetic results in mice for some of the compounds

| No. | IV 2 mg/kg | | | | PO 10 mg/kg | | F% |
|---|---|---|---|---|---|---|---|
| | AUC (nM*h) | T1/2 (h) | Cl ( L/h/kg) | Vz (L/Kg) | AUC(nM*h) | T1/2 (h) | |
| B12 | 4496 | 0.98 | 1.36 | 1.9 | 26825 | 0.81 | 113 |
| B17 | 3456 | 0.24 | 1.66 | 0.6 | 22548 | 0.47 | 127 |
| B29 | 1856 | 0.3 | 2.8 | 1.3 | 9318 | 0.43 | 95 |
| B60 | 118558 | 10.0 | 0.04 | 0.6 | 683144 | 9.0 | 115 |

(continued)

| No. | IV 2 mg/kg | | | | PO 10 mg/kg | | F% |
|---|---|---|---|---|---|---|---|
| | AUC (nM*h) | T1/2 (h) | Cl ( L/h/kg) | Vz (L/Kg) | AUC(nM*h) | T1/2 (h) | |
| B72 | 7427 | 0.78 | 0.77 | 0.9 | 65010 | 0.95 | 173 |
| B180 | 3368 | 0.16 | 1.64 | 0.4 | 46182 | 0.51 | 274 |
| B181 | 34287 | 7.3 | 0.14 | 1.4 | 241847 | 3.45 | 131 |
| B198 | 8698.689 | 0.681 | 0.64 | 0.6 | 65219.13 | 0.918 | 150 |
| B245 | 200473 | 10.8 | 0.018 | 0.3 | 806758 | 18.0 | 80 |
| B254 | 244714.8 | 5.73 | 0.019 | 0.2 | 1472866 | 5.791 | 126.4 |
| B256 | 37771.521 | 5.283 | 0.137 | 1.0 | 331109.5 | 8.613 | 175.3 |
| B257 | 49800 | 9.8 | 0.055 | 0.8 | 2113000 | 34.2 | 220 |
| B258 | 21390 | 14.2 | 0.125 | 2.6 | 80700 (PO 5 mg/kg) | 7.7 (PO 5 mg/kg) | 93 |

As can be seen from the results in Table 7, the compounds of the present invention had relatively good oral exposure and oral bioavailability in mice, wherein B60, B181, B245, B254, B256, B257, and B258 had a relatively high AUC and a relatively long half-life (T1/2); B258 had a relatively high Vz value and good tissue distribution.

**Experimental Example 6: hERG Inhibition Test**

The HEK293 cells were cultured in a DMEM medium containing 10% fetal bovine serum and 0.8 mg/mL G418 at 37 °C with 5% $CO_2$. The cells were digested by TrypLE™ Express and centrifuged. The cell density was adjusted to $2 \times 10^6$ cells/mL. The cells were gently mixed for 15-20 min using a shaker equilibrated at room temperature, and subjected to patch clamping on a machine. The medium of the prepared cells was replaced with extracellular fluid. The intracellular fluid and the extracellular fluid were taken from the fluid pool, and respectively added to the intracellular fluid pool and the cell and the test substance pool of the QPlate chip. The voltage stimulation of the whole-cell hERG potassium current was recorded by the whole-cell patch clamp, and the test data was collected and stored by Qpatch. The compound was diluted 3-fold at an initial concentration of 30 μM, and 6 concentration points were set, with each drug concentration being set for two administrations over a period of at least 5 min. The current detected in compound-free extracellular fluid for each cell was served as its own control group, and the detection was repeated at least twice independently using two cells per concentration. All electrophysiological experiments were performed at room temperature.

For data analysis, the current acted for each drug concentration and the current of the blank control were standardized ( $\frac{\text{Peak tail current}_{\text{compound}}}{\text{Peak tail current}_{\text{vehicle}}}$ ), and then the inhibition rate corresponding to each drug concentration was calculated ( $1-\frac{\text{Peak tail current}_{\text{compound}}}{\text{Peak tail current}_{\text{vehicle}}}$ ). The mean and standard error were calculated for each concentration, and the half maximal inhibitory concentration of each compound was calculated: $Y=\text{Bottom}+\frac{\text{Top-Bottom}}{1+10\hat{}((\text{LogIC}_{50}\text{-C})*\text{HillSlope})}$. The dose-dependent effect was fitted non-linearly using the above equation, wherein Y represents the inhibition rate, C represents the concentration of the test substance, $IC_{50}$ is the half maximal inhibitory concentration, and HillSlope represents the Hill coefficient. Curve fitting and calculation of $IC_{50}$ were performed by Graphpad software.

The hERG inhibitory activity data for some of the compounds are shown in Table 8:

Table 8. hERG inhibitory activity data for some of the compounds

| No. | **hERG $IC_{50}$ μM** |
|---|---|
| B12 | >30 |
| B60 | >40 |
| B72 | >30 |
| B148 | >30 |

(continued)

| No. | **hERG $IC_{50}$ μM** |
|---|---|
| B178 | >30 |
| B180 | >30 |
| B181 | >30 |
| B257 | >30 |

As can be seen from the results in Table 8, the compounds of the present invention had no hERG inhibitory activity.

**Experimental Example 7: Cytochrome Oxidase P450 Inhibition Test**

1) Preparation of buffer:

100 mM K-Buffer: 9.5 mL of stock solution A was mixed with 40.5 mL of stock solution B, the total volume was adjusted to 500 mL with ultrapure water, and the buffer was titrated to pH 7.4 with KOH or $H_3PO_4$.
Starting material A (1 M potassium dihydrogen phosphate): 136.5 g of potassium dihydrogen phosphate in 1 L of water.
Stock B (1 M potassium dihydrogen phosphate): 174.2 g of potassium dihydrogen phosphate in 1 L of water.

2) Preparation of test substance

The test substance powder was prepared into a stock solution with a certain concentration using DMSO or other organic solvents, which was then further diluted using a proper organic solvent.

3) *In vitro* incubation

The extracorporeal incubation system for CYP450 enzyme metabolism phenotype study was biochemical reaction of a prepared liver microsome, a redox coenzyme, and an enzyme-specific selective inhibitor in simulated physiological temperature and environment.

4) Detection of parent drug or metabolite

The concentration of the parent drug or a metabolite thereof in the incubation solution was determined by LC-MS/MS.
The CYP inhibitory activity data for some of the compounds are shown in Table 9:

Table 9. CYP inhibitory activity data for some of the compounds

| No. | **CYP1A2 $IC_{50}$ μM** | **CYP2C9 $IC_{50}$ μM** | **CYP2C19 $IC_{50}$ μM** | **CYP2D6 $IC_{50}$ μM** | **CYP3A4 $IC_{50}$ μM** |
|---|---|---|---|---|---|
| B12 | >10 | >10 | >10 | >10 | >10 |
| B60 | >10 | >10 | >10 | >10 | >10 |
| B180 | >10 | >10 | >10 | >10 | >10 |
| B181 | >30 | >30 | >30 | >30 | >30 |

As can be seen from the results in Table 9, the compounds of the present invention had no CYP liver enzyme inhibitory activity.

**Experimental Example 8: PXR Activation Test**

In this example, the compound was tested for PXR activation.
100 μL of stably transfected DPX2 cells were seeded per well of a 96-well cell culture plate ($4.5 \times 10^5$ cells/mL in Puracyp medium), followed by placing the 96-well plate in a 37 °C incubator. After 24 h, the 96-well plate was taken out from the incubator, the medium was replaced with 100 μL of the test compound (at a final concentration of 10 μM and 30 μM),

positive control compound rifampicin (at a final concentration of 10 μM), and the DMSO treatment group (at a final concentration of 0.1%), and 3 replicate wells were set. The test plate was placed back into the incubator. After 24 h, the medium was replaced with the freshly prepared test compound and rifampicin solution, and the test plate was again placed back into the incubator. After the compound was treated for 48 h, the test plate was taken out from the incubator. The medium in the wells was discarded, the plate was washed twice with PBS, 50 μL of medium containing 1× CellTiter-Fluor™ Cell Viability Assay reagent was added, and the mixture was incubated at 37 °C for 30 min. The test plate was taken out and equilibrated to room temperature, and the fluorescence signal under the conditions of 400 nm excitation light/505 nm emission light was detected by a microplate reader. Subsequently, 50 μL of medium containing ONE-Glo Assay reagent was added to each well, and the plate was shaken, mixed well, and incubated at room temperature for 5 min. Chemiluminescence was detected by a microplate reader. The data was analyzed to evaluate the PXR activation of the test compound.

The PXR activation data for some of the compounds are shown in Table 10:

Table 10. PXR activation data for some of the compounds

| No. | **PXR activation fold (10 μM)** | **PXR activation fold (30 μM)** |
|---|---|---|
| B60 | 7.06 | 21.71 |
| B72 | 4.55 | 14.96 |
| B178 | 7.47 | 34.49 |
| B180 | 2.26 | 25.05 |
| B181 | 3.63 | 3.77 |
| B257 | 3.37 | 7.87 |

As can be seen from the results in Table 10, the compound of the present invention had relatively weak activation activity on PXR at 10 μM and medium activation activity on PXR at 30 μM. The activation activity of B181 and B257 on PXR at 30 μM was also very weak, much lower than the other compounds.

**Experimental Example 9: Pharmacodynamic Model of Mouse Tumor**

In this example, the *in vivo* efficacy of the compound in a mouse xenograft tumor model after intragastric (PO) administration was tested.

Human colorectal adenocarcinoma cells HCT116-MTAP-KO were cultured in monolayer *in vitro* under the condition of McCoy's 5A medium containing 10% fetal bovine serum and 1% penicillin and streptomycin, and the culture was carried out at 37 °C with 5% CO2. The cells were digested with trypsin three times a week for passaging. When the saturation degree of the cells was 80-90%, the cells were collected, counted and inoculated. 0.1 mL ($5 \times 10^6$ cells) of HCT116-MTAP-KO cells were subcutaneously inoculated on the right back of each mouse. On day 16 after inoculation of the cells, the mean tumor volume reached 154 $mm^3$. The mice were randomly grouped for administration and intragastrically dosed once daily. Changes in body weight and tumor volume were recorded. After a certain number of days of administration, the experiment was ended. Tumor volume changes and mouse body weight changes were counted and analyzed. AG270 (CAS: 2201056-66-6) was the positive control compound.

Table 11. Human colorectal adenocarcinoma cell HCT116-MTAP-KO tumor pharmacodynamic model

| Compound No. | Days of administration | Tumor growth inhibition percentage (TGI) |
|---|---|---|
| AG270 | 28 | 200 mg/kg administered once a day TGI = 33.82% |
| B257 | 28 | 1 mg/kg administered once a day TGI = 51.34%<br>3 mg/kg administered once a day TGI = 87.78% |
| B257 | 15 | 1 mg/kg administered once a day TGI = 77.29% |
| B258 | 15 | 3 mg/kg administered once a day TGI = 52.48%<br>10 mg/kg administered once a day TGI = 72.58%<br>30 mg/kg administered once a day TGI = 81.2%<br>100 mg/kg administered once a day TGI = 83.75% |

Human pancreatic cancer cells KP-4 were cultured in monolayer *in vitro* under the condition of 1640 medium containing 10% fetal bovine serum and 1% penicillin and streptomycin, and the culture was carried out at 37 °C with 5% CO2. The cells were digested with trypsin three times a week for passaging. When the saturation degree of the cells was 80-90%, the cells were collected, counted and inoculated. 0.1 mL ($5 \times 10^6$ cells) of KP-4 cells were subcutaneously inoculated on the right back of each mouse. On day 34 after inoculation of the cells, the mean tumor volume reached 154 $mm^3$. The mice were randomly grouped for administration and intragastrically dosed once daily. Changes in body weight and tumor volume were recorded. After 29 days of administration, the experiment was ended. Tumor volume changes and mouse body weight changes were counted and analyzed. AG270 (CAS: 2201056-66-6) was the positive control compound.

Table 12. Human pancreatic cancer cell KP-4 tumor pharmacodynamic model

| Compound No. | Days of administration | Tumor growth inhibition percentage (TGI) |
|---|---|---|
| AG270 | 29 | 200 mg/kg administered once a day TGI = 57.48% |
| B257 | 29 | 0.3 mg/kg administered once a day TGI = 66.25%<br>1 mg/kg administered once a day TGI = 90.06%<br>3 mg/kg administered once a day TGI = 97.08% |

As can be seen from the results in Table 11 and Table 12, the compound of the present invention had relatively good *in vivo* efficacy.

**Experimental Example 10**: Test of Saturation Solubility of Samples in FaSSIF Solution

1. Experimental process

1.1 Preparation of FaSSIF solution

Preparation of buffer (pH 6.5):

About 0.21 g of sodium hydroxide, about 2.24 g of sodium dihydrogen phosphate dihydrate, and 3.09 g of sodium chloride were weighed out and dissolved in 500 mL of water, and then the pH was adjusted to 6.5 with 1 N sodium hydroxide or 1 N hydrochloric acid.

112 mg of FaSSIF solid was weighed out and placed in a 50 mL volumetric flask, and the above buffer was added for dissolution, diluted to volume, mixed well, and left to stand at room temperature for 2 h or more.

1.2 Preparation of sample

Test sample: about 1 mg of the test sample was taken, and 1 mL of FaSSIF solution was added. The mixture was stirred at room temperature overnight and centrifuged, and the supernatant was collected and injected for analysis.

Control substance solution: about 1.5 mg of the test sample was precisely weighed out and placed in a 50 mL volumetric flask, and DMF was added for dissolution, diluted to volume, and mixed well.

1.3 Preparation of mobile phase

Mobile phase A: 1000 mL of purified water was accurately weighed out, 1 mL of formic acid was added, and the mixture was mixed well and degassed with ultrasonic.

Mobile phase B: acetonitrile.

1.4 Chromatographic conditions (Table 13)

Table 13

| Instrument | HPLC |
|---|---|
| Chromatographic column | Waters XBridge C18, 4.6*50mm 3.5um |
| Sample tray (°C) | off |

(continued)

| | | |
|---|---|---|
| Column temperature (°C) | 30 | |
| UV wavelength (nm) | 254 | |
| Needle wash solution | MeOH | |
| Flow rate (mL/min) | 1.0 | |
| Run time (min) | 6 | |
| Gradient elution table | | |
| Time (min) | Mobile phase A (%) | Mobile phase B (%) |
| 0 | 90 | 10 |
| 0.5 | 90 | 10 |
| 6 | 10 | 90 |
| 7 | 10 | 90 |
| 7.1 | 90 | 10 |
| 10 | 90 | 10 |

2. Result

The peak of the main component of the control substance and the peak of the test substance consistent with the retention time of the peak of the main component of the control substance were integrated, and the concentration thereof in the FaSSIF solution was calculated according to external standard method. The test results are shown in Table 14.

Table 14

| Compound No. | FaSSIF(pH = 6.5) (μg/mL) |
|---|---|
| B60 | 20.9 |
| B181 | 20.7 |
| B182 | 17.6 |
| B188 | 7.4 |
| B257 | 248 |
| B258 | 131 |

As can be seen from the results in Table 13, the compound of the present application had relatively good solubility.

It should be understood that the above examples are exemplary and are not intended to encompass all possible implementations encompassed by the claims. Various modifications and changes may also be made on the above examples without departing from the scope of the present disclosure. Likewise, various technical features of the above examples may also be combined in any combination to form additional examples of the present application that may not be explicitly described. Therefore, the above examples only represent several embodiments of the present application, and do not limit the scope of the present application.

## Claims

1. A compound of formula (I) or a stereoisomer, a tautomer, a solvate, a hydrate, a prodrug, a stable isotopic derivative, or a pharmaceutically acceptable salt thereof,

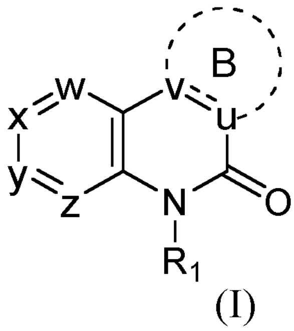

(I)

wherein:

w is $CR_3$ or N;
x is $CR_4$ or N;
y is $CR_5$ or N;
z is $CR_6$ or N;
v and u are each independently selected from C or N;
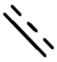 indicates that it may be a single bond or double bond;
$R_3$ and $R_5$ are each independently selected from hydrogen, alkyl, alkenyl, alkynyl, alkoxy, sulfonyl, halogen, cycloalkyl, cyano, amino, acyl, hydroxyl, heteroaryl, heteroaryloxy, heterocyclyl, heterocyclyloxy, and cycloalkyloxy; $R_3$ is optionally further substituted with one or more $R_{3a}$, and $R_5$ is optionally further substituted with one or more $R_{5a}$, wherein each $R_{3a}$ and each $R_{5a}$ are independently selected from alkyl, alkenyl, alkynyl, alkoxy, alkylamino, dialkylamino, halogen, sulfonyl, cycloalkyl, cyano, amino, acyl, hydroxyl, heteroaryl, heteroaryloxy, cycloalkyloxy, heterocyclyl, and heterocyclyloxy; optionally, $R_{3a}$ is further substituted with one or more $R_{3b}$, and $R_{5a}$ is further substituted with one or more $R_{5b}$, wherein each $R_{3b}$ and each $R_{5b}$ are independently selected from alkyl, halogen, hydroxyl, alkoxy, amino, and heterocyclyloxy;
$R_4$ and $R_6$ are each independently selected from hydrogen, alkyl, alkenyl, alkynyl, alkoxy, alkylthio, sulfonyl, halogen, cycloalkyl, cyano, amino, and acyl; optionally, $R_4$ is further substituted with one or more $R_{4a}$, and $R_6$ is further substituted with one or more $R_{6a}$, wherein each $R_{4a}$ and each $R_{6a}$ are independently selected from alkyl, halogen, amino, alkylamino, and dialkylamino;
$R_1$ is $-X_1-R_7$, wherein $X_1$ is a bond or alkylene; optionally, $X_1$ is substituted with one or more $X_{1a}$, and each $X_{1a}$ is independently selected from halogen, alkyl, and cycloalkyl; $R_7$ is selected from cycloalkyl, aryl, heteroaryl, and heterocyclyl, wherein $R_7$ is optionally substituted with one or more $R_{7a}$, and each $R_{7a}$ is independently selected from alkyl, alkoxy, hydroxyl, sulfonyl, halogen, cyano, alkenyl, alkynyl, carboxyl, acyl, amino, cycloalkyl, aryl, heterocyclyl, heteroaryl, oxo, and ureido; optionally, $R_{7a}$ is further substituted with one or more $R_{7b}$, wherein $R_{7b}$ is independently selected from halogen, alkyl, alkoxy, hydroxyl, amino, alkenyl, alkynyl, alkylamino, dialkylamino, sulfonyl, heterocyclyl, and heteroaryl;
B is selected from 5-, 6-, or 7-membered aryl or heteroaryl, wherein B is optionally substituted with one or more $R_2$; each $R_2$ is independently selected from hydrogen, halogen, alkyl, alkoxy, alkenyl, alkynyl, cycloalkyl, cycloalkyloxy, heterocyclyl, heterocyclyloxy, aryl, heteroaryl, heteroaryloxy, cyano, amino, acyl, sulfonyl, and oxo, or two adjacent $R_2$ form a 3- to 7-membered carbocyclic ring, heterocyclic ring, aromatic ring, or heteroaromatic ring; optionally, $R_2$ or the 3- to 7-membered carbocyclic ring, heterocyclic ring, aromatic ring, or heteroaromatic ring formed by two adjacent $R_2$ is further substituted with one or more $R_{2a}$, wherein each $R_{2a}$ is independently selected from halogen, alkyl, heterocyclyl, heterocyclyloxy, amino, heteroaryl, cycloalkyl, aryl, alkylamino, dialkylamino, hydroxyl, sulfonyl, alkoxy, cyano, alkenyl, acyl, alkynyl, and oxo; optionally, $R_{2a}$ is further substituted with one or more $R_{2b}$, wherein each $R_{2b}$ is independently selected from alkyl substituted with 0-3 halogens, amino, alkylamino, dialkylamino, alkoxy substituted with 0-3 halogens, halogen, hydroxyl, unsubstituted heterocyclyl or heterocyclyl substituted with alkyl, cyano, and oxo, preferably selected from alkyl, amino, alkylamino, dialkylamino, alkoxy, halogen, hydroxyl, and oxo.

**2.** The compound of formula (I) or the stereoisomer, the tautomer, the solvate, the hydrate, the prodrug, the stable isotopic derivative, or the pharmaceutically acceptable salt thereof according to claim 1, wherein B is a 6-membered heteroaryl selected from:

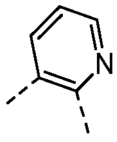, 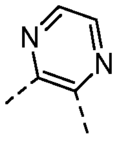, 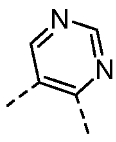, 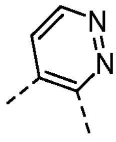, 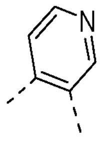, 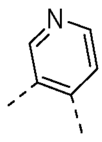, 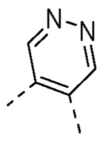, 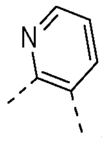, and 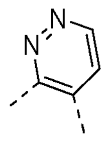,

wherein B is optionally substituted with one or more $R_2$.

**3.** The compound of formula (I) or the stereoisomer, the tautomer, the solvate, the hydrate, the prodrug, the stable isotopic derivative, or the pharmaceutically acceptable salt thereof according to claim 1, wherein the compound has a structure of formula (IA):

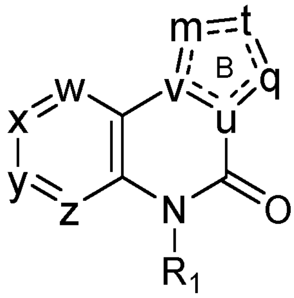

(IA)

wherein:

m is selected from N, O, S, $NR_9$, and $CR_9$;
t is selected from N, O, S, $NR_8$, and $CR_8$;
q is selected from N, O, S, $NR_2$, and $CR_2$;
each $R_2$, each Rs, and each $R_9$ are independently selected from hydrogen, halogen, alkyl, alkoxy, alkenyl, alkynyl, cycloalkyl, cycloalkyloxy, heterocyclyl, heterocyclyloxy, aryl, heteroaryl, heteroaryloxy, cyano, amino, acyl, sulfonyl, and oxo, or adjacent $R_8$ and $R_2$ form a 3- to 7-membered carbocyclic ring, heterocyclic ring, aromatic ring, or heteroaromatic ring, or adjacent $R_8$ and $R_9$ form a 3- to 7-membered carbocyclic ring, heterocyclic ring, aromatic ring, or heteroaromatic ring;
optionally, $R_2$ is further substituted with one or more $R_{2a}$;
optionally, $R_8$ is further substituted with one or more $R_{8a}$;
optionally, $R_9$ is further substituted with one or more $R_{9a}$;
optionally, the 3- to 7-membered carbocyclic ring, heterocyclic ring, aromatic ring, or heteroaromatic ring formed by adjacent $R_8$ and $R_2$ is further substituted with one or more $R_{2a}$ or $R_{8a}$;
optionally, the 3- to 7-membered carbocyclic ring, heterocyclic ring, aromatic ring, or heteroaromatic ring formed by adjacent $R_8$ and $R_9$ is further substituted with one or more $R_{9a}$ or $R_{8a}$;
each $R_{2a}$, each $R_{9a}$, or each $R_{8a}$ is independently selected from halogen, alkyl, heterocyclyl, heterocyclyloxy, amino, heteroaryl, acyl, cycloalkyl, aryl, alkylamino, dialkylamino, hydroxyl, sulfonyl, alkoxy, cyano, alkenyl, alkynyl, and oxo;
optionally, $R_{2a}$ is further substituted with one or more $R_{2b}$;
optionally, $R_{8a}$ is further substituted with one or more $R_{8b}$;
optionally, $R_{9a}$ is further substituted with one or more $R_{9b}$;
each $R_{2b}$, each $R_{8b}$, or each $R_{9b}$ is independently selected from alkyl substituted with 0-3 halogens, alkoxy substituted with 0-3 halogens, halogen, hydroxyl, unsubstituted heterocyclyl or heterocyclyl substituted with alkyl, cyano, and oxo, preferably selected from alkyl, alkoxy, halogen, hydroxyl, and oxo.

**4.** The compound of formula (I) or the stereoisomer, the tautomer, the solvate, the hydrate, the prodrug, the stable isotopic derivative, or the pharmaceutically acceptable salt thereof according to claim 3, wherein the compound has a structure of formula ($IB_1$), ($IB_2$), ($IB_3$), ($IB_4$), or (IBs):

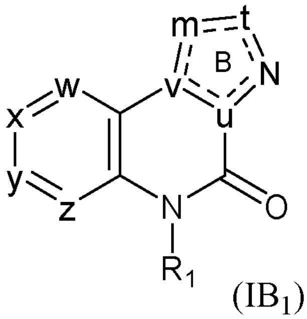
$(IB_1)$,
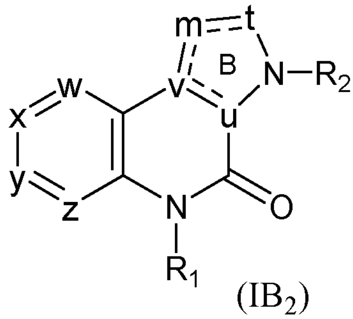
$(IB_2)$,
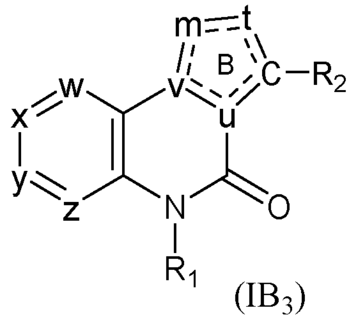
$(IB_3)$,

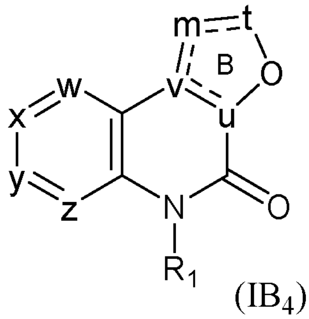
$(IB_4)$, and
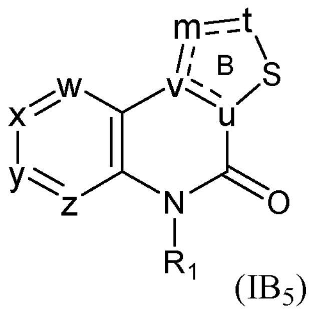
$(IB_5)$,

wherein m is selected from N, O, S, $NR_9$, and $CR_9$;
t is selected from N, O, S, $NR_8$, and $CR_8$,
wherein $R_8$ or $R_9$ is each independently selected from hydrogen, halogen, alkyl, alkoxy, alkenyl, alkynyl, cycloalkyl, cycloalkyloxy, heterocyclyl, heterocyclyloxy, aryl, heteroaryl, heteroaryloxy, cyano, amino, acyl, sulfonyl, and oxo, or adjacent $R_8$ and $R_9$ form a 3- to 7-membered carbocyclic ring, heterocyclic ring, or heteroaromatic ring; optionally, $R_8$ is further substituted with one or more $R_{8a}$; optionally, $R_9$ is further substituted with one or more $R_{9a}$; optionally, the 3- to 7-membered carbocyclic ring, heterocyclic ring, or heteroaromatic ring formed by adjacent $R_8$ and $R_9$ is further substituted with one or more $R_{8a}$ or $R_{9a}$; $R_{8a}$ or $R_{9a}$ is each independently selected from halogen, alkyl, heterocyclyl, heterocyclyloxy, amino, heteroaryl, cycloalkyl, aryl, alkylamino, dialkylamino, hydroxyl, acyl, sulfonyl, alkoxy, cyano, alkenyl, alkynyl, and oxo; optionally, $R_{8a}$ is further substituted with one or more $R_{8b}$; optionally, $R_{9a}$ is further substituted with one or more $R_{9b}$; $R_{8b}$ or $R_{9b}$ is each independently selected from alkyl substituted with 0-3 halogens, alkoxy substituted with 0-3 halogens, halogen, hydroxyl, unsubstituted heterocyclyl or heterocyclyl substituted with alkyl, cyano, and oxo, preferably selected from alkyl, alkoxy, halogen, hydroxyl, and oxo;
wherein in the formula $(IB_1)$, $(IB_2)$, $(IB_4)$, or $(IB_5)$, at least one of v, u, m, and t is a substituted or unsubstituted heteroatom; in the formula $(IB_3)$, at least two of v, u, m, and t are substituted or unsubstituted heteroatoms;
wherein when y is $CR_5$, $R_5$ is not hydrogen.

**5.** The compound of formula (I) or the stereoisomer, the tautomer, the solvate, the hydrate, the prodrug, the stable isotopic derivative, or the pharmaceutically acceptable salt thereof according to any one of claims 3-4, wherein the compound has a structure of formula $(IC_1)$, $(IC_2)$, $(IC_3)$, $(IC_4)$, or (ICs):

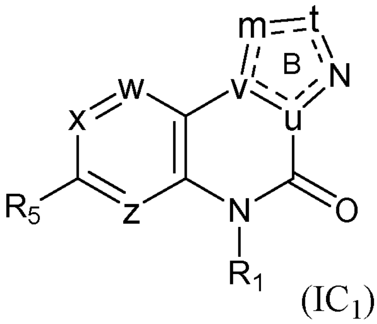
$(IC_1)$,
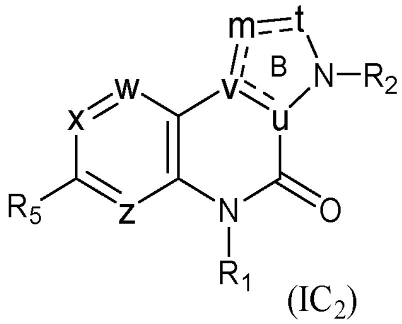
$(IC_2)$,
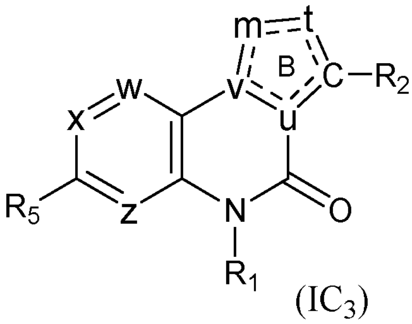
$(IC_3)$,

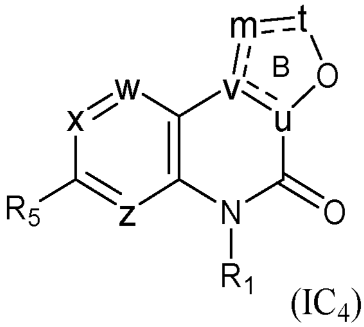
(IC4),

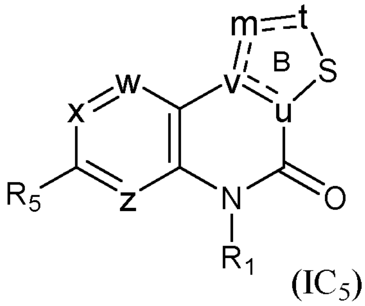
(IC5);

wherein in the formula ($IC_1$), ($IC_2$), ($IC_4$), or ($IC_5$), at least one of v, u, m, and t is a substituted or unsubstituted heteroatom; in the formula ($IC_3$), at least two of v, u, m, and t are substituted or unsubstituted heteroatoms; wherein w is $CR_3$, and x is $CR_4$;

wherein $R_5$ is selected from halogen, cyano, amino, hydroxyl, $C_1$-$C_6$ alkyl, $C_1$-$C_6$ alkoxy, $C_2$-$C_6$ alkenyl, $C_2$-$C_6$ alkynyl, cycloalkyl having 3-10 ring atoms, cycloalkyloxy having 3-10 ring atoms, heterocyclyl having 4-10 ring atoms, heterocyclyloxy having 4-10 ring atoms, and heteroaryl having 5-10 ring atoms; optionally, $R_5$ is further substituted with one or more $R_{5a}$, wherein each $R_{5a}$ is independently selected from $C_1$-$C_6$ alkyl, $C_1$-$C_6$ alkoxy, halogen, cycloalkyl having 3-10 ring atoms, cyano, amino, hydroxyl, alkylamino, dialkylamino, and oxo;

$R_1$ is $-X_1-R_7$, wherein $X_1$ is a bond or methylene; optionally, $X_1$ is substituted with one or more $X_{1a}$, wherein each $X_{1a}$ is independently selected from halogen and $C_1$-$C_3$ alkyl; $R_7$ is selected from cycloalkyl having 4-10 ring atoms, aryl having 6-12 ring atoms, heteroaryl having 5-12 ring atoms, and heterocyclyl having 3-12 ring atoms; wherein $R_7$ is optionally substituted with one or more $R_{7a}$, wherein each $R_{7a}$ is independently selected from oxo, halogen, $C_1$-$C_6$ alkyl, cycloalkyl having 3-10 ring atoms, $C_1$-$C_6$ alkoxy, cycloalkyloxy having 3-6 ring atoms, cyano, amino, hydroxyl, $C_2$-$C_6$ alkenyl, $C_2$-$C_6$ alkynyl, sulfonyl, acyl, carboxyl, heterocyclyl having 3-12 ring atoms, heteroaryl having 5-10 ring atoms, and ureido; optionally, $R_{7a}$ is further substituted with one or more $R_{7b}$, wherein $R_{7b}$ is independently selected from halogen, $C_1$-$C_6$ alkyl, $C_1$-$C_6$ alkoxy, hydroxyl, amino, $C_2$-$C_6$ alkenyl, $C_2$-$C_6$ alkynyl, $C_1$-$C_6$ alkylamino, di($C_1$-$C_6$)alkylamino, sulfonyl, heterocyclyl having 3-6 ring atoms, and heteroaryl having 5-6 ring atoms; wherein when $X_1$ is methylene, $R_7$ is not substituted or unsubstituted aryl.

**6.** The compound or the stereoisomer, the tautomer, the solvate, the hydrate, the prodrug, the stable isotopic derivative, or the pharmaceutically acceptable salt thereof according to any one of claims 1 and 3-5, wherein ring B is selected from the following structures:

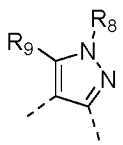
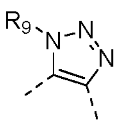
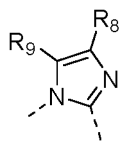
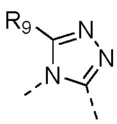
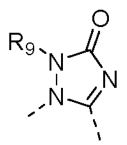
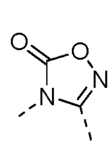
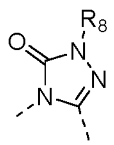
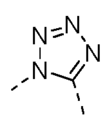

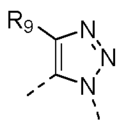
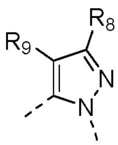
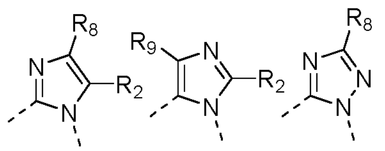
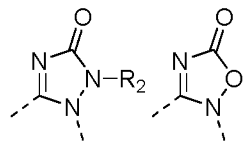
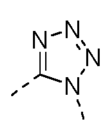

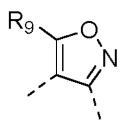
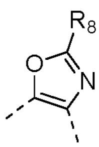
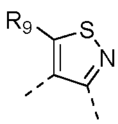
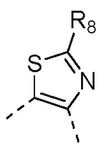
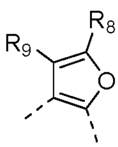
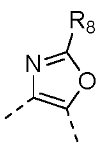
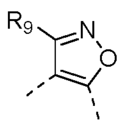
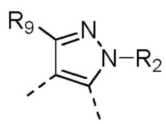

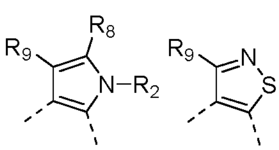
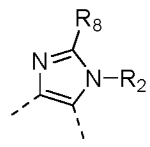
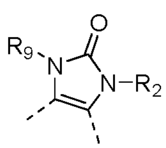
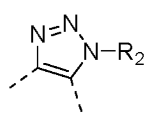
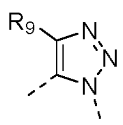
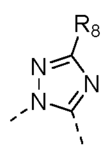

**7.** The compound or the stereoisomer, the tautomer, the solvate, the hydrate, the prodrug, the stable isotopic derivative, or the pharmaceutically acceptable salt thereof according to any one of claims 1 and 3-6, wherein the compound has optionally the following structures:

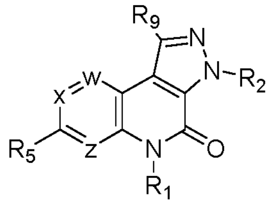
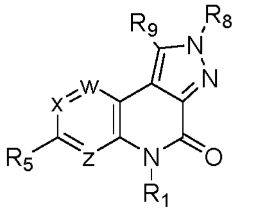
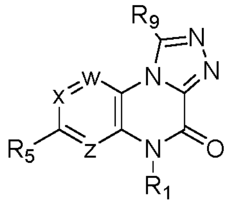
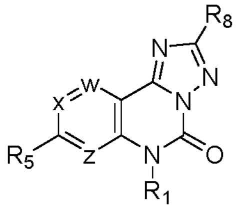
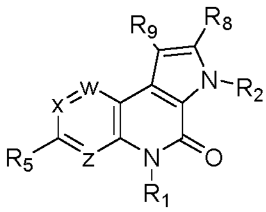
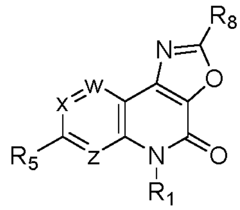

**8.** The compound or the stereoisomer, the tautomer, the solvate, the hydrate, the prodrug, the stable isotopic derivative, or the pharmaceutically acceptable salt thereof according to any one of claims 1 and 3-7, wherein the compound has optionally the following structures:

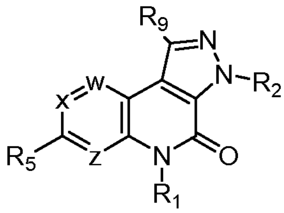
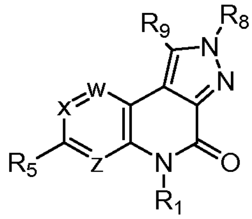

**9.** The compound or the stereoisomer, the tautomer, the solvate, the hydrate, the prodrug, the stable isotopic derivative, or the pharmaceutically acceptable salt thereof according to any one of claims 1-8, wherein:

w is $CR_3$, and $R_3$ is selected from hydrogen, $C_1$-$C_6$ alkyl, $C_2$-$C_6$ alkenyl, $C_2$-$C_6$ alkynyl, $C_1$-$C_6$ alkoxy, sulfonyl, halogen, cycloalkyl having 3-10 ring atoms, cyano, amino, acyl, hydroxyl, heteroaryl having 5-10 ring atoms, heteroaryloxy having 5-10 ring atoms, heterocyclyl having 5-10 ring atoms, heterocyclyloxy having 5-10 ring atoms, and cycloalkyloxy having 3-10 ring atoms; preferably, $R_3$ is selected from hydrogen, $C_1$-$C_4$ alkyl, $C_2$-$C_4$ alkenyl, $C_2$-$C_4$ alkynyl, $C_1$-$C_4$ alkoxy, halogen, cycloalkyl having 3-6 ring atoms, cyano, amino, acyl, hydroxyl, heteroaryl having 5-6 ring atoms, heteroaryloxy having 5-6 ring atoms, heterocyclyl having 5-6 ring atoms, heterocyclyloxy having 5-6 ring atoms, and cycloalkyloxy having 3-6 ring atoms; more preferably, $R_3$ is selected from hydrogen, methyl, ethyl, n-propyl, isopropyl, methoxy, ethoxy, propoxy, and piperidinyloxy;
optionally, $R_3$ is further substituted with one or more $R_{3a}$, wherein each $R_{3a}$ is independently selected from $C_1$-$C_6$ alkyl, $C_2$-$C_6$ alkenyl, $C_2$-$C_6$ alkynyl, $C_1$-$C_6$ alkoxy, $C_1$-$C_6$ alkylamino, di($C_1$-$C_6$ alkyl)amino, halogen, sulfonyl, cycloalkyl having 3-10 ring atoms, cyano, amino, acyl, hydroxyl, heteroaryl having 5-10 ring atoms, heteroaryloxy having 5-10 ring atoms, cycloalkyloxy having 3-10 ring atoms, heterocyclyl having 5-10 ring atoms, and heterocyclyloxy having 5-10 ring atoms; preferably, $R_{3a}$ is each independently selected from $C_1$-$C_3$ alkyl, $C_2$-$C_4$ alkenyl, $C_2$-$C_4$ alkynyl, $C_1$-$C_3$ alkoxy, $C_1$-$C_3$ alkylamino, di($C_1$-$C_3$ alkyl)amino, halogen, sulfonyl, cycloalkyl having 3-6 ring atoms, cyano, amino, acyl, hydroxyl, heteroaryl having 5-6 ring atoms, heteroaryloxy having 5-6 ring atoms, cycloalkyloxy having 3-6 ring atoms, heterocyclyl having 5-6 ring atoms, and heterocyclyloxy having 5-6 ring atoms; more preferably, $R_{3a}$ is each independently selected from methyl and dimethylamino; optionally, $R_{3a}$ is further substituted with one or more $R_{3b}$, wherein each $R_{3b}$ is independently selected from $C_1$-$C_3$ alkyl, halogen, hydroxyl, $C_1$-$C_3$ alkoxy, and amino.

**10.** The compound or the stereoisomer, the tautomer, the solvate, the hydrate, the prodrug, the stable isotopic derivative, or the pharmaceutically acceptable salt thereof according to any one of claims 1-9, wherein:

x is $CR_4$, and $R_4$ is selected from hydrogen, $C_1$-$C_6$ alkyl, $C_2$-$C_6$ alkenyl, $C_2$-$C_6$ alkynyl, $C_1$-$C_6$ alkoxy, $C_1$-$C_6$ alkylthio, sulfonyl, halogen, cycloalkyl having 3-10 ring atoms, cyano, amino, and acyl; preferably, $R_4$ is selected from hydrogen, $C_1$-$C_4$ alkyl, $C_2$-$C_4$ alkenyl, $C_2$-$C_4$ alkynyl, $C_1$-$C_4$ alkoxy, $C_1$-$C_4$ alkylthio, sulfonyl, halogen, cycloalkyl having 3-6 ring atoms, cyano, amino, and acyl; more preferably, $R_4$ is selected from hydrogen, methyl, ethyl, n-propyl, isopropyl, fluorine, chlorine, bromine, and cyano;
optionally, $R_4$ is further substituted with one or more $R_{4a}$, wherein each $R_{4a}$ is independently selected from $C_1$-$C_6$ alkyl, halogen, amino, $C_1$-$C_6$ alkylamino, and di($C_1$-$C_6$)alkylamino; preferably, $R_{4a}$ is each independently selected from $C_1$-$C_3$ alkyl, halogen, amino, $C_1$-$C_3$ alkylamino, and di($C_1$-$C_3$)alkylamino.

**11.** The compound or the stereoisomer, the tautomer, the solvate, the hydrate, the prodrug, the stable isotopic derivative, or the pharmaceutically acceptable salt thereof according to any one of claims 1-10, wherein:

$R_5$ is selected from halogen, cyano, amino, hydroxyl, $C_1$-$C_6$ alkyl, $C_2$-$C_6$ alkenyl, $C_2$-$C_6$ alkynyl, $C_1$-$C_6$ alkoxy, cycloalkyl having 3-10 ring atoms, cycloalkyloxy having 3-10 ring atoms, heterocyclyl having 4-10 ring atoms, and heteroaryl having 5-10 ring atoms; preferably, $R_5$ is selected from halogen, $C_1$-$C_6$ alkyl, $C_1$-$C_6$ alkoxy, cycloalkyl having 3-6 ring atoms, cycloalkyloxy having 3-6 ring atoms, heterocyclyl having 4-6 ring atoms, and heteroaryl having 5-6 ring atoms; more preferably, $R_5$ is selected from chlorine, fluorine, bromine, iodine, cyano, ethyl, n-propyl, isopropyl, methyl, cyclopropyl, methoxy, and cyclopropyloxy;
optionally, $R_5$ is further substituted with one or more $R_{5a}$, wherein each $R_{5a}$ is independently selected from $C_1$-$C_6$ alkyl, $C_1$-$C_6$ alkoxy, halogen, cycloalkyl having 3-10 ring atoms, cyano, amino, and hydroxyl; preferably, $R_{5a}$ is each independently selected from $C_1$-$C_6$ alkyl, halogen, cyano, and cycloalkyl having 3-6 ring atoms; more preferably, $R_{5a}$ is each independently selected from fluorine and cyano.

**12.** The compound or the stereoisomer, the tautomer, the solvate, the hydrate, the prodrug, the stable isotopic derivative, or the pharmaceutically acceptable salt thereof according to any one of claims 1-11, wherein:

z is $CR_6$ or N;
$R_6$ is selected from hydrogen, $C_1$-$C_6$ alkyl, $C_2$-$C_6$ alkenyl, $C_2$-$C_6$ alkynyl, $C_1$-$C_6$ alkoxy, $C_1$-$C_6$ alkylthio, sulfonyl, halogen, cycloalkyl having 3-10 ring atoms, cyano, amino, and acyl; preferably, $R_6$ is selected from hydrogen, $C_1$-$C_4$ alkyl, $C_2$-$C_4$ alkenyl, $C_2$-$C_4$ alkynyl, $C_1$-$C_4$ alkoxy, $C_1$-$C_4$ alkylthio, sulfonyl, halogen, cycloalkyl having 3-6 ring atoms, cyano, amino, and acyl; more preferably, $R_6$ is selected from hydrogen, fluorine, chlorine, methyl, ethyl, n-propyl, isopropyl, cyclopropyl, methoxy, cyclopropyloxy, and cyano;
optionally, $R_6$ is further substituted with one or more $R_{6a}$, wherein each $R_{6a}$ is independently selected from $C_1$-$C_6$ alkyl, halogen, amino, $C_1$-$C_6$ alkylamino, and di($C_1$-$C_6$)alkylamino; preferably, $R_{6a}$ is each independently selected from $C_1$-$C_3$ alkyl, halogen, amino, $C_1$-$C_3$ alkylamino, and di($C_1$-$C_3$)alkylamino.

**13.** The compound or the stereoisomer, the tautomer, the solvate, the hydrate, the prodrug, the stable isotopic derivative, or the pharmaceutically acceptable salt thereof according to any one of claims 1-12, wherein:

$R_1$ is -$X_1$-$R_7$, wherein $X_1$ is a bond or methylene;
optionally, $X_1$ is substituted with one or more $X_{1a}$, wherein each $X_{1a}$ is independently selected from halogen and $C_1$-$C_3$ alkyl, preferably methyl;
$R_7$ is selected from cycloalkyl having 4-10 ring atoms, aryl having 6-12 ring atoms, heteroaryl having 5-12 ring atoms, and heterocyclyl having 3-12 ring atoms;
preferably, $R_7$ is selected from the following groups:

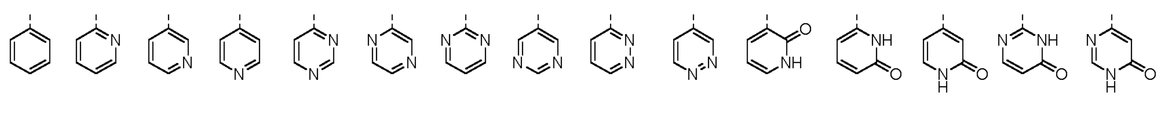

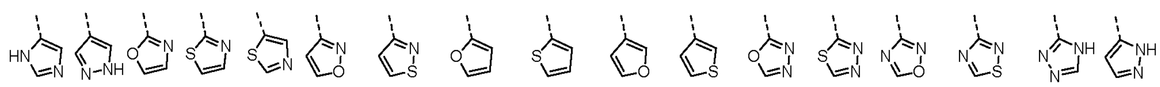

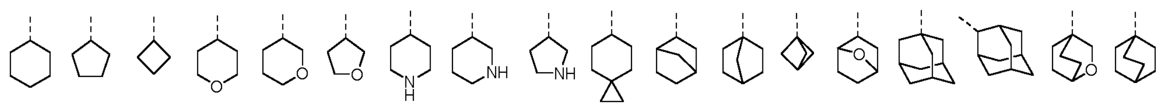

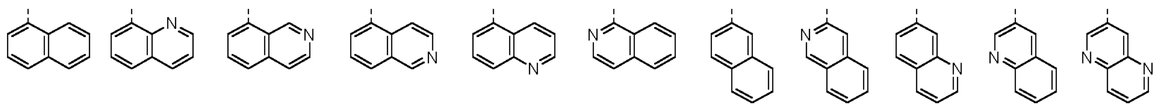

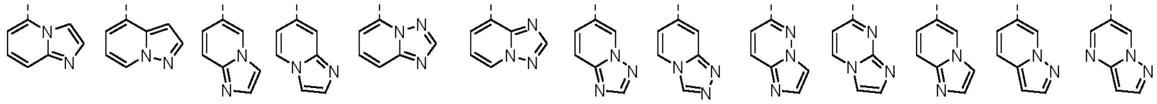

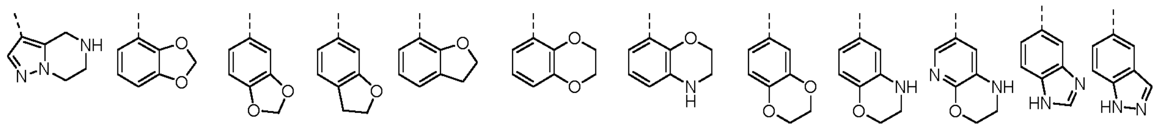

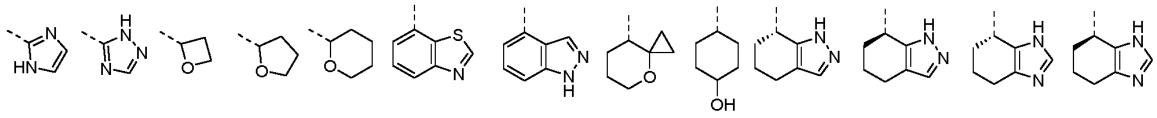

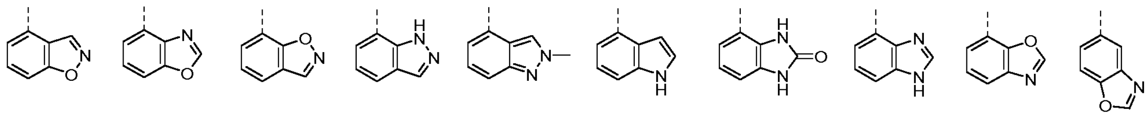,

more preferably, $R_7$ is selected from the following groups:

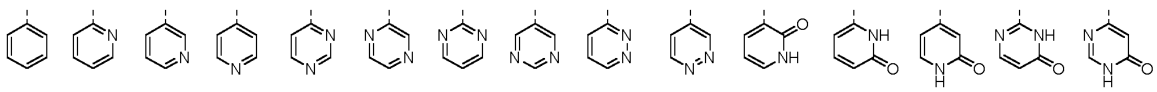

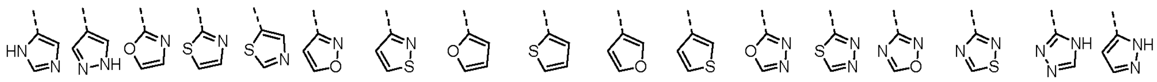

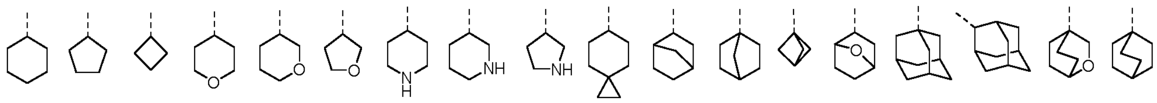

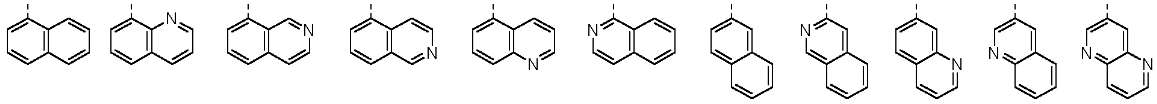

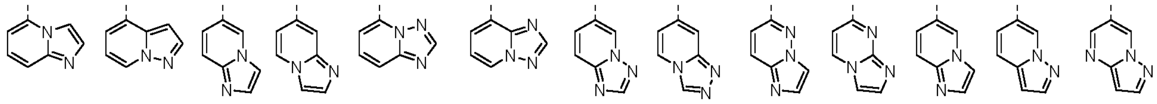

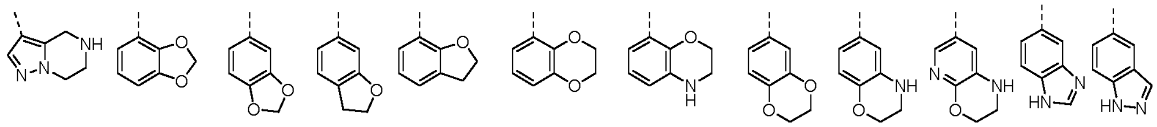

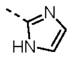 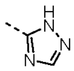 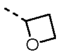 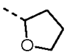 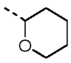 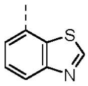 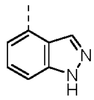 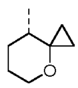 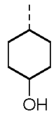 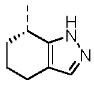 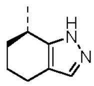 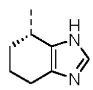 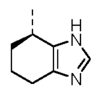 ,

$R_7$ is optionally substituted with one or more $R_{7a}$, wherein each $R_{7a}$ is independently selected from oxo, halogen, $C_1$-$C_6$ alkyl, cycloalkyl having 3-10 ring atoms, $C_1$-$C_6$ alkoxy, cycloalkyloxy having 3-6 ring atoms, cyano, amino, hydroxyl, $C_2$-$C_6$ alkenyl, $C_2$-$C_6$ alkynyl, sulfonyl, acyl, carboxyl, heterocyclyl having 3-12 ring atoms, heteroaryl having 5-10 ring atoms, and ureido; preferably, $R_{7a}$ is each independently selected from oxo, halogen, $C_1$-$C_4$ alkyl, cycloalkyl having 3-6 ring atoms, $C_1$-$C_4$ alkoxy, cycloalkyloxy having 3-4 ring atoms, cyano, amino, hydroxyl, $C_2$-$C_4$ alkenyl, $C_2$-$C_4$ alkynyl, sulfonyl, acyl, carboxyl, heterocyclyl having 3-6 ring atoms, heteroaryl having 5-6 ring atoms, and ureido; more preferably, $R_{7a}$ is each independently selected from fluorine, chlorine, bromine, hydroxyl, cyano, methoxy, methyl, cyclopropyloxy, and cyclopropyl; most preferably, $R_{7a}$ is each independently selected from fluorine, chlorine, bromine, hydroxyl, cyano, methoxy, methyl, cyclopropyloxy;

optionally, $R_{7a}$ is further substituted with one or more $R_{7b}$, wherein $R_{7b}$ is independently selected from halogen, $C_1$-$C_6$ alkyl, $C_1$-$C_6$ alkoxy, hydroxyl, amino, $C_2$-$C_6$ alkenyl, $C_2$-$C_6$ alkynyl, $C_1$-$C_6$ alkylamino, di($C_1$-$C_6$)alkylamino, sulfonyl, heterocyclyl having 3-6 ring atoms, and heteroaryl having 5-6 ring atoms; preferably, each $R_{7b}$ is independently selected from halogen, $C_1$-$C_3$ alkyl, $C_1$-$C_3$ alkoxy, hydroxyl, amino, $C_2$-$C_4$ alkenyl, $C_2$-$C_4$ alkynyl, $C_1$-$C_3$ alkylamino, di($C_1$-$C_3$)alkylamino, sulfonyl, heterocyclyl having 3-6 ring atoms, and heteroaryl having 5-6 ring atoms; more preferably, each $R_{7b}$ is independently selected from fluorine, chlorine, and bromine;

when $X_1$ is methylene, $R_7$ is not substituted or unsubstituted aryl.

**14.** The compound or the stereoisomer, the tautomer, the solvate, the hydrate, the prodrug, the stable isotopic derivative, or the pharmaceutically acceptable salt thereof according to any one of claims 1-13, wherein

$R_2$ is selected from hydrogen, halogen, $C_1$-$C_6$ alkyl, $C_1$-$C_6$ alkoxy, $C_2$-$C_6$ alkenyl, $C_2$-$C_6$ alkynyl, cycloalkyl having 3-10 ring atoms, cycloalkyloxy having 3-10 ring atoms, heterocyclyl having 4-10 ring atoms, heterocyclyloxy having 4-10 ring atoms, aryl having 6-10 ring atoms, heteroaryl having 5-10 ring atoms, heteroaryloxy having 5-10 ring atoms, cyano, amino, acyl, sulfonyl, and oxo; preferably, $R_2$ is selected from hydrogen, halogen, $C_1$-$C_4$ alkyl, $C_1$-$C_4$ alkoxy, $C_2$-$C_4$ alkenyl, $C_2$-$C_4$ alkynyl, cycloalkyl having 3-6 ring atoms, cycloalkyloxy having 3-6 ring atoms, heterocyclyl having 5-6 ring atoms, heterocyclyloxy having 5-6 ring atoms, aryl having 6-10 ring atoms, heteroaryl having 5-6 ring atoms, heteroaryloxy having 5-6 ring atoms, cyano, amino, acyl, sulfonyl, and oxo; more preferably, $R_2$ is selected from hydrogen, methyl, ethyl, n-propyl, isopropyl, cyano, methoxy, and oxo;

optionally, $R_2$ is further substituted with one or more $R_{2a}$, wherein each $R_{2a}$ is independently selected from halogen, $C_1$-$C_6$ alkyl, heterocyclyl having 4-10 ring atoms, heterocyclyloxy having 4-10 ring atoms, amino, heteroaryl having 5-10 ring atoms, cycloalkyl having 3-10 ring atoms, aryl having 6-10 ring atoms, $C_1$-$C_6$ alkylamino, di($C_1$-$C_6$)alkylamino, hydroxyl, sulfonyl, $C_1$-$C_6$ alkoxy, cyano, $C_2$-$C_6$ alkenyl, $C_2$-$C_6$ alkynyl, and oxo; preferably, each $R_{2a}$ is independently selected from halogen, $C_1$-$C_4$ alkyl, heterocyclyl having 5-6 ring atoms, heterocyclyloxy having 5-6 ring atoms, amino, heteroaryl having 5-6 ring atoms, cycloalkyl having 3-6 ring atoms, aryl having 6-10 ring atoms, $C_1$-$C_3$ alkylamino, di($C_1$-$C_3$)alkylamino, hydroxyl, sulfonyl, $C_1$-$C_4$ alkoxy, cyano, $C_2$-$C_4$ alkenyl, $C_2$-$C_4$ alkynyl, and oxo; more preferably, each $R_{2a}$ is independently selected from methyl, chlorine, fluorine, phenyl, cyclopropyl, oxo, amino, methylamino, dimethylamino, methoxy, hydroxyl, pyridinyl, pyrazolyl, and pyrimidinyl; most preferably, each $R_{2a}$ is independently selected from methyl, chlorine, fluorine, phenyl, cyclopropyl, oxo, amino, methylamino, dimethylamino, methoxy, hydroxyl, pyridinyl, and pyrazolyl;

optionally, $R_{2a}$ is further substituted with one or more $R_{2b}$, wherein each $R_{2b}$ is independently selected from $C_1$-$C_3$ alkyl substituted with 0-3 halogens, $C_1$-$C_3$ alkoxy substituted with 0-3 halogens, halogen, hydroxyl, unsubstituted 5-6 membered heterocyclyl or 5-6 membered heterocyclyl substituted with $C_1$-$C_3$ alkyl, cyano, and oxo, preferably $C_1$-$C_3$ alkyl, $C_1$-$C_3$ alkoxy, halogen, hydroxyl, and oxo; more preferably, each $R_{2b}$ is independently selected from methoxy and methyl.

**15.** The compound or the stereoisomer, the tautomer, the solvate, the hydrate, the prodrug, the stable isotopic derivative, or the pharmaceutically acceptable salt thereof according to any one of claims 3-14, wherein:

$R_8$ and $R_9$ are each independently selected from hydrogen, halogen, $C_1$-$C_6$ alkyl, $C_1$-$C_6$ alkoxy, $C_2$-$C_6$ alkenyl, $C_2$-$C_6$ alkynyl, cycloalkyl having 3-10 ring atoms, cycloalkyloxy having 3-10 ring atoms, heterocyclyl having 4-10 ring atoms, heterocyclyloxy having 4-10 ring atoms, aryl having 6-10 ring atoms, heteroaryl having 5-10 ring atoms, heteroaryloxy having 5-10 ring atoms, cyano, amino, acyl, sulfonyl, and oxo, or adjacent $R_8$ and $R_9$ form a 5-6 membered carbocyclic ring, heterocyclic ring, or heteroaromatic ring; preferably, $R_8$ and $R_9$ are each

independently selected from hydrogen, halogen, $C_1$-$C_4$ alkyl, $C_1$-$C_4$ alkoxy, $C_2$-$C_4$ alkenyl, $C_2$-$C_4$ alkynyl, cycloalkyl having 3-6 ring atoms, cycloalkyloxy having 3-6 ring atoms, heterocyclyl having 5-6 ring atoms, heterocyclyloxy having 5-6 ring atoms, aryl having 6 ring atoms, heteroaryl having 5-6 ring atoms, heteroaryloxy having 5-6 ring atoms, cyano, amino, acyl, sulfonyl, and oxo, or adjacent $R_8$ and $R_9$ form a 5-6 membered heterocyclic ring or heteroaromatic ring; more preferably, $R_8$ and $R_9$ are each independently selected from hydrogen, methyl, ethyl, n-propyl, isopropyl, cyano, methoxy, oxo, cyclopropyl, tetrahydropyrrolyl, tetrahydrofuranyl, phenyl, and propenyl, or adjacent $R_8$ and $R_9$ form

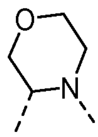

or

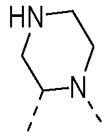

;

optionally, $R_8$ is further substituted with one or more $R_{8a}$; optionally, $R_9$ is further substituted with one or more $R_{9a}$; optionally, the carbocyclic ring, heterocyclic ring, or heteroaromatic ring formed by adjacent $R_8$ and $R_9$ is further substituted with one or more $R_{8a}$ or $R_{9a}$;

$R_{8a}$ or $R_{9a}$ is each independently selected from halogen, $C_1$-$C_6$ alkyl, heterocyclyl having 4-10 ring atoms, heterocyclyloxy having 4-10 ring atoms, amino, heteroaryl having 5-10 ring atoms, cycloalkyl having 3-10 ring atoms, aryl having 6-10 ring atoms, $C_1$-$C_6$ alkylamino, di($C_1$-$C_6$)alkylamino, hydroxyl, sulfonyl, $C_1$-$C_6$ alkoxy, cyano, $C_2$-$C_6$ alkenyl, $C_2$-$C_6$ alkynyl, and oxo; preferably, each $R_{8a}$ or each $R_{9a}$ is independently selected from halogen, $C_1$-$C_4$ alkyl, heterocyclyl having 5-6 ring atoms, heterocyclyloxy having 5-6 ring atoms, amino, heteroaryl having 5-6 ring atoms, cycloalkyl having 3-6 ring atoms, aryl having 6 ring atoms, $C_1$-$C_3$ alkylamino, di($C_1$-$C_3$)alkylamino, hydroxyl, sulfonyl, $C_1$-$C_4$ alkoxy, cyano, $C_2$-$C_4$ alkenyl, $C_2$-$C_4$ alkynyl, and oxo; more preferably, each $R_{8a}$ or each $R_{9a}$ is independently selected from methyl, chlorine, fluorine, bromine, phenyl, cyclopropyl, oxo, amino, methylamino, dimethylamino, methoxy, hydroxyl, pyridinyl, pyrazolyl, sulfonyl, morpholinyl, tetrahydrofuranyl, isoxazolyl, and pyrimidinyl; most preferably, each $R_{8a}$ or each $R_{9a}$ is independently selected from methyl, chlorine, fluorine, bromine, phenyl, cyclopropyl, oxo, amino, methylamino, dimethylamino, methoxy, hydroxyl, pyridinyl, pyrazolyl, sulfonyl, morpholinyl, tetrahydrofuranyl, and isoxazolyl;

optionally, $R_{8a}$ is further substituted with one or more $R_{8b}$; optionally, $R_{9a}$ is further substituted with one or more $R_{9b}$; $R_{8b}$ or $R_{9b}$ is each independently selected from $C_1$-$C_3$ alkyl substituted with 0-3 halogens, $C_1$-$C_3$ alkoxy substituted with 0-3 halogens, halogen, hydroxyl, unsubstituted 5-6 membered heterocyclyl or 5-6 membered heterocyclyl substituted with $C_1$-$C_3$ alkyl, cyano, and oxo, preferably $C_1$-$C_3$ alkyl, $C_1$-$C_3$ alkoxy, halogen, hydroxyl, and oxo; preferably, $R_{8b}$ or $R_{9b}$ is each independently selected from methoxy and methyl.

**16.** The compound or the stereoisomer, the tautomer, the solvate, the hydrate, the prodrug, the stable isotopic derivative, or the pharmaceutically acceptable salt thereof according to any one of claims 3-15, wherein:

w is $CR_3$, x is $CR_4$, y is $CR_5$, and z is $CR_6$ or N;

$R_3$ is selected from hydrogen, $C_1$-$C_6$ alkyl, $C_2$-$C_6$ alkenyl, $C_2$-$C_6$ alkynyl, $C_1$-$C_6$ alkoxy, sulfonyl, halogen, cycloalkyl having 3-10 ring atoms, cyano, amino, acyl, hydroxyl, heteroaryl having 5-10 ring atoms, heteroaryloxy having 5-10 ring atoms, heterocyclyl having 5-10 ring atoms, heterocyclyloxy having 5-10 ring atoms, and cycloalkyloxy having 3-10 ring atoms;

optionally, $R_3$ is further substituted with one or more $R_{3a}$, wherein each $R_{3a}$ is independently selected from $C_1$-$C_6$ alkyl, $C_2$-$C_6$ alkenyl, $C_2$-$C_6$ alkynyl, $C_1$-$C_6$ alkoxy, $C_1$-$C_6$ alkylamino, di($C_1$-$C_6$ alkyl)amino, halogen, sulfonyl, cycloalkyl having 3-10 ring atoms, cyano, amino, acyl, hydroxyl, heteroaryl having 5-10 ring atoms, heteroaryloxy having 5-10 ring atoms, cycloalkyloxy having 3-10 ring atoms, heterocyclyl having 5-10 ring atoms, and heterocyclyloxy having 5-10 ring atoms;

optionally, $R_{3a}$ is further substituted with one or more $R_{3b}$, wherein each $R_{3b}$ is independently selected from $C_1$-$C_3$ alkyl, halogen, hydroxyl, $C_1$-$C_3$ alkoxy, and amino;

$R_4$ is selected from hydrogen, $C_1$-$C_6$ alkyl, $C_2$-$C_6$ alkenyl, $C_2$-$C_6$ alkynyl, $C_1$-$C_6$ alkoxy, $C_1$-$C_6$ alkylthio, sulfonyl,

halogen, cycloalkyl having 3-10 ring atoms, cyano, amino, and acyl;
optionally, $R_4$ is further substituted with one or more $R_{4a}$, wherein each $R_{4a}$ is independently selected from $C_1$-$C_6$ alkyl, halogen, amino, $C_1$-$C_6$ alkylamino, and di($C_1$-$C_6$)alkylamino;
$R_5$ is selected from halogen, cyano, amino, hydroxyl, $C_1$-$C_6$ alkyl, $C_2$-$C_6$ alkenyl, $C_2$-$C_6$ alkynyl, $C_1$-$C_6$ alkoxy, cycloalkyl having 3-10 ring atoms, cycloalkyloxy having 3-10 ring atoms, heterocyclyl having 4-10 ring atoms, and heteroaryl having 5-10 ring atoms;
optionally, $R_5$ is further substituted with one or more $R_{5a}$, wherein each $R_{5a}$ is independently selected from $C_1$-$C_6$ alkyl, $C_1$-$C_6$ alkoxy, halogen, cycloalkyl having 3-10 ring atoms, cyano, amino, and hydroxyl;
$R_6$ is selected from hydrogen, $C_1$-$C_6$ alkyl, $C_2$-$C_6$ alkenyl, $C_2$-$C_6$ alkynyl, $C_1$-$C_6$ alkoxy, $C_1$-$C_6$ alkylthio, sulfonyl, halogen, cycloalkyl having 3-10 ring atoms, cyano, amino, and acyl;
optionally, $R_6$ is further substituted with one or more $R_{6a}$, wherein each $R_{6a}$ is independently selected from $C_1$-$C_6$ alkyl, halogen, amino, $C_1$-$C_6$ alkylamino, and di($C_1$-$C_6$)alkylamino;
$R_1$ is -$X_1$-$R_7$, wherein $X_1$ is a bond or methylene;
optionally, $X_1$ is substituted with one or more $X_{1a}$, wherein each $X_{1a}$ is independently selected from halogen and $C_1$-$C_3$ alkyl;
$R_7$ is selected from cycloalkyl having 4-10 ring atoms, aryl having 6-12 ring atoms, heteroaryl having 5-12 ring atoms, and heterocyclyl having 3-12 ring atoms;
$R_7$ is optionally substituted with one or more $R_{7a}$, wherein each $R_{7a}$ is independently selected from oxo, halogen, $C_1$-$C_6$ alkyl, cycloalkyl having 3-10 ring atoms, $C_1$-$C_6$ alkoxy, cycloalkyloxy having 3-6 ring atoms, cyano, amino, hydroxyl, $C_2$-$C_6$ alkenyl, $C_2$-$C_6$ alkynyl, sulfonyl, acyl, carboxyl, heterocyclyl having 3-12 ring atoms, heteroaryl having 5-10 ring atoms, and ureido;
optionally, $R_{7a}$ is further substituted with one or more $R_{7b}$, wherein $R_{7b}$ is independently selected from halogen, $C_1$-$C_6$ alkyl, $C_1$-$C_6$ alkoxy, hydroxyl, amino, $C_2$-$C_6$ alkenyl, $C_2$-$C_6$ alkynyl, $C_1$-$C_6$ alkylamino, di($C_1$-$C_6$)alkylamino, sulfonyl, heterocyclyl having 3-6 ring atoms, and heteroaryl having 5-6 ring atoms;
when $X_1$ is methylene, $R_7$ is not substituted or unsubstituted aryl;
$R_2$ is selected from hydrogen, halogen, $C_1$-$C_6$ alkyl, $C_1$-$C_6$ alkoxy, $C_2$-$C_6$ alkenyl, $C_2$-$C_6$ alkynyl, cycloalkyl having 3-10 ring atoms, cycloalkyloxy having 3-10 ring atoms, heterocyclyl having 4-10 ring atoms, heterocyclyloxy having 4-10 ring atoms, aryl having 6-10 ring atoms, heteroaryl having 5-10 ring atoms, heteroaryloxy having 5-10 ring atoms, cyano, amino, acyl, sulfonyl, and oxo;
optionally, $R_2$ is further substituted with one or more $R_{2a}$, wherein each $R_{2a}$ is independently selected from halogen, $C_1$-$C_6$ alkyl, heterocyclyl having 4-10 ring atoms, heterocyclyloxy having 4-10 ring atoms, amino, heteroaryl having 5-10 ring atoms, cycloalkyl having 3-10 ring atoms, aryl having 6-10 ring atoms, $C_1$-$C_6$ alkylamino, di($C_1$-$C_6$)alkylamino, hydroxyl, sulfonyl, $C_1$-$C_6$ alkoxy, cyano, $C_2$-$C_6$ alkenyl, $C_2$-$C_6$ alkynyl, and oxo;
optionally, $R_{2a}$ is further substituted with one or more $R_{2b}$, wherein each $R_{2b}$ is independently selected from $C_1$-$C_3$ alkyl substituted with 0-3 halogens, $C_1$-$C_3$ alkoxy substituted with 0-3 halogens, halogen, hydroxyl, unsubstituted 5-6 membered heterocyclyl or 5-6 membered heterocyclyl substituted with $C_1$-$C_3$ alkyl, cyano, and oxo, preferably $C_1$-$C_3$ alkyl, $C_1$-$C_3$ alkoxy, halogen, hydroxyl, and oxo;
$R_8$ and $R_9$ are each independently selected from hydrogen, halogen, $C_1$-$C_6$ alkyl, $C_1$-$C_6$ alkoxy, $C_2$-$C_6$ alkenyl, $C_2$-$C_6$ alkynyl, cycloalkyl having 3-10 ring atoms, cycloalkyloxy having 3-10 ring atoms, heterocyclyl having 4-10 ring atoms, heterocyclyloxy having 4-10 ring atoms, aryl having 6-10 ring atoms, heteroaryl having 5-10 ring atoms, heteroaryloxy having 5-10 ring atoms, cyano, amino, acyl, sulfonyl, and oxo, or adjacent $R_8$ and $R_9$ form a 5-6 membered carbocyclic ring, heterocyclic ring, or heteroaromatic ring;
optionally, $R_8$ is further substituted with one or more $R_{8a}$; optionally, $R_9$ is further substituted with one or more $R_{9a}$; optionally, the carbocyclic ring, heterocyclic ring, or heteroaromatic ring formed by adjacent $R_8$ and $R_9$ is further substituted with one or more $R_{8a}$ or $R_{9a}$;
$R_{8a}$ or $R_{9a}$ is each independently selected from halogen, $C_1$-$C_6$ alkyl, heterocyclyl having 4-10 ring atoms, heterocyclyloxy having 4-10 ring atoms, amino, heteroaryl having 5-10 ring atoms, cycloalkyl having 3-10 ring atoms, aryl having 6-10 ring atoms, $C_1$-$C_6$ alkylamino, di($C_1$-$C_6$)alkylamino, hydroxyl, sulfonyl, $C_1$-$C_6$ alkoxy, cyano, $C_2$-$C_6$ alkenyl, $C_2$-$C_6$ alkynyl, and oxo;
optionally, $R_{8a}$ is further substituted with one or more $R_{8b}$; optionally, $R_{9a}$ is further substituted with one or more $R_{9b}$; $R_{8b}$ or $R_{9b}$ is each independently selected from $C_1$-$C_3$ alkyl substituted with 0-3 halogens, $C_1$-$C_3$ alkoxy substituted with 0-3 halogens, halogen, hydroxyl, unsubstituted 5-6 membered heterocyclyl or 5-6 membered heterocyclyl substituted with $C_1$-$C_3$ alkyl, cyano, and oxo, preferably $C_1$-$C_3$ alkyl, $C_1$-$C_3$ alkoxy, halogen, hydroxyl, and oxo.

**17.** The compound or the stereoisomer, the tautomer, the solvate, the hydrate, the prodrug, the stable isotopic derivative, or the pharmaceutically acceptable salt thereof according to any one of claims 3-16, wherein:

w is $CR_3$, x is $CR_4$, y is $CR_5$, and z is $CR_6$ or N;
$R_3$ is selected from hydrogen, $C_1$-$C_4$ alkyl, $C_2$-$C_4$ alkenyl, $C_2$-$C_4$ alkynyl, $C_1$-$C_4$ alkoxy, halogen, cycloalkyl having 3-6 ring atoms, cyano, amino, acyl, hydroxyl, heteroaryl having 5-6 ring atoms, heteroaryloxy having 5-6 ring atoms, heterocyclyl having 5-6 ring atoms, heterocyclyloxy having 5-6 ring atoms, and cycloalkyloxy having 3-6 ring atoms;
optionally, $R_3$ is further substituted with one or more $R_{3a}$, wherein each $R_{3a}$ is independently selected from $C_1$-$C_3$ alkyl, $C_2$-$C_4$ alkenyl, $C_2$-$C_4$ alkynyl, $C_1$-$C_3$ alkoxy, $C_1$-$C_3$ alkylamino, di($C_1$-$C_3$ alkyl)amino, halogen, sulfonyl, cycloalkyl having 3-6 ring atoms, cyano, amino, acyl, hydroxyl, heteroaryl having 5-6 ring atoms, heteroaryloxy having 5-6 ring atoms, cycloalkyloxy having 3-6 ring atoms, heterocyclyl having 5-6 ring atoms, and heterocyclyloxy having 5-6 ring atoms;
optionally, $R_{3a}$ is further substituted with one or more $R_{3b}$, wherein each $R_{3b}$ is independently selected from $C_1$-$C_3$ alkyl, halogen, hydroxyl, $C_1$-$C_3$ alkoxy, and amino;
$R_4$ is selected from hydrogen, $C_1$-$C_4$ alkyl, $C_2$-$C_4$ alkenyl, $C_2$-$C_4$ alkynyl, $C_1$-$C_4$ alkoxy, $C_1$-$C_4$ alkylthio, sulfonyl, halogen, cycloalkyl having 3-6 ring atoms, cyano, amino, and acyl;
optionally, $R_4$ is further substituted with one or more $R_{4a}$, wherein each $R_{4a}$ is independently selected from $C_1$-$C_3$ alkyl, halogen, amino, $C_1$-$C_3$ alkylamino, and di($C_1$-$C_3$)alkylamino;
$R_5$ is selected from halogen, $C_1$-$C_6$ alkyl, $C_1$-$C_6$ alkoxy, cycloalkyl having 3-6 ring atoms, cycloalkyloxy having 3-6 ring atoms, heterocyclyl having 4-6 ring atoms, and heteroaryl having 5-6 ring atoms;
optionally, $R_5$ is further substituted with one or more $R_{5a}$, wherein each $R_{5a}$ is independently selected from $C_1$-$C_6$ alkyl, halogen, cyano, and cycloalkyl having 3-6 ring atoms;
$R_6$ is selected from hydrogen, $C_1$-$C_4$ alkyl, $C_2$-$C_4$ alkenyl, $C_2$-$C_4$ alkynyl, $C_1$-$C_4$ alkoxy, $C_1$-$C_4$ alkylthio, sulfonyl, halogen, cycloalkyl having 3-6 ring atoms, cyano, amino, and acyl;
optionally, $R_6$ is further substituted with one or more $R_{6a}$, wherein each $R_{6a}$ is independently selected from $C_1$-$C_3$ alkyl, halogen, amino, $C_1$-$C_3$ alkylamino, and di($C_1$-$C_3$)alkylamino;
$R_1$ is $-X_1-R_7$, wherein $X_1$ is a bond or methylene;
optionally, $X_1$ is substituted with one or more $X_{1a}$, wherein $X_{1a}$ is methyl;
$R_7$ is selected from the following groups:

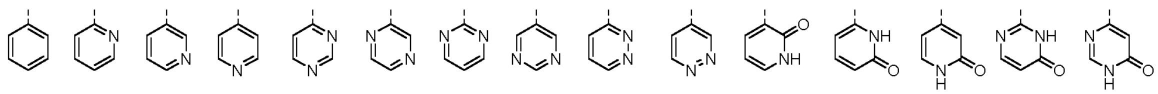

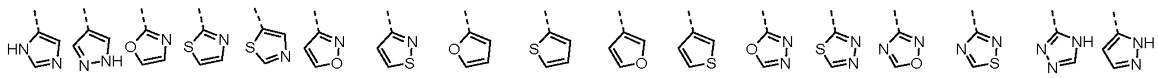

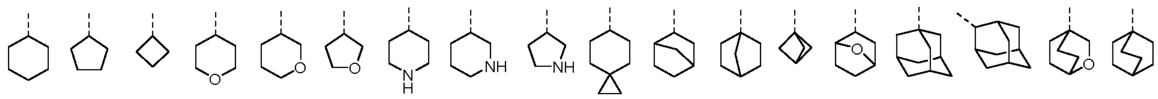

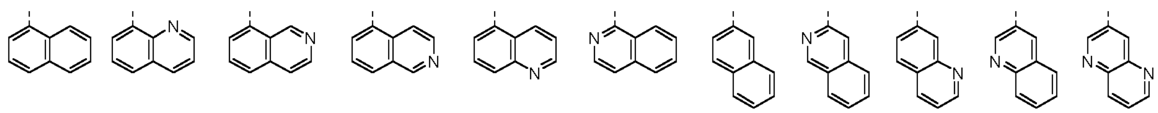

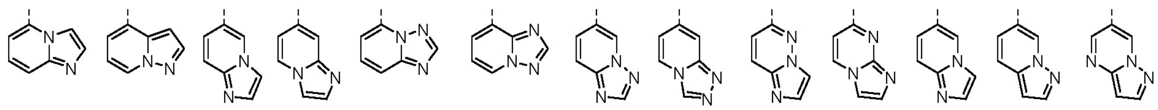

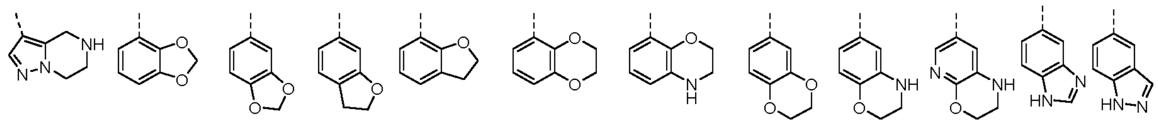

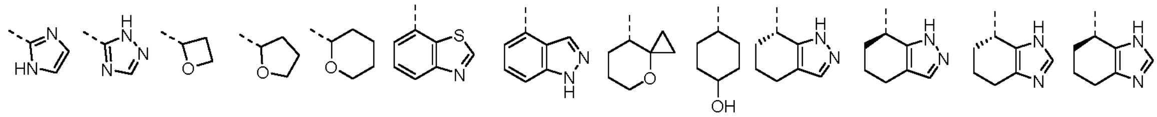

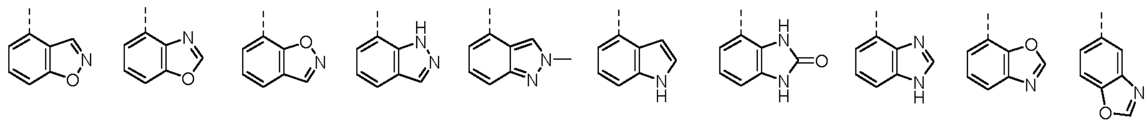,

preferably, $R_7$ is selected from the following groups:

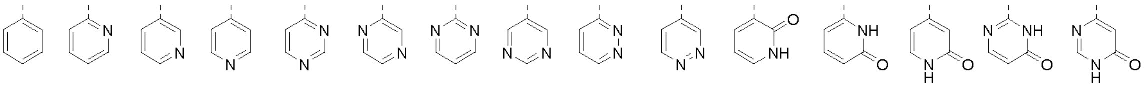

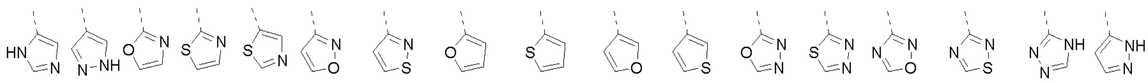

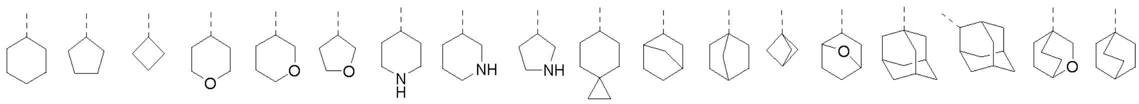

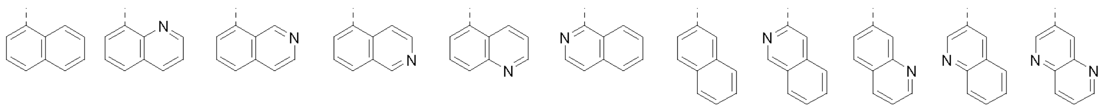

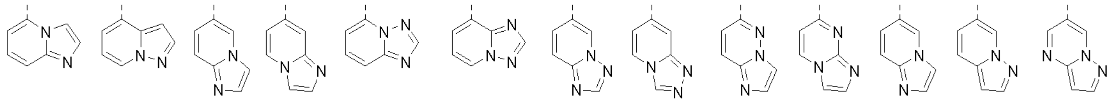

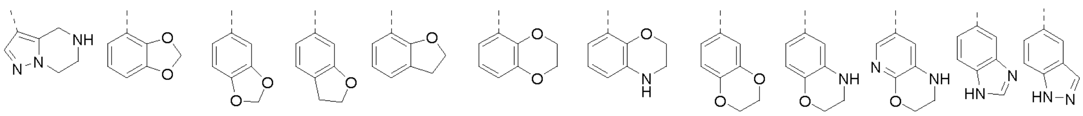

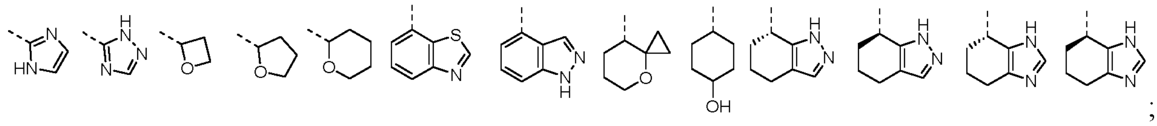;

$R_7$ is optionally substituted with one or more $R_{7a}$, wherein each $R_{7a}$ is independently selected from oxo, halogen, $C_1$-$C_4$ alkyl, cycloalkyl having 3-6 ring atoms, $C_1$-$C_4$ alkoxy, cycloalkyloxy having 3-4 ring atoms, cyano, amino, hydroxyl, $C_2$-$C_4$ alkenyl, $C_2$-$C_4$ alkynyl, sulfonyl, acyl, carboxyl, heterocyclyl having 3-6 ring atoms, heteroaryl having 5-6 ring atoms, and ureido;

optionally, $R_{7a}$ is further substituted with one or more $R_{7b}$, wherein $R_{7b}$ is each independently selected from halogen, $C_1$-$C_3$ alkyl, $C_1$-$C_3$ alkoxy, hydroxyl, amino, $C_2$-$C_4$ alkenyl, $C_2$-$C_4$ alkynyl, $C_1$-$C_3$ alkylamino, di($C_1$-$C_3$) alkylamino, sulfonyl, heterocyclyl having 3-6 ring atoms, and heteroaryl having 5-6 ring atoms;

when $X_1$ is methylene, $R_7$ is not substituted or unsubstituted aryl;

$R_2$ is selected from hydrogen, halogen, $C_1$-$C_4$ alkyl, $C_1$-$C_4$ alkoxy, $C_2$-$C_4$ alkenyl, $C_2$-$C_4$ alkynyl, cycloalkyl having 3-6 ring atoms, cycloalkyloxy having 3-6 ring atoms, heterocyclyl having 5-6 ring atoms, heterocyclyloxy having 5-6 ring atoms, aryl having 6-10 ring atoms, heteroaryl having 5-6 ring atoms, heteroaryloxy having 5-6 ring atoms, cyano, amino, acyl, sulfonyl, and oxo;

optionally, $R_2$ is further substituted with one or more $R_{2a}$, wherein each $R_{2a}$ is independently selected from halogen, $C_1$-$C_4$ alkyl, heterocyclyl having 5-6 ring atoms, heterocyclyloxy having 5-6 ring atoms, amino,

heteroaryl having 5-6 ring atoms, cycloalkyl having 3-6 ring atoms, aryl having 6-10 ring atoms, $C_1$-$C_3$ alkylamino, di($C_1$-$C_3$)alkylamino, hydroxyl, sulfonyl, $C_1$-$C_4$ alkoxy, cyano, $C_2$-$C_4$ alkenyl, $C_2$-$C_4$ alkynyl, and oxo;
optionally, $R_{2a}$ is further substituted with one or more $R_{2b}$, wherein each $R_{2b}$ is independently selected from methoxy, methyl, trifluoromethyl, difluoromethyl, difluoromethoxy, *N*-methylpiperazinyl, and cyano, preferably methoxy and methyl;
$R_8$ and $R_9$ are each independently selected from hydrogen, halogen, $C_1$-$C_4$ alkyl, $C_1$-$C_4$ alkoxy, $C_2$-$C_4$ alkenyl, $C_2$-$C_4$ alkynyl, cycloalkyl having 3-6 ring atoms, cycloalkyloxy having 3-6 ring atoms, heterocyclyl having 5-6 ring atoms, heterocyclyloxy having 5-6 ring atoms, aryl having 6 ring atoms, heteroaryl having 5-6 ring atoms, heteroaryloxy having 5-6 ring atoms, cyano, amino, acyl, sulfonyl, and oxo, or adjacent $R_8$ and $R_9$ form a 5-6 membered heterocyclic ring or heteroaromatic ring;
optionally, $R_8$ is further substituted with one or more $R_{8a}$; optionally, $R_9$ is further substituted with one or more $R_{9a}$; optionally, the heterocyclic ring or heteroaromatic ring formed by adjacent $R_8$ and $R_9$ is further substituted with one or more $R_{8a}$ or $R_{9a}$;
$R_{8a}$ or $R_{9a}$ is each independently selected from halogen, $C_1$-$C_4$ alkyl, heterocyclyl having 5-6 ring atoms, heterocyclyloxy having 5-6 ring atoms, amino, heteroaryl having 5-6 ring atoms, cycloalkyl having 3-6 ring atoms, aryl having 6 ring atoms, $C_1$-$C_3$ alkylamino, di($C_1$-$C_3$)alkylamino, hydroxyl, sulfonyl, $C_1$-$C_4$ alkoxy, cyano, $C_2$-$C_4$ alkenyl, $C_2$-$C_4$ alkynyl, and oxo;
optionally, $R_{8a}$ is further substituted with one or more $R_{8b}$; optionally, $R_{9a}$ is further substituted with one or more $R_{9b}$; $R_{8b}$ or $R_{9b}$ is each independently selected from methoxy, methyl, trifluoromethyl, difluoromethyl, difluoromethoxy, *N*-methylpiperazinyl, and cyano, preferably methoxy and methyl.

**18.** The compound or the stereoisomer, the tautomer, the solvate, the hydrate, the prodrug, the stable isotopic derivative, or the pharmaceutically acceptable salt thereof according to any one of claims 3-17, wherein:

w is $CR_3$, x is $CR_4$, y is $CR_5$, and z is $CR_6$ or N;
$R_3$ is selected from hydrogen, methyl, ethyl, *n*-propyl, isopropyl, methoxy, ethoxy, propoxy, and piperidinyloxy;
optionally, $R_3$ is further substituted with one or more $R_{3a}$, wherein each $R_{3a}$ is independently selected from methyl and dimethylamino;
optionally, $R_{3a}$ is further substituted with one or more $R_{3b}$, wherein each $R_{3b}$ is independently selected from $C_1$-$C_3$ alkyl, halogen, hydroxyl, $C_1$-$C_3$ alkoxy, and amino;
$R_4$ is selected from hydrogen, methyl, ethyl, *n*-propyl, isopropyl, fluorine, chlorine, bromine, and cyano;
optionally, $R_4$ is further substituted with one or more $R_{4a}$, wherein each $R_{4a}$ is independently selected from $C_1$-$C_3$ alkyl, halogen, amino, $C_1$-$C_3$ alkylamino, and di($C_1$-$C_3$)alkylamino;
$R_5$ is selected from chlorine, fluorine, bromine, iodine, cyano, ethyl, *n*-propyl, isopropyl, methyl, cyclopropyl, methoxy, and cyclopropyloxy;
optionally, $R_5$ is further substituted with one or more $R_{5a}$, wherein each $R_{5a}$ is independently selected from fluorine and cyano;
$R_6$ is selected from hydrogen, fluorine, chlorine, methyl, ethyl, *n*-propyl, isopropyl, cyclopropyl, methoxy, cyclopropyloxy, and cyano;
optionally, $R_6$ is further substituted with one or more $R_{6a}$, wherein each $R_{6a}$ is independently selected from $C_1$-$C_3$ alkyl, halogen, amino, $C_1$-$C_3$ alkylamino, and di($C_1$-$C_3$)alkylamino;
$R_1$ is $-X_1-R_7$, wherein $X_1$ is a bond or methylene;
optionally, $X_1$ is substituted with one or more $X_{1a}$, wherein $X_{1a}$ is methyl;
$R_7$ is as defined in claim 17;
$R_7$ is optionally substituted with one or more $R_{7a}$, wherein each $R_{7a}$ is independently selected from fluorine, chlorine, bromine, hydroxyl, cyano, methoxy, methyl, cyclopropyloxy, and cyclopropyl, preferably selected from fluorine, chlorine, bromine, hydroxyl, cyano, methoxy, methyl, and cyclopropyloxy;
optionally, $R_{7a}$ is further substituted with one or more $R_{7b}$, wherein $R_{7b}$ is independently selected from fluorine, chlorine, and bromine;
when $X_1$ is methylene, $R_7$ is not substituted or unsubstituted aryl;
$R_2$ is selected from hydrogen, methyl, ethyl, *n*-propyl, isopropyl, cyano, methoxy, and oxo;
optionally, $R_2$ is further substituted with one or more $R_{2a}$, wherein each $R_{2a}$ is independently selected from methyl, chlorine, fluorine, phenyl, cyclopropyl, oxo, amino, methylamino, dimethylamino, methoxy, hydroxyl, pyridinyl, pyrazolyl, and pyrimidinyl, preferably selected from methyl, chlorine, fluorine, phenyl, cyclopropyl, oxo, amino, methylamino, dimethylamino, methoxy, hydroxyl, pyridinyl, and pyrazolyl;
optionally, $R_{2a}$ is further substituted with one or more $R_{2b}$, wherein each $R_{2b}$ is independently selected from methoxy, methyl, trifluoromethyl, difluoromethyl, difluoromethoxy, *N*-methylpiperazinyl, and cyano, preferably methoxy and methyl;

$R_8$ and $R_9$ are each independently selected from hydrogen, methyl, ethyl, *n*-propyl, isopropyl, cyano, methoxy, oxo, cyclopropyl, tetrahydropyrrolyl, tetrahydrofuranyl, phenyl, and propenyl, or adjacent $R_8$ and $R_9$ form

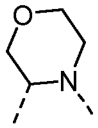 or 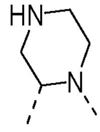;

optionally, $R_8$ is further substituted with one or more $R_{8a}$; optionally, $R_9$ is further substituted with one or more $R_{9a}$; optionally,

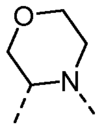 or 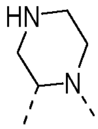

formed by adjacent $R_8$ and $R_9$ is further substituted with one or more $R_{8a}$ or $R_{9a}$;
$R_{8a}$ or $R_{9a}$ is each independently selected from methyl, chlorine, fluorine, bromine, phenyl, cyclopropyl, oxo, amino, methylamino, dimethylamino, methoxy, hydroxyl, pyridinyl, pyrazolyl, sulfonyl, morpholinyl, tetrahydrofuranyl, isoxazolyl, and pyrimidinyl; preferably, each $R_{8a}$ or each $R_{9a}$ is independently selected from methyl, chlorine, fluorine, bromine, phenyl, cyclopropyl, oxo, amino, methylamino, dimethylamino, methoxy, hydroxyl, pyridinyl, pyrazolyl, sulfonyl, morpholinyl, tetrahydrofuranyl, and isoxazolyl;
optionally, $R_{8a}$ is further substituted with one or more $R_{8b}$; optionally, $R_{9a}$ is further substituted with one or more $R_{9b}$; $R_{8b}$ or $R_{9b}$ is each independently selected from methoxy, methyl, trifluoromethyl, difluoromethyl, difluoromethoxy, *N*-methylpiperazinyl, and cyano, preferably methoxy and methyl.

**19.** The compound or the stereoisomer, the tautomer, the solvate, the hydrate, the prodrug, the stable isotopic derivative, or the pharmaceutically acceptable salt thereof according to any one of claims 1 and 3-5, wherein the compound has optionally the following structures:

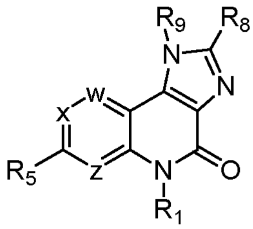,

wherein x, w, z, $R_1$, $R_5$, $R_8$, and $R_9$ are as defined in any one of claims 1 and 3-5, and $R_4$ is not H.

**20.** The compound or the stereoisomer, the tautomer, the solvate, the hydrate, the prodrug, the stable isotopic derivative, or the pharmaceutically acceptable salt thereof according to claim 19, wherein:

w is $CR_3$, and $R_3$ is selected from hydrogen, $C_1$-$C_6$ alkyl, $C_2$-$C_6$ alkenyl, $C_2$-$C_6$ alkynyl, $C_1$-$C_6$ alkoxy, sulfonyl, halogen, cycloalkyl having 3-10 ring atoms, cyano, amino, acyl, hydroxyl, heteroaryl having 5-10 ring atoms, heteroaryloxy having 5-10 ring atoms, heterocyclyl having 5-10 ring atoms, heterocyclyloxy having 5-10 ring atoms, and cycloalkyloxy having 3-10 ring atoms; preferably, $R_3$ is selected from hydrogen, $C_1$-$C_4$ alkyl, $C_2$-$C_4$ alkenyl, $C_2$-$C_4$ alkynyl, $C_1$-$C_4$ alkoxy, halogen, cycloalkyl having 3-6 ring atoms, cyano, amino, acyl, hydroxyl, heteroaryl having 5-6 ring atoms, heteroaryloxy having 5-6 ring atoms, heterocyclyl having 5-6 ring atoms, heterocyclyloxy having 5-6 ring atoms, and cycloalkyloxy having 3-6 ring atoms; more preferably, $R_3$ is selected from hydrogen, methyl, ethyl, *n*-propyl, isopropyl, methoxy, ethoxy, propoxy, and piperidinyloxy;
optionally, $R_3$ is further substituted with one or more $R_{3a}$, wherein each $R_{3a}$ is independently selected from $C_1$-$C_6$ alkyl, $C_2$-$C_6$ alkenyl, $C_2$-$C_6$ alkynyl, $C_1$-$C_6$ alkoxy, $C_1$-$C_6$ alkylamino, di($C_1$-$C_6$ alkyl)amino, halogen, sulfonyl, cycloalkyl having 3-10 ring atoms, cyano, amino, acyl, hydroxyl, heteroaryl having 5-10 ring atoms, heteroaryloxy having 5-10 ring atoms, cycloalkyloxy having 3-10 ring atoms, heterocyclyl having 5-10 ring atoms, and

heterocyclyloxy having 5-10 ring atoms; preferably, $R_{3a}$ is each independently selected from $C_1$-$C_3$ alkyl, $C_2$-$C_4$ alkenyl, $C_2$-$C_4$ alkynyl, $C_1$-$C_3$ alkoxy, $C_1$-$C_3$ alkylamino, di($C_1$-$C_3$ alkyl)amino, halogen, sulfonyl, cycloalkyl having 3-6 ring atoms, cyano, amino, acyl, hydroxyl, heteroaryl having 5-6 ring atoms, heteroaryloxy having 5-6 ring atoms, cycloalkyloxy having 3-6 ring atoms, heterocyclyl having 5-6 ring atoms, and heterocyclyloxy having 5-6 ring atoms; more preferably, $R_{3a}$ is each independently selected from methyl and dimethylamino; optionally, $R_{3a}$ is further substituted with one or more $R_{3b}$, wherein each $R_{3b}$ is independently selected from $C_1$-$C_3$ alkyl, halogen, hydroxyl, $C_1$-$C_3$ alkoxy, and amino.

**21.** The compound or the stereoisomer, the tautomer, the solvate, the hydrate, the prodrug, the stable isotopic derivative, or the pharmaceutically acceptable salt thereof according to any one of claims 19-20, wherein:

x is $CR_4$, and $R_4$ is selected from $C_1$-$C_6$ alkyl, $C_2$-$C_6$ alkenyl, $C_2$-$C_6$ alkynyl, $C_1$-$C_6$ alkoxy, $C_1$-$C_6$ alkylthio, sulfonyl, halogen, cycloalkyl having 3-10 ring atoms, cyano, amino, and acyl; preferably, $R_4$ is selected from $C_1$-$C_4$ alkyl, $C_2$-$C_4$ alkenyl, $C_2$-$C_4$ alkynyl, $C_1$-$C_4$ alkoxy, $C_1$-$C_4$ alkylthio, sulfonyl, halogen, cycloalkyl having 3-6 ring atoms, cyano, amino, and acyl; more preferably, $R_4$ is selected from methyl, ethyl, *n*-propyl, isopropyl, fluorine, chlorine, bromine, and cyano;

optionally, $R_4$ is further substituted with one or more $R_{4a}$, wherein each $R_{4a}$ is independently selected from $C_1$-$C_6$ alkyl, halogen, amino, $C_1$-$C_6$ alkylamino, and di($C_1$-$C_6$)alkylamino; preferably, $R_{4a}$ is each independently selected from $C_1$-$C_3$ alkyl, halogen, amino, $C_1$-$C_3$ alkylamino, and di($C_1$-$C_3$)alkylamino.

**22.** The compound or the stereoisomer, the tautomer, the solvate, the hydrate, the prodrug, the stable isotopic derivative, or the pharmaceutically acceptable salt thereof according to any one of claims 19-21, wherein:

$R_5$ is selected from halogen, cyano, amino, hydroxyl, $C_1$-$C_6$ alkyl, $C_2$-$C_6$ alkenyl, $C_2$-$C_6$ alkynyl, $C_1$-$C_6$ alkoxy, cycloalkyl having 3-10 ring atoms, cycloalkyloxy having 3-10 ring atoms, heterocyclyl having 4-10 ring atoms, and heteroaryl having 5-10 ring atoms; preferably, $R_5$ is selected from halogen, $C_1$-$C_6$ alkyl, $C_1$-$C_6$ alkoxy, cycloalkyl having 3-6 ring atoms, cycloalkyloxy having 3-6 ring atoms, heterocyclyl having 4-6 ring atoms, and heteroaryl having 5-6 ring atoms; more preferably, $R_5$ is selected from chlorine, fluorine, bromine, iodine, cyano, ethyl, n-propyl, isopropyl, methyl, cyclopropyl, methoxy, and cyclopropyloxy;

optionally, $R_5$ is further substituted with one or more $R_{5a}$, wherein each $R_{5a}$ is independently selected from $C_1$-$C_6$ alkyl, $C_1$-$C_6$ alkoxy, halogen, cycloalkyl having 3-10 ring atoms, cyano, amino, and hydroxyl; preferably, $R_{5a}$ is each independently selected from $C_1$-$C_6$ alkyl, halogen, cyano, and cycloalkyl having 3-6 ring atoms; more preferably, $R_{5a}$ is each independently selected from fluorine and cyano.

**23.** The compound or the stereoisomer, the tautomer, the solvate, the hydrate, the prodrug, the stable isotopic derivative, or the pharmaceutically acceptable salt thereof according to any one of claims 19-22, wherein:

z is $CR_6$ or N;

$R_6$ is selected from hydrogen, $C_1$-$C_6$ alkyl, $C_2$-$C_6$ alkenyl, $C_2$-$C_6$ alkynyl, $C_1$-$C_6$ alkoxy, $C_1$-$C_6$ alkylthio, sulfonyl, halogen, cycloalkyl having 3-10 ring atoms, cyano, amino, and acyl; preferably, $R_6$ is selected from hydrogen, $C_1$-$C_4$ alkyl, $C_2$-$C_4$ alkenyl, $C_2$-$C_4$ alkynyl, $C_1$-$C_4$ alkoxy, $C_1$-$C_4$ alkylthio, sulfonyl, halogen, cycloalkyl having 3-6 ring atoms, cyano, amino, and acyl; more preferably, $R_6$ is selected from hydrogen, fluorine, chlorine, methyl, ethyl, *n*-propyl, isopropyl, cyclopropyl, methoxy, cyclopropyloxy, and cyano;

optionally, $R_6$ is further substituted with one or more $R_{6a}$, wherein each $R_{6a}$ is independently selected from $C_1$-$C_6$ alkyl, halogen, amino, $C_1$-$C_6$ alkylamino, and di($C_1$-$C_6$)alkylamino; preferably, $R_{6a}$ is each independently selected from $C_1$-$C_3$ alkyl, halogen, amino, $C_1$-$C_3$ alkylamino, and di($C_1$-$C_3$)alkylamino.

**24.** The compound or the stereoisomer, the tautomer, the solvate, the hydrate, the prodrug, the stable isotopic derivative, or the pharmaceutically acceptable salt thereof according to any one of claims 19-23, wherein:

$R_1$ is -$X_1$-$R_7$, wherein $X_1$ is a bond or methylene;

optionally, $X_1$ is substituted with one or more $X_{1a}$, wherein each $X_{1a}$ is independently selected from halogen and $C_1$-$C_3$ alkyl, preferably methyl;

$R_7$ is selected from cycloalkyl having 4-10 ring atoms, aryl having 6-12 ring atoms, heteroaryl having 5-12 ring atoms, and heterocyclyl having 3-12 ring atoms;

preferably, $R_7$ is selected from the following groups:

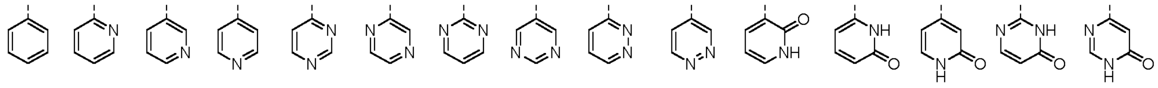
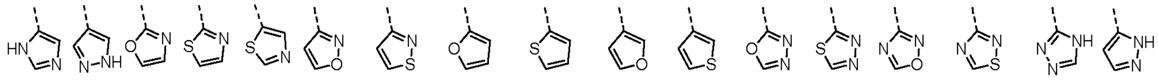
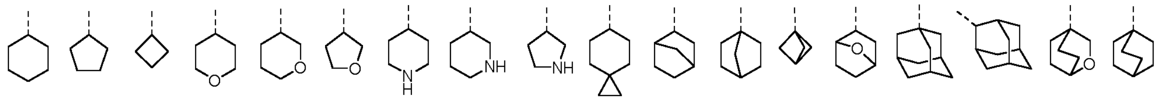
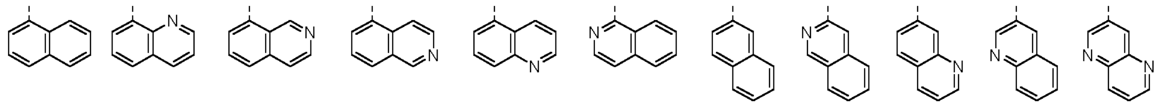
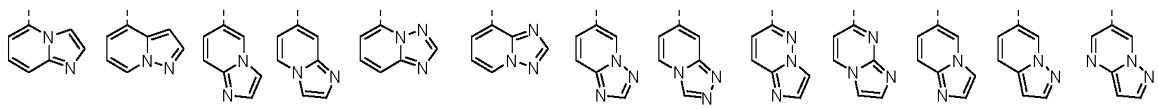
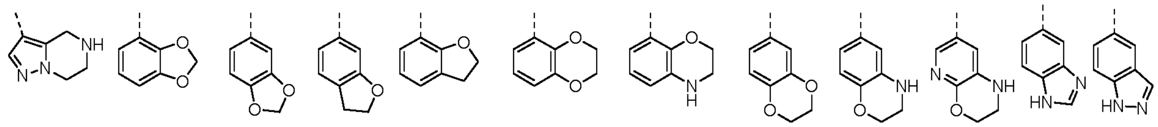
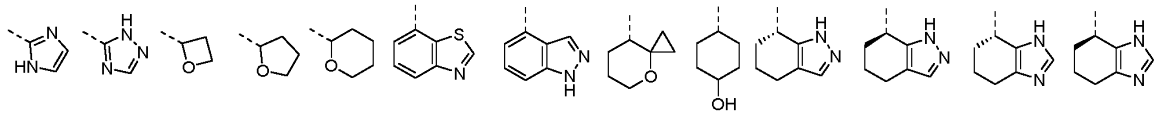
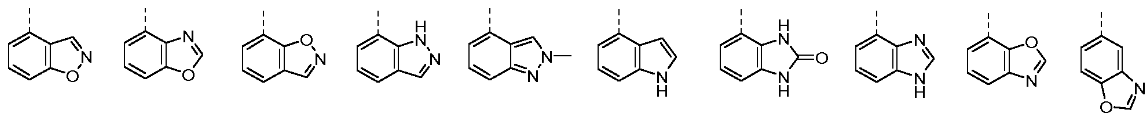
,

more preferably, $R_7$ is selected from the following groups:

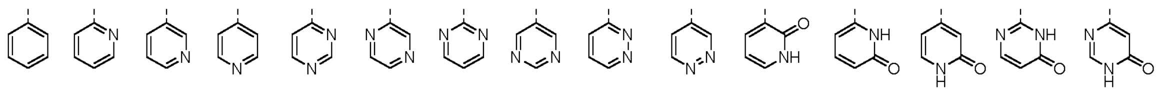
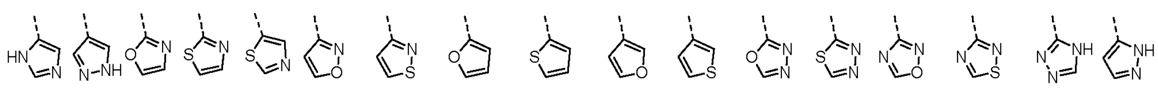
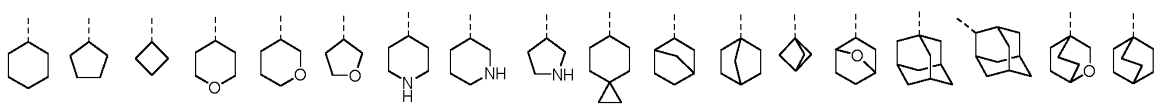
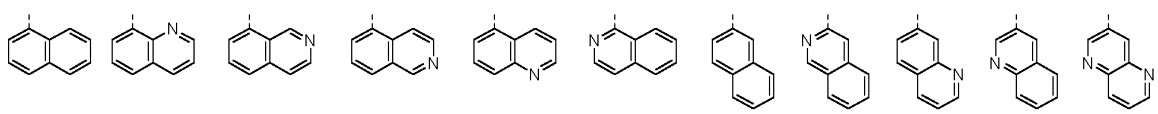

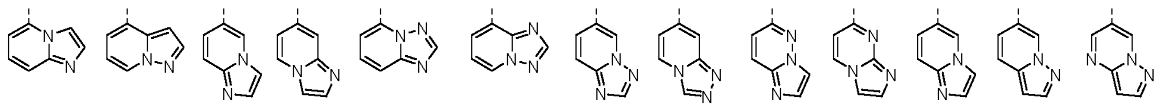

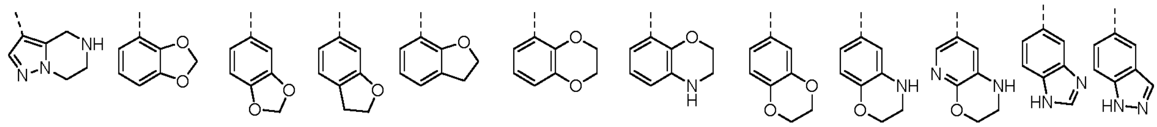

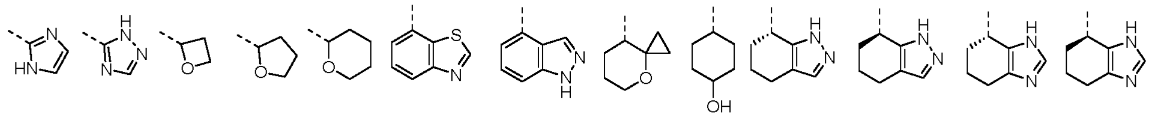
,

$R_7$ is optionally substituted with one or more $R_{7a}$, wherein each $R_{7a}$ is independently selected from oxo, halogen, $C_1$-$C_6$ alkyl, cycloalkyl having 3-10 ring atoms, $C_1$-$C_6$ alkoxy, cycloalkyloxy having 3-6 ring atoms, cyano, amino, hydroxyl, $C_2$-$C_6$ alkenyl, $C_2$-$C_6$ alkynyl, sulfonyl, acyl, carboxyl, heterocyclyl having 3-12 ring atoms, heteroaryl having 5-10 ring atoms, and ureido; preferably, $R_{7a}$ is each independently selected from oxo, halogen, $C_1$-$C_4$ alkyl, cycloalkyl having 3-6 ring atoms, $C_1$-$C_4$ alkoxy, cycloalkyloxy having 3-4 ring atoms, cyano, amino, hydroxyl, $C_2$-$C_4$ alkenyl, $C_2$-$C_4$ alkynyl, sulfonyl, acyl, carboxyl, heterocyclyl having 3-6 ring atoms, heteroaryl having 5-6 ring atoms, and ureido; more preferably, $R_{7a}$ is each independently selected from fluorine, chlorine, bromine, hydroxyl, cyano, methoxy, methyl, cyclopropyloxy, and cyclopropyl;

optionally, $R_{7a}$ is further substituted with one or more $R_{7b}$, wherein $R_{7b}$ is independently selected from halogen, $C_1$-$C_6$ alkyl, $C_1$-$C_6$ alkoxy, hydroxyl, amino, $C_2$-$C_6$ alkenyl, $C_2$-$C_6$ alkynyl, $C_1$-$C_6$ alkylamino, di($C_1$-$C_6$)alkylamino, sulfonyl, heterocyclyl having 3-6 ring atoms, and heteroaryl having 5-6 ring atoms; preferably, each $R_{7b}$ is independently selected from halogen, $C_1$-$C_3$ alkyl, $C_1$-$C_3$ alkoxy, hydroxyl, amino, $C_2$-$C_4$ alkenyl, $C_2$-$C_4$ alkynyl, $C_1$-$C_3$ alkylamino, di($C_1$-$C_3$)alkylamino, sulfonyl, heterocyclyl having 3-6 ring atoms, and heteroaryl having 5-6 ring atoms; more preferably, each $R_{7b}$ is independently selected from fluorine, chlorine, and bromine;

when $X_1$ is methylene, $R_7$ is not substituted or unsubstituted aryl.

**25.** The compound or the stereoisomer, the tautomer, the solvate, the hydrate, the prodrug, the stable isotopic derivative, or the pharmaceutically acceptable salt thereof according to any one of claims 19-24, wherein:

$R_8$ and $R_9$ are each independently selected from hydrogen, halogen, $C_1$-$C_6$ alkyl, $C_1$-$C_6$ alkoxy, $C_2$-$C_6$ alkenyl, $C_2$-$C_6$ alkynyl, cycloalkyl having 3-10 ring atoms, cycloalkyloxy having 3-10 ring atoms, heterocyclyl having 4-10 ring atoms, heterocyclyloxy having 4-10 ring atoms, aryl having 6-10 ring atoms, heteroaryl having 5-10 ring atoms, heteroaryloxy having 5-10 ring atoms, cyano, amino, acyl, sulfonyl, and oxo, or adjacent $R_8$ and $R_9$ form a 5-6 membered carbocyclic ring, heterocyclic ring, or heteroaromatic ring; preferably, $R_8$ and $R_9$ are each independently selected from hydrogen, halogen, $C_1$-$C_4$ alkyl, $C_1$-$C_4$ alkoxy, $C_2$-$C_4$ alkenyl, $C_2$-$C_4$ alkynyl, cycloalkyl having 3-6 ring atoms, cycloalkyloxy having 3-6 ring atoms, heterocyclyl having 5-6 ring atoms, heterocyclyloxy having 5-6 ring atoms, aryl having 6 ring atoms, heteroaryl having 5-6 ring atoms, heteroaryloxy having 5-6 ring atoms, cyano, amino, acyl, sulfonyl, and oxo, or adjacent $R_8$ and $R_9$ form a 5-6 membered heterocyclic ring or heteroaromatic ring; more preferably, $R_8$ and $R_9$ are each independently selected from hydrogen, methyl, ethyl, *n*-propyl, isopropyl, cyano, methoxy, oxo, cyclopropyl, tetrahydropyrrolyl, tetrahydrofuranyl, phenyl, and propenyl, or adjacent $R_8$ and $R_9$ form

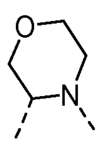

or

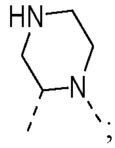

optionally, $R_8$ is further substituted with one or more $R_{8a}$; optionally, $R_9$ is further substituted with one or more $R_{9a}$; optionally, the carbocyclic ring, heterocyclic ring, or heteroaromatic ring formed by adjacent $R_8$ and $R_9$ is further substituted with one or more $R_{8a}$ or $R_{9a}$;

$R_{8a}$ or $R_{9a}$ is each independently selected from halogen, $C_1$-$C_6$ alkyl, heterocyclyl having 4-10 ring atoms, heterocyclyloxy having 4-10 ring atoms, amino, heteroaryl having 5-10 ring atoms, cycloalkyl having 3-10 ring atoms, aryl having 6-10 ring atoms, $C_1$-$C_6$ alkylamino, di($C_1$-$C_6$)alkylamino, hydroxyl, sulfonyl, $C_1$-$C_6$ alkoxy, cyano, $C_2$-$C_6$ alkenyl, $C_2$-$C_6$ alkynyl, and oxo; preferably, each $R_{8a}$ or each $R_{9a}$ is independently selected from halogen, $C_1$-$C_4$ alkyl, heterocyclyl having 5-6 ring atoms, heterocyclyloxy having 5-6 ring atoms, amino, heteroaryl having 5-6 ring atoms, cycloalkyl having 3-6 ring atoms, aryl having 6 ring atoms, $C_1$-$C_3$ alkylamino, di($C_1$-$C_3$)alkylamino, hydroxyl, sulfonyl, $C_1$-$C_4$ alkoxy, cyano, $C_2$-$C_4$ alkenyl, $C_2$-$C_4$ alkynyl, and oxo; more preferably, each $R_{8a}$ or each $R_{9a}$ is independently selected from methyl, chlorine, fluorine, bromine, phenyl, cyclopropyl, oxo, amino, methylamino, dimethylamino, methoxy, hydroxyl, pyridinyl, pyrazolyl, sulfonyl, morpholinyl, tetrahydrofuranyl, isoxazolyl, and pyrimidinyl;

optionally, $R_{8a}$ is further substituted with one or more $R_{8b}$; optionally, $R_{9a}$ is further substituted with one or more $R_{9b}$; $R_{8b}$ or $R_{9b}$ is each independently selected from $C_1$-$C_3$ alkyl substituted with 0-3 halogens, $C_1$-$C_3$ alkoxy substituted with 0-3 halogens, halogen, hydroxyl, unsubstituted 5-6 membered heterocyclyl or 5-6 membered heterocyclyl substituted with $C_1$-$C_3$ alkyl, cyano, and oxo, preferably $C_1$-$C_3$ alkyl, $C_1$-$C_3$ alkoxy, halogen, hydroxyl, and oxo; preferably, $R_{8b}$ or $R_{9b}$ is each independently selected from methoxy and methyl.

**26.** A compound or a stereoisomer, a tautomer, a solvate, a hydrate, a prodrug, a stable isotopic derivative, or a pharmaceutically acceptable salt thereof, wherein the compound is selected from:

| | | | | | |
|---|---|---|---|---|---|
| | | B2 | 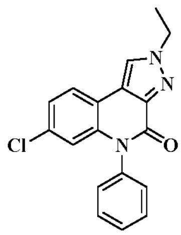 | B3 | 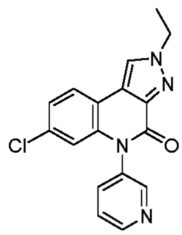 |
| B4 | 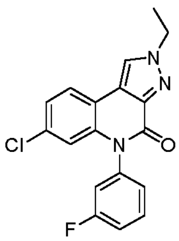 | B5 | 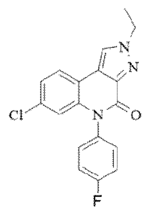 | B6 | 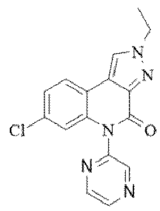 |
| B7 | 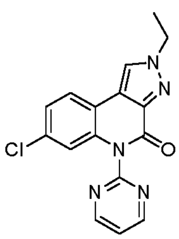 | B8 | 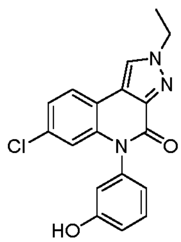 | B9 | 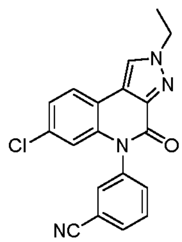 |

(continued)

| | | | | | |
|---|---|---|---|---|---|
| B10 | 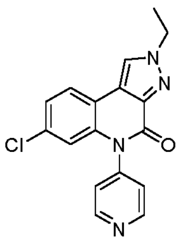 | B11 | 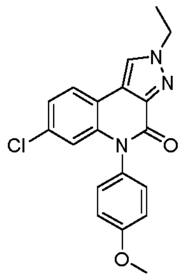 | B12 | 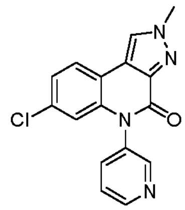 |
| B13 | 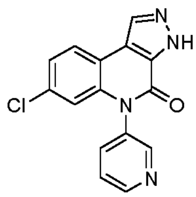 | B14 | 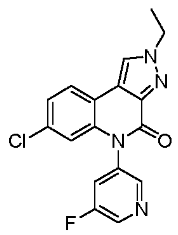 | B15 | 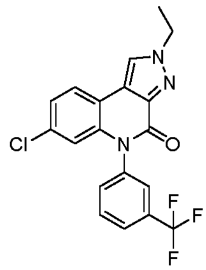 |
| B16 | 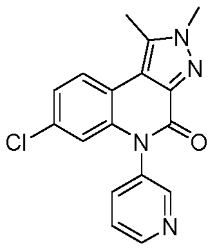 | B17 | 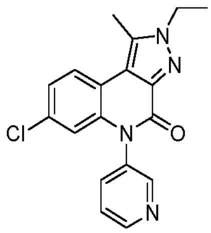 | B18 | 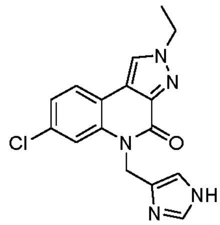 |
| B19 | 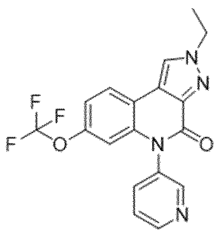 | B20 | 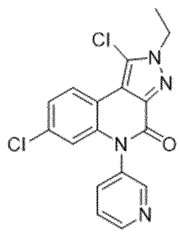 | B21 | 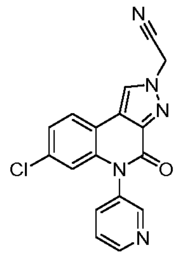 |
| B22 | 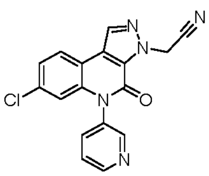 | B23 | 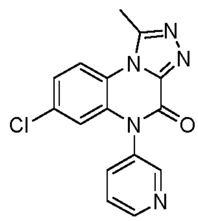 | B24 | 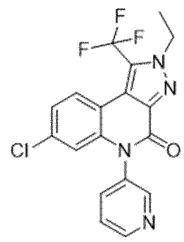 |
| B25 | 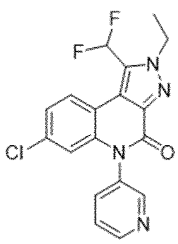 | B26 | 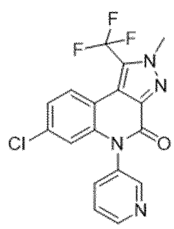 | B27 | 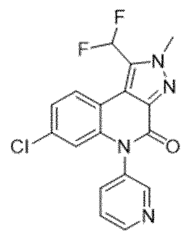 |

(continued)

| | | | | | |
|---|---|---|---|---|---|
| B28 | 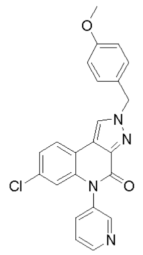 | B29 | 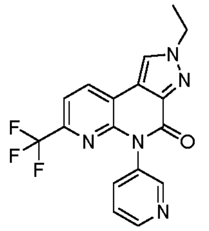 | B30 | 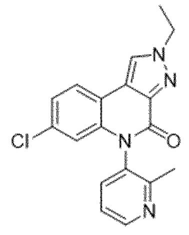 |
| B31 | 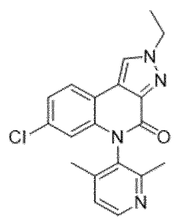 | B32 | 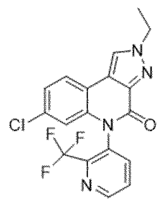 | B33 | 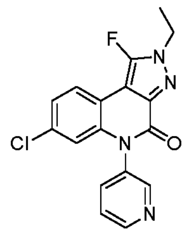 |
| B34 | 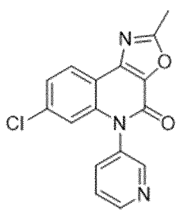 | B35 | 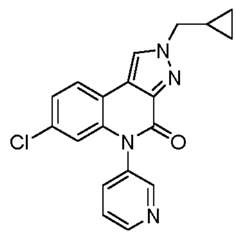 | B36 | 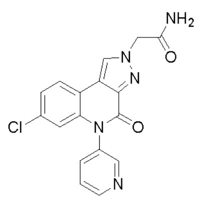 |
| B37 | 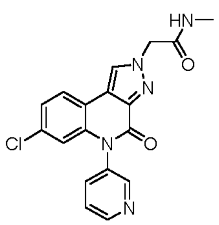 | B38 | 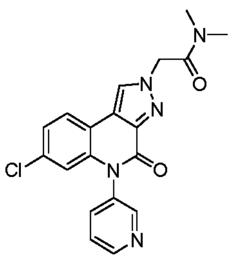 | B39 | 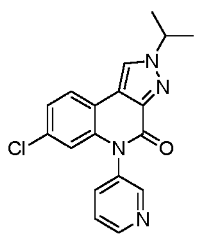 |
| B40 | 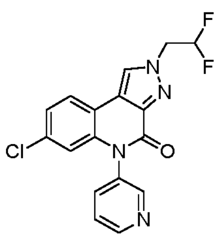 | B41 | 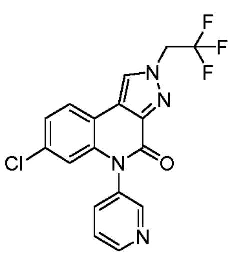 | B42 | 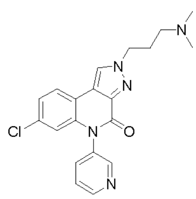 |
| B43 | 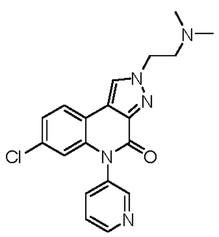 | B44 | 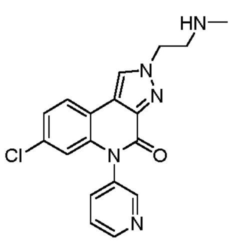 | B45 | 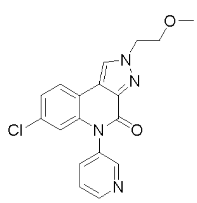 |

(continued)

| | | | | | |
|---|---|---|---|---|---|
| B46 | 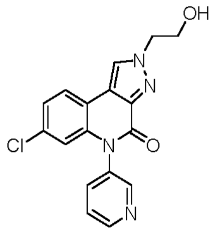 | B47 | 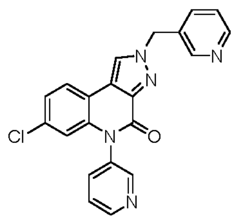 | B48 | 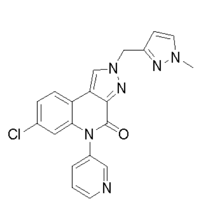 |
| B49 | 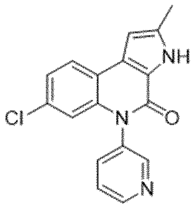 | B50 | 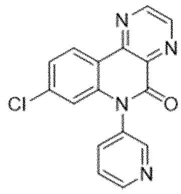 | B51 | 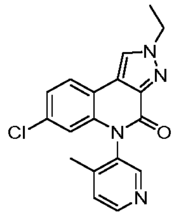 |
| B52 | 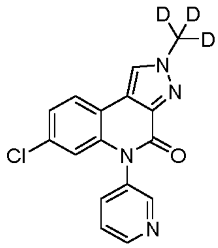 | B53 | 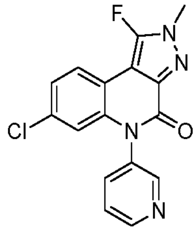 | B54 | 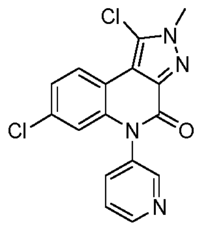 |
| B55 | 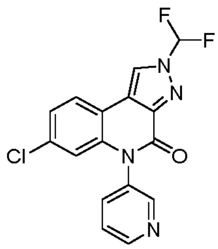 | B56 | 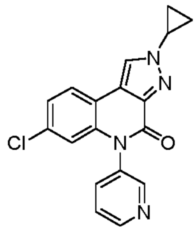 | B57 | 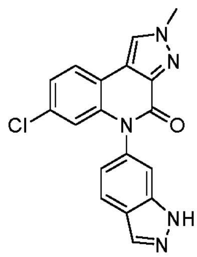 |
| B58 | 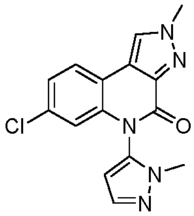 | B59 | 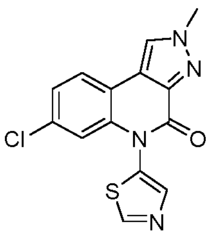 | B60 | 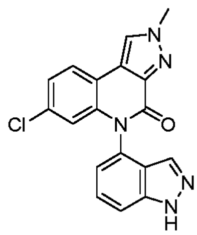 |
| B61 | 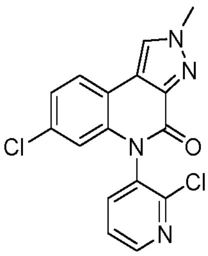 | B62 | 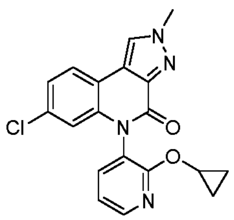 | B63 | 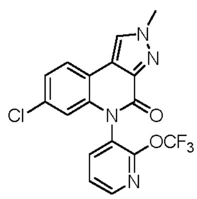 |

(continued)

| | | | | | |
|---|---|---|---|---|---|
| B64 | 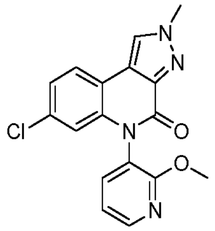 | B65 | 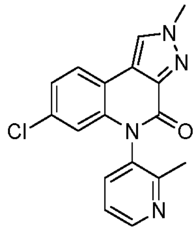 | B66 | 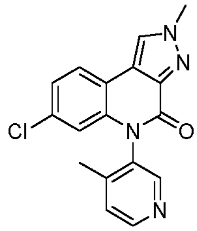 |
| B67 | 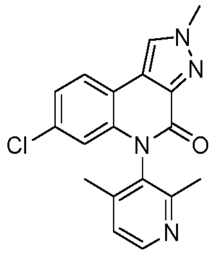 | B68 | 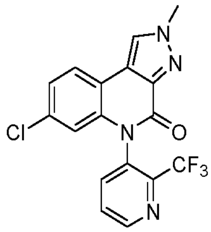 | B69 | 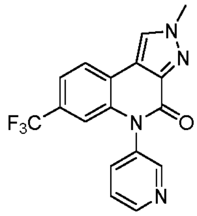 |
| B70 | 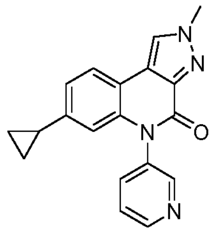 | B71 | 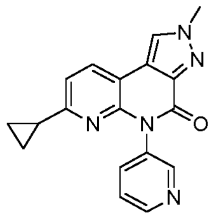 | B72 | 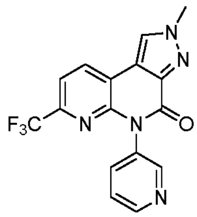 |
| B73 | 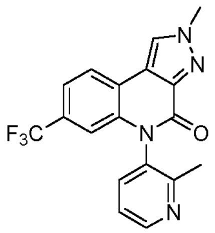 | B74 | 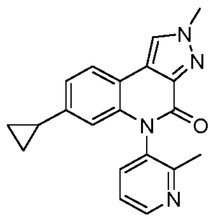 | B75 | 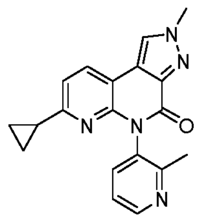 |
| B76 | 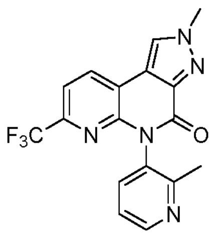 | B77 | 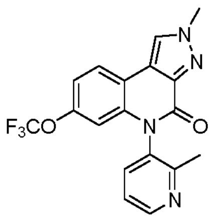 | B78 | 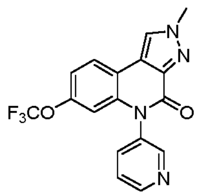 |
| B79 | 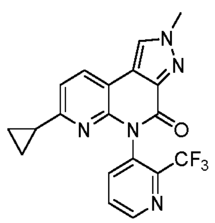 | B80 | 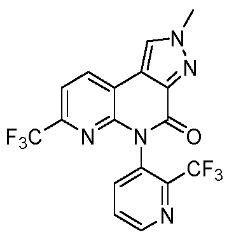 | B81 | 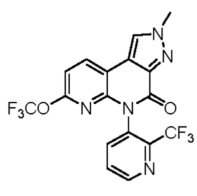 |

(continued)

| | | | | | |
|---|---|---|---|---|---|
| B82 | 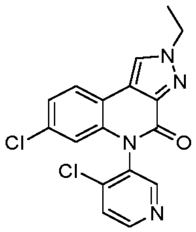 | B83 | 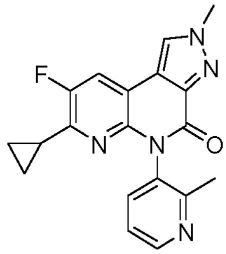 | B84 | 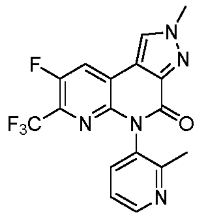 |
| B85 | 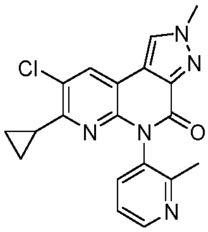 | B86 | 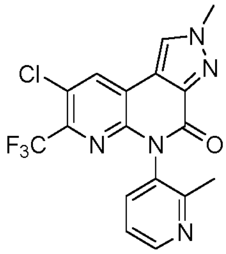 | B87 | 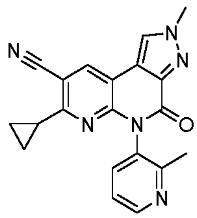 |
| B88 | 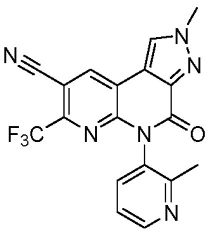 | B89 | 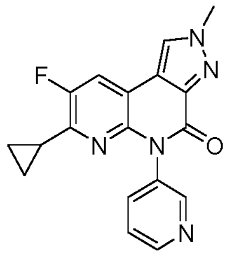 | B90 | 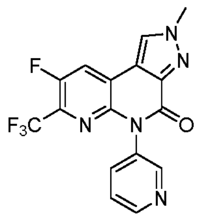 |
| B91 | 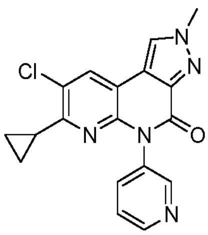 | B92 | 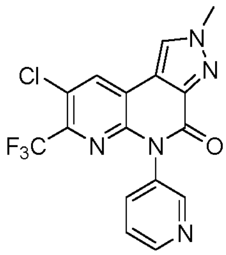 | B93 | 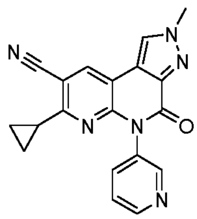 |
| B94 | 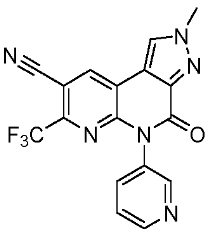 | B95 | 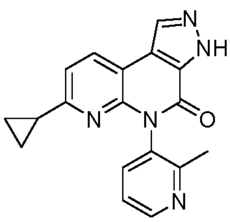 | B96 | 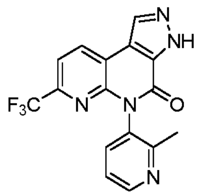 |
| B97 | 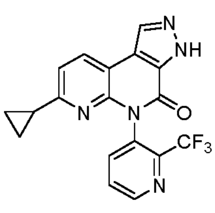 | B98 | 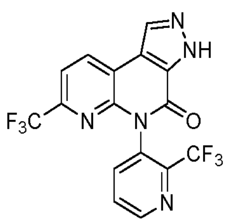 | B99 | 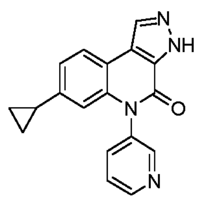 |

(continued)

| | | | | | |
|---|---|---|---|---|---|
| B100 | 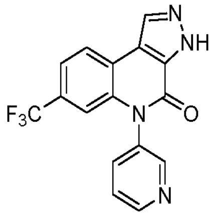 | B101 | 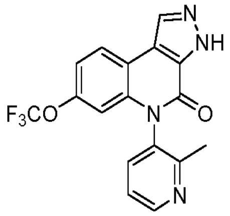 | B102 | 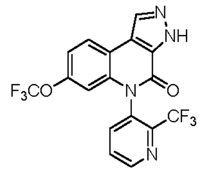 |
| B103 | 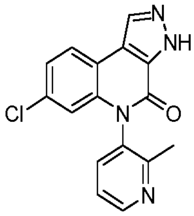 | B104 | 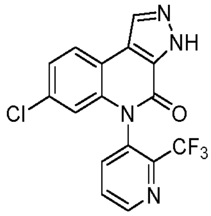 | B105 | 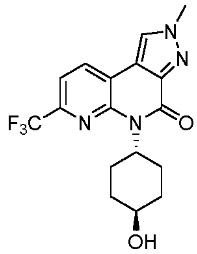 |
| B106 | 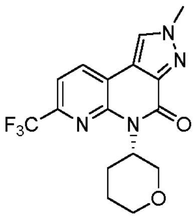 | B107 | 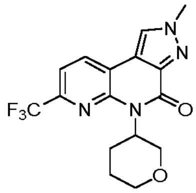 | B108 | 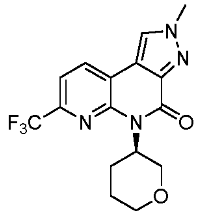 |
| B109 | 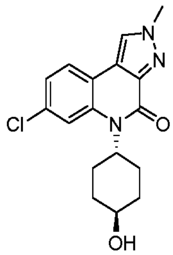 | B110 | 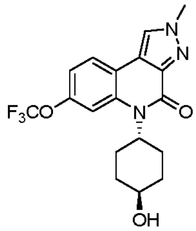 | B111 | 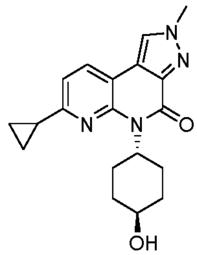 |
| B112 | 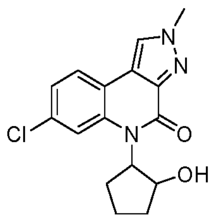 | B113 | 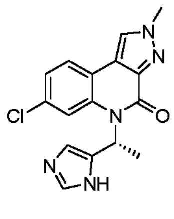 | B114 | 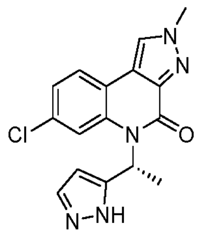 |
| B115 | 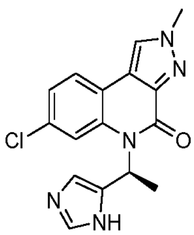 | B116 | 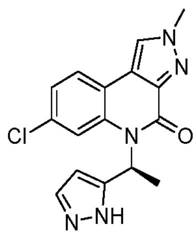 | B117 | 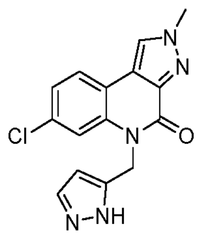 |
| B118 | 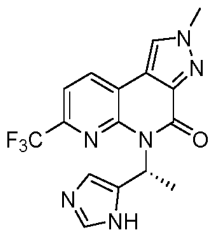 | B119 | 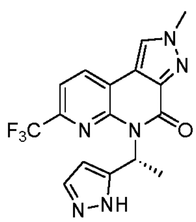 | B120 | 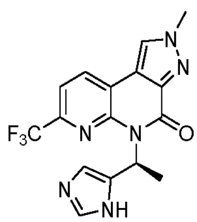 |

(continued)

| | | | | | |
|---|---|---|---|---|---|
| B121 | 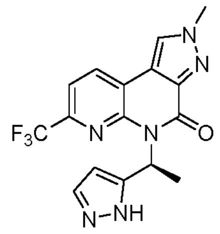 | B122 | 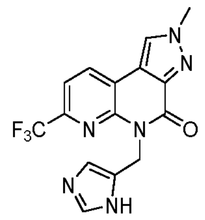 | B123 | 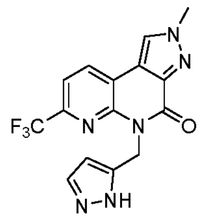 |
| B124 | 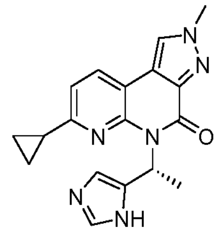 | B125 | 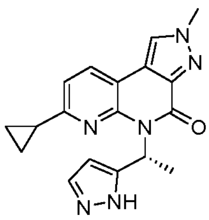 | B126 | 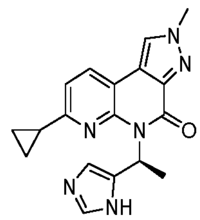 |
| B127 | 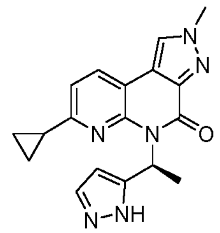 | B128 | 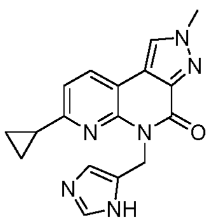 | B129 | 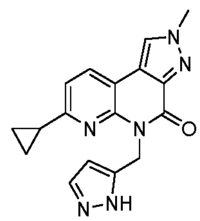 |
| B130 | 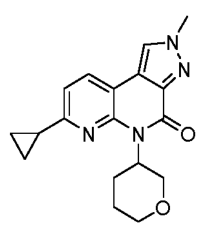 | B131 | 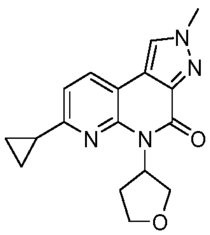 | B132 | 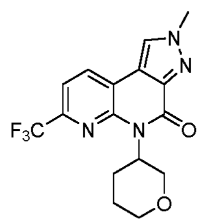 |
| B133 | 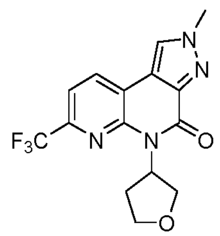 | B134 | 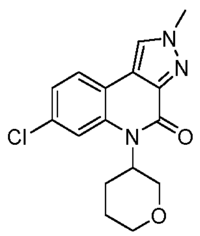 | B135 | 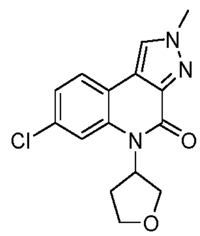 |
| B136 | 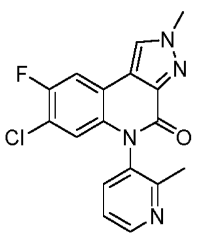 | B137 | 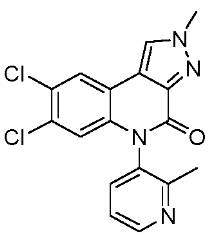 | B138 | 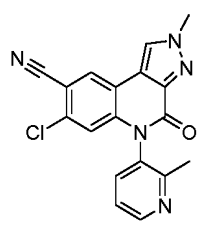 |
| B139 | 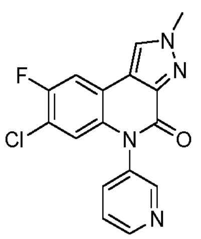 | B140 | 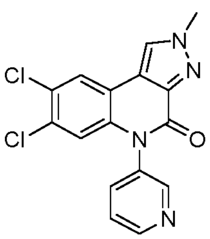 | B141 | 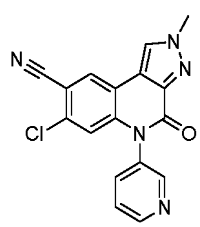 |

(continued)

| | | | | | |
|---|---|---|---|---|---|
| B142 | 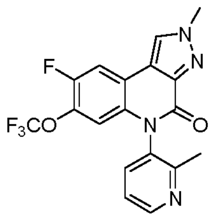 | B143 | 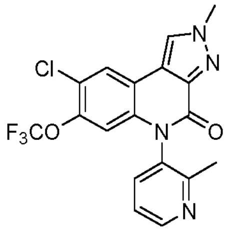 | B144 | 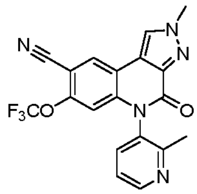 |
| B145 | 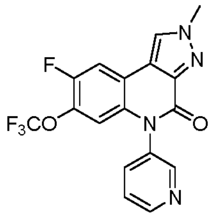 | B146 | 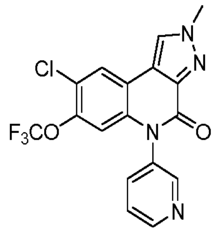 | B147 | 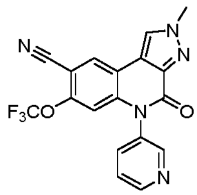 |
| B148 | 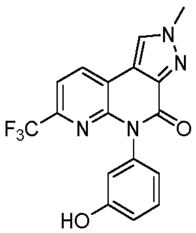 | B149 | 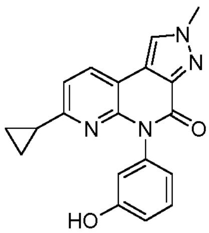 | B150 | 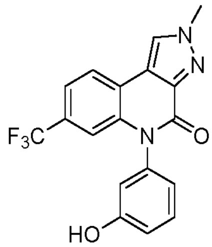 |
| B151 | 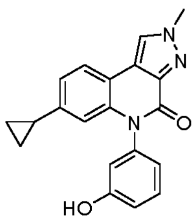 | B152 | 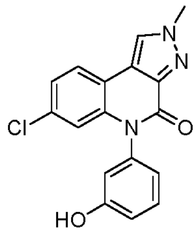 | B153 | 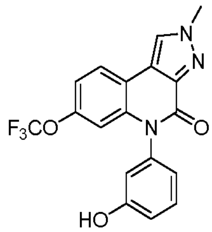 |
| B154 | 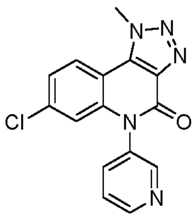 | B155 | 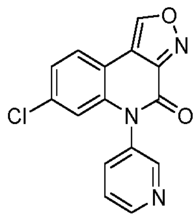 | B156 | 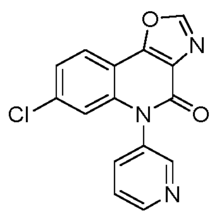 |
| B157 | 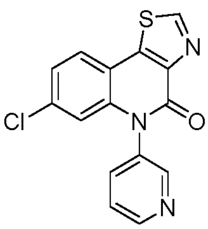 | B158 | 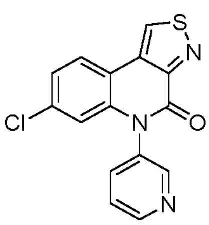 | B159 | 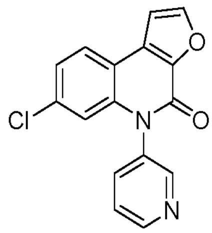 |
| B160 | 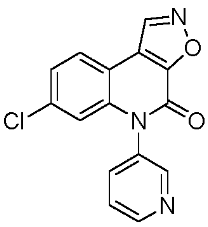 | B161 | 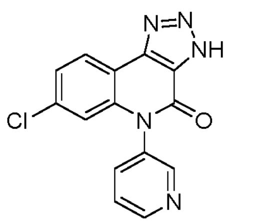 | B162 | 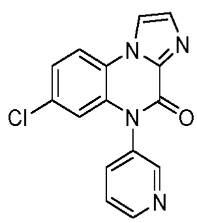 |

(continued)

| | | | | | |
|---|---|---|---|---|---|
| B163 | 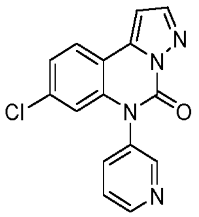 | B164 | 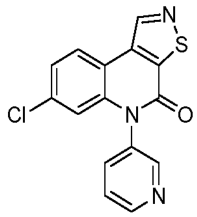 | B165 | 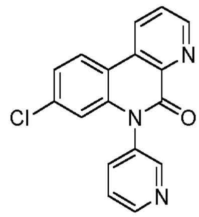 |
| B166 | 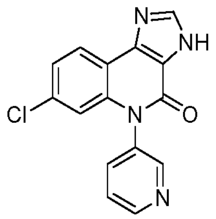 | B167 | 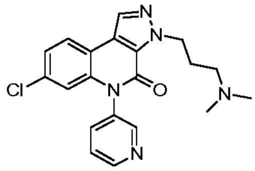 | B168 | 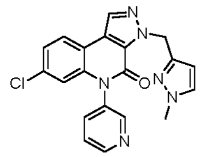 |
| B169 | 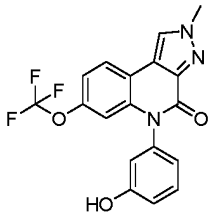 | B170 | 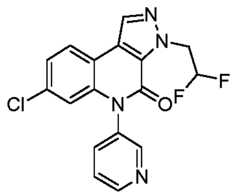 | B171 | 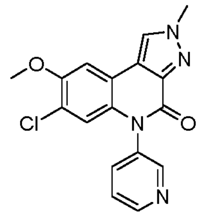 |
| B172 | 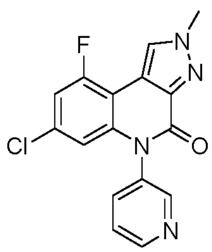 | B173 | 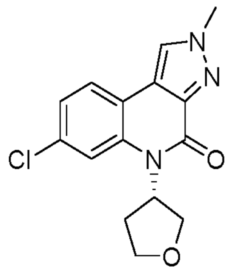 | B174 | 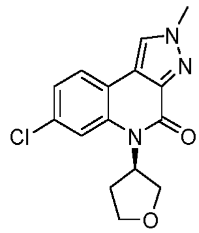 |
| B175 | 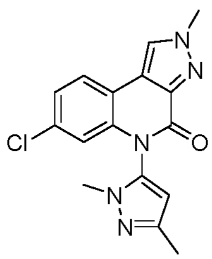 | B176 | 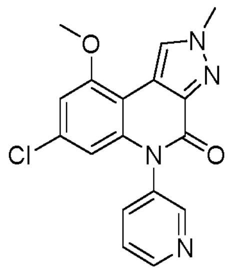 | B177 | 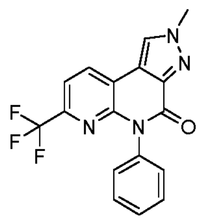 |
| B178 | 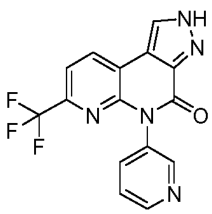 | B179 | 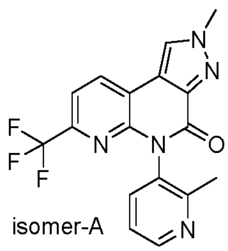 isomer-A | B180 | 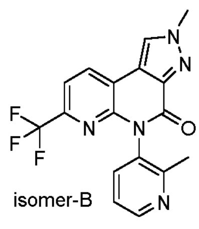 isomer-B |

(continued)

| | | | | | |
|---|---|---|---|---|---|
| B181 | 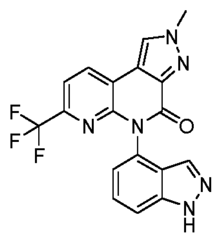 | B182 | 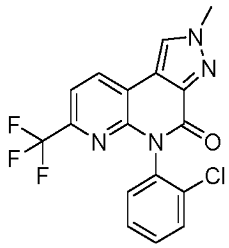 | B183 | 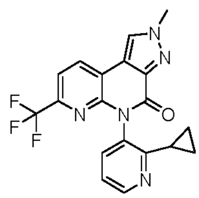 |
| B184 | 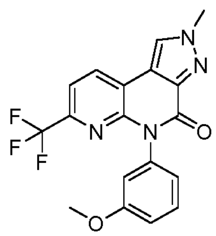 | B185 | 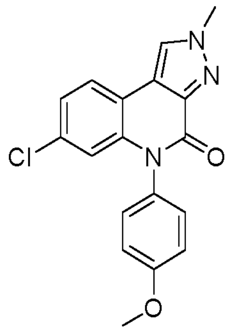 | B186 | 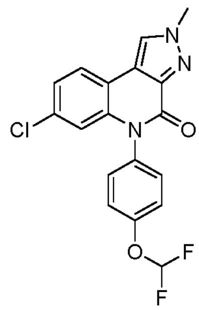 |
| B187 | 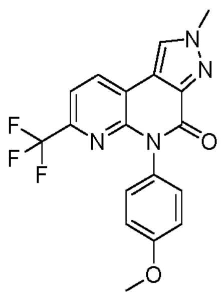 | B188 | 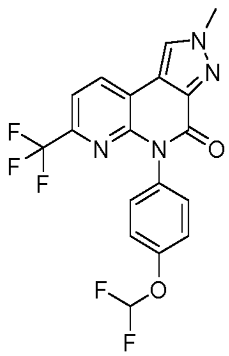 | B189 | 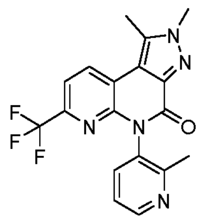 |
| B190 | 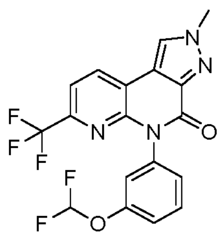 | B191 | 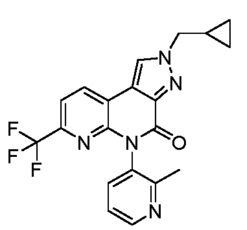 | B192 | 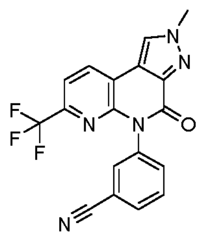 |
| B193 | 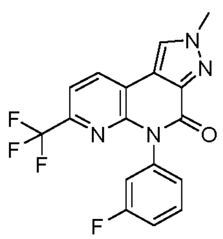 | B194 | 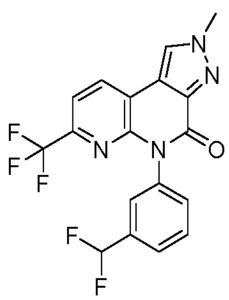 | B195 | 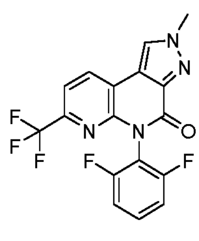 |

(continued)

| | | | | | |
|---|---|---|---|---|---|
| B196 | 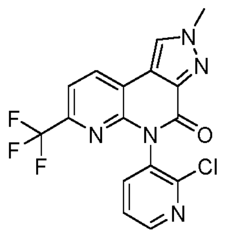 | B197 | 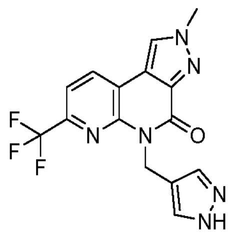 | B198 | 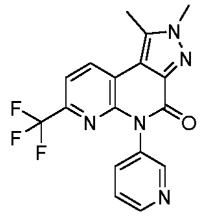 |
| B199 | 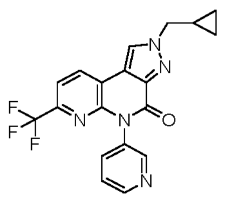 | B200 | 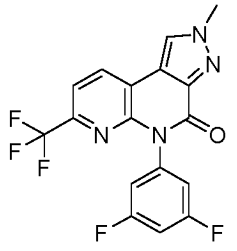 | B201 | 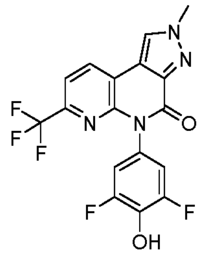 |
| B202 | 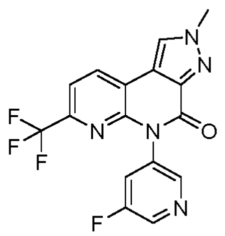 | B203 | 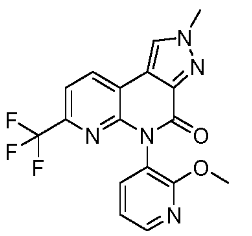 | B204 | 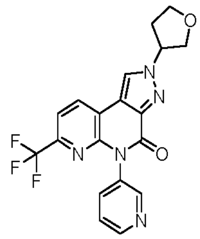 |
| B205 | 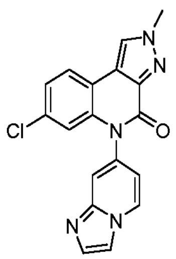 | B206 | 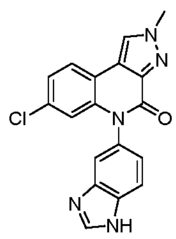 | B207 | 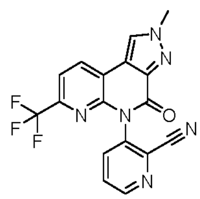 |
| B208 | 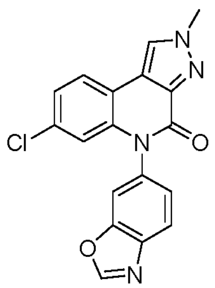 | B209 | 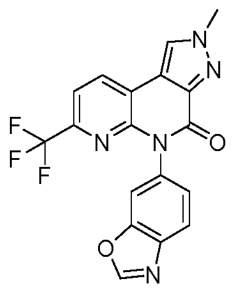 | B210 | 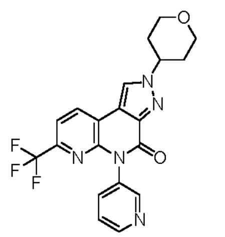 |
| B211 | 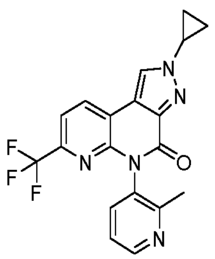 | B212 | 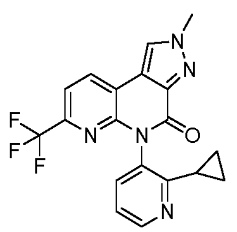 Isomer A | B213 | 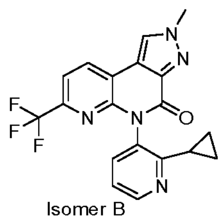 Isomer B |

(continued)

| | | | | | |
|---|---|---|---|---|---|
| B214 | 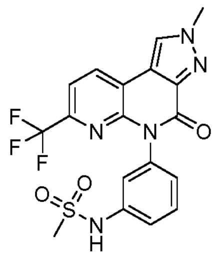 | B215 | 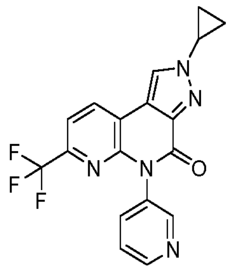 | B216 | 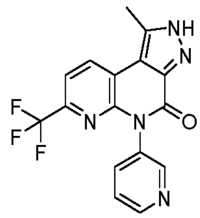 |
| B217 | 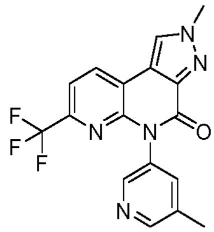 | B218 | 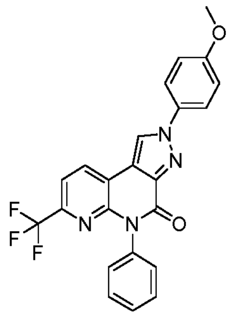 | B219 | 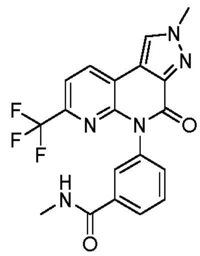 |
| B220 | 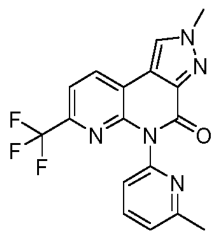 | B221 | 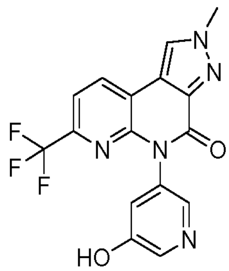 | B222 | 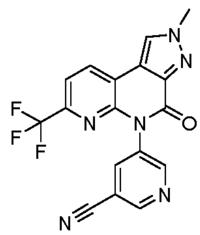 |
| B223 | 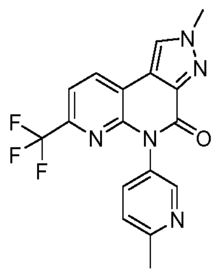 | B224 | 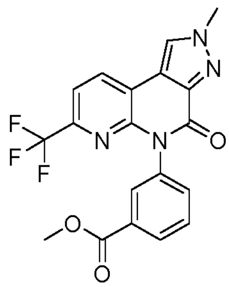 | B225 | 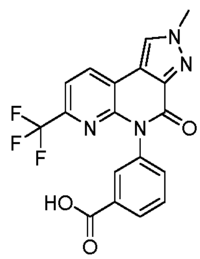 |
| B226 | 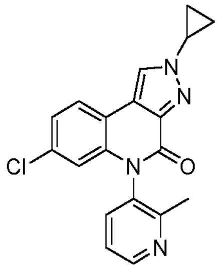 | B227 | 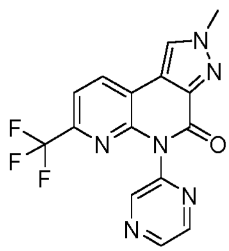 | B228 | 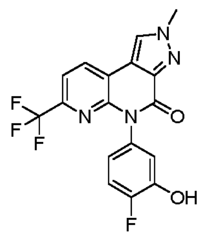 |

(continued)

| | | | | | |
|---|---|---|---|---|---|
| B229 | 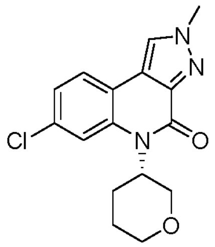 | B230 | 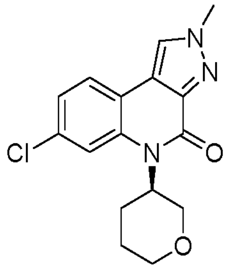 | B231 | 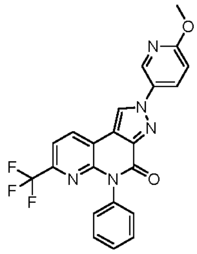 |
| B232 | 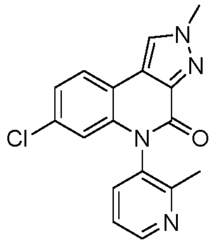 IsomerA | B233 | 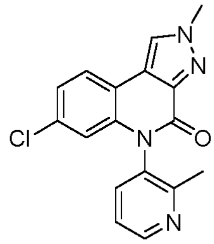 IsomerB | B234 | 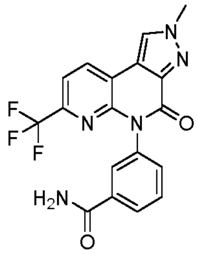 |
| B235 | 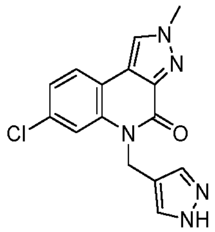 | B236 | 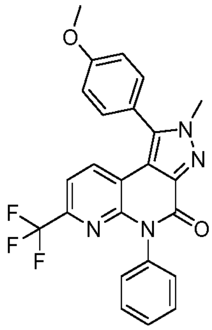 | B237 | 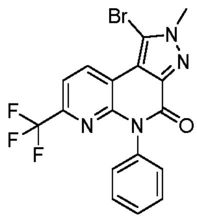 |
| B238 | 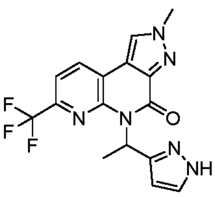 | B239 | 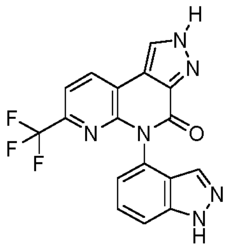 | B240 | 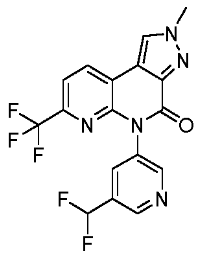 |
| B241 | 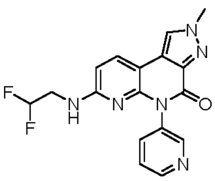 | B242 | 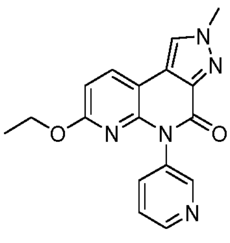 | B243 | 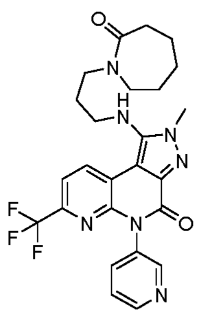 |

(continued)

| | | | | | |
|---|---|---|---|---|---|
| B244 | 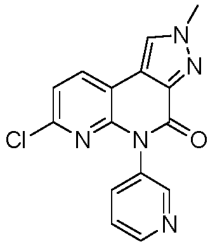 | B245 | 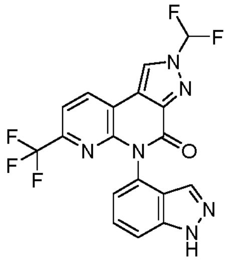 | B246 | 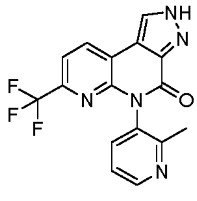 Isomer A |
| B247 | 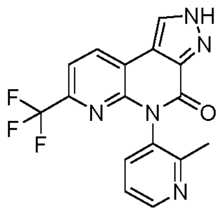 Isomer B | B248 | 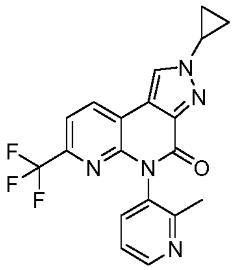 Isomer A | B249 | 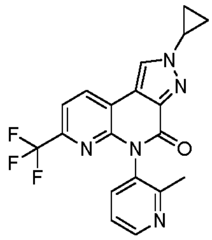 Isomer B |
| B250 | 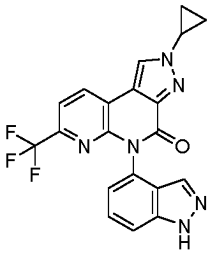 | B251 | 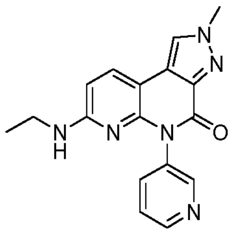 | B252 | 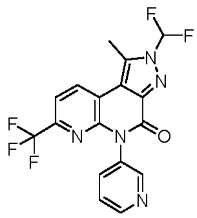 |
| B253 | 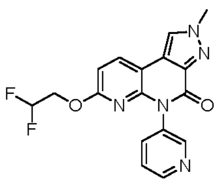 | B254 | 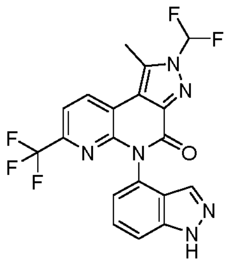 | B255 | 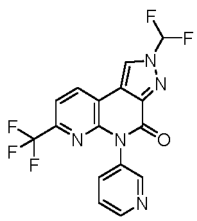 |
| B256 | 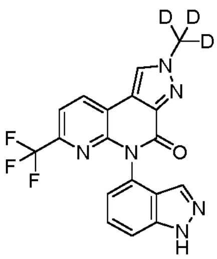 | B257 | 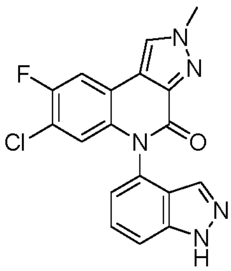 | B258 | 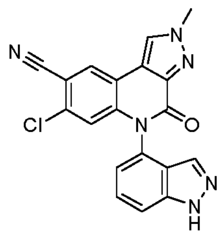 |
| B259 | 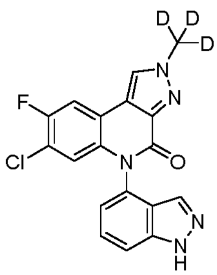 | B260 | 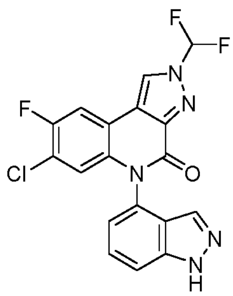 | B261 | 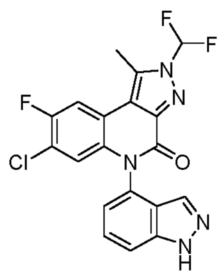 |

(continued)

| | | | | | |
|---|---|---|---|---|---|
| B262 | 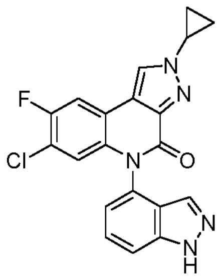 | B263 | 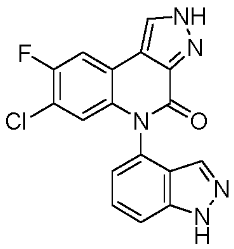 | B264 | 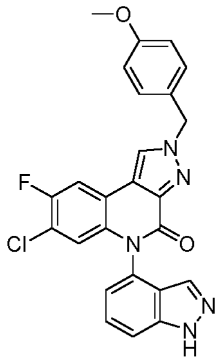 |
| B265 | 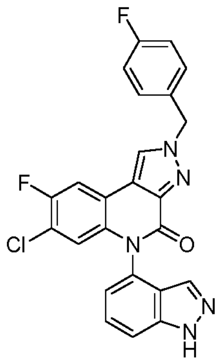 | B266 | 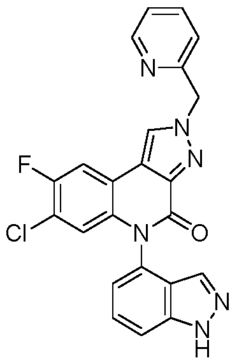 | B267 | 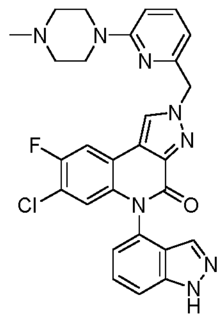 |
| B268 | 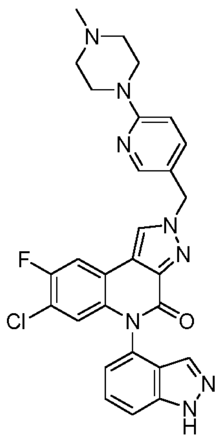 | B269 | 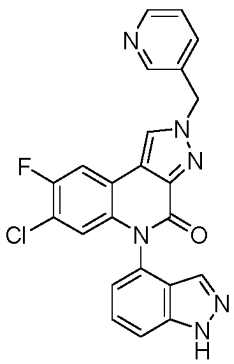 | B270 | 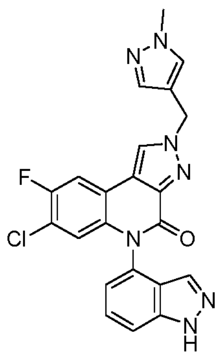 |
| B271 | 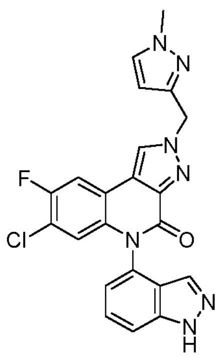 | B272 | 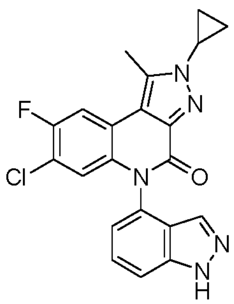 | B273 | 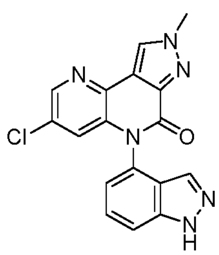 |

(continued)

| | | | | | |
|---|---|---|---|---|---|
| B274 | 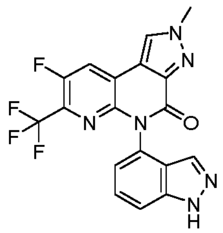 | B275 | 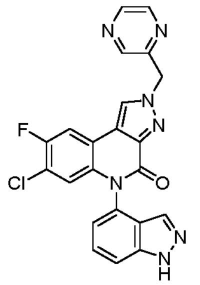 | B276 | 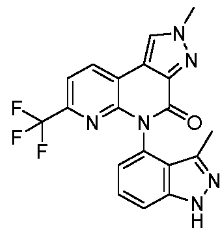 |
| B277 | 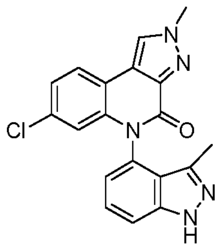 | B278 | 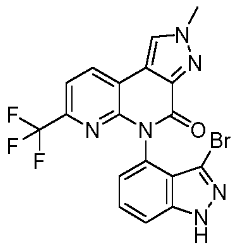 | B279 | 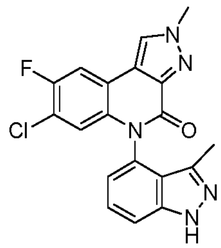 |
| B280 | 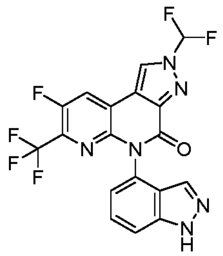 | B281 | 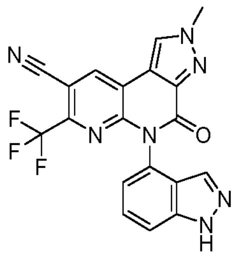 | B282 | 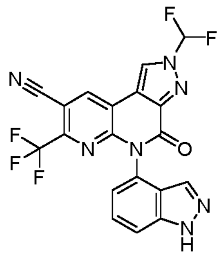 |
| B283 | 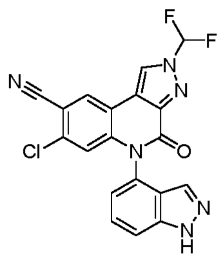 | B284 | 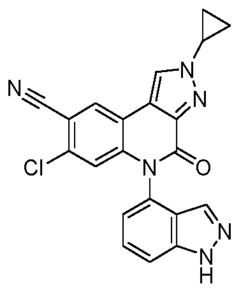 | B285 | 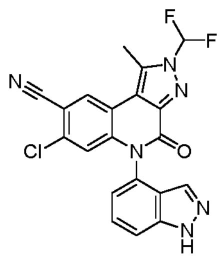 |
| B286 | 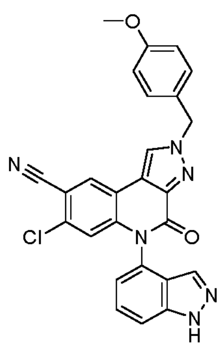 | B287 | 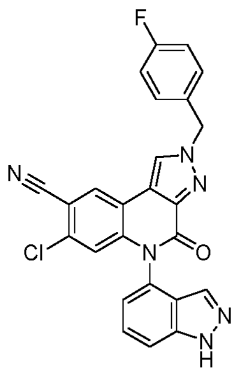 | B288 | 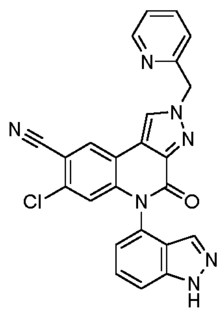 |

(continued)

| | | | | | |
|---|---|---|---|---|---|
| B289 | 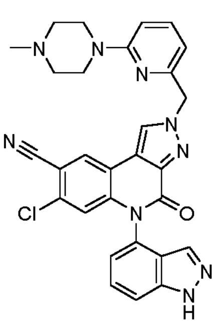 | B290 | 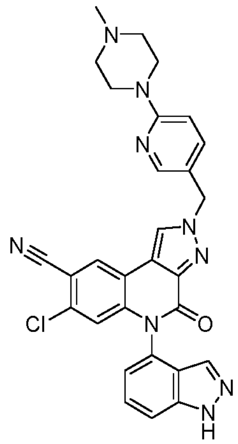 | B291 | 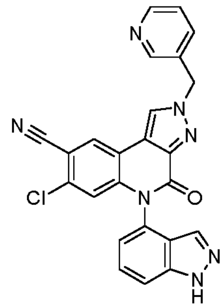 |
| B292 | 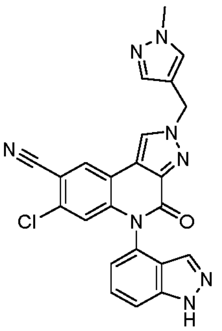 | B293 | 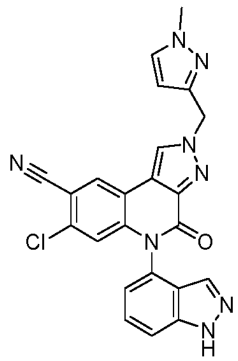 | B294 | 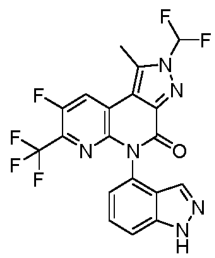 |
| B295 | 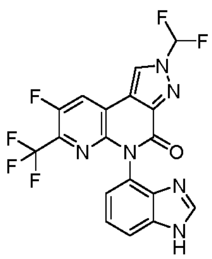 | B296 | 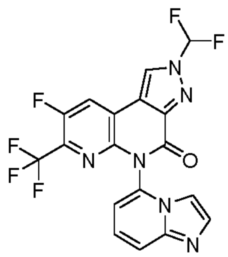 | B297 | 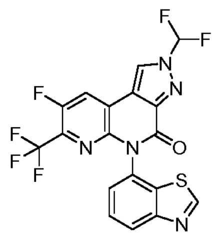 |
| B298 | 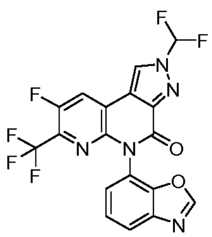 | B299 | 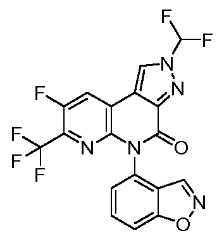 | B300 | 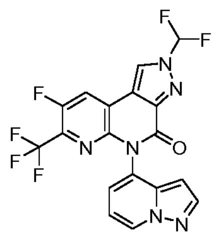 |
| B301 | 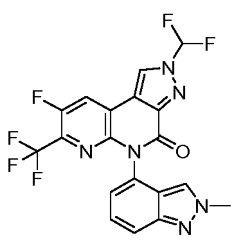 | B302 | 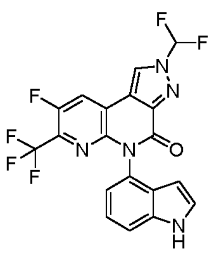 | B303 | 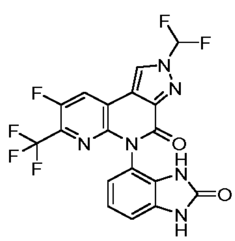 |

(continued)

| | | | | | |
|---|---|---|---|---|---|
| B304 | 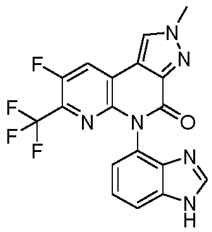 | B305 | 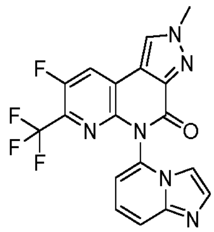 | B306 | 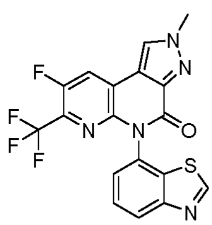 |
| B307 | 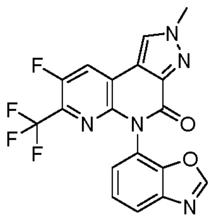 | B308 | 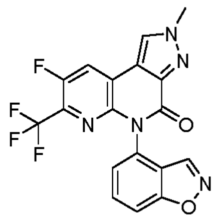 | B309 | 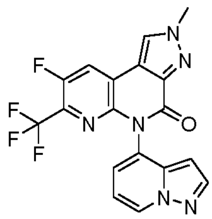 |
| B310 | 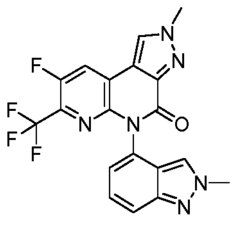 | B311 | 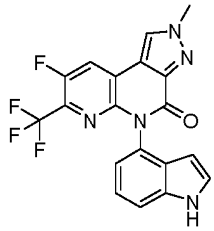 | B312 | 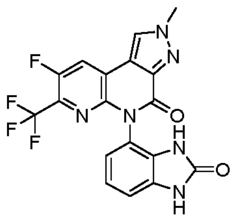 |
| B313 | 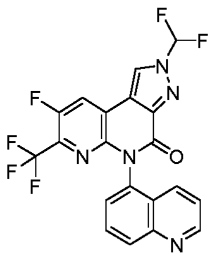 | B314 | 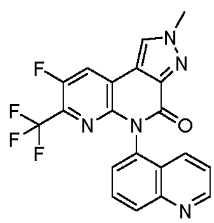 | B315 | 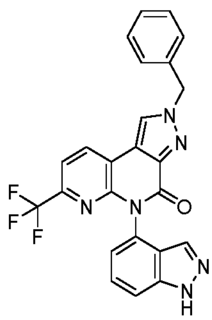 |
| B316 | 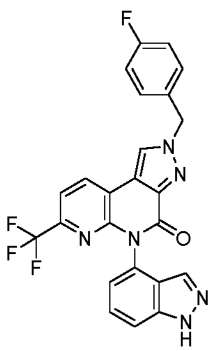 | B317 | 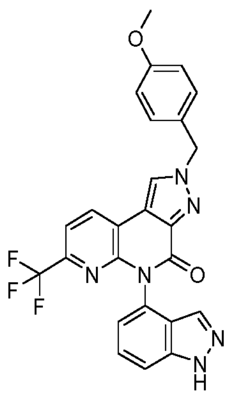 | B318 | 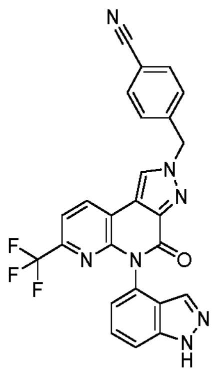 |

(continued)

| | | | | | |
|---|---|---|---|---|---|
| B319 | 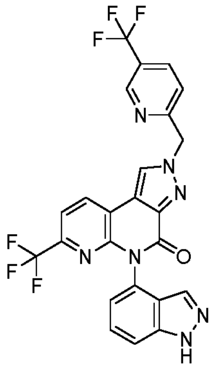 | B320 | 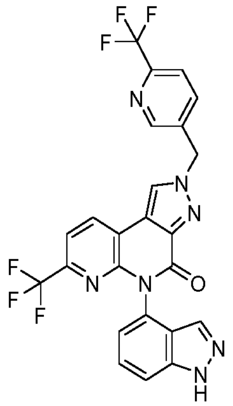 | B321 | 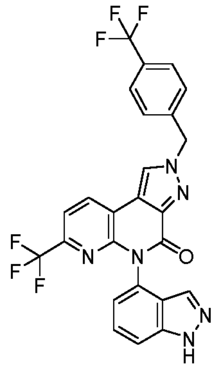 |
| B322 | 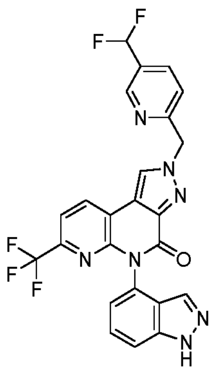 | B323 | 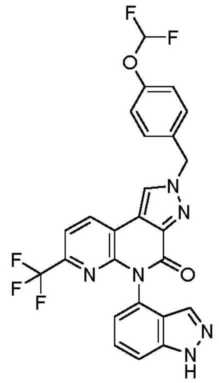 | B324 | 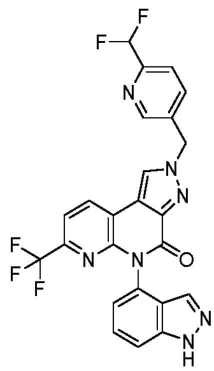 |
| B325 | 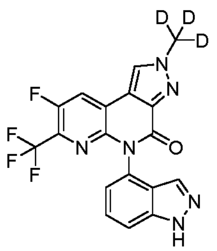 | B326 | 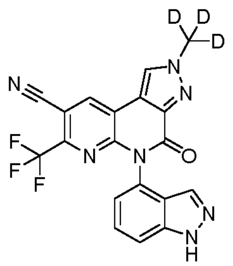 | B327 | 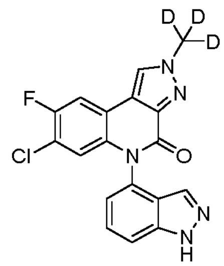 |
| B328 | 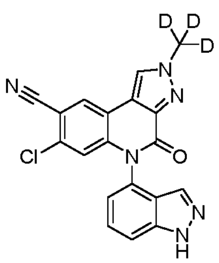 | B329 | 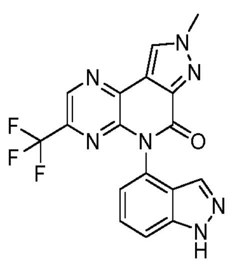 | B330 | 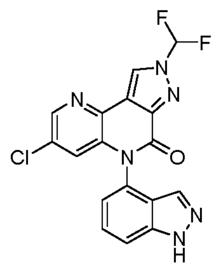 |
| B331 | 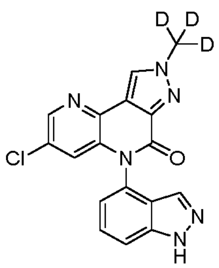 | B332 | 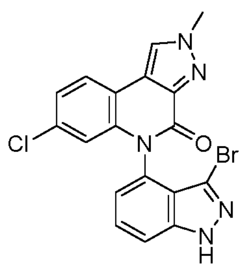 | B333 | 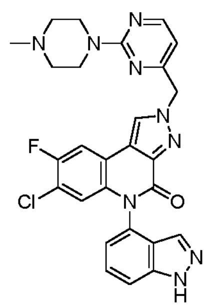 |

(continued)

| | | | | | |
|---|---|---|---|---|---|
| B334 | 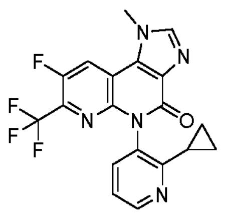 | B335 | 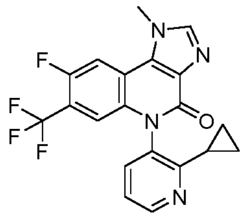 | B336 | 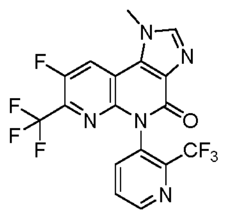 |
| B337 | 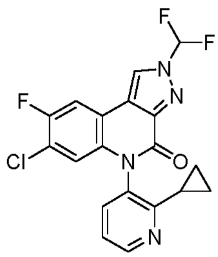 | B338 | 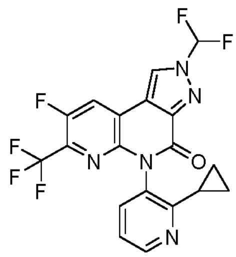 | B339 | 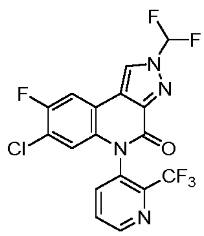 |
| B340 | 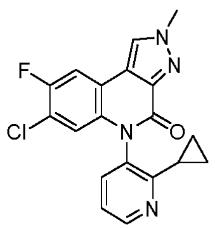 | B341 | 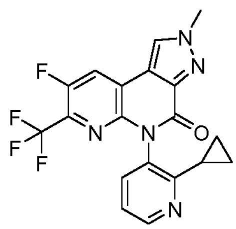 | B342 | 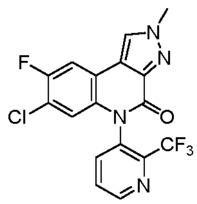 |
| B343 | 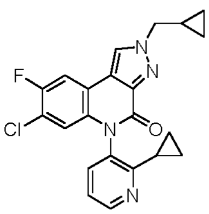 | B344 | 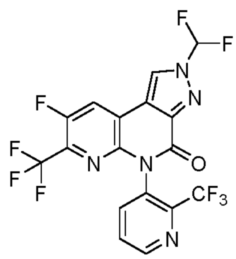 | B345 | 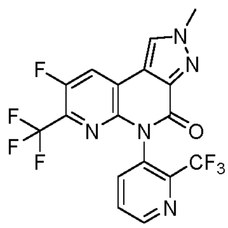 |
| B346 | 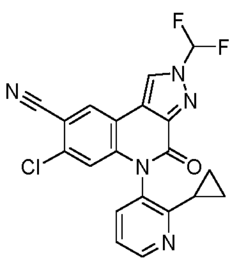 | B347 | 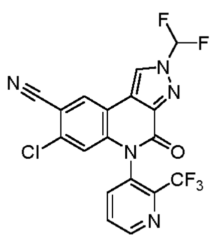 | B348 | 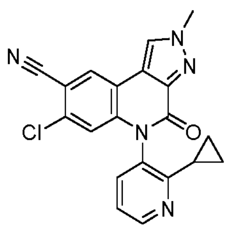 |
| B349 | 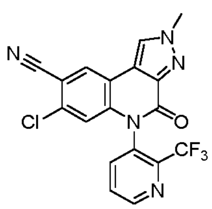 | B350 | 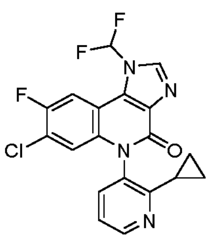 | B351 | 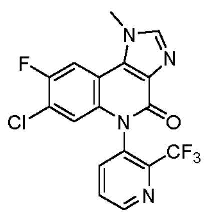 |

(continued)

| | | | | | |
|---|---|---|---|---|---|
| B352 | 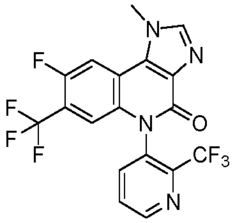 | B353 | 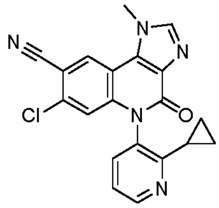 | B354 | 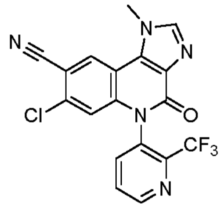 |
| B355 | 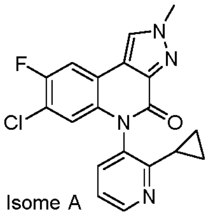 Isome A | B356 | 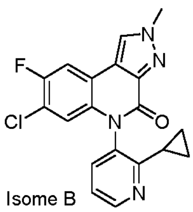 Isome B | B357 | 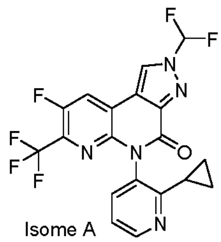 Isome A |
| B358 | 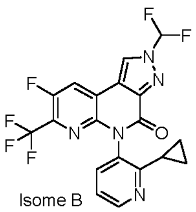 Isome B | B359 | 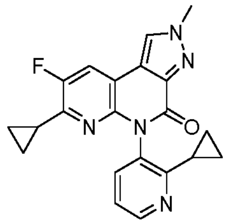 | B360 | 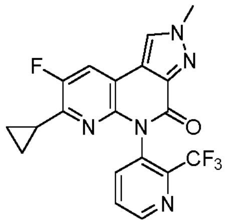 |
| B361 | 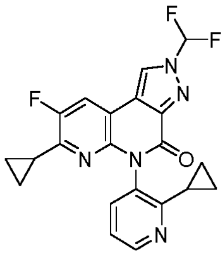 | B362 | 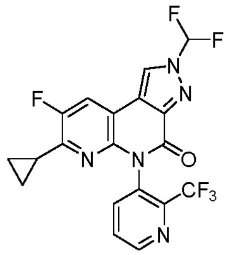 | B363 | 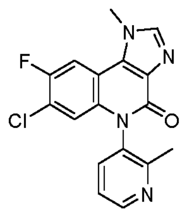 |
| B364 | 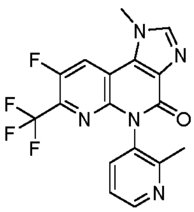 | B365 | 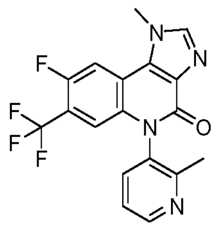 | B366 | 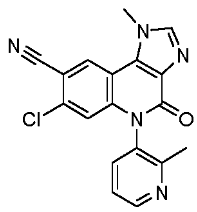 |
| B367 | 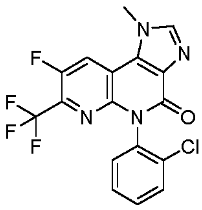 | B368 | 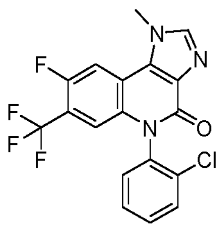 | B369 | 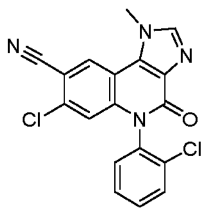 |
| B370 | 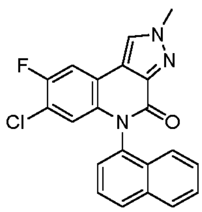 | B371 | 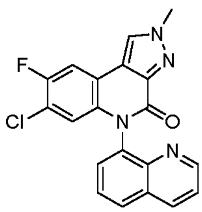 | B372 | 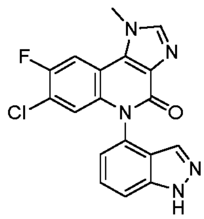 |

(continued)

| | | | | | |
|---|---|---|---|---|---|
| B373 | 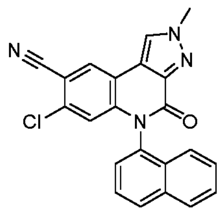 | B374 | 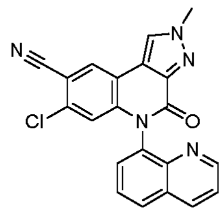 | B375 | 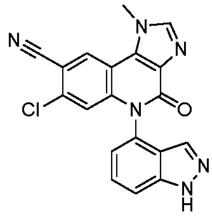 |
| B376 | 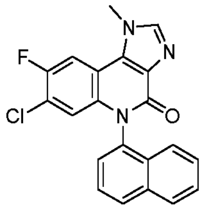 | B377 | 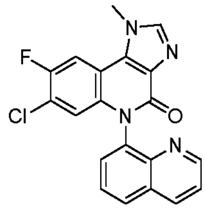 | B378 | 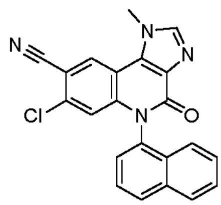 |
| B379 | 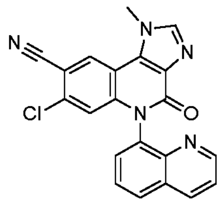 | B380 | 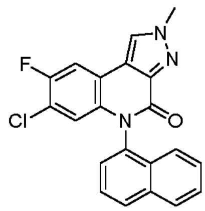 | B381 | 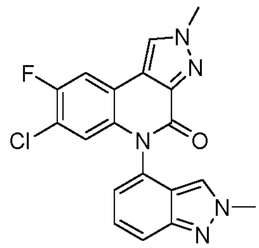 |
| B382 | 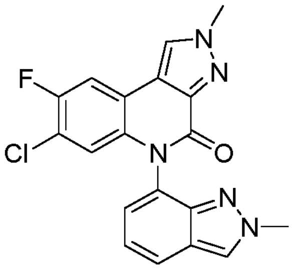 | B383 | 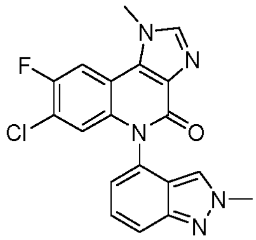 | B384 | 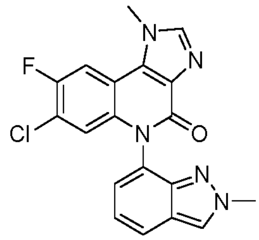 |
| B385 | 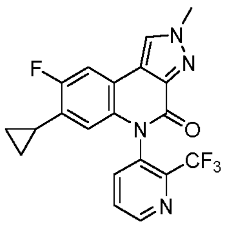 | B386 | 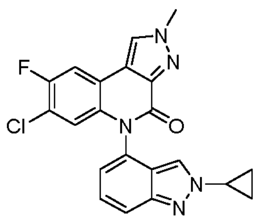 | B387 | 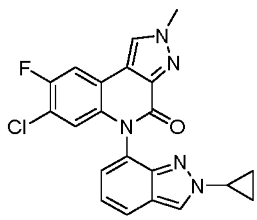 |
| B388 | 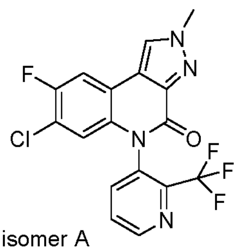 isomer A | B389 | 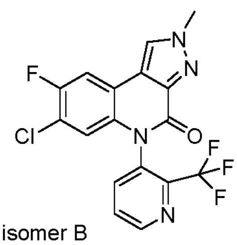 isomer B | B390 | 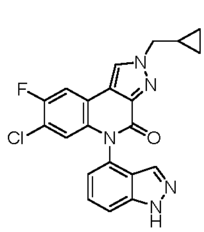 |
| B391 | 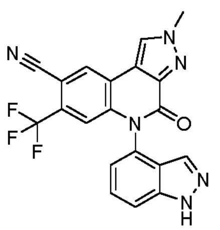 | B392 | 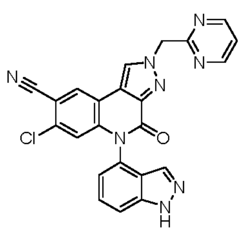 | B393 | 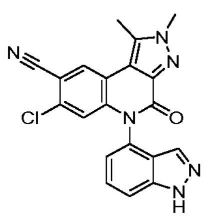 |

(continued)

| | | | | | |
|---|---|---|---|---|---|
| B394 | 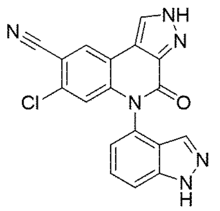 | B395 | 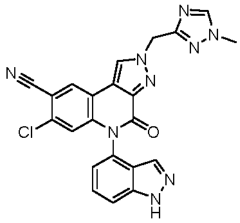 | B396 | 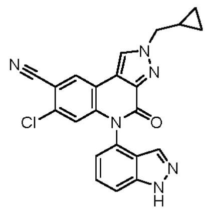 |
| B397 | 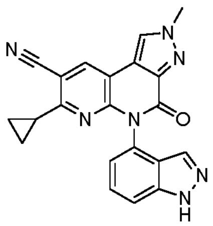 | B398 | 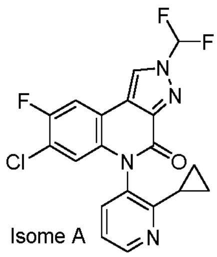 Isome A | B399 | 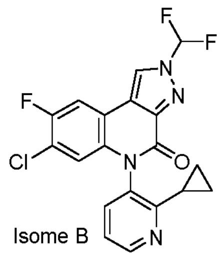 Isome B |
| B400 | 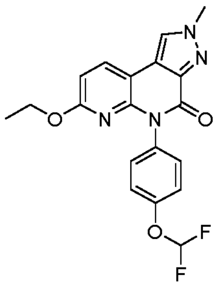 | B401 | 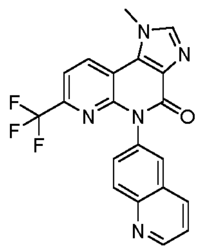 | B402 | 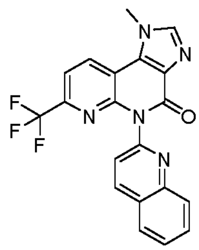 |
| B403 | 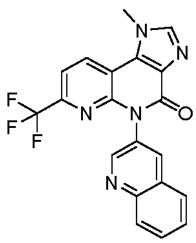 | B404 | 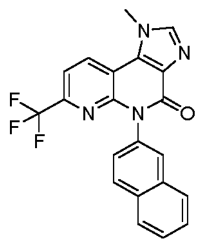 | B405 | 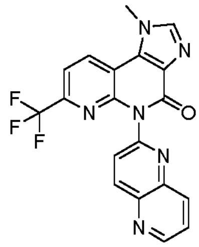 |
| B406 | 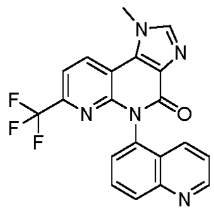 | B407 | 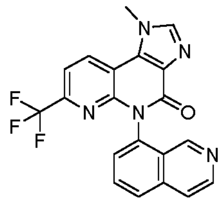 | B408 | 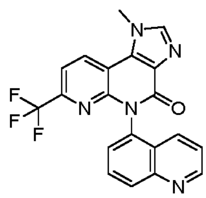 |
| B409 | 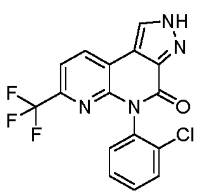 | B410 | 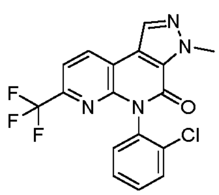 | B411 | 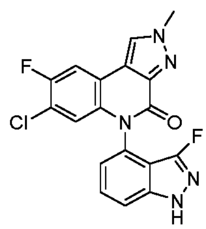 |

(continued)

| | | | | | |
|---|---|---|---|---|---|
| B412 | 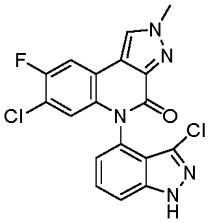 | B413 | 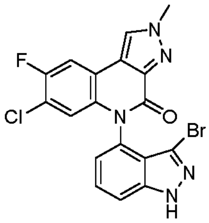 | B414 | 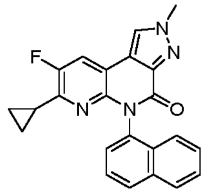 |
| B415 | 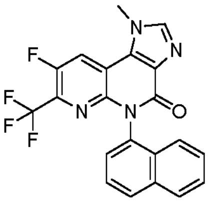 | B416 | 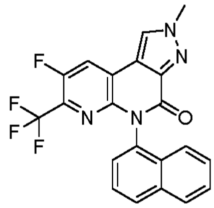 | B417 | 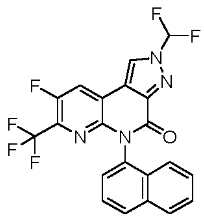 |
| B418 | 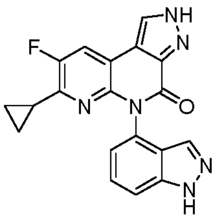 | B419 | 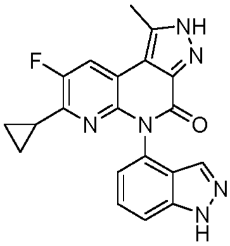 | B420 | 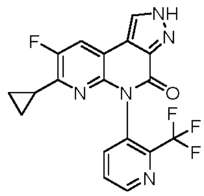 |
| B421 | 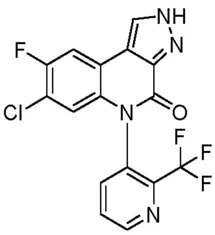 | B422 | 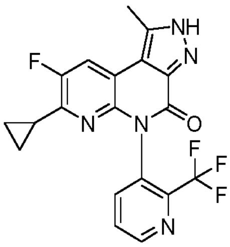 | B423 | 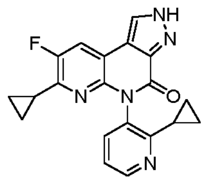 |
| B424 | 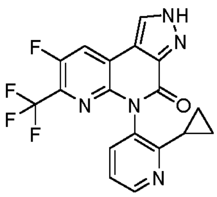 | B425 | 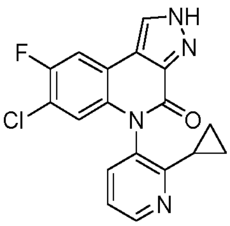 | B427 | 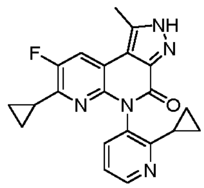 |
| B428 | 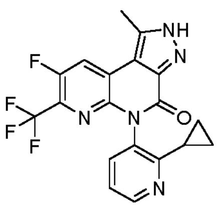 | B429 | 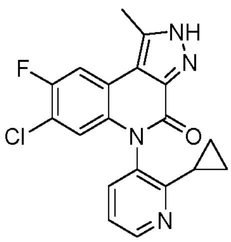 | B430 | 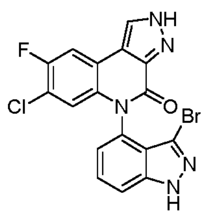 |

**27.** A pharmaceutical composition comprising a therapeutically effective amount of the compound or the stereoisomer, the tautomer, the solvate, the hydrate, the prodrug, the stable isotopic derivative, or the pharmaceutically acceptable salt thereof according to any one of claims 1-26, and one or more pharmaceutically acceptable carriers, diluents, or excipients.

**28.** The compound or the stereoisomer, the tautomer, the solvate, the hydrate, the prodrug, the stable isotopic derivative,

or the pharmaceutically acceptable salt thereof according to any one of claims 1-26, or the pharmaceutical composition according to claim 27 for use in inhibiting methionine adenosyltransferase 2A (MAT2A) or in preparing a methionine adenosyltransferase 2A (MAT2A) inhibitor drug.

**29.** The compound or the stereoisomer, the tautomer, the solvate, the hydrate, the prodrug, the stable isotopic derivative, or the pharmaceutically acceptable salt thereof according to any one of claims 1-26, or the pharmaceutical composition according to claim 27 for use in treating and/or preventing cancer, or in preparing a drug for treating and/or preventing cancer, wherein preferably, the cancer comprises those in which a gene encoding methylthioadenosine phosphorylase (MTAP) is deleted and/or is not fully functional.

**30.** A method for treating and/or preventing cancer, comprising administering to a patient a therapeutically effective amount of the compound or the stereoisomer, the tautomer, the solvate, the hydrate, the prodrug, the stable isotopic derivative, or the pharmaceutically acceptable salt thereof according to any one of claims 1-26, or the pharmaceutical composition according to claim 27, wherein preferably, the cancer comprises those in which a gene encoding methylthioadenosine phosphorylase (MTAP) is deleted and/or is not fully functional.

**INTERNATIONAL SEARCH REPORT**

International application No. **PCT/CN2023/073092**

**A. CLASSIFICATION OF SUBJECT MATTER**

C07D471/04(2006.01)i;C07D471/14(2006.01)i;A61K31/519(2006.01)i;A61K31/4745(2006.01)i;A61K31/4375(2006.01)i; A61P35/00(2006.01)i

According to International Patent Classification (IPC) or to both national classification and IPC

**B. FIELDS SEARCHED**

Minimum documentation searched (classification system followed by classification symbols)

C07D,A61K,A61P

Documentation searched other than minimum documentation to the extent that such documents are included in the fields searched

Electronic data base consulted during the international search (name of data base and, where practicable, search terms used)

WPI, CNTXT, CNKI, STN (REGISTRY, CAPLUS); MAT2A, MTAP, cancer, pyrazolo, quinolin 3w one, 甲硫氨酸腺苷转移酶, 甲硫腺苷磷酸化酶, 癌症, 吡唑并, 喹啉, 酮, STN中结构式检索, structural formula search in STN

**C. DOCUMENTS CONSIDERED TO BE RELEVANT**

| Category* | Citation of document, with indication, where appropriate, of the relevant passages | Relevant to claim No. |
|---|---|---|
| PX | WO 2022143864 A1 (JIANGSU SIMCERE PHARMACEUTICAL CO., LTD.) 07 July 2022 (2022-07-07)<br>claims 1-15, description, pages 16-26 | 1-30 |
| E | WO 2023016562 A1 (TYK MEDICINES, INC.) 16 February 2023 (2023-02-16)<br>claims 6-10 | 1, 27, 29-30 |
| X | WO 2006107771 A2 (3M INNOVATIVE PROPERTIES COMPANY et al.) 12 October 2006 (2006-10-12)<br>description, page 75, Part E | 1, 3-4, 6-10, 12-15 |
| X | ACS. "RN2227016-38-6等化合物 (Compounds, RN2227016-38-6 etc.)" *Registry,* 14 June 2018 (2018-06-14),<br>pp. 1-18 | 1, 3-7, 19-25 |
| A | WO 2020139992 A1 (AGIOS PHARMACEUTICALS, INC.) 02 July 2020 (2020-07-02)<br>abstract, claims 1-2, 35-38 | 1-30 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 May 2023** | **16 May 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/073092**

**C. DOCUMENTS CONSIDERED TO BE RELEVANT**

| Category* | Citation of document, with indication, where appropriate, of the relevant passages | Relevant to claim No. |
|---|---|---|
| A | WO 2020243376 A1 (AGIOS PHARMACEUTICALS, INC.) 03 December 2020 (2020-12-03) abstract, claims 1-2, 35-41 | 1-30 |
| A | CN 113166078 A (IDEAYA BIOSCIENCES INC.) 23 July 2021 (2021-07-23) entire document | 1-30 |
| A | CN 111936499 A (AGIOS PHARMACEUTICALS, INC.) 13 November 2020 (2020-11-13) entire document | 1-30 |

Form PCT/ISA/210 (second sheet) (July 2022)

**INTERNATIONAL SEARCH REPORT**

International application No.
**PCT/CN2023/073092**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **30**
because they relate to subject matter not required to be searched by this Authority, namely:

   The subject matter of claim 30 belongs to methods for treatment of diseases in the human body (PCT Rule 39.1(iv)). The search is carried out on the basis of the use of the compound or pharmaceutical composition in the preparation of a drug for treating corresponding diseases.

2. ☑ Claims Nos.: **1-7**
because they relate to parts of the international application that do not comply with the prescribed requirements to such an extent that no meaningful international search can be carried out, specifically:

   Claim 1 covers a large number of known compounds in the prior art, and claims 2-7 directly or indirectly refer to claim 1, which make it difficult for the examiner to perform an exhaustive search of the prior art for the scope of claims 1-7. The search is carried out on the basis of general formula (I) in claim 1 as the structural general formula defined in claims 8 and 19.

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/073092**

| Patent document cited in search report | | | Publication date (day/month/year) | Patent family member(s) | | | Publication date (day/month/year) |
|---|---|---|---|---|---|---|---|
| WO | 2022143864 | A1 | 07 July 2022 | TW | 202227443 | A | 16 July 2022 |
| WO | 2023016562 | A1 | 16 February 2023 | None | | | |
| WO | 2006107771 | A2 | 12 October 2006 | US | 2009069299 | A1 | 12 March 2009 |
| | | | | US | 8138173 | B2 | 20 March 2012 |
| | | | | AU | 2006232294 | A1 | 12 October 2006 |
| | | | | EP | 1863813 | A2 | 12 December 2007 |
| | | | | CA | 2603063 | A1 | 12 October 2006 |
| | | | | WO | 2006107771 | A3 | 04 October 2007 |
| | | | | JP | 2008538119 | A | 09 October 2008 |
| WO | 2020139992 | A1 | 02 July 2020 | None | | | |
| WO | 2020243376 | A1 | 03 December 2020 | None | | | |
| CN | 113166078 | A | 23 July 2021 | IL | 283743 | A | 29 July 2021 |
| | | | | KR | 20210103498 | A | 23 August 2021 |
| | | | | CO | 2021008895 | A2 | 30 July 2021 |
| | | | | CL | 2021001508 | A1 | 21 January 2022 |
| | | | | US | 11130759 | B1 | 28 September 2021 |
| | | | | US | 11084798 | B1 | 10 August 2021 |
| | | | | JP | 2022512156 | A | 02 February 2022 |
| | | | | TW | 202039487 | A | 01 November 2020 |
| | | | | SG | 11202105469 | YA | 29 June 2021 |
| | | | | US | 2022106276 | A1 | 07 April 2022 |
| | | | | PH | 12021551274 | A1 | 06 December 2021 |
| | | | | MA | 54452 | A | 16 March 2022 |
| | | | | BR | 112021010842 | A2 | 24 August 2021 |
| | | | | EP | 3894396 | A1 | 20 October 2021 |
| | | | | US | 11046691 | B1 | 29 June 2021 |
| | | | | CA | 3121236 | A1 | 18 June 2020 |
| | | | | AU | 2019395338 | A1 | 29 July 2021 |
| | | | | WO | 2020123395 | A1 | 18 June 2020 |
| CN | 111936499 | A | 13 November 2020 | MX | 2020010005 | A | 14 October 2020 |
| | | | | PH | 12020551507 | A1 | 13 September 2021 |
| | | | | TW | 201946629 | A | 16 December 2019 |
| | | | | TWI | 719437 | B | 21 February 2021 |
| | | | | US | 2021115045 | A1 | 22 April 2021 |
| | | | | US | 11524960 | B2 | 13 December 2022 |
| | | | | EP | 3774805 | A1 | 17 February 2021 |
| | | | | AR | 115326 | A1 | 23 December 2020 |
| | | | | EA | 202092320 | A1 | 09 March 2021 |
| | | | | MA | 52232 | A | 17 February 2021 |
| | | | | AU | 2019243289 | A1 | 24 September 2020 |
| | | | | AU | 2019243289 | B2 | 12 January 2023 |
| | | | | CA | 3094476 | A1 | 03 October 2019 |
| | | | | SG | 11202009195 | WA | 29 October 2020 |
| | | | | KR | 20200138769 | A | 10 December 2020 |
| | | | | BR | 112020020104 | A2 | 26 January 2021 |
| | | | | IL | 277665 | A | 30 November 2020 |
| | | | | JP | 2021519783 | A | 12 August 2021 |
| | | | | WO | 2019191470 | A1 | 03 October 2019 |
| | | | | WO | 2019191470 | A8 | 12 December 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **RUIZHI WANG et al.** *Molecular Carcinogenesis*, 2018, vol. 57 (9), 1201-1212
- **T. LI et al.** *J. Cancer*, 2016, vol. 7 (10), 1317-1327
- **T. ZHANG et al.** *Acta Histochemica*, 2013, vol. 115, 48-55
- **K MARJON et al.** found that S-methyl-5'-thioadenosine phosphorylase (MTAP)-deficient cancer was more sensitive to signaling pathways targeting MAT2A/PRMT5/RIOK1. *Cell Reports*, 2016, vol. 15 (3), 574-587
- *CHEMICAL ABSTRACTS*, 2201056-66-6